# EUROPEAN PATENT SPECIFICATION

(11) **EP 1 381 590 B1**
(45) Date of publication and mention of the grant of the patent: **20.06.2007**
(21) Application number: 02739172.1
(22) Date of filing: 15.04.2002
(51) Int. Cl.: C07C 237/30, C07C 255/59, C07C 225/20, C07D 213/74, C07D 307/52, C07D 333/36

(54) **3,4-DI-SUBSTITUTED CYCLOBUTENE-1,2-DIONES AS CXC-CHEMOKINE RECEPTOR LIGANDS**
3,4-DISUBSTITUIERTE CYCLOBUTEN-1,2-DIONE ALS CXC-CHEMOKINREZEPTORLIGANDEN
CYCLOBUTÈNE-1,2-DIONES DISUBSTITUÉES EN POSITIONS 3,4 UTILISÉES COMME LIGANDS DE RECEPTEURS DE LA CHIMIOKINE CXC

(30) Priority: 16.04.2001 US 284026 P
(43) Date of publication of application: 21.01.2004
(73) Proprietor: SCHERING CORPORATION, Kenilworth, NJ 07083-0530 (US); Pharmacopeia Drug Discovery, Inc., Cranbury, New Jersey 08512 (US)
(72) Inventor: TAVERAS, Arthur, G., Denville, NJ 07834 (US); AKI, Cynthia, J., Livingston, NJ 07039 (US); BOND, Richard, W., Union, NJ 07083 (US); CHAO, Jianping, Summit, NJ 07901 (US); DWYER, Michael, Scotch Plains, NJ 07076 (US); FERREIRA, Johan, A., Bensalem, PA 19020 (US); CHAO, Jianhua, Pompton Lakes, NJ 07442 (US); YU, Younong, Piscataway, NJ 08854 (US); BALDWIN, John, J., Gwynedd Valley, PA 19437 (US); KAISER, Bernd, Wallingford, CT 06492 (US); LI, Ge, LuJuaZui Garden 20135 Shanghai (CN); MERRITT, J., Robert, Ewing, NJ 08618 (US); NELSON, Kingsley, H., Mebane, NC 27302 (US); ROKOSZ, Laura, L., Union, NJ 07083 (US)
(74) Representative: UEXKÜLL & STOLBERG
(86) International application number: PCT/US2002/012681
(87) International publication number: WO 2002/083624

(56) References cited:
- WO-A-00/35855
- WO-A-01/64208
- WO-A-95/14005
- WO-A-96/14300
- WO-A-96/15103
- WO-A-98/33763
- BUTERA J A ET AL: "Design and SAR of Novel Potassium Channel Openers Targeted for Urge Urinary Incontinence.1. N-Cyanoguanidine Bioisosteres Possessing in Vivo Bladder Selectivity" JOURNAL OF MEDICINAL CHEMISTRY, AMERICAN CHEMICAL SOCIETY. WASHINGTON, US, vol. 43, no. 6, February 2000 (2000-02), pages 1187-1202, XP002168956 ISSN: 0022-2623
- PATENT ABSTRACTS OF JAPAN vol. 018, no. 361 (C-1222), 7 July 1994 (1994-07-07) & JP 06 092915 A (SUMITOMO METAL IND LTD), 5 April 1994 (1994-04-05)

## Description

### CROSS REFERENCE TO RELATED APPLICATION

This application claims the benefit of US Provisional Application 60/284,026, filed April 16, 2001.

### FIELD OF THE INVENTION

The present invention relates to novel substituted cyclobutenedione compounds, pharmaceutical compositions containing the compounds, and the use of the compounds and formulations in treating CXC chemokine-mediated diseases.

### BACKGROUND OF THE INVENTION

Chemokines are chemotactic cytokines that are released by a wide variety of cells to attract macrophages, T-cells, eosinophils, basophils, neutrophils and endothelial cells to sites of inflammation and tumor growth. There are two main classes of chemokines, the CXC-chemokines and the CC- chemokines. The class depends on whether the first two cysteines are separated by a single amino acid (CXC-chemokines) or are adjacent (CC-chemokines). The CXC-chemokines include interleukin-8 (IL-8), neutrophil-activating protein-1 (NAP-1), neutrophil-activating protein-2 (NAP-2), GROα, GROβ, GROγ, ENA-78, GCP-2, IP-10, MIG and PF4. CC chemokines include RANTES, MIP-1α, MIP-2β, monocyte chemotactic protein-1 (MCP-1), MCP-2, MCP-3 and eotaxin. Individual members of the chemokine families are known to be bound by at least one chemokine receptor, with CXC-chemokines generally bound by members of the CXCR class of receptors, and CC-chemokines by members of the CCR class of receptors. For example, IL-8 is bound by the CXCR-1 and CXCR-2 receptors.

Since CXC-chemokines promote the accumulation and activation of neutrophils, these chemokines have been implicated in a wide range of acute and chronic inflammatory disorders including psoriasis and rheumatoid arthritis. Baggiolini et al., FEBS Lett. 307, 97 (1992); Miller et al., Crit. Rev. Immunol. 12, 17 (1992); Oppenheim et al., Annu. Fev. Immunol. 9, 617 (1991); Seitz et al., J. Clin. Invest. 87, 463 (1991); Miller et al., Am. Rev. Respir. Dis. 146, 427 (1992); Donnely et al., Lancet 341, 64.3 (1993).

ELRCXC chemokines including IL-8, GROα, GROβ, GROγ, NAP-2, and ENA-78 (Strieter et al. 1995 JBC 270 p. 27348-57) have also been implicated in the induction of tumor angiogenesis (new blood vessel growth). All of these chemokines are believed to exert their actions by binding to the 7 transmembrane G-protein coupled receptor CXCR2 (also known as IL-8RB), while IL-8 also binds CXCR1 (also known as IL-8RA). Thus, their angiogenic activity is due to their binding to and activation of CXCR2, and possible CXCR1 for IL-8, expressed on the surface of vascular endothelial cells (ECs) in surrounding vessels.

Many different types of tumors have been shown to produce ELRCXC chemokines and their production has been correlated with a more aggressive phenotype (Inoue et al. 2000 Clin Cancer Res 6 p. 2104-2119) and poor prognosis (Yoneda et. al. 1998 J Nat Cancer Inst 90 p. 447-454). Chemokines are potent chemotactic factors and the ELRCXC chemokines have been shown to induce EC chemotaxis. Thus, these chemokines probably induce chemotaxis of endothelial cells toward their site of production in the tumor. This may be a critical step in the induction of angiogenesis by the tumor. Inhibitors of CXCR2 or dual inhibitors of CXCR2 and CXCR1 will inhibit the angiogenic activity of the ELRCXC chemokines and therefore block the growth of the tumor. This anti-tumor activity has been demonstrated for antibodies to IL-8 (Arenberg et al. 1996 J Clin Invest 97 p. 2792-2802), ENA-78 (Arenberg et al. 1998 J Clin Invest 102 p. 465-72), and GROα (Haghnegahdar et al. J. Leukoc Biology 2000 67 p. 53-62).

Many tumor cells have also been shown to express CXCR2 and thus tumor cells may also stimulate their own growth when they secrete ELRCXC chemokines. Thus, along with decreasing angiogenesis, inhibitors of CXCR2 may directly inhibit the growth of tumor cells.

Hence, the CXC-chemokine receptors represent promising targets for the development of novel anti-inflammatory and anti-tumor agents.

There remains a need for compounds that are capable of modulating activity at CXC-chemokine receptors. For example, conditions associated with an increase in IL-8 production (which is responsible for chemotaxis of neutrophil and T-cell subsets into the inflammatory site and growth of tumors) would benefit by compounds that are inhibitors of IL-8 receptor binding.

### SUMMARY OF THE INVENTION

This invention provides compounds of the formula (I): 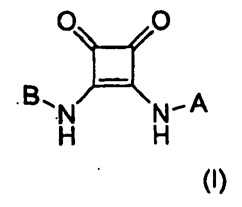 or a pharmaceutically acceptable salt or solvate thereof wherein
A is selected from the group consisting of 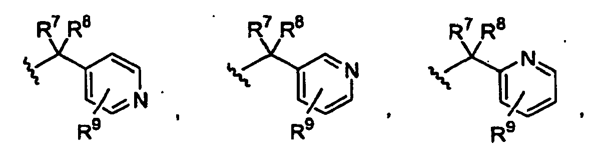 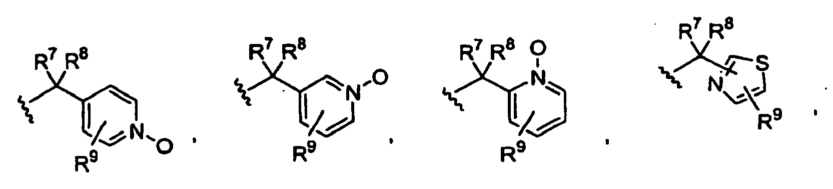 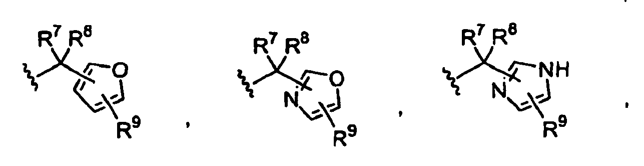 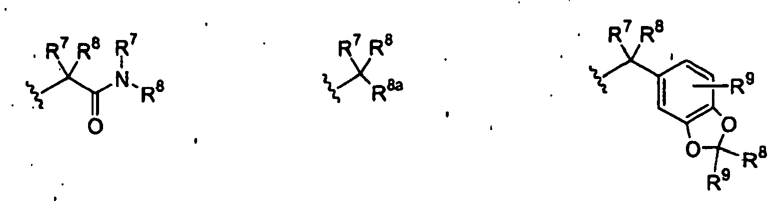 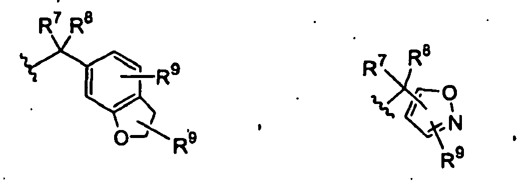 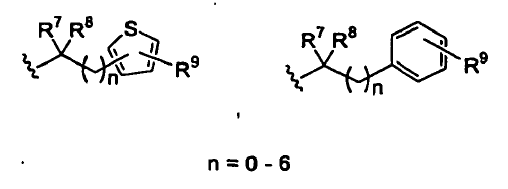 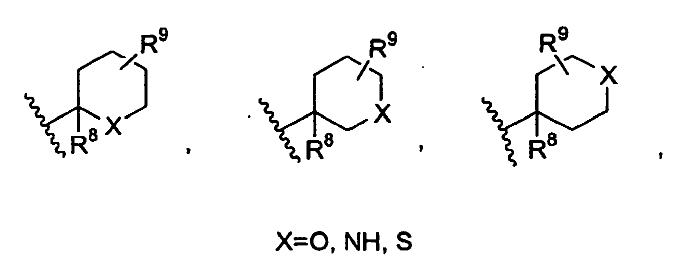 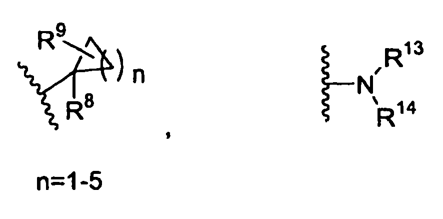 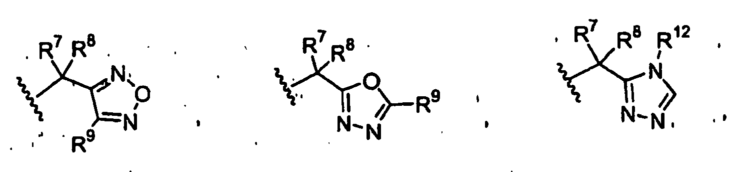 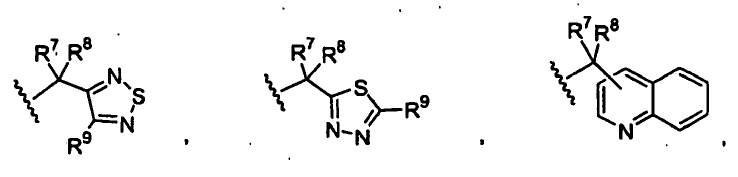 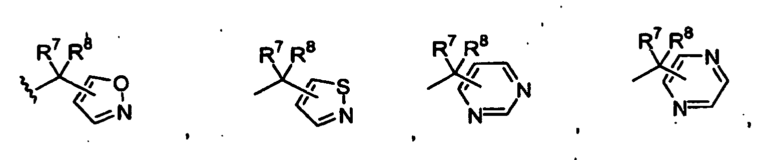 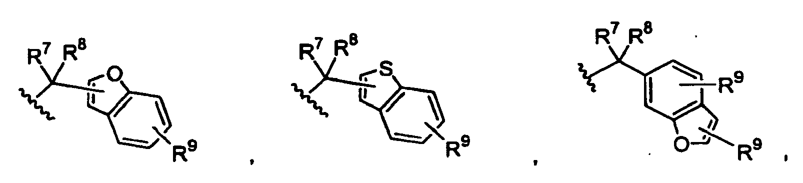 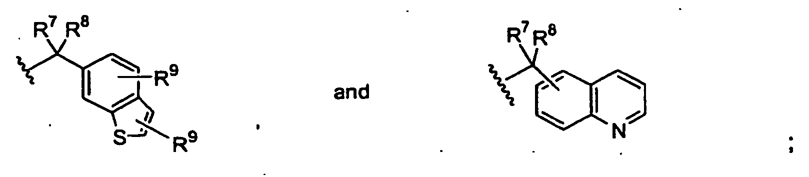 B is selected from the group consisting of 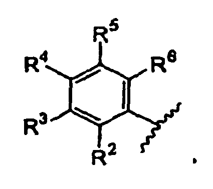 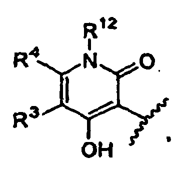 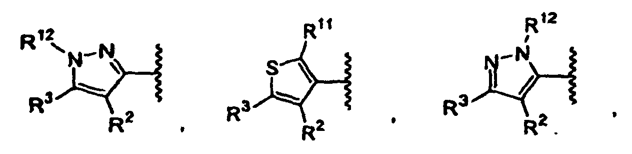 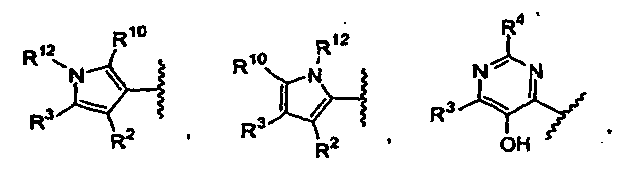 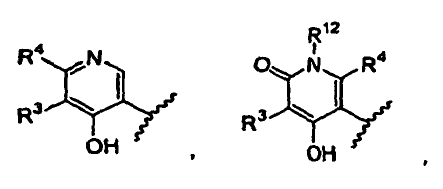 and 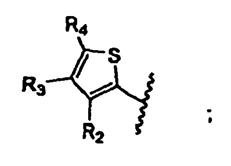 R² is OH;
R³ is selected from the group consisting of -C(O)NHR¹⁷, -C(O)NR¹³R¹⁴ and -C(O)NR¹³OR¹⁴; 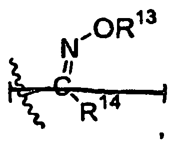 R⁴ is selected from the group consisting of hydrogen, cyano, halogen, alkyl, alkoxy, -OH, -CF₃, -OCF₃, -NO₂, -C(O)R¹³, -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, 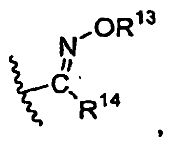 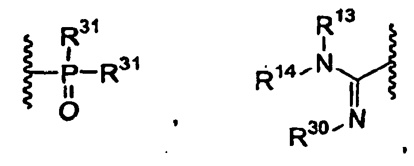 -NHC(O)R¹⁷, unsubstituted or substituted aryl and unsubstituted or substituted heteroaryl,
wherein the substituents on the substituted R³ and R⁴ groups are the same or different and independently selected from 1-6 R⁹ groups;
R⁵ and R⁶ are the same or different and are independently selected from the group consisting of hydrogen, halogen, alkyl, alkoxy, -CF₃, -OCF₃, -NO₂, -C(O)R¹³, -C(O)OR¹³, -C(O)NR¹³R¹⁴, -SO₍ₜ₎NR¹³R¹⁴, -C(O)NR¹³OR¹⁴, cyano, an unsubstituted or substituted aryl and unsubstituted or substituted heteroaryl group,
wherein the substituents on the substituted R⁵ and R⁶ groups are the same or different and independently selected from 1-6 R⁹ groups;
R⁷ and R⁸ are the same or different and are independently selected from the group consisting of H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, -CO₂R¹³, -CONR¹³R¹⁴, fluoroalkyl, alkynyl, alkynylalkyl, alkenyl, alkenylalkyl and cycloalkenyl,
wherein the substituents on the substituted R⁷ and R⁸ groups are selected from the group consisting of
a) H,
b) halogen,
c) -CF₃,
d) -COR¹³,
e) -OR¹³,
f) -NR¹³R¹⁴,
g) -NO₂,
h) -CN,
i) -SO₂OR¹³,
j) -Si(alkyl)₃,
k) -Si(aryl)₃,
l) -(R¹³)₂R¹⁴Si,
m) -CO₂R¹³,
n) -C(O)NR¹³R¹⁴,
o) -SO₂NR¹³R¹⁴,
p) -SO₂R¹³,
q) -O(C=O)R¹³,
r) -O(C=O)NR¹³R¹⁴,
s) -NR¹³C(O)R¹⁴, and
t) -NR¹³CO₂¹⁴;
R^{8a} is selected from the group consisting of hydrogen, alkyl, cycloalkyl and cycloalkylalkyl;
R⁹ is independently selected from 1-6 of the group consisting of:
a) -R¹³,
b) halogen,
c) -CF₃,
d) -COR¹³,
e) -OR¹³,
f) -NR¹³R¹⁴,
g) -NO₂,
h) -CN,
i) -SO₂R¹³,
j) -SO₂NR¹³R¹⁴,
k) -NR¹³COR¹⁴,
l) -CONR¹³R¹⁴,
m)-NR¹³CO₂R¹⁴,
n) -CO₂R¹³, and
o) 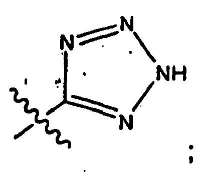
R¹⁰ and R¹¹ are the same or different and are independently selected from the group consisting of hydrogen, halogen, -CF₃, -OCF₃, -NR¹³R¹⁴, -NR¹³C(O)NR¹³R¹⁴, -OH, -C(O)OR¹³, -SH, -SO₍ₜ₎NR¹³R¹⁴, -SO₂R¹³, -NHC(O)R¹³, -NHSO₂NR¹³R¹⁴, -NHSO₂R¹³, -C(O)NR¹³R¹⁴, -C(O)NR¹³OR¹⁴, -OC(O)R¹³ and cyano;
R¹² is hydrogen, -OC(O)R¹³, or an unsubstituted or substituted aryl, unsubstituted or substituted heteroaryl, unsubstituted or substituted arylalkyl, unsubstituted or substituted cycloalkyl, unsubstituted or substituted alkyl, unsubstituted or substituted cycloalkylalkyl or unsubstituted or substituted heteroarylalkyl group,
wherein the substituents on the substituted R¹² groups are the same or different and independently selected from 1-6 R⁹ groups;
R¹³ and R¹⁴ are the same or different and are independently selected from the group consisting of H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, and unsubstituted or substituted fluoroalkyl, or
R¹³ and R¹⁴ can be taken together when both are attached to a nitrogen atom to form an unsubstituted or substituted 3 to 7 membered heterocylic ring containing one to two heteroatoms selected from oxygen, sulfur and nitrogen,
wherein the substitutents on the substituted R¹³ and R¹⁴ groups are the same or different and independently selected from 1-6 of H, alkyl, aryl, arylalkyl, fluroalkyl, cycloalkyl, cycloalkylalkyl, heteroaryl, heteroarylalkyl, amino, -C(Q)OR¹⁵, -C(O)NR¹⁵R¹⁶, -S(O)ₜNR¹⁵R¹⁶, -C(O)R¹⁵, -SO₂R¹⁵, -NHC(O)NR ¹³R¹⁴ and halogen;
R¹⁵ and R¹⁶ are the same or different and are independently selected from the group consisting of H, alkyl, aryl, arylalkyl, cycloalkyl and heteroaryl;
R¹⁷ is -SO₂ alkyl, -SO₂ aryl, -SO₂ cycloalkyl or-SO₂heteroaryl;
R³⁰ is alkyl, cycloalkyl; -CN, -NO₂, or -SO₂R¹⁵;
R³¹ are the same or different and are independently selected from the group consisting of unsubstituted or substituted alkyl, unsubstituted or substituted aryl, unsubstituted or substituted heteroaryl and unsubstituted or substituted cycloalkyl;
wherein the substituents on the substituted R³¹ groups are the same or different and independently selected from 1-6 R⁹ groups; and
t is 0, 1 or 2.

### DETAILED DESCRIPTION OF THE INVENTION

This invention provides compounds of the formula (I): 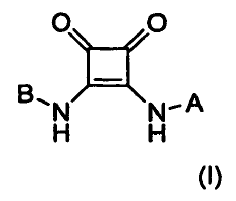 or a pharmaceutically acceptable salt or solvate thereof wherein the substituents are as defined in the Summary of the Invention.

Another aspect of the present invention is a pharmaceutical composition comprising the compound of formula (I) in combination or association with a pharmaceutically acceptable carrier or diluent.

Another aspect of the present invention is a method of treating an α-chemokine mediated disease in a mammal which comprises administering to a patient in need thereof of a therapeutically effective amount of the compound of formula (I), or a pharmaceutically acceptable salt or solvate thereof.

Examples of chemokine mediated diseases include psoriasis, atopic dermatitis, asthma, chronic obstructive pulmonary disease, adult respiratory distress syndrome, arthritis, inflammatory bowel disease, Crohn's disease, ulcerative colitis, septic shock, endotoxic shock, gram negative sepsis, toxic shock syndrome, stroke, cardiac and renal reperfusion injury, glomerulo-nephritis or thrombosis, alzheimer's disease, graft vs. host reaction, allograft rejections and malaria.

Another aspect of the present invention is a method of treating cancer, comprising administering to a patient in need thereof, concurrently or sequentially, a therapeutically effective amount of (a) a compound of formula (I), and (b) a microtubule affecting agent or antineoplastic agent or anti-angiogenesis agent or VEGF receptor kinase inhibitor or antibodies against the VEGF receptor or interferon, and/or c) radiation.

In further embodiments, a compound of formula (I) is combined with one of the following antineoplastic agents: gemcitabine, paclitaxel (Taxol®), 5-Fluorouracil (5-FU), cyclophosphamide (Cytoxan®), temozolomide, taxotere or Vincristine.

In another embodiment, the present invention provides a method of treating cancer, comprising administering, concurrently or sequentially, an effective amount of (a) a compound of formula (I), and (b) a microtubule affecting agent (*e.g.*, paclitaxel).

Unless indicated otherwise, the following definitions apply throughout the present specification and claims. These definitions apply regardless of whether a term is used by itself or in combination with other terms. Hence the definition of "alkyl" applies to "alkyl" as well as to the "alkyl" portions of "alkoxy", etc.

When any variable (*e.g.*, aryl, R²) occurs more than one time in any constituent, its definition on each occurrence is independent of its definition at every other occurrence. Also, combinations of substituents and/or variables are permissible only if such combinations result in stable compounds.

"Patient" includes both human and other mammals.

"Mammal" means humans and other animals. Preferably, mammal means humans.

"Alkyl" means a straight or branched saturated hydrocarbon chain having the designated number of carbon atoms. Where the number of carbon atoms is not specified, 1 to 20 carbons are intended. Preferred alkyl groups contain 1 to 12 carbon atoms in the chain. More preferred alkyl groups contain 1 to 6 carbon atoms in the chain.

"Alkoxy" means an alkyl-O group in which alkyl is as previously defined. Non-limiting examples of alkoxy groups include methoxy, ethoxy, n-propoxy, iso-propoxy and n-butoxy. The bond to the parent moiety is through the ether oxygen.

"Alkenyl" means an aliphatic hydrocarbon group containing at least one carbon-carbon double bond and which may be straight or branched. Where the number of carbon atoms is not specified, 2 to 20 carbons are intended. Preferred alkenyl groups have 2 to 12 carbon atoms in the chain; and more preferably 2 to 6 carbon atoms in the chain. Non-limiting examples of suitable alkenyl groups include ethenyl, propenyl, n-butenyl, 3-m ethyl but-2-enyl, n-pentenyl, octenyl and decenyl.

"Alkynyl" means an aliphatic hydrocarbon group containing at least one carbon-carbon triple bond and which may be straight or branched. Where the number of carbon atoms is not specified, 2 to 15 carbons are intended. Preferred alkynyl groups have 2 to 12 carbon atoms in the chain; and more preferably 2 to 4 carbon atoms in the chain. Non-limiting examples of suitable alkynyl groups include ethynyl, propynyl, 2-butynyl, 3-methylbutynyl, n-pentynyl, and decynyl.

"Aryl" means an aromatic monocyclic or multicyclic ring system comprising about 6 to about 14 carbon atoms, preferably about 6 to about 10 carbon atoms. Non-limiting examples of suitable aryl groups include phenyl, naphthyl, indenyl, tetrahydronaphthyl, indanyl, anthracenyl, fluorenyl and the like.

"Arylalkyl" means an aryl-alkyl group in which the aryl and alkyl groups are as defined. Non-limiting examples of suitable arylalkyl groups include benzyl, phenethyl and naphthleneylmethyl. The bond to the parent moiety is through the alkyl group.

"Cycloalkyl" means a non-aromatic ring system having 3 to 10 carbon atoms and one to three rings, preferably 5 to 10 carbon atoms. Preferred cycloalkyl rings contain 5 to 7 ring atoms. Non-limiting examples of cycloalkyl groups include cyclopropyl, cyclopentyl, cyclohexyl, cycloheptyl, norbornyl, adamantyl and the like.

"Cycloalkylalkyl" means a cycloalkyl group attached to the parent moiety through an alkyl group. Non -limiting examples include cyclopropylmethyl, cyclohexylmethyl and the like.

"Cycloalkenyl" means a non-aromatic mono or multicyclic ring system comprising 3 to 10 carbon atoms, preferably 5 to 10 carbon atoms which contains at least one carbon-carbon double bond. Preferred cycloalkenyl rings contain 5 to 7 ring atoms. Non-limiting examples of cycloalkyl groups include cyclopentenyl, cyclohexenyl, cycloheptenyl, norbornenyl and the like.

"Cycloalkenyl" means a non-aromatic mono or multicyclic ring system comprising 3 to 10 carbon atoms, preferably 5 to 10 carbon atoms which contains at least one carbon-carbon double bond. Preferred cycloalkenyl rings contain 5 to 7 ring atoms. Non-limiting examples of cycloalkyl groups include cyclopentenyl, cyclohexenyl, cycloheptenyl, norbornenyl and the like.

"Halo" means fluoro, chloro, bromo, or iodo groups. Preferred are fluoro, chloro or bromo, and more preferred are fluoro and chloro.

"Halogen" means fluorine, chlorine, bromine, or iodine. Preferred are fluorine, chlorine or bromine, and more preferred are fluorine and chlorine.

"Haloalkyl" means an alkyl group as defined above wherein one or more hydrogen atoms on the alkyl is replaced by a halo group defined above.

"Heterocyclyl" or "heterocyclic" means a non-aromatic saturated monocyclic or multicyclic ring system comprising 3 to 10 ring atoms, preferably 5 to 10 ring atoms, in which one or more of the atoms in the ring system is an element other than carbon, for example nitrogen, oxygen or sulfur, alone or in combination. There are no adjacent oxygen and/or sulfur atoms present in the ring system. Preferred heterocyclyls contain 5 to 6 ring atoms. The prefix aza, oxa or thia before the heterocyclyl root name means that at least a nitrogen, oxygen or sulfur atom respectively is present as a ring atom.

The nitrogen or sulfur atom of the heterocyclyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, thiazolidinyl, 1,3-dioxolanyl, 1,4-dioxanyl, tetrahydrofuranyl, tetrahydrothiophenyl, tetrahydrothiopyranyl, and the like.

The term heterocyclic acidic functional group is intended to include groups such as, pyrrole, imidazole, triazole, tetrazole, and the like.

"Heteroaryl" means an aromatic monocyclic or multicyclic ring system comprising 5 to 14 ring atoms, preferably 5 to 10 ring atoms, in which one or more of the ring atoms is an element other than carbon, for example nitrogen, oxygen or sulfur, alone or in combination. Preferred heteroaryls contain 5 to 6 ring atoms. The prefix aza, oxa or thia before the heteroaryl root name means that at least a nitrogen, oxygen or sulfur atom respectively, is present as a ring atom. A nitrogen atom of a heteroaryl can be optionally oxidized to the corresponding N-oxide. Non-limiting examples of suitable heteroaryls include pyridyl, pyrazinyl, furanyl, thienyl, pyrimidinyl, isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like.

"Heteroarylalkyl" means a heteroaryl-alkyl group where the bond to the parent moiety is through an alkyl group.

N-oxides can form on a tertiary nitrogen present in an R substituent, or on =N-in a heteroaryl ring substituent and are included in the compounds of formula I.

The term "prodrug," as used herein, represents compounds which are rapidly transformed *in vivo* to the parent compound of the above formula, for example, by hydrolysis in blood. A thorough discussion is provided in T. Higuchi and V. Stella, Prodrugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

As used herein, the term "composition" is intended to encompass a product comprising the specified ingredients in the specified amounts, as well as any product which results, directly or indirectly, from combination of the specified ingredients in the specified amounts. Also, "Bn" represents benzyl.

R¹³ and R¹⁴ when taken together with the nitrogen they are attached to in the groups -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴, -NHSO₂NR¹³R¹⁴ preferably form an unsubstituted or substituted 3 to 7 membered, saturated heterocyclic ring optionally containing one or two additional heteroatoms each independently selected from O, S or NR¹⁸ wherein R¹⁸ is selected from H, alkyl, aryl, heteroaryl, -C(O)R¹⁹, -SO₂R¹⁹ and -C(O)NR¹⁹R²⁰, wherein R¹⁹ and R²⁰ are the same or different and each is independently selected from alkyl, aryl and heteroaryl, wherein the substituents on the substituted cyclized R¹³ and R¹⁴ groups are the same or different and independently selected from 1 to 3 of alkyl, aryl, hydroxy, hydroxyalkyl, alkoxy, alkoxyalkyl, arylalkyl, fluoroalkyl, cycloalkyl, cycloalkylalkyl, heteroaryl, heteroarylalkyl, amino, -C(O)OR¹⁵, -C(O)NR¹⁵R¹⁶, -SOₜNR¹⁵R¹⁶, -C(O)R¹⁵, -SO₂R¹⁵, -NHC(O)NR¹⁵R¹⁶ and halogen, and wherein R¹⁵ and R¹⁶ are the same or different and are independently selected from the group consisting of H, alkyl, aryl, arylalkyl, cycloalkyl and heteroaryl.

In a preferred group of compounds of formula (1),
A is selected from the group consisting of: 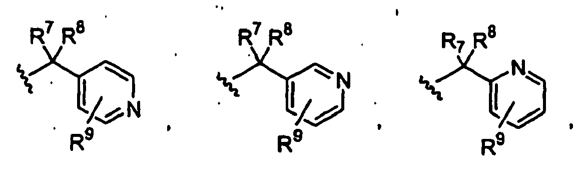 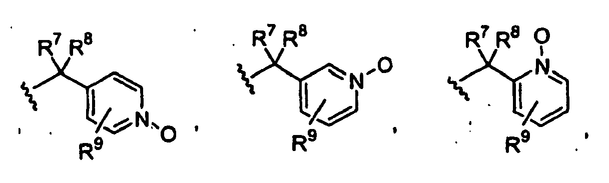 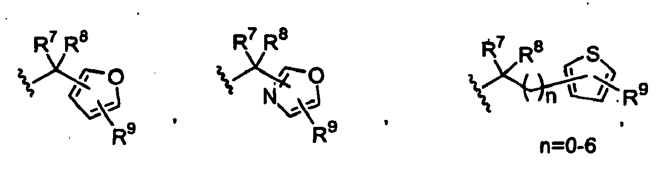 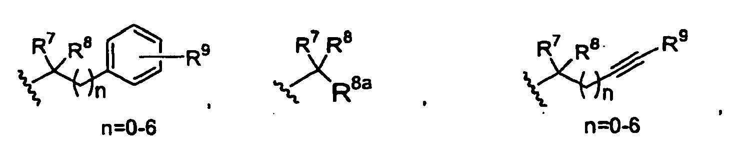 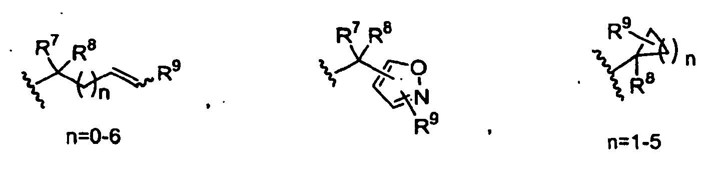 and 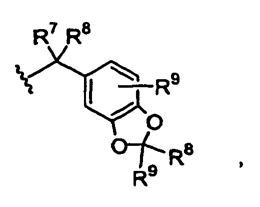 wherein,
R⁷ and R⁸ are the same or different and are independently selected from the group consisting of H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, -CO₂R¹³, -CONR¹³R¹⁴, fluoroalkyl, alkynyl, alkenyl, and cycloalkenyl,
wherein said substituents on said R⁷ and R⁸ substituted groups are selected from the group consisting of:
a) cyano,
b) -CO₂R¹³,
c) -C(O)NR¹³R¹⁴,
d) -SO₂NR¹³R¹⁴,
e) -NO₂,
f) -CF₃,
g) -OR¹³,
h) -NR¹³R¹⁴,
i) -O(C=O)R¹³,
j) -O(C=O)NR¹³R¹⁴, and
k) halogen; and
B is selected from the group consisting of: 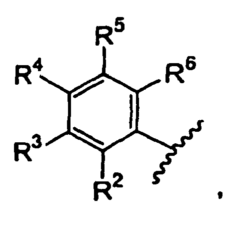 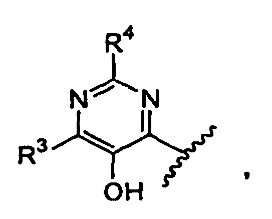 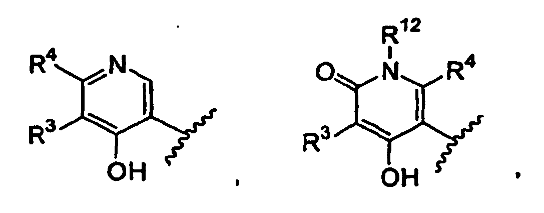 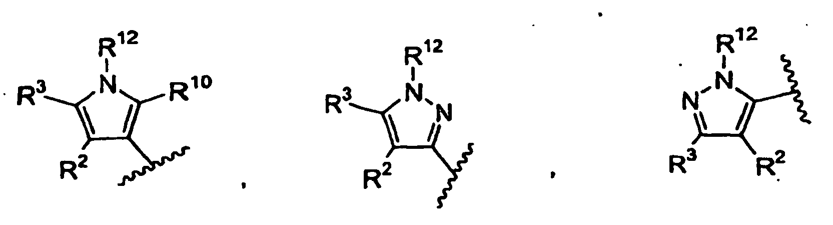 and 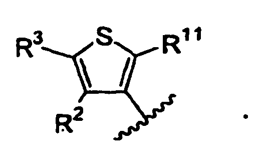 wherein R² to R⁶ and R¹⁰ to R¹⁴ are as defined above.

More preferably,
R⁷ and R⁸ are the same or different and are independently selected from H, alkyl, fluoroalkyl such as, -CF₃ and -CF₂CH₃; cycloalkyl and cycloalkylalkyl such as, for example, methyl, ethyl, t-butyl, isopropyl, cyclopropyl, cyclopropylmethyl and cyclohexyl, and
R⁹ is the same or different and is 1-3 moieties selected from the group consisting of H, halogen, alkyl, cycloalkyl, -CF₃, cyano, -OCH₃, and -NO₂;
B is selected from the group consisting of 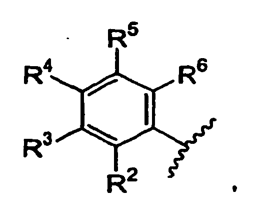 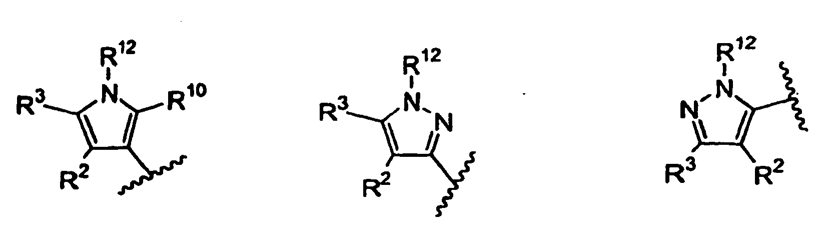 and 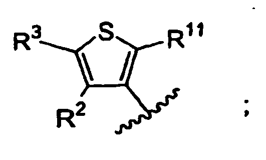 wherein
R² is OH;
R³ is -C(O) NR¹³R¹⁴;
R⁴ is H, -NO₂, cyano, -CH₃, halogen, or -CF₃;
R⁵ is H, -CF₃, -NO₂, halogen or cyano;
R⁶ is H, alkyl or -CF₃;
R¹⁰ and R¹¹ are the same or different and are independently selected from the group consisting of hydrogen, halogen, -CF₃, -NR¹³R¹⁴, -NR¹³C(O)NR¹³R¹⁴, -C(O)OR¹³, -SH, -SO₍ₜ₎NR¹³R¹⁴,-SO₂R¹³, -NHC(O)R¹³; -NHSO₂NR¹³R¹⁴, -NHSO₂R¹³, -C(O)NR¹³R¹⁴, -C(O)NR¹³OR¹⁴, -OC(O)R¹³, -COR¹³, -OR¹³, and cyano;
R¹³ and R¹⁴ are the same or different and are independently selected from methyl, ethyl and isopropyl; or
R¹³ and R¹⁴ when taken together with the nitrogen they are attached to in the groups -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴, -NHSO₂NR¹³R¹⁴ preferably form an unsubstituted or substituted 3 to 7 membered, saturated heterocyclic ring optionally containing one additional heteroatom selected from O, S or NR¹⁸ wherein R¹⁸ is selected from H, alkyl, aryl, heteroaryl, -C(O)R¹⁹, -SO₂R¹⁹ and -C(O)NR¹⁹R²⁰, wherein R¹⁹ and R²⁰ are the same or different and each is independently selected from alkyl, aryl and heteroaryl, wherein the substituents on the substituted cyclized R¹³ and R¹⁴ groups are the same or different and independently selected from 1 to 3 of alkyl, aryl, hydroxy, hydroxyalkyl, alkoxy, alkoxyalkyl, arylalkyl, fluoroalkyl, cycloalkyl, cycloalkylalkyl, heteroaryl, heteroarylalkyl, amino, -C(O)OR¹⁵, -C(O)NR¹⁵R¹⁶, -SOₜNR¹⁵R¹⁶, -C(O)R¹⁵, -SO₂R¹⁵, -NHC(O)NR¹⁵R¹⁶ and halogen, and wherein R¹⁵ and R¹⁶ are the same or different and are independently selected from the group consisting of H, alkyl, aryl, arylalkyl, cycloalkyl and heteroaryl;

Even more preferably; A is selected from the group consisting of: 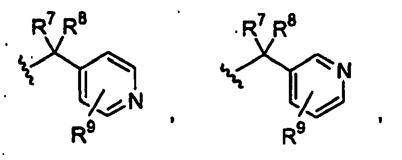 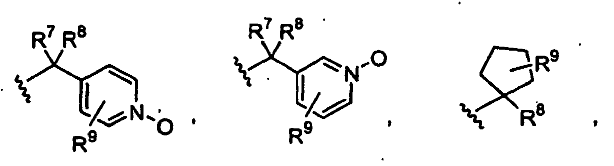 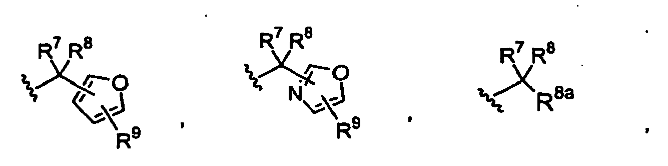 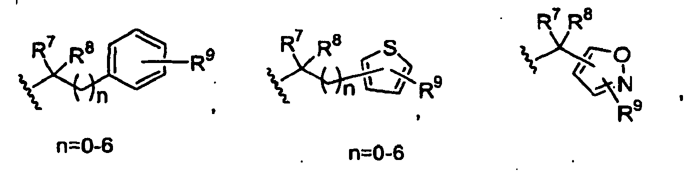 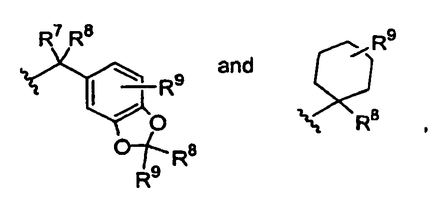 wherein,
R⁷ is H, fluoroalkyl, alkyl or cycloalkyl;
R⁸ is H, alkyl, -CF₂CH₃ or -CF₃;
R⁹ is H, F, Cl, Br, alkyl or -CF₃.

Still even more preferably, A is selected from the group consisting of 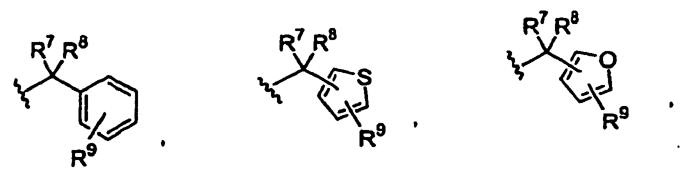 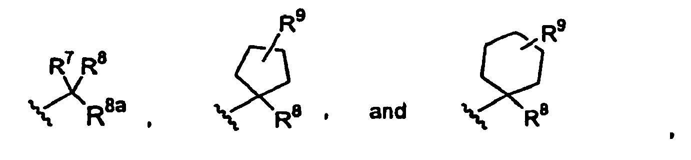 wherein,
R⁷ is H, -CF₃, -CF₂CH₃, methyl, ethyl, isopropyl, cyclopropyl or t-butyl;
R⁸ is H;
R⁹ is H, F, Cl, Br, alkyl or -CF₃, and
B is: 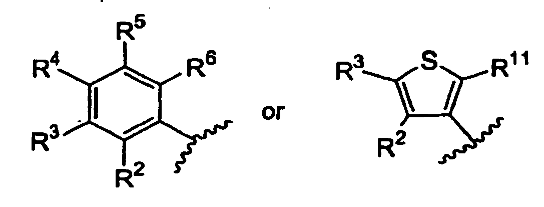 wherein:
R² is OH;
R³ is -C(O)NR¹³R¹⁴;
R⁴ is H, -NO₂, cyano, -CH₃ or -CF₃:
R⁵ is H, -CF₃, -NO₂, halogen or cyano; and
R⁶ is H, alkyl or -CF₃;
R¹¹ is H, halogen or alkyl; and
R¹³ and R¹⁴ are the same or different and are independently methyl, ethyl or isopropyl; or
R¹³ and R¹⁴ when taken together with the nitrogen they are attached to in the groups -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴, -NHSO₂NR¹³R¹⁴ preferably form an unsubstituted or substituted 3 to 7 membered, saturated heterocyclic ring optionally containing one additional heteroatom selected from O, S or NR¹⁸ wherein R¹⁸ is selected from H, alkyl, aryl, heteroaryl, -C(O)R¹⁹, -SO₂R¹⁹ and -C(O)NR¹⁹R²⁰, wherein R¹⁹ and R²⁰ are the same or different and each is independently selected from alkyl, aryl and heteroaryl, wherein the substituents on the substituted cyclized R¹³ and R¹⁴ groups are the same or different and independently selected from 1 to 3 of alkyl, aryl; hydroxy, hydroxyalkyl, alkoxy, alkoxyalkyl, arylalkyl, fluoroalkyl, cycloalkyl, cycloalkylalkyl, heteroaryl, heteroarylalkyl, amino, -C(O)OR¹⁵, -C(O)NR¹⁵R¹⁶, -SOₜNR¹⁵R¹⁶, -C(O)R¹⁵, -SO₂R¹⁵, -NHC(O)NR¹⁵R¹⁶ and halogen, and wherein R¹⁵ and R¹⁶ are the same or different and are independently selected from the group consisting of H, alkyl, aryl, arylalkyl, cycloalkyl and heteroaryl;

Yet even still more preferred,
A is selected from the group consisting of 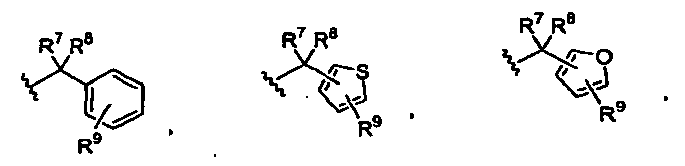 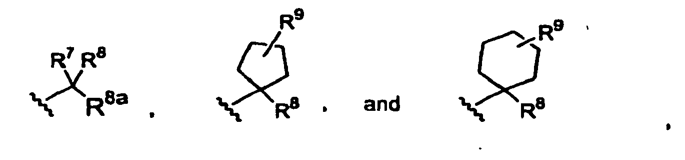 and B is: 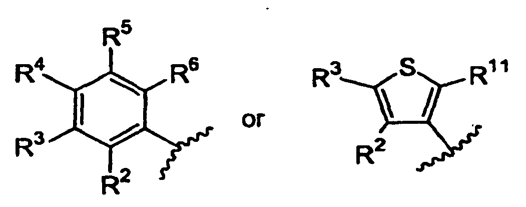 wherein:
R² is OH;
R³ is -C(O)NR¹³R¹⁴;
R⁴ is H, -NO₂, -CF₃, -CH₃ or cyano,
R⁵ is H, halogen, -NO₂, cyano or -CF₃;
R⁶ is H, -CF₃ or alkyl,
R⁷ is H, -CF₃, -CF₂CH₃, methyl, ethyl, isopropyl, cyclopropyl or t-butyl;
R⁸ is H;
R⁹ is H, F, Cl, Br, alkyl; cycloalkyl or -CF₃;
R¹¹ is H, halogen or alkyl; and
R¹³ and R¹⁴ are independently methyl or ethyl.

Most preferably,
A is selected from the group consisting of 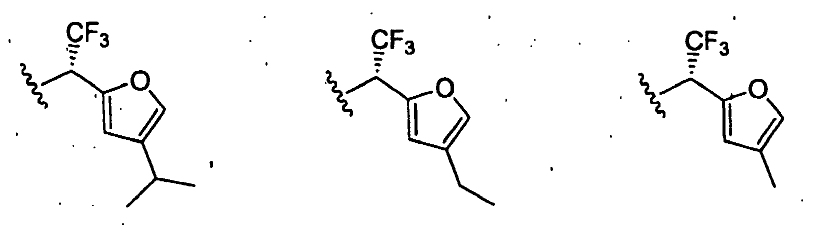 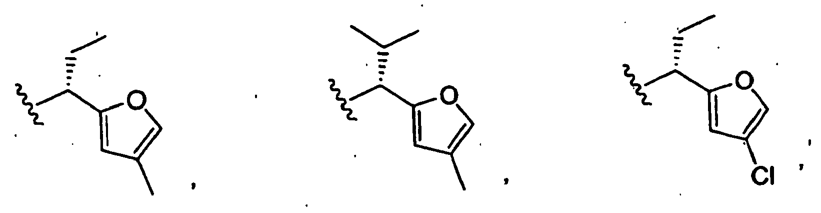 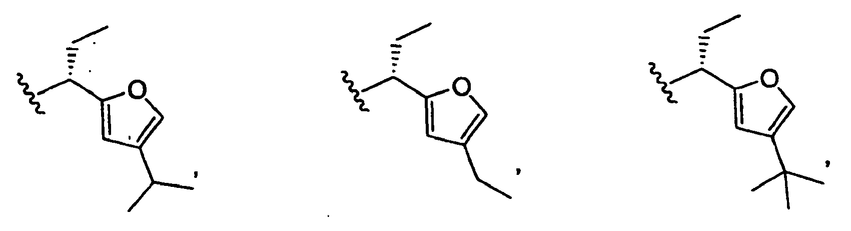 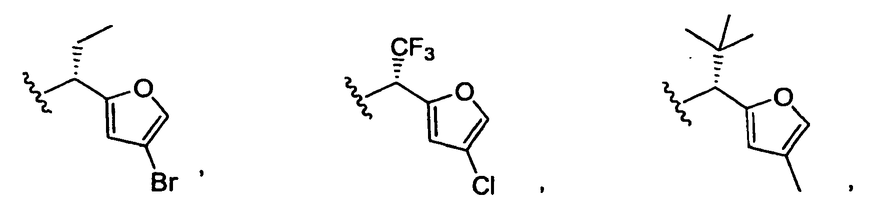 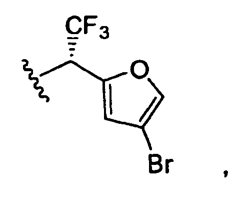 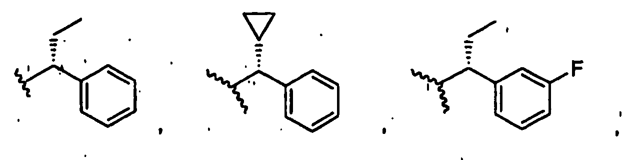 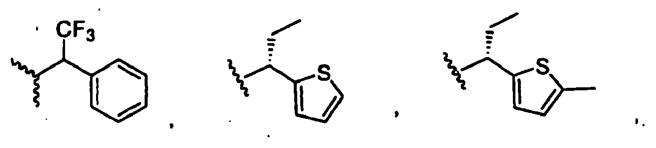 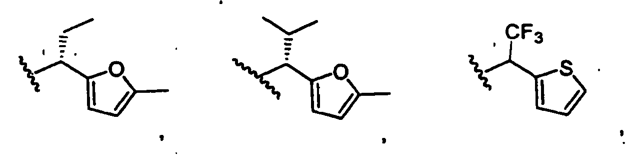 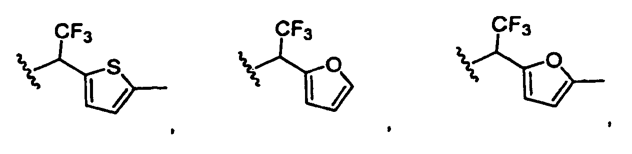 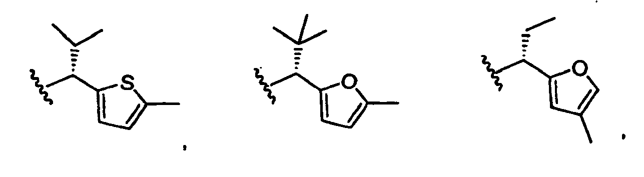 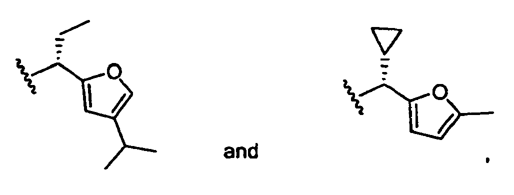 and B is 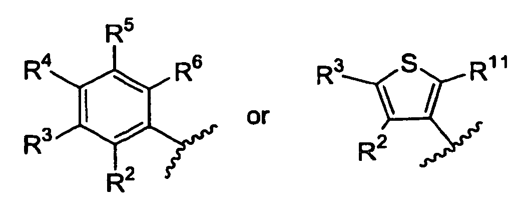 wherein,
R² is -OH;
R³ is -CONR¹³R¹⁴ ;
R⁴ is H, -CH₃ or -CF₃;
R⁵ is H or cyano;
R⁶ is H, -CH₃ or -CF₃;
R¹¹ is H, and
R¹³ and R¹⁴ are methyl.

Representative embodiments of this invention are described below. The embodiments have been numbered for purposes of reference thereto.

Embodiment No. 1 is directed to the methods of treatment described above using formula I, except the compounds used are those of formula IA: 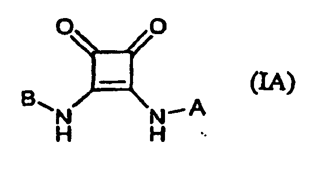 and their pharmaceutically acceptable salts (e.g., sodium or calcium salt) and solvates thereof, wherein:
A is selected from the group consisting of:
   (1) 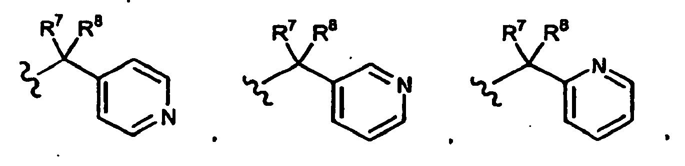 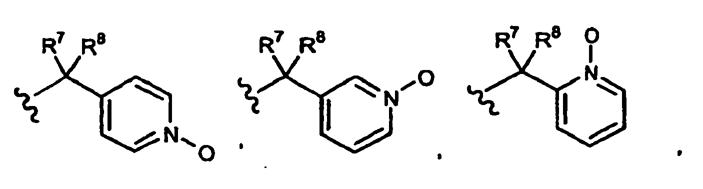 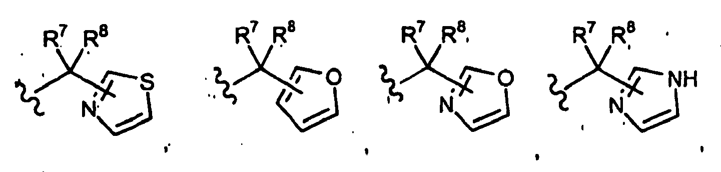 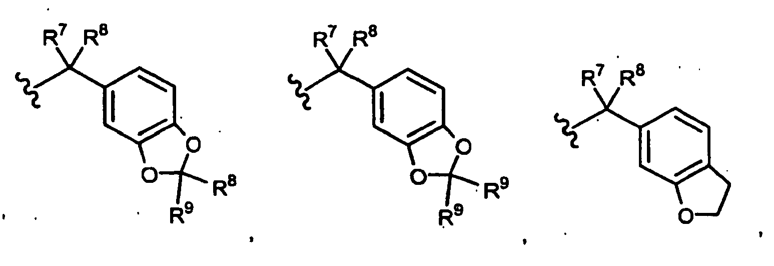 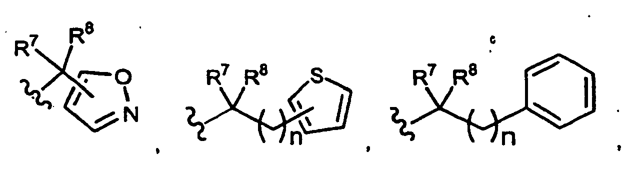 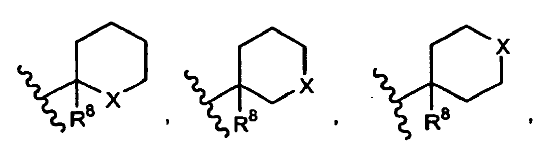 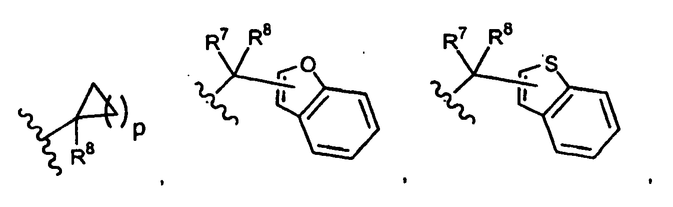 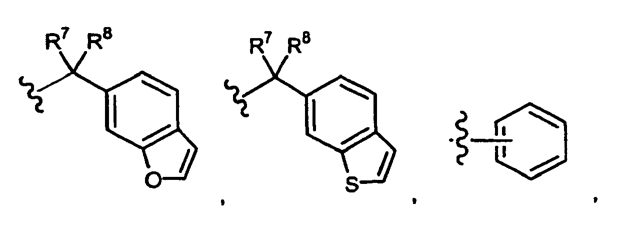 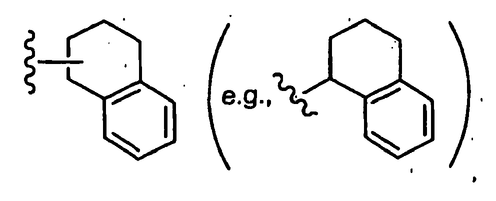 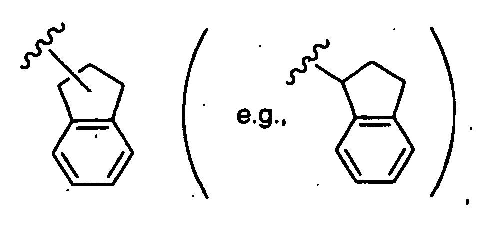 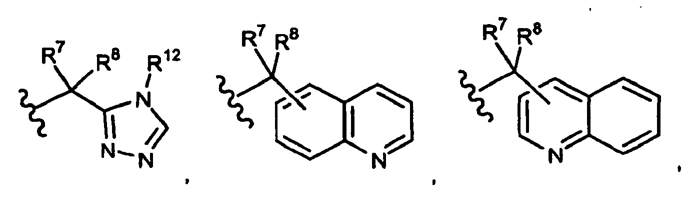 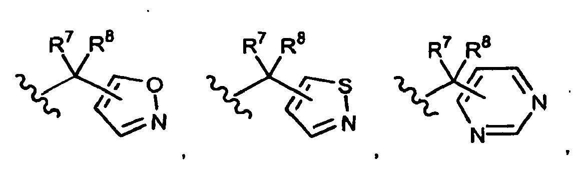 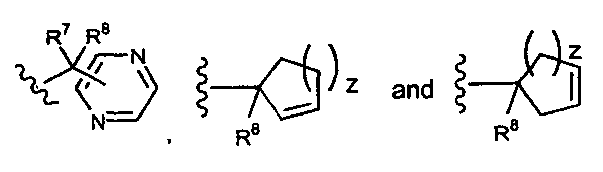
   (2) 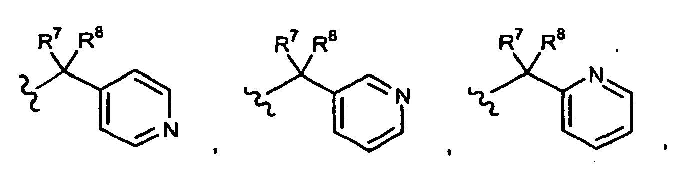 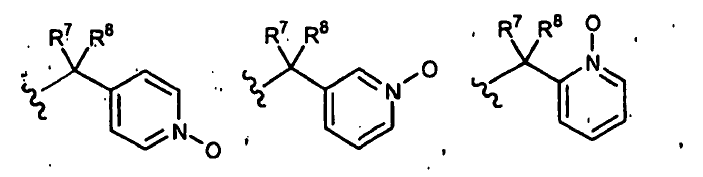 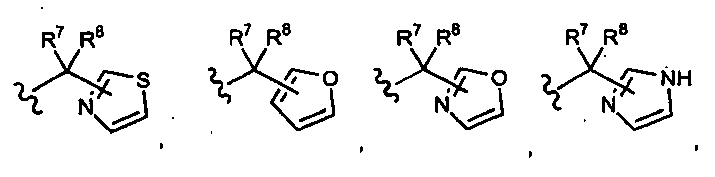 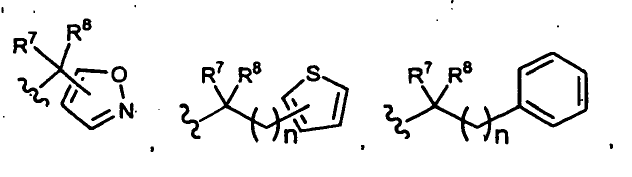 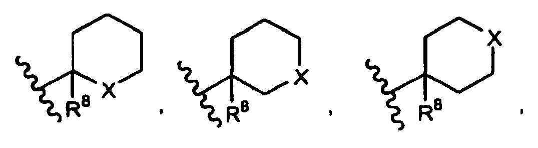 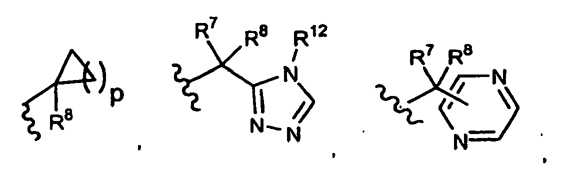 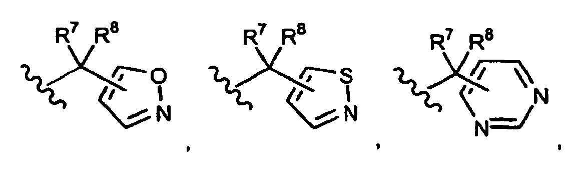 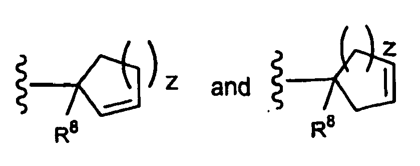 wherein the above rings of said A groups are substituted with 1 to 6 substituents each independently selected from the group consisting of: R⁹ groups;
   (3) 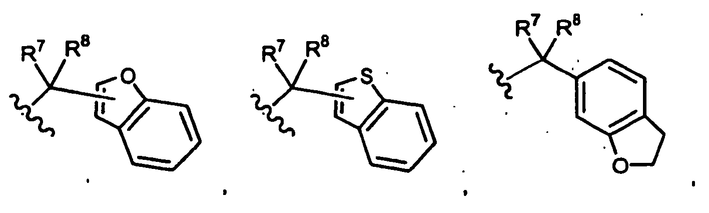 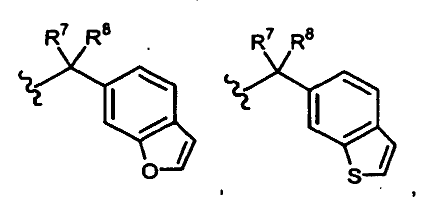 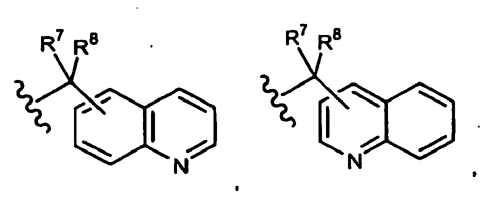 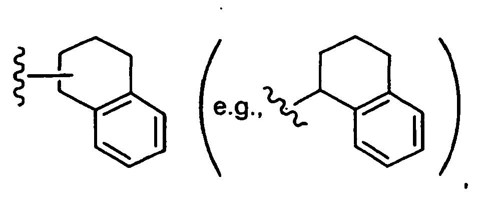 and 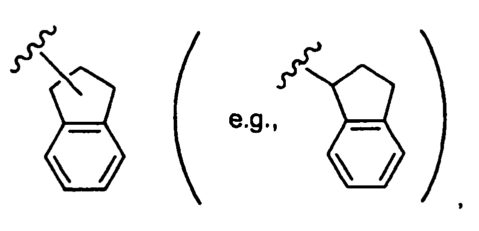 wherein one or both of the above rings of said A groups are substituted with 1 to 6 substituents each independently selected from the group consisting of: R⁹ groups;
   (4) 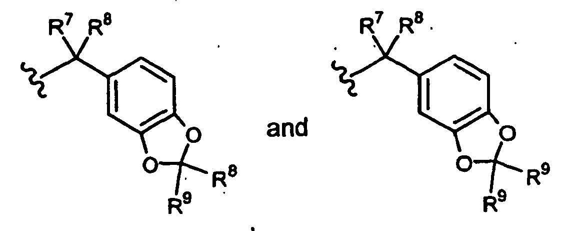 wherein the above phenyl rings of said A groups are substituted with 1 to 3 substituents each independently selected from the group consisting of: R⁹ groups; and
   (5) 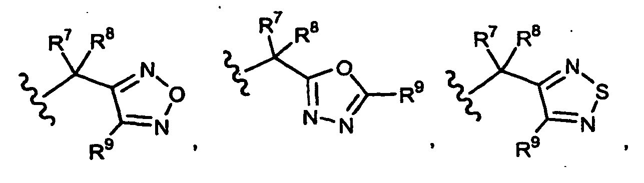 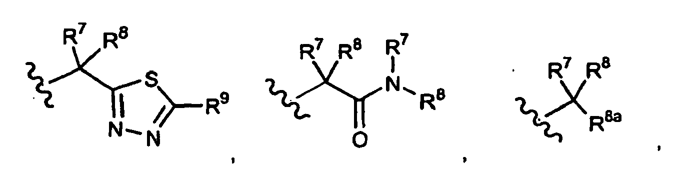 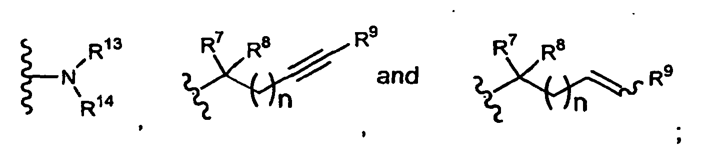
B is selected from the group consisting of 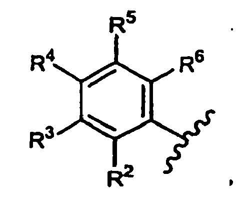 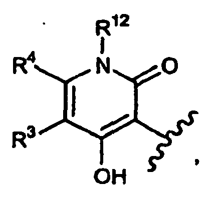 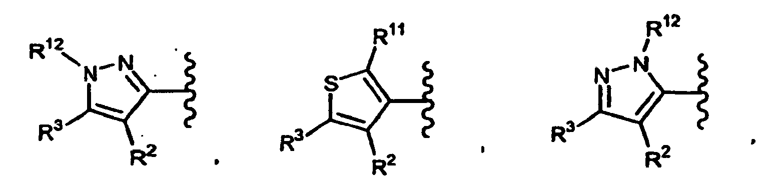 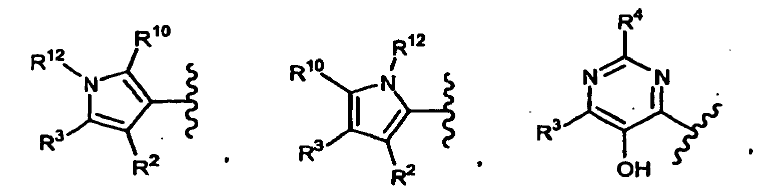 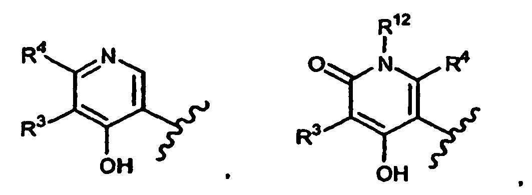 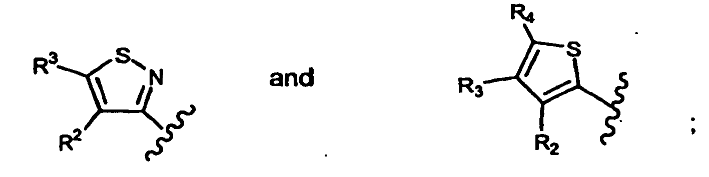 n is 0 to 6; p is 1 to 5; X is O, NH, or S; Z is 1 to 3;
   R² is OH; selected from the group consisting of: -C(O)NHR¹⁷, -C(O)NR¹³R¹⁴ and -C(O)NR¹³OR¹⁴;
   R⁴ is selected from the group consisting of: hydrogen, cyano, halogen, alkyl, alkoxy, -OH, -CF₃, -OCF₃, -NO₂, -C(O)R¹³; -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, unsubstituted or substituted aryl, unsubstituted or substituted heteroaryl, 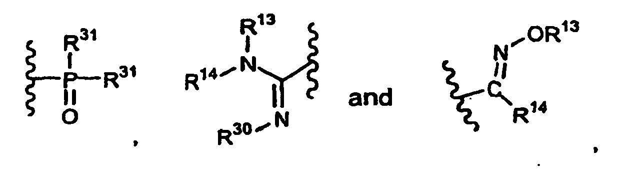 wherein there are 1 to 6 substituents on said substituted aryl group and each substitutent is independently selected from the group consisting of: R⁹ groups; and wherein there are 1 to 6 substituents on said substituted heteroaryl group and each substitutent is independently selected from the group consisting of: R⁹ groups;
   each R⁵ and R⁶ are the same or different and are independently selected from the group consisting of hydrogen, halogen, alkyl, alkoxy, -CF₃, -OCF₃, -NO₂, -C(O)R¹³, -C(O)OR¹³, -C(O)NR¹³R¹⁴, -SO₍ₜ₎NR¹³R¹⁴, -C(O)NR¹³OR¹⁴, cyano, unsubstituted or substituted aryl, and unsubstituted or substituted heteroaryl group; wherein there are 1 to 6 substituents on said substituted aryl group and each substitutent is independently selected from the group consisting of: R⁹ groups; and wherein there are 1 to 6 substituents on said substituted heteroaryl group and each substitutent is independently selected from the group consisting of: R⁹ groups;
   each R⁷ and R⁸ is independently selected from the group consisting of: H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, -CO₂R¹³, -CONR¹³R¹⁴, alkynyl, alkenyl, and cycloalkenyl; and wherein there are one or more (e.g., 1 to 6) substituents on said substituted R⁷ and R⁸ groups, wherein each substitutent is independently selected from the group consisting of:
   a) halogen,
   b) -CF₃,
   c) -COR¹³,
   d) -OR¹³,
   e) -NR¹³R¹⁴,
   f) -NO₂,
   g) -CN,
   h) -SO₂OR¹³,
   i) -Si(alkyl)₃, wherein each alkyl is independently selected,
   j) -Si(aryl)_{3,} wherein each alkyl is independently selected,
   k) -(R¹³)₂R¹⁴Si, wherein each R¹³ is independently selected,
   l) -CO₂R¹³,
   m) -C(O)NR¹³R¹⁴,
   n) -SO₂NR¹³R¹⁴,
   o) -SO₂R¹³,
   p) -OC(O)R¹³,
   q) -OC(O)NR¹³R¹⁴,
   r) -NR¹³C(O)R¹⁴, and
   s) -NR¹³CO₂R¹⁴;
   (fluoroalkyl is one non-limiting example of an alkyl group that is subsituted with halogen);
   R^{8a} is selected from the group consisting of: hydrogen, alkyl, cycloalkyl and cycloalkylalkyl;
   each R⁹ is independently selected from the group consisting of:
   a) -R¹³,
   b) halogen,
   c) -CF₃,
   d) -COR¹³,
   e) -OR¹³,
   f) -NR¹³R¹⁴,
   g) -NO₂,
   h) -CN,
   i) -SO₂R¹³,
   j) -SO₂NR¹³R¹⁴,
   k) -NR¹³COR¹⁴,
   l) -CONR¹³R¹⁴,
   m) -NR¹³CO₂R¹⁴,
   n) -CO₂R¹³,
   o) 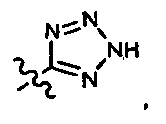
   p) alkyl substituted with one or more (e.g., one) -OH groups (e.g., -(CH₂)_{q}OH, wherein q is 1-6, usually 1 to 2, and preferably 1),
   q) alkyl substituted with one or more (e.g., one) -NR¹³R¹⁴ group (e.g., -(CH₂)qNR¹³R¹⁴, wherein q is 1-6, usually 1 to 2, and preferably 1), and
   r) -N(R¹³)SO₂R¹⁴ (e.g., R¹³ is H and R¹⁴ is alkyl, such as methyl);
   each R¹⁰ and R¹¹ is independently selected from the group consisting of hydrogen, alkyl (e.g., C₁ to C₆, such as methyl), halogen, -CF₃, -OCF₃, -NR¹³R¹⁴, -NR ¹³C(O)NR ¹³R¹⁴, -OH, -C(O)OR ¹³, -SH, -SO₍ₜ₎NR¹³R¹⁴, -SO₂R¹³, -NHC(O)R ¹³, -NHSO₂NR¹³R¹⁴, -NHSO₂R¹³, -C(O)NR ¹³R¹⁴, -C(O)NR¹³OR¹⁴, -OC(O)R¹³ and cyano;
   R¹² is selected from the group consisting of: hydrogen, -C(O)OR¹³, unsubstituted or substituted aryl, unsubstituted or substituted heteroaryl, unsubstituted or substituted arylalkyl, unsubstituted or substituted cycloalkyl, unsubstituted or substituted alkyl, unsubstituted or substituted cycloalkylalkyl, and unsubstituted or substituted heteroarylalkyl group; wherein there are 1 to 6 substituents on the substituted R¹² groups and each substituent is independently selected from the group consisting of: R⁹ groups;
   each R¹³ and R¹⁴ is independently selected from the group consisting of: H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, unsubstituted or substituted heterocyclic, unsubstituted or substituted fluoroalkyl, and unsubstituted or substituted heterocycloalkylalkyl (wherein "heterocyloalkyl" means heterocyclic); wherein there are 1 to 6 substituents on said substituted R¹³ and R¹⁴ groups and each substituent is independently selected from the group consisting of: alkyl, -CF₃, -OH, alkoxy, aryl, arylalkyl, fluroalkyl, cycloalkyl, cycloalkylalkyl, heteroaryl, heteroarylalkyl, -N(R⁴⁰)₂, -C(O)OR¹⁵, -C(O)NR¹⁵R¹⁶, -S(O)ₜNR¹⁵R¹⁶, -C(O)R¹⁵, -SO₂R¹⁵ provided that R¹⁵ is not H, halogen, and -NHC(O)NR¹⁵R¹⁶; or
   R¹³ and R¹⁴ taken together with the nitrogen they are attached to in the groups -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴, -NHSO₂NR¹³R¹⁴ form an unsubstituted or substituted saturated heterocyclic ring (preferably a 3 to 7 membered heterocyclic ring), said ring optionally containing one additional heteroatom selected from the group consisting of: O, S and NR¹⁸; wherein there are 1 to 3 substituents on the substituted cyclized R¹³ and R¹⁴ groups (i.e., there is 1 to 3 substituents on the ring formed when the R¹³ and R¹⁴ groups are taken together with the nitrogen to which they are bound) and each substituent is independently selected from the group consisting of: alkyl, aryl, hydroxy, hydroxyalkyl, alkoxy, alkoxyalkyl, arylalkyl, fluoroalkyl, cycloalkyl, cycloalkylalkyl, heteroaryl, heteroarylalkyl, amino, -C(O)OR¹⁵, -C(O)NR¹⁵R¹⁶, -SOₜNR¹⁵R¹⁶, -C(O)R¹⁵, -SO₂R¹⁵ provided that R¹⁵ is not H, -NHC(O)NR¹⁵R¹⁶, -NHC(O)OR¹⁵, halogen, and a heterocylcoalkenyl group (i.e., a heterocyclic group that has at least one, and preferably one, double bond in a ring, e.g., 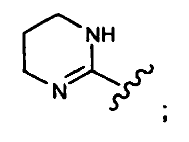 each R¹⁵ and R¹⁶ is independently selected from the group consisting of: H, alkyl, aryl, arylalkyl, cycloalkyl and heteroaryl;
   R¹⁷ is selected from the group consisting of: -SO₂alkyl, -SO₂aryl, -SO₂cycloalkyl, and -SO₂heteroaryl;
   R¹⁸ is selected from the group consisting of: H, alkyl, aryl, heteroaryl, -C(O)R¹⁹, -SO₂R¹⁹ and -C(O)NR¹⁹R²⁰;
   each R¹⁹ and R²⁰ is independently selected from the group consisting of: alkyl, aryl and heteroaryl;
   R³⁰ is selected from the group consisting of: alkyl, cycloalkyl, -CN, -NO₂, or -SO₂R¹⁵ provided that R¹⁵ is not H;
   each R³¹ is independently selected from the group consisting of: unsubstituted or substituted alkyl, unsubstituted or substituted aryl, unsubstituted or substituted heteroaryl and unsubstituted or substituted cycloalkyl; wherein there are 1 to 6 substituents on said substituted R³¹ groups and each substituent is independently selected from the group consisting of: R⁹ groups;
   each R⁴⁰ is independently selected from the group consisting of: H, alkyl and cycloalkyl; and
   t is 0, 1 or 2.

In Embodiment No. 1, at least one (e.g., 1 to 3, and usually one) compound of formula IA are used.

Embodiment No. 2 is directed to the use of at least one (e.g., 1 to 3, and usually 1) compound of formula IA for the manufacture of a medicament for the treatment of the diseases described above in the methods of treatment using formula I.

Embodiment No. 3 is directed to compounds of formula IA wherein B is selected from the group consisting of:
(1) 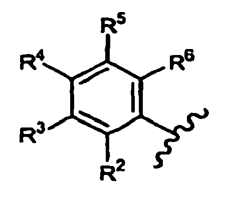 wherein R³ -C(O)NR¹³R¹⁴;
   and all, other substituents are as defined for formula I or IA;
(2) 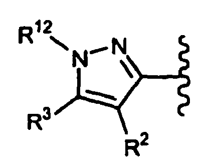 wherein all substituents are as defined for formula I or IA;
(3) 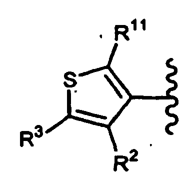 wherein all substituents are as defined for formula I or IA;
(4) 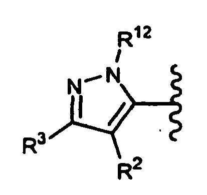 wherein all substituents are as defined for formula I or IA;
(5) 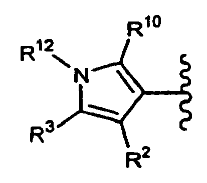 wherein all substituents are as defined for formula I or IA;
(6) 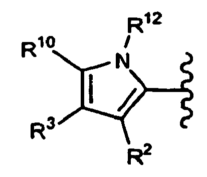 wherein all substituents are as defined for formula I or IA; and
(7) 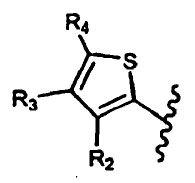 wherein all substituents are as defined for formula I or IA.

Embodiment No. 4 is directed to compounds of formula IA wherein B is: 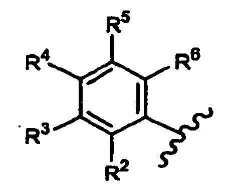 wherein R³ is -C(O)NR¹³R¹⁴.
and all other substituents are as defined in formula I or IA.

Embodiment No. 5 is directed to compounds of formula IA wherein B is: 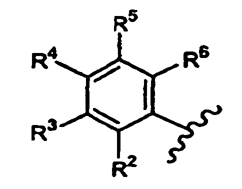 R³ is -C(O)NR¹³R¹⁴, and all other substituents are as defined in formula I or IA.

Embodiment No. 6 is directed to compounds of formula IA wherein B is 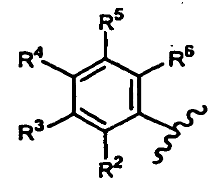 R³ is -C(O)NR¹³R¹⁴, R¹³ and R¹⁴ are each the same or different alkyl group, and all other substituents are as defined in formula I or IA.

Embodiment No. 7 is directed to compounds of formula IA wherein B is 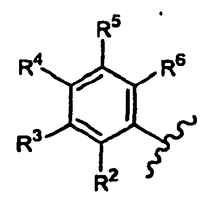 R² is -OH, R³ is -C(O)NR¹³R¹⁴, R¹³ and R¹⁴ are each the same or different alkyl group, and all other substituents are as defined in formula I or IA.

Embodiment No. 10 is directed to compounds of formula IA wherein B is: 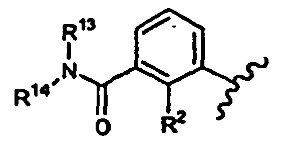 R², R¹³, and R¹⁴ are as defined for compounds of formula I or IA, and all other substituents are as defined in formula I or IA.

Embodiment No. 11 is directed to compounds of formula IA wherein B is: 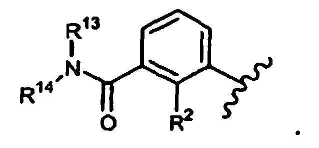 R² is -OH, R¹³ and R¹⁴ are as defined for compounds of formula I or IA, and all other substituents are as defined in formula I or IA.

Embodiment No. 12 is directed to compounds of formula IA wherein B is: 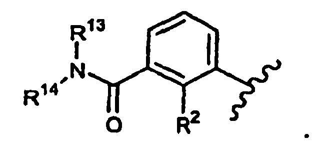 R² is as defined for compounds of formula I or IA, R¹³ and R¹⁴ are the same or different alkyl group, and all other substituents areas defined for compounds of formula I or IA.

Embodiment No. 13 is directed to compounds of formula IA wherein B is: 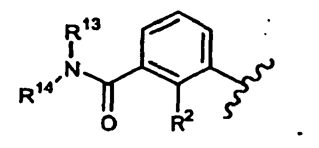 R² is -OH, R¹³ and R¹⁴ are the same or different alkyl group, and all other substituents areas defined for compounds of formula I or IA.

Embodiment No. 16 is directed to compounds of formula IA wherein B is as described in Embodiments Nos. 6, 7, 10 and 11, except that R¹³ and R¹⁴ are each methyl, and all other substituents are as defined in formula I or IA.

Embodiment No. 17 is directed to compounds of formula IA wherein B is selected from the group consisting of: 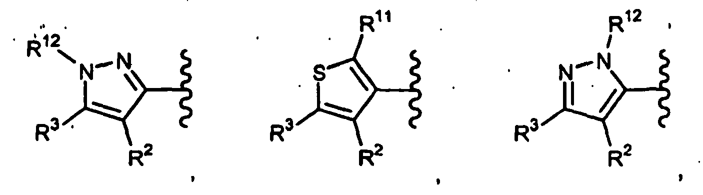 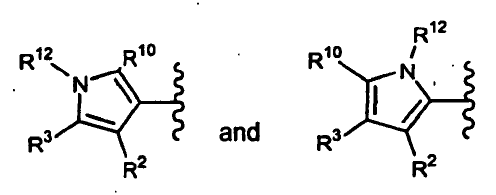 wherein all substituents are as defined for formula I or IA.

Embodiment No. 18 of this invention is directed to compounds of formula IA wherein B is: 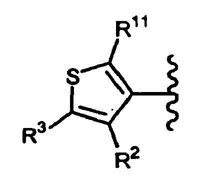 wherein all substituents are as defined for formula I or IA.

Embodiment No. 19 is directed to compounds of formula IA wherein B is: 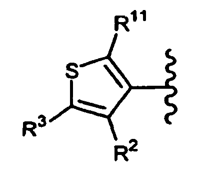 R¹¹ is H, and all other substituents are as defined in formula I or IA.

Embodiment No. 20 is directed to compounds of formula IA wherein B is: 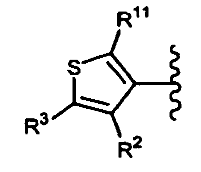 R² is -OH, and all other substituents are as defined in formula I or IA.

Embodiment No. 21 is directed to compounds of formula IA wherein B is: 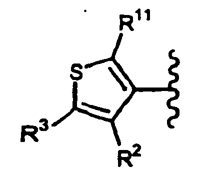 R³ is -C(O)NR¹³R¹⁴, and all other substituents are as defined in formula I or IA.

Embodiment No. 23 is directed to compounds of formula IA wherein B is: 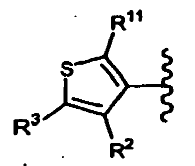 R² is -OH, R³ is -C(O)NR¹³R¹⁴, and all other substituents are as defined in formula I or IA.

Embodiment No. 25 is directed to compounds of formula IA wherein B is: 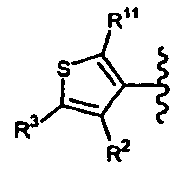 R² is -OH, R³ is -C(O)NR¹³R¹⁴, R¹¹ is H, and all other substituents are as defined in formula I or IA.

Embodiment No. 27 is directed to compounds of formula IA wherein B is: 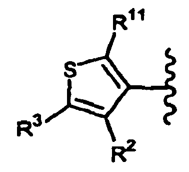 R² is-OH, R³ is -C(O)NR¹³R¹⁴, R¹¹ is H, and R¹³ and R¹⁴ are independently selected from the group consisting of: alkyl, unsubstituted heteroaryl and substituted heteroaryl, and all other substituents are as defined in formula I or IA. In general, one of R¹³ or R¹⁴ is alkyl (e.g., methyl). An example of a substituted heteroaryl group is

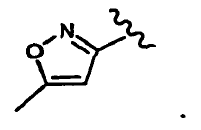

Embodiment No. 29 is directed to compounds of formula IA wherein B is: 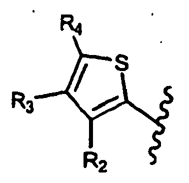 and all substituents are as defined in formula I or IA.

Embodiment No. 30 is directed to compounds of formula IA wherein B is as described in any one of the Embodiment Nos. 3 to 29, and A is as defined in all of the preferred descriptions above for A in formula I, or A is as described for formula IA.

Embodiment No. 31 is directed to compounds of formula IA wherein B is as described in any one of the Embodiment Nos. 3 to 29, and A is: 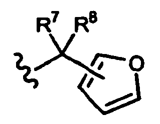 wherein the furan ring is unsubstituted or substitued as described in the definition of A for formula I or IA, and all other substitutents are as defined for formula IA.

Embodiment No. 32 is directed to compounds of formula IA wherein B is described in any one of the Embodiment Nos. 3 to 29, and A is 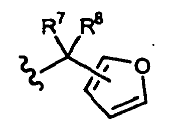 wherein the furan ring is substituted and all other substituents are as defined for formula I or IA.

Embodiment No. 33 is directed to compounds of formula IA wherein B is as described in any one of the Embodiment Nos. 3 to 29,and A is 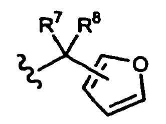 wherein the furan ring is substituted with at least one (e.g., 1 to 3, or 1 to 2) alkyl group and all other substituents are as defined for formula I or IA.

Embodiment No. 34 is directed to compounds of formula IA wherein B is as described in any one of the Embodiment Nos. 3 to 29, A is 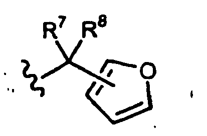 wherein the furan ring is substituted with one alkyl group and all other substituents are as defined for formula I or IA.

Embodiment No. 35 is directed to compounds of formula IA wherein B is as described in any one of the Embodiment Nos. 3 to 29, and A is 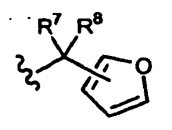 wherein the furan ring is substituted with one C, to C₃ alkyl group (e.g., methyl or isopropyl), and all other substituents are as defined for formula I or IA.

Embodiment No. 36 is directed to compounds of formula IA wherein B is as described in any one of the Embodiment Nos. 3 to 29, and A is 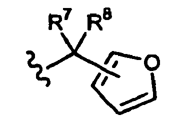 as defined in any one of the Embodiment Nos.31 to 35, except that R⁷ and R⁸ are the same or different and each is selected from the group consisting of: H and alkyl.

Embodiment No. 37 is directed to compounds of formula IA wherein B is as described in any one of the Embodiment Nos. 3 to 29, and A is 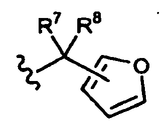 as defined in any one of the Embodiment Nos. 31 to 35, except that R⁷ is H, and R⁸ is alkyl (e.g., ethyl or t-butyl).

Embodiment No. 38 is directed to any one of the Embodiment Nos. 3 to 37 wherein the compound of formula IA is a pharmaceutically acceptable salt.

Embodiment No. 39 is directed to any one of the Embodiment Nos. 3 to 37 wherein the compound of formula IA is a sodium salt.

Embodiment No. 40 is directed to any one of the Embodiment Nos. 3 to 37 wherein the compound of formula IA is a calcium salt.

Embodiment No. 41 is directed to a pharmaceutically acceptable salt of any one of the representative compounds of this invention.

Embodiment No. 42 is directed to a sodium salt of any one of the representative compounds of this invention.

Embodiment No. 43 is directed to a calcium salt of any one of the representative compounds of this invention.

Embodiment No. 44 is directed to a pharmaceutical composition comprising at least one (e.g., 1 to 3, usually 1) compound of formula IA as described in any one of the Embodiment Nos. 3 to 43 in combination with a pharmaceutically acceptable carrier.

Embodiment No. 45 is directed to a method of treating any one of the diseases described above comprising administering to a patient in need of such treatment an effective amount (e.g., a therapeutically effective amount) of a compound of formula IA as described in any one of the Embodiment Nos. 3 to 43. The diseases referred to in this embodiment are those described in the methods of treatment using compounds of formula I.

Embodiment No. 46 is directed to the use of a compound of formula IA as described in any one of the Embodiment Nos. 3 to 43 for the manufacture of a medicament for the treatment any one of the diseases described above. The diseases referred in this embodiment are those described in the methods of treatment using compounds of formula I.

Representative compounds of the invention include but are not limited to:

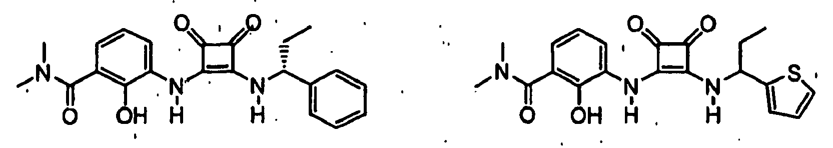

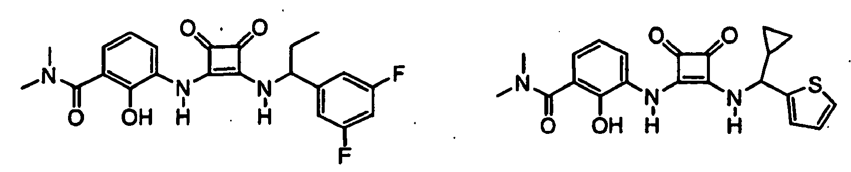

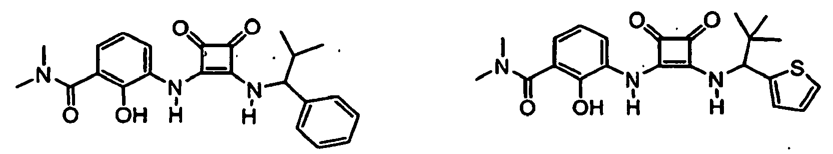

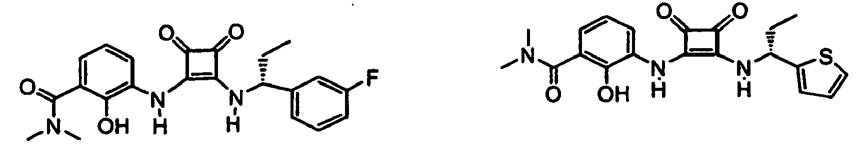

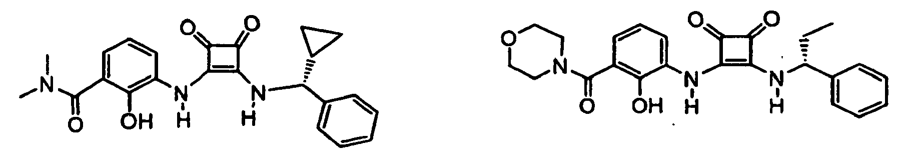

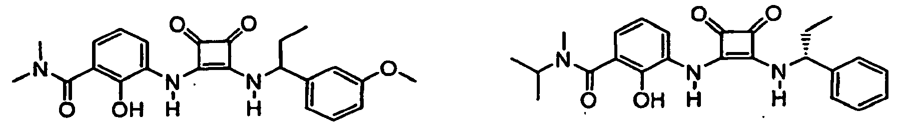

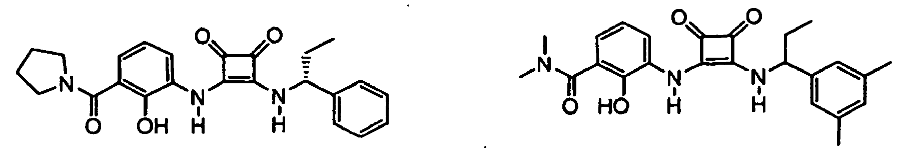

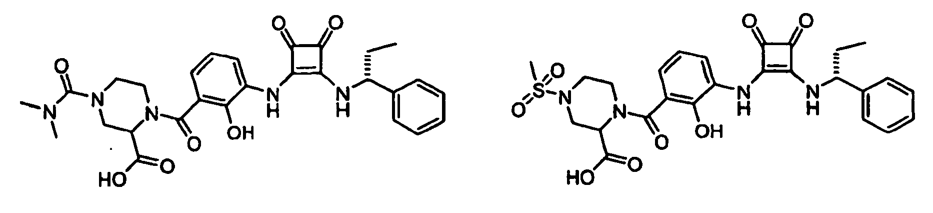

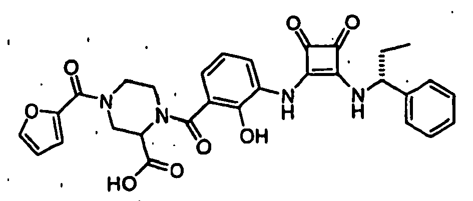

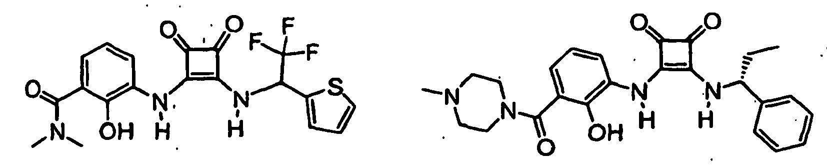

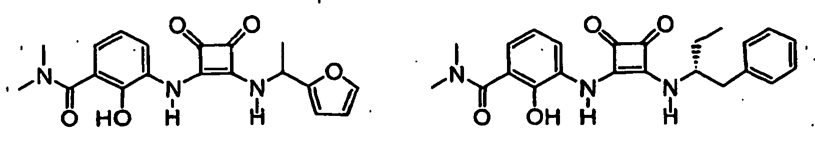

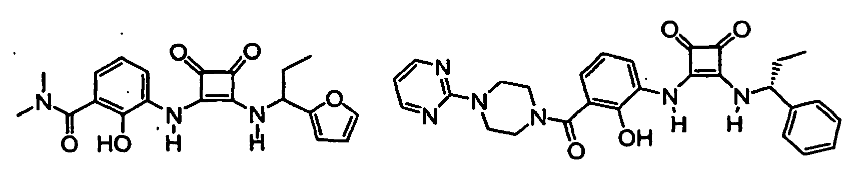

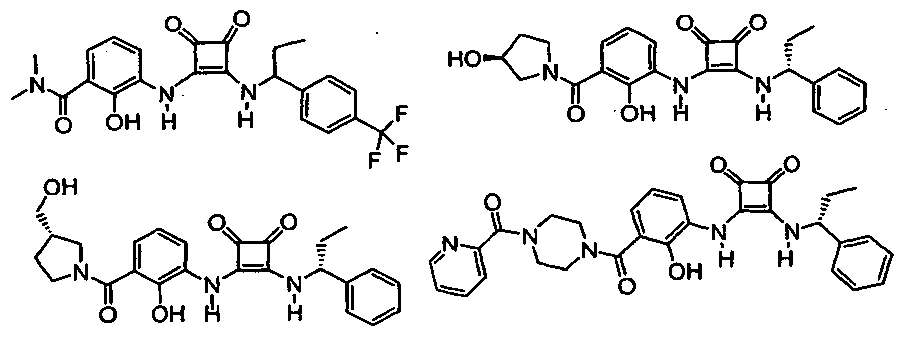

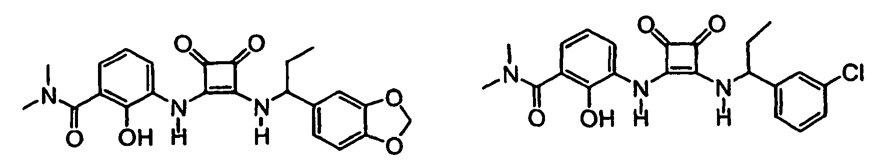

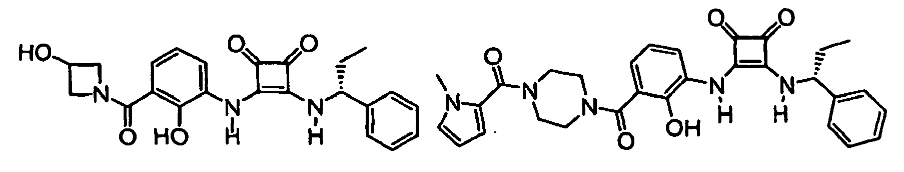

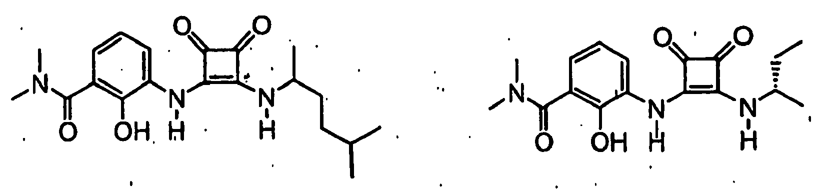

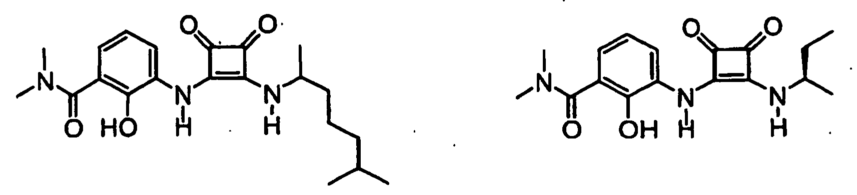

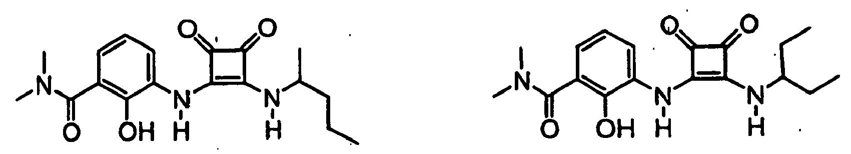

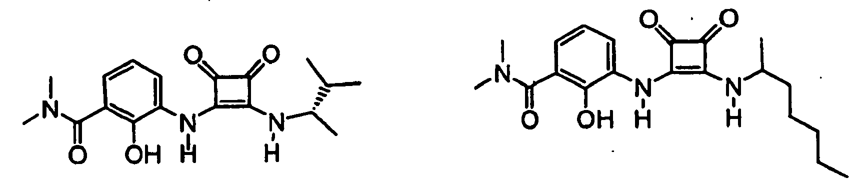

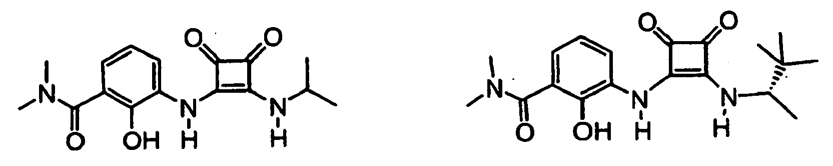

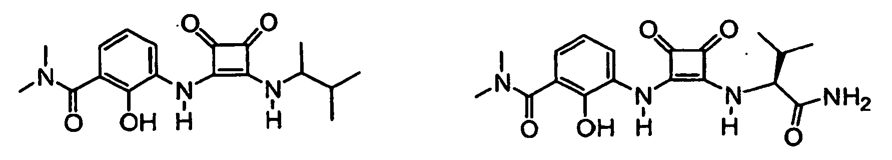

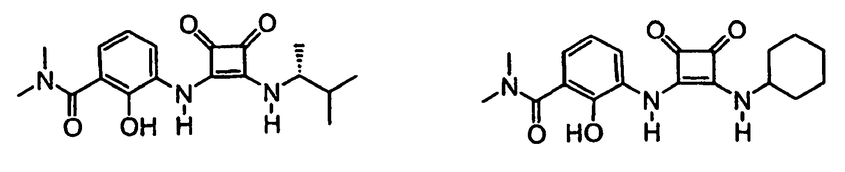

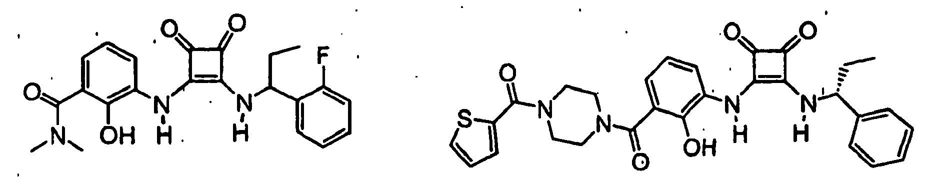

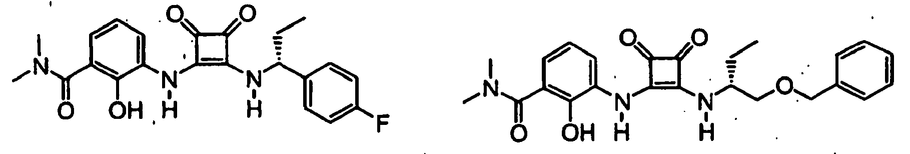

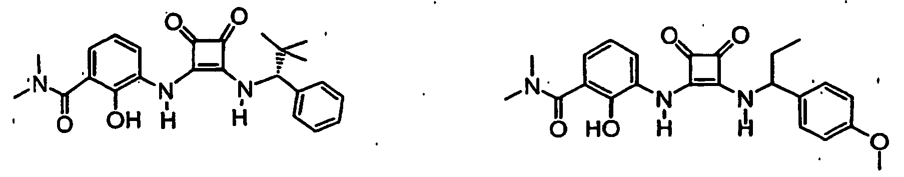

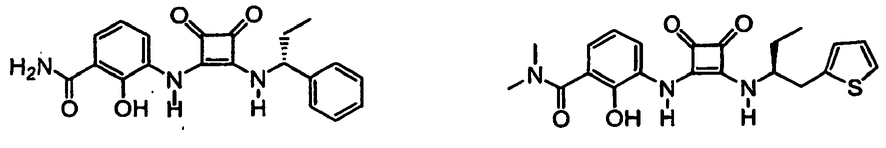

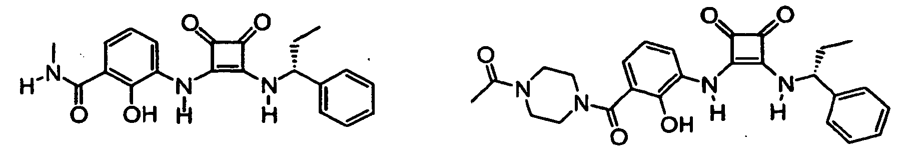

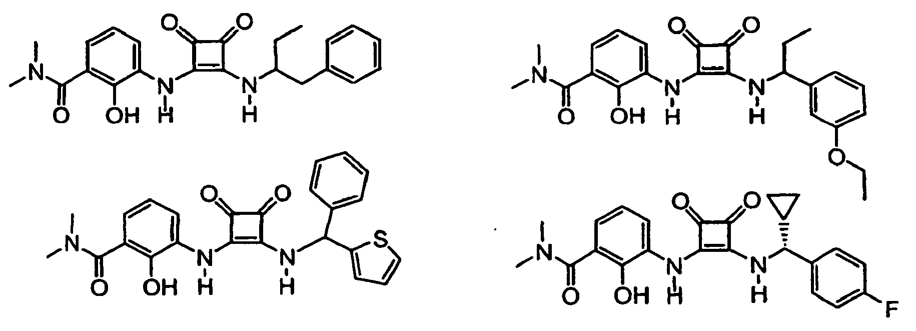

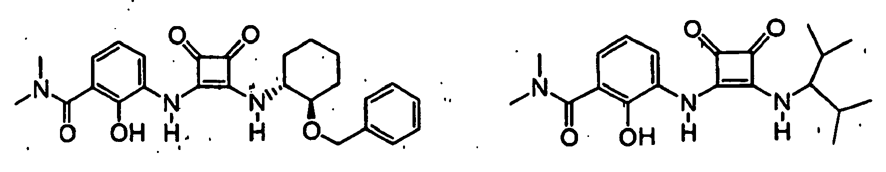

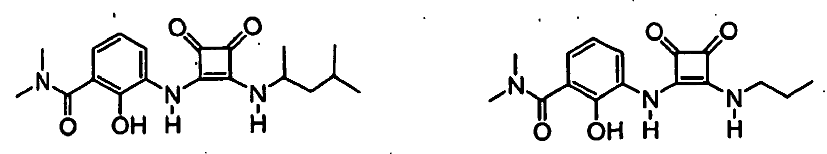

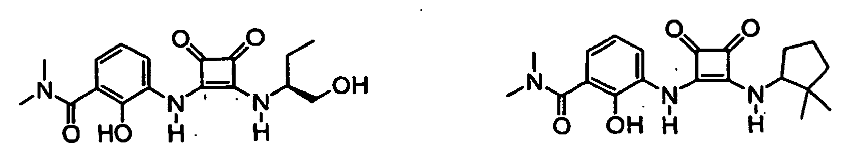

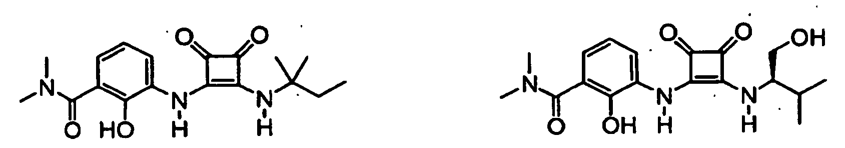

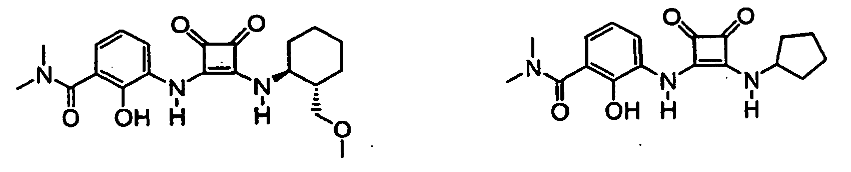

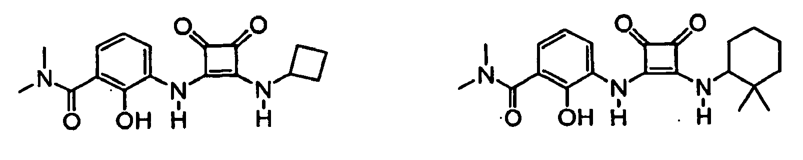

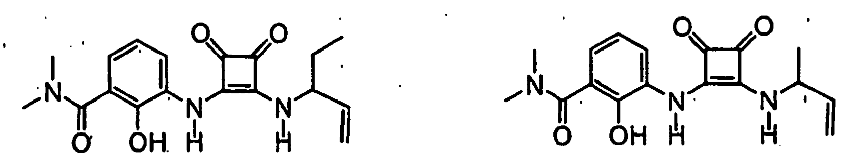

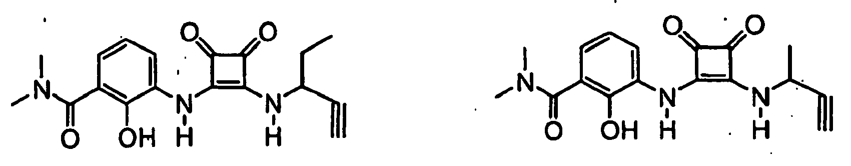

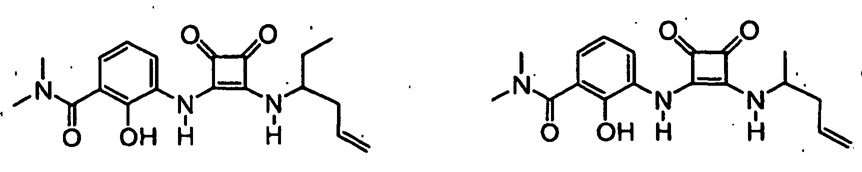

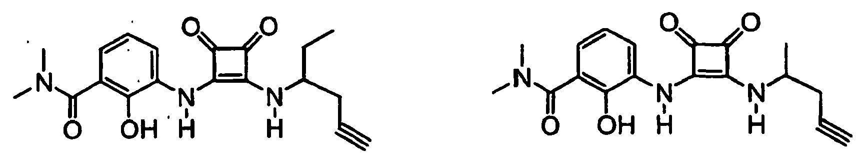

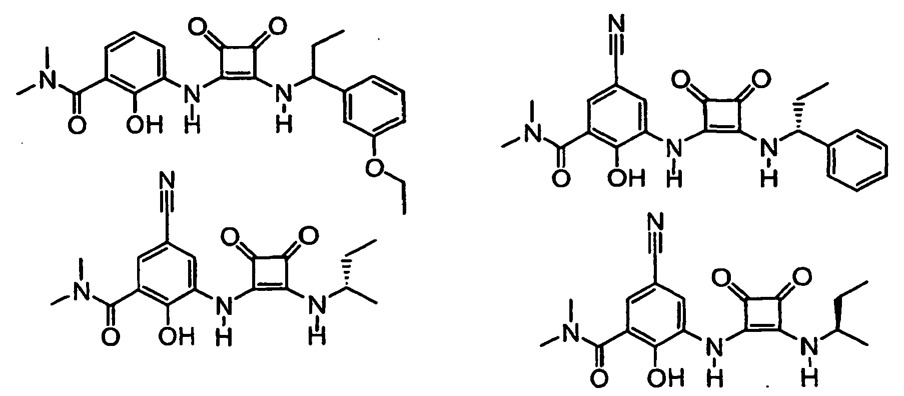

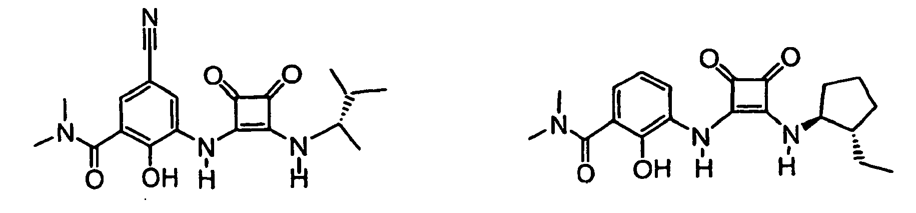

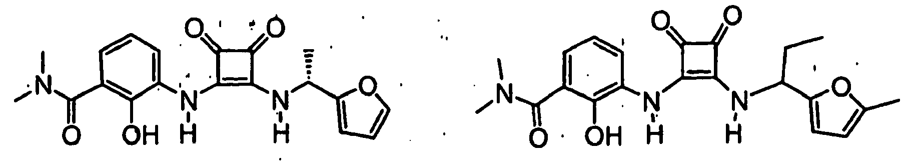

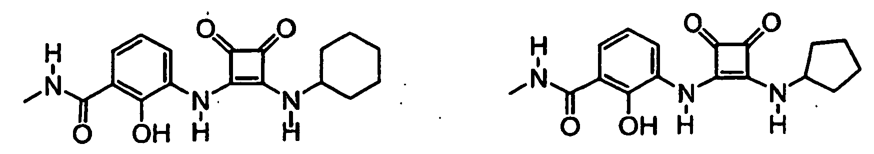

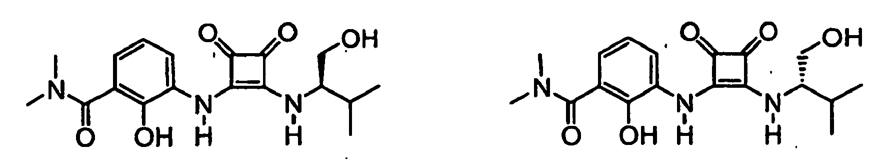

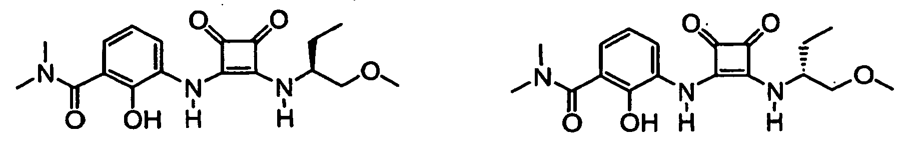

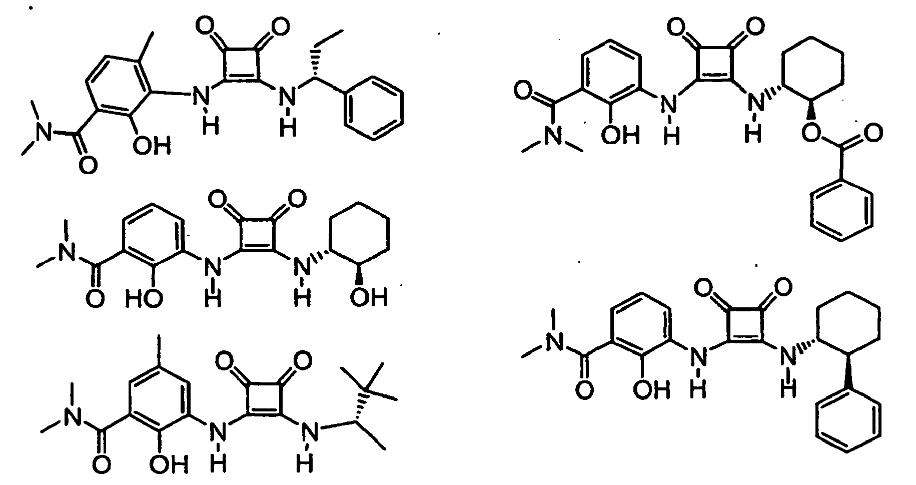

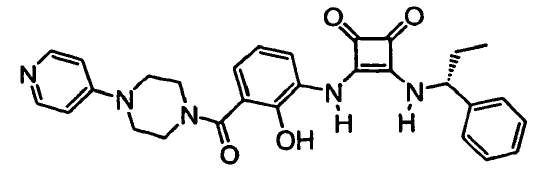

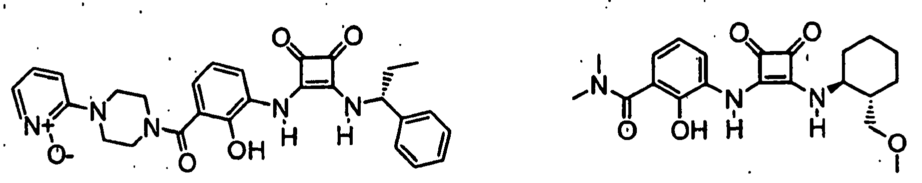

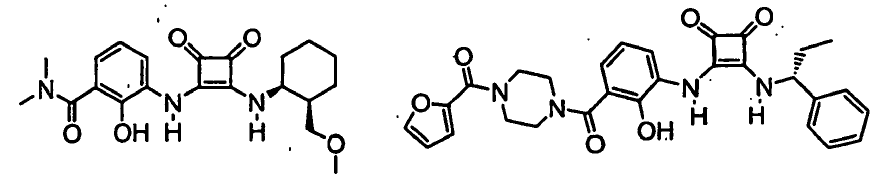

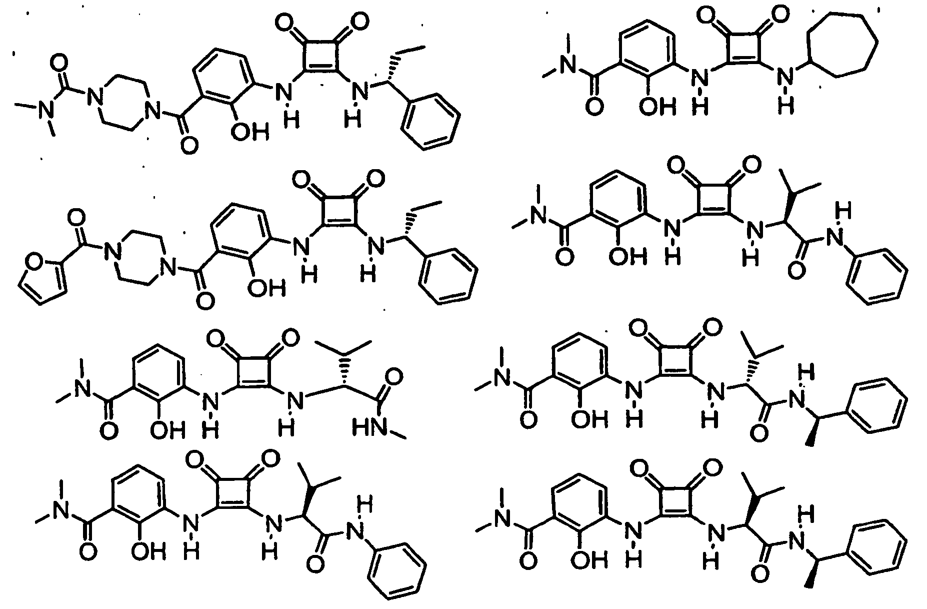

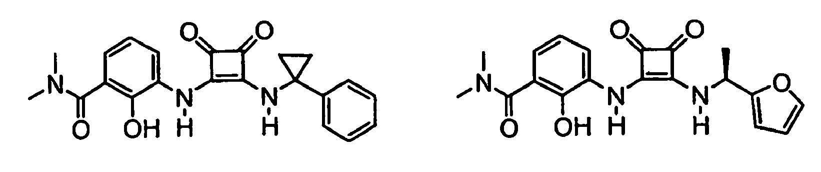

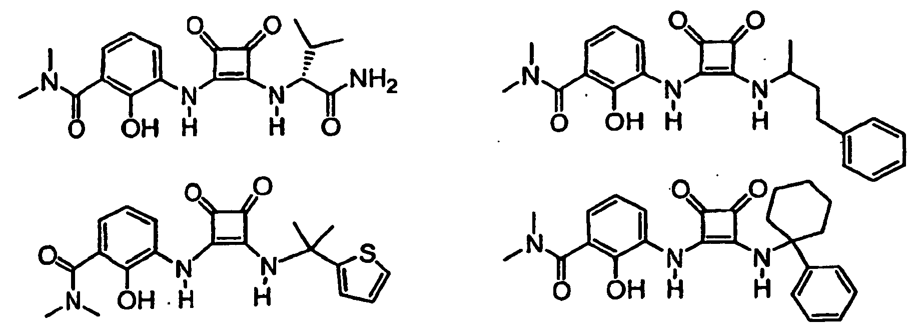

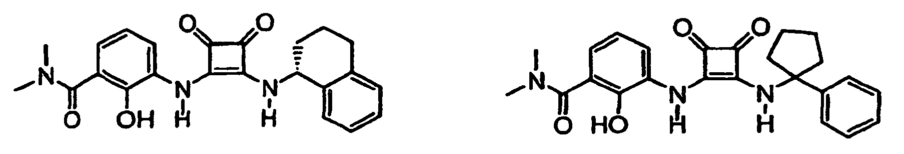

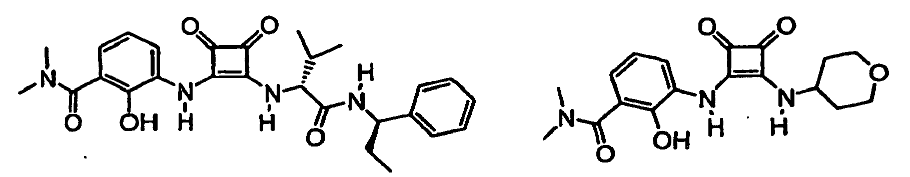

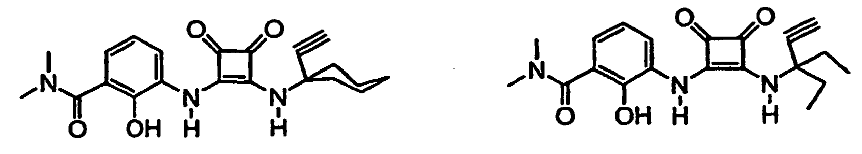

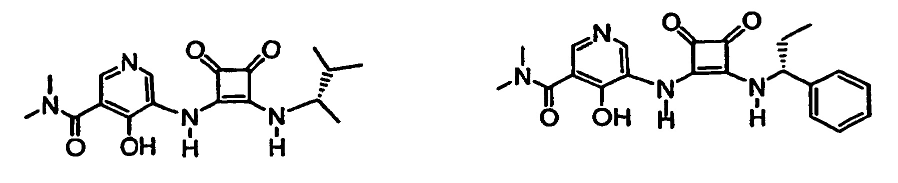

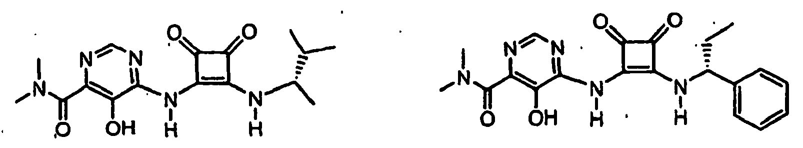

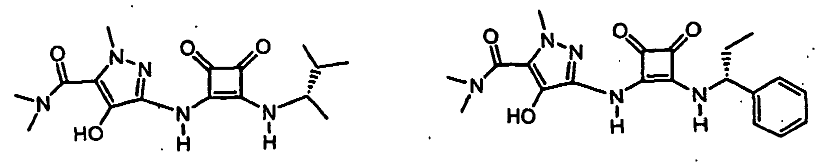

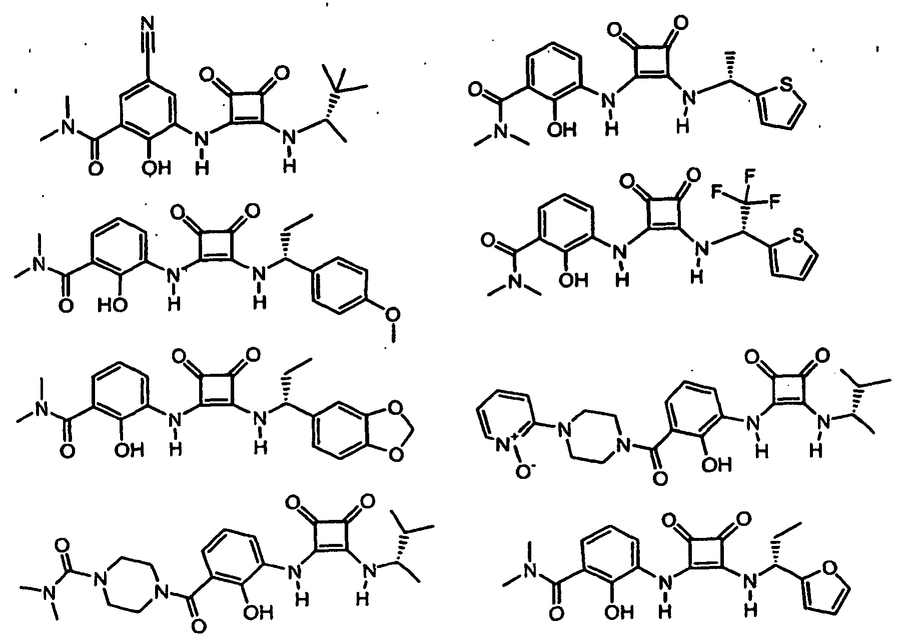

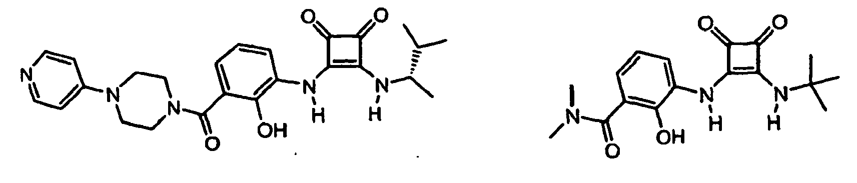

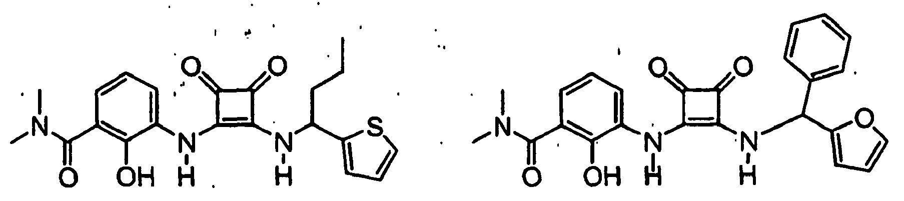

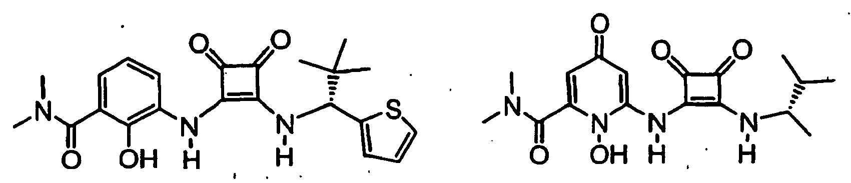

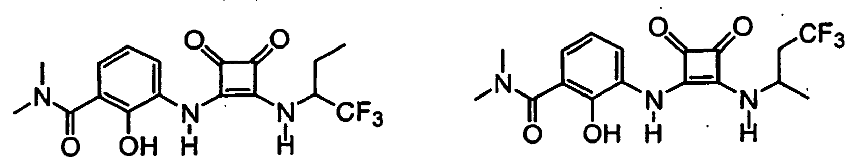

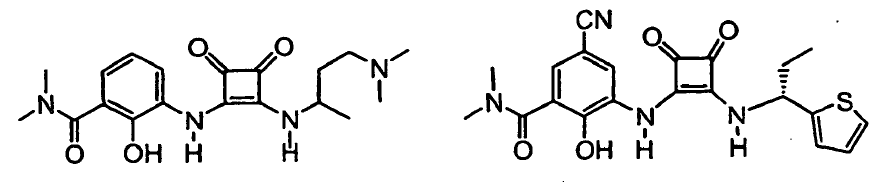

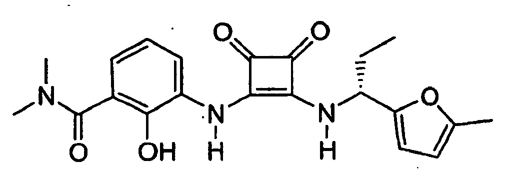

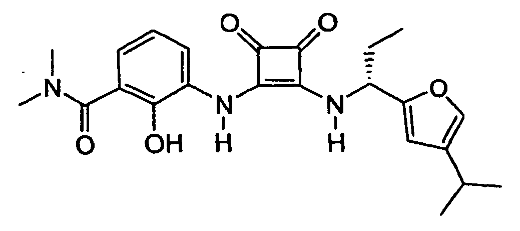

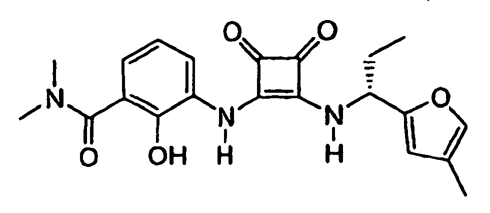

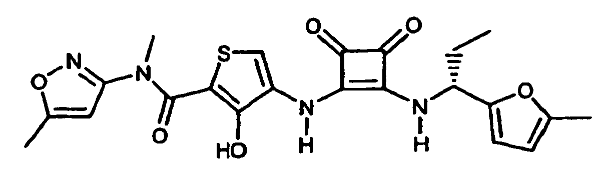

Preferred compounds of the invention include:

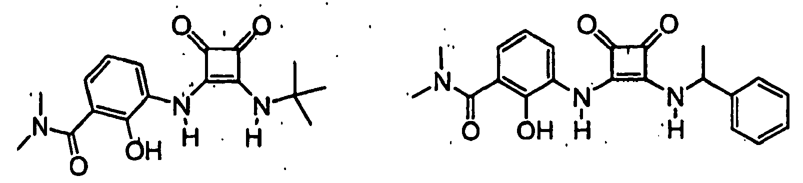

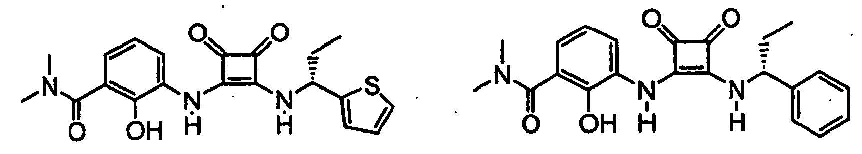

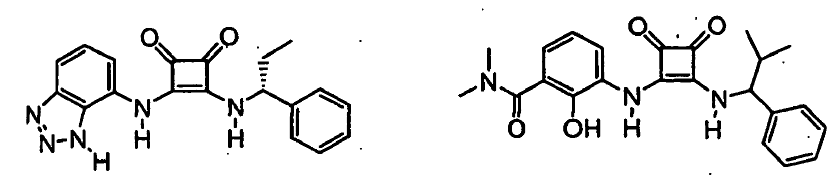

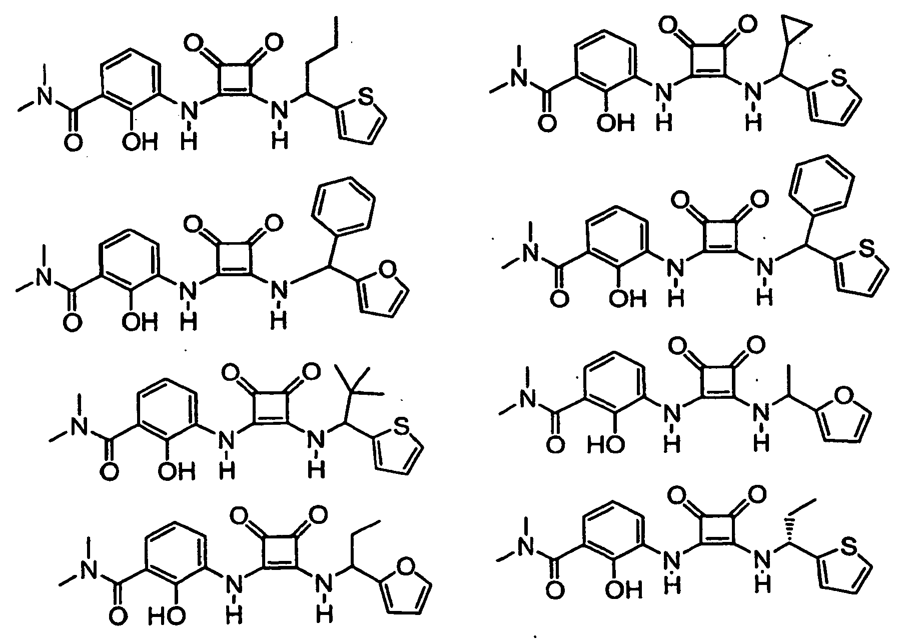

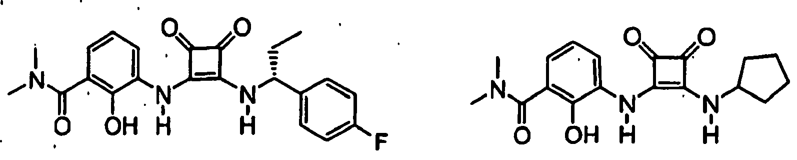

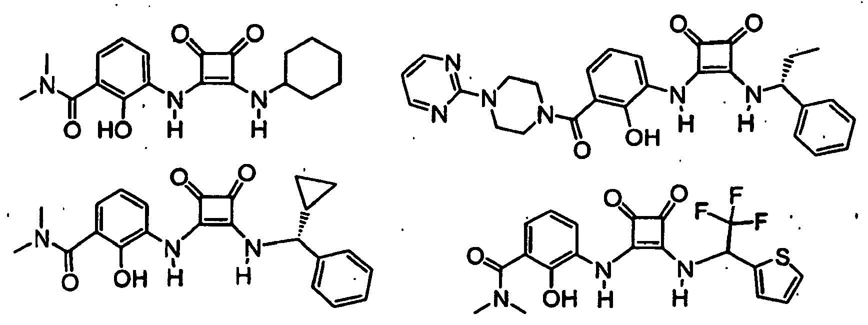

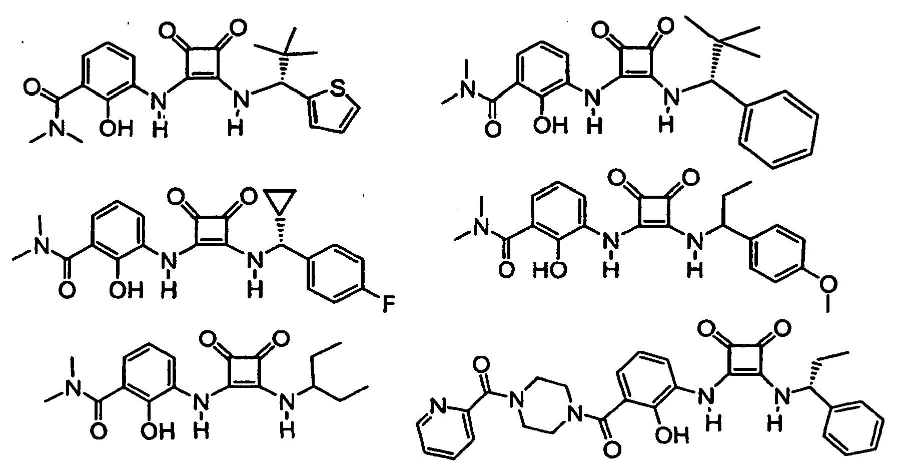

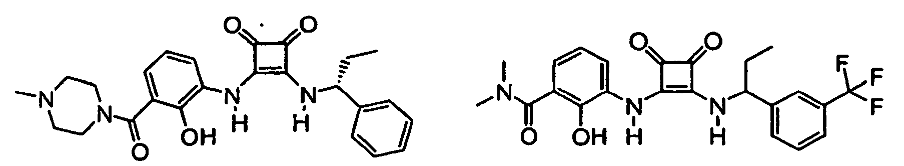

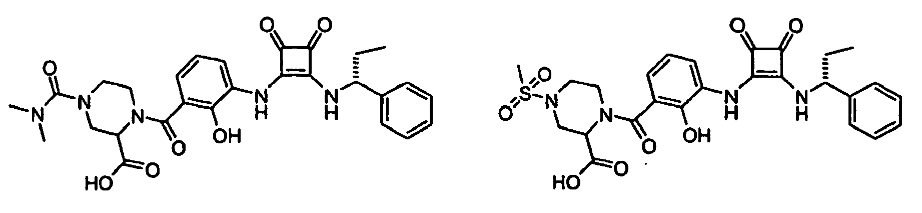

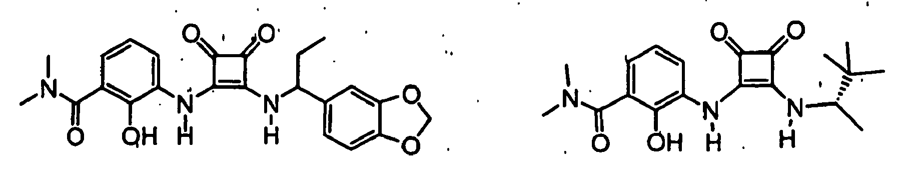

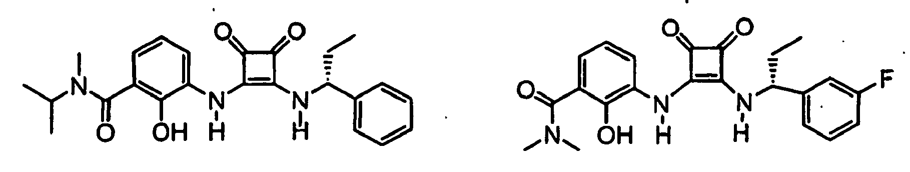

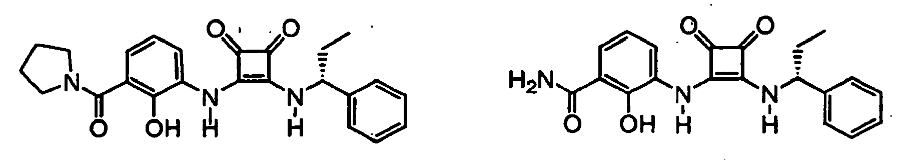

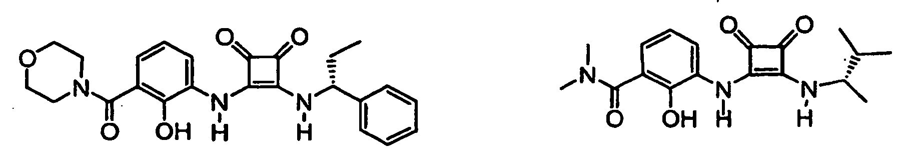

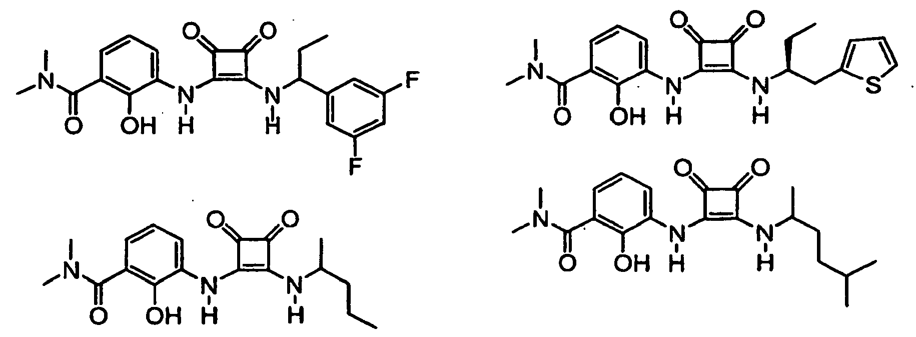

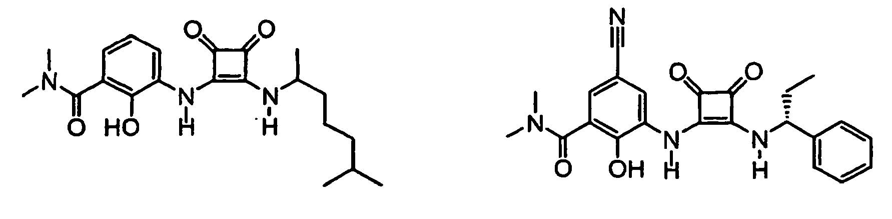

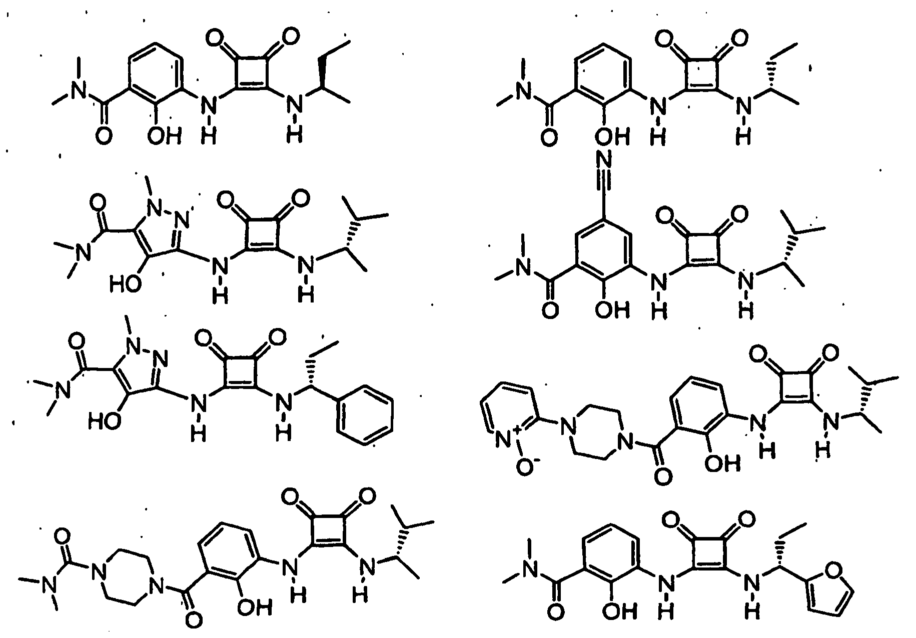

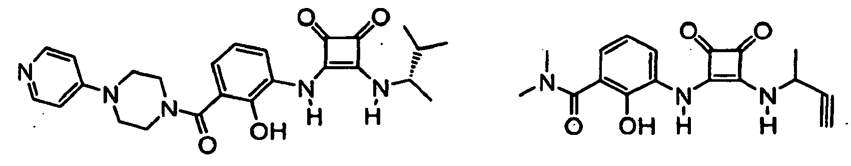

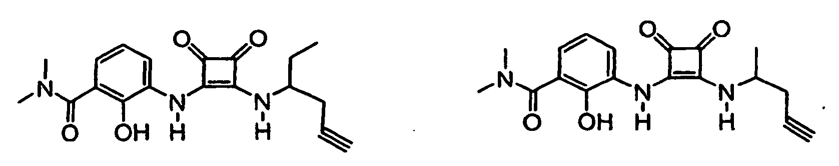

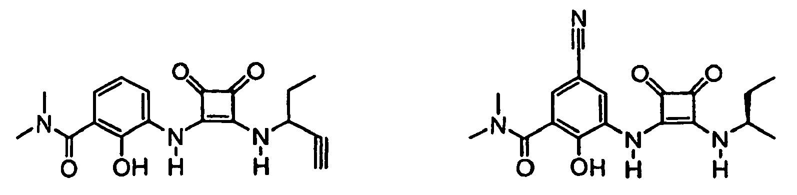

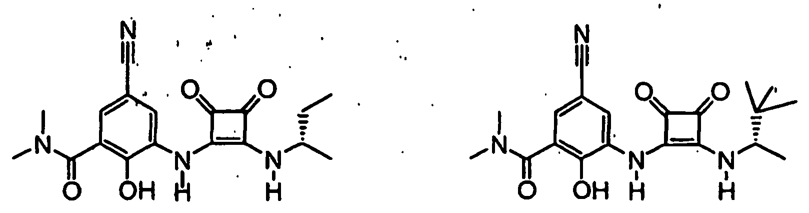

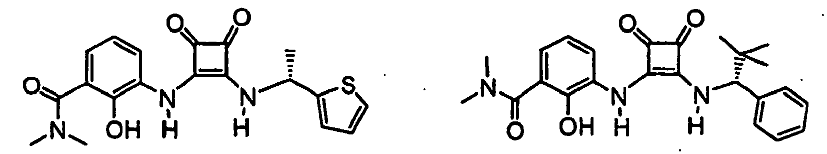

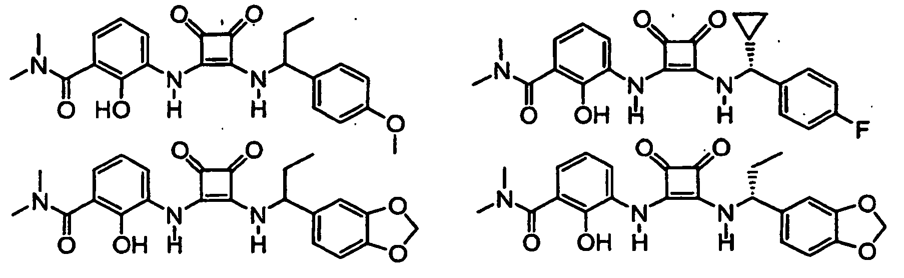

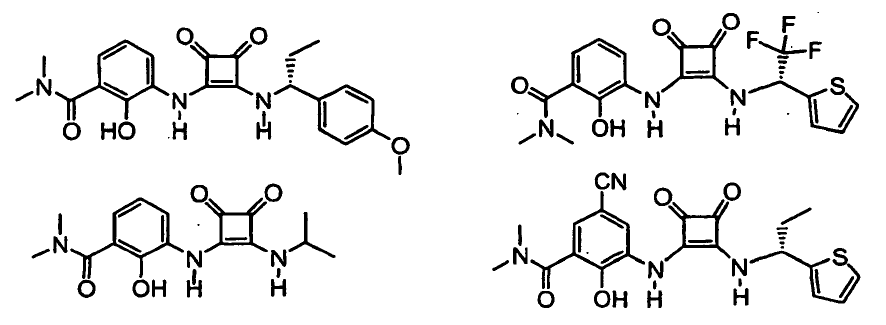

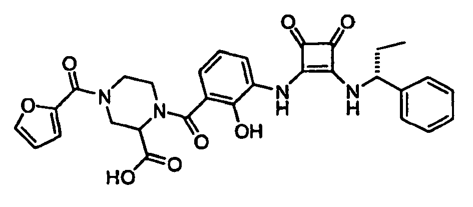

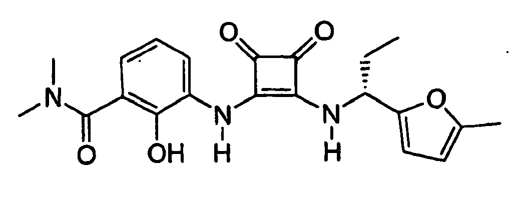

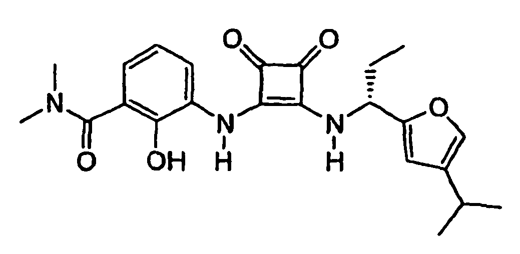

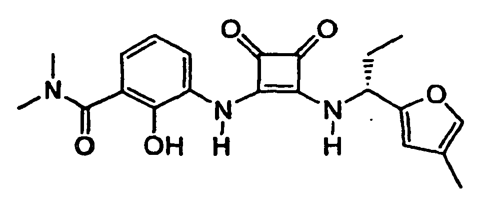

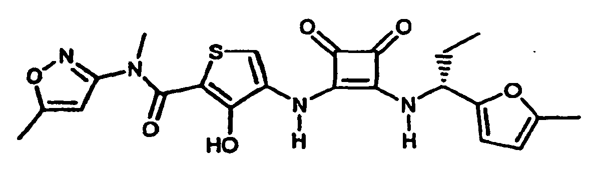

A more preferred group of compounds includes:

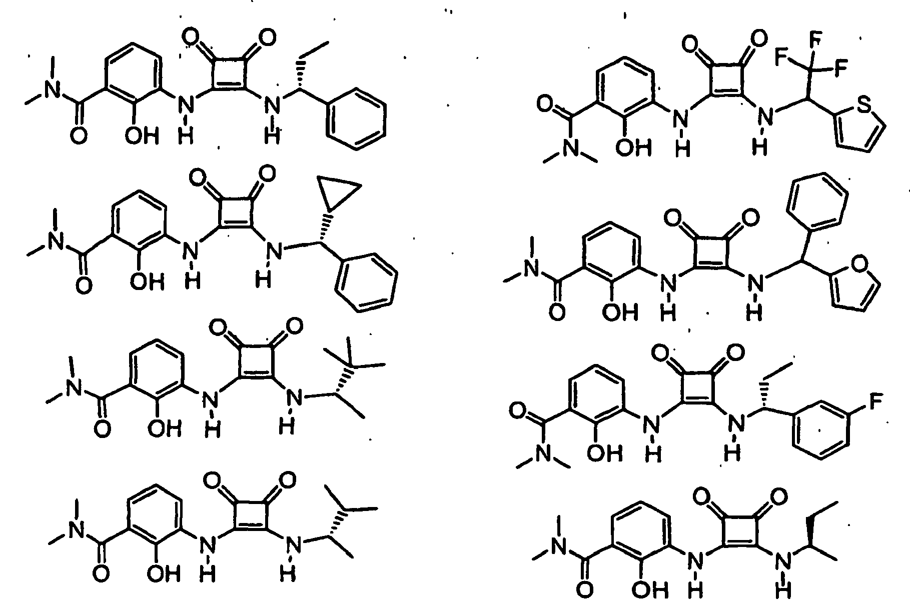

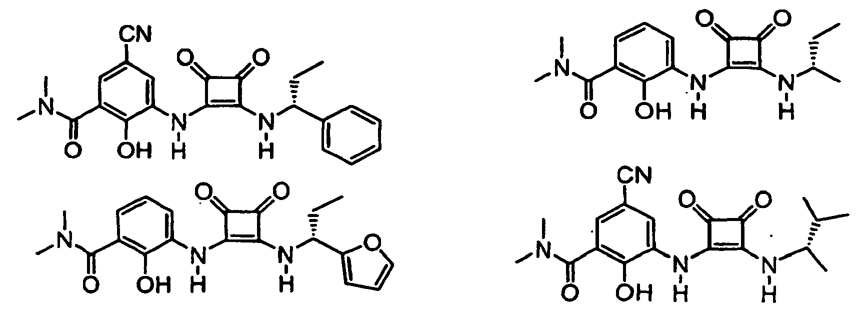

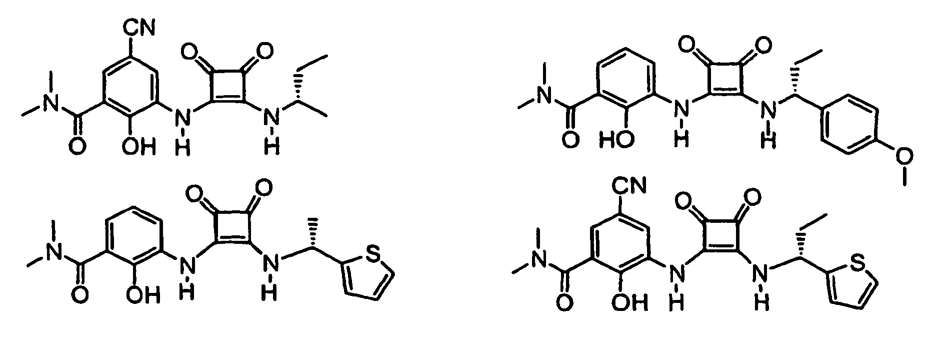

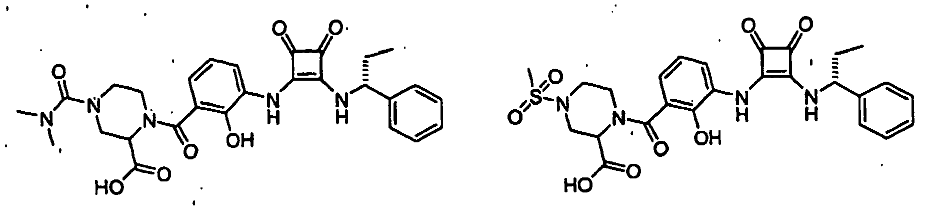

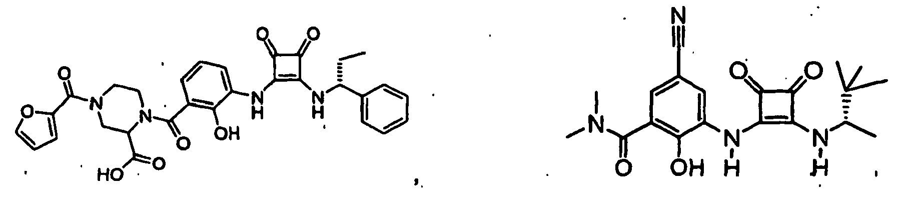

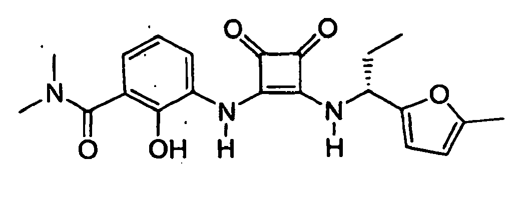

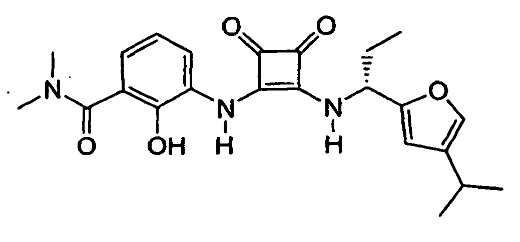

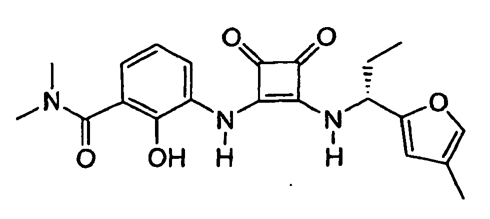

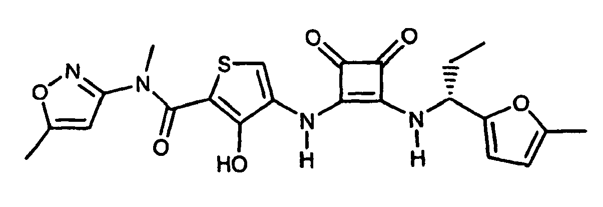

A most preferred group of compounds includes:

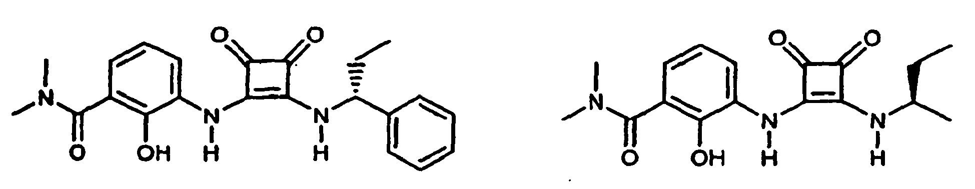

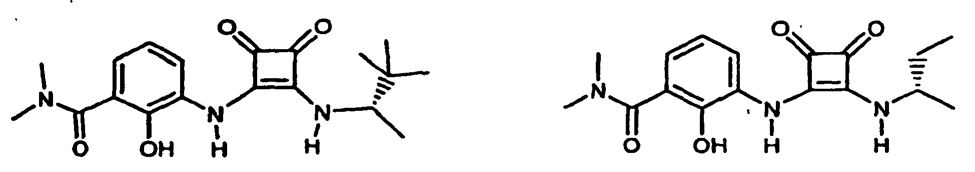

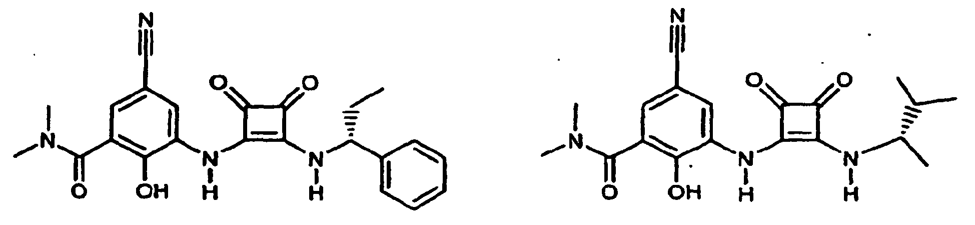

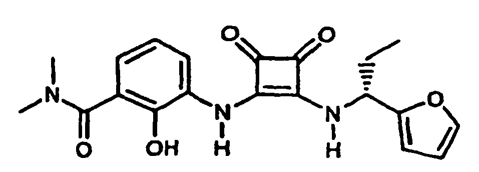

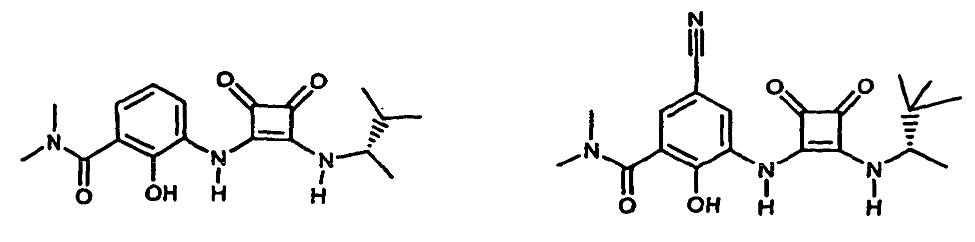

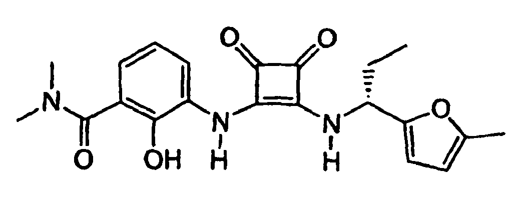

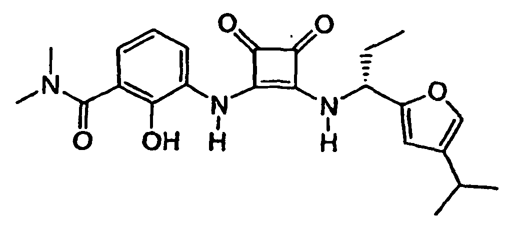

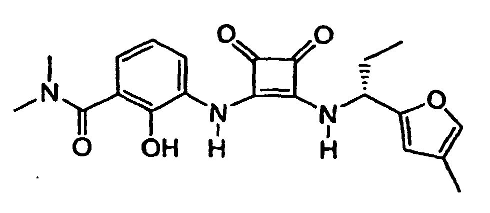

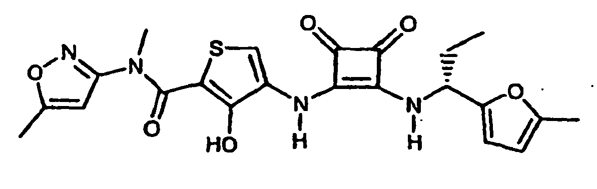

Certain compounds of the invention may exist in different stereoisomeric forms (e.g., enantiomers, diastereoisomers and atropisomers). The invention contemplates all such stereoisomers both in pure form and in admixture, including racemic mixtures. Isomers can be prepared using conventional methods.

Certain compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those skilled in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

Compounds of formula I or IA can exist in unsolvated and solvated forms, including hydrated forms. In general, the solvated forms, with pharmaceutically acceptable solvents such as water, ethanol and the like, are equivalent to the unsolvated forms for the purposes of this invention.

In a preferred embodiment, a compound of formula (1) or IA is combined with one of the following antineoplastic agents: gemcitabine, paclitaxel (Taxol®), 5-Fluorourcil (5-FU), cyclophosphamide (Cytoxan®), temozolomide, or Vincristine.

In another preferred embodiment, the present invention provides a method of treating cancer, comprising administering, concurrently or sequentially, and effective amount of a compound of formula (1) or IA and a microtubule affecting agent e.g., paclitaxel.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g., magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutical acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal composition can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation may be varied or adjusted from about 0.01 mg to about 1000 mg, preferably from about 0.01 mg to about 750 mg, more preferably from about 0.01 mg to about 500 mg, and most preferably from about 0.01 mg to about 250 mg, according to the particular application.

The actual dosage employed may be varied depending upon the requirements of the patient and the severity of the condition being treated. Determination of the proper dosage regimen for a particular situation is within the skill of the art. For convenience, the total dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 0.04 mg/day to about 4000 mg/day, in two to four divided doses.

Another aspect of the invention is a method treating cancer, comprising administering to a patient in need thereof, concurrently or sequentially, a therapeutically effective amount of (a) a compound of formula (I) or IA and (b) an atineoplastic agent, microtubule affecting agent or anti-angiogenesis agent.

Classes of compounds that can be used as the chemotherapeutic agent (antineoplastic agent) include: alkylating agents, antimetabolites, natural products and their derivatives, hormones and steroids (including synthetic analogs), and synthetics. Examples of compounds within these classes are given below.

Alkylating agents (including nitrogen mustards, ethylenimine derivatives, alkyl sulfonates, nitrosoureas and triazenes): Uracil mustard, Chlormethine, Cyclophosphamide (Cytoxan^{®}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide.

Antimetabolites (including folic acid antagonists, pyrimidine analogs, purine analogs and adenosine deaminase inhibitors): Methotrexate, 5-Fluorouracil; Floxuridine, Cytarabine, 6-Mercaptopurine, 6-Thioguanine, Fludarabine phosphate, Pentostatine, and Gemcitabine.

Natural products and their derivatives (including vinca alkaloids, antitumor antibiotics, enzymes, lymphokines and epipodophyllotoxins): Vinblastine, Vincristine, Vindesine, Bleomycin, Dactinomycin, Daunorubicin, Doxorubicin, Epirubicin, Idarubicin, paclitaxel (paclitaxel is commercially available as Taxol^{®} and is described in more detail below in the subsection entitled "Microtubule Affecting Agents"), Mithramycin, Deoxyco-formycin, Mitomycin-C, L-Asparaginase, Interferons (especially IFN-a), Etoposide, and Teniposide.

Hormones and steroids (including synthetic analogs): 17α-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, Zoladex.

Synthetics (including inorganic complexes such as platinum coordination complexes): Cisplatin, Carboplatin, Hydroxyurea, Amsacrine, Procarbazine, Mitotane, Mitoxantrone, Levamisole, and Hexamethylmelamine.

Methods for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 2002 edition (Medical Economics Company, Montvale, NJ 07645-1742, USA); the disclosure of which is incorporated herein by reference thereto.

As used herein, a microtubule affecting agent is a compound that interferes with cellular mitosis, *i.e.*, having an anti-mitotic effect, by affecting microtubule formation and/or action. Such agents can be, for instance, microtubule stabilizing agents or agents that disrupt microtubule formation.

Microtubule affecting agents useful in the invention are well known to those of skill in the art and include, but are not limited to allocolchicine (NSC 406042), Halichondrin B (NSC 609395), colchicine (NSC 757), colchicine derivatives (e.g., NSC 33410), dolastatin 10 (NSC 376128), maytansine (NSC 153858), rhizoxin (NSC 332598), paclitaxel (Taxol^{®}, NSC 125973), Taxol^{®} derivatives (*e.g.*, derivatives (*e.g.*, NSC 608832), thiocolchicine (NSC 361792), trityl cysteine (NSC 83265), vinblastine sulfate (NSC 49842), vincristine sulfate (NSC 67574), epothilone A, epothilone, and discodermolide (see Service, (1996) Science, 274:2009) estramustine, nocodazole, MAP4, and the like. Examples of such agents are also described in the scientific and patent literature, see, *e.g.,* Bulinski (1997) J. Cell Sci. 110:3055-3064;Panda (1997) Proc. Natl. Acad. Sci. USA 94:10560-10564; Muhlradt (1997) Cancer Res. 57:3344-3346; Nicolaou (1997) Nature 387:268-272; Vasquez (1997) Mol. Biol. Cell. 8:973-985; Panda (1996) J. Biol. Chem. 271:29807-29812.

Particularly preferred agents are compounds with paclitaxel-like activity. These include, but are not limited to paclitaxel and paclitaxel derivatives (paclitaxel-like compounds) and analogues. Paclitaxel and its derivatives are available commercially. In addition, methods of making paclitaxel and paclitaxel derivatives and analogues are well known to those of skill in the art (*see, e.g.,* U.S. Patent Nos: 5,569,729; 5,565,478; 5,530,020; 5,527,924; 5,508,447; 5,489,589; 5,488,116; 5,484,809; 5,478,854; 5,478,736; 5,475,120; 5,468,769; 5,461;169; 5,440,057; 5,422,364; 5,411,984; 5,405,972; and 5,296,506).

More specifically, the term "paclitaxel" as used herein refers to the drug commercially available as Taxol^{®}(NSC number: 125973). Taxol^{®} inhibits eukaryotic cell replication by enhancing polymerization of tubulin moieties into stabilized microtubule bundles that are unable to reorganize into the proper structures for mitosis. Of the many available chemotherapeutic drugs, paclitaxel has generated interest because of its efficacy in clinical trials against drug-refractory tumors, including ovarian and mammary gland tumors (Hawkins (1992) Oncology, 6: 17-23, Horwitz (1992) Trends Pharmacol. Sci. 13: 134-146, Rowinsky (1990) J. Natl. Canc. Inst. 82: 1247-1259).

Additional microtubule affecting agents can be assessed using one of many such assays known in the art, *e.g.*, a semiautomated assay which measures the tubulin-polymerizing activity of paclitaxel analogs in combination with a cellular assay to measure the potential of these compounds to block cells in mitosis (see Lopes (1997) Cancer Chemother. Pharmacol. 41:37-47).

Generally, activity of a test compound is determined by contacting a cell with that compound and determining whether or not the cell cycle is disrupted, in particular, through the inhibition of a mitotic event. Such inhibition may be mediated by disruption of the mitotic apparatus, *e.g.*, disruption of normal spindle formation. Cells in which mitosis is interrupted may be characterized by altered morphology (*e.g*., microtubule compaction, increased chromosome number, etc.).

In a preferred embodiment, compounds with possible tubulin polymerization activity are screened *in vitro.* In a preferred embodiment, the compounds are screened against cultured WR21 cells (derived from line 69-2 wap-ras mice) for inhibition of proliferation and/or for altered cellular morphology, in particular for microtubule compaction. *In vivo* screening of positive-testing compounds can then be performed using nude mice bearing the WR21 tumor cells. Detailed protocols for this screening method are described by Porter (1995) Lab. Anim. Sci., 45(2):145-150.

Other methods of screening compounds for desired activity are well known to those of skill in the art. Typically such assays involve assays for inhibition of microtubule assembly and/or disassembly. Assays for microtubule assembly are described, for example, by Gaskin et al. (1974) J. Molec. Biol., 89: 737-758. U.S. Patent No. 5,569,720 also provides *in vitro* and *in vivo* assays for compounds with paclitaxel-like activity.

Methods for the safe and effective administration of the above-mentioned microtubule affecting agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, NJ 07645-1742, USA); the disclosure of which is incorporated herein by reference thereto.

The amount and frequency of administration of the compounds of formula (I) or IA and the chemotherapeutic agents and/or radiation therapy will be regulated according to the judgment of the attending clinician (physician) considering such factors as age, condition and size of the patient as well as severity of the disease being treated. A dosage regimen of the compound of formula (I) or IA can be oral administration of from 10 mg to 2000 mg/day, preferably 10 to 1000 mg/day, more preferably 50 to 600 mg/day, in two to four (preferably two) divided doses, to block tumor growth. Intermittant therapy (*e.g.*, one week out of three weeks or three out of four weeks) may also be used.

The chemotherapeutic agent and/or radiation therapy can be administered according to therapeutic protocols well known in the art. It will be apparent to those skilled in the art that the administration of the chemotherapeutic agent and/or radiation therapy can be varied depending on the disease being treated and the known effects of the chemotherapeutic agent and/or radiation therapy on that disease. Also, in accordance with the knowledge of the skilled clinician, the therapeutic protocols (e.g., dosage amounts and times of administration) can be varied in view of the observed effects of the administered therapeutic agents (i.e., antineoplastic agent or radiation) on the patient, and in view of the observed responses of the disease to the administered therapeutic agents.

In the methods of this invention, a compound of formula (I) or IA is administered concurrently or sequentially with a chemotherapeutic agent and/or radiation. Thus, it is not necessary that, for example, the chemotherapeutic agent and the compound of formula (I) or IA, or the radiation and the compound of formula (I) or IA, should be administered simultaneously or essentially simultaneously. The advantage of a simultaneous or essentially simultaneous administration is well within the determination of the skilled clinician.

Also, in general, the compound of formula (I) or IA and the chemotherapeutic agent do not have to be administered in the same pharmaceutical composition, and may, because of different physical and chemical characteristics, have to be administered by different routes. For example, the compound of formula (I) or IA may be administered orally to generate and maintain good blood levels thereof, while the chemotherapeutic agent may be administered intravenously. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

The particular choice of a compound of formula (I) or IA, and chemotherapeutic agent and/or radiation will depend upon the diagnosis of the attending physicians and their judgement of the condition of the patient and the appropriate treatment protocol.

The compound of formula (I) or IA, and chemotherapeutic agent and/or radiation may be administered concurrently (e.g., simultaneously, essentially simultaneously or within the same treatment protocol) or sequentially, depending upon the nature of the proliferative disease, the condition of the patient, and the actual choice of chemotherapeutic agent and/or radiation to be administered in conjunction (i.e., within a single treatment protocol) with the compound of formula (I) or IA.

If the compound of formula (I) or IA, and the chemotherapeutic agent and/or radiation are not administered simultaneously or essentially simultaneously, then the initial order of administration of the compound of formula (I) and IA, and the chemotherapeutic agent and/or radiation, may not be important. Thus, the compound of formula (I) or IA may be administered first, followed by the administration of the chemotherapeutic agent and/or radiation; or the chemo-therapeutic agent and/or radiation may be administered first, followed by the administration of the compound of formula (I) or IA. This alternate administration may be repeated during a single treatment protocol. The determination of the order of administration, and the number of repetitions of administration of each therapeutic agent during a treatment protocol, is well within the knowledge of the skilled physician after evaluation of the disease being treated and the condition of the patient.

For example, the chemotherapeutic agent and/or radiation may be administered first, especially if it is a cytotoxic agent, and then the treatment continued with the administration of the compound of formula (I) or IA followed, where determined advantageous, by the administration of the chemotherapeutic agent and/or radiation, and so on until the treatment protocol is complete.

Thus, in accordance with experience and knowledge, the practicing physician can modify each protocol for the administration of a component (therapeutic agent-*i.e.*, the compound of formula (I) or IA, chemotherapeutic agent or radiation) of the treatment according to the individual patient's needs, as the treatment proceeds.

The attending clinician, in judging whether treatment is effective at the dosage administered, will consider the general well-being of the patient as well as more definite signs such as relief of disease-related symptoms, inhibition of, tumor growth, actual shrinkage of the tumor, or inhibition of metastasis. Size of the tumor can be measured by standard methods such as radio-logical studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

### BIOLOGICAL EXAMPLES

The compounds of the present invention are useful in the treatment of CXC-chemokine mediated conditions and diseases. This utility is manifested in their ability to inhibit IL-8 and GRO-α chemokine as demonstrated by the following *in vitro* assays.

### Receptor Binding Assays:

### CXCR1 SPA Assay

For each well of a 96 well plate, a reaction mixture of 10 µg hCXCR1-CHO overexpressing membranes (Biosignal) and 200 µg/well WGA-SPA beads (Amersham) in 100 µl was prepared in CXCR1 assay buffer (25 mM HEPES, pH 7.8, 2 mM CaCl₂, 1mM MgCl₂, 125 mM NaCl, 0.1% BSA) (Sigma). A 0.4 nM stock of ligand, [1251]-IL-8 (NEN) was prepared in the CXCR1 assay buffer. 20X stock solutions of test compounds were prepared in DMSO (Sigma). A 6 X stock solution of IL-8 (R&D) was prepared in CXCR2 assay buffer. The above solutions were added to a 96-well assay plate (PerkinElmer) as follows: 10 µl test compound or DMSO, 40 µl CXCR1 assay buffer or IL-8 stock, 100 µl of reaction mixture, 50 µl of ligand stock (Final [Ligand] = 0.1 nM). The assay plates were shaken for 5 minutes on plate shaker, then incubated for 8 hours before cpm/well were determined in Microbeta Trilux counter (PerkinElmer). % Inhibition of Total binding-NSB (250 nM IL-8) was determined for IC50 values. Compounds of this invention had an IC₅₀ of <20µM. The most preferred compounds had a Kᵢ within the range of 3nM to 1120nM.

### CXCR2 SPA Assay

For each well of a 96 well plate, a reaction mixture of 4 µg hCXCR2-CHO overexpressing membranes (Biosignal) and 200 µg/well WGA-SPA beads (Amersham) in 100 µl was prepared in CXCR2 assay buffer (25 mM HEPES, pH 7.4. 2 mM CaCl₂, 1mM MgCl₂). A 0.4 nM stock of ligand, [1251]-IL-8 (NEN), was prepared in the CXCR2 assay buffer. 20X stock solutions of test compounds were prepared in DMSO (Sigma). A 6 X stock solution of GRO-α (R&D) was prepared in CXCR2 assay buffer. The above solutions, were added to a 96-well assay plate (PerkinElmer or Coming) as follows: 10 µl test compound or DMSO, 40 ul CXCR2 assay buffer or GRO-α stock, 100 µl of reaction mixture, 50 µl of ligand stock (Final [Ligand] = 0.1 nM). When 40 X stock solutions of test compounds in DMSO were prepared, then the above protocol was used except instead 5 µl test compound or DMSO and 45 µl CXCR2 assay buffer were used. The assay plates were shaken for 5 minutes on a plate shaker, then incubated for 2-8 hours before cpm/well were determined in Microbeta Trilux counter (PerkinElmer). % Inhibition of total binding minus non-specific binding (250 nM Gro-α or 50 µM antagonist) was determined and IC50 values calculated. Compounds of this invention had an IC₅₀ of <5µM. The most preferred compounds had a Kᵢ within the range of 0.8nM to 40nM.

### Calcium Fluorescence Assay (FLIPR)

HEK 293 cells stably transfected with hCXCR2 and Gα1/q were plated at 10,000 cells per well in a Poly-D-Lysine Black/Clear plate (Becton Dickinson) and incubated 48 hours at 5% CO₂, 37°C. The cultures were then incubated with 4 mM fluo-4, AM (Molecular Probes) in Dye Loading Buffer (1% FBS, HBSS w. Ca & Mg, 20 mM HEPES (Cellgro), 2.5 mM Probenicid (Sigma) for 1 hour. The cultures were washed with wash buffer (HBSS w Ca, & Mg, 20 mM HEPES, Probenicid (2.5 mM)) three times, then 100 µl/well wash buffer was added.

During incubation, compounds were prepared as 4X stocks in 0.4% DMSO (Sigma) and wash buffer and added to their respective wells in the first addition plate. IL-8 or GRO-α (R&D Systems) concentrations were prepared 4X in wash buffer + 0.1% BSA and added to their respective wells in second addition plate.

Culture plate and both addition plates were then placed in the FLIPR imaging system to determine change in calcium fluorescence upon addition of compound and then ligand. Briefly, 50 µl of compound solutions or DMSO, solution was added to respective wells and change in calcium fluorescence measured by the FLIPR for 1 minute. After a 3 minute incubation within the instrument, 50 µl of ligand was then added and the change in calcium fluorescence measured by the FLIPR instrument for I minute. The area under each stimulation curve was determined and values used to determine % Stimulation by compound (agonist) and % Inhibition of Total Calcium response to ligand (0.3 nM IL-8 or GRO-α) for IC50 values of the test compounds.

### Chemotaxis assays for 293-CXCR2

A chemotaxis assay is setup using Fluorblok inserts (Falcon) for 293-CXCR2 cells (HEK-293 cells overexpressing human CXCR2). The standard protocol used at present is as follows:
1. Inserts are coated with collagenIV (2ug/ml) for 2 hrs at 37°C.
2. The collagen is removed and inserts are allowed to air dry overnight.
3. Cells are labeled with 10uM calcein AM (Molecular Probes) for 2 hrs. Labeling is done in complete media with 2% FBS.
4. Dilutions of compound are made in minimal media (0.1% BSA).and placed inside the insert which is positioned inside the well of a 24 well plate. Within the well is IL-8 at a concentration of 0.25nM in minimal media. Cells are washed and resuspended in minimal media and placed inside the insert at a concentration of 50,000 cells per insert.
5. Plate is incubated for 2hrs and inserts are removed and placed in a new 24 well. Fluorescence is detected at excitation=485 nM and emission=530 nM.

### Cytotoxicity Assays

A cytotoxicity assay for CXCR2 compounds is conducted on 293-CXCR2 cells. Concentrations of compounds are tested for toxicity at high concentrations to determine if they may be used for further evaluation in binding and cell based assays. The protocol is as follows:
1. 293-CXCR2 cells are plated overnight at a concentration of 5000 cells per well in complete media.
2. Dilutions of compound are made in minimal media w/0.1 % BSA. Complete media is poured off and the dilutions of compound are added. Plates are incubated for 4, 24 and 48hrs. Cells are labeled with 10uM calcein AM for 15 minutes to determine cell viability. Detection method is the same as above.

### Soft Agar Assay

10,000 SKMEL-5 cells/well are placed in a mixture of 1.2% agar and complete media with various dilutions of compound. Final concentration of agar is 0.6%. After 21 days viable cell colonies are stained with a solution of MTT (1mg/ml in PBS). Plates are then scanned to determine colony number and size. IC₅₀ is determined by comparing total area vs. compound concentration.

Compounds of formula (I) or IA may be produced by processes known to those skilled in the art in the following reaction schemes and in the preparations and examples below.

A general procedure for the preparation of compounds of formula I or IA is as follows:

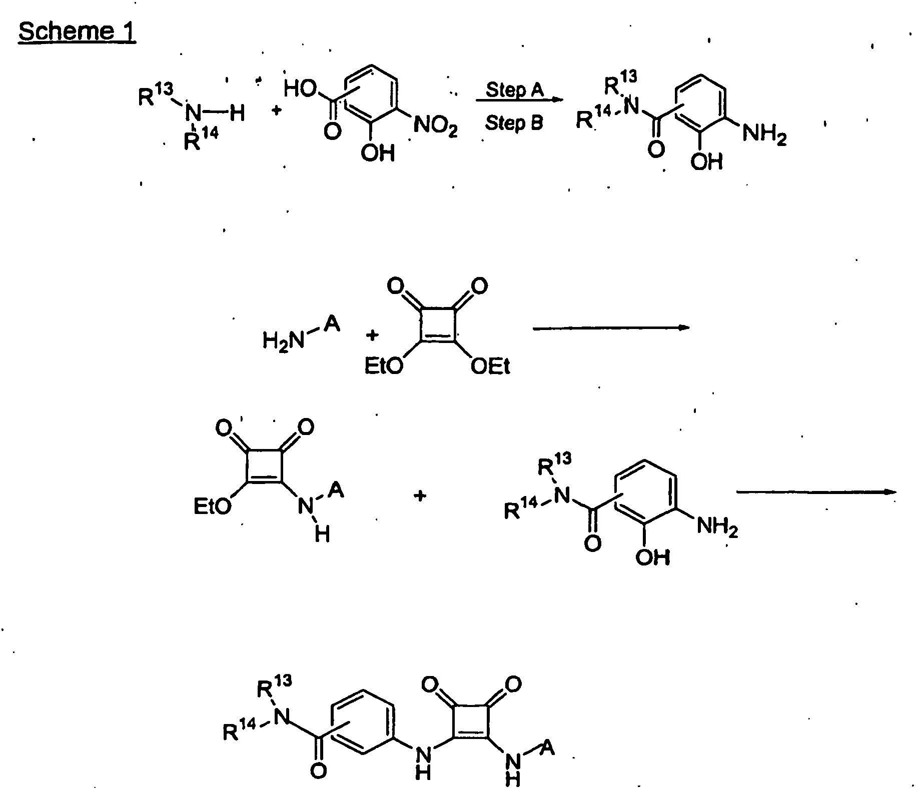

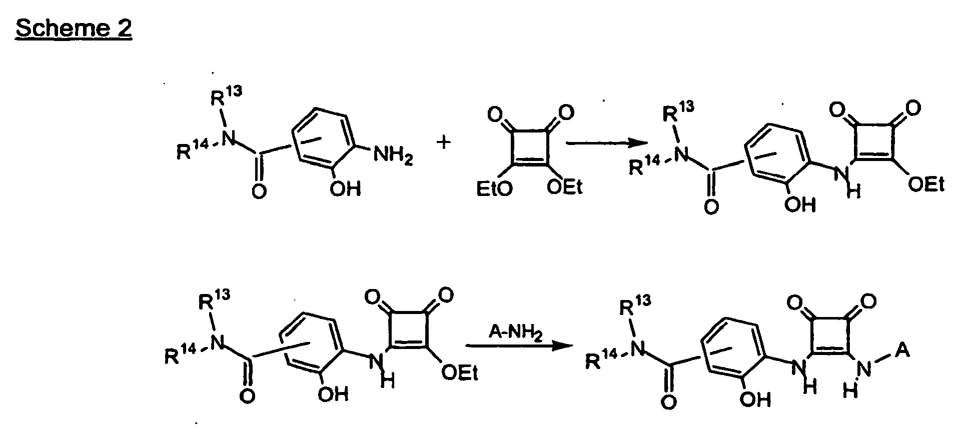

### Scheme 1

An amine is condensed (Step A) with a nitrosalicylic acid under standard coupling conditions and the resulting nitrobenzamide is reduced (Step B) under hydrogen atmosphere in the presence of a suitable catalyst. The remaining partner required for the synthesis of the final target is prepared by condensing an aryl amine with the commercially available diethylsquarate to give the aminoethoxysquarate product. Subsequent condensation of this intermediate with the aminobenzamide prepared earlier provides the desired chemokine antagonist (Scheme 1).

### Scheme 2

Alternatively, the aminobenzamide of Scheme 1 is first condensed with commercially available diethylsquarate to give an alternate monoethoxy intermediate. Condensation of this intermediate with an amine gives the desired chemokine antagonist.

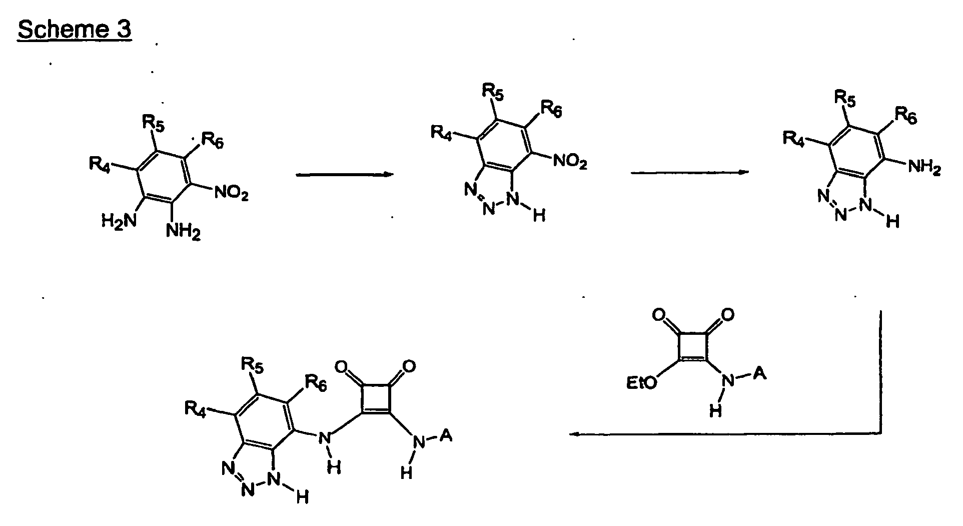

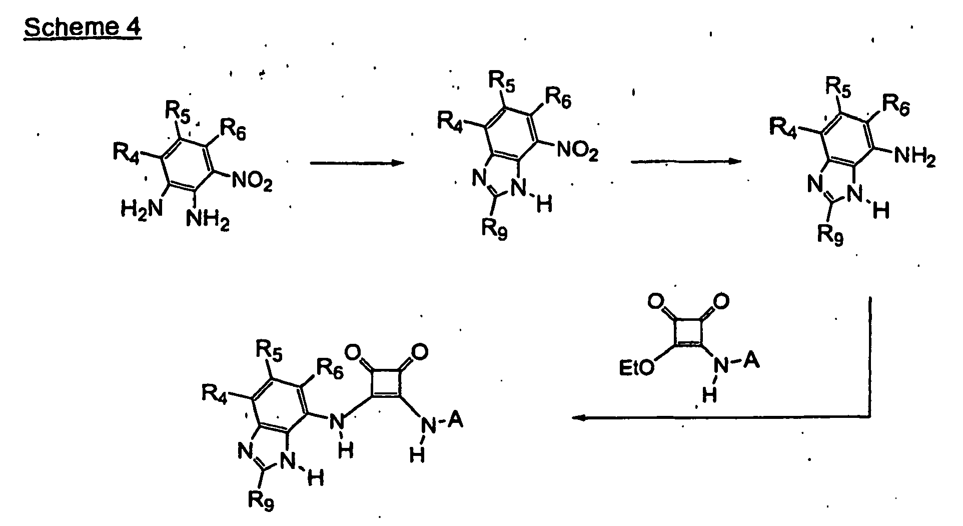

### Scheme 3

Benztriazole compounds of Formula (I) or IA are prepared by stirring nitrophenylenediamines with sodium nitrite in acetic acid at 60°C to afford the nitrobenzotriazole intermediate (Scheme 3). Reduction of the nitro group in the presence of palladium catalyst and hydrogen atmosphere provides the amine compound. Subsequent condensation of this intermediate with the aminooethoxysquarate prepared earlier (Scheme 1) provides the desired chemokine antagonist.

### Scheme 4

Condensation of nitrophenylenediamines with anhydrides or activated acids at reflux (Scheme 4) affords benzimidazole intermediates which after reduction with hydrogen gas and palladium catalyst and condensation with the aminoethoxysquarate previously prepared (Scheme 1) affords benzimidazole chemokine antagonists.

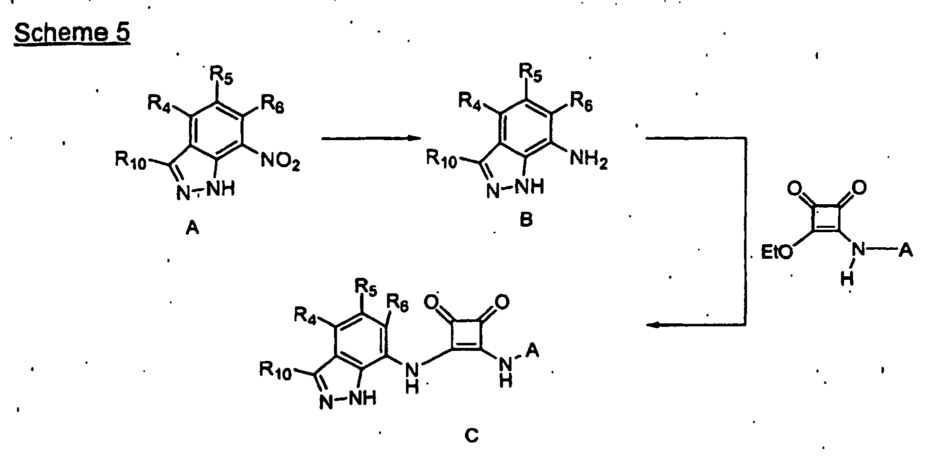

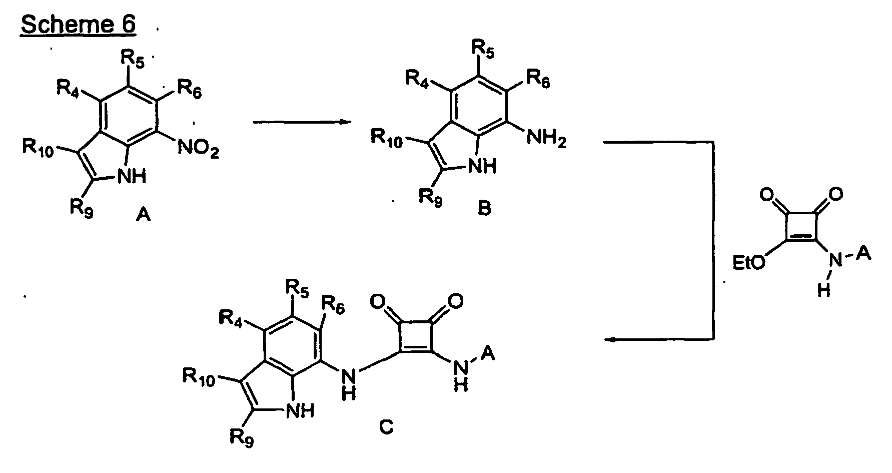

### Scheme 5

Indazole structures of Formula (I) or IA can be prepared according to Scheme 5 by reduction of nitroindazole A (J. Am. Chem Soc. 1943, 65,1804-1805) to give aminoindazole B and subsequent condensation with the aminoethoxysquarate prepared earlier (Scheme 1).

### Scheme 6

Indole structures of Formula (I) or IA can be prepared according to Scheme 6 by reduction of nitroindole A (J. Med. Chem. 1995, 38, 1942-1954) to give aminoindole B and subsequent condensation with the aminoethoxysquarate prepared earlier (Scheme 1).

The invention disclosed herein is exemplified by the following preparations and examples which should not be construed to limit the scope of the disclosure. Alternative mechanistic pathways and analogous structures may be apparent to those skilled in the art.

### PREPARATIVE EXAMPLE 1

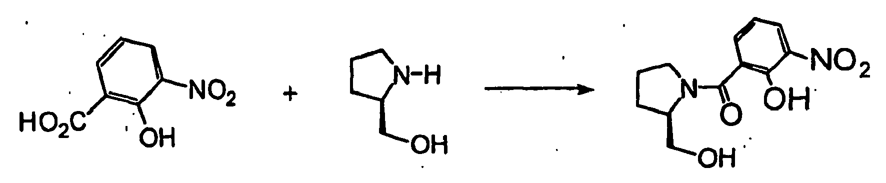

3-Nitrosalicylic acid (500 mg, 2.7 mmol), DCC (563 mg) and ethyl acetate (10 mL) were combined and stirred for 10 min. *(R)* -(-)-2-pyrrolidinemethanol (0.27 mL) was added and the resulting suspension was stirred at room temperature overnight. The solid was filtered and the filtrate washed with 1N NaOH. The aqueous phase was acidified and extracted with EtOAc. The resulting organic phase was dried over anhydrous MgSO₄, filtered and concentrated *in vacuo.* Purification of the residue by preparative plate chromatography (silica gel, 5% MeOH/CH₂Cl₂ saturated with AcOH) gave the product (338 mg, 46%, MH⁺ = 267).

### PREPARATIVE EXAMPLE 2

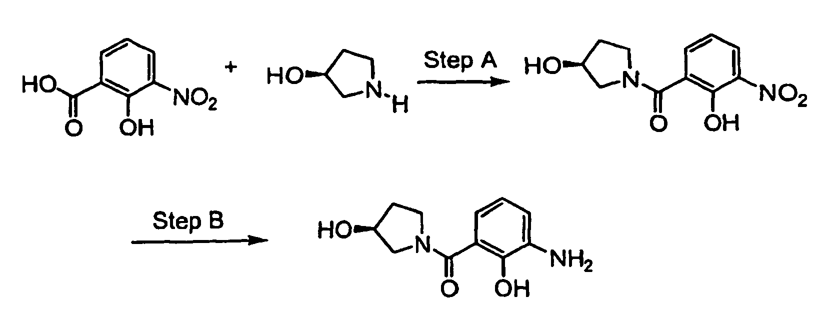

### Step A

3-Nitrosalicylic acid (9.2 g), bromotripyrrolidinophosphonium hexafluorophosphate (PyBroP, 23 g) and N,N-diisopropylethylamine (DIEA, 26 mL) in anhydrous CH₂Cl₂ (125 mL) were combined and stirred at 25°C for 30 min. *(R)* -(+)-3-pyrrolidinol (8.7 g) in CH₂Cl₂ (25 mL) was added over 25 min and the resulting suspension was stirred at room temperature overnight. The mixture was extracted with 1M NaOH (aq) and the organic phase was discarded. The aqueous phase was acidified with 1M HCl (aq), extracted with EtOAc, dried over anhydrous Na₂SO₄, filtered and concentrated *in vacuo* to afford the crude product (7 g) which was used without further purification.

### Step B

The crude product from Step A above was stirred with 10% Pd/C (0.7 g) in MeOH (100 mL) under a hydrogen gas atmosphere overnight. The reaction mixture was filtered through celite, the filtrate concentrated *in vacuo,* and the resulting residue purified by column chromatography (silica gel, 10% MeOH/CH₂Cl₂ saturated with NH₄OH) to give the product (2.5 g, 41%, MH+=223).

### PREPARATIVE EXAMPLE 2.1

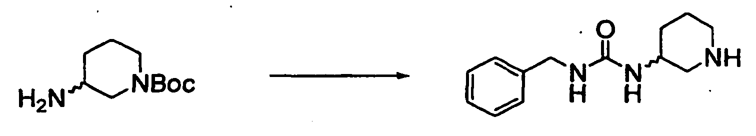

To N-BOC-3-(amino)piperidine (0.5 g) dissolved in CH₂Cl₂ (10 mL) was added benzylisocyanate (3 mmol). After stirring for 2 hrs, amine scavenger resin (1.9 mmol) was added and the mixture was stirred overnight, filtered, the resin back-washed with CH₂Cl₂ and methanol, and the organics concentrated *in vacuo.* Stirring of the crude material in 4N HCl/dioxane (40 mL) for 2.5 hrs before concentrating *in vacuo* gave the title compound (41 %, MH+=369).

### PREPARATIVE EXAMPLE 2.2 - 2.6

Following the procedures set forth in Preparative Example 2.1 but using the isocyanate (or chloroformate) indicated in the Table below, the amines were obtained and used without further purification.

| Prep Ex. | Amine | Isocyanate | Amine |
|---|---|---|---|
| 2.2 | 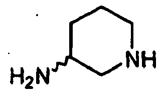 | 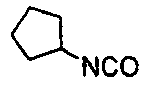 | 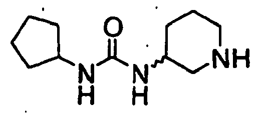 |
| 2.3 | 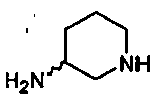 | 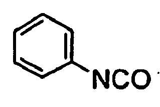 | 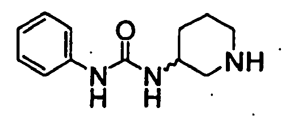 |
| 2.4 | 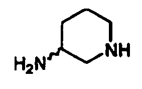 | 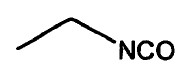 | 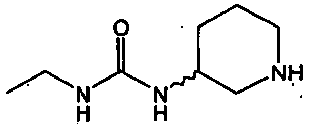 |
| 2.5 | 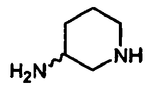 | 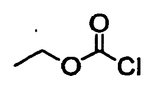 | 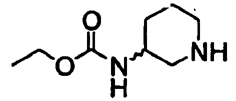 |
| 2.6 | 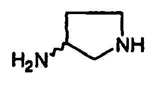 | 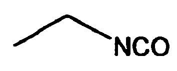 | 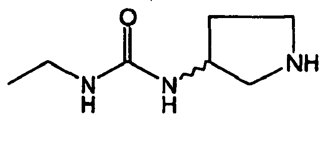 |

### PREPARATIVE EXAMPLE 2.7

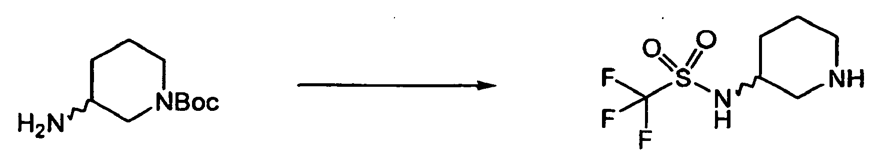

To N-BOC-3-(amino)piperidine (5 mmol) dissolved in CH₂Cl₂ (30 mL) was added trifluoromethanesulfonic anhydride (5 mmol) and the mixture was stirred overnight. The mixture was concentrated *in vacuo,* diluted with CH₂Cl₂ (10 mL) and treated with trifluoroacetic acid (10 mL). After stirring for 2 hr, the mixture was concentrated *in vacuo* to give the title compound (43%, MH+=233.1).

### PREPARATIVE EXAMPLE 2.8

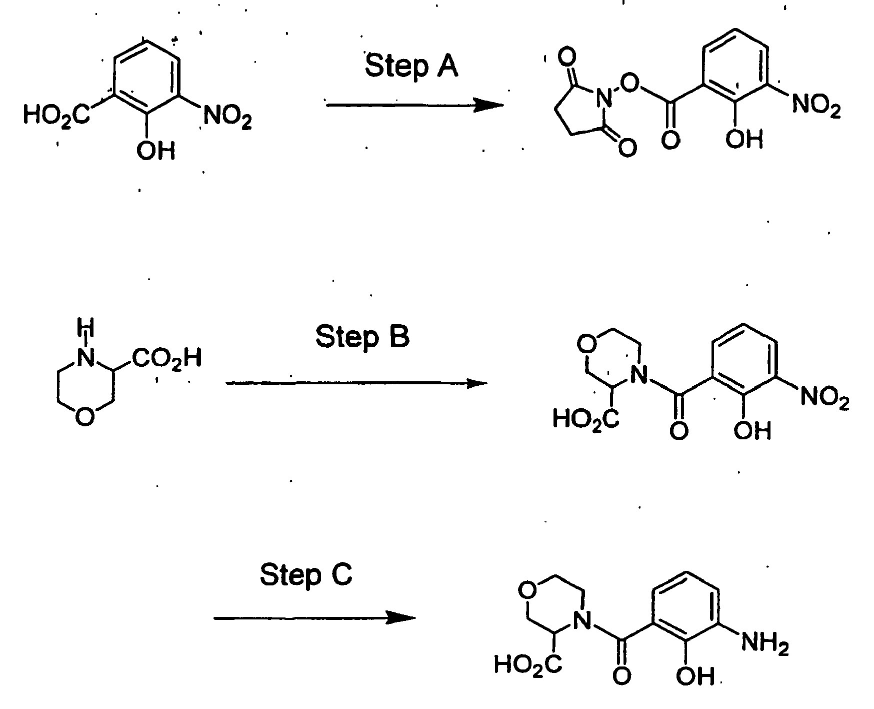

### Step A

3-Nitrosalicylic acid (5 mmol) and N-hydroxysuccinimide (5 mmol) were added to a solution of 2% DMF/CH₂Cl₂, followed by DCC (5 mmol). After stirring for 2 hr, the mixture was filtered and concentrated *in vacuo* and the residue used directly in Step B.

### Step B

The product from Step A above was suspended in DMF and to this was added morpholino-2-carboxylic acid HCl (5 mmol) in CH₂Cl₂ (10 mL)/DMF (5 mL) and diisopropylethylamine (10 mmol). The mixture was stirred overnight, filtered, basified with 1*N* NaOH (50 mL), washed with CH₂Cl₂, acidified with 5N HCl and extracted with EtOAc. The organic phase was dried over Na₂SO₄, filtered and concentrated *in vacuo* to give the desired compound which was used directly in Step C (MH+=296).

### Step C

Following a similar procedure as in Preparative Example 2 Step B, but using the product from Step B above, the title compound was obtained (23%, MH+=267).

### PREPARATIVE EXAMPLE 2.9

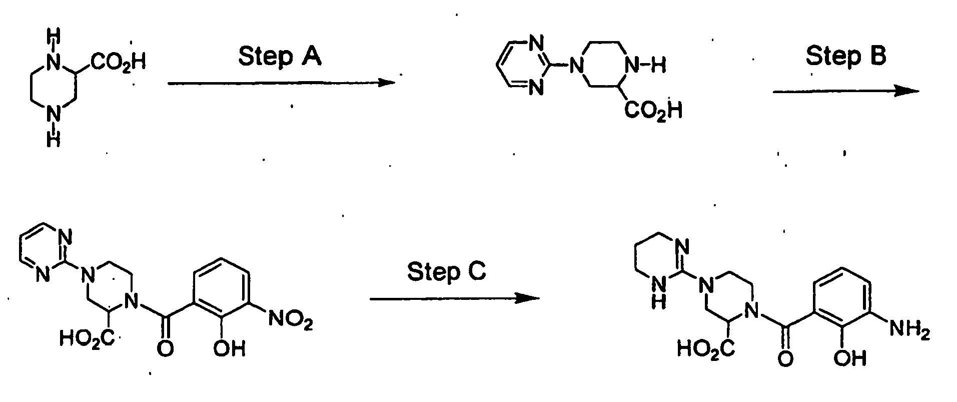

### Step A

2-Piperazinecarboxylic acid and 2-chloro-1,3-pyrimidine were stirred with triethylamine and MeOH. After stirring overnight at reflux, the mixture was filtered and concentrated *in vacuo* to give the desired compound which was used directly in Step B (MH+ = 209).

### Step B

Following a similar procedure as Preparative Example 2.8, Step B except using the product from Preparative Example 2.9 Step A above, the desired compound was obtained (41 %, MH+ = 374).

### Step C

Following a similar procedure as in Preparative Example 2, Step B, but using the product from Step B above, the desired compound was obtained (99%, MH+=344).

### PREPARATIVE EXAMPLE 2.10

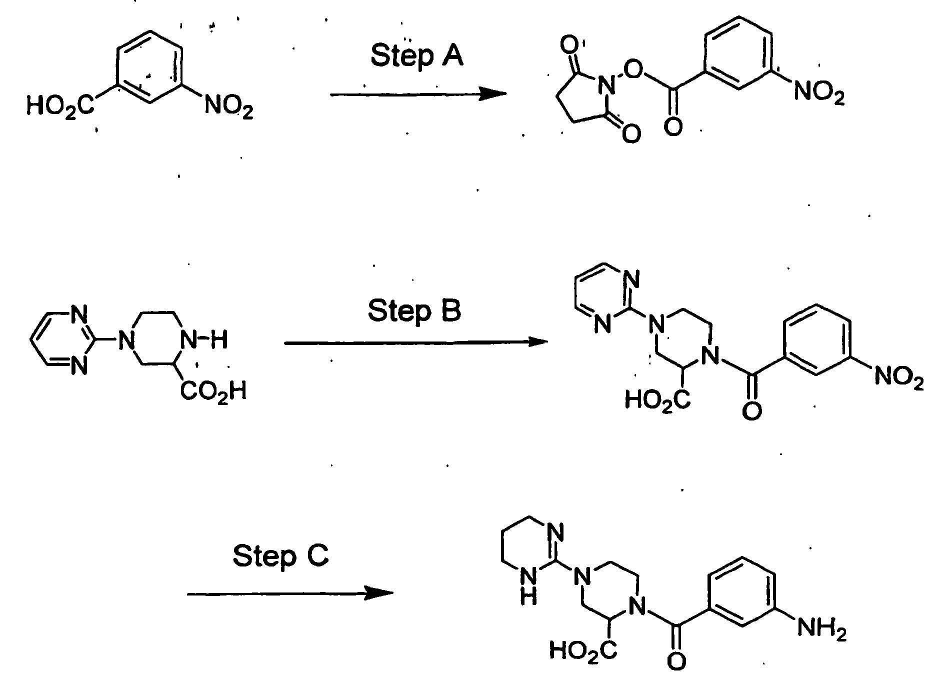

### Step A

Following a similar procedure as Preparative Example 2.8, Step A except using 3-nitrobenzoic acid, the desired compound was obtained and used directly in Step B.

### Step B

Following a similar procedure as Preparative Example 2.8, Step B except using the products from Preparative Example 2.9, Step A and Preparative Example 2.10, Step A, the desired compound was obtained (86%).

### Step C

Following a similar procedure as in Preparative Example 2, Step B, but using the product from Step B above, the desired compound was obtained (67%, MH+=331).

### PREPARATIVE EXAMPLE 2.11

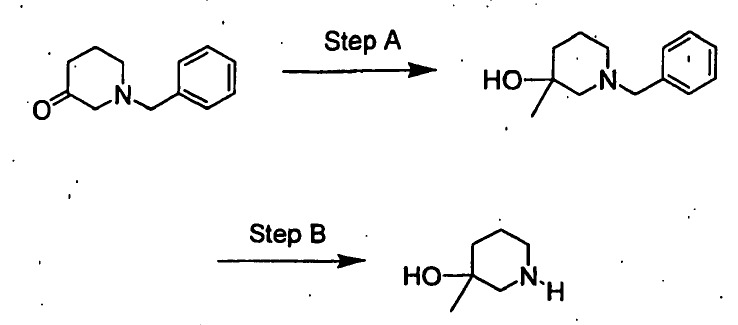

### Step A

N-Benzylpiperidone (2 g, HCl salt, hydrate) was stirred with THF (20 mL), concentrated to dryness, and placed under high vac. The residue was diluted in THF (20 mL), and methyllithium was added (2.5 eq of 1.6*N* in Et₂O) via syringe. After stirring for 3 hr, the mixture was concentrated *in vacuo,* diluted with water, extracted with CH₂Cl₂, and dried over Na₂SO₄. Filtration and concentrating *in vacuo* gave the desired product.(50%, MH+ = 205).

### Step B

Following a similar procedure as in Preparative Example 2, Step B, but using the product from Step A above, the title compound was obtained (95%, MH+=116).

### PREPARATIVE EXAMPLE 2.12

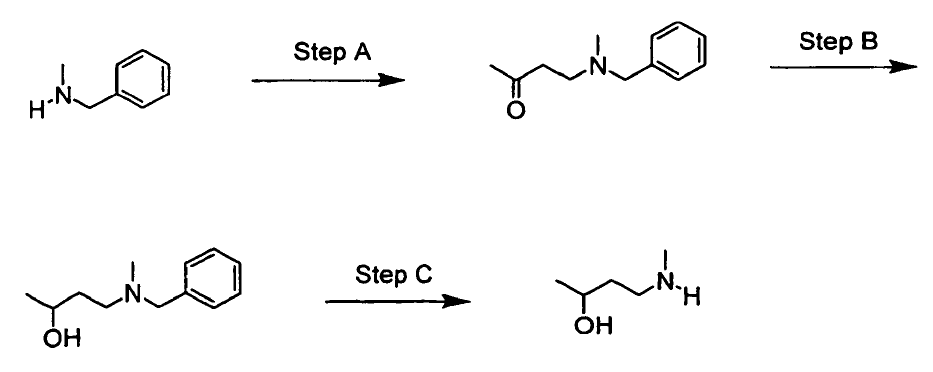

### Step A

To N-benzyl-N-methylamine (20 mmol) dissolved in acetone (50 mL) was added concentrated HCl (20 mmol), paraformaldehyde (30 mmol) and 2-propanol (2 mL). After stirring at reflux overnight, the mixture was concentrated *in vacuo,* diluted with water, basified to pH 14 and extracted with ether. The organic phase was dried over Na₂SO₄, filtered and concentrated *in vacuo* to give the desired product (98%) which was used directly in.Step B.

### Step B

The product from Step A above (500 mg) was dissolved in MeOH (20 mL) and to this was added NaBH₄ (50 mg). After stirring for 10 min, the solution was concentrated *in vacuo* to give the desired compound which was used directly in Step C without purification.

### Step C

The product from Step B above was diluted with MeOH (20 mL) and to this was added AcOH (0.1 mL), a catalytic amount of Pd/C (10%) and the mixture stirred under H₂ atmosphere (balloon) overnight. The mixture was filtered, 4N HCl in dioxane (1 mL) was added, and the mixture was concentrated *in vacuo* to give the desired compound that was used directly without purification.

### PREPARATIVE EXAMPLE 2.13

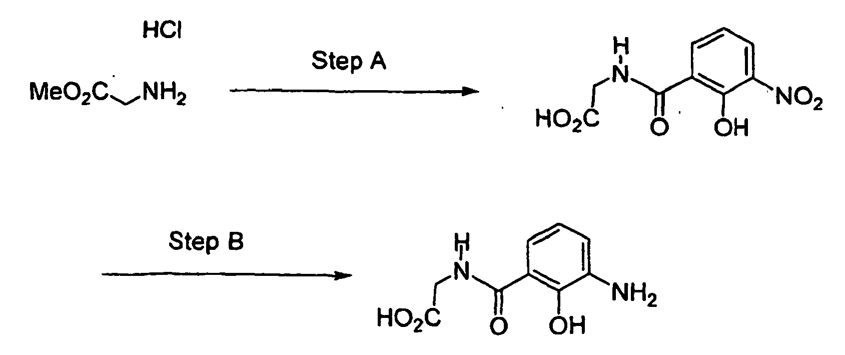

### Step A

Following a similar procedure as Preparative Example 2, Step A except using methyl glycinate, the desired ester was obtained. The mixture was poured into 200 mL of 1*N* NaOH, then extracted with dichloromethane. The pH was adjusted to 1 and NaCl was added until saturation. After several hours, the resulting precipitate was filtered and washed with cold water to give the desired product (42%).

### Step B

Following a similar procedure as in Preparative Example 2 Step B, but using the product from Step A above, the title compound was obtained (95%).

### PREPARATIVE EXAMPLE 2.14

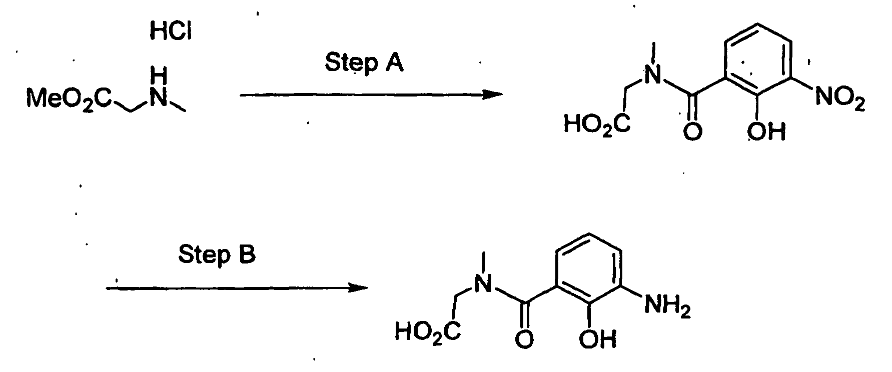

### Step A

Following a similar procedure as in Preparative Example 2.13, Step A except using methyl N-methylglycinate, the desired product was obtained (18%).

### Step B

Following a similar procedure as in Preparative Example 2, Step B, but using the product from Step A above, the title compound was obtained (95%, MH+ = 225).

### PREPARATIVE EXAMPLE 2.15

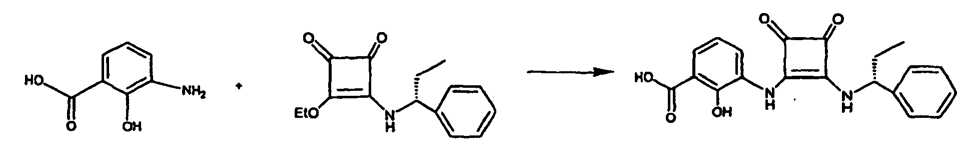

The cyclobutenedione intermediate from Preparative Example 87 (200mg), DIEA (1.00ul), 3-aminosalicylic acid (120mg) and EtOH (4ml) were combined and heated to reflux overnight to give the title compound (90%, MH+=367).

### PREPARATIVE EXAMPLE 2.16

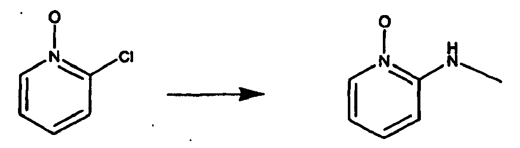

The above n-oxide (2g) was combined with H₂NMe/H₂O (15cm³) and heated to 140°C overnight. Potassium carbonate (1.3g) added and the mixture concentrated *in vacuo.* Extraction with EtOH and concentration of the filtrate *in vacuo* gave 1.56g of crude amine (MH+=125).

### PREPARATIVE EXAMPLE 3-10.50

Following the procedures set forth in Preparative Examples 1-2 but using the carboxylic acid, amine, and coupling agent [DCC (Prep. Ex. 1) or PyBrop (Prep. Ex. 2)] listed in the Table below, the indicated amide products were obtained and used without further purification.

| Prep Ex. | Carboxylic acid | Amine | Product | 1. Coupling Agent |
|---|---|---|---|---|
| | | | | 2. % Yield |
| | | | | 3. MH⁺ |
| 3 | 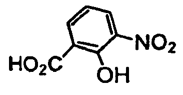 | 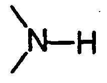 | 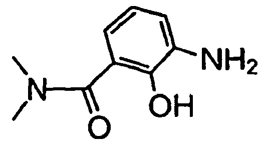 | 1. PyBrop |
| | | | | 2. 87%, 86% |
| | | | | 3. 181 |
| 4 | 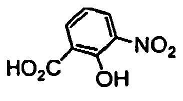 | 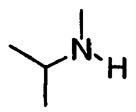 | 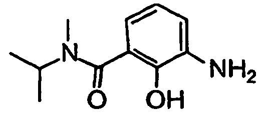 | 1. PyBroP |
| | | | | 2. 49% |
| | | | | 3. 209 |
| 5 | 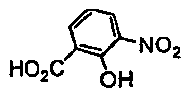 | NH₃ | 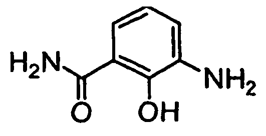 | 1. PyBroP |
| | | | | 2. 95% |
| | | | | 3. 153 |
| 6 | 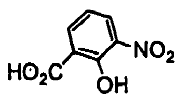 | -NH₂ | 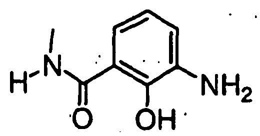 | 1. PyBroP |
| | | | | 2. 83% |
| | | | | 3. 167 |
| 7 | 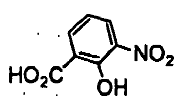 | 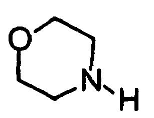 | 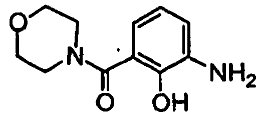 | 1. PyBroP |
| | | | | 2. 76% |
| | | | | 3. 223 |
| 8 | 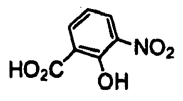 | 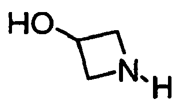 | 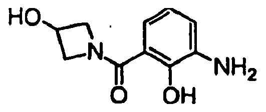 | 1. PyBroP |
| | | | | 2.65,53 |
| | | | | 3.209 |
| 9 | 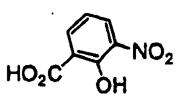 | 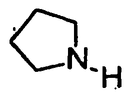 | 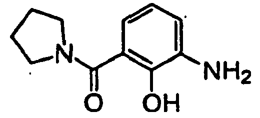 | 1. PyBroP |
| | | | | 2.59,69 |
| | | | | 3. 207 |
| 10 | 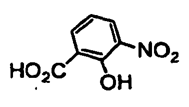 | 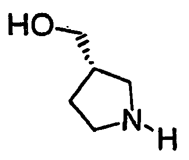 | 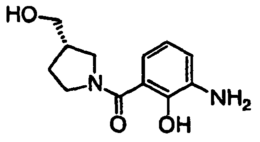 | 1. PyBroP |
| | | | | 2.49,86 |
| | | | | 3.237 |
| 10.1 | 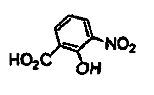 | 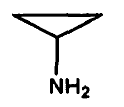 | 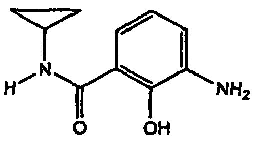 | 1. PyBroP |
| | | | | 2. 30,88 |
| | | | | 3. 193 |
| 10.2 | 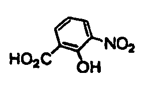 | 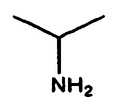 | 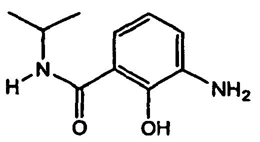 | 1. PyBroP |
| | | | | 2. 26,87 |
| | | | | 3. 195 |
| 10.3 | 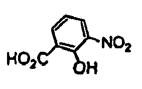 | 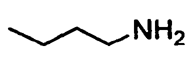 | 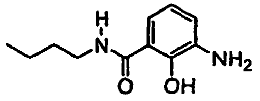 | 1. PyBroP |
| | | | | 2. 38 |
| | | | | 3. 209 |
| 10.4 | 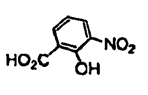 | 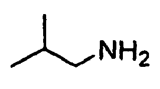 | 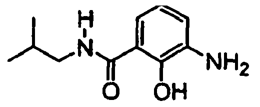 | 1. PyBroP |
| | | | | 2. 29 |
| | | | | 3. 209 |
| 10.5 | 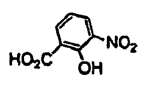 | 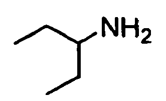 | 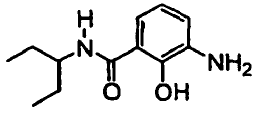 | 1. PyBroP |
| | | | | 2. 38 |
| | | | | 3. 223 |
| 10.6 | 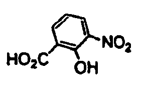 | 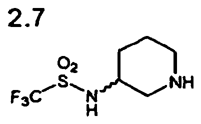 | 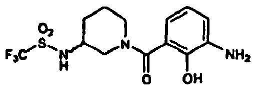 | 1. PyBroP |
| | | | | 2. 32,99 |
| | | | | 3. 367.9 |
| 10.7 | 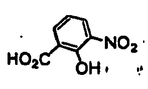 | 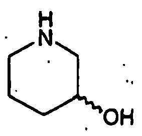 | 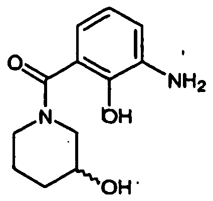 | 1. PyBroP |
| | | | | 2. 35,99 |
| | | | | 3. 237 |
| 10.8 | 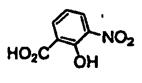 | 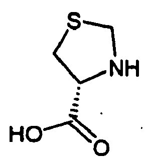 | 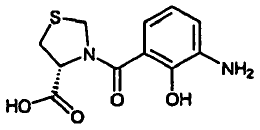 | 1. DCC |
| | | | | 2. 30,99 |
| | | | | 3. 269 |
| 10.9 | 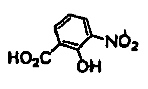 | 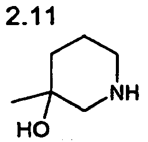 | 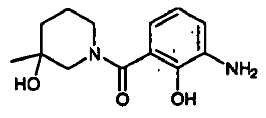 | 1. PyBroP |
| | | | | 2. 58,95 |
| | | | | 3. 233.1 |
| 10.10 | 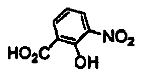 | 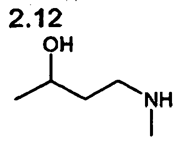 | 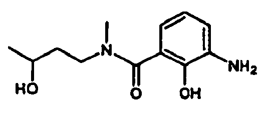 | 1. PyBroP |
| | | | | 2. 42,95 |
| | | | | 3. 238.9 |
| 10.13 | 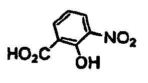 | 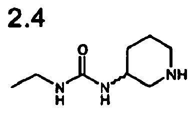 | 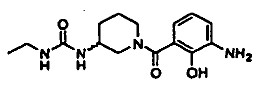 | 1. PyBroP |
| | | | | 2. 51,95 |
| | | | | 3. 307 |
| 10.14 | 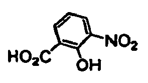 | 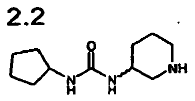 | 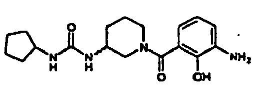 | 1. PyBroP |
| | | | | 2. 55 |
| | | | | 3. 347 |
| 10.15 | 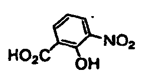 | 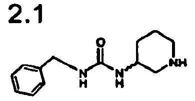 | 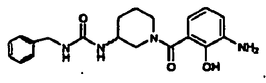 | 1. PyBroP |
| | | | | 2. 41 |
| | | | | 3. 369.1 |
| 10.16 | 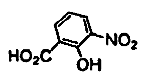 | 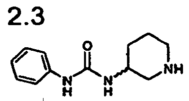 | 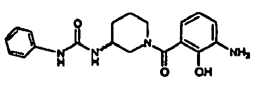 | 1. PyBroP |
| | | | | 2. 56 |
| | | | | 3. 354.9 |
| 10.17 | 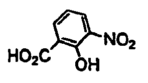 | 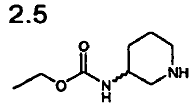 | 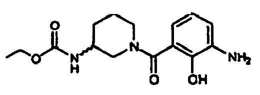 | 1. PyBroP |
| | | | | 2. 56 |
| | | | | 3. 308 |
| 10.18 | 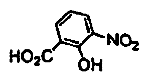 | 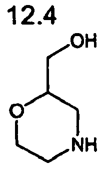 | 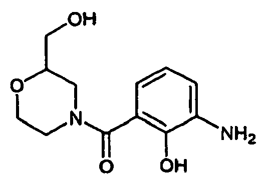 | 1. PyBroP |
| | | | | 2. 10,95 |
| | | | | 3. 252.9 |
| 10.19 | 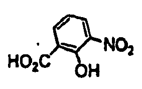 | 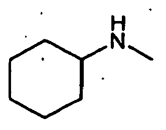 | 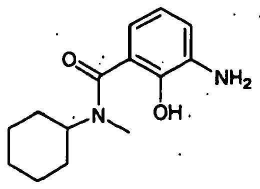 | 1. PyBroP |
| | | | | 2. 42,95 |
| | | | | 3. 249 |
| 10.20 | 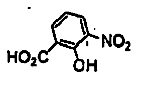 | 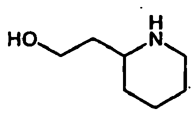 | 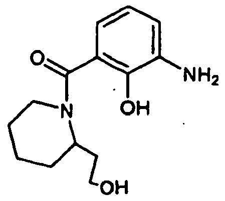 | 1. PyBroP |
| | | | | 2. 15,95 |
| | | | | 3. 264.9 |
| 10.21 | 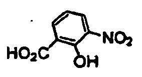 | 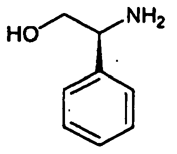 | 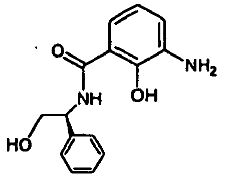 | 1. PyBroP |
| | | | | 2. 64,95 |
| | | | | 3. 273 |
| 10.22 | 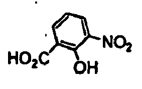 | 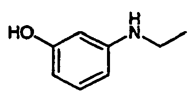 | 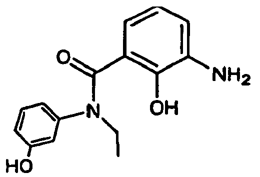 | 1. PyBroP |
| | | | | 2. 45,95 |
| | | | | 3. 273 |
| 10.23 | 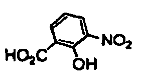 | 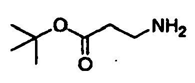 | 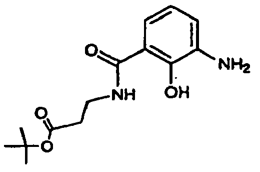 | 1. PyBroP |
| | | | | 2. 44,95 |
| | | | | 3. 281 |
| 10.24 | 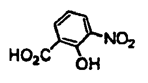 | 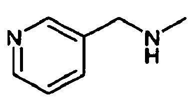 | 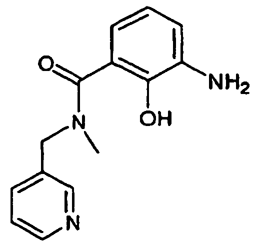 | 1. PyBroP |
| | | | | 2. 41,95 |
| | | | | 3. 281.1 |
| 10.25 | 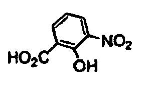 | 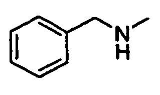 | 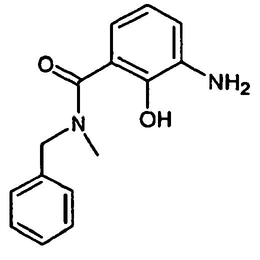 | 1. PyBroP |
| | | | | 2. 48,95 |
| | | | | 3. 257 |
| 10.26 | 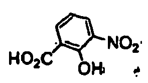 | 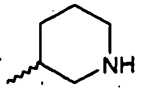 | 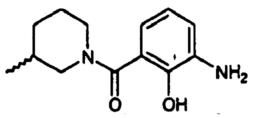 | 1. DCC |
| | | | | 2. 15,99 |
| | | | | 3. 235 |
| 10.28 | 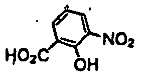 | 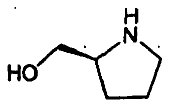 | 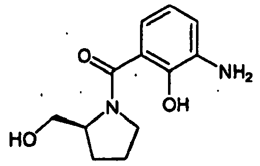 | 1. PyBroP |
| | | | | 2. 52,95 |
| | | | | 3. 237.1 |
| 10.29 | 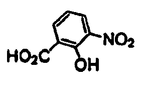 | 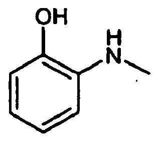 | 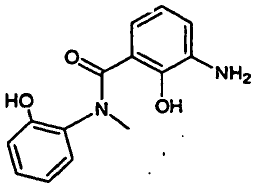 | 1. PyBroP |
| | | | | 2. 31,95 |
| | | | | 3. 259.1 |
| 10.30 | 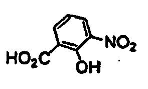 | 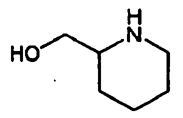 | 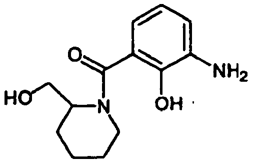 | 1. PyBroP |
| | | | | 2. 54,95 |
| | | | | 3. 250.9 |
| 10.31 | 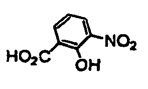 | 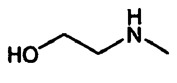 | 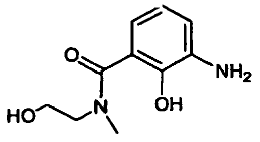 | 1. PyBroP |
| | | | | 2. 64,95 |
| | | | | 3. 210.9 |
| 10.32 | 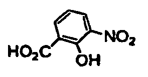 | 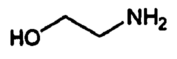 | 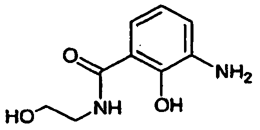 | 1. PyBroP |
| | | | | 2. 47,95 |
| | | | | 3. 197 |
| 10.33 | 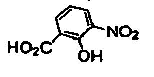 | 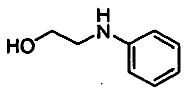 | 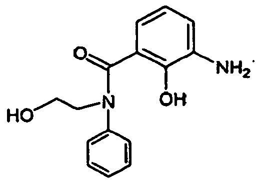 | 1. PyBroP |
| | | | | 2. 47,95 |
| | | | | 3. 273 |
| 10.34 | 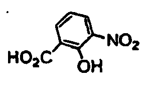 | 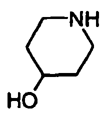 | 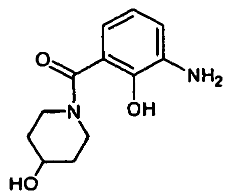 | 1. PyBroP |
| | | | | 2. 51,95 |
| | | | | 3. 237.1 |
| 10.35 | 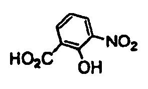 | 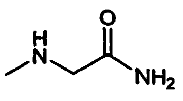 | 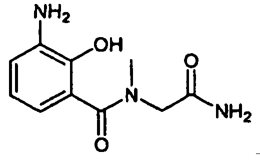 | 1. PyBroP |
| | | | | 2. 60,90 |
| | | | | 3. 224 |
| 10.36 | 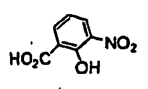 | 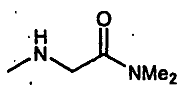 | 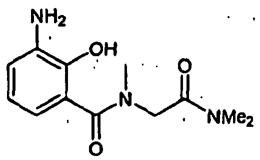 | 1. PyBroP |
| | | | | 2. 65,99 |
| | | | | 3. 252 |
| 10.37 | 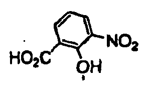 | 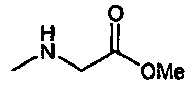 | 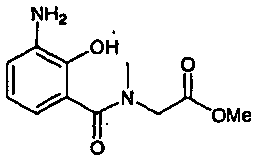 | 1. PyBroP |
| | | | | 2. 58,99 |
| | | | | 3. 239 |
| 10.38 | 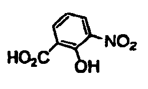 | 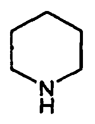 | 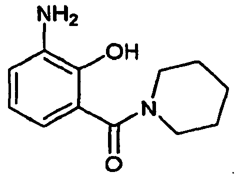 | 1. PyBroP |
| | | | | 2. 35,99 |
| | | | | 3. 221.1 |
| 10.39 | 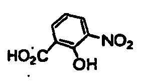 | 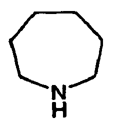 | 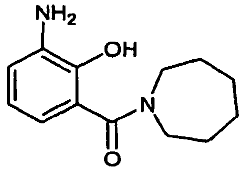 | 1. PyBroP |
| | | | | 2. 42,99 |
| | | | | 3. 235.2 |
| 10.40 | 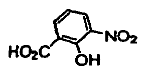 | 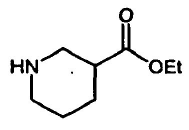 | 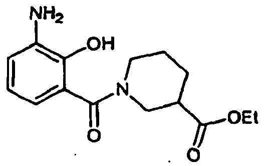 | 1. DCC |
| | | | | 2. 32,99 |
| | | | | 3. 293.1 |
| 10.41 | 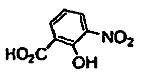 | 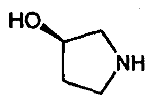 | 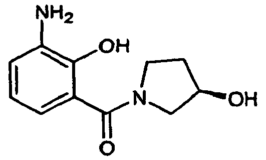 | 1. PyBroP |
| | | | | 2. 45,99 |
| | | | | 3. 223.1 |
| 10.42 | 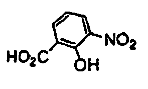 | 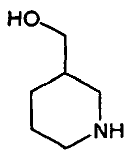 | 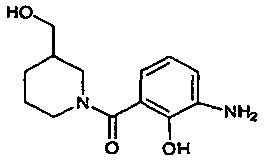 | 1. PyBroP |
| | | | | 2. 55,81 |
| | | | | 3. 251.1 |
| 10.43 | 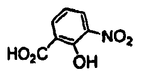 | 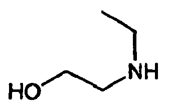 | 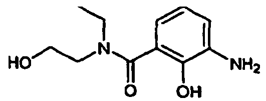 | 1. PyBroP |
| | | | | 2. 68,66 |
| | | | | 3. 224.9 |
| 10.44 | 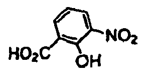 | 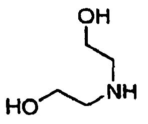 | 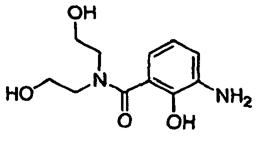 | 1. PyBroP |
| | | | | 2. 68,66 |
| | | | | 3. 241.1 |
| 10.45 | 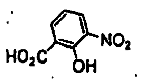 | 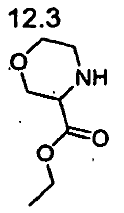 | 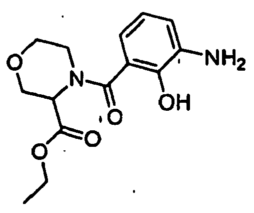 | 1. PyBroP |
| | | | | 2. 44,40 |
| | | | | 3. 295 |
| 10.46 | 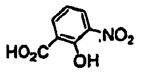 | 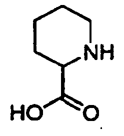 | 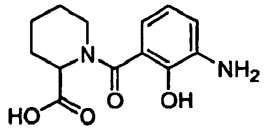 | 1. DCC |
| | | | | 2. 37,81 |
| | | | | 3. 265 |
| 10.47 | 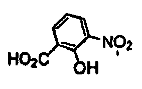 | 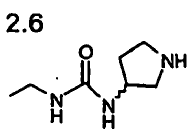 | 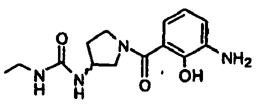 | 1. PyBroP |
| | | | | 2. 71,95 |
| | | | | 3. 293.1 |
| 10.48 | 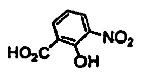 | 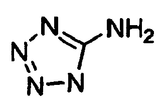 | 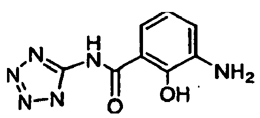 | 1. PyBroP |
| | | | | 2: 35,99 |
| | | | | 3. 220.9 |
| 10.49 | 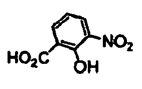 | 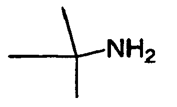 | 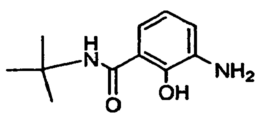 | 1. DCC |
| | | | | 2. 16,99 |
| | | | | 3. 209.0 |
| 10.50 | 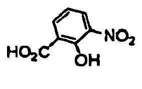 | 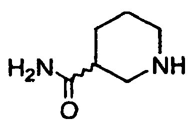 | 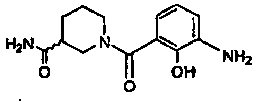 | 1. DCC |
| | | | | 2. 18,99 |
| | | | | 3. 264.0 |

### PREPARATIVE EXAMPLE 10.55

### Alternative Procedure for Preparative Example 3

### Step A

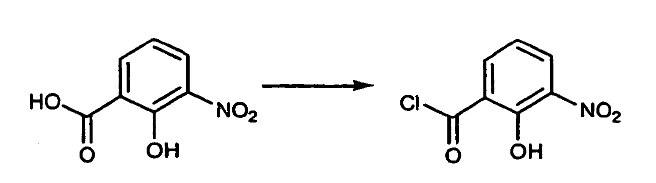

To the nitrosalicylic acid (3 g) dissolved dichloromethane (150 mL) at room temperature was added oxalyl chloride (4.3 mL) and DMF (0.01 eq.). After stirring for one day the mixture was concentrated in a vacuum to give a semi solid which was used directly in step B.

### Step B

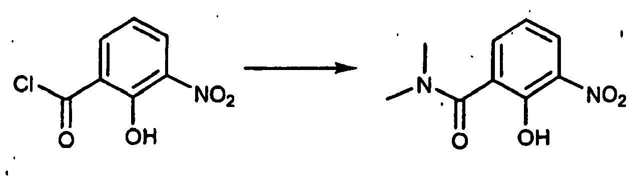

To the material from step A diluted in dichloromethane (50 mL) and cooled to 0° C was added dimethyl amine in THF (2*N* solution, 24.6 mL) and triethylamine (4 eq.). After stirring for 24 hours at room temperature the mixture was concentrated in vacuo, diluted with 1M sodium hydroxide (30 mL) and after a half hour was washed with dichloromethane. The aqueous phase was acidified with 6M HCl (aq), extracted with dichloromethane and the organic phase was washed with water, dried over Na₂SO₄ and concentrated to give the title compound (3.2 g, 93%).

### Step C

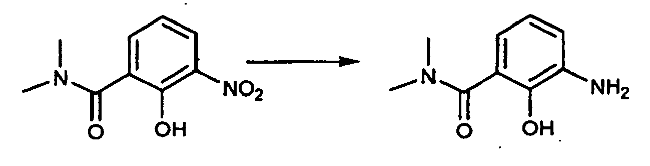

A mixture of the product from step B above (6 g), 10% Pd/C (0.6 g), and EtOH (80 mL) was stirred in a parr shaker under hydrogen (40 psi) at room temperature for 2 days. Filtration through celite and concentration *in vacuo* afforded the title product (5.1 g, 99%, MH⁺ = 181).

### PREPARATIVE EXAMPLE 11

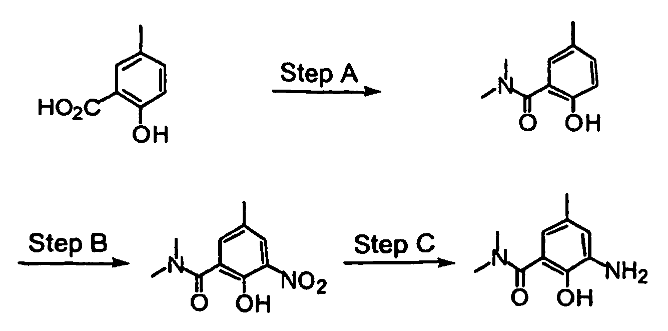

### Step A

Following a similar procedure as in Preparative Example 1 except using dimethylamine (2*M* in THF, 33 mL) and 5-methylsalicylic acid (5 g), the desired product was prepared (6.5 g).

### Step B

Nitric acid (0.8 mL) in H₂SO₄ was added to a cooled (-20°C) suspension of the product from Step A above (3 g) in H₂SO₄ (25 mL). The mixture was treated with 50% NaOH (aq) dropwise, extracted with CH₂Cl₂, dried over anhydrous MgSO₄, filtered and concentrated *in vacuo* to give the product as a crude solid (2.1 g, 44%, MH⁺ = 225).

### Step C

The product was prepared in the same manner as described in Step B of Preparative Example 2 (0.7 g, 99%, MH⁺ = 195).

### PREPARATIVE EXAMPLE 11.1

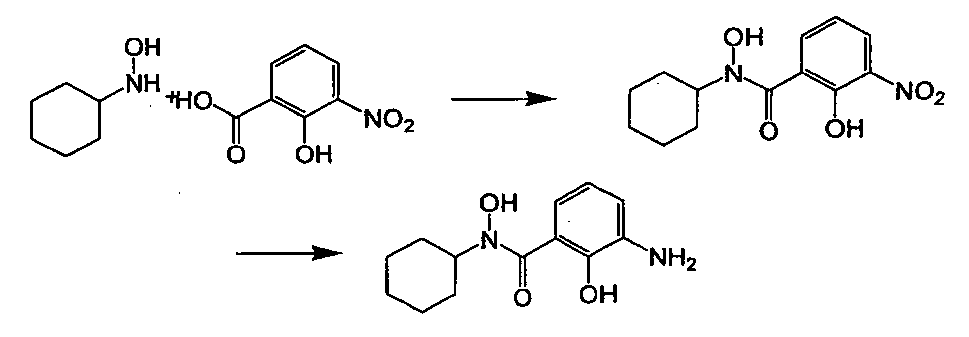

### Step A

The above amine was reacted with the acid using the procedure set forth in Preparative Example 2, Step A to yield the desired amide (54%).

### Step B

Na₂S₂O₄ (1.22g) was dissolved in water (4ml) followed by the addition of NH₃/H₂O (300ul). The solution ws then added to the product from Step A (200 mg) in dioxane (4ml) and stirred for 30min. The crude material was purified via flash column chromatography (CH₂Cl₂/MeOH, 20:1) to give 100mg of product (56%, MH+=251).

### PREPARATIVE EXAMPLE 11.2

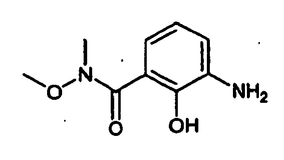

Following the procedures set forth in Preparative Example 11.1, Steps A and B, but using N-methylmethoxylamine, the title compound was obtained (86%, MH+=181).

### PREPARATIVE EXAMPLE 11.10

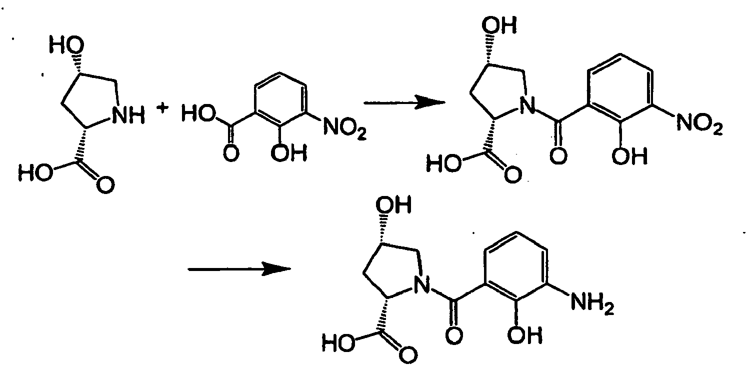

### Step A

Following the procedure set forth in Preparative Example 1, but using N-hydroxysuccinimide and 2% DMF in CH₂Cl₂, the desired amide was obtained (33%, MH+=297).

### Step B

Following the procedure set forth in Preparative Example 2, Step B, the amine was prepared (99%, MH+=267).

### PREPARATIVE EXAMPLE 11.11 - 11.18

Following the procedures set forth in Preparative Examples 11.11 but using the carboxylic acid, amine, and coupling agent DCC indicated, the indicated amide products were obtained and used without further purification.

| Prep Ex. | Carboxylic acid | Amine | Product | 1. % Yield |
|---|---|---|---|---|
| | | | | 2. MH⁺ |
| 11.11 | 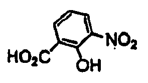 | 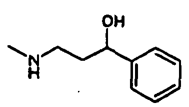 | 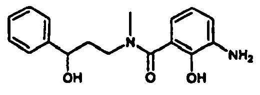 | 1. 45,92 |
| | | | | 2. 310.0 |
| 11.12 | 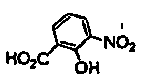 | 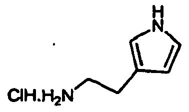 | 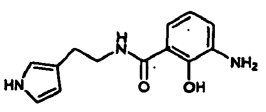 | 1. 45,95 |
| | | | | 2. 247.2 |
| 11.13 | 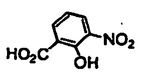 | 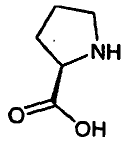 | 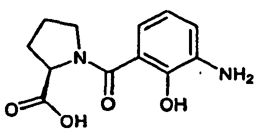 | 1. 85,85 |
| | | | | 2. 251.1 |
| 11.14 | 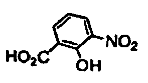 | 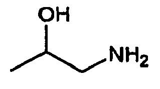 | 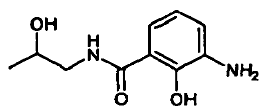 | 1. 99,92 |
| | | | | 2. 211.1 |
| 11.15 | 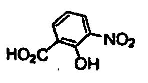 | 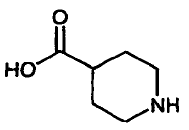 | 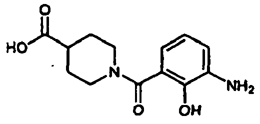 | 1. 48,84 |
| | | | | 2. 265 |
| 11.16 | 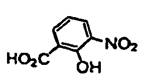 | 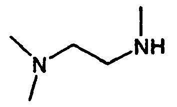 | 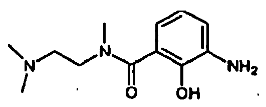 | 1. 78,91 |
| | | | | 2. 238.1 |
| 11.17 | 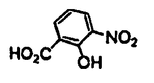 | 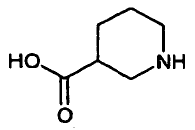 | 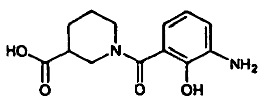 | 1. 67,90 |
| | | | | 2. 265.1 |
| 11.18 | 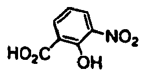 | 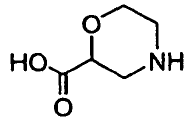 | 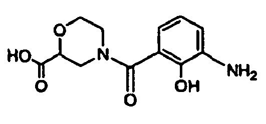 | 1. 28,99 |
| | | | | 2. 267 |

### PREPARATIVE EXAMPLE 12

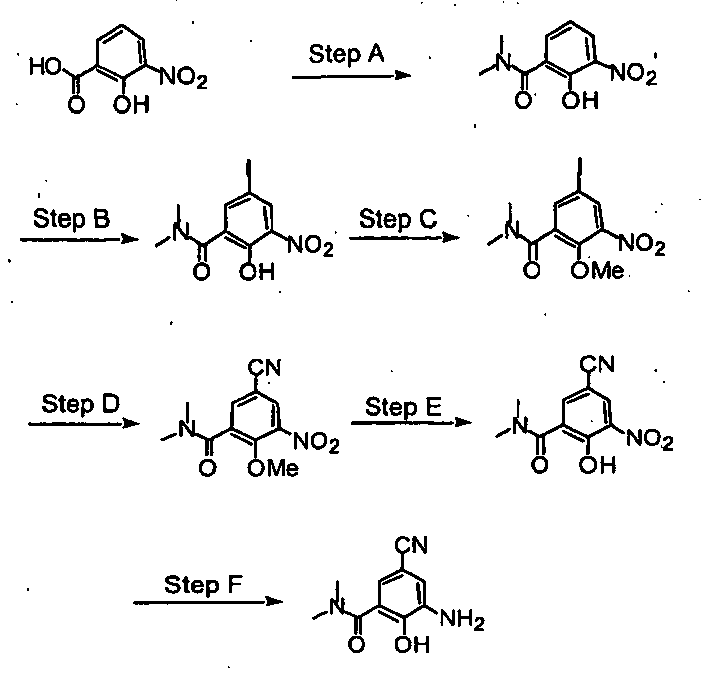

### Step A

Following a similar procedure as described in Preparative Example 2 Step A except using dimethylamine in place of R-(+)-3-pyrrolidinol, the desired product was prepared.

### Step B

The product from step A above (8 g) was combined with iodine (9.7 g), silver sulfate (11.9 g), EtOH (200 mL) and water (20 mL) and stirred overnight. Filtration, concentration of the filtrate, re-dissolution in CH₂Cl₂ and washing with 1*M* HCl (aq) gave an organic solution which was dried over anhydrous MgSO₄, filtered and concentrated *in vacuo* to afford the product (7.3 g, 57%, MH⁺ = 337).

### Step C

The product from Step B above (3.1 g) was combined with DMF(50 mL) and Mel (0.6 mL). NaH (60% in mineral oil, 0.4 g) was added portionwise and the mixture was stirred overnight. Concentration *in vacuo* afforded a residue which was diluted with CH₂Cl₂, washed with 1*M* NaOH (aq), dried over anhydrous MgSO₄, filtered and concentrated *in vacuo.* Purification through a silica gel column (EtOAc/Hex, 1:1) gave the desired compound (1.3 g, 41 %, MH⁺ = 351).

### Step D

The product from Step D above (200 mg), Zn(CN)₂ (132 mg), Pd(PPh₃)₄ (130 mg) and DMF (5 mL) were heated at 80°C for 48 hrs, then cooled to room temperature and diluted with EtOAc and 2M NH₄OH. After shaking well, the organic extract was dried over anhydrous MgSO₄, filtered, concentrated *in vacuo* and purified by preparative plate chromatography (Silica, EtOAc/Hex, 1:1) to give the desired compound (62 mg, 44%, MH⁺ = 250).

### Step E

BBr₃ (1.3 mL, 1*M* in CH₂Cl₂) was added to a CH₂Cl₂ solution (5 mL) of the product from step D above (160 mg) and stirred for 30 min. The mixture was diluted with water, extracted with CH₂Cl₂, dried over anhydrous MgSO₄, filtered, and concentrated *in vacuo* to give the desired compound (158 mg, MH⁺ = 236).

### Step F

A mixture of the product from step E above (160 mg), platinum oxide (83%, 19 mg), and EtOH (20 mL) was stirred under hydrogen (25-40 psi) for 1.5 hr. Filtration through celite and concentration *in vacuo* afforded the product (165 mg, MH⁺ = 206).

### PREPARATIVE EXAMPLE 12.1

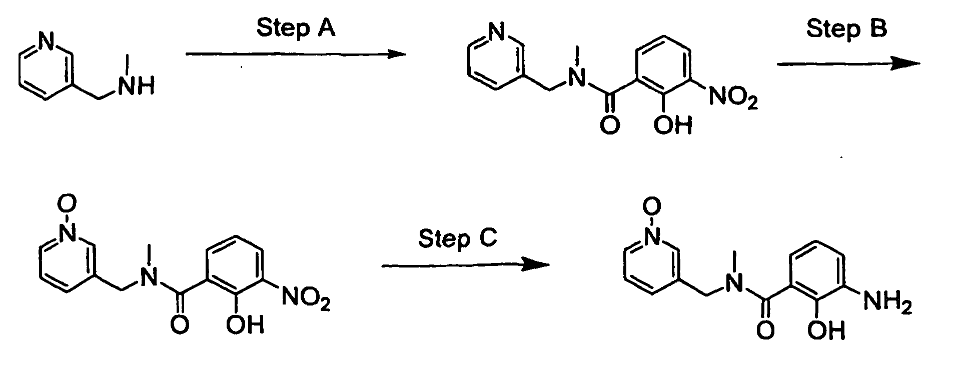

### Step A

Following a similar procedure as in Preparative Example 2. Step A except using 3-(methylaminomethyl)pyridine and 3-nitrosalicylic acid, the desired compound was prepared (41%).

### Steg B

The compound from Step A above. (0.3 g) was diluted with chloroform (15 mL) and stirred with *m*CPBA (0.4 g) for 2 hr. Purification by column chromatography (silica, 10% MeOH/CH₂Cl₂), gave the pyridyl N-oxide (0.32 g, 100%, MH⁺ = 303.9).

### Step C

Following a similar procedure as in Preparative Example 11.1, Step B, but using the product from Step B above, the desired compound was obtained (15%. MH+=274).

### PREPARATIVE EXAMPLE 12.2

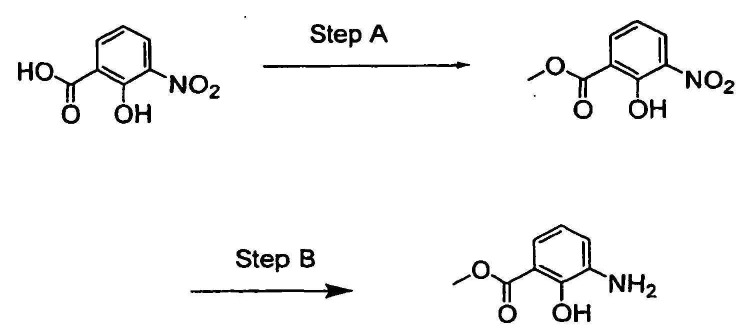

### Step A

3-Nitrosalicylic acid (4 g) in MeOH (100 mL) and concentrated H₂SO₄ (1 mL) were stirred at reflux overnight, concentrated *in vacuo,* diluted with CH₂Cl₂, and dried over Na₂SO₄. Purification by column chromatography (silica, 5% MeOH/CH₂Cl₂) gave the methyl ester (2.8 g, 65%).

### Step B

Following a similar procedure as in Preparative Example 2, Step B, but using the product from Step A above, the desired compound was obtained (95%, MH+=167.9).

### PREPARATIVE EXAMPLE 12.3

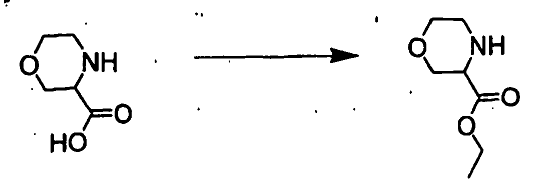

To morpholine-2-carboxilic acid (200mg) in EtOH (40mL) at 0°C was added acetyl chloride (3mL) and the mixture was stirred at reflux overnight. Concentration *in vacuo,* dilution with CH₂Cl₂ and washing with NaHCO₃ (aq) gave the title compound (99%, MH⁺ = 160.1).

### PREPARATIVE EXAMPLE 12.4

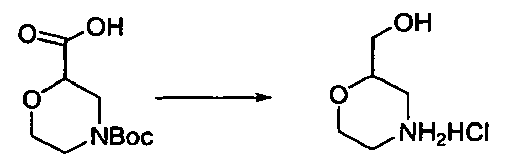

To N-Boc morpholine-2-carboxylic acid (2g) in THF (5ml) at 0°C was added a solution of borane.THF complex (1N, 10.38ml) and the mixture was stirred for 30min at 0°C, and for 2hr at room temperature. Water (200ml) was added to the reaction and the mixture extracted with CH₂Cl₂, dried with Na₂SO₄, and concentrated *in vacuo* to give 490mg of product (26%). The product was then stirred in 4N HCl/dioxane to give the amine salt.

### PREPARATIVE EXAMPLE 13

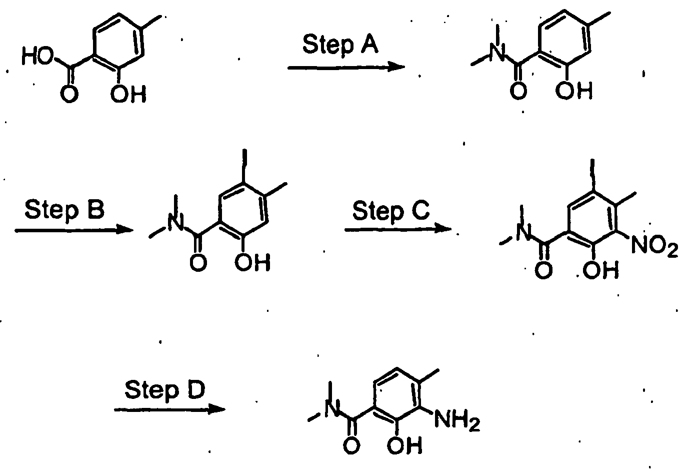

### Step A

Following a similar procedure as in Preparative Example 1 except using dimethylamine (2*M* in THF, 50 mL) and 4-methylsalicylic acid (15 g), the desired compound was prepared (6.3 g, 35%).

### Step B

The product from step A above (1.5 g) was combined with iodine (2.1 g), NaHCO₃ (1.1 g), EtOH (40 mL) and water (10 mL) and stirred overnight. Filtration, concentration of the filtrate, re-dissolution in CH₂Cl₂ and washing with 1*M* HCl (aq) gave an organic solution which was dried over anhydrous MgSO₄, filtered and concentrated *in vacuo.* Purification by flash column chromatography (silica gel, 0.5-0.7% MeOH/CH₂Cl₂) gave the product (0.5 g, 20%, MH⁺ = 306).

### Step C

Nitric acid (3.8 mL) in AcOH (10 mL) was added to the product from Step B above (0.8 g) and the mixture was stirred for 40 min. The mixture was diluted with water and extracted with CH₂Cl₂, dried over anhydrous MgSO₄, filtered and concentrated *in vacuo* to give the product as an orange solid (0.8 g, 92%, MH⁺ = 351).

### Step D

A mixture of the product from step C above (800 mg), 10% Pd/C (100mg), and EtOH/MeOH (40 mL) was stirred in a parr shaker under hydrogen (45 psi) for 1.5 hr. Filtration through celite and concentration *in vacuo* afforded the title product after purification by preparative plate chromatography (Silica, 10% MeOH/CH₂Cl₂. saturated with NH₄OH) to give the product (92 mg, 22%, MH⁺ = 195).

### PREPARATIVE EXAMPLE 13.1

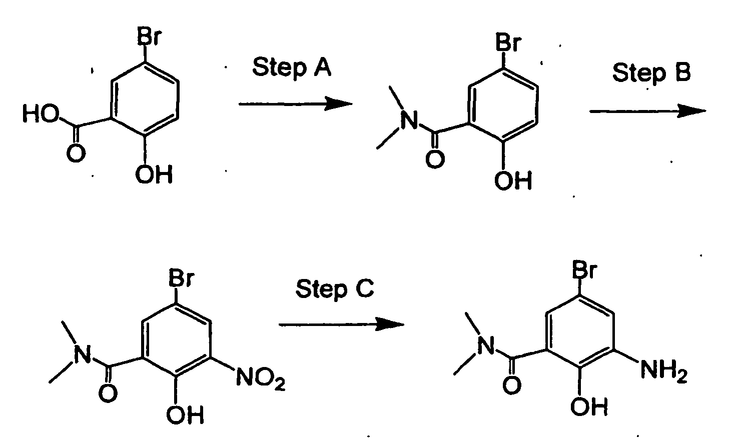

### Step A

Following a similar procedure as in Preparative Example 2, Step A except using dimethylamine (2M in THF, 23 ml) and 5-bromosalicylic acid (5g), the desired compound was prepared (4.2g, 75%, MH+=244).

### Step B

Nitric acid (10ml) in AcOH (100ml) was added to the product from Step A above (2g) and the mixture was stirred for 20 min. The mixture was diluted with water and extracted with CH₂Cl₂, dried over anhydrous MgSO₄, filtered and concentrated *in vacuo* to give the product as a yellow solid (1.9g, 80%, MH+=289).

### Step C

The product from Step B above (1.9g) was partially dissolved in EtOH(50ml). Conc HCl in EtOH (5ml in 40ml), followed by SnCl₂.2H₂O (5.74g) was added and stirred at room temperature overnight. The crude reaction was concentrated *in vacuo,* diluted with CH₂Cl₂ and washed with NaHCO₃, dried over- anhydrous MgSO₄, filtered and concentrated *in vacuo* to give the product as a solid (185mg, 9%, MH+=259).

### PREPARATIVE EXAMPLE 13.2

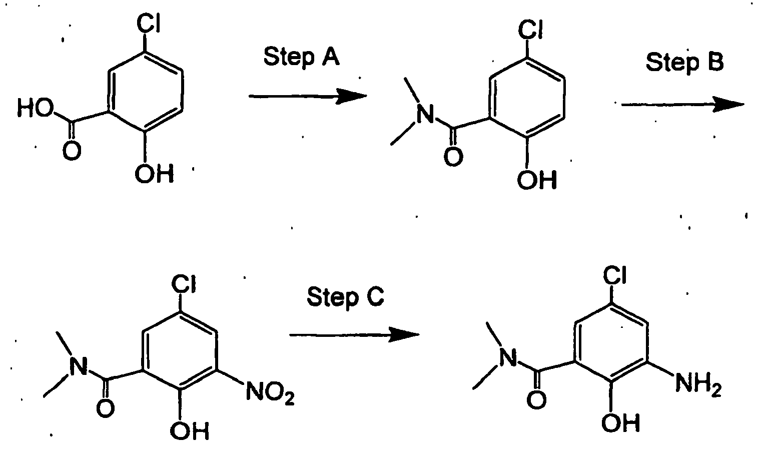

### Step A

Following a similar procedure as in Preparative Example 2, Step A, except using dimethylamine (2M in THF, 29 ml) and 5-chlorosalicylic acid (5g), the desired compound was prepared (4.5g, 78%, MH+=200).

### Step B

Nitric acid (10ml) in AcOH (100ml) was added to the product from Step A above (2g) and the mixture was stirred for 20 min. The mixture was diluted with water and extracted with CH₂Cl₂, dried over anhydrous MgSO₄, filtered and concentrated *in vacuo* to give the product as a solid (2.2g, 88%, MH+=245).

### Step C

The product from Step B above (2.2g) was partially dissolved in EtOH(50ml). Conc HCl in EtOH (5ml in 40ml), followed by SnCl₂.2H₂O (7.01g) was added and stirred at room temperature overnight. The crude reaction was concentrated *in vacuo,* diluted with CH₂Cl₂ and neutralized with NaOH. The entire emulsion was filtered though celite, the layers were separated and the organic layer was dried over anhydrous MgSO₄, filtered and concentrated *in vacuo* to give a solid (540mg, 22%, MH+=215).

### PREPARATIVE EXAMPLE 13.3

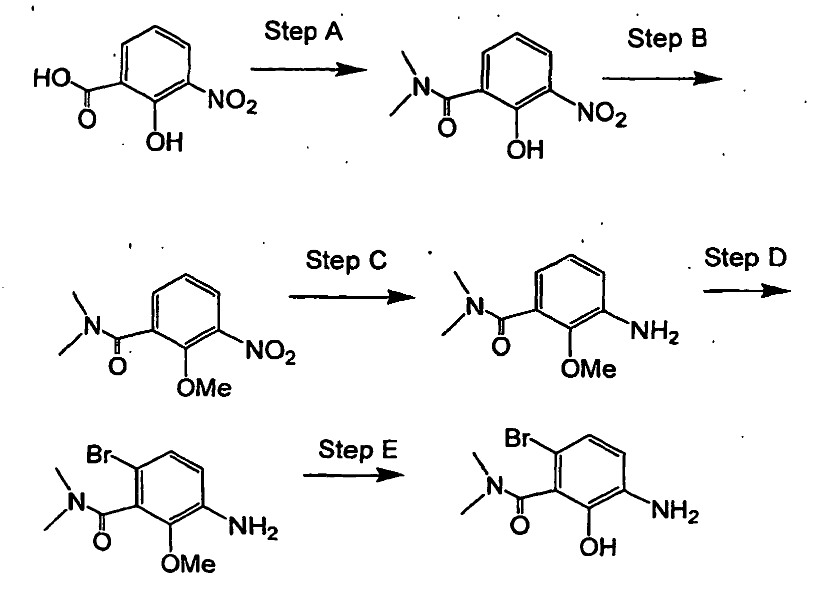

### Step A

3-Nitrosalicylic acid (10g), PyBroP (20.52g), and DIEA (28ml) in anhydrous CH₂Cl₂ (200ml) were combined and stirred at room temperature for 10 min. Dimethylamine (2M in THF, 55ml) was added and let the reaction stir over the weekend. The mixture was extracted with 1N NaOH (aq) and the organic phase was discarded. The aqueous phase was acidified with 1N HCl (aq), extracted with CH₂Cl₂, dried over anhydrous MgSO₄, filtered and concentrated *in vacuo.* The oil was taken up in ether and a solid crashed out, triterated in ether to give 4.45g of a solid (39%, MH+=211).

### Step B

The product from Step A (2.99g), K₂CO₃ (9.82g), and iodomethane (8.84ml) were combined in acetone and heated to reflux overnight. The reaction was filtered and concentrated *in vacuo.* The oil was taken up in CH₂Cl₂ and washed with 1N NaOH, dried over anhydrous MgSO₄, filtered and concentrated *in vacuo* to give 3.3g of an oil (99%, MH+=225).

### Step C

The crude product from Step B (3.3g) was stirred with 10% Pd/C (350mg) in EtOH (50ml) under a hydrogen gas atmosphere at 20psi overnight. The reaction mixture was filtered through celite and the filtrate was concentrated *in vacuo* to give 2.34 g of a solid (85%, MH+=195).

### Step D

The product from Step C (469mg) was dissolved in AcOH (6ml). 1.95M Br₂ in AcOH (1.23ml) was added dropwise to the reaction and the mixture was stirred at room temperature for 1 hour. 50% NaOH was added to the reaction at 0°C and the mixture was extracted with CH₂Cl₂, dried over anhydrous MgSO₄, filtered and concentrated *in vacuo.* The crude mixture was purified by preparative plate chromatography (Silica, 5% MeOH/ CH₂Cl₂) to give the desired product (298mg, 23%, MH+=273).

### Step E

BBr₃ (2.14ml, 1M in CH₂Cl₂) was added to a CH₂Cl₂ solution (8ml) of the product from Step D above (290mg) and stirred overnight. A solid formed and was filtered, taken up in MeOH/ CH₂Cl₂ and purified by preparative plate chromatography (Silica, 5% MeOH/ CH₂Cl₂) to give the desired product (137mg, 49%, MH+=259).

### PREPARATIVE EXAMPLE 13.4

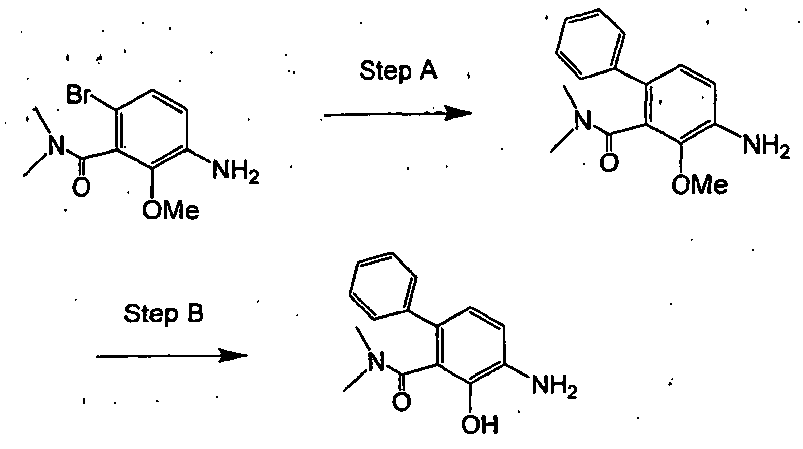

### Step A

To the product from Preparative Example 13.3 Step D (200mg) was added phenylboronic acid (98mg), PdCl₂(PPh₃)₂ (51 mg), and Na₂CO₃ (155mg) in THF/H₂O (4ml/1ml). The solution was heated at 80°C overnight. EtOAc was added to reaction and washed with 1N NaOH. The organic layer was dried over anhydrous MgSO₄, filtered and concentrated *in vacuo.* The crude mixture was purified by preparative plate chromatography (5% MeOH/ CH₂Cl₂) to give 128mg of an oil (65%, MH+=271).

### Step B

Following a similar procedure as in Preparative Example 13.3 Step E and using the product from Step A above, the desired compound was prepared (0.1 g, 69%, MH+=257.1).

### PREPARATIVE EXAMPLE 13.5-13.7

Following the procedures set forth in Preparative Example13.4 but using the boronic acid from the Preparative Example indicated in the Table below, the amine products were obtained.

| Prep Ex. | Boronic Acid | Product | 1. Yield (%) |
|---|---|---|---|
| | | | 2. MH⁺ |
| 13.5 | 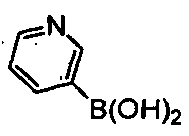 | 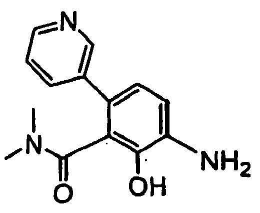 | 1. 15% |
| | | | 2. 258 |
| 13.6 | 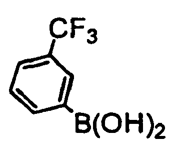 | 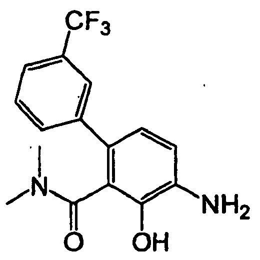 | 1. 32% |
| | | | 2. 325 |
| 13.7 | 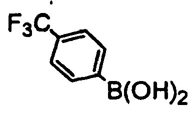 | 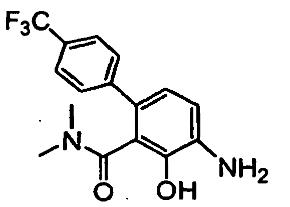 | 1. 18% |
| | | | 2. 325 |

### Preparative Example 13.8

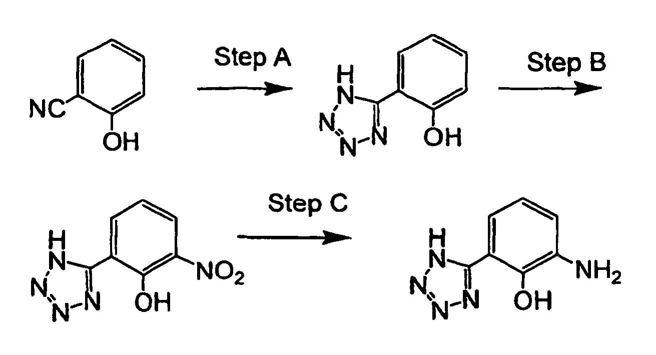

### Step A

2-Cyanophenol (500mg), sodium azide (819mg), and triethylamine hydrochloride (1.73g) were combined in anhydrous toluene and heated to 99°C overnight. After the reaction cooled down, product was extracted with H₂O. Aqueous layer was acidified with conc. HCl dropwise giving a precipitate, which was filtered to give the product (597mg, 87%, MH+=163).

### Step B

Nitric acid (0.034ml) in AcOH (5ml) was added to the product from Step A above (100mg) in AcOH and the mixture was allowed to stir for 1hr. CH₂Cl₂ and H₂O were added to reaction. The organic layer was dried over anhydrous MgSO₄, filtered and concentrated *in vacuo* to give an oil. Trituration in ether gave the product as a solid (12mg, 9%, MH+=208).

### Step C

The product from step C (56mg) was stirred with 10% Pd/C (20mg) in EtOH/MeOH (15ml) under a hydrogen gas atmosphere overnight. The reaction mixture was filtered through celite, the filtrate was concentrated *in vacuo* to give 29mg of a solid (62%, MH+=178).

### PREPARATIVE EXAMPLE 13.9

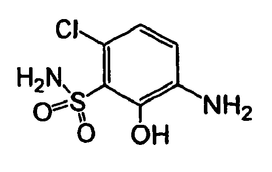

The amine was prepared following the procedure disclosed in WO Patent Application 01/68570.

### PREPARATIVE EXAMPLE 13.10

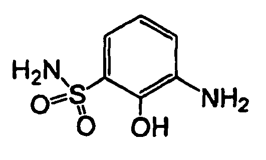

The amine was prepared following, the procedure disclosed in WO Patent Application 01/68570.

### PREPARATIVE EXAMPLE 13.11

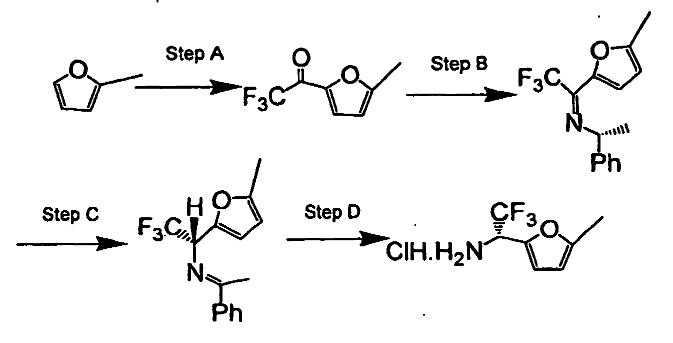

### Step A

Following the procedure described in Preparative Example 88.2, Step A, the ketone was prepared (6.4g, 36%).

### Step B

To a solution of ketone (1g) and 2-R-methylbenzylamine (0.73ml) in anhydrous toluene (20ml) was added 1N TiCl₄ in toluene (3ml) at room temperature for 1.5hrs. The precipitate was filtered and the filtrate was concentrated *in vacuo* and purified via flash column chromatography (Hex/EtOAc, 18/1) to give 800mg of product (71%).

### Step C

The imine from above (760mg) and DBU (800ul) were stirred without solvent for 4hr. The crude reaction was concentrated *in vacuo* and purified via flash column chromatography (Hex/EtOAc, 8/1) to give 600mg of product (79%).

### Step D

The imine from Step C (560mg) was dissolved in ether (8ml). 3N HCl (5ml) added and let stir at room temperature overnight. The ether layer was separated and concentrated *in vacuo* to give 400mg of the amine hydrochloride product (93%).

### PREPARATIVE EXAMPLE 13.12

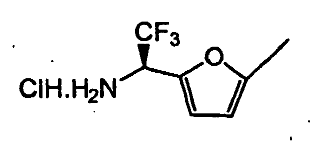

The title compound was prepared similarly as in Preparative Example 13.11, but using the 2-S-methylbenzylamine instead of 2-R-methylbenzylamine (69%).

### PREPARATIVE EXAMPLE 13.13

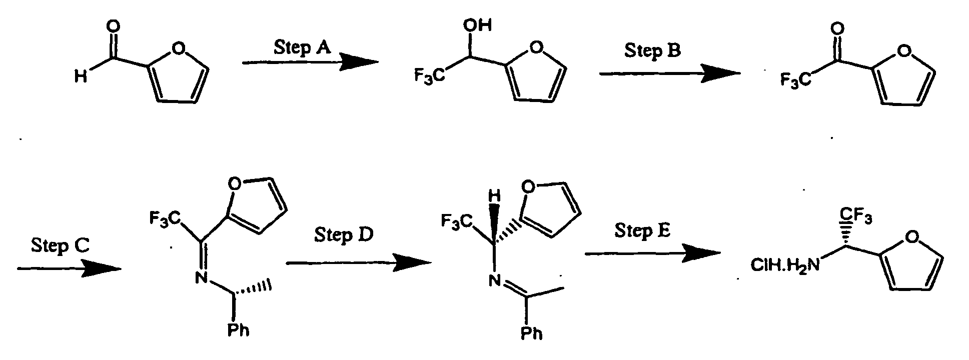

### Step A

At room temperature, CsF (60mg) was added to a mixture of furfuraldehyde (1.3ml) and TMS-CF₃ (2.5g) and stirred at room temperature (24 h) and refluxed for another 12h. 3N HCl (40ml) was added and after 4hr, the mixture was extracted with ether, washed with brine, dried over MgSO₄, and concentrated *in vacuo* to give the product (2.6g, 100%).

### Step B

To a solution of alcohol from above (2.6g) in CH₂Cl₂ at room temperature was added Dess-Martin reagent (10g) portionwise and 1 drop of water. After stirring for 3hr at room temperature, 10% Na₂S₂O₃ (60ml) was added and after stirring overnight, the solid was filtered off and the filtrate was extracted with CH₂Cl₂. The organic layer was washed with saturated sodium' bicarbonate, dried with MgSO₄, filtered and concentrated *in vacuo.* Ether/hexane (1:2; 30ml) was added to the residue, filtered, and filtrate concentrated *in vacuo* to give the product (2g, 78%).

### Steg C

Following the procedures described in Preparative Example 13.11, Steps B, C and D, the amine salt was prepared.

### PREPARATIVE EXAMPLES 13.15-13.17

Following the procedure set forth in Preparative Example 13.13, but using the prepared or commercially available aldehydes, the optically pure amine products in the Table below were obtained.

| Prep Ex. | Aldehyde | Amine | Product | Yield (%) |
|---|---|---|---|---|
| 13.15 | 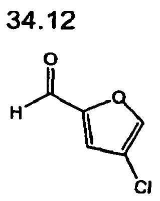 | 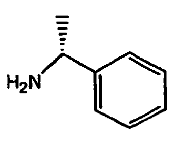 | 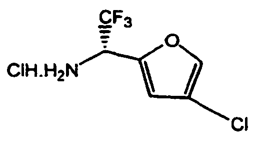 | 20% |
| 13.16 | 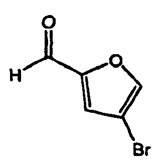 | 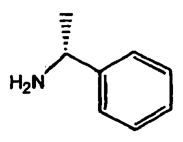 | 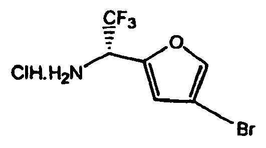 | 31% |
| 13.17 | 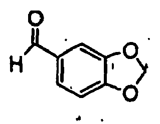 | 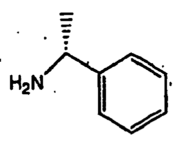 | 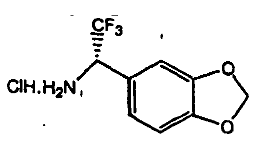 | 66% |

### PREPARATIVE EXAMPLE 13.18

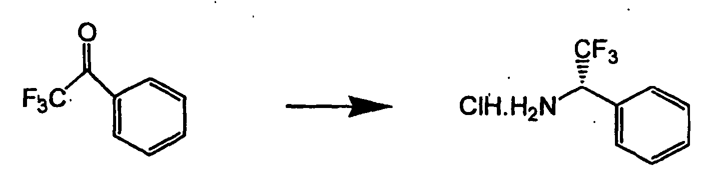

The title compound was prepared from trifluorophenylketone according to the procedures described in Preparative Example 13.11, Steps B, C, and D (68%).

### PREPARATIVE EXAMPLE 13.19

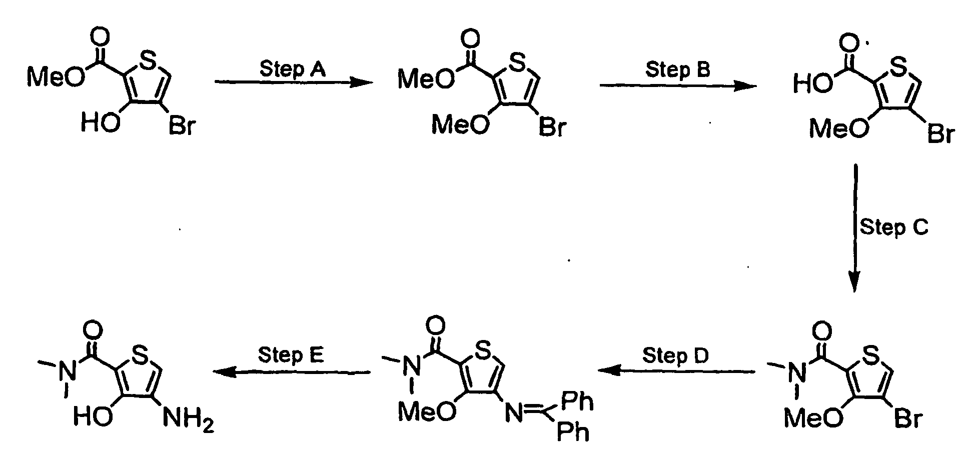

### Step A

Methyl-3-hydroxy-4-bromo-2-thiophenecarboxylate (10.0 g, 42.2 mmol) was dissolved in 250 mL of acetone. Potassium carbonate (30.0 g, 217.4 mmol) was added followed by a solution of iodomethane (14.5 mL, 233.0 mmol). The mixture was heated to reflux and continued for 6 h. After cooled to room temperature, the mixture was filtered, the solid material was rinsed with acetone (-200 mL). The filtrate and rinsing were concentrated under reduced pressure to a solid, further dried on high vacuum, yielding 13.7 g (100%) of methyl-3-methoxy-4-bromo-2-thiophenecarboxylate (MH⁺ = 251.0).

### Step B

Methyl-3-methoxy-4-bromo-2-thiophenecarboxylate (13.7 g), available from step A, was dissolved in 75 mL of THF, and added with a 1.0 M sodium hydroxide aqueous solution (65 mL, 65.0 mmol). The mixture was stirred at room temperature for 24 h. A 1.0 M hydrogen chloride aqueous solution was added dropwise to the mixture until pH was approximately 2. The acidic mixture was extracted with CH₂Cl₂ (100 mL x 2, 50 mL). The combined organic extracts were washed with brine (40 mL), dried with Na₂SO₄, and concentrated under reduced pressure to a solid, 10.0 g (100%, over two steps) of 3-methoxy-4-bromo-2-thiophenecarboxylic acid (MH⁺ = 237.0).

### Step C

To a stirred solution of 3-methoxy-4-bromo-2-thiophenecarboxylic acid (6.5 g, 27.4 mmol) in 140 mL of CH₂Cl₂, obtained from step B, was added bromotripyrrolidinophosphonium hexafluorophosphate (PyBrop, 12.8 g, 27.5 mmol), a 2.0 M solution of dimethyl amine in THF (34.5mL, 69.0 mmol), and diisopropylethyl amine (12.0 mL, 68.7 mmol). After 3 d, the mixture was diluted with 100 mL of CH₂Cl₂, and washed with a 1.0 M sodium hydroxide aqueous solution (30 mL x 3) and brine (30 mL). The organic solution was dried with Na₂SO₄, filtered, and concentrated to an oil. This crude oil product was purified by flash column chromatography, eluting with CH₂Cl₂-hexanes (1:1, v/v). Removal of solvents afforded a solid, further dried on high vacuum, yielding 6.76 g (93 %) of *N,N'*-dimethyl-3-methoxy-4-bromo-2-thiophenecarboxamide (MH⁺ = 265.0, M+2 = 266.1).

### Step D

An oven dried three-neck round bottom flask was equipped with a refluxing condenser, charged sequentially with palladium acetate (95 mg, 0.42 mmol), *(R)-*BINAP (353 mg, 0.57 mmol), cesium carbonate (9.2 g, 28.33 mmol), and *N,N'-*dimethyl-3-methoxy-4-bromo-2-thiophenecarboxamide (3.74 g, 14.2 mmol, from step C). The solid mixture was flushed with nitrogen. Toluene (95 mL) was added to the solid mixture followed by benzophenone imine (3.6 mL, 21.5 mmol). The mixture was heated to reflux and continued for 10 h. A second batch of palladium acetate (95 mg, 0.42 mmol) and (*R*)-BINAP (353 mg. 0.57 mmol) in 5 mL of toluene was added. Refluxing was continued for 14 h. The third batch of palladium acetate (30 mg, 0.13 mmol) and (*R*)-BINAP (88 mg, 0.14 mmol) was added, and reaction continued at 110°C for 24 h. The mixture was cooled to room temperature, diluted with ether (50 mL), filtered through a layer of Celite, rinsing with ether. The filtrate and rinsing were concentrated under reduced pressure to an oil, which was purified twice by flash column chromatography using CH₂Cl₂ and CH₂Cl₂-MeOH (200:1) as eluents. Removal of solvents afforded 4.1 g (79 %) of the amido-thiophene diphenylimine product as a solid (MH⁺ = 365.1).

### Step E

To a stirred solution of thiophene imine (5.09 g, 13.97 mmol), obtained from step D, in 140 mL of CH₂Cl₂ at -78°C was added dropwise a 1.0 M solution of boron tribromide in CH₂Cl₂. The mixture was stirred for 3 h while the temperature of the cooling bath was increased slowly from -78°C to -15°C. 100 mL of H₂O was added, the mixture was stirred at room temperature for 30 min, then the two layers were separated. The organic layer (as A) was extracted with H₂O (30 mL x 2). The aqueous layer and aqueous extracts were combined, washed with CH₂Cl₂ (30 mL), and adjusted to pH - 8 using a saturated NaHCO₃ aqueous solution. The neutralized aqueous solution was extracted with CH₂Cl₂ (100 mL x 3), the extracts were washed with brine, dried with Na₂SO₄, and concentrated under reduced pressure to a light yellow solid, 1.49 g of *N,N*'-dimethyl-3-hydroxy-4-amino-2-thiophenecarboxamide (first crop). The previous separated organic layer A and organic washing were combined, stirred with 30 mL of a 1.0 M HCl aqueous solution for 1 h. The two layers were separated, the aqueous layer was washed with CH₂Cl₂ (30 mL) and adjusted to pH ∼8 using a saturated NaHCO₃ aqueous solution, and the separated organic layer and organic washing were combined as organic layer B. The neutralized aqueous solution was extracted with CH₂Cl₂ (30 mL x 4), the extracts were washed with brine, dried by Na₂SO₄, and concentrated under reduced pressure to give 0.48g of a solid as the second crop of the titled product. Organic layer B from above was washed with brine, and concentrated to an oil, which was separated by preparative TLC (CH₂Cl₂-MeOH = 50:1) to afford 0.45 g of a solid as the third crop of the titled product. The overall yield of the product, *N*,*N'*-dimethyl-3-hydroxy-4-amino-2-thiophenecarboxamide, is 2.32 g (89%) (MH⁺ = 187.0).

### PREPARATIVE EXAMPLE 13.20

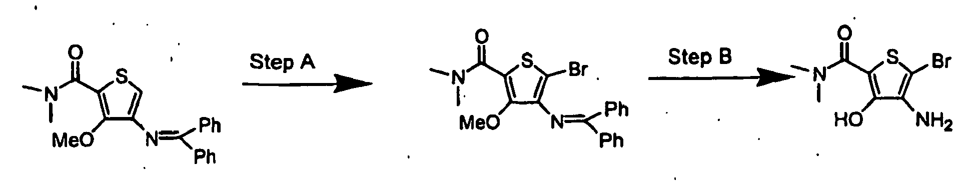

### Step A

To the product from Preparative Example 13.19 Step D (1.56g) in CH₂Cl₂ (55ml) was added potassium carbonate (1.8g) followed by dropwise addition of bromine (0.45ml). After 5hr of mixing, water (100ml) was added to the reaction and the layers were separated. The aqueous layer was extracted with CH₂Cl₂, which was then washed with brine, saturated sodium bicarbonate, and brine again. The organic layer was dried with Na₂SO₄, and concentrated *in vacuo.* The residue was purified via flash column chromatography (CH₂Cl₂) to yield 1.6g of product (83%).

### Step B

The product from above was reacted in the procedure set forth in Preparative Example 13.19 Step C to give the amine.

### PREPARATIVE EXAMPLE 13.21

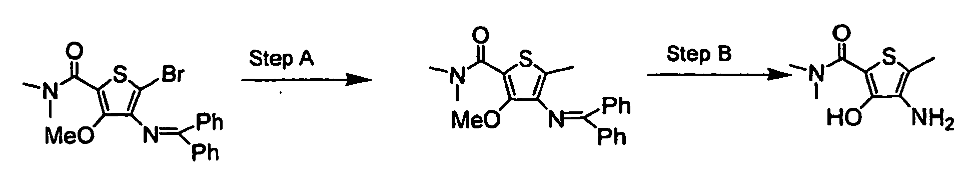

### Step A

To the product from Preparative Example 13.20, Step A (300mg) in THF (7ml) at -78 °C was added a solution of n-BuLi (1.6M in hexanes, 0.54ml). After 1 hr, iodomethane (0.42ml) was added dropwise. After 3 hrs of stirring at -78 °C, the reaction was warmed to room temperature overnight. Saturated ammonium chloride and water were added to the reaction and extracted with CH₂Cl₂. The organic layer was washed with saturated sodium bicarbonate and brine, dried over Na₂SO₄, and concentrated *in vacuo.* The crude product was purified by preparative plate chromatography (CH₂Cl₂-MeOH = 70:1 to 50:1) to afford the product (111mg, 43%).

### Step B

The product from above was reacted in the procedure set forth in Preparative Example 13.19, Step E to give the amine.

### PREPARATIVE EXAMPLE 13.22

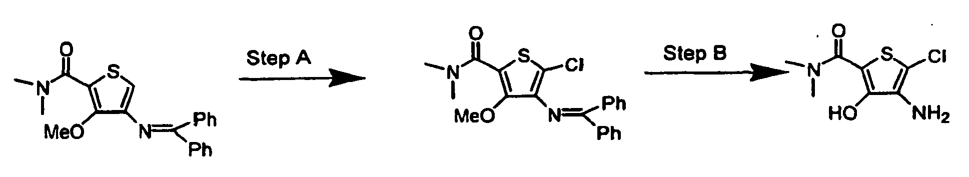

### Step A

To the product from Preparative Example 13.19 (400mg), Step D in CH₂Cl₂-pyridine (14ml) was added N-chlorosuccinimide (220mg). The mixture was stirred for 5hr and then diluted with CH₂Cl₂ and washed with water, saturated sodium bicarbonate and brine, and concentrated *in vacuo.* The crude product was purified via preparative plate chromatography (CH₂Cl₂-MeOH = 50:1) to give 180mg of product (64%).

### Step B

The product from above (274mg) was reacted in the procedure set forth in Preparative Example 13.19, Step E to give the amine (89mg, 58%).

### PREPARATIVE EXAMPLE 13.23

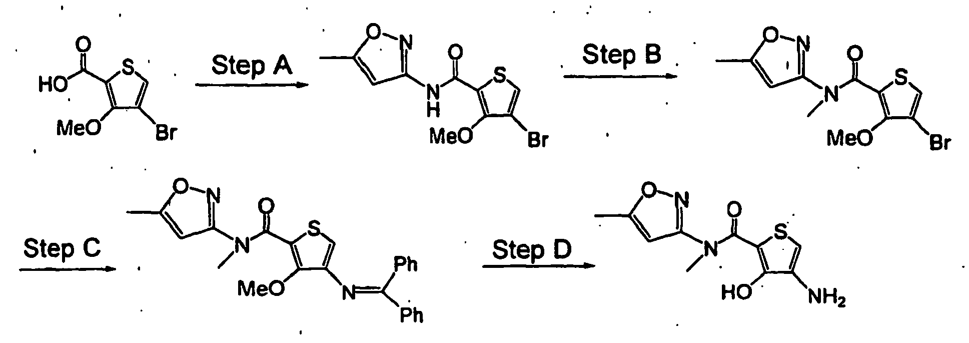

### Step A

To a stirred solution of acid (630mg) from Preparative Example 13.19, Step B in CH₂Cl₂ (25ml) was added oxalyl chloride (235ul) followed by a catalytic amount of DMF (10ul). The mixture was stirred for 1 hr, then potassium carbonate (1.8g) was added followed by 3-amino-5-methylisoxazole (443mg). The reaction stirred overnight and was quenched with water (25ml). Layers were separated and the organic layer was washed with brine, dried over Na₂SO₄, and concentrated *in vacuo.* The crude product was purified by preparative plate chromatography (CH₂Cl₂) to afford the product (580mg, 78%, MH+=317,319).

### Step B

The acid from the above (750mg) step was reacted following the procedure set forth in Preparative Example 13.3, Step B to yield 625mg of product (80%, MH+=331).

### Step C

The product from above was reacted following the procedure set forth in Preparative Example 13.19, Step D to yield 365mg of product (53%)

### Step D

The product from above was reacted following the procedure set forth in Preparative Example 13.19, Step E to give the amine product (MH+=254).

### PREPARATIVE EXAMPLE 13.25

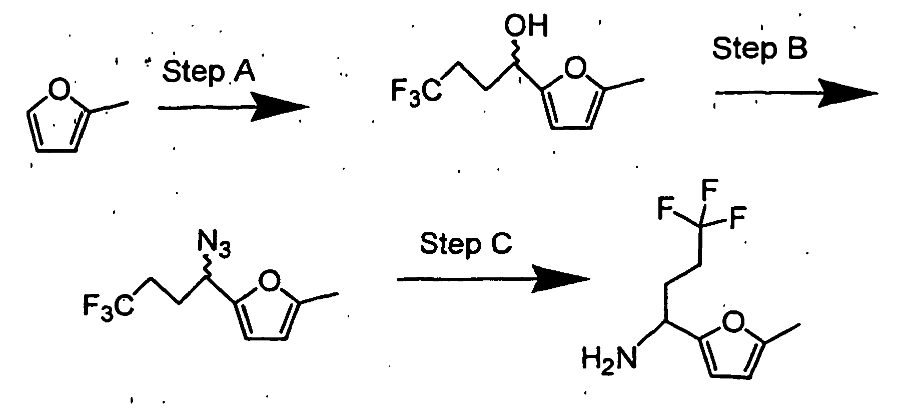

### Step A

To a solution of 2-methylfuran (1g) in ether (30ml) was added ri-BuLi (5.32ml) at -78°C. The reaction was warmed to room temperature and then refluxed at 38 °C for 1 hr. The reaction was cooled back down to -78°C where the furyl lithium was quenched with trifluorobutyraldehyde and let stir at room temperature overnight. Saturated ammonium chloride added and extracted with ether. Purified via flash column chromatography to yield pure product (2g, 80%)

### Step B

The azide was prepared using the procedure from Preparative Example 75.75, Step B and the alcohol (1g) from above and carried on crude to Step C below.

### Step C

The amine was prepared using the procedure from Preparative Example 75.75, Step C to yield 400mg of an oil (53%).

### PREPARATIVE EXAMPLE 13.26

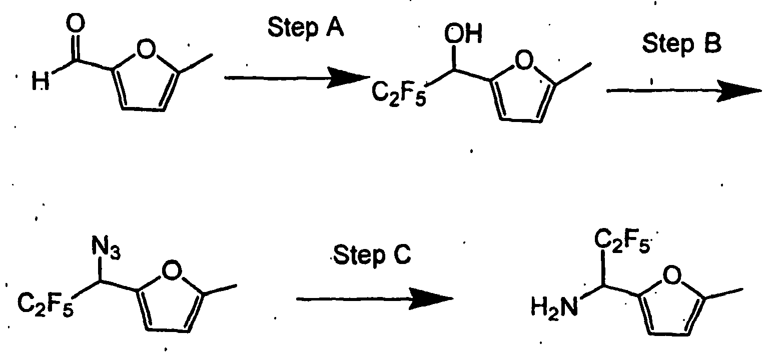

### Step A

Perfluoroiodide (3.6ml) was condensed at -78°C. Ether (125ml) was added followed by the methyllithium.lithiumbromide complex (1.5M in ether, 18.4ml). After 15min, a solution of 5-methylfuraldehyde (2.5ml) in ether was added dropwise. The reaction was warmed to -45 °C and let stir for 2hr. Saturated ammonium chloride (30ml) and water (30ml) were added and let stir at room temperature for 1 hr. The layers were separated and the aqueous layer was extracted with CH₂Cl₂. The organic layer was washed with brine, dried with Na₂SO₄, filtered and concentrated *in vacuo* to give 5.86g of product (100%).

### Step B

The alcohol from above was reacted to form the azide using the procedure set forth in Preparative Example 75.75 Step B.

### Step C

The azide from above was reacted to form the racemic amine using the procedure set forth in Preparative Example 75.75 Step C.

### PREPARATIVE EXAMPLE 13.27

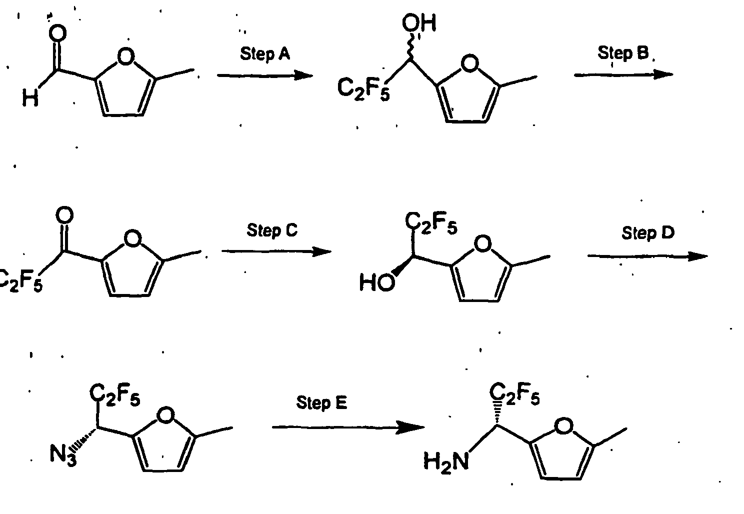

### Step A

Following the procedure set forth in Preparative Example 13.26, Step A, the alcohol was prepared (100%).

### Step B

To a solution of the alcohol (500mg) from step A above in CH₂Cl₂ (20ml) was added N-methyl-morpholine monohydrate (575mg) and a catalytic amount of tetrapropyl ammonium perruthenate (76mg). After 3hr, the mixture was diluted with hexane (10ml) and filtered through a silica pad, rinsing with hexane: CH₂Cl₂ (200ml). The filtrate was concentrated *in vacuo* to give 350mg of product (70.7%)

### Step C

The ketone (1.19g) from Step B was dissolved in THF (9.5ml) and cooled to 0 °C. A solution of S-methyl oxazoborolidine (1M in toluene, 1ml) followed by a solution of borane complexed with dimethylsulfide (9.5ml, 2M in THF) was added to the solution. The mixture was stirred at 0 °C for 30min and continued at room temperature for 5hr. The mixture was cooled back down to 0°C and methanol (15m1) was added dropwise to the mixture. After 30min, the mixture was concentrated *in vacuo* to give an oily residue.

The residue was dissolved in CH₂Cl₂ and washed with 1N HCl, water ; and brine. Dried with Na₂SO₄, filtered and concentrated *in vacuo.* The crude material was purified via flash column chromatography (Hex/CH₂Cl₂, 1:1) to afford 1.14g of an oil (67%).

### Step D

The alcohol (1.14g) from above was reacted to form the azide using the procedure set forth in Preparative Example 75.75 Step B.

### Step E

The azide (1.11 g) from above was stirred with 10% Pd/C (280mg) in EtOH (40ml) under a hydrogen gas atmosphere overnight. The reaction was filtered through celite, the filtrate was concentrated *in vacuo* to give 700mg of product (70%).

### PREPARATIVE EXAMPLE 13.28

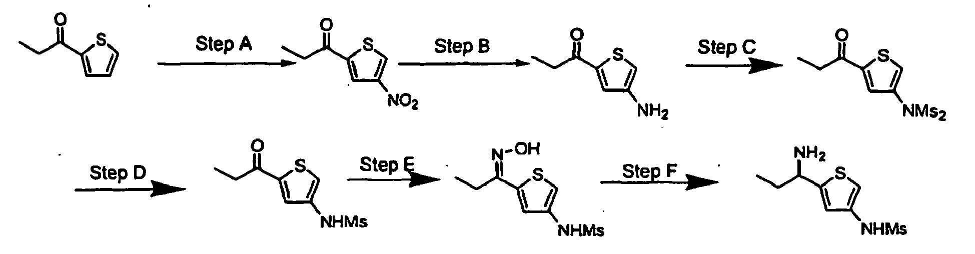

### Step A

To a stirred solution of 1-(2-thienyl)-1-propanone (3g) in acetic anhydride (6ml) at 0°C was added dropwise a solution of fuming nitric acid in acetic acid (2ml in 10ml). After 30min, the reaction was warmed to room temperature and let stir for 5hrs where a solid precipitated out. Ice was added to the reaction and the solid was filtered. The solid was purified by flash column chromatography (Hex/ CH₂Cl₂, 3:1 and 2:1) to yield 800mg of desired product (20%).

### Step B

The above nitro-thiophene compound (278mg) was reduced using the procedure set forth in Preparative Example 2, Step B to give 54mg of product (23%).

### Step C

The above amine (395mg), TEA (1ml) and methanesulfonylchloride (0.5ml) were combined in CH₂Cl₂ (35ml) and stirred at room temperature for 1hr. The reaction was quenched with saturated sodium bicarbonate (15ml). The organic layer was washed with brine, dried over Na₂SO₄, filtered and concentrated *in vacuo* to afford product (854mg, 100%).

### Step D

To the above product (854mg) in THF (25ml) was added dropwise a solution of tetrabutylammonium fluoride (1M in THF, 2.8ml). The mixture was stirred overnight, then diluted with CH₂Cl₂ (30ml), washed with ammonium chloride and brine, dried over over Na₂SO₄, filtered and concentrated *in vacuo* to afford product (2.36g, >100%).

### Step E

The ketone (2.36g) above was reacted via the procedure set forth in Preparative Example 88.2, Step B to yield 547mg of product (86.6%).

### Step F

To the product from step E (310mg) in dimethoxyethane (12ml) was added dropwise a solution of LAH (1M in ether, 3.8ml). The mixture was heated to reflux overnight. The reaction was cooled to room temperature, SiO₂ was added as well as water (1 ml) dropwise and let stir for 15min. The mixture was filtered and the filtrate was concentratred *in vacuo.* The crude product was purified by preparative plate chromatography (MeOH/ CH₂Cl₂, 15:1) to give the amine product (40mg, 14%).

### PREPARATIVE EXAMPLE 14

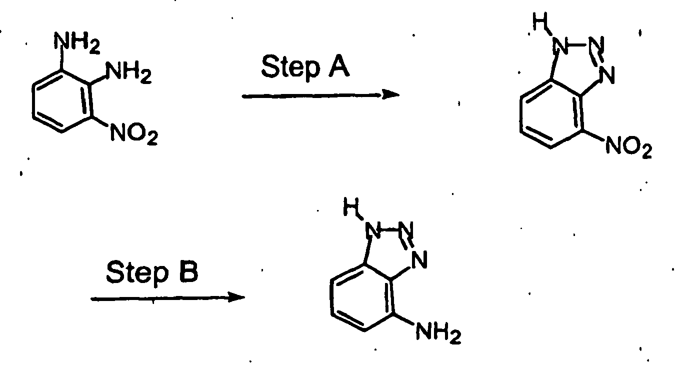

### Step A

3-Nitro-1,2-phenylenediamine(10 g), sodium nitrite (5.4 g) and acetic acid (20 mL) were heated at 60°C overnight, then concentrated *in vacuo,* diluted with water and extracted with EtOAc. The product precipitated from the organic phase (5.7 g) as a solid and used directly in step B.

### Step B

The product from Step A above (2.8 g) was stirred with 10% Pd/C (0.3 g) in MeOH (75 mL) under a hydrogen gas atmosphere overnight. The reaction mixture was filtered through celite and the filtrate concentrated *in vacuo,* to give the product (2.2 g, MH+=135).

### PREPARATIVE EXAMPLE 15

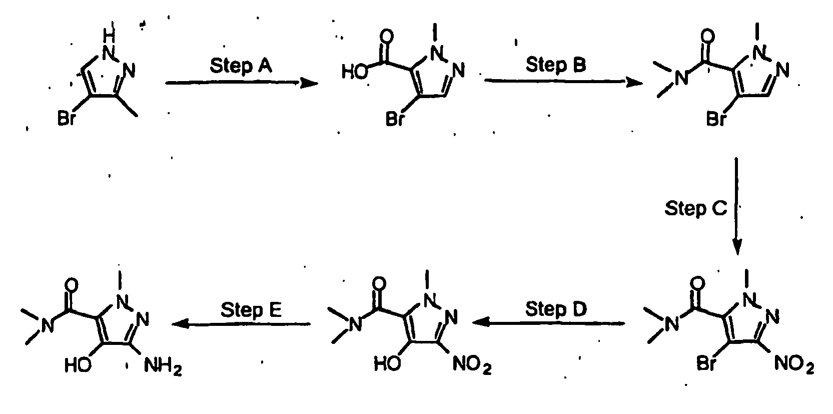

### Step A

N-methyl-4-bromopyrazole-3-carboxylic acid was prepared according to known methods, see: Yu. A. M.; Andreeva, M. A.; Perevalov, V. P.; Stepanov, V. I.; Dubrovskaya, V. A.; and Seraya, V. I. in Zh. Obs. Khim. (Journal of General Chemistry of the USSR) 1982, 52, 2592, and refs cited therein.

### Step B

To a solution of N-methyl-4-bromopyrazole-3-carboxylic acid (2.0 g), available from step A, in 65 mL of anhydrous DMF was added bromotripyrrolidinophosphonium hexafluorophosphate (PyBrop, 4.60 g), dimethyl amine (10 mL, 2.0 M in THF) and diisopropylethyl amine (5.2 mL) at 25 °C. The mixture was stirred for 26 h, and concentrated under reduced pressure to an oily residue. This residue was treated with a 1.0 M NaOH aqueous solution, and extracted with ethyl acetate (50 mL x 4). The organic extracts were combined, washed with brine, and dried with anhydrous Na₂SO₄. Removal of solvents yielded an oil, which was purified by preparative thin layer chromatography, eluting with CH₂Cl₂-MeOH (20:1), to give 1.09 g of the amide product (48%, MH⁺ = 232.0).

### Step C

To a solution of the amide (0.67 g), obtained from step B, in 8 mL of concentrated sulfuric acid at 0 °C was added potassium nitrate (1.16 g) in small portions. The cooling bath was removed and the mixture was heated at 110 °C for 6 h. After cooling to 25 °C, the mixture was poured into 80 mL of H₂O, and an additional 20 mL of H₂O was used as a rinse. The aqueous mixture was extracted with CH₂Cl₂ (100 mL x 4). The combined extracts were washed with brine (50 mL), sat. NaHCO₃ aqueous solution (50 mL), brine (50 mL), and dried with Na₂SO₄. Evaporation of solvent gave an oil, which solidified on standing. The crude product was purified by flash column chromatography, eluting with CH₂Cl₂-MeOH (1:0, 50:1 and 40:1). Removal of solvents afforded 0.521 g (65%) of the product as a solid (MH⁺ = 277.1)

### Step D

The product (61 mg) obtained from step C was dissolved in 3 mL of THF. To this solution at - 78 °C was added dropwise along the inside wall of the flask a 1.6 M solution of *n*-butyl lithium in hexane. After 45 min, a solution of methyl borate (0.1 mL) in THF (1.0 mL) was added. After 1.5 h, a solution of acetic acid in THF (0.25 mL, 1:10 v/v) was added to the cold mixture. Stirring was continued for 10 min, and a 30 wt % aqueous hydrogen peroxide solution (0.1 mL) was added. An additional portion of hydrogen peroxide aqueous solution (0.05 mL) was added 20 min later. The cooling bath was removed, and the mixture was stirred at 25 °C for 36 h. The mixture was poured into 30 mL of H₂O, and the aqueous mixture was extracted with ethyl acetate (30 mL x 4). The extracts were combined, washed with brine (10 mL), 5% NaHCO₃ aqueous solution (10 mL) and brine (10 mL). The organic layer was dried with Na₂SO₄ and concentrated under reduced pressure to a residue, which was then purified by preparative thin layer chromatography eluting with CH₂Cl₂-MeOH (20:1) to give the hydroxylated product (5 mg, 10%, MH⁺ = 215.3).

### Step E

By treating the hydroxylated product of Step E with H₂ under the conditions of 10% palladium on carbon in ethanol, one would obtain the desired hydroxyl-amino compound.

### PREPARATIVE EXAMPLE 16

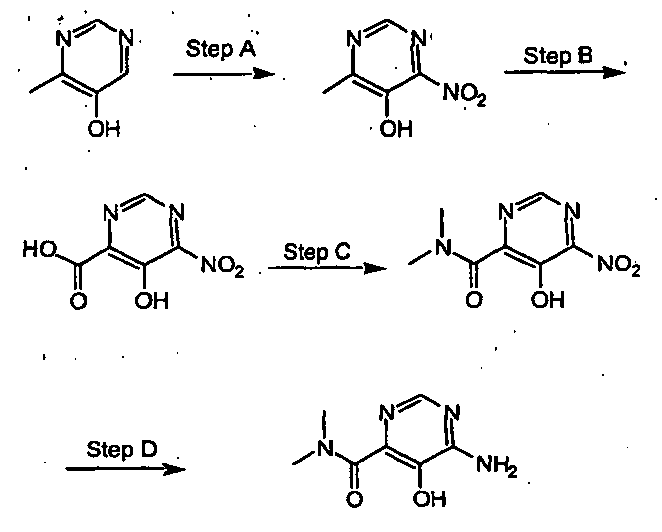

### Step A

Following a similar procedure used in Preparative Example 13, Step C except using the known compound, 4-methyl-pyrimidin-5-ol, the product can be prepared.

### Step B

Following a similar oxidation procedure used in Preparative Example 15, Step A except using the compound from Step A above, the product can be prepared.

### Step C

Following a similar procedure used in Preparative Example 11, Step A except using the compound from Step B above, the product can be prepared.

### Step D

Following a similar procedure used in Preparative Example 12, Step F except using the compound from Step C above, the product can be prepared.

### PREPARATIVE EXAMPLE 17

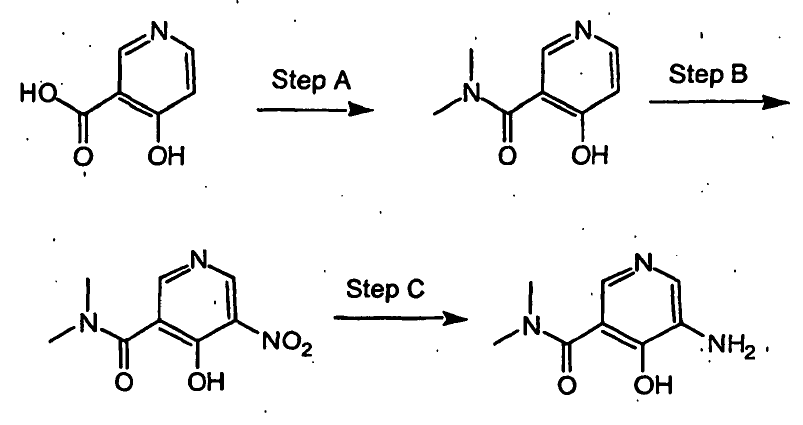

### Step A

Following a similar procedure used in Preparative Example 11, Step A except using the known 4-hydroxynicotinic acid, the product can be prepared.

### Step B

Following a similar procedure used in Preparative Example 13, Step C except using the compound from Step A above, the product can be prepared.

### Step C

Following a similar procedure used in Preparative Example 12, Step F except using the compound from Step C above, the product can be prepared.

### PREPARATIVE EXAMPLE 18

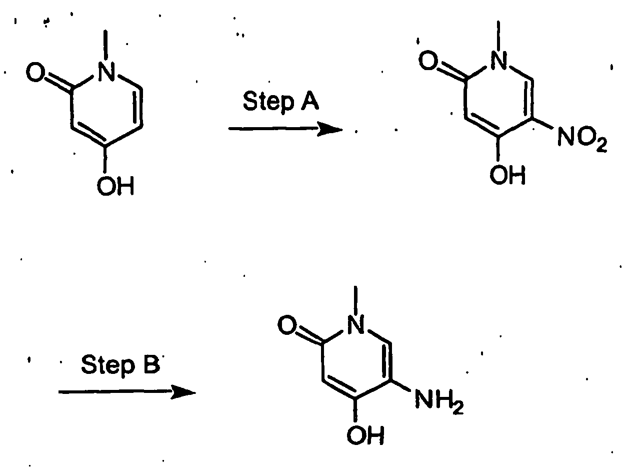

### Step A

Following a similar procedure used in Preparative Example 13, Step C except using the compound from Step A above, the product can be prepared.

### Step B

Stirring the compound from Step A above, a suitable Pt or Pd catalyst and EtOH under hydrogen atmosphere (1-4 atm) the product can be prepared.

### PREPARATIVE EXAMPLE 19

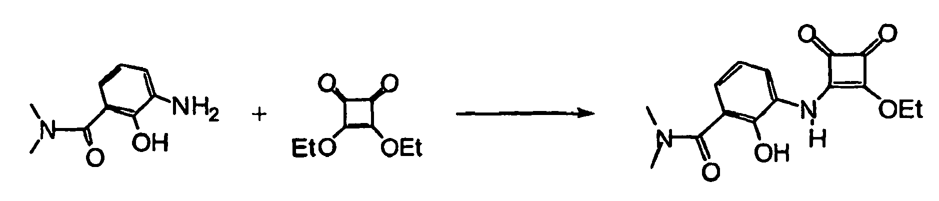

The product from Preparative Example 3 (14.6 g) dissolved in absolute EtOH (100 mL) was added dropwise over 4 hours to a stirred ethanolic (100 mL) solution of diethylsquarate (19 mL, 128 mmol). After 5 days, the reaction mixture was concentrated *in vacuo,* and the resulting residue purified by column chromatography (silica gel, 0-5% MeOH/CH₂Cl₂) gave the product (65%, MH⁺ = 305, mp = 178.6°C).

### PREPARATIVE EXAMPLE 19.1

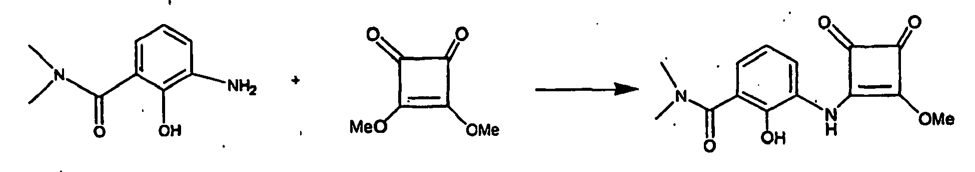

The amine from Prepartive Example 3 (5g) and dimethylsquarate (3.95g) in MeOH were stirred overnight. The precipitated product was filtered to give 6.32g of solid (78%, MH+=291.1)

### PREPARATIVE EXAMPLE 20-23.14

Following the procedures set forth in Preparative Example 19 but using the amine from the Preparative Example indicated in the Table below, the cyclobutenedione intermediates were obtained.

| Prep Ex. | Amine from | Product | 1. Yield (%) |
|---|---|---|---|
| | Prep Ex. | | 2. MH⁺ |
| 20 | 4 | 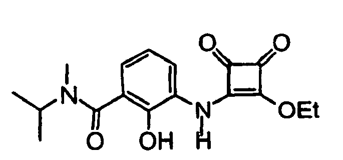 | 1. 85% |
| | | | 2. 333 |
| 21 | 11 | 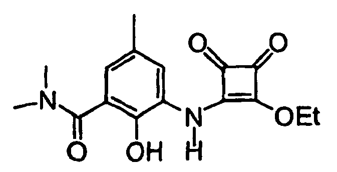 | 1. 44% |
| | | | 2. 319 |
| 21.1 | 6 | 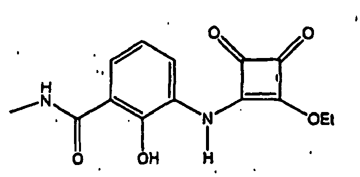 | 1. 9% |
| | | | 2. 291 |
| 22 | 2 | 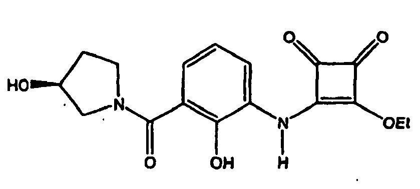 | 1. 38% |
| | | | 2. 347 |
| 23 | 14 | 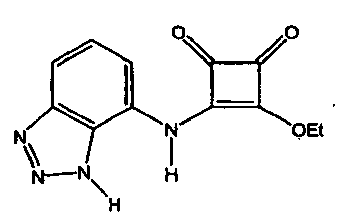 | 1. 51% |
| | | | 2. 259 |
| 23.1 | 10.1 | 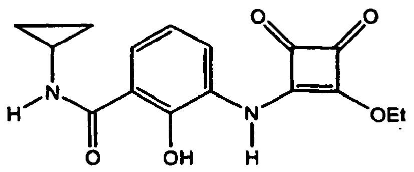 | 1. 62% |
| | | | 2: 317 |
| 23.2 | 10.2 | 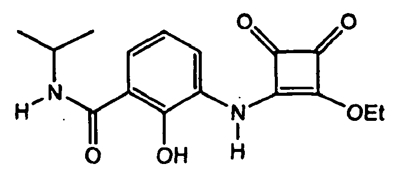 | 1. 61% |
| | | | 2. 319 |
| 23.3 | 12 | 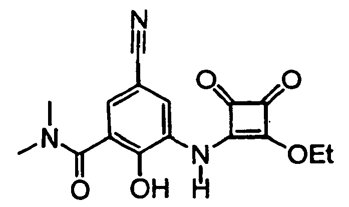 | 1. 40% |
| | | | 2. 330 |
| 23.4 | 10.3 | 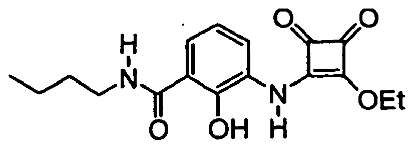 | 1. 42% |
| | | | 2. 333 |
| 23.5 | 10.4 | 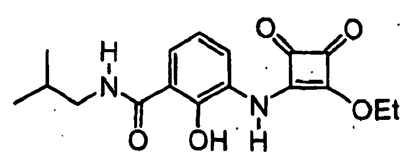 | 1. 40% |
| | | | 2. 333 |
| 23.6 | 10.5 | 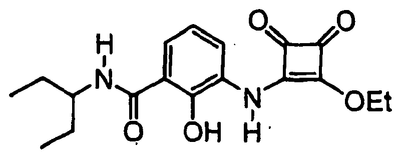 | 1. 37% |
| | | | 2. 347 |
| 23.7 | 13.2 | 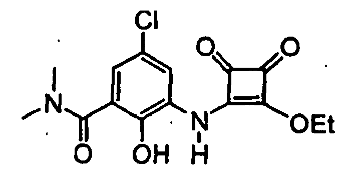 | 1. 39% |
| | | | 2. 339 |
| 23.8 | 13.1 | 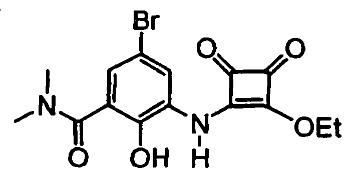 | 1. 42% |
| | | | 2. 383/385 |
| 23.9 | 13.19 | 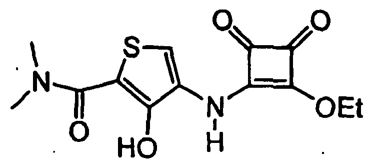 | 1. 51% |
| | | | 2. 311 |
| 23.10 | 13.20 | 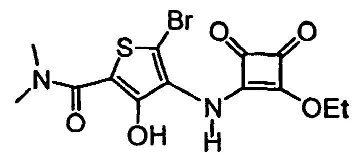 | 1. 67% |
| | | | 2. 389.1,390 |
| 23.11 | 13.3 | 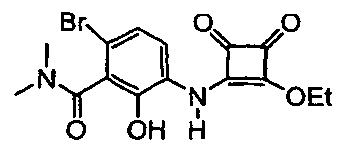 | 1. 52% |
| | | | 2. 383/385 |
| 23.12 | 13.21 | 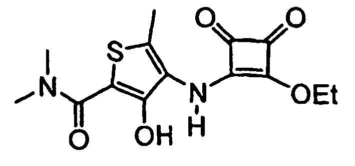 | 1. 76% |
| | | | 2. 325.1 |
| 23.13 | 13.22 | 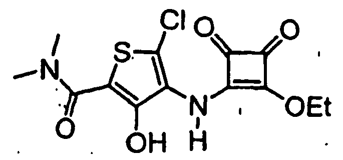 | 1. 54% |
| 23.14 | 13.23 | 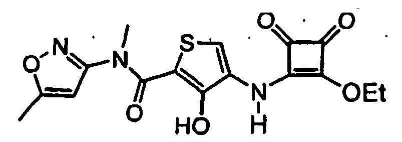 | 1. 62% |
| | | | 2. 378 |

### PREPARATIVE EXAMPLE 23.16-23.24

Following the procedures set forth in Preparative Example 19 but using the amine from the Preparative Example indicated in the Table below, the cyclobutenedione intermediate products were obtained.

| Prep Ex. | Amine from Prep Ex. | Product | Yield. (%) |
|---|---|---|---|
| 23.16 | 13.11 | 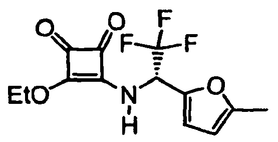 | 91% |
| 23.17 | 13.12 | 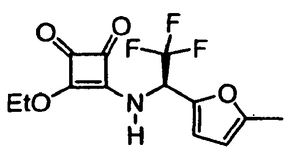 | 81% |
| 23.18 | 13.17 | 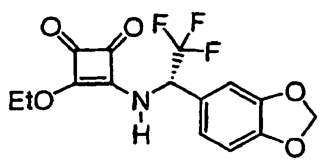 | 47% |
| 23.19 | 13.27 | 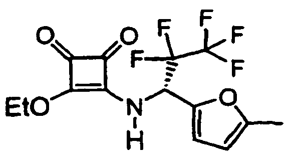 | 21% |
| 23.20 | 13.26 | 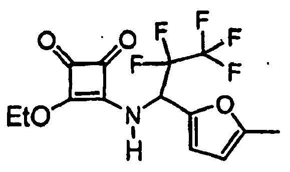 | 10% |
| 23.21 | 13.25 | 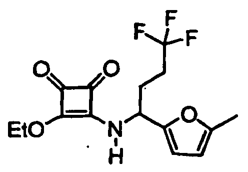 | 49% |
| 23.22 | 13.13 | 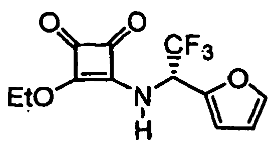 | 80% |
| 23.23 | 13.15 | 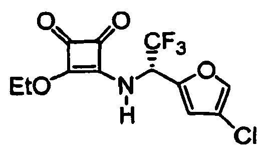 | 63% |
| 23.24 | 13.16 | 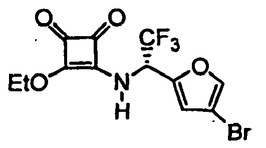 | 64% |

### PREPARATIVE EXAMPLE 24

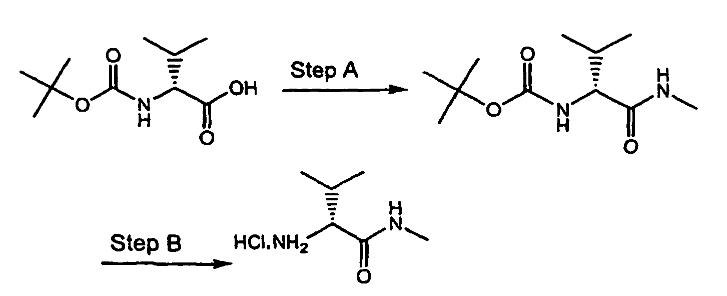

### Step A

To a solution of N-protected amino acid (1.5 g, 6.9 mmol) in CH₂Cl₂ (25 mL) at room temperature was added DIPEA (3.6 mL, 20.7 mmol), and PyBrop (3.4 g, 6.9 mmol) followed by MeNH₂ (6.9 mL, 13.8 mmol, 2.0 M in CH₂Cl₂). The resulting solution was stirred for 18 h at room temperature (until TLC analysis deemed the reaction to be complete). The resulting mixture was washed sequentially with 10% citric acid (3 x 20 mL), sat. aq. NaHCO₃ (3 x 20 mL), and brine (3 x 20 mL). The organic layer was dried (Na₂SO₄), filtered, and concentrated under reduced pressure. The crude product was purified by flash chromatography eluting with CH2Cl₂/MeOH (40:1) to afford 1.0 g (63% yield) of a, solid.

### Step B

To a round bottom charged with the N-protected amide (1.0 g, 4.35 mmol) (from Step A) was added 4N HCl/dioxane (10 mL) and the mixture was stirred at room temperature for 2 h. The mixture was diluted with Et₂O (20 mL) and concentrated under reduced pressure. The crude product was treated with Et₂O (2 x 20 mL) and concentrated under reduced pressure to afford 0.72 g (-100 % yield) of crude product as the HCl salt. This material was taken on without further purification or characterization.

### PREPARATIVE EXAMPLES 25-33.1

Following the procedure set forth in Preparative Example 24 but using the commercially available *N*-protected amino acids and amines in the Table below, the amine hydrochloride products were obtained.

| Prep Ex. | Amino acid | Amine | Product | 1.Yield (%) |
|---|---|---|---|---|
| 25 | 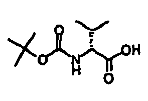 | NH₃ | 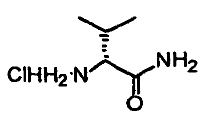 | 1. 70% |
| 26 | 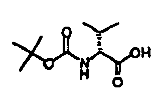 | 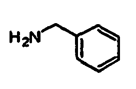 | 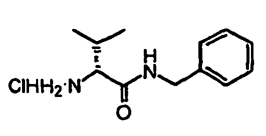 | 1. 71% |
| 27 | 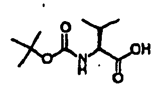 | 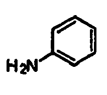 | 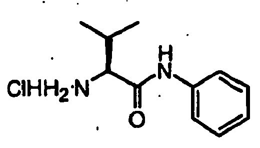 | 1. 66% |
| 28 | 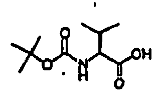 | 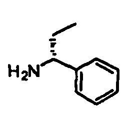 | 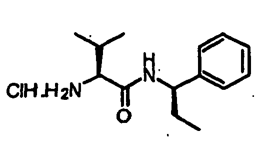 | 1. 65% |
| 29 | 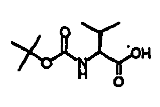 | 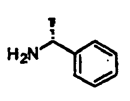 | 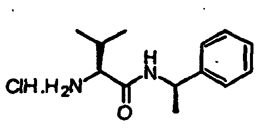 | 1. 90% |
| 30 | 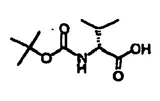 | 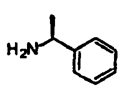 | 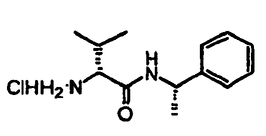 | 1. 68% |
| 31 | 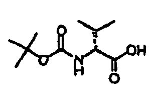 | 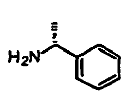 | 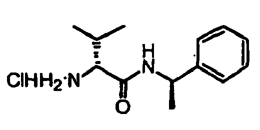 | 1. 68% |
| 32 | 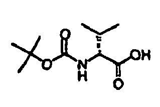 | 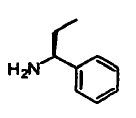 | 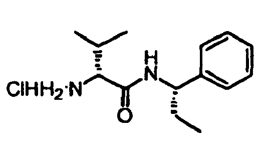 | 1. 97% |
| 33 | 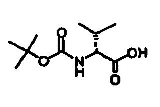 | 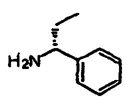 | 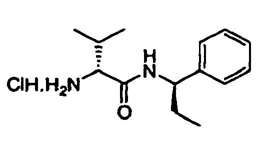 | 1. 97% |
| 33.1 | 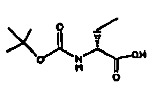 | 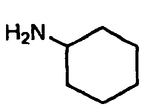 | 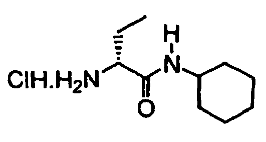 | 1. 20% |

### PREPARATIVE EXAMPLE 33.2

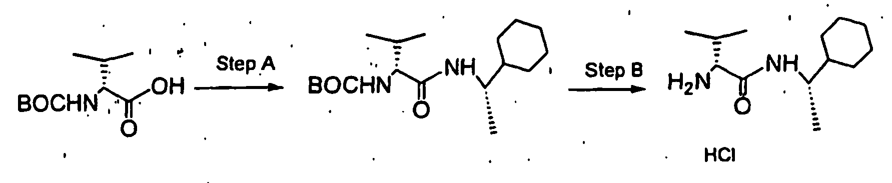

### Step A

BOC-valine (45mg) and PS-carbodiimide (200mg) were suspended in CH₂Cl₂ (4ml). After addition of the CH₂Cl₂-amine solution (0.138N, 1ml), the mixture was shaken overnight. The solution was filtered and the resin was washed, with more CH₂Cl₂, and the filtrate was concentrated *in vacuo* to yield the product, which was carried on directly in Step B.

### Step B

The crude material from Step A was dissolved in 4N HCl/dioxane (2.5ml) and stirred for 2h. The reaction was concentrated *in vacuo* to yield the desired amine hydrochloride, which was used directly in the next step.

### PREPARATIVE EXAMPLES 33.3-33.47

Following the procedure set forth in Example 33.2 but using the commercially available N-protected amino acids in the Table below, the amine hydrochloride products were obtained.

| Prep Ex. | Amino acid | Amine | Product |
|---|---|---|---|
| 33.3 | 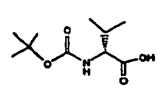 | 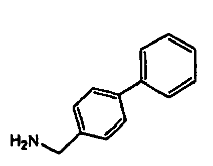 | 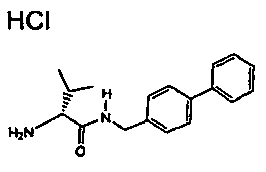 |
| 33.4 | 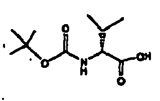 | 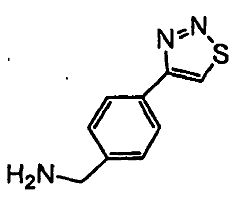 | 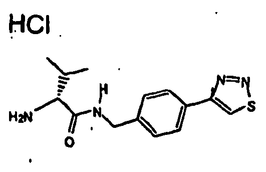 |
| 33.5 | 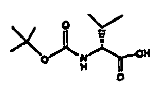 | 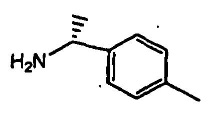 | 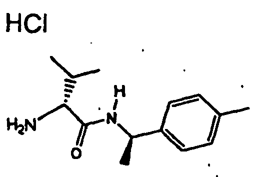 |
| 33.6 | 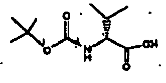 | 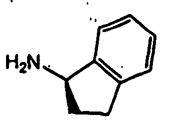 | 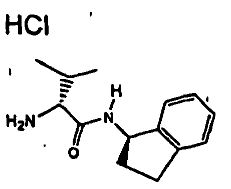 |
| 33.7 | 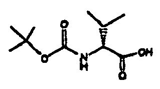 | 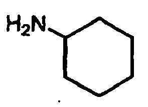 | 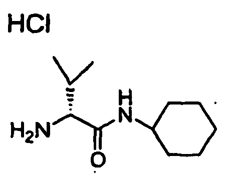 |
| 33.8 | 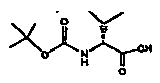 | 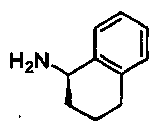 | 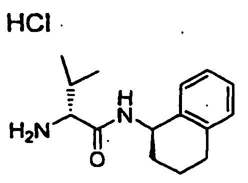 |
| 33.9 | 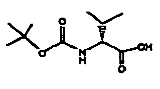 | 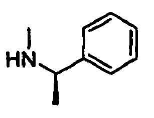 | 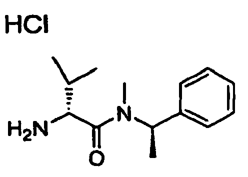 |
| 33.10 | 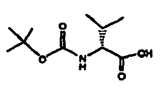 | 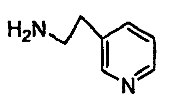 | 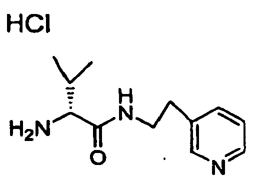 |
| 33.11 | 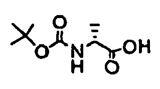 | 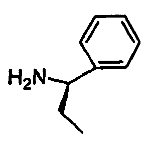 | 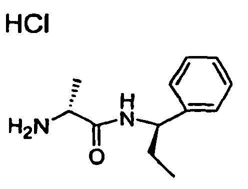 |
| 33.12 | 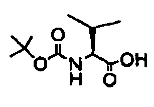 | 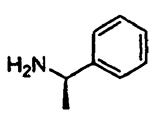 | 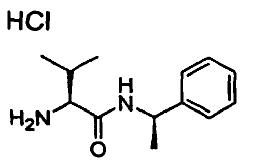 |
| 33.13 | 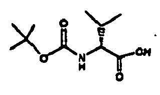 | 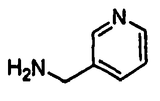 | 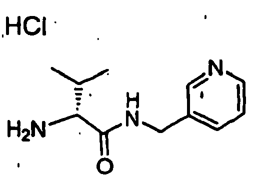 |
| 33.14 | 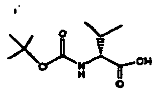 | 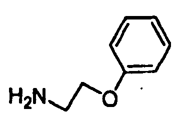 | 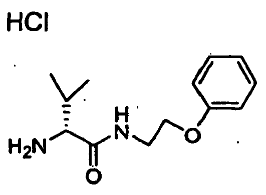 |
| 33.15 | 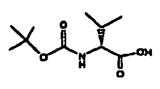 | 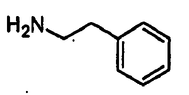 | 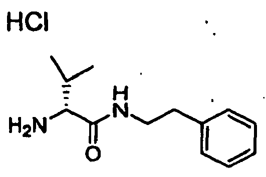 |
| 33.16 | 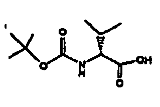 | 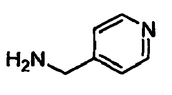 | 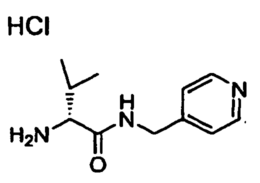 |
| 33.17 | 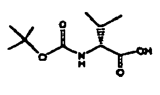 | 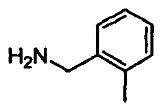 | 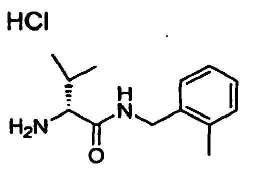 |
| 33.18 | 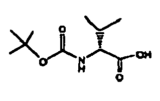 | 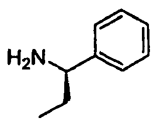 | 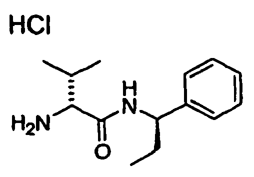 |
| 19 | 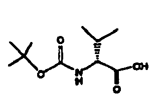 | 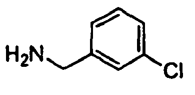 | 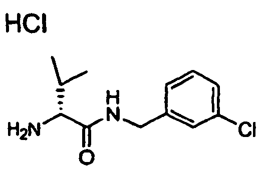 |
| 33.20 | 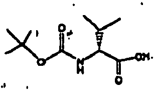 | 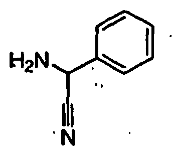 | 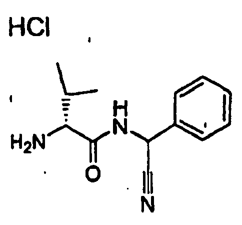 |
| 33.21 | 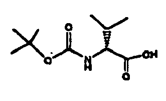 | 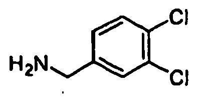 | 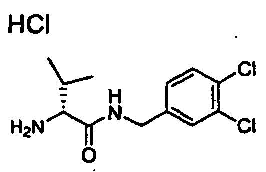 |
| 33.22 | 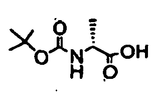 | 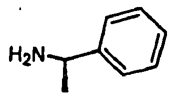 | 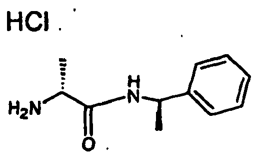 |
| 33.23 | 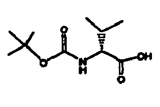 | 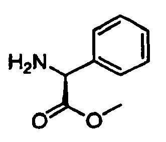 | 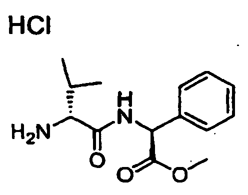 |
| 33.24 | 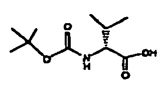 | 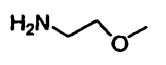 | 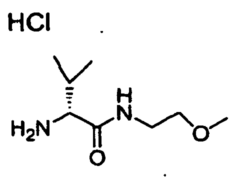 |
| 33.25 | 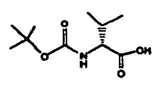 | 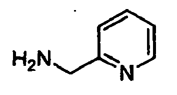 | 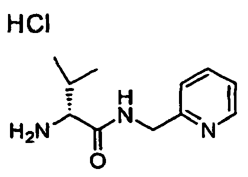 |
| 33.26 | 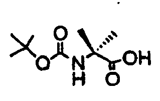 | 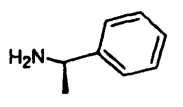 | 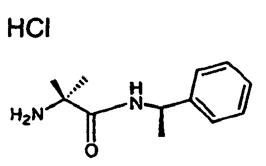 |
| 33.27 | 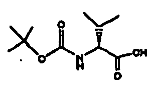 | 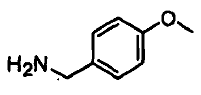 | 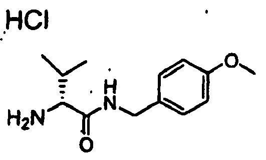 |
| 33.28 | 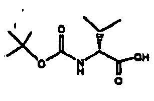 | 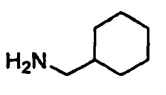 | 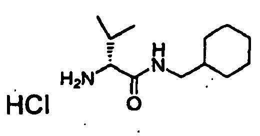 |
| 33.29 | 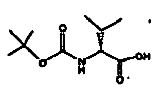 | 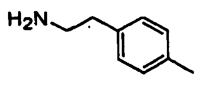 | 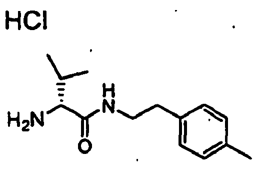 |
| 33.30 | 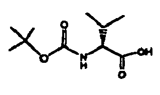 | 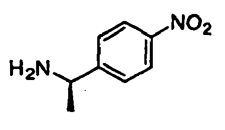 | 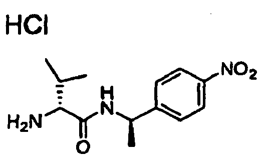 |
| 33.31 | 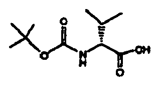 | 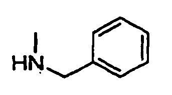 | 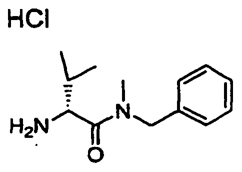 |
| 33.32 | 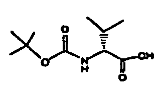 | 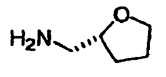 | 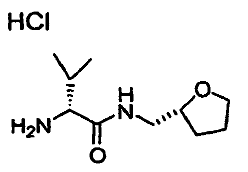 |
| 33.33 | 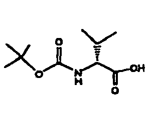 | 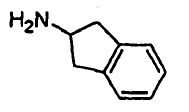 | 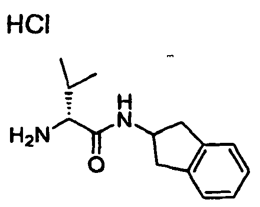 |
| 33.34 | 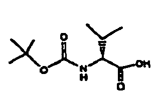 | 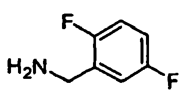 | 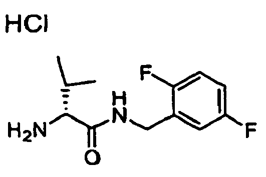 |
| 33.35 | 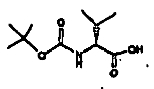 | 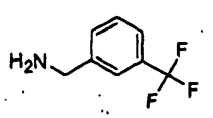 | 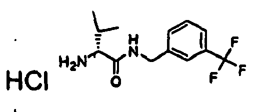 |
| 33.36 | 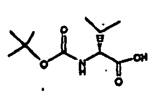 | 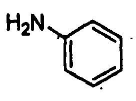 | 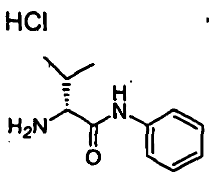 |
| 33.37 | 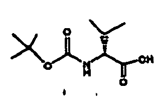 | 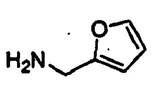 | 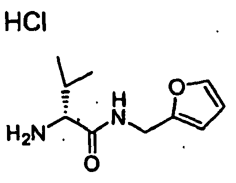 |
| 33.38 | 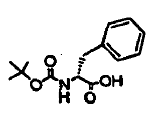 | 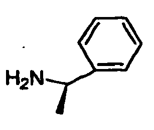 | 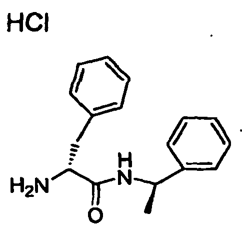 |
| 33.39 | 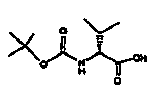 | 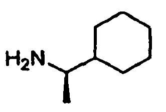 | 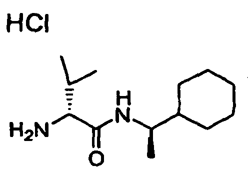 |
| 33.40 | 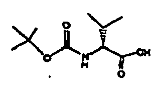 | 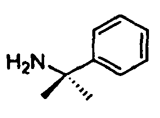 | 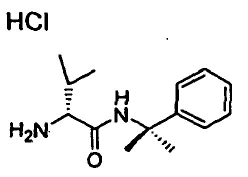 |
| 33.41 | 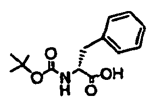 | 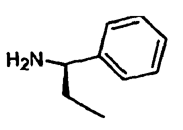 | 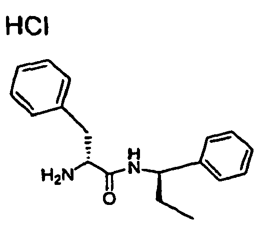 |
| 33.42 | 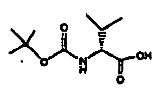 | 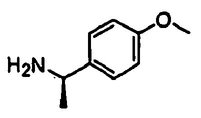 | 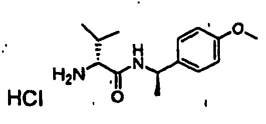 |
| 33.43 | 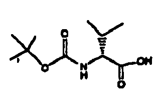 | 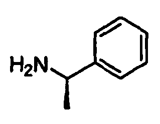 | 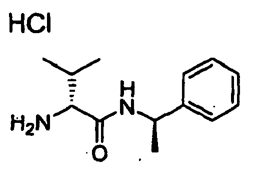 |
| 33.44 | 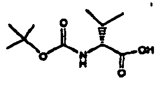 | 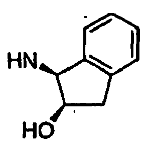 | 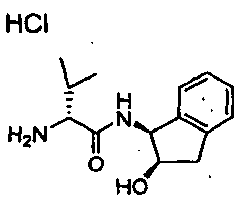 |
| 33.45 | 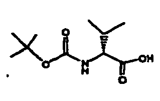 | 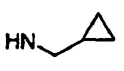 | 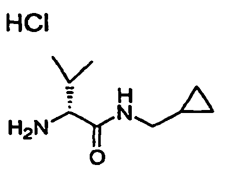 |
| 33.46 | 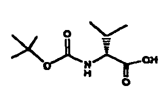 | 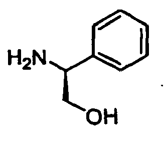 | 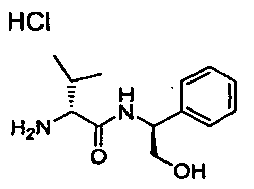 |
| 33.47 | 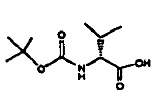 | 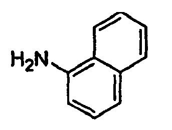 | 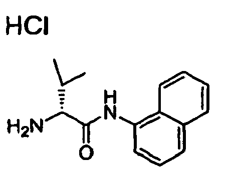 |

### Preparative Example 34

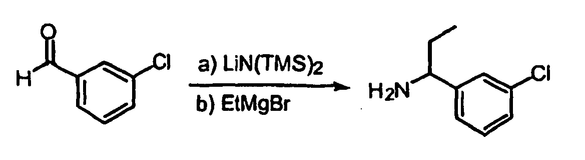

To a solution of 3-chlorobenzaldehyde (2.0 g, 14.2 mmol) in THF (5 mL) at 0 °C was added LiN(TMS)₂ (17.0 ml, 1.0 M in THF) dropwise and the resulting solution was stirred for 20 min. EtMgBr (6.0 mL, 3.0 M in Et₂O) was added dropwise and the mixture was refluxed for 24 h. The mixture was cooled to room temperature, poured into saturated aqueous NH₄Cl (50 mL), and then extracted with CH₂Cl₂ (3 x 50 volumes). The organic layers were combined, concentrated under reduced pressure.

The crude residue was stirred with 3 M HCl (25 mL) for 30 min and the aqueous layer was extracted with CH₂Cl₂ (3 x 15 mL) and the organic layers were discarded. The aqueous layer was cooled to 0 °C and treated with solid NaOH pellets until pH = 10 was attained. The aqueous layer was extracted with CH₂Cl₂ (3 x 15 mL) and the organic layers were combined. The organic layer was washed with brine (1 x 25 mL), dried (Na₂SO₄), and concentrated under reduced pressure to afford 1.6 g (66% yield) of the crude amine as an oil (MH⁺ 170). This material was determined to be >90% pure and was used without further purification.

### PREPARATIVE EXAMPLE 34.1

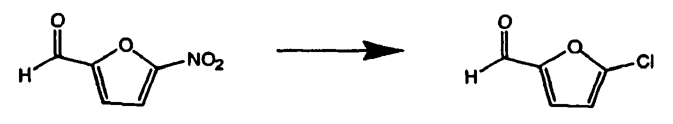

The aldehyde (3.5g) and conc. HCl (20ml) were combined and stirred overnight at 40°C. The reaction mixture was poured into cold water and extracted with ether, washed with satd. NaHCO₃ and brine, dried over anhydrous MgSO₄, filtered and concentrated *in vacuo* to give 1.76g of product (55%)

### PREPARATIVE EXAMPLE 34.2

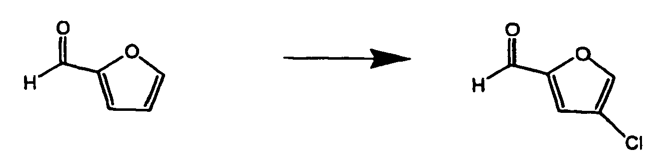

Chlorine was bubbled into 100ml of CH₂Cl₂ at 10°C. The aldehyde (3.73ml) was charged with 50ml of CHCl₃ and then cooled to 0°C. AlCl₃ was added portionwise, followed by the chlorine solution and let stir at room temperature overnight. The reaction was poured into 150ml of ice and 50ml of 3N HCl and stirred for 30min. Organic layer was washed with brine, dried with Na₂SO₄, and concentrated *in vacuo.* The crude product was purified via flash column chromatography (Hex/EtOAc 40/1) to yield 1.5g of pure product.

### PREPARATIVE EXAMPLE 34.3

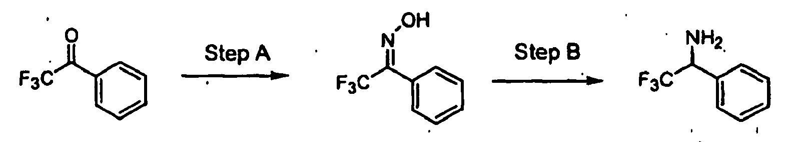

### Step A

The ketone (3.25g) was reacted following the procedure set forth in Preparative Example 88.2, Step B to give the oxime (3.5g, 99%).

### Step B

The product from step A (1.2g) was stirred with AcOH (3ml) and Pd/C (10%, 300mg) in EtOH (40ml) under a hydrogen atmosphere overnight. The reaction mixture was filtered through celite and the filtrate was concentrated *in vacuo.* The crude material dissolved in ether and washed with 2N NaOH, organic washed with brine, dried with Na₂SO₄, and concentrated *in vacuo* to give product (960mg, 86%).

### PREPARATIVE EXAMPLE 34.4

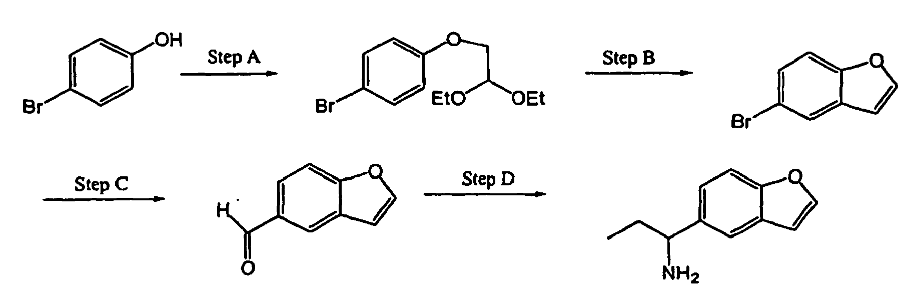

### Step A

To a suspension of NaH (1.45g) in DMF (25ml) under a nitrogen atmosphere was added p-bromophenol (5g) at 0°C. After stirring for 20min, BrCH₂CH(OEt)₂ (5.3ml) was added and the reaction was heated to reflux overnight. The solution was cooled and poured into ice water (80ml) and extracted with ether. The ether layer was washed with 1N NaOH and brine, dried with MgSO₄, filtered and concentrated *in vacuo* to give 8.4g of crude product (100%)

### Step B

To a solution of the product from Step A (8.4g) in benzene (50ml) was added polyphosphoric acid (10g). The mixture was heated at reflux for 4 hrs. The reaction was cooled to 0°C and poured into ice water (80ml) and extracted with ether. The ether layer was washed with saturated sodium bicarbonate and brine, dried with MgSO₄, filtered and concentrated *in vacuo* to give 4.9g of, crude product (85%)

### Step C

To a solution of the product from Step B (2g) in ether (20ml) at -78°C was added t-BuLi dropwise. After stirring for 20min, DMF (950mg) was added dropwise and the mixture was stirred at -25°C for 3hrs and then warmed to room temperature overnight. Saturated ammonium chloride was added and the solution was extracted with ether. The ether layer was washed with brine, dried with MgSO₄, filtered and concentrated *in vacuo* to give 980mg of crude product (67%).

### Step D

To a solution of aldehyde (400g) in ether (10ml) was added LiN(TMS)₂ (1M in THF, 3.3ml) at 0°C dropwise. The solution was stirred at 0°C for 30min and EtMgBr (3M in THF, 1.83ml) was added dropwise. The reaction was refluxed overnight, cooed to 0°C, quenched with saturated ammonium chloride and extracted with ether. The ether was stirred with 3N HCl (20ml), then the aqueous layer was basified with NaOH pellets and extracted with ether. The ether layer was washed with brine, dried with MgSO₄, filtered and concentrated *in vacuo* to give 220mg of product (46%).

### PREPARATIVE EXAMPLE 34.5

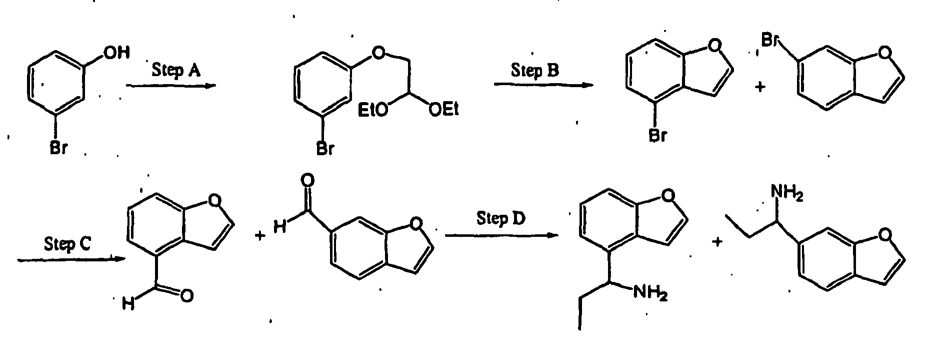

Following the procedures set forth in Preparative Example 34.4 Steps A through D, but using m-bromophenol (8g), both amines were formed and separated by preparative plate chromatography (63-65%, MH+=175).

### PREPARATIVE EXAMPLE 34.6

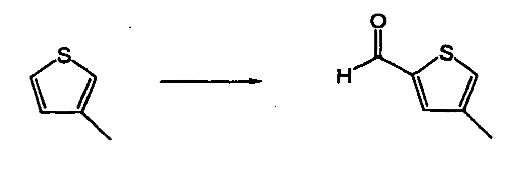

To a solution of 3-methyl-thiophene (5g) in ether (50ml) was added dropwise a solution of n-BuLi (1.6M in hexane, 32ml). The mixture was stirred for 1.5hr at room temperature. DMF (5.1 ml) was then added and let stir overnight. The mixture was poured into saturated ammonium chloride and extracted with ether. The ether layer was washed with brine, dried with Na₂SO₄, and concentrated *in vacuo.* The crude product was purified via flash column chromatography (EtOAc/Hex 20:1) to afford 5.27g of an oil (84%).

### PREPARATIVE EXAMPLE 34.7

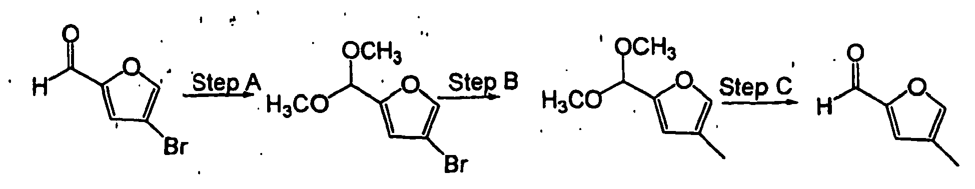

### Step A

To a solution of 4-bromo-2-furaldehyde (4g) in MeOH (75ml) was added trimethyl- orthoformate (3.8ml). A catalytic amount of *p*-toluene sulfonic acid (195mg) and the mixture was heated to reflux for 3.5hr. The reaction was cooled down and potassium carbonate was added. The mixture was filtered through a silica gel pad. The filtrate was concentrated *in vacuo,* dissolved in CH₂Cl₂ and filtered. The filtrate was again concentrated *in vacuo* to give 4.03g of product (80%).

### Step B

To a solution of the product from Step A (2.02g) in THF (80ml) at -78°C was added dropwise a solution of n-BuLi (2.5M in hexanes, 4.4ml) and stirred for 1.5hr. A solution of iodomethane (1.7m1) was added and let stir for 2.5hrs at -60°C. The cooling bath was removed and saturated ammonium chloride was added and let stir for 10min. The layers were separated and the organic layer was washed with brine, dried with Na₂SO₄, and concentrated *in vacuo* to afford 1.34g of crude product.

### Step C

The product from Step B (1.43g) was dissolved in acetone (50ml) and treated with a catalytic amount of p-toluene sulfonic acid (80mg). The mixture was heated to reflux for 2hr. The reaction was cooled down and solid potassium carbonate was added. The mixture was filtered through a silica gel pad and the filtrate was concentrated *in vacuo* to give 1.246g of crude product.

### PREPARATIVE EXAMPLE 34.8

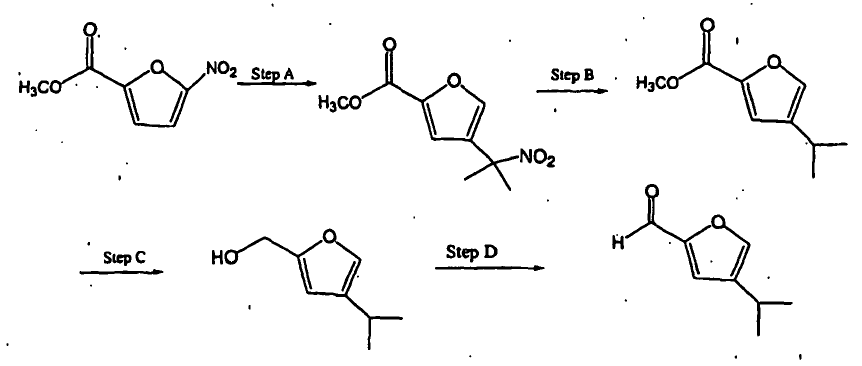

### Step A

To a stirred solution of potassium t-butoxide (2.5g) in HMPA (20ml) was added 2-nitropropane (2ml) dropwise. After 5min, a solution of methyl-5-nitro-2-furoate (3.2g) in HMPA (8ml) was added to the mixture and stirred for 16hr. Water was added and the aqueous mixture was extracted with EtOAc. The EtOAc layer was washed with water, dried with MgSO₄, filtered and concentrated *in vacuo.* The crude material was purified by flash column chromatography (Hex/EtOAc, 6:1) to yield 3.6g of product (90%).

### Step B

To a solution of the product from Step A (3.6g) in toluene (16ml) was added tributyltin hydride (5.4ml) followed by AIBN (555mg). The mixture was heated to 85°C for 3.5hr. After cooling, the mixture was separated by flash column chromatography (Hex/EtOAc, 7:1) to afford 2.06g of product (73%).

### Step C

To a solution of product from Step B (2.05g) in THF (60ml) at 0°C was added a solution of LAH (1M in ether, 12.8ml). The reaction was stirred at room temperature for 30min. Water and 1M NaOH was added until a precipitate formed, diluted with EtOAc, stirred for 30min and then filtered through a celite pad. The organic filtrate was concentrated *in vacuo* to give 1.56g of product (93%).

### Step D

To a solution of product from Step C (2.15g) in CH₂Cl₂ (100ml) was added Dess-Martin oxidant (7.26g) in CH₂Cl₂ (45ml) and stirred for 30min. The mixture was diluted with ether (200ml). The organic layer was washed with 1N NaOH, water and brine, dried with MgSO₄, filtered and concentrated *in vacuo* to give oil and solid. The material was extracted with ether and filtered. Some solid crystallized out from the filtrate, filtered again, and the filtrate was concentrated *in vacuo* to give 2.19g of product.

### PREPARATIVE EXAMPLE 34.9

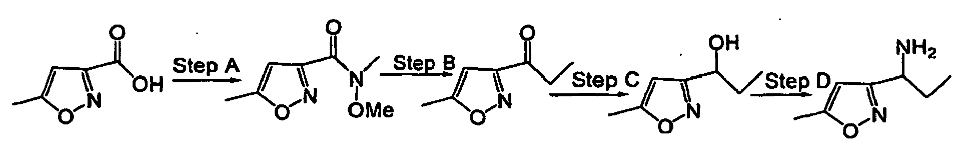

### Step A

To a solution of carboxylic acid (5g) in CH₂Cl₂ (400ml) at 0°C was added N(OCH₃)CH₃.HCl (11.5g), DEC (15.1g), HOBt (5.3g) and NMM (43ml) and stirred for 14hr. The mixture was diluted with CH₂Cl₂ (100ml) and the organic layer was washed with 10% HCl, saturated sodium bicarbonate and brine, dried with Na₂SO₄, and concentrated *in vacuo* to afford 5.74g of crude product (85%).

### Step B

To a solution of iodoethane (0.56ml) in ether (5ml) at -78°C was added a solution of t-BuLi (1.7M in pentane, 8.3ml) dropwise. The mixture was warmed to room temperature for 1 hr and transferred to a 100ml round bottom charged with the product from Step A (1g) in THF (12ml) at -78°C. The mixture was stirred at -78°C for 1hr and at 0°C for an additional 2hr. 1M HCl was added dropwise followed by CH₂Cl₂. The layers were separated and the organic layer was washed with brine, dried with Na₂SO₄, and concentrated *in vacuo* to afford 620mg of product (76%).

### Step C

To a solution of the product from Step B (6,20mg) in THF/MeOH (10:1) at 0°C was added NaBH₄ (250mg) in one portion. The mixture was stirred overnight at 0°C, concentrated *in vacuo* and the crude material was dissolved in CH₂Cl₂ and washed with 1N NaOH and brine, dried with Na₂SO₄, and concentrated *in vacuo* to afford 510mg of product.

### Step D

The above material was reacted in the procedures set forth in Preparative Example 75.75 Steps B and C to yield 170mg of amine product (28%).

### PREPARATIVE EXAMPLE 34.10

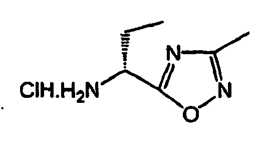

The above amine was made analogous to the procedures set forth in Patent WO96/22997 p.56, but using ethylglycine instead of benzylglycine in the DCC coupling.

### PREPARATIVE EXAMPLE 34.11

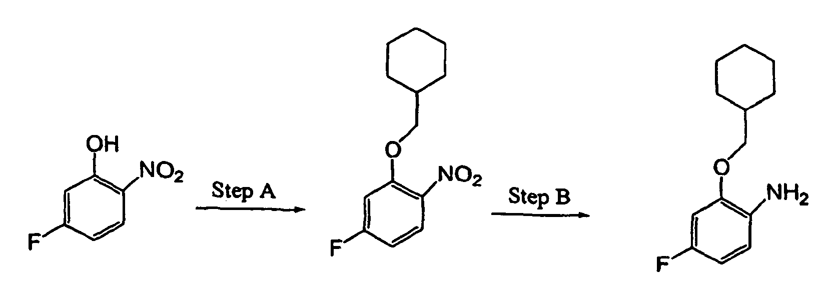

### Step A

To the nitro compound (3.14g) and cyclohexyl methanol (1.14g) in THF (50ml) was added PRH₃ (4.72g) and cooled to 0°C. Diisopropylazadicarboxylate (3.15ml) was added dropwise and let stir overnight. The reaction was concentrated *in vacuo* and purified via flash column chromatography (Hex/EtOAc, 30:1) to give product (3.3g), which was carried on directly to the next step.

### Step B

To the product from step A (3.3g) in EtOH (50ml) was added 10% Pd/C (1.7g) under a hydrogen atmosphere at 55psi and let stir overnight. The reaction was filtered through celite and concentrated *in vacuo* to give 3.2g of product.

### PREPARATIVE EXAMPLE 34.12

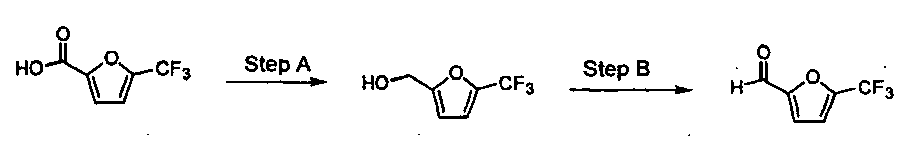

### Step A

A solution of acid (2g) in ether (20ml) was added dropwise to a suspension of LiAlH₄ (350mg) in ether (15ml) at 0°C. The solution was refluxed for 3hr and stirred at room temperature ovenright. 5% KOH was added and reaction was filtered, extracted with ether, dried with MgSO₄, filtered and concentrated *in vacuo* to give the product (1.46g, 79%, MH+=166).

### Step B

To a solution of alcohol from above (1.46g) in CH₂Cl₂ at room temperature was added Dess-Martin reagent (5.6g) portionwise and one drop of water and let stir over the weekend at room temperature. 10% Na₂S₂O₃ was added and stirred for 20min, extracted with CH₂Cl₂, washed with saturated sodium bicarbonate, dried with Na₂SO₄, and concentrated *in vacuo* to afford 1.1g of product (76%).

### PREPARATIVE EXAMPLE 34.13

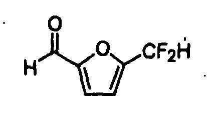

The above compound was prepared in the procedure set forth in EP Patent 0 555153 A1.

### PREPARATIVE EXAMPLE 34.14

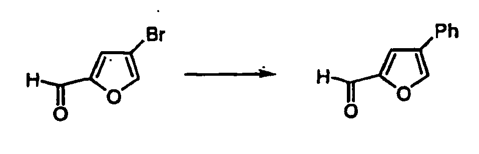

The aldehyde (500mg) from above was reacted following the procedure set forth in the Preparative Example 13.4, Step A to yield 372mg of product (76%).

### PREPARATIVE EXAMPLES 35-51.20

Following the procedure set forth in Preparative Example 34 but using the commercially available aldehydes and Grignard reagents listed in the Table below, the amine products below were obtained.

| Prep | Aldehyde | Grignard | Amine | 1.Yield (%) |
|---|---|---|---|---|
| Ex. | | Reagent | | 2. MH⁺ |
| 35 | 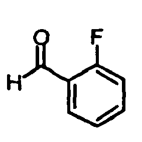 | EtMgBr | 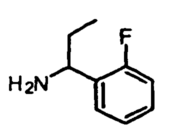 | 1. 65% |
| | | | | 2. 154 |
| 36 | 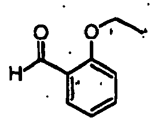 | EtMgBr | 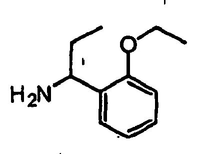 | 1. 75% |
| | | | | 2. 180. |
| 37 | 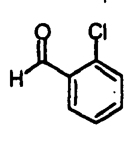 | EtMgBr | 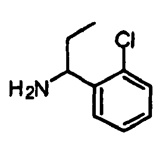 | 1. 78% |
| | | | | 2. 170 |
| 38 | 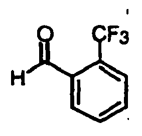 | EtMgBr | 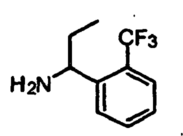 | 1. 34% |
| | | | | 2. 204 |
| 39 | 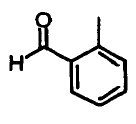 | EtMgBr | 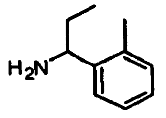 | 1. 68% |
| | | | | 2. 150 |
| 40 | 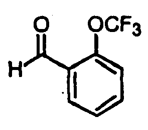 | EtMgBr | 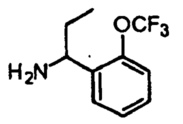 | 1. 40% |
| | | | | 2. 220 |
| 41 | 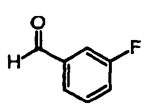 | EtMgBr | 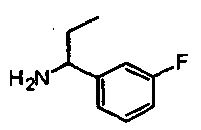 | 1. 73% |
| | | | | 2. 154 |
| 42 | 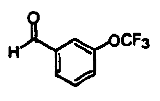 | EtMgBr | 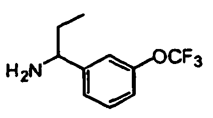 | 1. 52% |
| | | | | 2. 220 |
| 43 | 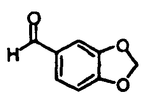 | EtMgBr | 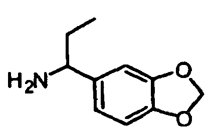 | 1. 55% |
| | | | | 2. 180 |
| 43 | 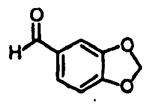 | EtMgBr | 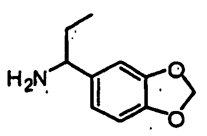 | 1. 55% |
| | | | | 2. 180 |
| 44 | 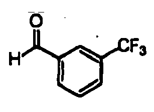 | EtMgBr | 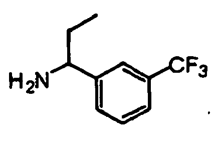 | 1. 20% |
| | | | | 2. 204 |
| 45 | 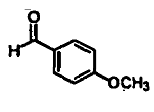 | EtMgBr | 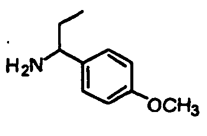 | 1. 80% |
| | | | | 2. 166 |
| 46 | 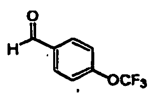 | EtMgBr | 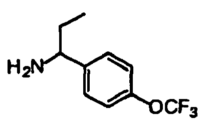 | 1. 35% |
| | | | | 2. 220 |
| 47 | 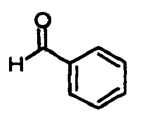 | *i*-PrMgBr | 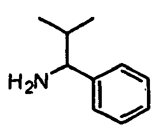 | 1. 20% |
| | | | | 2. 150 |
| 48 | 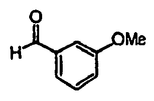 | EtMgBr | 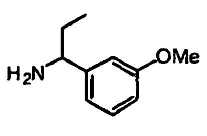 | 1. 77% |
| | | | | 2. [M-NH₂]⁺ = 149 |
| 49 | 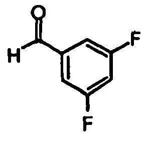 | EtMgBr | 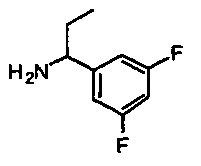 | 1. 77% |
| | | | | 2. 172 |
| 50 | 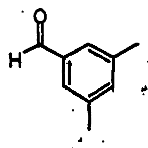 | EtMgBr | 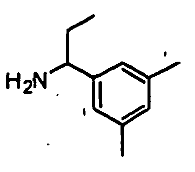 | 1. 78% |
| | | | | 2. [M-NH₂]⁺ = 147 |
| 51 | 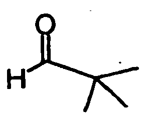 | EtLi | 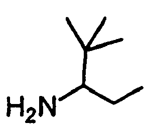 | 1. 10% |
| | | | | 2. 116 |
| 51.2 | 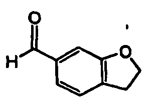 | EtMgBr | 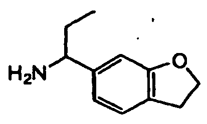 | 1. 37% |
| | | | | 2. 161 |
| 51.3 | 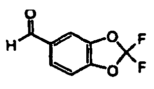 | EtMgBr | 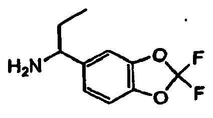 | 1. 63% |
| | | | | 2. 216 |
| 51.4 | 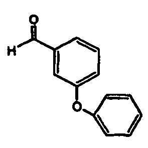 | EtMgBr | 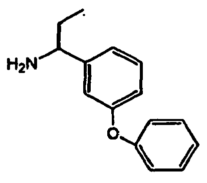 | 1. 71% |
| | | | | 2. 228 |
| 51.5 | 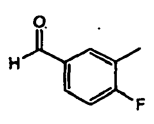 | EtMgBr | 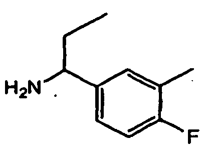 | 1. 89% |
| | | | | 2. 168 |
| 51.6 | 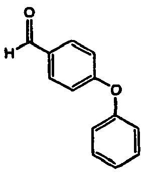 | EtMgBr | 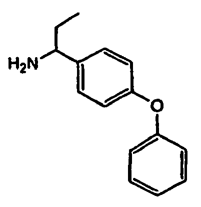 | 1. 20% |
| | | | | 2. 228 |
| 51.8 | 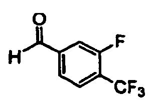 | EtMgBr | 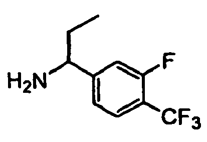 | 1. 36% |
| | | | | 2. 222 |
| 51.10 | 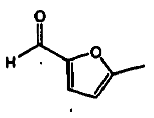 | 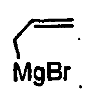 | 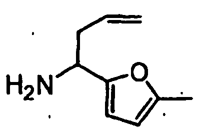 | 1. 95% |
| | | | | 2. 152.1 |
| 51.11 | 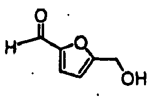 | EtMgBr | 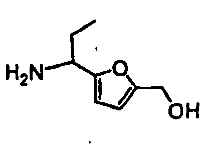 | 1. 61% |
| | | | | 2. 138.1 MH⁺-H₂O |
| 51.12 | 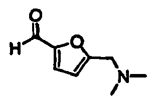 | EtMgBr | 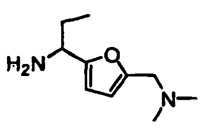 | 1. 70% |
| | | | | 2. 184.1 |
| 51.18 | 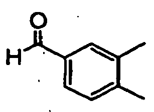 | EtMgBr | 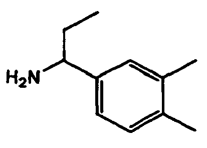 | 1. 42% |
| | | | | 2. 147 [M-NH₂]⁺ |
| 51.19 | 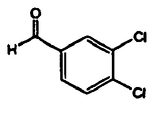 | EtMgBr | 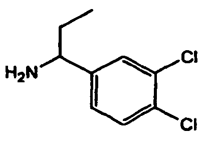 | 1. 67% |
| | | | | 2. 204 |
| 51.20 | 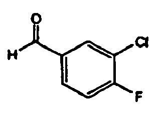 | EtMgBr | 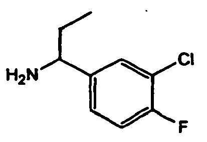 | 1. 33% |
| | | | | 2. 188 |

### PREPARATIVE EXAMPLES 51.25 - 51.31

Following the procedure set forth in Example 34 but using the commercially available aldehydes and Grignard reagents listed in the Table below, the amine products were obtained.

| Prep Ex. | Aldehyde | Grignard Reagent | Amine | Yield (%) |
|---|---|---|---|---|
| 51.25 | 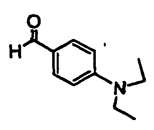 | EtMgBr | 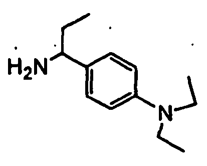 | 20% |
| 51.26 | 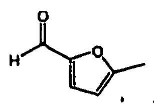 | 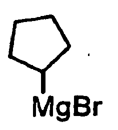 | 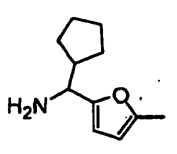 | 77% |
| 51.27 | 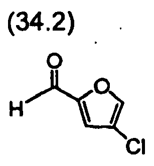 | EtMgBr | 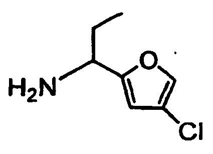 | 51% |
| 51.28 | 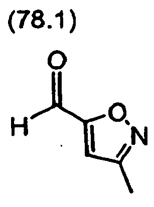 | 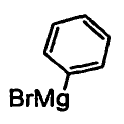 | 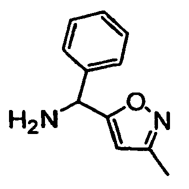 | 56% |
| 51.29 | 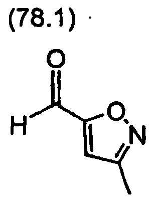 | 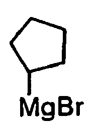 | 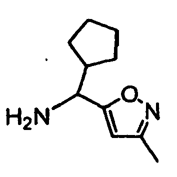 | 54% |
| 51.30 | 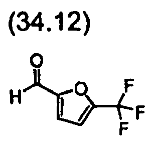 | EtMgBr | 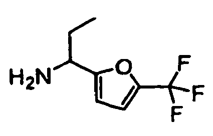 | 80% |
| 51.31 | 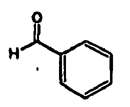 | 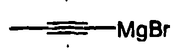 | 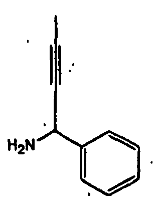 | 10% |

### PREPARATIVE EXAMPLE 52

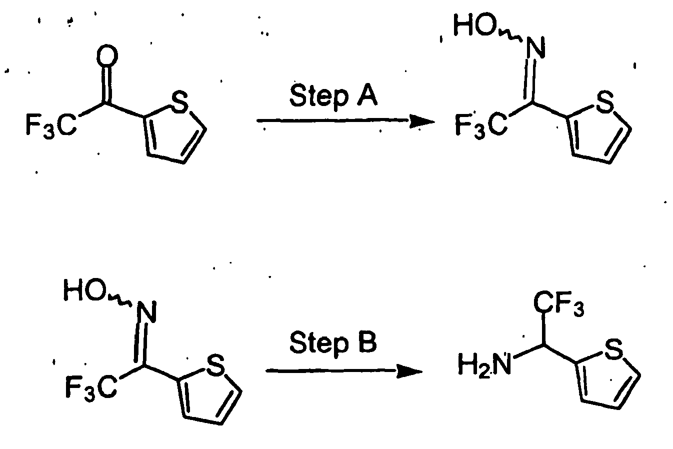

### Step A

A mixture of 2-(trifluoroacetyl)thiophene (2 mL, 15.6 mmol), hydroxylamine hydrochloride (2.2 g, 2 eq), diisopropylethylamine (5.5 mL, 2 eq) and MeOH (50 mL) was stirred at reflux for 48-72 hrs, then concentrated *in vacuo.* The residue was diluted with EtOAc, washed with 10% KH₂PO₄ and dried over Na₂SO4 (anhydrous). Filtration and concentration afforded the desired oxime (2.9 g, 96%) which was used directly in Step B without further purification.

### Step B

To a mixture of the product from Step A above in TFA (20 mL) was added Zn powder (3 g, 3 eq) portionwise over 30 min and stirred at room temperature overnight. The solid was filtered and the mixture reduced *in vacuo.* Aqueous NaOH (2 *M*) was added and the mixture was extracted several times with CH₂Cl₂. The organic phase was dried over anhydrous Na₂SO₄, filtered and concentrated to afford the desired product (1.4 g, 50%).

### PREPARATIVE EXAMPLES 53-61

Following the procedure set forth in Preparative Example 52 but using the commercially available ketones listed in the Table below, the following amines were obtained.

| Prep | Ketone | Amine | 1. Yield (%) |
|---|---|---|---|
| Example | | | 2. MH⁺ |
| 53 | 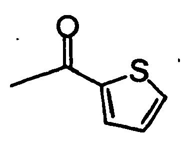 | 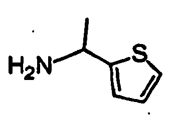 | 1. 11% |
| | | | 2. 128 |
| 54 | 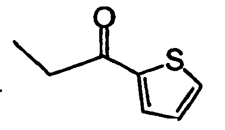 | 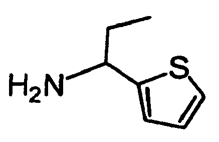 | 1. 33% |
| | | | 2. 142 |
| 55 | 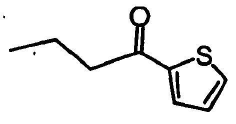 | 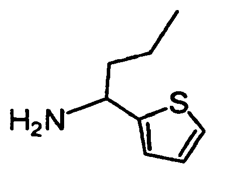 | 1. 49% |
| | | | 2. 156 |
| 56 | 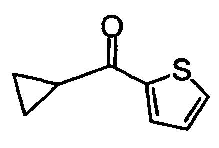 | 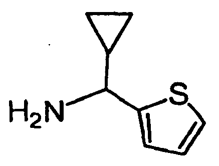 | 1. 5% |
| | | | 2. 154 |
| 57 | 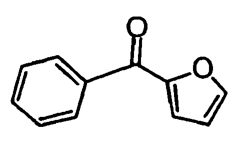 | 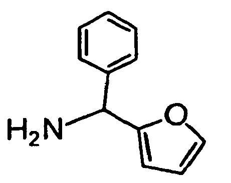 | 1. 47% |
| | | | 2. 174 |
| 58 | 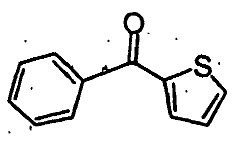 | 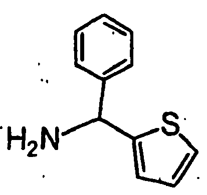 | 1. 71% |
| | | | 2. 190 |
| 59 | 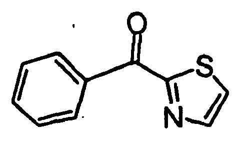 | 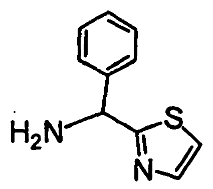 | 1. 78% |
| | | | 2. 191 |
| 60 | 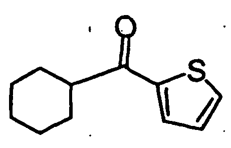 | 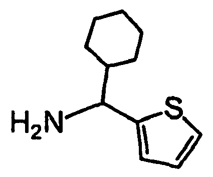 | 1. 80% |
| | | | 2. 190 |
| 61 | 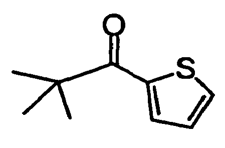 | 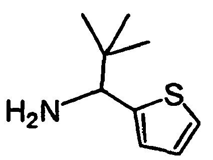 | 1. 9% |
| | | | 2. 156 |

### PREPARATIVE EXAMPLE 62

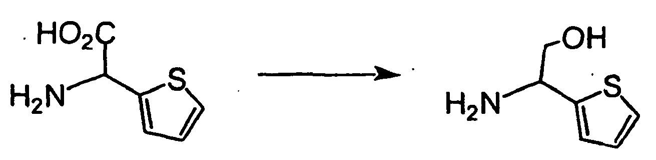

To a cooled (0-5°C) suspension of L-α-(2-thienyl)glycine (0.5 g) and LiBH₄ (2M in THF, 3.8 mL) in anhydrous THF (10 mL) was slowly added a THF (5 mL) solution of iodine (0.8.g). After stirring at room temperature for 15 min, the mixture was stirred at relux overnight. After cooling to room temperature, MeOH was added dropwise until gas evolution ceased and after 30 min, the mixture was evaporated. The oily residue was stirred in 20 mL KOH for 4 hrs, diluted with brine and extracted with EtOAc.

The organic phase was dried over anhydrous MgSO₄, filtered and concentrated *in vacuo* to afford a crude mixture: Purification by.flash column chromatography (50% EtOAc/'CH₂CI₂, silica) afforded the product (0.3 g, 63%, MH⁺ = 144).

### PREPARATIVE EXAMPLE 63

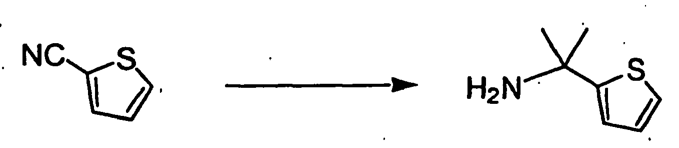

CeCl₃-7H₂O was dried at 140-150°C for 22 hr. To this solid was added THF (80 mL, anhydrous) and after stirring for 2 hr, the suspension was cooled to -78°C and to it was added methyl lithium over 30 min. After stirring for an additional 30 min 2-thiophenecarbonitrile dissolved in anhydrous THF (4.5 mL) was added and the resulting mixture stirred for an additional 4.5 hr at -78°C. Concentrated aqueous NH₃ (25 mL) was added and the mixture was warmed to room temperature and filtered through celite. The filtrate was extracted with dichloromethane, dried over anhydrous Na₂SO₄, filtered and concentrated *in vacuo* to afford a crude mixture. Purification by flash column chromatography (5% MeOH, CH₂Cl₂, silica) afforded the desired product (1.2 g, 62%).

### PREPARATIVE EXAMPLE 64

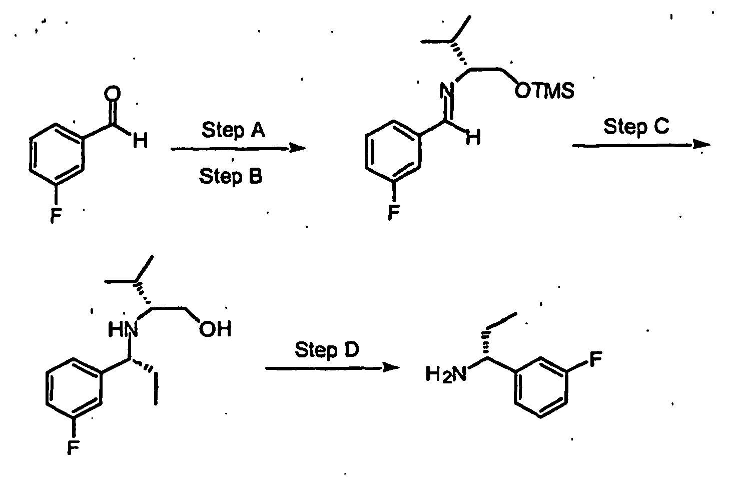

### Step A

To a solution of (D)-valinol (4.16 g, 40.3 mmol) in CH₂Cl₂ (60 mL) at 0 °C was added MgSO₄ (20 g) followed by dropwise addition of 3-fluorobenzaldehyde (5.0 g, 40.3 mmol). The heterogenous solution was stirred at 0°C for 2h and was allowed to warm to room temperature and stir overnight (14h). The mixture was filtered and the drying agent was washed with CH₂Cl₂ (2 x 10 mL). The filtrate was concentrated under reduced pressure to afford 8.4 g (100%) of an oil which was taken onto the next step without further purification.

### Steg B

To a solution of the imine (8.4 g, 40.2 mmol) from Step A in CH₂Cl₂ (60 mL) at room temperature was added Et₃N (6.2 mL, 44.5 mmol) followed by dropwise addition of TMSCI (5.7 mL, 44.5 mmol). The mixture was stirred for 6h at room temperature whereupon the ppt that had formed was filtered off and washed with CH₂Cl₂ (2x10 mL). The combined filtrate was concentrated under reduced pressure and was taken up in Et₂O/hexane (1:1/150 mL). The precipitate was filtered off and the filtrate was concentrated under reduced pressure to afford 10.1 g (89%) of the protected imine as an oil. This material was taken onto the next step without further purification.

### Step C

To a solution of Etl (4.0 g, 25.6 mmol) in Et₂O (40 mL) at -78 °C was added t-BuLi (30.1 mL, 51.2 mmol, 1.7 M in pentane) and the mixture was stirred for 10 min. The mixture was warmed to room temperature, stirred for 1 h, and was recooled to -40 °C. A solution of the imine (6.0 g, 21.4 mmol) from Step B in Et₂O (30 mL) was added dropwise via addition funnel to afford a bright orange mixture. The reaction mixture was stirred for 1.5 h at -40 °C then 3M HCl (50 mL) was added and the mixture was allowed to warm to room temperature. Water (50 mL) was added and the layers were separated. The aqueous layer was extracted with Et₂O (2 x 30 mL) and the organic layers were combined and discarded. The aqueous layer was cooled to 0 °C and carefully treated with solid NaOH pellets until pH = 12 was attained. The aqueous layer was extracted with Et₂O (3 x 30 mL) and the combined layers were washed with brine (1 x 30 mL). The organic layer was dried (Na₂SO₄), filtered, and concentrated under reduced pressure to afford 4.8 g (94% yield) of the amine as an oil. This material was taken on crude to the next step without further purification.

### Steg D

To a solution of amine (4.5 g, 18.8 mmol) from Step C in MeOH (80 mL) at room temperature was added MeNH₂ (25 mL, 40% in water) followed by addition of a solution of H₅lO₆ (14.0 g, 61.4 mmol) in H₂O (25 mL). The heterogenous mixture was stirred for 1.5 h (until the reaction was complete by TLC) and the precipitate was filtered off. The resulting filtrate was diluted with water (50 mL) and the mixture was extracted with Et₂O (4 x 60 mL). The combined organic layers were concentrated to a volume of ~30 mL whereupon 3M HCl (75 mL) was added. The mixture was stirred overnight (12h at room temperature) after which the mixture was concentrated to remove the volatiles. The aqueous layer was extracted with Et₂O (3 x 40 mL) and the organic layers were discarded. The aqueous layer was cooled to 0 °C and was carefully treated with solid NaOH pellets until pH ∼12 was reached. The aqueous layer was extracted with Et₂O (3 x 60 mL) and the combined organic layers were dried (MgSO₄). The organic layer was concentrated under reduced pressure to afford 2.8 g (97% yield) of the desired amine as an oil [MH⁺ 154]. This compound was proven to be >85% pure by ¹H NMR and was used crude in the subsequent coupling step.

### PREPARATIVE EXAMPLES 65-75.10

Following the procedure set forth in Preparative Example 64 but using the commercially available aldehydes, amino alcohols, and organolithium reagents in the Table below, the optically pure amine products in the Table below were obtained.

| Prep | Aldehyde | Amino | Organo | Product | 1.Yield (%) |
|---|---|---|---|---|---|
| Ex. | | Alcohol | lithium | | 2. MH⁺ |
| 65 | 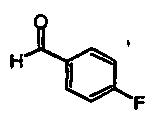 | 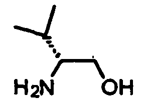 | EtLi | 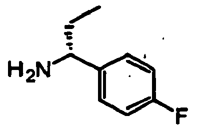 | 1. 62% |
| | | | | | 2. 154 |
| 66 | 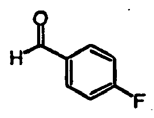 | 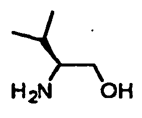 | EtLi | 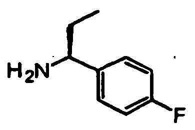 | 1. 70% |
| | | | | | 2. 154 |
| 67 | 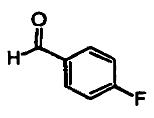 | 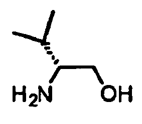 | 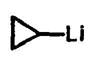 | 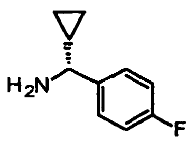 | 1. 54% |
| | | | | | 2. 166 |
| 68 | 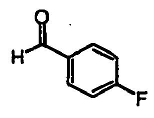 | 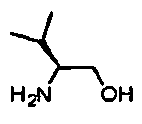 | 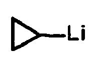 | 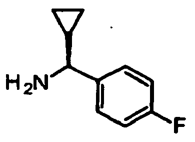 | 1. 67% |
| | | | | | 2. 166 |
| 69 | 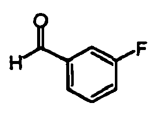 | 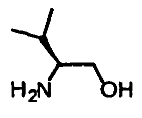 | EtLi | 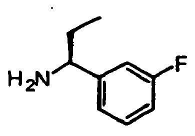 | 1. 67% |
| | | | | | 2. 154 |
| 70 | 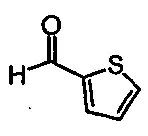 | 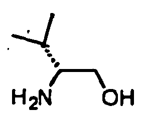 | EtLi | 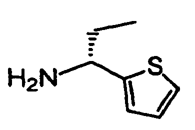 | 1. 42% |
| | | | | | 2. 142 |
| 71 | 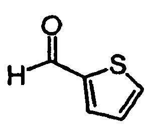 | 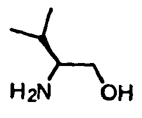 | EtLi | 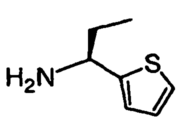 | 1. 36% |
| | | | | | 2. 142 |
| 72 | 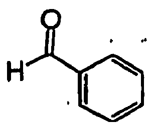 | 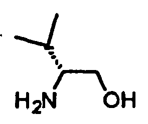 | 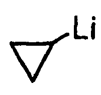 | 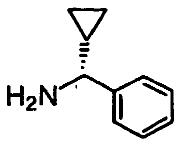 | 1. 62% |
| | | | | | 2. 148 |
| 73 | 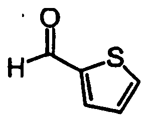 | 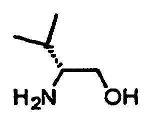 | t-BuLi | 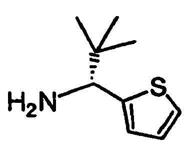 | 1. 27% |
| | | | | | 2. 256 |
| 74 | 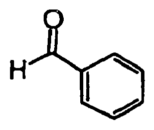 | 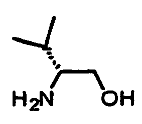 | t-BuLi | 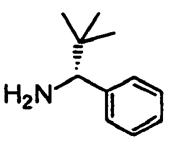 | 1. 15% |
| | | | | | 2. 164 |
| 75 | 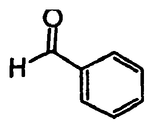 | 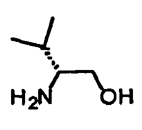 | 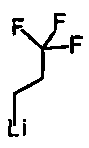 | 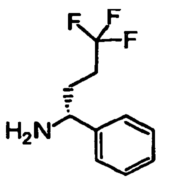 | 1. 7% |
| | | | | | 2. 204 |
| 75.1 | 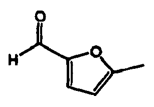 | 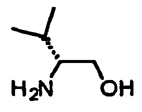 | EtLi | 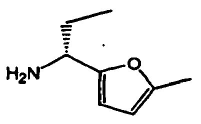 | 1. 65% |
| | | | | | 2. 123 [M-NH₂]⁺ |
| 75.2 | 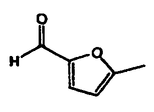 | 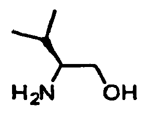 | EtLi | 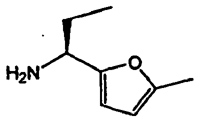 | 1. 62% |
| | | | | | 2. 123 [M-NH₂]⁺ |
| 75.3 | 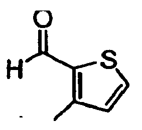 | 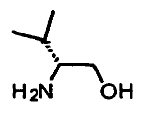 | EtLi | 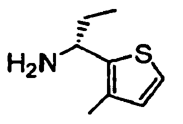 | 1. 93% |
| | | | | | 2. 139 [M-NH₂]⁺ |
| 75.4 | 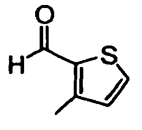 | 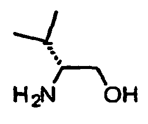 | tBuLi | 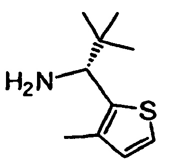 | 1. 50% |
| | | | | | 2. 167 [M-NH₂]⁺ |
| 75.5 | 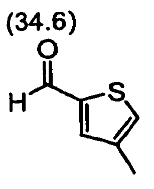 | 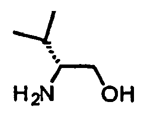 | tBuLi | 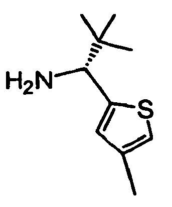 | 1. 48% |
| | | | | | 2. 167 [M-NH₂]⁺ |
| 75.6 | 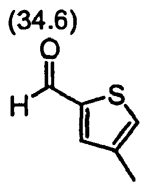 | 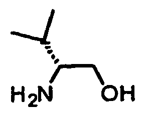 | EtLi | 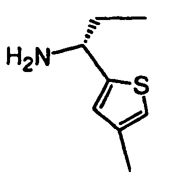 | 1. 97% |
| | | | | | 2. 139 [M-NH₂]⁺ |
| 75.7 | 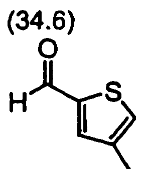 | 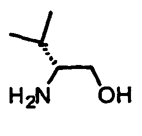 | iPrLi | 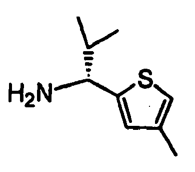 | 1. 87% |
| | | | | | 2. 153 [M-NH₂]⁺ |
| 75.8 | 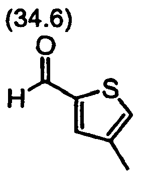 | 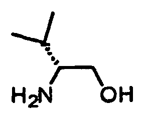 | 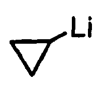 | 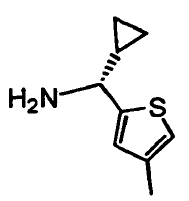 | 1. 94% |
| | | | | | 2. 151 [M-NH₂]⁺ |
| 75.9 | 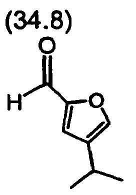 | 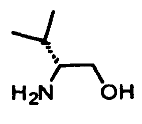 | EtLi | 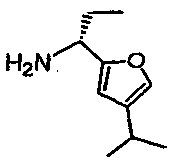 | 1. 75% |
| | | | | | 2. 151 [M-NH₂]⁺ |
| 75.10 | 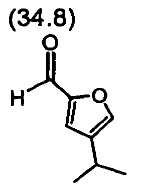 | 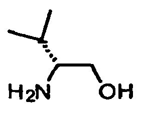 | tBuLi | 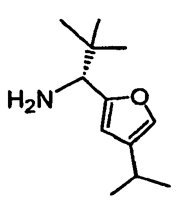 | 1. 30% |
| | | | | | 2. 179 [M-NH₂]⁺ |

### PREPARATIVE EXAMPLES 75.11-75.59

Following the procedure set forth in Preparative Example 64 but using the prepared or commercially available aldehydes, amino alcohols, and organolithium reagents in the Table below and carrying the amine on crude, the optically pure amine products in the Table below were obtained.

| Prep Ex. | Aldehyde | Amino Alcohol | Organo lithium | Product | Yield (%) |
|---|---|---|---|---|---|
| 75.11 | 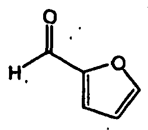 | 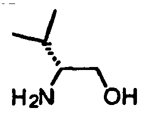 | 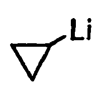 | 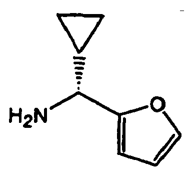 | 52% |
| 75.12 | 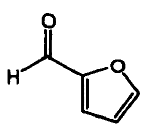 | 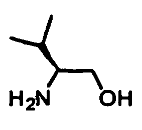 | 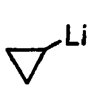 | 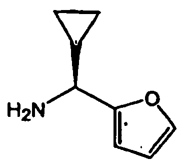 | 50% |
| 75.13 | 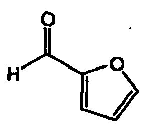 | 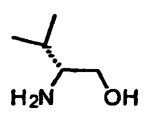 | iPrLi | 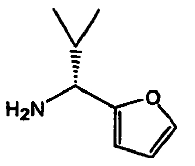 | 57% |
| 75.14 | 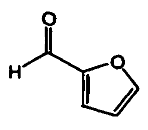 | 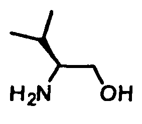 | iPrLi | 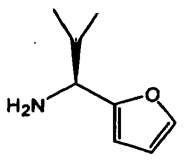 | 54% |
| 75.15 | 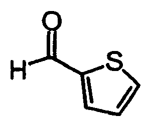 | 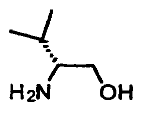 | iPrLi | 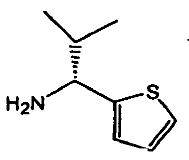 | 58% |
| 75.16 | 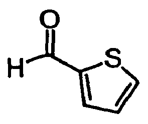 | 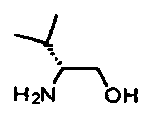 | 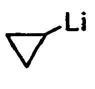 | 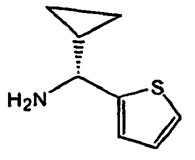 | 61% |
| 75.17 | 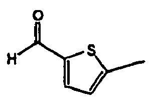 | 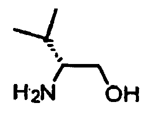 | EtLi | 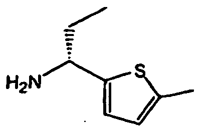 | 72% |
| 75.18 | 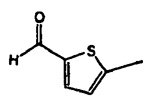 | 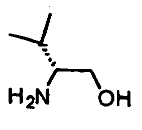 | 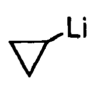 | 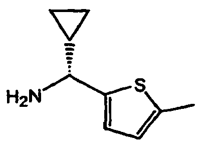 | 68% |
| 75.19 | 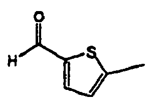 | 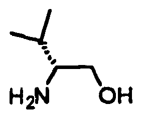 | iPrLi | 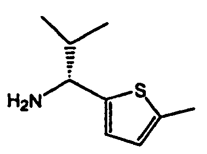 | 77% |
| 75.20 | 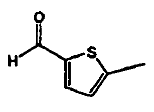 | 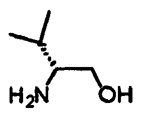 | t-BuLi | 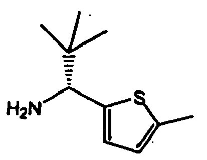 | 15% |
| 75.21 | 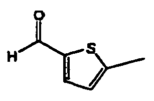 | 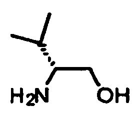 | MeLi | 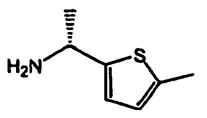 | 50% |
| 75.22 | 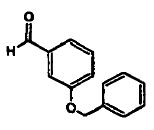 | 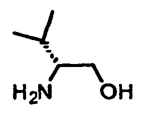 | EtLi | 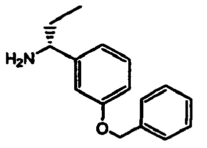 | 23% |
| 75.24 | 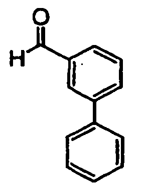 | 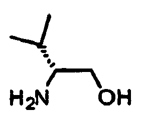 | EtLi | 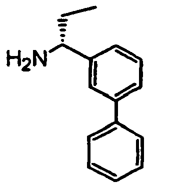 | 20% |
| 75.27 | 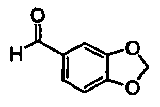 | 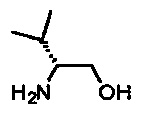 | EtLi | 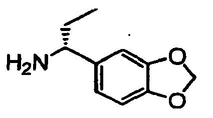 | 65% |
| 75.28 | 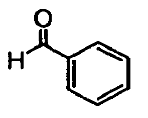 | 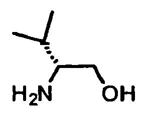 | iPrli | 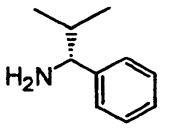 | 61% |
| 75.29 | 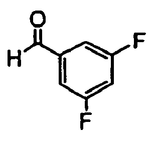 | 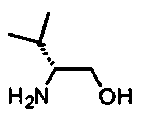 | EtLi | 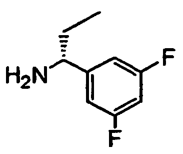 | 90% |
| 75.30 | 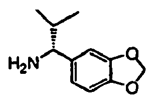 | 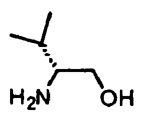 | iPrLi | 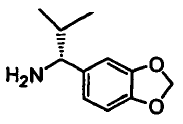 | 62% |
| 75.31 | 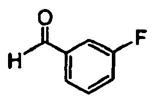 | 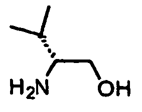 | iPrLi | 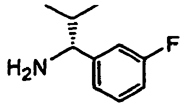 | 43% |
| 75.32 | 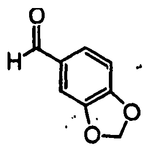 | 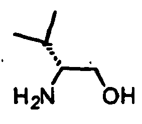 | 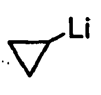 | 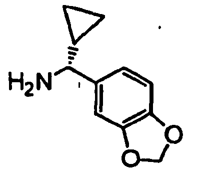 | 50% |
| 75.33 | 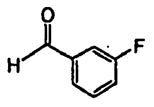 | 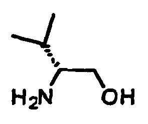 | 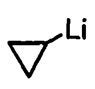 | 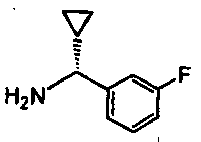 | 50% |
| 75.34 | 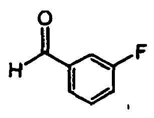 | 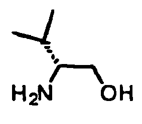 | tBuLi | 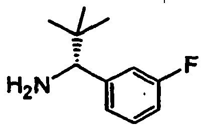 | 51% |
| 75.35 | 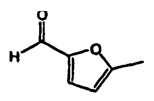 | 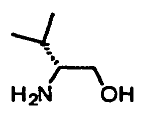 | MeLi | 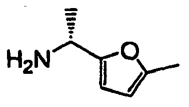 | 51% |
| 75.36 | 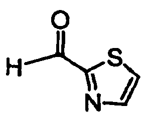 | 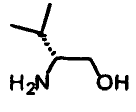 | tBuLi | 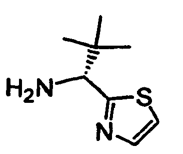 | 57% |
| 75.37 | 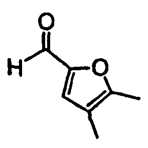 | 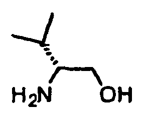 | tBuLi | 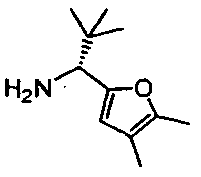 | 60% |
| 75.38 | 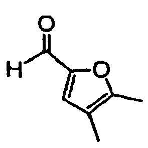 | 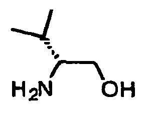 | EtLi | 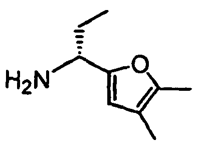 | 73% |
| 75.39 | 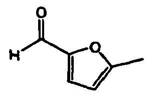 | 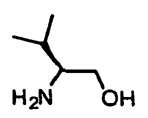 | MeLi | 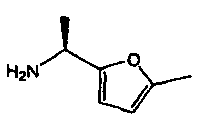 | 48% |
| 75.41 | 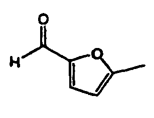 | 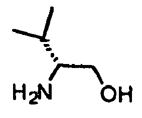 | 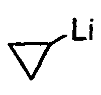 | 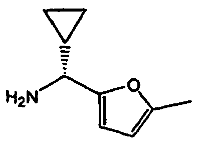 | 52% |
| 75.42 | 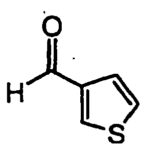 | 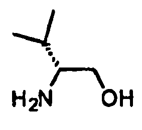 | EtLi | 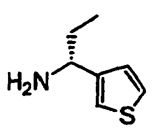 | 40% |
| 75.43 | 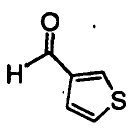 | 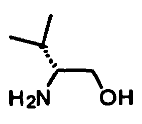 | tBuLi | 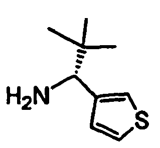 | 20% |
| 75.44 | 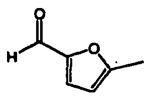 | 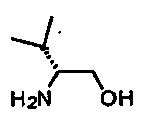 | t-BuLi | 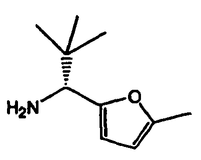 | 79% |
| 75.45 | 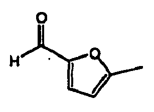 | 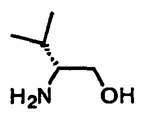 | iPrLi | 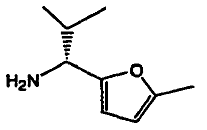 | 55% |
| 75.46 | 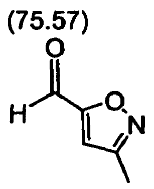 | 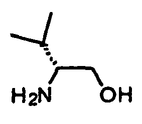 | tBuLi | 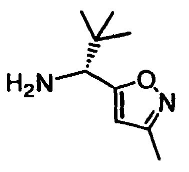 | 39% |
| 75.47 | 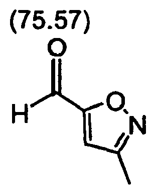 | 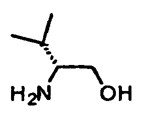 | iPrLi | 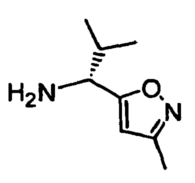 | 55% |
| 75.48 | 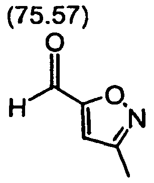 | 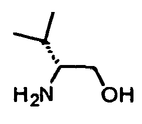 | 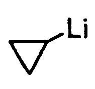 | 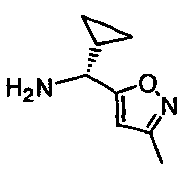 | 34% |
| 75.49 | 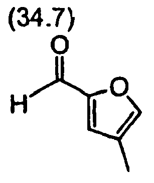 | 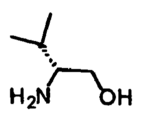 | EtLi | 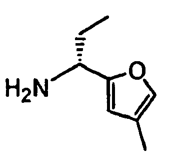 | 61% |
| 75.50 | 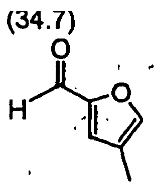 | 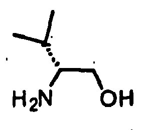 | tBuLi | 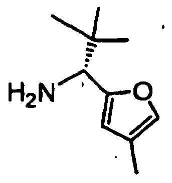 | 25% |
| 75.51 | 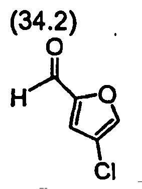 | 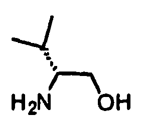 | iPrLi | 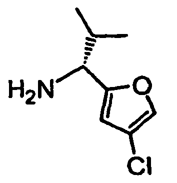 | 33% |
| 75.52 | 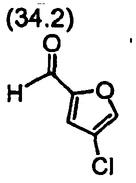 | 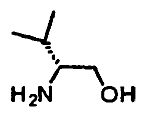 | tBuLi | 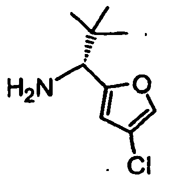 | 30% |
| 75.53 | 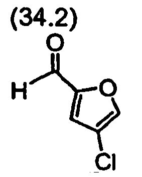 | 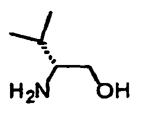 | EtLi | 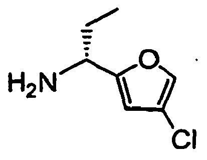 | 39% |
| 75.54 | 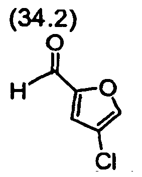 | 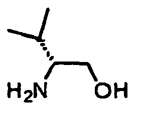 | 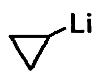 | 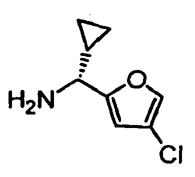 | 38% |
| 75.55 | 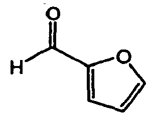 | 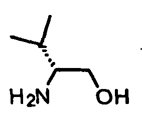 | EtLi | 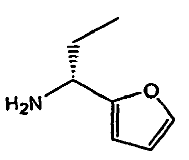 | 64% |
| 75.56 | 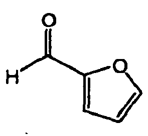 | 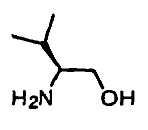 | EtLi | 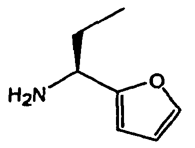 | 46% |
| 75.57 | 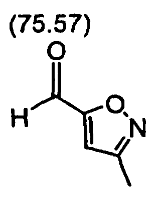 | 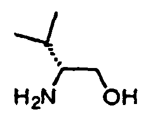 | EtLi | 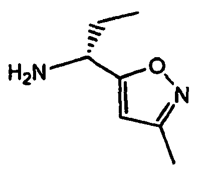 | 62% |
| 75.58 | 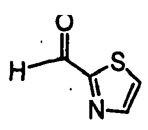 | 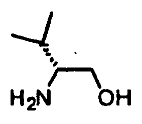 | iPrLi | 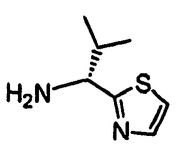 | 24% |
| 75.59 | 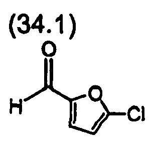 | 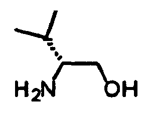 | EtLi | 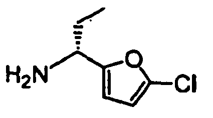 | 70% |

### PREPARATIVE EXAMPLE 75.75

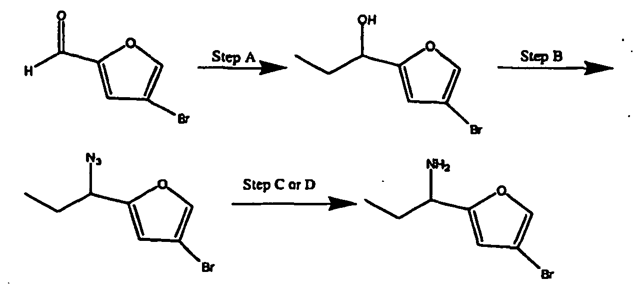

### Step A

To a solution of aldehyde (2.5g) in ether (50ml) at 0°C was added EtMgBr (4.56ml) dropwise. The heterogenous mixture was stirred for 2hr at 0°C and then poured into a beaker of saturated ammonium chloride (25ml), ice and CH₂Cl₂ (30ml). After the biphasic mixture stirred for 10min, the organic layer was separated, washed with brine, dried over Na₂SO₄, filtered, and concentrated *in vacuo* to afford the product (2.41 g, 95%)

### Step B

To a solution of alcohol from Step A above (1g) in toluene at room temperature was added DPPA. The mixture was cooled to 0°C and DBU was added and let stir for 12hr at room temperature. The layers were separated and the organic layer was washed with water, 1N HCl and dried over Na₂SO₄, filtered, and concentrated *in vacuo.* Purified by preparative plate chromatography (hexane/EtOAc 20/1) to give the product (840mg, 75%).

### Step C

To a solution of azide (730mg) from Step B above in THF (7ml) was added PPh₃ (1g). The heterogenous solution was stirred for 12hr, whereupon water (1.5m1) was added. The mixture was refluxed overnight, cooled to room temperature and concentrated *in vacuo.* Ether and 1N HCl were added to the residue. The aqueous layer was cooled to 0°C, basified with NaOH pellets and extracted with ether. The ether layer was dried over MgSO₄, filtered, and concentrated *in vacuo* to afford the product (405mg, 62%).

### Step D

To a solution of azide in THF at -10°C was added LiAlH₄ portionwise. The heterogenous solution was stirred at room temperature for 1 hr and then refluxed for 4hr. The solution was cooled to 0°C and water, 2M NaOH and ether were added to the reaction. The mixture was filtered through a celite pad. The filtrate was treated with 3N HCl. The aqueous layer was cooled to 0°C, basified with NaOH pellots and extracted with ether. The ether layer was dried over MgSO₄, filtered, and concentrated *in vacuo* to afford the product.

### PREPARATIVE EXAMPLE 75.76-75.90

Following a similar procedure set forth in Preparative Example 75.75, and using the reduction procedure indicated, the following amines were obtained.

| Prep Ex. | Aldehyde | Reducing Step | Product | % Yield |
|---|---|---|---|---|
| 75.76 | 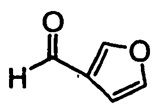 | D | 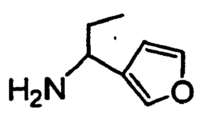 | 43% |
| 75.77 | 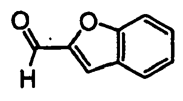 | C | 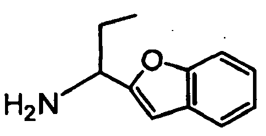 | 36% |
| 75.78 | 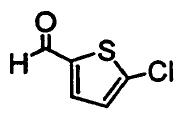 | D | 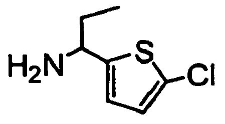 | 32% |
| 75.79 | 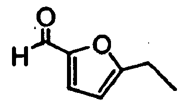 | C | 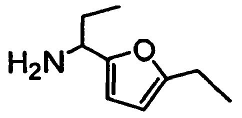 | 42% |
| 75.80 | 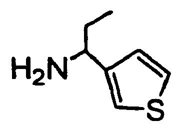 | D | 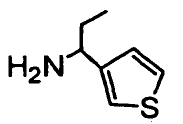 | 56% |
| 75.81 | 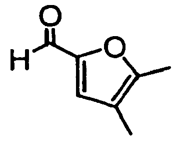 | D | 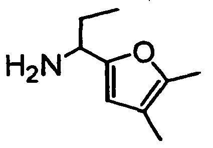 | 35% |
| 75.82 | 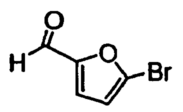 | C | 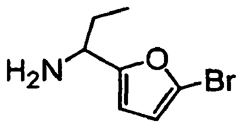 | 13% |
| 75.83 | 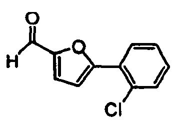 | C | 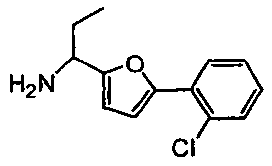 | 42% |
| 75.84 | 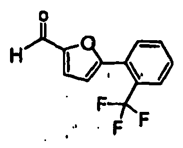 | C | 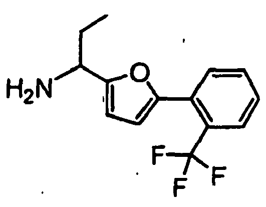 | 39% |
| 75.85 | 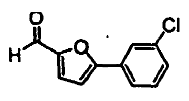 | C | 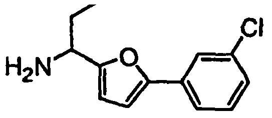 | 26% |
| 75.86 | 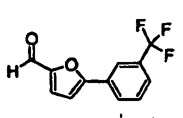 | c | 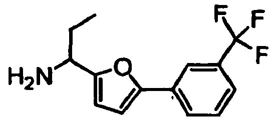 | 25% |
| 75.87 | 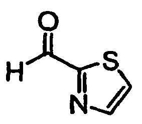 | C | 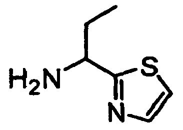 | 14% |
| 75.88 | 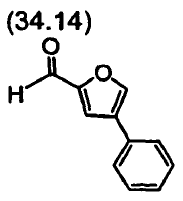 | C | 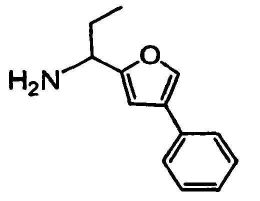 | 49% |
| 75.89 | 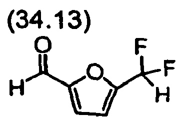 | C | 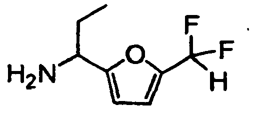 | 34% |
| 75.90 | 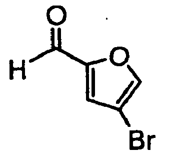 | C | 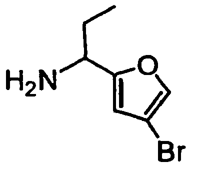 | 44% |

### PREPARATIVE EXAMPLE 76

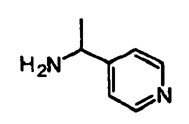

The desired compound was prepared according to methods previously described in J. Med. Chem. 1996, 39, 3319-3323.

### PREPARATIVE EXAMPLE 76.1

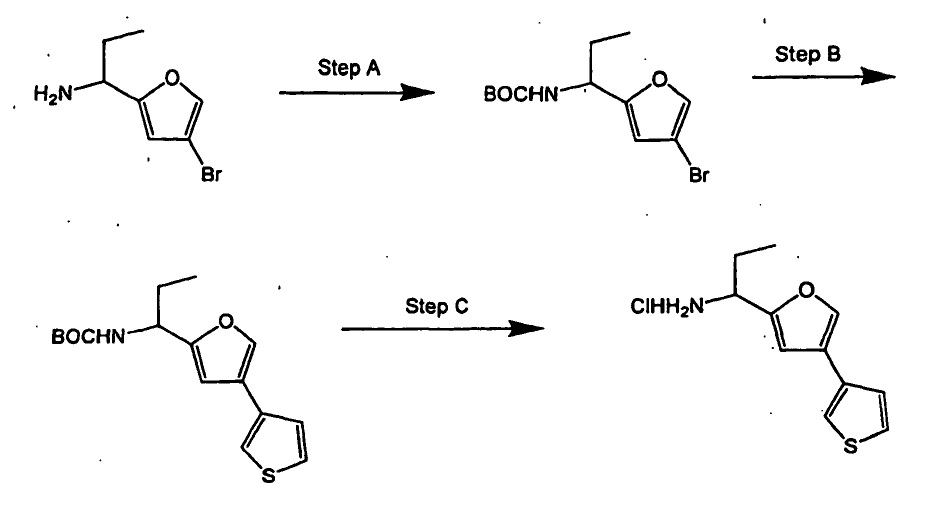

### Step A

To a solution of amine from Preparative Example 75.90 (2.22g) in CH₂Cl₂ (50ml) at 0°C was added TEA (3.03ml) followed by BOC₂O (2.85g). The heterogenous mixture was allowed to stir at room temperature overnight. 10% Citric acid was added to the reaction and the layers were separated. The organic layer was washed with saturated sodium bicarbonate, brine and dried with Na₂SO₄, filtered, and concentrated *in vacuo.* The crude material was purified by flash column chromatography (Hex/EtOAc 10:1) to afford 2.7g of an oil (81%).

### Step B

Following the procedure from Preparative Example 13.4, Step A, but using the product from Step A above (450mg) and 3-thiophene boronic acid (284mg), the product was prepared (325mg, 71%).

### Step C

To the product from Step B (325g) was added 4M HCl in dioxane (1.31 ml) and let stir for 1hr. The reaction was concentrated *in vacuo* and taken up in CH₂Cl₂ and concentrated *in vacuo* again. This procedure was repeated 5 times to afford a semisolid (89%).

### PREPARATIVE EXAMPLE 76.2-76.3

Following the procedures set forth in Preparative Example 76.1, but using the commercially available boronic acids, the indicated amines were prepared.

| Prep Ex. | Boronic Acid | Product | Yield (%) |
|---|---|---|---|
| 76.2 | 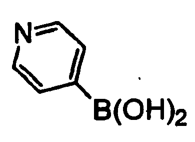 | 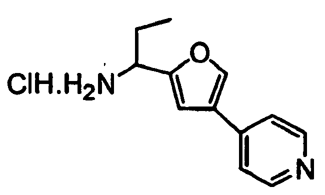 | 70% |
| 76.3 | 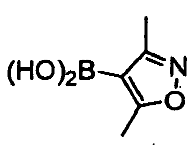 | 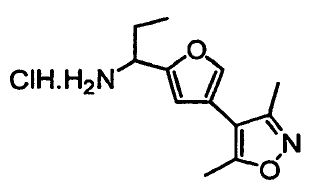 | 35% |

### PREPARATIVE EXAMPLE 76.10

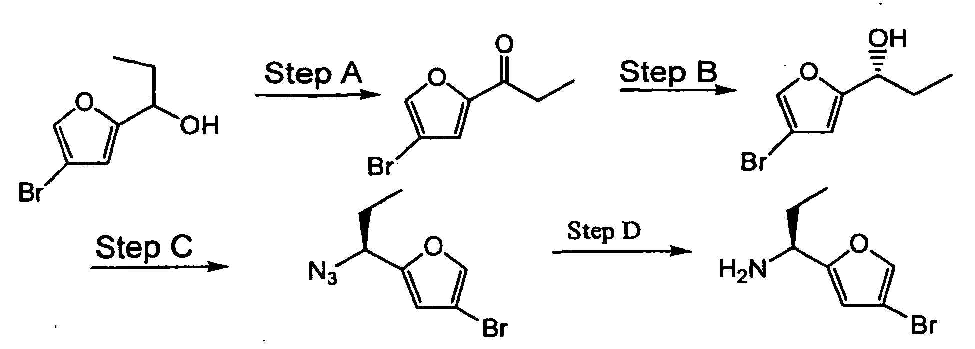

### Step A

The product from Preparative Example 75.75, Step A (2.5g) was reacted via the Preparative Example 13.11, Step B to give the ketone (1.93g, 78%).

To a solution of ketone from Step A above (500mg) in THF (5ml) at 0°C was added S-2-methyl-CBS-oxazaborolidine (0.98ml) dropwise followed by BH₃Me₂S (1.48ml). The mixture was stirred at 0°C for 2hr and was allowed to warm to room temperature and stir overnight. The mixture was cooled to 0°C and treated with MeOH (10ml). After stirring for 20min, the reaction was concentrated *in vacuo.* The residue was dissolved in CH₂Cl₂ and washed with 1M HCl, saturated sodium bicarbonate, water and brine, dried over Na₂SO₄, filtered, and concentrated *in vacuo.* The crude material was purified by preparative plate chromatography (Hex/EtOAc 4:1) to afford 650mg of an oil (89%).

### Step C

The chiral alcohol from Step B above was reacted via the Preparative Example 75.75 Step B to give the azide.

### Step D

The azide from Step C above was reacted via the Preparative Example 75.75 Step C to give the amine product.

### PREPARATIVE EXAMPLE 76.11

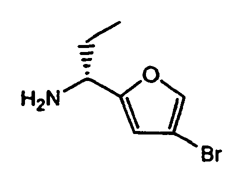

The desired compound was prepared as in Preparative Example 76.10, but using the R-2-methyl-CBS-oxazaborolidine in step B.

### PREPARATIVE EXAMPLE 77

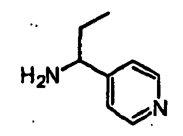

The desired compound was prepared according to methods previously described in J. Med. Chem. 1996, 39, 3319-3323.

### PREPARATIVE EXAMPLE 78

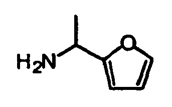

The desired compound was prepared according to methods previously described in Chem. Pharm. Bull. 1991, 39, 181-183.

### PREPARATIVE EXAMPLE 78.1

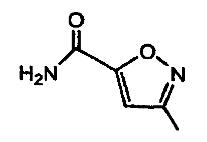

The desired compound was prepared according to methods previously described in J. Organometallic Chem. 1998, 567, 31-37.

### PREPARATIVE EXAMPLE 79

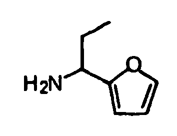

The desired compound was prepared according to methods previously described in Chem. Pharm. Bull. 1991, 39, 181-183.

### PREPARATIVE EXAMPLE 80

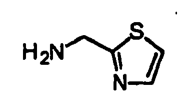

The desired compound was prepared according to methods previously described in a) Synthesis 1987, 998-1001, b) Synthesis 1996, 641-646 and c) J. Med. Chem. 1991, 34, 2176-2186.

### PREPARATIVE EXAMPLE 81

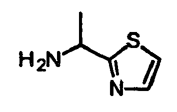

The desired compound was prepared according to methods previously described in a) Synthesis 1987, 998-1001, b) Synthesis 1996, 641-646 and c) J. Med. Chem. 1991, 34, 2176-2186.

### PREPARATIVE EXAMPLE 82

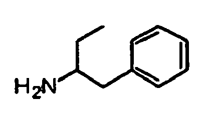

The desired compound was prepared according to methods previously described in J. Med. Chem. 1988, 31, 2176-2186.

### PREPARATIVE EXAMPLE 83

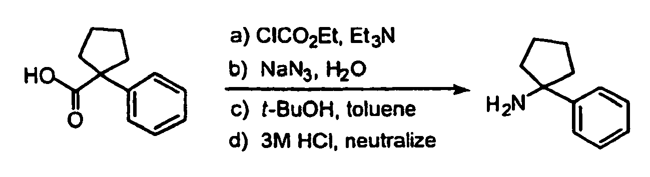

To a solution of carboxylic acid (1.5 g, 7.89 mmol) in H₂O/acetone (1:10/12 mL total) at 0°C was added Et₃N (1.43 mL, 10.3 mmol) followed by addition of ethyl chloroformate (0.83 mL, 8.68 mmol). The resulting mixture was stirred for 30 min after which a solution of NaN₃ (0.77g, 11.8mmol) in H₂O (2 mL) was added dropwise. The resultant heterogenous mixture was stirred for 1 h at 0°C, then cold water (5 mL) and Et₂O (10 mL) were added. The layers were separated and the aqueous layer was extracted with Et₂O (2×10 mL). The organic layers were combined, toluene (20 mL) was added, and the organic layers were dried (MgSO₄) and concentrated under reduced pressure to a volume of 20 mL. *t*-BuOH (5 mL) was added and the mixture was refluxed for 12h. The mixture was concentrated under reduced pressure and the crude residue was taken up in 3M HCl (30 mL) and was heated at reflux for 12h. The mixture was cooled to room temperature and extracted with Et₂O (3 x 15 mL). The aqueous layer was cooled to 0 °C and solid NaOH pellets were added until pH ~12 was reached. The aqueous layer was extracted with Et₂O (3 x 30 mL) and the combined organic layers were dried (MgSO₄) and concentrated under reduced pressure to afford 0.78 g (61% yield) of an oil [MH⁺ 162]. This material was used without further purification.

### PREPARATIVE EXAMPLE 84

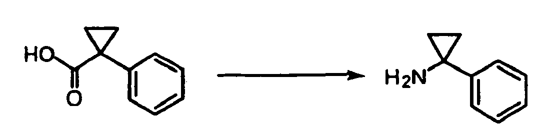

The corresponding cyclopropyl analog was prepared according to the procedure outlined in Preparative Example 83.

### PREPARATIVE EXAMPLE 85

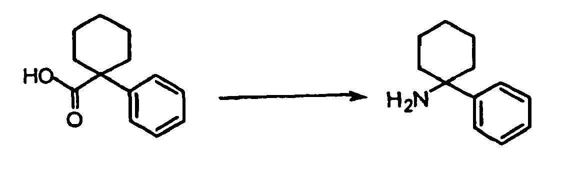

The corresponding cyclohexyl analog was prepared according to the procedure outlined in Preparative Example 83.

### PREPARATIVE EXAMPLE 86

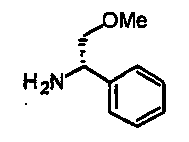

The desired compound was prepared according to methods previously described in J. Org. Chem. 1978, 43, 892-898.

### PREPARATIVE EXAMPLE 87

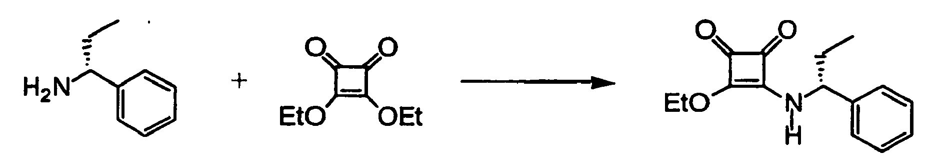

A mixture of (R)-(+)phenylpropanolamine (8.2 g), 3,4-diethoxy-3-cyclobutene-1,2-dione (10 g) and absolute EtOH (75 mL) was stirred at 0-25°C for 12 hrs. Filtration and concentration of the filtrate gave a syrup which was chilled in the freezer to give a solid. Trituration of the solid with diethyl ether gave the desired product (10.5 g, 71%, MH⁺ = 260).

### PREPARATIVE EXAMPLE 87.1

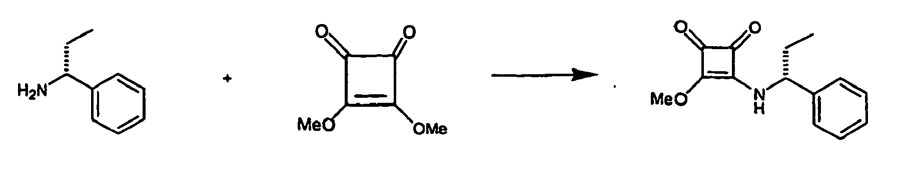

(R)-1-phenyl propylamine (4.82ml) and 3,4-dimethoxy-3-cylclobutene-1,2-dione (5.03g) were combined in MeOH (40ml) and stirred overnight. Reaction concentrated *in vacuo* and purified via flash column chromatography (MeOH/CH₂Cl₂, 1:40) to yield 2.75g of product (31%, MH+=246).

### PREPARATIVE EXAMPLE 88

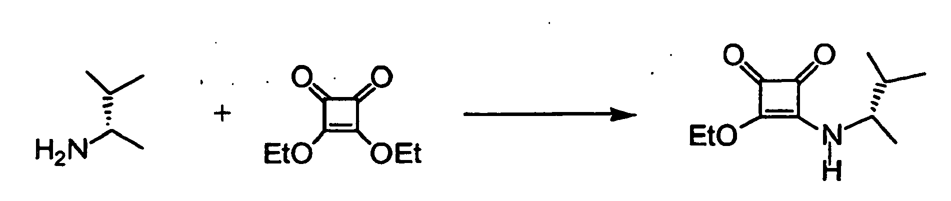

A mixture of (S)-(+)-3-methyl-2-butylamine (3.0 g), 3,4-diethoxy-3-cyclobutene-1,2-dione (5 g) and absolute EtOH (100 mL) was stirred at 0-25°C for 12 hrs. Filtration and concentration of the filtrate gave a syrup which solidified upon dilution with Et₂O. Trituration of the solid with diethyl ether gave the desired product as a solid (4.4 g, 72%, MH⁺ = 212).

### PREPARATIVE EXAMPLE 88.1

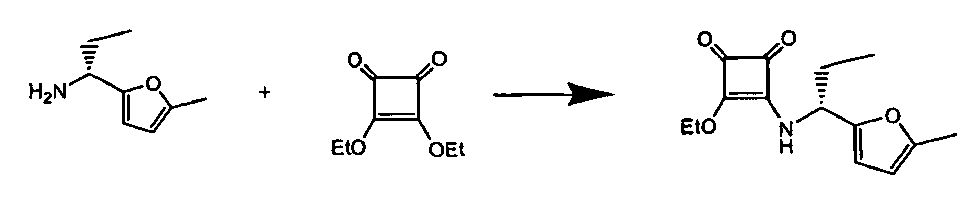

A mixture of amine from Preparative Example 75.1 (370mg), 3,4-diethoxy-3-cyclobutene-1,2-dione (0.39ml) and absolute EtOH (5ml) was stirred at room temperature overnight. Purification by preparative plate chromatography (3% EtOH/CH₂Cl₂) afforded the desired product (263mg, 37%).

### PREPARATIVE EXAMPLE 88.2

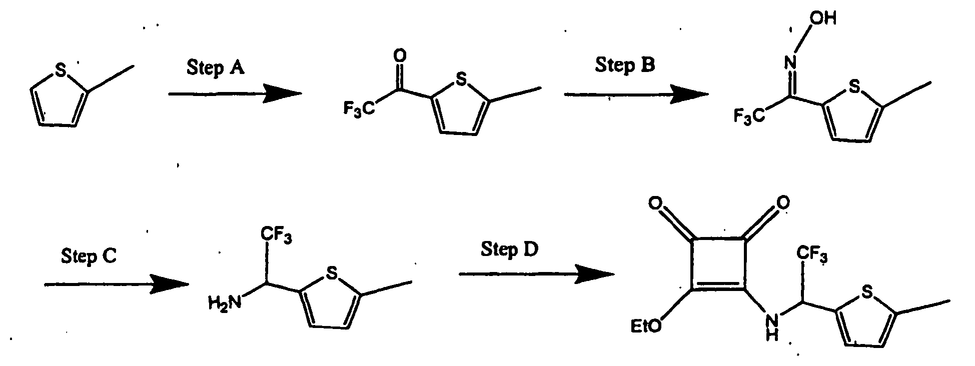

### Step A

2-Methylthiophene (3g) was dissolved in THF and cooled to -40°C. N-butyllithium (2.5M in hexane,12.24ml) added dropwise and let stir at -40°C for 30min. CuBr.(CH₃)₂S (6.29g) added and let warm to -25°C where the trifluoroaceticanhydride (4.32ml) was added. The reaction was stirred at -15°C over the weekend. The reaction was quenched with saturated ammonium chloride and extracted with EtOAc. The organic layer washed with brine, dried with MgSO₄, filtered and concentrated *in vacuo* to give 4.59g of an oil (78%).

### Step B

The product from Step A (4.58g), hydroxylamine hydrochloride (3g), sodium acetate (4.4g), EtOH (75ml) and H₂O (7.5ml) were combined and heated to 75°C overnight. The reaction was concentrated *in vacuo*, taken up 1N HCl, extracted with ether, dried with MgSO₄, filtered and concentrated *in vacuo* to give 4.58g of the product (93%, MH+=210).

### Step C

The product from Step B above (4.5g) was dissolved in TFA (40ml) and cooled to 0°C. Zn powder (4.2g) was added portionwise and let reaction warm to room temperature and stir overnight. The reaction was concentrated *in vacuo,* taken up in 1N NaOH, extracted with ether, dried with MgSO₄, filtered and concentrated *in vacuo* to give 3.43g of the product (80%).

### Step D

The product from Step C (526mg), 3,4-diethoxy-3-cyclobutene-1,2-dione (0.4ml) and absolute EtOH (10ml) was stirred at room temperature overnight. Purification by preparative plate chromatography (1.0% EtOAc/Hex) to give 178mg of product (21%, MH+=320).

### PREPARATIVE EXAMPLE 88.3

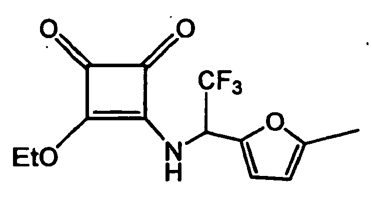

Following a similar procedure as described in Preparative Example 88.2, but instead using 2-methylfuran, the above cyclobutenedione intermediate was prepared.

### PREPARATIVE EXAMPLE 88.4

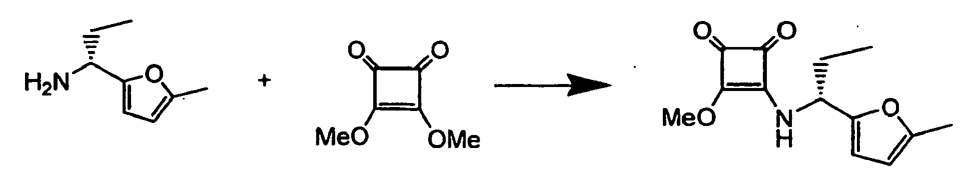

The amine from Preparative Example 75.1 (973mg) and the dimethoxysquarate (870mg) were dissolved in MeOH (20ml) and stirred for 3 days. The reaction was concentrated *in vacuo* and purified via flash column chromatography (MeOH/CH₂Cl₂, 1 %) to yield 325mg of product (19%, MH+=249.8).

### PREPARATIVE EXAMPLE 88.5

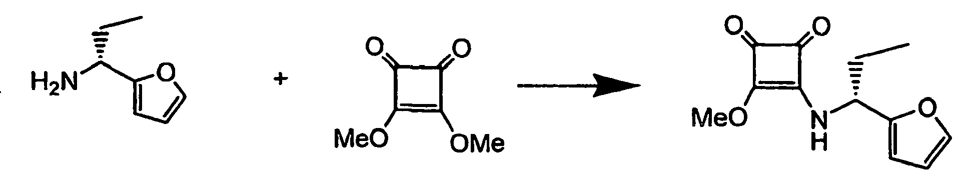

The amine from Preparative Example 75.9 (323mg) and the dimethoxysquarate (426mg) were dissolved in MeOH (10ml) and stirred over the weekend. The reaction was concentrated *in vacuo* and purified via flash column chromatography (MeOH/CH₂Cl₂, 1:20) to yield 407mg of product (57%, MH+=235.8).

### PREPARATIVE EXAMPLE 89

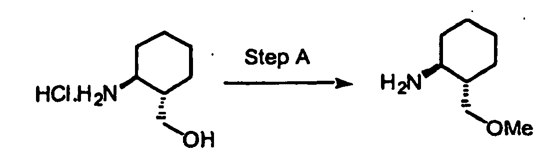

To a solution of KH (0.45 g, 11.3 mmol) in THF (15 mL) at room temperature was added amine hydrochloride (0.85 g, 5.1 mmol) portionwise to afford a heterogenous reaction mixture. The mixture was allowed to stand overnight (12h) and Mel (0.32 mL, 5.1 mmol) was added dropwise. The mixture was stirred for 6h after which the mixture was carefully poured into cold brine (125 mL). The mixture was extracted with Et₂O (3 x 25 mL) and the organic layers were combined. The organic layer was dried (Na₂SO₄), filtered, and concentrated under reduced pressure to afford the crude product as an oil. This material was carried on crude to the coupling step without further purification or characterization.

### PREPARATIVE EXAMPLE 89.1

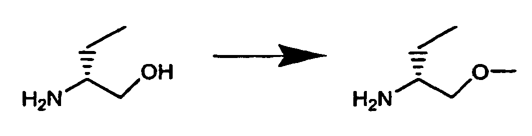

To a solution of KH (1.1g) in THF (20ml) at room temperature was added (R)-2-amino-1-butanol 48ml) dropwise to afford a heterogenous mixture. The mixture was allowed to stand overnight (18hr) and then Mel (1.59ml) was added dropwise. The mixture was stirred for 4hr after which brine was added. Extracted with ether, dried with K₂CO₃, filtered and concentrated *in vacuo* to afford 1.75g of an oil.

### PREPARATIVE EXAMPLE 89.2

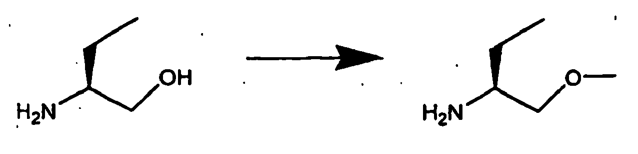

To a solution of KH (1.1g) in THF (20ml) at room temperature was added (S)-2-amino-1-butanol 48ml) dropwise to afford a heterogenous mixture. The mixture was allowed to stand overnight (18hr) and then Mel (1.59ml) was added dropwise. The mixture was stirred for 4hr after which brine was added. Extracted with ether, dried with K₂CO₃, filtered and concentrated *in vacuo* to afford 1.75g of an oil.

### PREPARATIVE EXAMPLE 90

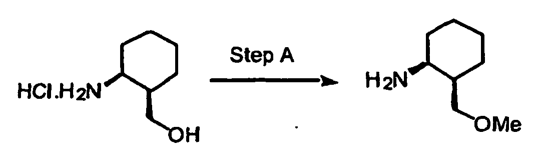

The corresponding cis analog was prepared in an analogous fashion utilizing the procedure described in Preparative Example 89. This material was also used without further purification.

### PREPARATIVE EXAMPLE 91

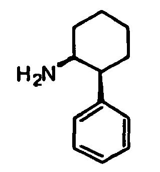

The desired compound was prepared according to methods previously described in J. Org. Chem. 1987, 52, 4437-4444.

### PREPARATIVE EXAMPLE 92

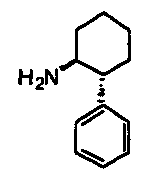

The desired compound was prepared according to methods previously described in Bull. Chem. Soc. Jpn. 1962, 35, 11-16.

### PREPARATIVE EXAMPLE 93

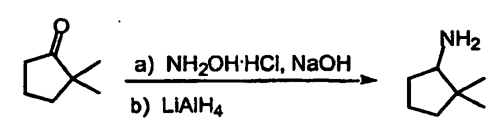

The desired amine was prepared from the corresponding ketone according to standard methods previously described in a) Synthesis 1987, 998-1001, b) Synthesis 1996, 641-646 and c) J. Med. Chem. 1991, 34, 2176-2186.

### PREPARATIVE EXAMPLE 94

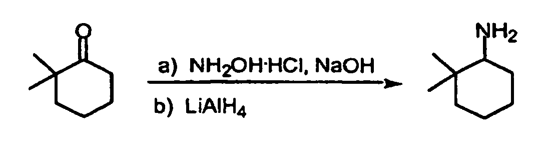

The desired amine was prepared from the corresponding ketone according to standard methods previously described in a) Synthesis 1987, 998-1001, b) Synthesis 1996, 641-646 and c) J. Med. Chem. 1991, 34, 2176-2186.

### PREPARATIVE EXAMPLE 95

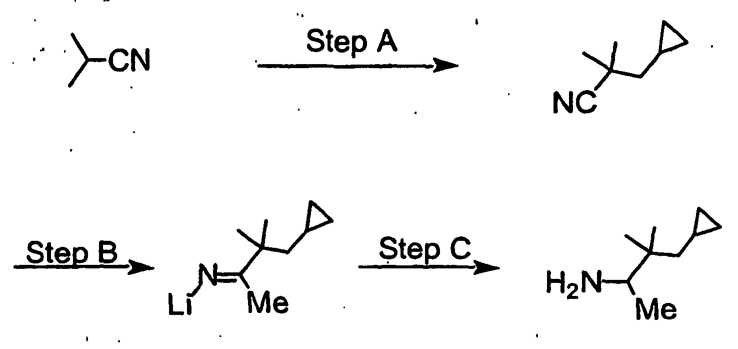

### Step A

Lithium hexamethyldisilylazide (34 mL, 1*M* in THF) was added dropwise to a -78°C THF (20 mL) solution of isobutyronitrile (2.8 mL). After 40 min, cyclopropylmethylbromide (5 g) was added and the mixture warmed to and stirred at 25°C overnight. After cooling to 0°C. 1*M* HCl (aq) was added and the mixture was extracted with diethyl ether, dried over anhydrous Na₂SO₄, filtered and concentrated *in vacuo* at 0°C to give the desired product (4.5 g).

### Step B

Methyl Lithium (17 mL, 1.4 *M* in Et₂O) was added to the product from Step A above (1.5 g) in Et₂O (anhydrous) at 0°C. The mixture was stirred at 0-25°C overnight, then diluted with 3*M* HCl (aq), extracted with CH₂Cl₂, dried over anhydrous Na₂SO₄, filtered, concentrated *in vacuo* at 0°C and used directly in Step C.

### Step C

The product from Step B above was added to a slurry of NaBH₄ (1.4 g) in isopropanol (50 mL) at 0°C, then the mixture was stirred at reflux for 8 hr and at room temperature for 48 hrs. Water was added and the mixture was stirred for 30 min, then extracted with diethyl ether, dried over anhydrous Na₂SO₄, filtered and concentrated *in vacuo.* The residue was diluted with CH₂Cl₂ and extracted with 3M HCl. The organic phase was discarded and the aqueous phase was basified with NaOH (aq) and extracted with CH₂Cl₂. Drying over anhydrous Na₂SO₄, filtering, and concentration *in vacuo* gave the desired compound (0.5 g).

### PREPARATIVE EXAMPLE 96

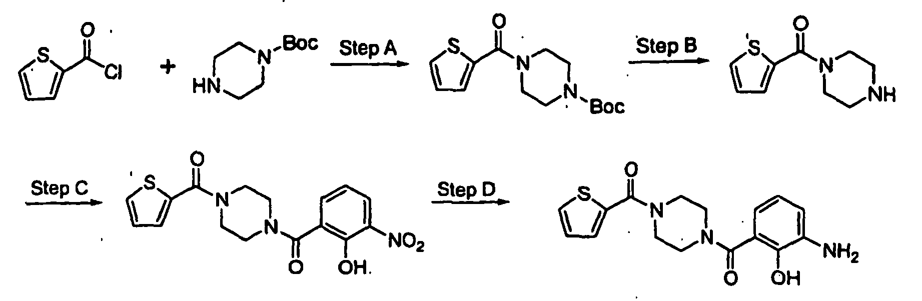

### Step A

2-Thiophenecarbonyl chloride (2.0mL, 18.7mmol) was dissolved in 100mL dichloromethane. After addition of diisopropylethylamine (4.1mL, 23.4mmol) and Boc-piperazine (3.66g. 19.7mmol), the mixture was stirred for 4h at room temperature. The resulting mixture was put into water (500mL) and acidified with 3N HCl to pH~1. Extraction with dichloromethane (2x100mL) and drying over sodium sulfate resulted in sufficiently pure product that was used in the next step without any further purification. ¹H NMR (300MHz, d₆-DMSO) 1.60 (s, 9H), 3.29 (dd, 4H), 3.69 (dd, 4H), 7.23 (dd, 1H). 7.49 (d, 1H), 7.79 (d, 1H).

### Step B

The crude material from Step A was dissolved in trifluoroacetic acid/dichloromethane (75mL, 4/1). After stirring for 2h, the reaction mixture was put into 1N sodium hydroxide (400mL). Extraction with dichloromethane (2x100mL) and drying over sodium sulfate resulted in sufficiently pure product that was used in Step C without any further purification. ¹H NMR (300MHz, d₆-DMSO) 2.81 (dd, 4H), 3.63 (dd, 4H), 7.21 (dd, 1H), 7.46 (d, 1H), 7.82 (d, 1H).

### Step C

The crude material (3.50g, 17.8mmol) from Step B was dissolved in dichloromethane (100mL). After addition of diisopropylethylamine (18.7mL, 107mmol), 3-nitrosalicylic acid (3.3g, 18.0mmol), and PyBrOP (10.4g, 22.3mmol), the resulting mixture was stirred over night at room temperature before being put into 1N sodium hydroxide (200mL). Extraction with dichloromethane (2x200mL) removed all PyBrOP by-products. The aqueous phase was acidified with 3N HCl and subsequently extracted with dichloromethane (3x 100mL). The combined organic phases of the acidic extraction were dried over sodium sulfate, concentrated, and finally purified by column chromatography (dichloromethane/methanol = 10/1) to yield the desired product (2.31g, 34 % over 3 steps). ¹H NMR (300MHz, d₆-DMSO) 3.30-3.90 (m, 8H), 7.10-8.20 (m, double signals due to E/Z-isomers, 6H), 10.82 (s, 1H).

### Step D

The nitro-compound (2.3g, 6.4mmol) from Step C was dissolved in methanol (50mL) and stirred with 10% Pd/C under a hydrogen gas atmosphere over night. The reaction mixture was filtered through Celite and washed thoroughly with methanol. Finally, the filtrate was concentrated *in vacuo* and purified by column chromatography (dichloromethane/methanol = 10/1) to yield the desired product (1.78g, 84%). ¹H NMR (300MHz, d₆-DMSO) 3.30-3.90 (m, 8H), 7.22 (m, 2H), 7.55 (d, 1H), 7.71 (d, 1H), 7.88 (d, 1H), 8.15 (d, 1H), 10.85 (bs, 1H).

### PREPARATIVE EXAMPLE 97

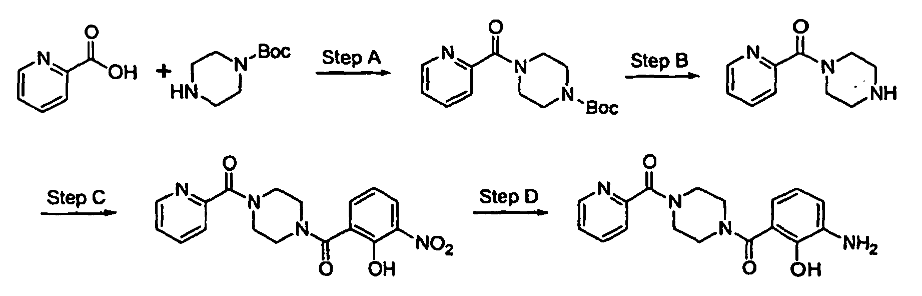

### Step A

Picolinic acid (3.0g, 24.3mmol) was suspended in SOCl₂ (15mL). After addition of dimethylformamide (5 drops), the reaction mixture was stirred for 4 hours. Evaporation of the solvent yielded the corresponding acid chloride as HCl-salt. Without any further purification, the solid was suspended in 120mL dichloromethane. After addition of diisopropylethylamine (12.7mL, 73mmol) and Boc-piparazine (4.8g, 25.5mmol), the reaction was stirred over night at room temperature. The resulting mixture was put into water (500mL) and extracted with dichloromethane (2x100mL). Drying over sodium sulfate resulted in sufficiently pure product that was used in Step B without any further purification. ¹H NMR (300MHz, d₆-DMSO) 1.63 (s, 9H), 3.21 (dd, 4H), 3.61 (dd, 4H), 7.57 (dd, 1H), 7.63 (d, 1H), 7.98 (dd, 1H), 8.70 (d, 1H).

### Step B

The crude material from Step A was dissolved in trifluoroacetic acid/dichloromethane (75mL, 4/1). After stirring for 2days, the reaction mixture was put into 1N sodium hydroxide (400mL). Extraction with dichloromethane (2×100mL) and drying over sodium sulfate resulted in sufficiently pure product that was used in Step C without any further purification. ¹H NMR (300MHz, d₆-DMSO) 2.77 (dd, 2H), 2.83 (dd, 1H). 3.38 (dd, 2H), 3.64 (dd, 1H), 7.58 (dd, 1H), 7.62 (d, 1H). 8.00 (dd, 1H). 8.67 (d, 1H).

### Step C

The crude material (1.35g, 7.06mmol) from Step B was dissolved in dichloromethane (50mL). After addition of diisopropylethylamine (3.7mL, 21.2mmol), 3-nitrosalicylic acid (1.36g, 7.41 mmol), and PyBrOP (3.62g, 7.77mmol), the resulting mixture was stirred over night at room temperature before being put into 1N sodium hydroxide (300mL). Extraction with dichloromethane (2x100mL) removed any PyBrOP products. The aqueous phase was acidified with 3N HCl. Adjustment of the pH with saturated sodium carbonate solution to almost neutral crushed the desired compound out of solution. The aqueous phase was subsequently extracted with dichloromethane (3x 100mL).
The combined organic layers of the neutral extraction were dried over sodium sulfate, concentrated, and finally purified by column chromatography (dichloromethane/methanol = 20/1) to yield the desired product (1.35g, 16% over 3 steps). ¹H NMR (300MHz, d₆-DMSO) 3.30-3.95. (m, 8H), 7.22 (m, 1H), 7.61 (m, 1H), 7.73 (d, 2H), 8.03 (m,1H), 8.17 (m, 1H), 8.69 (m, 1H), 10.82 (s; 1H).

### Step D

The nitro-compound (1.35g, 3.79mmol) from Step C was dissolved in methanol (60mL) and stirred with 10% Pd/C under a hydrogen gas atmosphere over night. The reaction mixture was filtered through Celite and washed thoroughly with methanol. Finally, the filtrate was concentrated *in vacuo* and purified by column chromatography (dichloromethane/methanol = 20/1) to yield the desired product (1.10g, 89 %). ¹H NMR (300MHz, d₆-DMSO) 3.50-3.85 (m, 8H), 6.47 (dd 1H), 6.74 (m, 2H), 7.59 (dd, 1H), 7.71 (d, 1H), 8.04 (dd, 1H), 8.68 (d, 1H).

### PREPARATIVE EXAMPLE 98

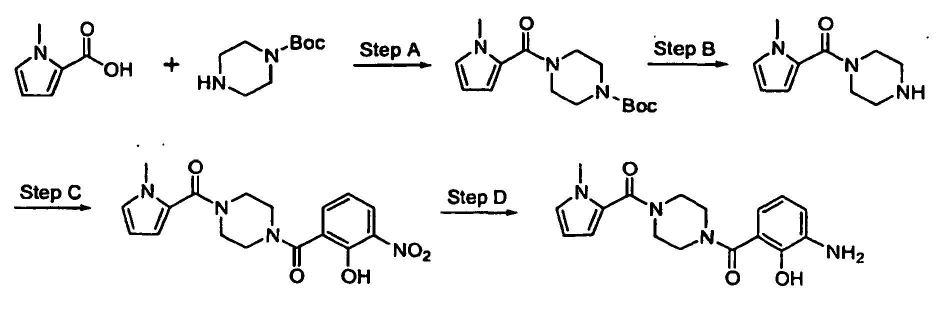

### Step A

1-Methyl-2-pyrrolecarboxylic acid (2.5g, 20.0mmol) was dissolved in dichloromethane (50mL). After addition of PyBrOP (16.3g, 35.0mmol), diisopropylethylamine (14.0mL, 73.0mmol) and Boc-piparazine (5.5g, 30.0mmol), the reaction was stirred over night at room temperature before being put into 1N sodium hydroxide (200mL). Extraction with dichloromethane (2x100mL) removed all PyBrOP by-products. The aqueous phase was acidified with 3N HCl. Adjustment of the pH with saturated sodium carbonate solution to almost neutral precipitated the desired compound. The aqueous phase was subsequently extracted with dichloromethane (3x 100mL). The combined organic phases of the neutral extraction were dried over sodium sulfate. Removal of the solvent resulted in sufficiently pure product that was used in Step B without any further purification. ¹H NMR (300MHz, d₆-DMSO) 1.59 (s, 9H) 3.21 (dd, 4H), 3.61 (dd, 4H), 3.74 (s, 3H), 6.11 (dd, 1H), 6.33 (d, 1H), 7.01 (d, 1H).

### Step B

The crude material from Step A was dissolved in trifluoroacetic acid/dichloromethane (75mL, 4/1). After stirring for 3h, the reaction mixture was put into 1N sodium hydroxide (400mL). Extraction with dichloromethane (3x100mL) and drying over sodium sulfate resulted in sufficiently pure product that was used in Step C without any further purification. ¹H NMR (300MHz, d₆-DMSO) 2.79 (dd, 4H), 3.62 (dd, 4H), 3.76 (s, 3H), 6.11 (dd, 1H), 6.37 (d, 1H), 6.96 (d, 1H).

### Step C

The crude material (3.15g, 16.3mmol) from Step B was dissolved in dichloromethane (100mL). After addition of diisopropylethylamine (8.5mL, 49.0mmol), 3-nitrosalicylic acid (3.13g, 17.1 mmol), and PyBrOP (9.11 g, 19.6mmol), the resulting mixture was stirred over night at room temperature before being put into 1N sodium hydroxide (400mL). Extraction with dichloromethane (2x100mL) removed all PyBrOP products. The aqueous phase was then carefully acidified with 3N HCl until the color of the solution changes from orange to yellow and the desired compound crashed out of solution.
The aqueous phase was subsequently extracted with dichloromethane (3x 100mL). The combined organic layers of the acidic extraction were dried over sodium sulfate and concentrated *in vacuo* to yield the desired product. ¹H NMR (300MHz, d₆-DMSO) 3.35-3.85 (m, 8H), 3.79 (s, 3H), 6.13 (dd, 1H), 6.45 (d, 1H). 7.01 (s, 1H). 7.22 (dd, 1H), 7.70 (d, 1H), 8.16 (d, 1H), 10.83 (s, 2H).

### Step D

The crude nitro-compound from Step C was suspended in methanol (60mL) and stirred with 10% Pd/C under a hydrogen gas atmosphere over night. The reaction mixture was filtered through Celite and washed thoroughly with methanol. The filtrate was concentrated *in vacuo* and purified by column chromatography (dichloromethane/methanol = 10/1) to yield the desired product (2.61 g, 40 % for 4 steps). ¹H NMR (300MHz, d₆-DMSO) 3.45-4.80 (m, 8H), 3.79 (s, 3H), 6.17 (dd, 1H), 6.45 (m, 2H), 6.78 (m, 2H), 7.01 (d, 1H).

### PREPARATIVE EXAMPLE 99

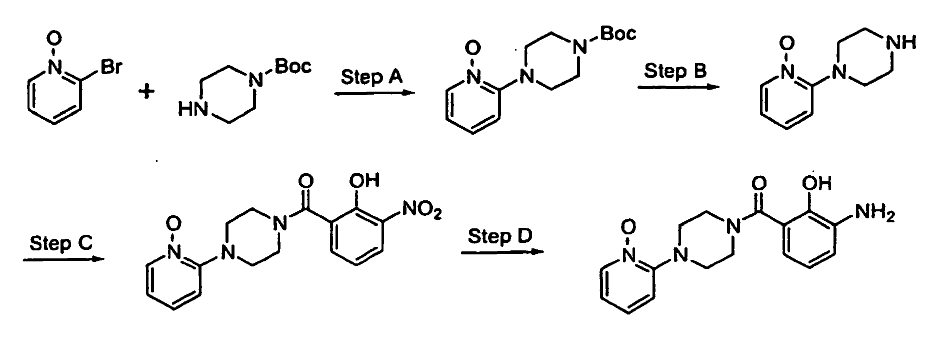

### Step A

2-Bromopyridine N-oxide hydrochloride (1.1.3g, 5.37mmol) and, Boc-piperazine (1.50g, 8.06mmol) were heated to 80° C in pyridine (10mL) over night. The reaction mixture was put into water (300mL) and then extracted with dichloromethane (2x100mL). The,combined organic phases were dried over sodium sulfate, concentrated, and finally purified by column chromatography (dichloromethane/methanol = 10/1) to yield the desired product (500mg, 33 %).
¹H NMR (300MHz, d-CDCl₃) 1.60 (s, 9H), 3.46 (dd, 4H), 3.78 (dd, 4H), 6.99 (m, 2H), 7.37 (dd, 1H), 8.33 (d, 1H).

### Step B

The purified product (500mg, 1.79mmol) was stirred for 30 min with 4N HCl/dioxane (15mL). Evaporation of the solvent yielded the crude amine (465mg) as multiple HCl-salt which was used in Step C without any further purification.
¹H NMR (300MHz, d₆-DMSO) 3.38 (m, 4H), 4.81 (m, 4H), 7.34 (dd, 1H), 7.55 (d, 1H), 7.86 (dd, 1H), 8.55 (d, 1H).

### Step C

The crude material (370mg, 1.48mmol) from Step B was suspended in dichloromethane (20mL). After addition of diisopropylethylamine (2.6mL, 14.8mmol), 3-nitrosalicylic acid (406mg, 2.22mmol), and PyBrOP (1.21g, 2.59mmol), the mixture was stirred over night at room temperature before being put into 1N sodium hydroxide (50mL). Extraction with dichloromethane (2x50mL) removed all PyBrOP products. The aqueous phase was then carefully acidified (pH - 4-5) with 3N HCl and extracted with dichloromethane (3x 50mL). The combined organic layers of the acidic extraction were dried over sodium sulfate, concentrated *in vacuo* and purified by column chromatography (dichloromethane/methanol = 10/1) to yield the desired product (330mg, 65%).
LCMS calculated: 344.1, found: (M+1)⁺ 345.1

### Step D

Sodium hydrosulfite (1.05g)was dissolved in water (3.0mL) to yield a 1.5N solution. Addition of dioxane (3.0mL) was followed by injection of conc. ammonium hydroxide (0.60mL, yields a 1.0N concentration). After addition of the nitro-compound (100mg, 0.29mmol), the reaction mixture was stirred for 0.5h. Subsequently, the solvent was removed and the residue suspended in dichloromethane/methanol (10/1). Filtration through Celite removed most of the salts. Final purification by column chromatography (dichloromethane/methanol = 5/1) yielded the desired product (68mg, 75%).
LCMS calculated: 314.14, found: (M+1)⁺ 315.1

### PREPARATIVE EXAMPLE 100

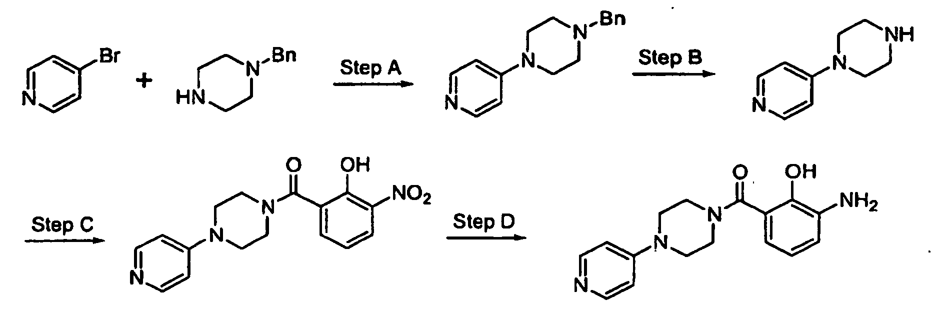

### Step A

4-Bromopyridine hydrochloride (3.0g, 15.4mmol) was dissolved in water (15mL). After addition of N-benzylpiperazine (14.8mL, 85.0mmol) and 500mg copper sulfate, the reaction mixture was heated overnight to 140° C. The resulting product was extracted with ether (5x75mL), dried over sodium sulfate and concentrated. Final purification by column chromatography (dichloromethane/methanol/NH₄OH = 10/1/0.1) yielded the desired product (2.16g, 55%). ¹H NMR (300MHz, d-CDCl₃) 2.68 (dd, 4H), 3.45 (dd, 4H), 6.76 (d, 2H), 7.40 (m, 5H), 8.38 (d, 2H).

### Step B

The benzylamine (2.16g, 8.54mmol) from Step A, ammonium formate (2.71 g, 43.0mmol) and Pd(C) (10%, 1.0g) was suspended in methanol (50mL) and refluxed for 3h. The palladium was filtered off and the filtrate was concentrated. The sufficiently pure product was used in Step C without any further purification. ¹H NMR (300MHz, d-CDCl₃) 2.48 (bs, 1H), 3.13 (dd, 4H), 3.41 (dd, 4H), 7.78 (d, 2H), 8.39 (d, 2H).

### Step C

The crude material (1.15g, 7.06mmol) from Step B was dissolved in dichloromethane (50mL). After addition of diisopropylethylamine (4.7mL, 42.4mmol), 3-nitrosalicylic acid (1.94g, 10.6mmol), and PyBrOP (5.78g, 12.3mmol), the resulting mixture was stirred over night at room temperature before being put into 1N sodium hydroxide (300mL). Extraction with dichloromethane (2x100mL) removed all PyBrOP products. The aqueous phase was carefully acidified to pH - 5-6 with 3N HCl and extracted with dichloromethane (3x 100mL). The combined organic layers of the neutral extraction were dried over sodium sulfate, concentrated, and finally purified by column chromatography (dichloromethane/methanol/NH₄OH = 10/1/0.1) to yield the desired product (850mg, 37% for 2 steps).

### Step D

The nitro-compound (850mg, 2.59mmol) from Step C was dissolved in methanol (40mL) and stirred with 10% Pd/C under a hydrogen gas atmosphere over night. The reaction mixture was filtered through Celite and washed thoroughly with methanol. Finally, the filtrate was concentrated *in vacuo* and purified by column chromatography (dichloromethane/methanol/
NH₄OH = 10/1/0.1) to yield the desired product (650g, 84 %). ¹H NMR (300MHz, d₆-DMSO) 3.40-3.75 (bm, 8H), 6.49 (dd, 1H), 6.76 (m, 2H), 6.93 (d, 2H), 8.28 (d, 2H).

### PREPARATIVE EXAMPLE 101

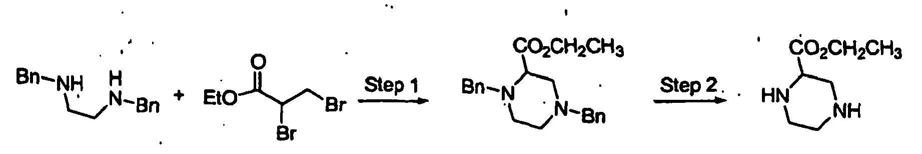

### Step 1

N,N'-Dibenzyl-ethane-1,2-diamine (20mL, 0.0813mol), triethylamine (22.66mL, 0.1626mol) and benzene (100mL) were combined in a round bottom flask. A solution of 2,3-dibromo-propionic acid ethyl ester (11.82mL, 0.0813mol) in benzene (50mL) was added dropwise. The solution was refluxed over night and monitored by TLC (20% ethyl acetate/hexane). The reaction was cooled to room temperature, then filtered and washed with benzene. The filtrate was concentrated then purified by column chromatography (15% ethyl acetate/hexane). The product was isolated as an oil (25.42g, 0.0752mol, 92%). MS: calculated: 338.20, found: 339.2
¹H NMR (300 MHz, CDCl₃) 1.23 (t, 3H), 2.48 (m, 3H), 2.62 (m, 1H), 2.73 (m, 1H), 3.07 (m, 1H), 3.30 (m, 1H), 3.42 (d, 1H), 3.56 (m, 2H), 3.91 (d, 1H), 4.17 (m, 2H), 7.27 (m, 10H).

### Step 2

In a Parr shaker vessel, the ester (25.43g, 0.075mol) and methanol (125mL) were combined. The vessel was purged with argon and palladium catalyst (5% on carbon, 2.5g) was added. The system was shaken under an atmosphere of hydrogen overnight. TLC (20% ethyl acetate/hexane) indicated that reaction was complete. The reaction mixture was filtered through a pad of Celite and washed with methanol. The filtrate was concentrated and the product isolated as a solid (11.7g, 0.074mol, 98%).
MS: calculated: 158.11, found:159.2 ¹H NMR (300 MHz, CDCl₃) 1.27 (t, 3H), 2.70 (m, 4H), 2.96 (m, 1H), 3.13 (dd, 1H), 3.43 (dd, 1H), 4.18 (m, 2H).

### PREPARATIVE EXAMPLE 102

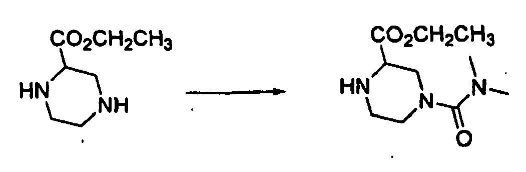

Piperazine-2-carboxylic acid ethyl ester (3.11g, 0.0197mol), diisopropylethylamine (5.15mL, 0.0296mol) and methylene chloride (200mL) were combined in a round bottom flask. While stirring at room temperature, a solution of N,N-dimethylcarbamoyl chloride (1.81 mL, 0.0197mol) in methylene chloride (20mL) was added dropwise. The reaction was stirred for one hour. After this time the reaction was concentrated and carried on to the next step without further purification. (99% yield).
MS: calculated: 229.14, found:230.1
¹H NMR (300 MHz, CDCl₃) 1.30 (t, 3H), 2.85 (s, 6H), 3.10 (m, 3H), 3.31 (m, 2H), 3.60 (m, 2H), 4.21 (q, 2H).

### PREPARATIVE EXAMPLE 103-104

Following the procedure described for Example 102, the Products listed in the table below were prepared using the commercially available chloride shown and piperazine-2-carboxylic acid ethyl ester from Preparative Example 101.

| Example | Chloride | Product | 1.Yield (%) |
|---|---|---|---|
| | | | 2. (M+1)⁺ |
| 103 | 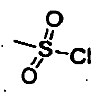 | 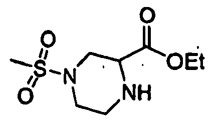 | 1. 99% |
| | | | 2. 237.1 |
| 104 | 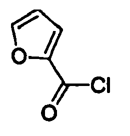 | 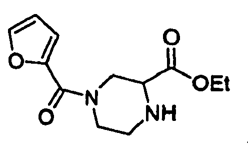 | 1. 62% |
| | | | 2. 253.1 |

PREPARATIVE EXAMPLE 105

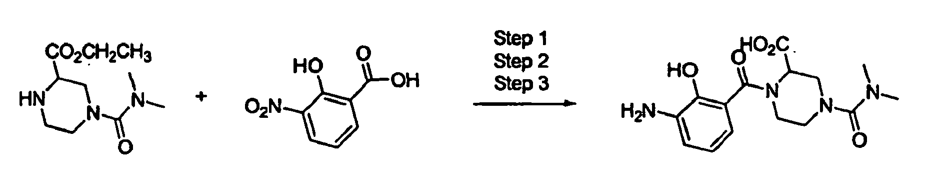

### Step 1

3-Nitrosalicylic acid (3.61 g, 0.0197g), DCC (2.03g, 0.0099mol) and ethyl acetate (130mL) were combined in a round bottom flask and stirred for 15min. 4-Dimethylcarbamoyl-piperazine-2-carboxylic acid ethyl ester (4.51 g. 0.0197g) was added, and the reaction was stirred for 72 hours. The reaction mixture was concentrated then dissolved in dichloromethane. The organic phase was washed once with 0.1N sodium hydroxide. The aqueous phase was back extracted once with dichloromethane. The aqueous phase was acidified and wash three times with ethyl acetate. The aqueous phase was concentrated and purified by column chromatography (5% methanol/DCM).
MS: calculated: 394.15, found:395.0
¹H NMR (300 MHz, CDCl₃) 1.32 (t, 3H), 2.86 (m, 7H), 3.15 (m, 1H), 3.51 (m, 4H), 4.24 (m, 3H), 7.15 (m, 1H), 7.66 (m, 1H), 8.20 (m, 1H), 10.86 (bs, 1H).

### Step 2

4-Dimethylcarbamoyl-1-(2-hydroxy-3-nitro-benzoyl)-piperazine-2-carboxylic acid ethyl ester (0.80g, 0.002mol) and methanol (50mL) were combined in a round bottom flask. The system was purged with argon. To the solution was added 5% palladium on carbon (~100mg). The flask was purged with hydrogen and stirred overnight. The reaction was filtered through a pad of celite and washed with methanol. The material was concentrated then purified by column chromatography (6% methanol/DCM). Isolated product (0.74g, 0.002mol, 100%).
MS: calculated: 364.17, found:365.1
¹H NMR (300 MHz, CDCl₃) 1.27 (t, 3H), 2.85 (m, 8H), 3.18 (1H), 3.45 (m. 3H), 4.19 (m, 3H), 3.90 (m, 3H)

### Step 3

1-(3-Amino-2-hydroxy-benzoyl)-4-dimethylcarbamoyl-piperazine-2-carboxylic acid ethyl ester (0.74g, 0.002mol) was suspended in a solution of dioxane (10mL) and water (10mL). Lithium hydroxide (0.26g, 0.0061 mol) was added and the mixture stirred for two hours. The solution was acidified to pH=6 with 3N HCl then extracted with butanol. The extracts were combined, dried over sodium sulfate and concentrated.
MS: calculated: 336.14, found:337.1
¹H NMR (300 MHz, CD₃OD) 2.86 (m, 7H), 3.23 (m, 3H), 3.54 (m, 3H), 6.92 (m, 2H), 7.23 (m, 1H).

### PREPARATIVE EXAMPLE 106-107

Following the procedure described for Example 105, the Products listed in the table below were prepared using the amine from the Preparative Example indicated and 3-nitrosalacylic acid.

| Example | Aniline | Product | 1. Yield (%) |
|---|---|---|---|
| | | | 2. (M+1)⁺ |
| | | | 3. Note |
| 106 | 103 | 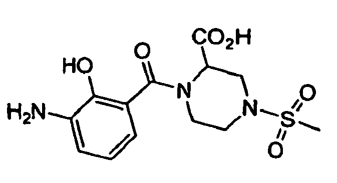 | 1. 91% |
| | | | 2. Not observed |
| | | | 3. Rainey nickel used in Step 2 |
| 107 | 104 | 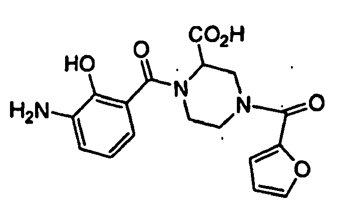 | 1. 24% |
| | | | 2. 360.0 |
| | | | 3. For Step 1 used PyBrop/ DIEA in DCM |

### PREPARATIVE EXAMPLE 108

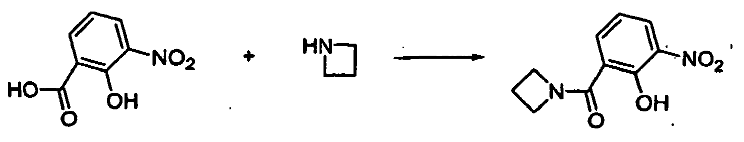

### Step A

3-Nitrosalicylic acid (1.0g, 5.5mmol) was dissolved in ethyl acetate (20mL). 1,3-Dicyclohexylcarbodiimide (0.568g, 2.8mmol) was added and the mixture was stirred for approximately 10 minutes and cooled to 0°C. During this time a precipitate formed. Azetidine (0.39mL, 5.8mmol) was added and the reaction was stirred overnight and allowed to warm to room temperature. After this time the reaction was cooled to 0°C and filtered. The collected solid was washed with chilled ethyl acetate. The filtrate was concentrated and purified by column chromatography (80% EtOAc/Hex) to give the product (476mg, 39.0%).
¹H NMR (300 MHz, CDCl₃) δ2.40(m, 2H), 4.38(m, 4H), 6.97(m, 1H), 7.62(d, 1H), 8.12(d, 1H), 12.88(m, 1H) ppm.

### Step B

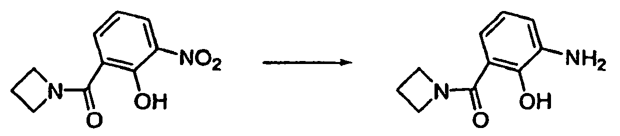

The nitro compound (0.48g, 2.1 mmol) from Preparative Example 32 Step A was dissolved in methanol (25ml) and stirred with 10% Pd/C under a hydrogen gas atmosphere overnight. The reaction mixture was filtered through celite, the filtrate concentrated *in vacuo* to give the product (344mg, 90%). ¹H NMR (300 MHz, CDCl₃) δ2.52(m, 2H), 4.57(bs, 4H), 6.75(m, 1H), 6.90(m, 2H), 12.71 (bs, 1H) ppm.

### PREPARATIVE EXAMPLE 109

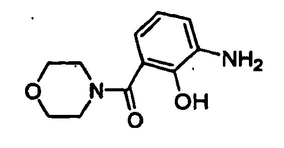

In essentially the same manner as described in Preparative Example 108 above, the morpholino-amine product was obtained.

### PREPARATIVE EXAMPLE 110

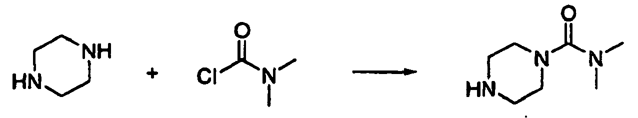

Piperazine (4.9g, 0.057mol) was dissolved in dichloromethane (100mL). N,N'-Dimethylcarbamoyl chloride (1.0mL, 0.011mol) was added dropwise to the solution at room temperature. The reaction was stirred for one hour. After this time 1N potassium hydroxide (200mL) was added. The layers were separated and the aqueous layer was extracted three times with dichloromethane. The organic fractions were combined and dried over sodium sulfate. Filtration and concentration provided the product, without further purification, as an oil (1.16g, 13%).
¹H NMR (CDCl₃, 300 MHz) 1.95 (s, 1H), 2.83 (s, 6H), 2.86 (m, 4H), 3.20 (m, 4H).
MS: calculated: 157.12, found: 158.1.

### PREPARATIVE EXAMPLE 111

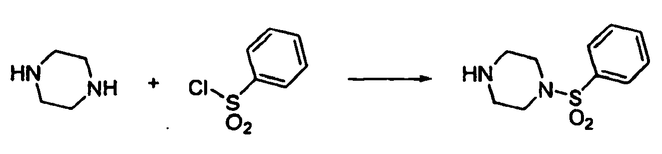

Piperazine (4.9g, 0.057mol) was dissolved in 1N HCl (100mL). A solution of phenylsulfonylchloride (1.45mL, 0.011 mol) in acetonitrile (25mL) was added dropwise to the solution at room temperature. The reaction was stirred for 30 minutes. After this time the reaction was extracted two times with ethyl acetate. The solution was then made basic,with 1N potassium hydroxide and extracted three times with dichloromethane. The dichloromethane fractions were combined and dried over magnesium sulfate. Filtration and concentration provided the product, without further purification, as a solid (1.22g, 9.4%).
¹H NMR (CDCl₃, 300 MHz) 2.94 (m, 8H), 7.56 (m, 3H), 7.76 (m, 2H).
MS: calculated: 226.08, found: 227.1.

### PREPARATIVE EXAMPLE 112

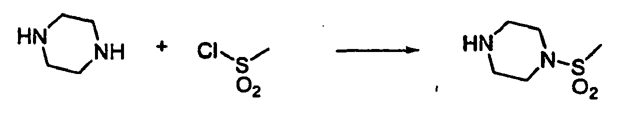

Piperazine (4.9g, 0.057mol) was dissolved in dichloromethane (100mL). Methanesulfonyl chloride (0.85mL, 0.011 mol) was added dropwise to the solution at room temperature. The reaction was stirred for 30 minutes. After this time 1N potassium hydroxide (200mL) was added. The layers were separated and the aqueous layer was extracted three times with dichloromethane. The organic fractions were combined and dried over sodium sulfate. Filtration and concentration provided the product, without further purification, as a solid (1.07g, 11 %).
¹H NMR (CDCl₃, 300 MHz) 1.75 (s, 1H), 2.78 (s, 3H), 2.97 (m, 4H), 3.20 (m, 4H).
MS: calculated: 164.06, found: 165.1.

### PREPARATIVE EXAMPLE 113

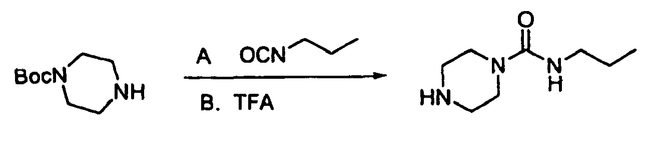

### Step A

Boc-Piperazine (3.0g, 0.0161 mol) was dissolved in dichlorometharie (100mL). Propylisocyanate (1.51 mL, 0.0161 mol) was added to the solution at room temperature. The reaction was stirred for over night. After this time the reaction was diluted with 1N potassium hydroxide (200mL) and extracted six times with dichloromethane. The organic fractions were combined and dried over magnesium sulfate. Filtration and concentration provided the product as a solid.

### Step B

The product of Step A above, was dissolved in a 30% trifluoroacetic acid/dichloromethane solution and stirred overnight. After this time a 1N potassium hydroxide solution (200 mL) was added to the reaction. The aqueous layer was extracted a total of six times with dichloromethane. The organic fractions were combined and dried over sodium sulfate. Filtration and concentration provided the product (1.37g, 50%).
¹H NMR (CDCl₃, 300 MHz) 0.92 (t, 3H), 1.52 (m, 2H), 2.89 (m, 4H), 3.01 (s, 1H), 3.18 (m, 2H), 3.37 (m, 4H), 4.61 (bs, 1H).
MS: calculated: 171.14, found: 172.0.

### PREPARATIVE EXAMPLE 114

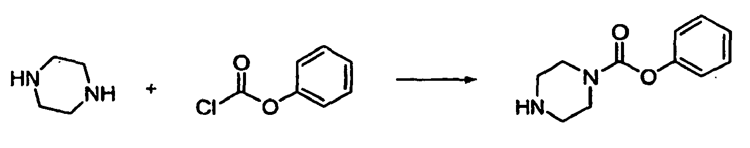

Piperazine (4.9g, 0.0569mol) was dissolved in 1N HCl (70mL). A solution of phenylchloroformate (1.43mL, 0.0114mol) in acetonitrile (25mL) was added dropwise to the solution at room temperature. The reaction was stirred for 30 minutes. After this time the reaction was extracted two times with ethyl acetate. The solution was then made basic with 1N potassium hydroxide and extracted three times with dichloromethane. The dichloromethane fractions were combined and dried over magnesium sulfate. Filtration and concentration provided the product, without further purification, as a solid (2.12g, 18%).
¹H NMR (CDCl₃, 300 MHz) 1.78 (s, 1H), 2.91 (m, 4H), 3.59 (m, 4H), 7.11 (2H), 7.19 (m, 1H), 7.36 (m, 2H).
MS: calculated: 206.24, found: 207.1.

### PREPARATIVE EXAMPLE 115-117

Following the procedure described for Example 112, the Products listed in the table below were prepared using the commercially available chloroformate shown and piperazine.

| Example | Chloroformate | Product | 1.Yield (%) |
|---|---|---|---|
| | | | 2. (M+1)⁺ |
| 115 | 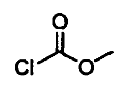 | 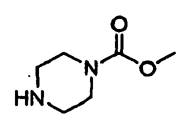 | 1. 54% |
| | | | 2. 144.9 |
| 116 | 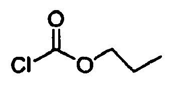 | 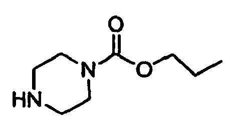 | 1. 17% |
| | | | 2. 173.0 |
| 117 | 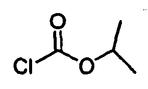 | 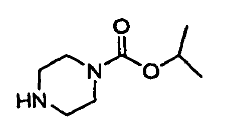 | 1. 69% |
| | | | 2. 173.0 |

### PREPARATIVE EXAMPLE 118

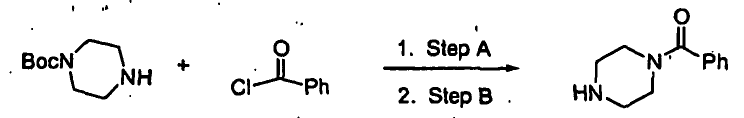

### Step A

Boc-Piperazine (3.01g, 0.0161mol) was dissolved in dichloromethane (100mL) along with diisopropylethylamine (5.61 mL, 0.0322mol). Benzoylchloride (1.87mL, 0.0161 mol) was added dropwise to the solution at room temperature. The reaction was stirred for several hours. After this time the reaction was concentrated and the product was purified by column chromatography (10% MeOH/DCM). Boc-Protected product was isolated as a solid (5.21 g).
¹H NMR (CDCl₃, 300 MHz) 1.47 (s, 9H), 3.45 (m, 8H), 7.41 (m, 5H).
MS: calculated: 290.16, found: 290.8.

### Step B

The product from Step A above, was dissolved in a 50% trifluoroacetic acid/dichloromethane solution and stirred overnight. After this time the reaction was diluted with 1N potassium hydroxide (200mL) and the organic layer was separated. The aqueous phase was then extracted six times with dichloromethane. The organic fractions were combined and dried over magnesium sulfate. Filtration and concentration provided product (2.93g).
¹H NMR (CDCl₃, 300 MHz) 1.92 (s, 1H), 2.87 (m, 4H), 3.52 (m, 4H). 7.39 (s, 5H).
MS: calculated: 190.11, found: 191.1.

### PREPARATIVE EXAMPLE 119

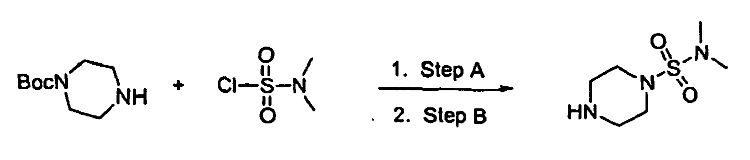

### Step A

Boc-Piperazine (3.0g, 0.0161 mol) was dissolved in dichloromethane (100mL) along with diisopropylethylamine (3.1mL, 0.0177mol). N,N'-dimethylsulfamoyl chloride (1.73mL, 0.0161mol) was added dropwise to the solution at room temperature. The reaction was stirred for several hours. After this time the reaction was diluted with water (100mL). The layers were separated and the aqueous layer was extracted six times with dichloromethane. The organic fractions were combined and dried over magnesium sulfate. Filtration and concentration provided the product, without further purification, as a solid (4.53g).
¹H NMR (CDCl₃, 300 MHz) 1.47 (s, 9H), 2.84 (s, 6H), 3.21 (m, 4H), 3,48 (m, 4H).
MS: calculated: 293.14, found: 194.1 (M-Boc)⁺.

### Step B

The product from Step A above, was dissolved in a 30% trifluoroacetic acid/dichloromethane solution and stirred overnight. After this time the reaction was diluted with water and 1N potassium hydroxide was used to make the aqueous layer slightly basic. The aqueous layer was extracted a total of seven times with dichloromethane. The organic fractions were combined and dried over sodium sulfate. Filtration and concentration provided the product (2.96g).
¹H NMR (CDCl₃, 300 MHz) 2.03 (s, 1H), 2.83 (s, 6H), 2.92 (m, 4H), 3.23 (m, 4H).
MS: calculated: 193.09, found: 194.1.

### PREPARATIVE EXAMPLE 120

### Step A

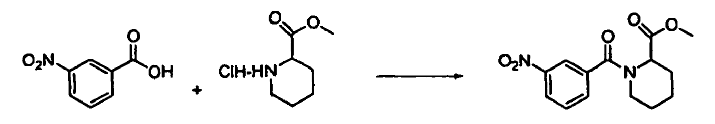

In essentially the same manner as that described in Preparative Example 105, Step 1, using 3-nitrobenzoic acid instead of 3-nitrosalicylic acid, the methyl ester product was prepared.

### step B

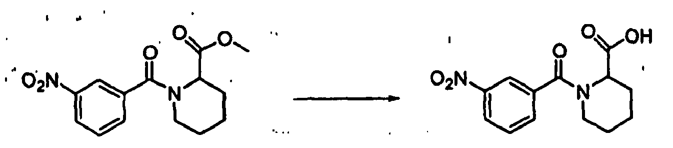

The methyl, ester (1.79g, 6.1 mmol) from Step A above, was dissolved in dioxane/water (20mL/15mL) at room temperature. Lithium hydroxide (0.258g, 6.2mmol) was added to the solution. After a few hours more lithium hydroxide was added (0.128g, 3.0mmol) and the reaction was stirred for another hour. After this time the reaction was concentrated and then taken up in water. The solution was extracted two times with ether. The aqueous phase was then acidified and extracted three times with ethyl acetate. The organic fractions were then dried over sodium sulfate, filtered and concentrated. Product was isolated by column chromatography (95% EtOAc/Hex, 0.05% HOAc) to give the product (1.66 g, 98%)
¹H NMR (300 MHz, CDCl₃) 1.49(m, 2H), 1.68 (m, 1H), 1.82(m, 2H), 2.44(m, 1H) 3.32(m, 1H), 3.58(m, 1H), 5.57(m, 1H), 7.65(m, 1H), 7.80(m, 1H), 8.32(m, 2H), 10.04(bs, 1Hppm).

### Step C

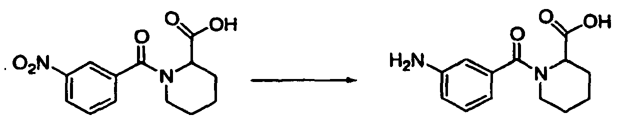

The nitro compound was dissolved in an excess of methanol (20mL) and covered by a blanket of argon. 5% Palladium on carbon was added (catalytic) and a hydrogen balloon was attached to the flask. The atmosphere of the system was purged under vacuum and replaced with hydrogen. This step was repeated for a total of three times. The reaction was then stirred under hydrogen overnight. After this time the balloon was removed and the solution was filtered through celite followed by several rinses with methanol. The filtrate was concentrated and dried on the vacuum line to provide the desired aniline product (1.33 g, 90%).
¹H NMR (300 MHz, CDCl₃) 1.40(m, 2H), 1.50(m, 1H), 1.68(m, 2H), 2.33(m, 1H) 3.18(m, 1H), 3.62(m, 1H), 5.39(m, 1H), 6.12(bs, 2H), 6.75(m, 2H), 7.12(m, 1H)ppm. Mass Spectra, calculated: 248, found: 249.1 (M+1)⁺

### PREPARATIVE EXAMPLES 121-123

Following the procedure described in Preparative Example 120, but using the commercially available amine and benzoic acid indicated, the intermediate products in the table below were obtained.

| Ex. | Carboxylic Acid | Amine | Product | 1.Yield (%) |
|---|---|---|---|---|
| | | | | 2. (M+1)⁺ |
| | | | | 3. Note |
| 121 | 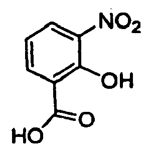 | 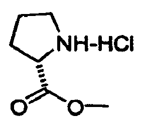 | 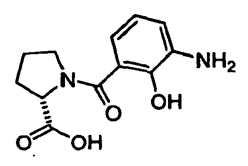 | 1. 21% |
| | | | | 2. 251.0 |
| 122 | 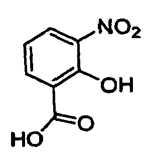 | 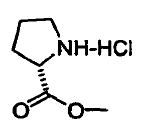 | 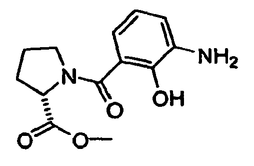 | 1. 21% |
| | | | | 2. 265.0 |
| | | | | 3. Skipped step B |
| 123 | 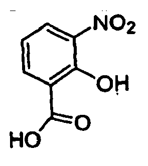 | 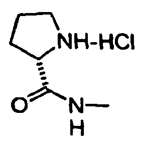 | 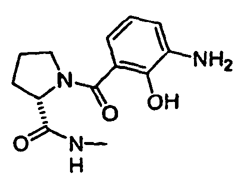 | 1. 15% |
| | | | | 2. 264.0 |
| | | | | 3. Skipped step B |

### PREPARATIVE EXAMPLE 124

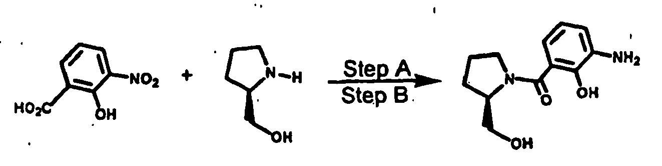

### Step A

3-Nitrosalicylic acid (500 mg, 2.7 mmol), 1,3-dicyclohexylcarbodiimide (DCC) (563 mg) and ethyl acetate (10 mL) were combined and stirred for 10 min. (*R)*-(-)-2-pyrrolidinemethanol (0.27 mL) was added and the resulting suspension was stirred at room temperature overnight. The solid was filtered off and the filtrate was either concentrated down and directly purified or washed with 1N NaOH. The aqueous phase was acidified and extracted with EtOAc. The resulting organic phase was dried over anhydrous MgSO₄, filtered and concentrated *in vacuo.* Purification of the residue by preparative plate chromatography (silica gel, 5% MeOH/CH₂Cl₂ saturated with AcOH) gave the desired compound (338 mg, 46%, MH⁺ = 267).

### Step B

The product from Step A above was stirred with 10% Pd/C under a hydrogen gas atmosphere overnight. The reaction mixture was filtered through celite, the filtrate concentrated *in vacuo,* and the resulting residue purified by column chromatography (silica gel, 4% MeOH/CH₂Cl₂ saturated with NH₄OH) to give the product (129mg, 43%, MH+=237).

### PREPARATIVE EXAMPLES 125-145

Following the procedure described for Preparative Example 124, but using the commercially available amine or the amine from the Preparative Example indicated and 3-nitrosalicylic acid, the products in the table below were obtained.

| Ex. | Amine Comm.Avait./ From Prep. Ex. | Product | 1.Yield (%) |
|---|---|---|---|
| | | | 2. (M+1)⁺ |
| 125 | 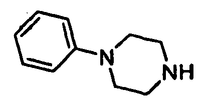 | 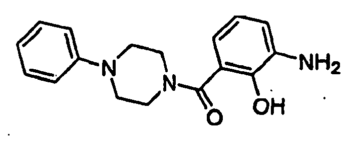 | 1. 37% |
| | | | 2. 298.1 |
| 126 | 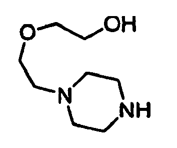 | 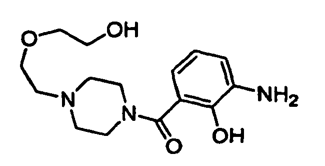 | 1. 31 % |
| | | | 2. 310.1 |
| 127 | 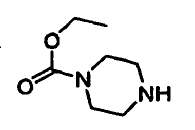 | 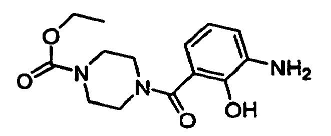 | 1. 68% |
| | | | 2. 294.1 |
| 128 | 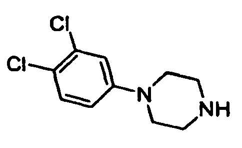 | 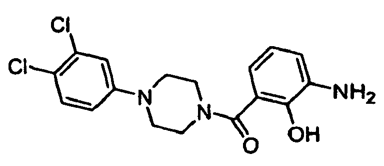 | 1. 54% |
| | | | 2. 365.9 |
| 129 | 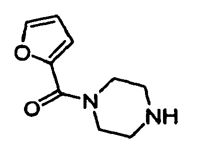 | 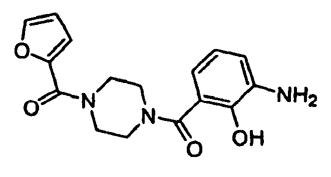 | 1. 45% |
| | | | 2. 316.1 |
| 130 | 110 | 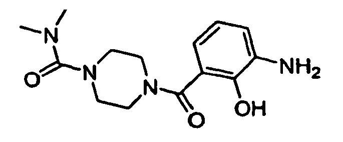 | 1. 59% |
| | | | 2. 293.1 |
| 131 | 111 | 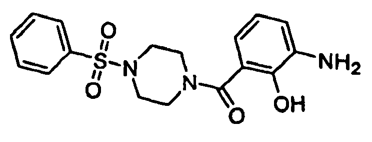 | 1. 32% |
| | | | 2. 362.0 |
| 132 | 114 | 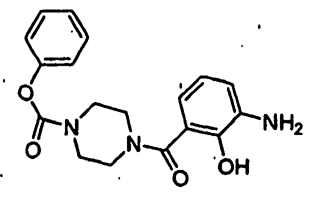 | 1. 36% |
| | | | 2. 342.0 |
| 133 | 112 | 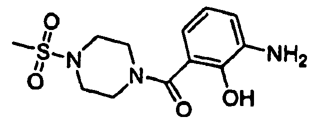 | 1. 65% |
| | | | 2. 300.0 |
| 134 | 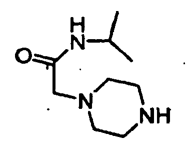 | 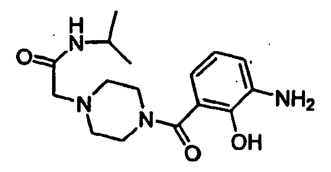 | 1. 48% |
| | | | 2. 321.1 |
| 135 | 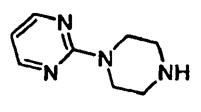 | 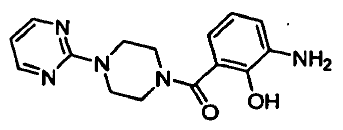 | 1. 50% |
| | | | 2. 300.1 |
| 136 | 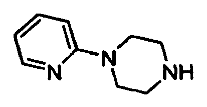 | 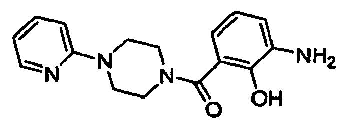 | 1. 56% |
| | | | 2. 299.2 |
| 137 | 115 | 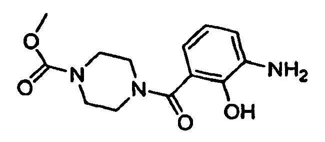 | 1. 79% |
| | | | 2. 280.1 |
| 138 | 116 | 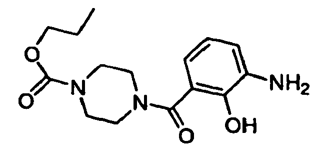 | 1. 64% |
| | | | 2. 307.1 |
| 139 | 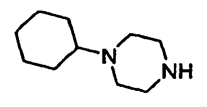 | 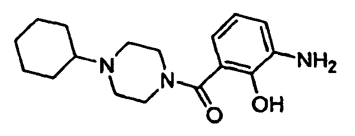 | 1. 73% |
| | | | 2. 304.2 |
| 140 | 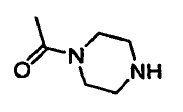 | 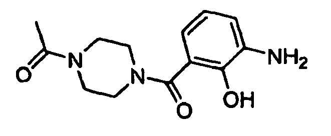 | 1. 34% |
| | | | 2. 264.0 |
| 141 | 117 | 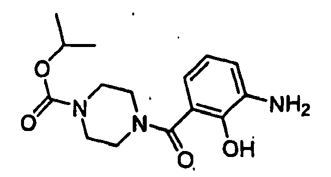 | 1. 40% |
| | | | 2. 307.1 |
| | | | |
| 142 | 113 | 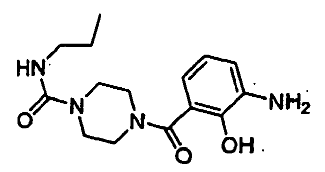 | 1. 91% |
| | | | 2. 307.1 |
| 143 | 118 | 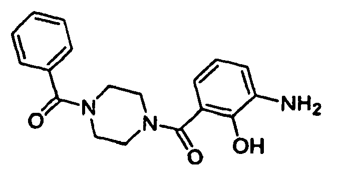 | 1. 9,0% |
| | | | 2. 326.0 |
| 144 | 119 | 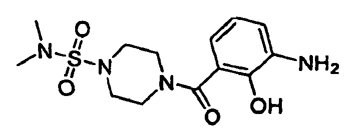 | 1. 42% |
| | | | 2. 329.0 |
| 145 | | 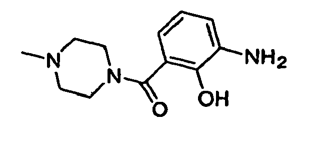 | 1. 6.5% |
| | | | 2. 236.1 |

### PREPARATIVE EXAMPLE 146

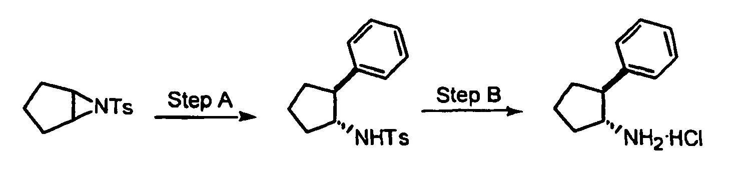

### Step A

To a solution of tosylaziridine [J. Am. Chem. Soc. 1998, 120, 6844-6845) (0.5 g, 2.1 mmol) and Cu(acac)2 (55 mg, 0.21 mmol) in THF (5 mL) at 0 °C was added PhMgBr (3.5 ml, 3.0 M in THF) diluted with THF (8 mL) dropwise over 20 min. The resulting solution was allowed to gradually warm to rt and was stirred for 12h. Sat. aq. NH₄Cl (5 mL), was added and the mixture was extracted with Et₂O (3 x 15 mL). The organic layers were combined, washed with brine (1x10 mL), dried (MgSO₄) and concentrated under reduced pressure. The crude residue was purified by preparative TLC eluting with hexane/EtOAc (4:1) to afford 0.57 g (86% yield) of a solid. The purified tosylamine was taken on directly to the next step.

### Step B

To a solution of tosylamine (0.55 g, 1.75 mmol) in NH₃ (20 mL) at -78°C was added sodium (0.40 g, 17.4 mmol). The resulting solution was stirred at -78 °C for 2 h whereupon the mixture was treated with solid NH₄Cl and allowed to warm to rt. Once the NH₃ had boiled off, the mixture was partitioned between water (10 mL) and CH₂Cl₂ (10 mL). The layers were separated and the aqueous layer was extracted with CH₂Cl₂ (2 x10 mL). The organic layers were combined,), dried (NaSO₄), and concentrated under reduced pressure to a volume of ∼20 mL. 4N HCl in dioxane (5 mL) was added and the mixture was stirred for 5 min. The mixture was concentrated under reduced pressure and the resultant crude residue was recrystallized from EtOH/Et₂O to afford 0.30 g (87% yield) of a solid.

### PREPARATIVE EXAMPLES 147-156.10

Following the procedure set forth in Preparative Example 146 but using the requisite tosylaziridines and Grignard reagents listed in the Table below, the following racemic amine hydrochloride products were obtained.

| Prep Ex. | Tosyl aziridine | Grignard Reagent | Amine hydrochloride | 1.Yield (%) |
|---|---|---|---|---|
| 147 | 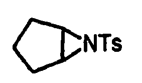 | MeMgBr | 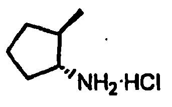 | 1. 19% |
| 148 | 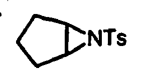 | EtMgBr | 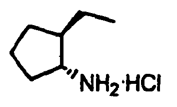 | 1. 56% |
| 149 | 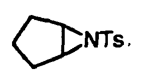 | *n*-PrMgBr | 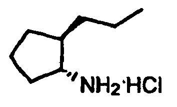 | 1. 70% |
| 150 | 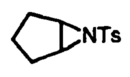 | *i*-PrMgCl | 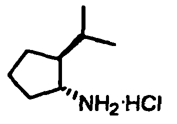 | 1. 41% |
| 151 | 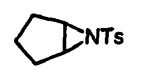 | BnMgCl | 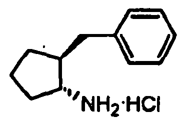 | 1. 61% |
| 152 | 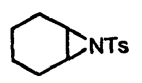 | MeMgBr | 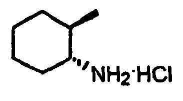 | 1. 61% |
| 153 | 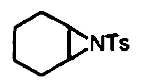 | EtMgBr | 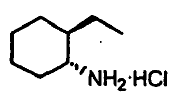 | 1. 66% |
| 154 | 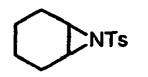 | *n*-PrMgBr | 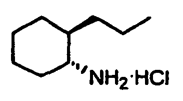 | 1. 80% |
| 155 | 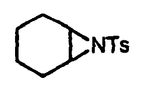 | *i*-PrMgBr | 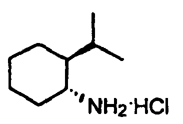 | 1. 27% |
| 156 | 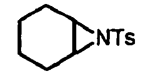 | BnMgCl | 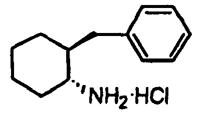 | 1. 79% |
| 156.1 | 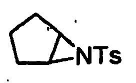 | 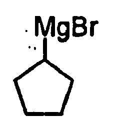 | 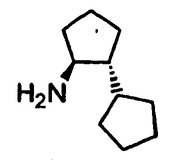 | 52% |
| 156.2 | 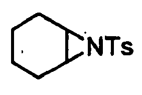 | 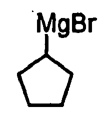 | 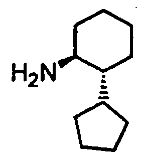 | 49% |
| 156.3 | 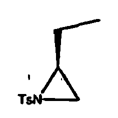 | 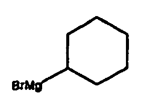 | 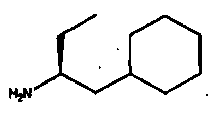 | 61% |
| 156.4 | 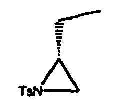 | 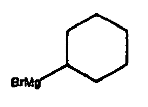 | 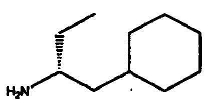 | 57% |
| 156.5 | 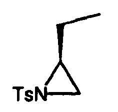 | 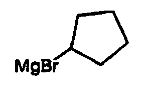 | 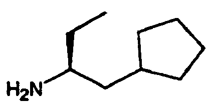 | 64% |
| 156.6 | 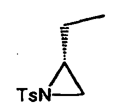 | 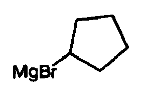 | 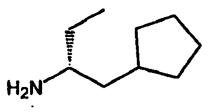 | 64% |
| 156.7 | 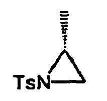 | 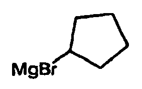 | 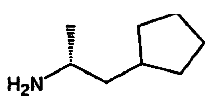 | 45% |
| 156.8 | 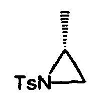 | 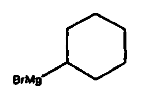 | 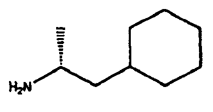 | 23% |
| 156.9 | 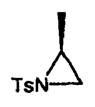 | 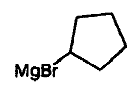 | 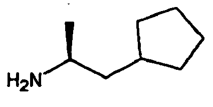 | 40% |
| 156.10 | 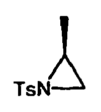 | 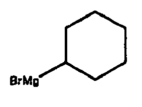 | 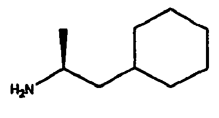 | 15% |

### PREPARATIVE EXAMPLE 156.11

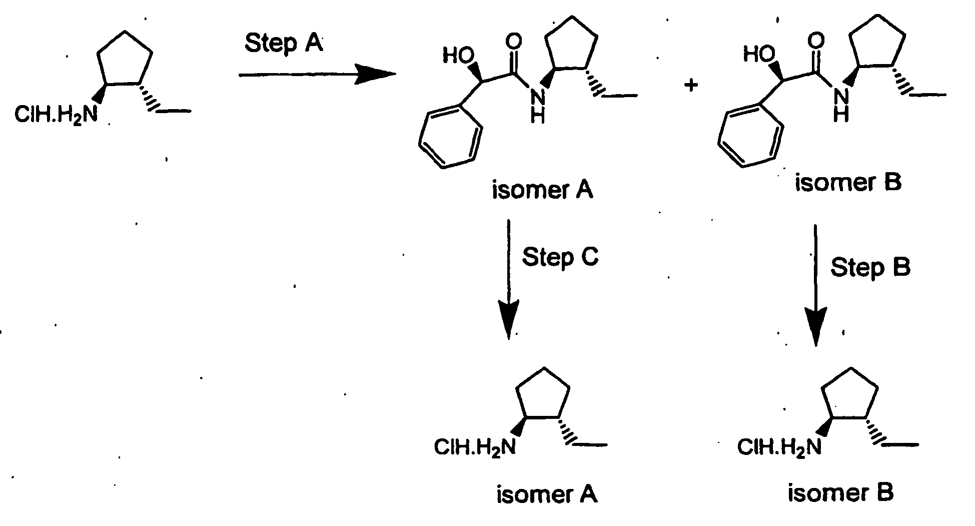

### Step A

To a solution of the amine (118mg) from Preparative Example 148 in CH₂Cl₂ (10ml) was added triethylamine (120ul), R-Mandelic Acid (164mg), DCC (213mg) and DMAP (8.8mg)and let stir for 40hr. The mixture was diluted with CH₂Cl₂ and washed with saturated ammonium chloride, dried over Na₂SO₄, filtered, and concentrated *in vacuo.* The crude material was purified by preparative plate chromatography (Hex/EtOAc 4:1) to afford both isomers (A, 86mg, 45%) (B, 90mg, 48%).

### Step B

To isomer B (90mg) from above in dioxane (5ml) was added 6M H₂SO₄ (5ml). The reaction was heated to 80°C over the weekend. 2M NaOH added to basify the reaction and extracted with ether. Ether layer washed with brine, dried over Na₂SO₄, filtered, and concentrated *in vacuo.* The residue was stirred in 4N HCl in dioxane for 30min, concentrated *in vacuo* and recrystallized in EtOH/ether to afford 55mg of product (98%).

### Step C

Isomer A (86mg) was reacted..following the procedure set forth in Step B above to give the amine salt.

### PREPARATIVE EXAMPLE 156.12

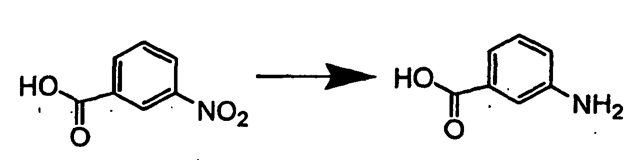

The above nitro compound was reduced following the Preparative Example 2, Step B.

### PREPARATIVE EXAMPLE 156.13

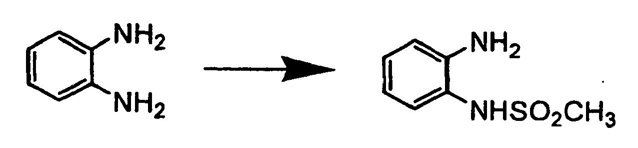

To a solution of 1,2-phenylenediame (1.5g) in CH₂Cl₂ (30ml) at 0°C was added TEA (2.91 ml), followed by dropwise addition of MeSO₂Cl (1.07ml). The mixture was allowed to warm to room temperature and stir overnight. 1M HCl added and the layers were separated. The aqueous layer was adjusted to pH=11 with solid NaOH, extracted with CH₂Cl₂. The basified aqueous layer was then neutralized using 3N HCl and extracted with CH₂Cl₂, dried with Na₂SO₄, filtered, and concentrated *in vacuo* to give 1.8g of product (71%).

### PREPARATIVE EXAMPLE 156.14

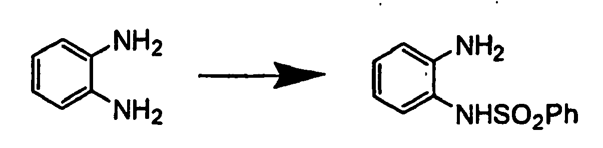

The above compound was prepared using the procedure set forth in Preparative Example 156.13, but using PhSO₂Cl.

### PREPARATIVE EXAMPLE 156.15

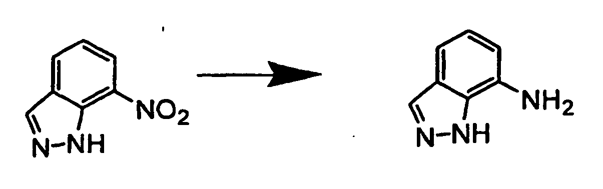

The nitro compound was reduced following a similar procedure as in Preparative Example 2, Step B.

### PREPARATIVE EXAMPLE 156.16

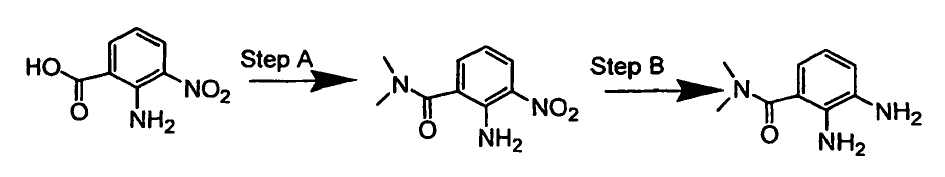

### Step A

The known acid (410mg) above (J.Med.Chem. 1996, 34,4654.) was reacted following the procedure set forth in Preparative Example 2, Step A to yield 380mg of an oil (80%).

### Step B

The amide (200mg) from above was reacted following the procedure set forth in Preparative Example 2, Step B to yield 170mg of an oil (100%).

### PREPARATIVE EXAMPLE 156.17

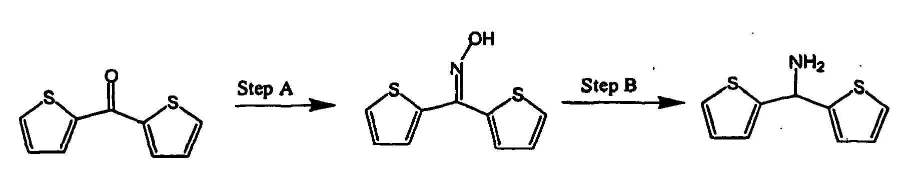

### Step A

To a solution of ketone (500mg) in EtOH/water (3:1, 4ml) at room temperature was added hydroxylamine hydrochloride (214mg) followed by NaOH to afford a heterogenous mixture. The reaction was not complete so another equivalent of hydroxylamine hydrochloride was added and refluxed overnight. The reaction was cooled to 0°C and treated with 3N HCl and extracted with CH₂Cl_{2.} washed with brine, dried over Na₂SO₄, filtered, and concentrated *in vacuo* to give 500mg of product (92%).

### Step B

To a solution of oxime (300mg) in THF (5ml) at 0°C was added LiAlH₄ (266mg) portionwise. The heterogenous solution was stirred at room temperature for 14hr and then refluxed for 8hr. The solution was cooled to 0°C and water, 2M NaOH, water and ether were added to the reaction. The mixture was filtered through a celite pad. The filtrate was treated with 3N HCl. The aqueous layer was cooled to 0°C, basified with NaOH pellets and extracted with ether. The ether layer was dried over MgSO₄, filtered, and concentrated *in vacuo* to afford the product (143mg, 69%).

### PREPARATIVE EXAMPLE 156.18

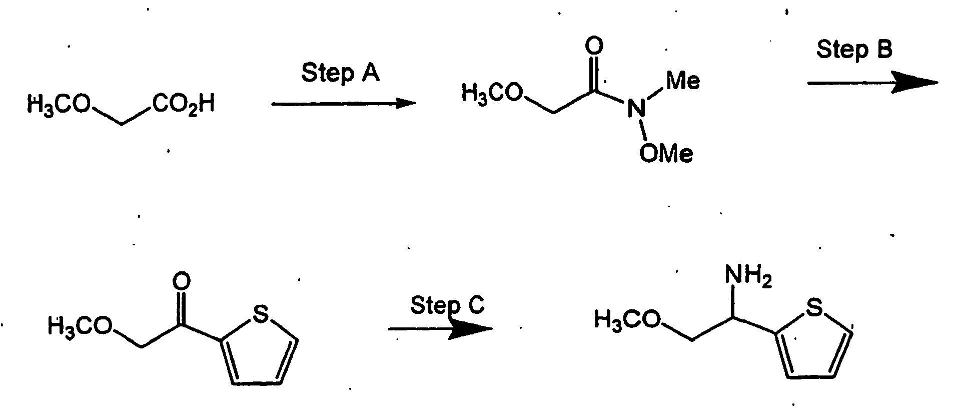

### Step A

Methoxyacetic acid (14 mL) in CH₂Cl₂ (120 mL) and cooled in an ice-water bath was treated with DMF (0.9 mL) and oxalyl chloride (21 mL). After stirring at RT overnight, the mixture was concentrated *in vacuo* and redissolved in CH₂Cl₂ (120 mL). N-methyl-N-methoxylamine (20 g) was added and the mixture stirred at RT overnight. Filtration and concentration *in vacuo* afforded the desired amide (21 g, 89%).

### Step B

To a solution of the above amide (260mg) in THF (5ml) at -78 °C was added a solution of 2-thienyllithium (1M in THF, 2.15m1). The solution was stirred for 2hr at -78 °C and warmed to -20 °C for an additional 2hr. The reaction was quenched with saturated ammonium chloride and extracted with CH₂Cl_{2.} washed with brine, dried over Na₂SO₄, filtered, and concentrated *in vacuo* to give 250mg of product (82%).

### Step C

The ketone from above (250mg) was reacted via the procedure set forth in Preparative Example 156.17 Steps A and B to yield 176 mg of the amine (79%).

### PREPARATIVE EXAMPLE 156.19

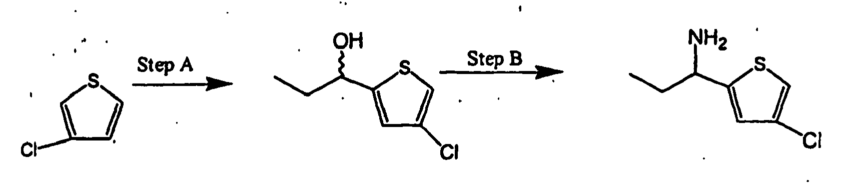

### Step A

To a solution of 3-chlorothiophene (1.16ml) in ether (20ml) at -10 °C was added n-BuLi (2.5M in hexane, 5ml). After solution was stirred at -10°C for 20min, propionaldehyde (0.82ml) in ether (20ml) was added dropwise and let warm to room temperature slowly. The reaction was quenched with saturated ammonium chloride and extracted with CH₂Cl₂, washed with brine, dried over Na₂SO₄, filtered, and concentrated *in vacuo* to give 1.37g of product (62%).

### Step B

The alcohol from Step A above was reacted via the procedures set forth in Preparative Example 75.75, Steps B and C to give the amine.

### PREPARATIVE EXAMPLE 156.20

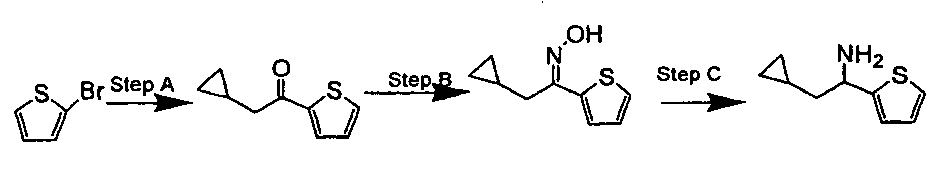

### Step A

To a solution of magnesium metal (360mg) in THF (15ml) at 0°C was added 2-bromothiophene (1.45ml) in THF (10ml) dropwise over 20min. The solution was warmed to room temperature for 3hr, recooled to 0°C whereupon a solution of cyclopropylacetonitrile (1g) in ether (30ml) was added dropwise via a syringe and let warm to room temperature and stir overnight. 3M HCl was added and washed with CH₂Cl₂. The aqueous layer was basified with NaOH pellets and extracted with ether, dried with Na₂SO₄, filtered, and concentrated *in vacuo* to give 625mg of product (68%).

### Step B

The ketone was reacted via the procedure set forth in Preparative Example 156.17 Step A to give the oxime.

### Step C

The oxime from above was reacted via the procedure set forth in Preparative Example 156.17 Step B to give the amine.

### PREPARATIVE EXAMPLE 156.21

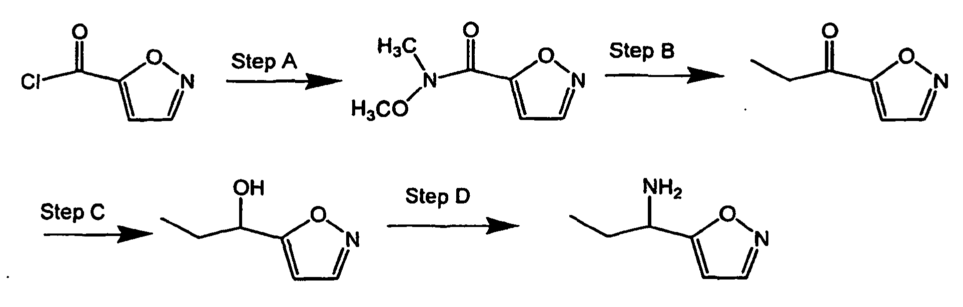

### Step A

To a solution of -CH₃ONHCH₃.HCl (780mg) and acid chloride (1g) in CH₂Cl₂ at 0 °C was added dry pyridine (1.35ml) to afford a heterogenous mixture, The solution was warmed to room temperature and stirred overnight. 1M HCl was added to the reaction and the organic layer was separated, washed with brine, dried with Na₂SO₄, filtered, and concentrated *in vacuo* to give 1g of product (85%).

### Step B

To a solution of Etl (614ul) in ether (5ml) at -78°C was added t-BuLi (1.7M in pentane, 9ml) dropwise. The mixture was warmed to room temperature for 1hr, cooled to -78°C where the amide (1g) from Step A in THF (4ml) was added and allowed to warm to 0 °C for 2hr. 1M HCl was added to the reaction and extracted with CH₂Cl₂, washed with brine, dried with Na₂SO₄, filtered, and concentrated *in vacuo* to give 500mg of product (63%).

### Step C

To a solution of ketone (800mg) in THF/water (10:1, 20ml) at 0°C was added sodium borohydride (363mg) portionwise. The solution was stirred for 2hr at 0 °C. The mixture was concentrated *in vacuo,* the residue was dissolved in CH₂Cl₂, washed with 1N NaOH and brine, dried with Na₂SO₄, filtered, and concentrated *in vacuo* to give 560mg of product (69%).

### Step D

The alcohol from above was reacted via the procedures set forth in Preparative Example 75.75, Steps B and C to give the amine (176mg, 59%).

### PREPARATIVE EXAMPLE 156.22

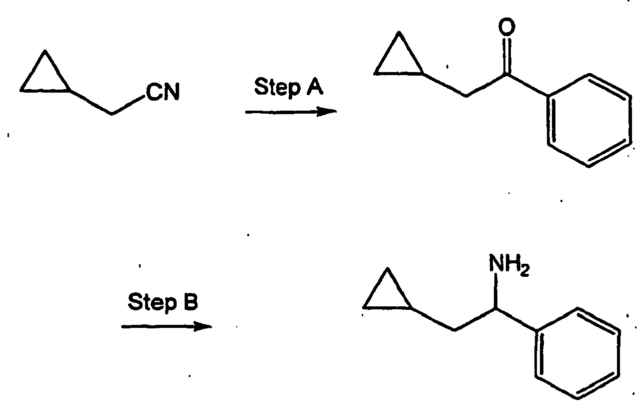

### Step A

Cyclopropylacetonitrile (12 mmol) in Et₂O (50 mL) at 0°C was treated with PhMgBr (14 mmol) and the mixture was stirred for 2 hrs at 0°C, then at RT overnight. Hydrochloric acid (3 *M*) was added, and after stirring for an additional 12 hrs, the mixture was extracted with CH₂Cl₂, washed with brine, dried over Na₂SO₄, filtered and concentrated *in vacuo* to give the desired ketone (1.34 g, 70%).

### Sten B

Following the procedures set forth in Preparative Example 156.20 Steps B and C, the amine was prepared.

### PREPARATIVE EXAMPLE 156.23

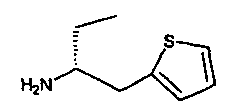

The above amine was prepared using the procedures set forth in WO Patent Publication 98/11064.

### PREPARATIVE EXAMPLE 157

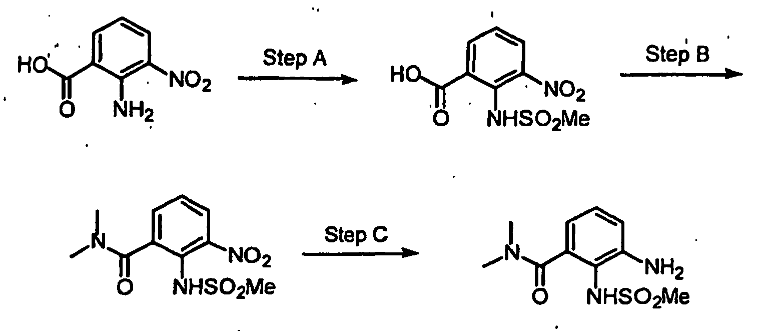

### Step A

By taking the known carboxylic acid [J. Med. Chem. 1996, 39, 4654-4666] and subjecting it to the conditions outlined in Preparative Example 112, the product can be prepared.

### Step B

Following a similar procedure used in Preparative Example 2, Step A, except using dimethylamine and the compound from Step A above, the product can be prepared.

### Step C

Following a similar procedure used in Preparative Example 2, Step B, except using the compound from Step B above, the product can be prepared.

### PREPARATIVE EXAMPLE 158

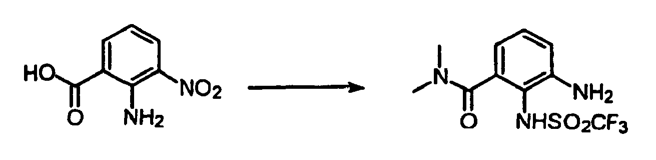

Following a similar procedure used in Preparative Example 157, Steps A-C, except using trifluoromethylsulfonylchloride in Step A above, the product can be prepared.

### PREPARATIVE EXAMPLE 500.1

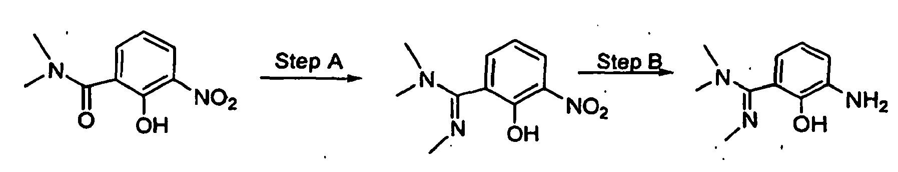

### Step A

By using the nitro-amide from Preparative Example 13.3, Step A, the amidine structure can be prepared following a similar procedure to that in Tetrahedron Lett., 2000, 41 (11), 1677-1680.

### Step B

By using the product from Step A and the procedure set forth in Preparative Example 2, Step B, one could obtain the desired amine-amidine.

### ALTERNATE PREPARATIVE EXAMPLE 500.2

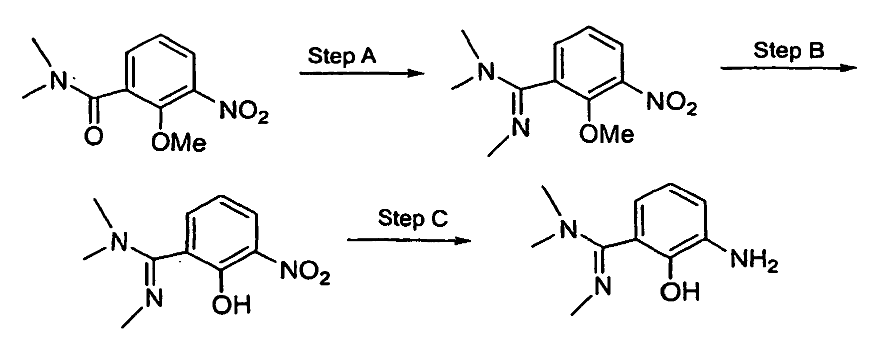

### Step A

By treating the nitro-amide from Preparative Example 13.3, Step B with POCl₃ and subsequently MeNH₂, according to procedures known in the art, one would obtain the desired compound.

### Step B

By treating the product from Step A according to the procedure set forth in Preparative Example 13.3, Step E, one could obtain the desired compound.

### Step C

By using the product from Step B and the procedure set forth in Preparative Example 2 Step B, one would obtain the desired compound.

### PREPARATIVE EXAMPLE 500.3

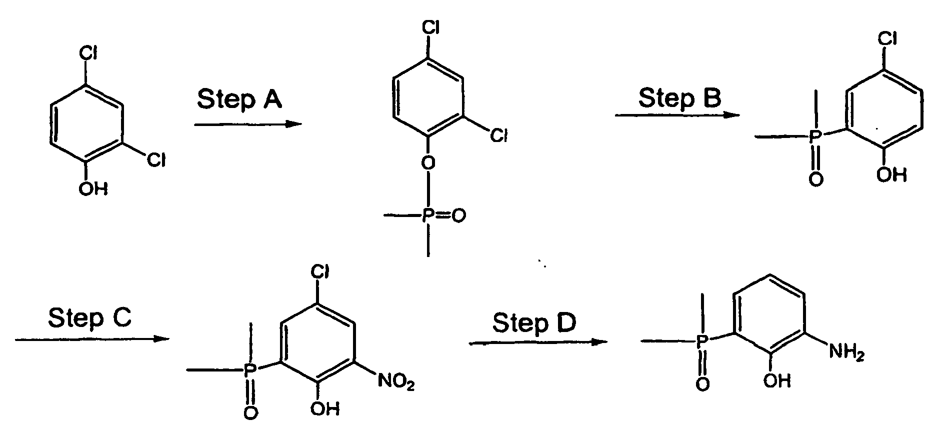

### Step A

By following a similar procedure as that described in Zh. Obshch. Khim., 27, 1957, 754, 757., but instead using 2,4-dichlorophenol and dimethylphosphinic chloride, one would obtain the desired compound.

### Step B

By following a similar procedure as that described in J. Organomet. Chem.; 317, 1986, 11-22, one would obtain the desired compound.

### Step C

By following a similar procedure as that described in J. Amer. Chem. Soc., 77, 1955, 6221, one would obtain the desired compound.

### Step D

By following a similar procedure as that described in J. Med. Chem., 27, 1984, 654-659, one would obtain the desired compound.

### ALTERNATE PREPARATIVE EXAMPLE 500.4

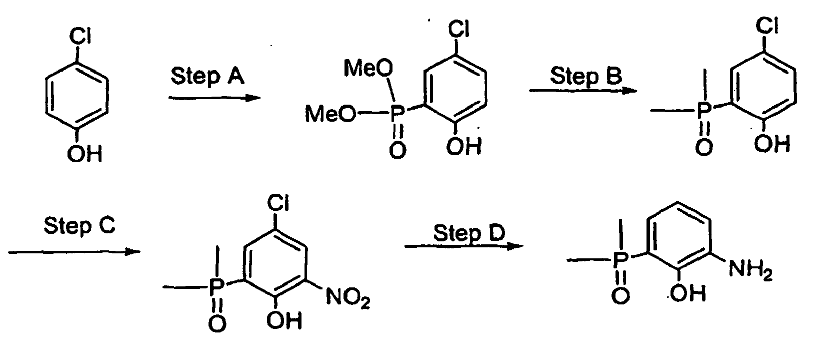

### Step A

By following a similar procedure as that described in Phosphorous, Sulfur Silicon Relat. Elem.; EN; 61, 12, 1991, 119-129, but instead using 4-chlorophenol, one would obtain the desired compound.

### Step B

By using a similar procedure as that in Phosphorous, Sulfur Silicon Relat. Elem.; EN; 61, 12, 1991, 119-129, but instead using MeMgBr, the desired compound could be prepared.

### Step C

By following a similar procedure as that described in J. Amer. Chem. Soc., 77, 1955, 6221, one would obtain the desired compound.

### Step D

By following- a similar procedure as that described in J. Med. Chem., 27,1984, 654-659, one would obtain the desired compound.

### PREPARATIVE EXAMPLE 500.5

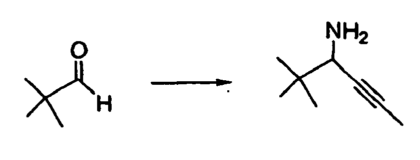

By following a similar procedure as that set forth in J. Org. Chem. 1998, 63, 2824-2828, but using CH₃CCMgBr, one could obtain the desired compound.

### PREPARATIVE EXAMPLE 500.6

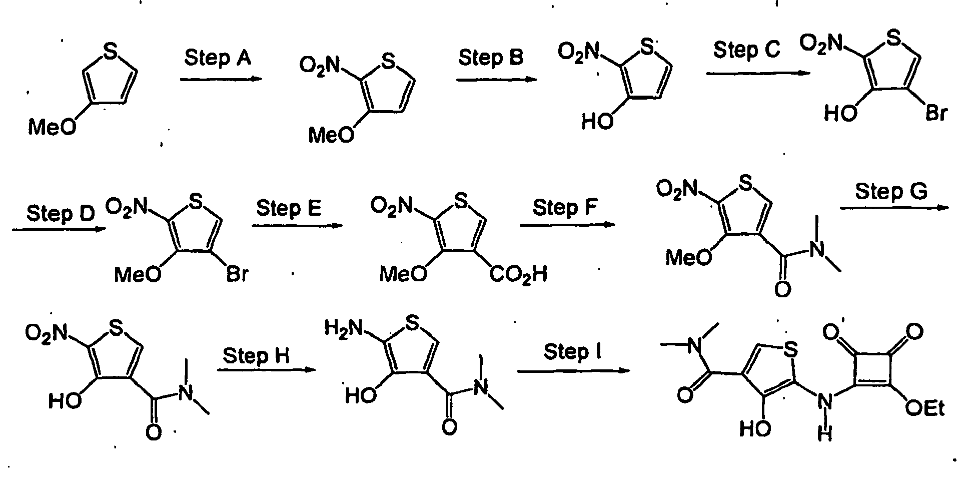

### Step A

By following the procedure set forth in Preparative Example 13.1, Step B using 3-methoxythiophene, one can obtain the desired product.

### Step B

By using the product from step A and following the procedure set forth in Preparative Example 13.19, Step E, the desired compound can be obtained.

### Step C

By using the product from Step B and following the procedure set forth in Preparative Example 13.20, Step A, one can obtain the desired compound.

### Step D

By using the product from Step C and following the procedure set forth in Preparative Example 13.3, Step B, the desired compound can be obtained.

### Step E

By treating the product from Step D with n-BuLi at -78°C in THF and quenching the resulting anion with CO₂ according to standard literature procedure, one would obtain the desired compound following aqueous acid work up.

### Step F

By using the product from Step E and the procedure set forth in Prepartive Example 13.19, Step C, one could obtain the desired compound.

### Step G

By using the product from step F and following the procedure set forth in Preparative Example 13.19, Step E, the desired compound can be obtained.

### Step H

By using the product from Step G and following the procedure set forth in Preparative Example 2, Step B, the desired compound can be obtained.

### Step I

By using the product from Step H and following the procedure set forth in Preparative Example 19, the desired compound can be prepared.

### EXAMPLE 200

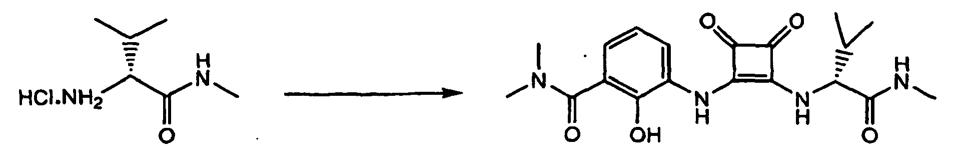

To a solution of the HCl salt product (83 mg, 0.50 mmol) from Preparative Example 24, in EtOH (3 mL) at room temperature was added Et₃N (55 µL, 0.50 mmol) and the mixture was stirred for 10 min. The cyclobutenedione (100 mg, 0.33 mmol) from Preparative Example 19 in EtOH was then added in a single portion and the mixture was stirred for 12 h at room temperature.
The mixture was concentrated under reduced pressure and was purified by preparative TLC (4 x 1000 µM plates) eluting with CH₂Cl₂/MeOH (25: 1) to afford 116 mg (91 % yield) of the desired product as a solid [MH+ 389.1, mp 241-243 °C].

### EXAMPLES 201-209

Following the procedure set forth in Preparative Example 200 but using the appropriate amine hydrochlorides from Preparative Examples 25-33 as identified and the cyclobutenedione intermediate from Preparative Example 19, the cyclobutenedione products in the Table below were obtained.

| Ex. | (Prep Ex.) Amine | Product | 1. Yield (%) |
|---|---|---|---|
| | | | 2. MH⁺ |
| | | | 3. mp (°C) |
| 201 | 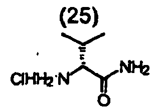 | 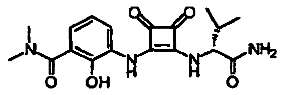 | 1. 89% |
| | | | 2. 375.1 |
| | | | 3. 255.5-257.3 |
| 202 | 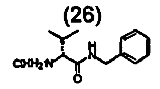 | 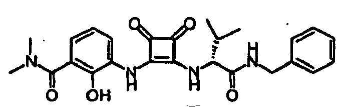 | 1. 92% |
| | | | 2. 465.1 |
| | | | 3. 149.0-152.3 |
| 203 | 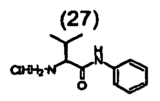 | 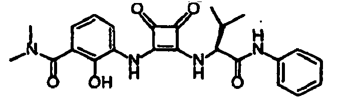 | 1. 68% |
| | | | 2. 451.1 |
| | | | 3. 282-284 |
| 204 | 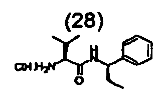 | 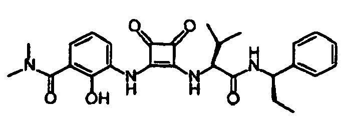 | 1. 74% |
| | | | 2. 493.1 |
| | | | 3. 141 |
| 205 | 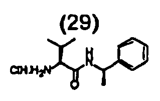 | 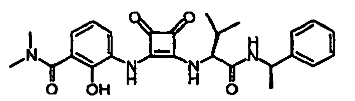 | 1. 48% |
| | | | 2. 479.1 |
| | | | 3. 142 |
| 206 | 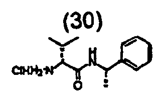 | 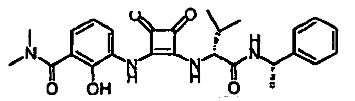 | 1. 41% |
| | | | 2. 479.1 |
| | | | 3. 142 |
| 207 | 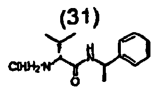 | 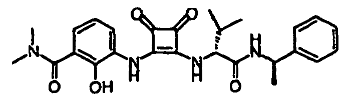 | 1. 59% |
| | | | 2. 479.1 |
| | | | 3. 141 |
| 208 | 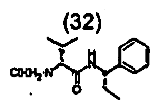 | 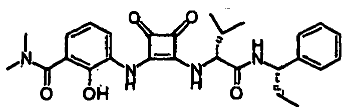 | 1. 34% |
| | | | 2. 493.1 |
| | | | 3. 140 |
| 209 | 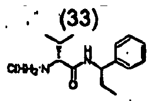 | 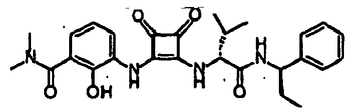 | 1. 40% |
| | | | 2. 493.1 |
| | | | 3. 142 |
| 209.1 | (33.1) | 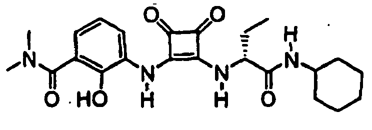 | 1. 59% |
| | | | 2. 143-145 |

### EXAMPLE 209.2

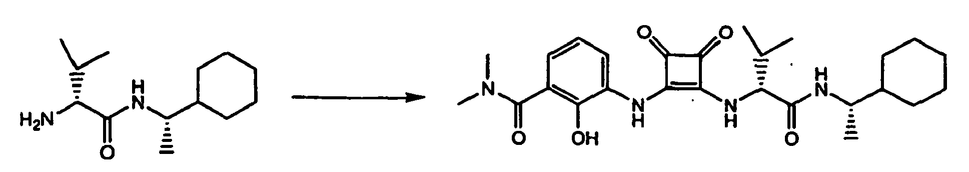

The crude amine product from Preparative Example 33.2 and the cyclobutendione component from Preparative Example 19.1 (36mg) were dissolved in MeOH/ DIEA (2.5ml/5/1) and irradiated via microwave (50W, 1 hr). The reaction was concentrated *in vacuo* and purified by Gilson semi-prep. HPLC to give the final product (68%, MH+=485.2).

### EXAMPLES 209.3-209.50

Following the procedure set forth in Example 209.2, but using the prepared amine from the Preparative Example indicated in the Table below, the following cyclobutenedione products were obtained.

| Ex. | (Prep Ex.) | Product | 1. Yield (%) |
|---|---|---|---|
| | Amine | | 2. MH⁺ |
| 209.3 | (33.3) | 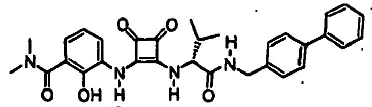 | 1. 50% |
| | | | 2. 541.2 |
| 209.4 | (33.4) | 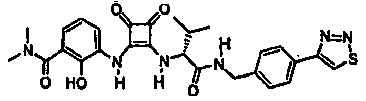 | 1. 32% |
| | | | 2. 549.1 |
| 209.5 | (33.5) | 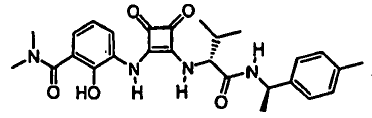 | 1. 65% |
| | | | 2. 493.1 |
| 209.6 | (33.6) | 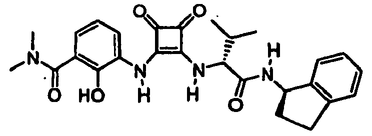 | 1. 64%. |
| | | | 2. 491.1 |
| 209.10 | (33.7) | 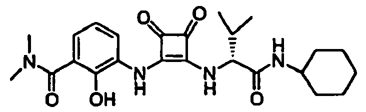 | 1. 90% |
| | | | 2. 457.2 |
| 209.11 | (33.8) | 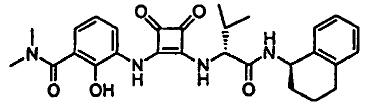 | 1. 35% |
| | | | 2. 505.0 |
| 209.12 | (33.9) | 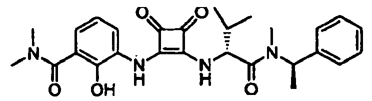 | 1. 70% |
| | | | 2. 493.1 |
| 209.13 | (33.10) | 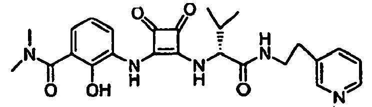 | 1. 75% |
| | | | 2. 480.2 |
| 209.14 | (33.11) | 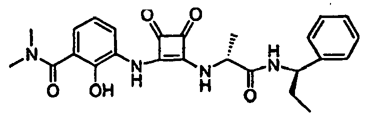 | 1. 74% |
| | | | 2. 465.1 |
| 209.15 | (33.12) | 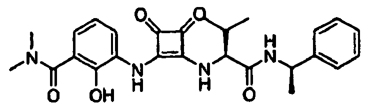 | 1. 62% |
| | | | 2. 479.1 |
| 209.16 | (33.13) | 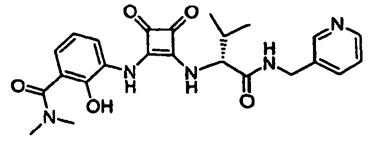 | 1. 31% |
| | | | 2. 466.2 |
| 209.17 | (33.14) | 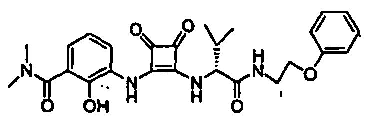 | 1. 79% |
| | | | 2. 495.2 |
| 209.18 | (33.15) | 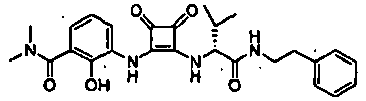 | 1. 99% |
| | | | 2. 479.2 |
| 209.19 | (33.16) | 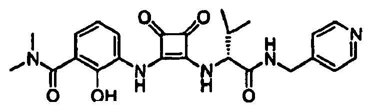 | 1. 47% |
| | | | 2. 466.2 |
| 209.20 | (33.17) | 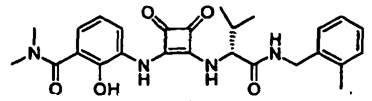 | 1. 72% |
| | | | 2. 479.1 |
| 209.21 | (33.18) | 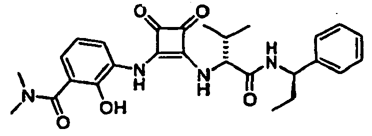 | 1. 92% |
| | | | 2. 493.1 |
| 209.22 | (33.19) | 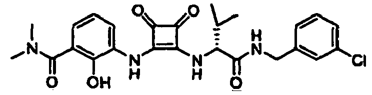 | 1. 47% |
| | | | 2. 499.1 |
| 209.23 | (33.20) | 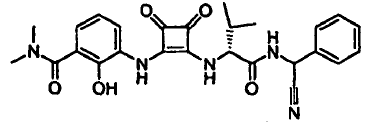 | 1. 7% |
| | | | 2. 490.0 |
| 209.24 | (33.21) | 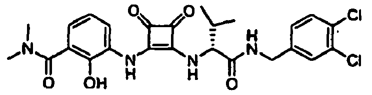 | 1. 15% |
| | | | 2. 533.1 |
| 209.25 | (33.22) | 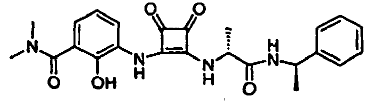 | 1. 88% |
| | | | 2. 451.1 |
| 209.26 | (33.23) | 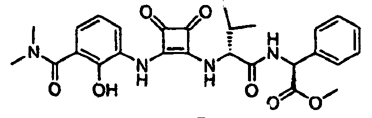 | 1. 26% |
| | | | 2. 523.0 |
| 209.27 | (33.24) | 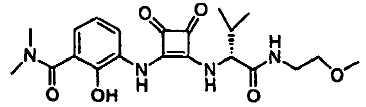 | 1. 54% |
| | | | 2. 433.1 |
| 209.28 | (33.25) | 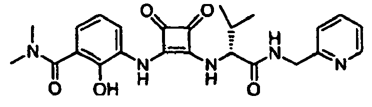 | 1. 59% |
| | | | 2. 466.2 |
| 209.29 | (33.26) | 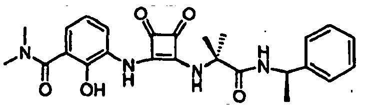 | 1. 66% |
| | | | 2. 560.2 |
| 209.30 | (33.27) | 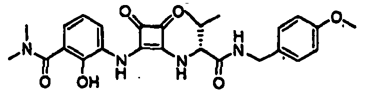 | 1.98% |
| | | | 2. 495.1 |
| 209.31 | (33.28) | 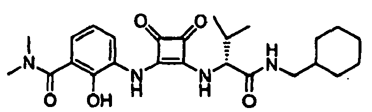 | 1.99%. |
| | | | 2.471.2 |
| 209.32 | (33.29) | 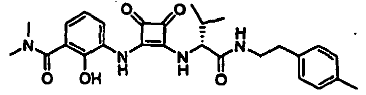 | 1.99% |
| | | | 2.471.2 |
| 209.33 | (33.30) | 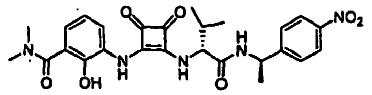 | 1.18% |
| | | | 2. 524.2 |
| 209.34 | (33.31) | 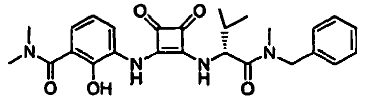 | 1.78% |
| | | | 2.479.2 |
| 209.35 | (33.32) | 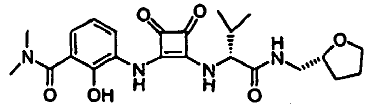 | 1.71% |
| | | | 2. 459.2 |
| 209.36 | (33.33) | 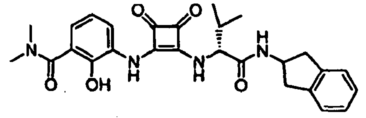 | 1.5% |
| | | | 2. 491.0 |
| 209.37 | (33.34) | 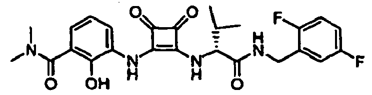 | 1. 27% |
| | | | 2. 501.1 |
| 209.38 | (33.35) | 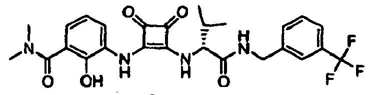 | 1. 26% |
| | | | 2. 533.1 |
| 209.39 | (33.36) | 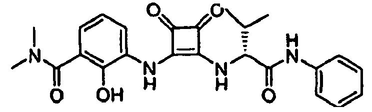 | 1. 48% |
| | | | 2. 451.1 |
| 209.40 | (33.37) | 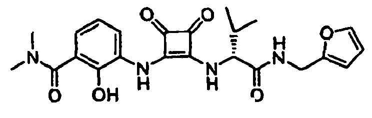 | 1. 99% |
| | | | 2. 455.1 |
| 209.41 | (33.38) | 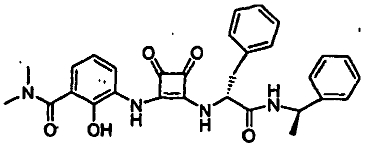 | 1. 88% |
| | | | 2. 527.1 |
| 209.42 | (33.39) | 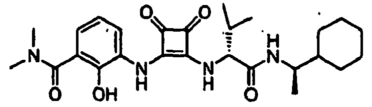 | 1.74% |
| | | | 2.485.2 |
| 209.43 | (33.40) | 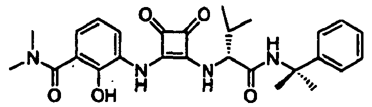 | 1.20% |
| | | | 2.492.5 |
| 209.44 | (33.41) | 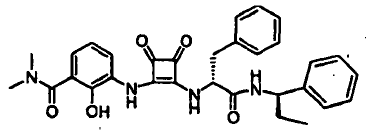 | 1. 68% |
| | | | 2.541.1 |
| 209.45 | (33.42) | 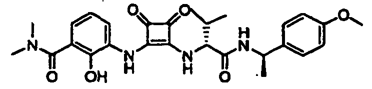 | 1.13% |
| | | | 2. 508.9 |
| 209.46 | (33.43) | 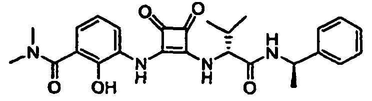 | 1. 86% |
| | | | 2. 479.1 |
| 209.47 | (33.44) | 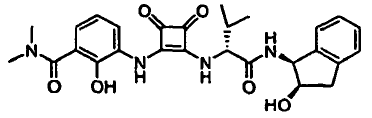 | 1. 34% |
| | | | 2. 507.0 |
| 209.48 | (33.45) | 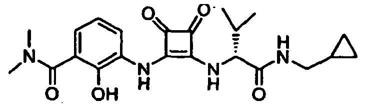 | 1. 56% |
| | | | 2. 429.1 |
| 209.49 | (33.46) | 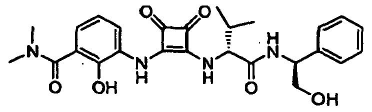 | 1.18% |
| | | | 2. 495.0 |
| 209.50 | (33.47) | 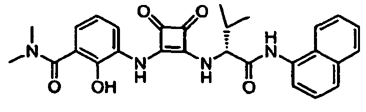 | 1. 22% |
| | | | 2. 501.0 |

### EXAMPLE 210

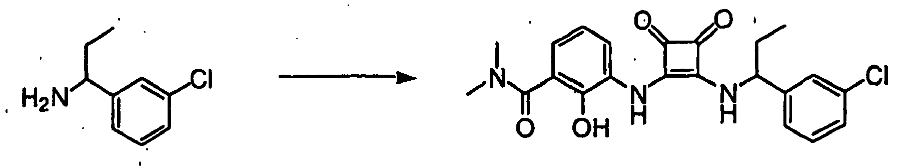

To a solution of amine (0.17 g, 1 mmol) from Preparative Example 34 in EtOH (3 mL) at room temperature was added the cyclobutenedione from Preparative Example 19 (100 mg, 0.33 mmol) in one portion. The resulting mixture was stirred for 5h (until TLC analysis revealed reaction complete) and was concentrated under reduced pressure. The crude residue was redissolved in CH₂Cl₂ (15 mL) and was washed sequentially with 10% KH₂PO₄ (2 x 15 mL) and brine (1 x 15 mL). The organic layer was dried (Na₂SO₄) and concentrated under reduced pressure to afford the crude adduct. The crude product was purified by prep TLC (4 x 1000 uM plates) eluting with CH₂Cl₂/MeOH (20:1) to afford 83 mg (59% yield) of the desired product as a solid.

### EXAMPLES 211-260

Following the procedure set forth in Example 210 but using the commercially available amine or the prepared amine from the Preparative Example indicated in the Table below, the following cyclobutenedione products were obtained.

| Ex. | (Prep Ex) | Product | 1. Yield (%) |
|---|---|---|---|
| | Amine | | 2. MH⁺ |
| | | | 3.mp (°C) |
| 211 | 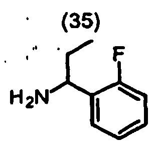 | 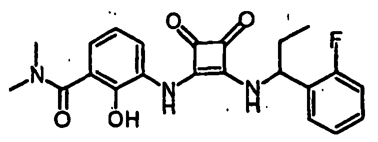 | 1. 75% |
| | | | 2. 412:1 |
| | | | 3. 126 |
| 212 | 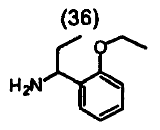 | 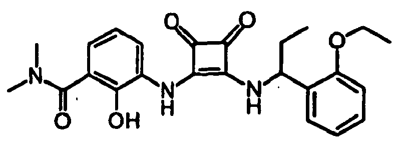 | 1. 42% |
| | | | 2. 438.1 |
| | | | 3. 106 |
| 213 | 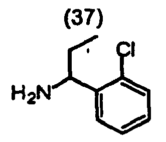 | 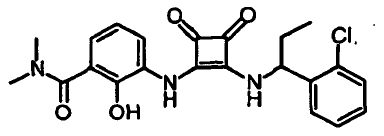 | 1. 73% |
| | | | 2. 428.1 |
| | | | 3. 139 |
| 214 | 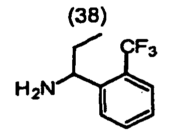 | 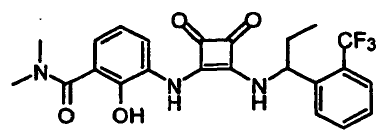 | 1. 40% |
| | | | 2. 462.1 |
| | | | 3. 160 |
| 215 | 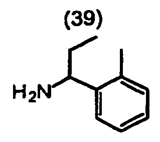 | 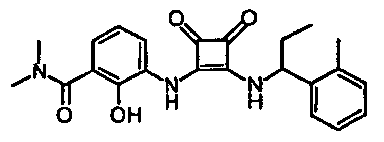 | 1.52% |
| | | | 2. 408.1 |
| | | | 3. 126 |
| 216 | 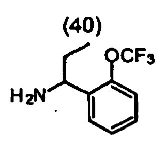 | 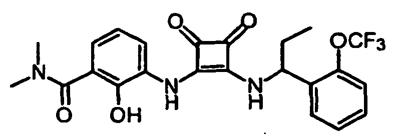 | 1. 32% |
| | | | 2. 478.1 |
| | | | 3. 176 |
| 217 | 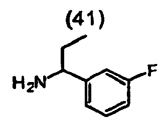 | 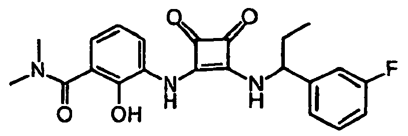 | 1. 50% |
| | | | 2. 412.1 |
| | | | 3. 126 |
| 218 | 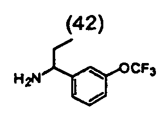 | 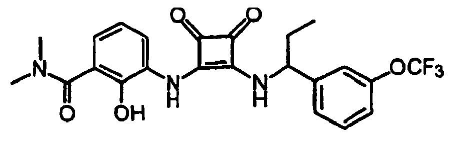 | 1. 55% |
| | | | 2. 478.1 |
| | | | 3. 110 |
| 219 | 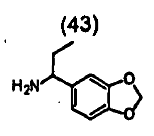 | 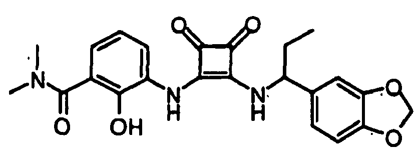 | 1. 67% |
| | | | 2. 438.1 |
| | | | 3. 122 |
| 220 | 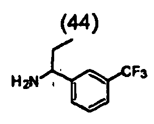 | 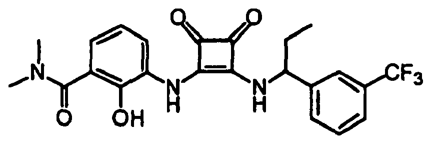 | 1. 73% |
| | | | 2. 462.1 |
| | | | 3. 118 |
| 221 | 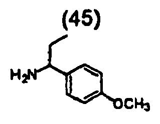 | 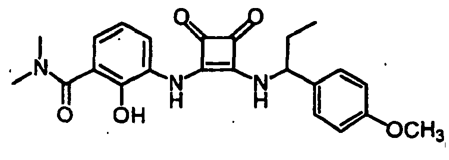 | 1. 67% |
| | | | 2. 424:1 |
| | | | 3. 100 |
| 222 | 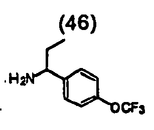 | 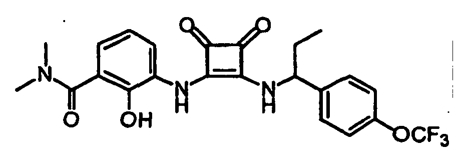 | 1. 61% |
| | | | 2. 478.1 |
| | | | 3. 114 |
| 223 | 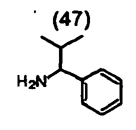 | 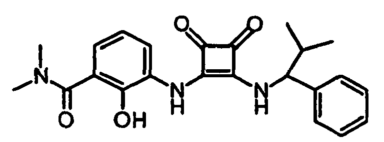 | 1. 50% |
| | | | 2. 408.1 |
| | | | 3. 157-159 |
| 224 | 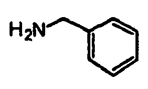 | 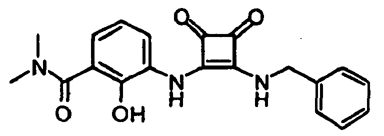 | 1. 75% |
| | | | 2. 366.1 |
| | | | 3. 110-112 |
| 225 | 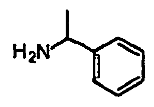 | 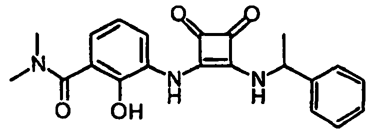 | 1. 81% |
| | | | 2. 380.1 |
| | | | 3. 118-120 |
| 226 | 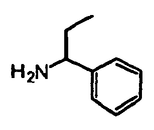 | 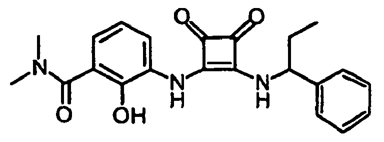 | 1. 69% |
| | | | 2. 394.1 |
| | | | 3. 123-125 |
| 227 | 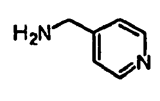 | 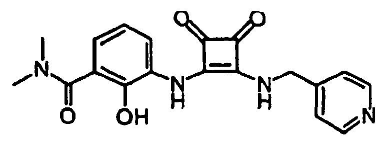 | 1. 80% |
| | | | 2. 367.1 |
| | | | 3. 122-125 |
| 228 | 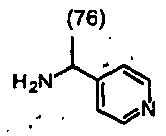 | 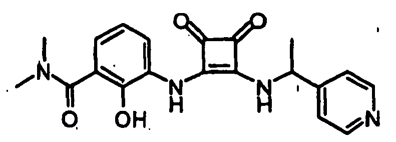 | 1. 72% |
| | | | 2. 381.1 |
| | | | 3. 133-135 |
| 229 | 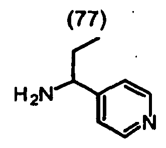 | 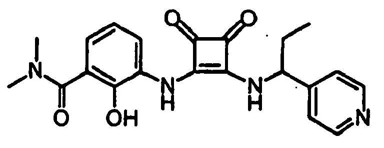 | 1. 81% |
| | | | 2. 395.1 |
| | | | 3. 141-143 |
| 230 | 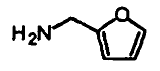 | 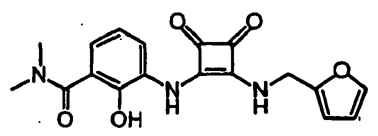 | 1. 75% |
| | | | 2. 356.1 |
| | | | 3. 103-104 |
| 231 | 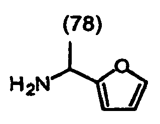 | 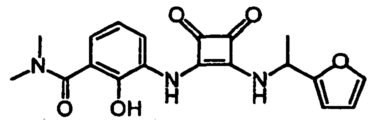 | 1. 24% |
| | | | 2. 370.1 |
| | | | 3. 101 |
| 232 | 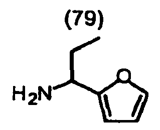 | 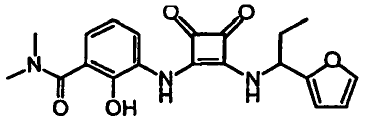 | 1. 16% |
| | | | 2. 384.1 |
| | | | 3. 70 |
| 233 | 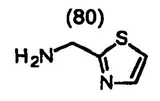 | 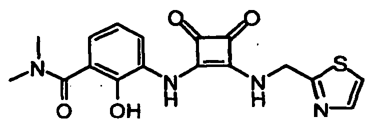 | 1. 72% |
| | | | 2. 373.4 |
| | | | 3. 104-106 |
| 234 | 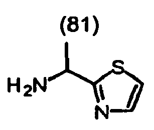 | 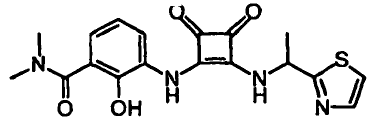 | 1. 34% |
| | | | 2. 387.1 |
| | | | 3. 99 |
| 235 | 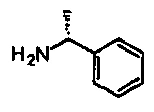 | 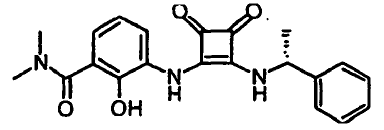 | 1. 48% |
| | | | 2. 380.1 |
| | | | 3. 118-120 |
| 236 | 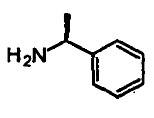 | 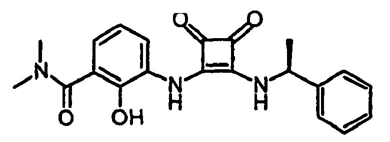 | 1. 72% |
| | | | 2. 380.1 |
| | | | 3. 119-120 |
| 237 | 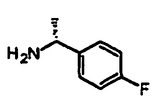 | 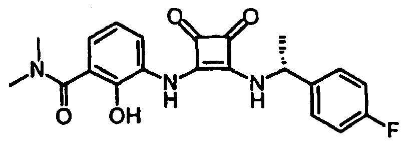 | 1. 72% |
| | | | 2. 398.1 |
| | | | 3. 121-123 |
| 238 | 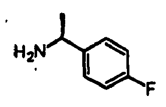 | 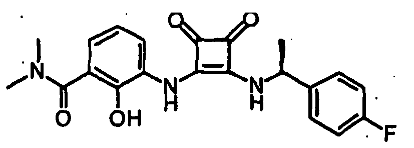 | 1. 44% |
| | | | 2. 398.1 |
| | | | 3. 121-123 |
| 239 | 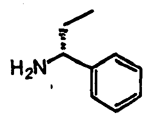 | 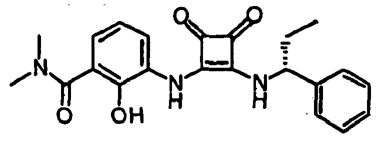 | 1. 60% |
| | | | 2. 394.1 |
| | | | 3. 123-124 |
| 240 | 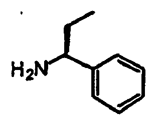 | 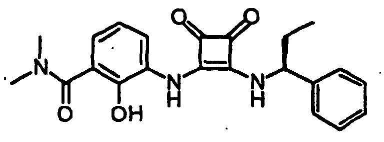 | 1. 52% |
| | | | 2. 394.1 |
| | | | 3. 122-124 |
| 241 | 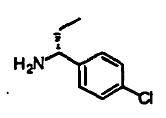 | 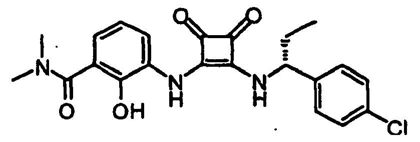 | 1. 34% |
| | | | 2. 428.4 |
| | | | 3. 157-159 |
| 242 | 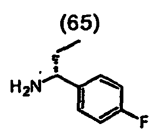 | 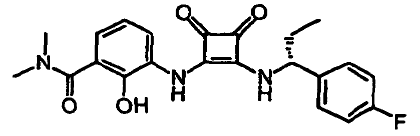 | 1. 70% |
| | | | 2. 412.1 |
| | | | 3. 109-112 |
| 243 | 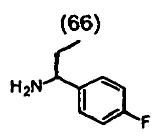 | 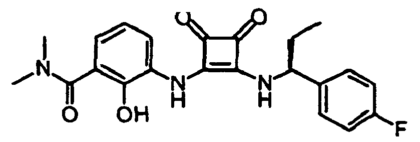 | 1. 69% |
| | | | 2. 412.1 |
| | | | 3. 110-112 |
| 244 | 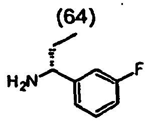 | 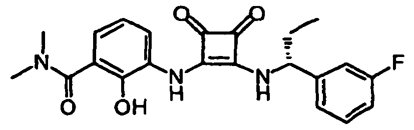 | 1. 89% |
| | | | 2. 412.1 |
| | | | 3. 126 |
| 245 | 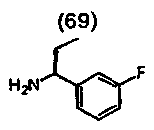 | 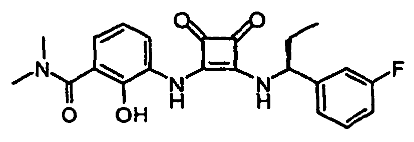 | 1. 81% |
| | | | 2. 412.1 |
| | | | 3. 126 |
| 246 | 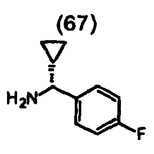 | 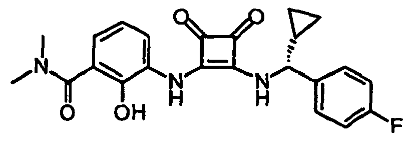 | 1. 65% |
| | | | 2. 424.1 |
| | | | 3. 121-124 |
| 247 | 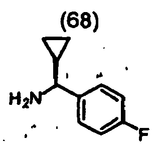 | 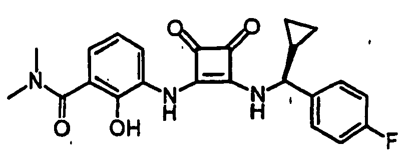 | 1. 73% |
| | | | 2. 424.4 |
| | | | 3. 122-124 |
| 248 | 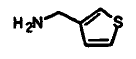 | 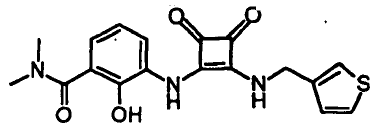 | 1. 29% |
| | | | 2. 372.1 |
| | | | 3. 219-221 |
| 249 | 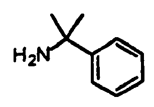 | 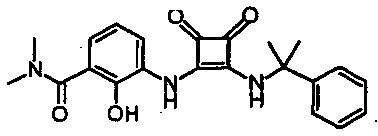 | 1. 66% |
| | | | 2. 394.1 |
| | | | 3. 132-135 |
| 250 | 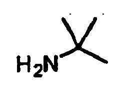 | 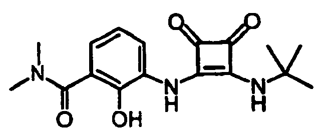 | 1. 72% |
| | | | 2. 332 |
| 251 | 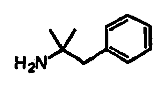 | 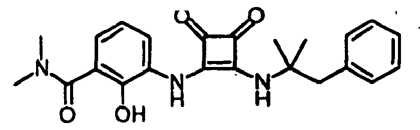 | 1. 74% |
| | | | 2. 408.1 |
| | | | 3. 121-123 |
| 252 | 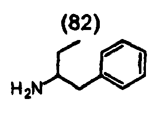 | 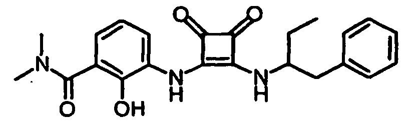 | 1. 76% |
| | | | 2. 408.1 |
| | | | 3. 102-104 |
| 253 | 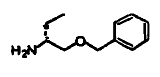 | 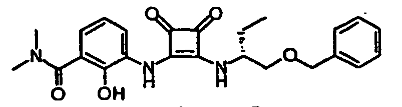 | 1. 72% |
| | | | 2. 438.1 |
| | | | 3. 75-77 |
| 254 | 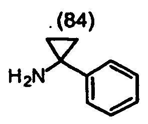 | 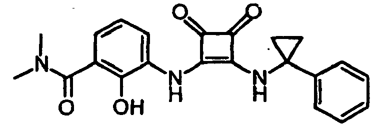 | 1. 80% |
| | | | 2. 392.1 |
| | | | 3. 98-101 |
| 255 | 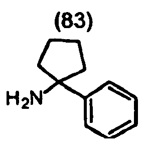 | 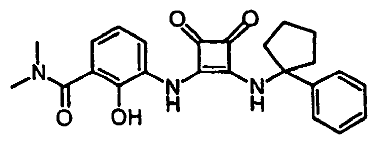 | 1. 72% |
| | | | 2. 420.1 |
| | | | 3. 200-205 |
| 256 | 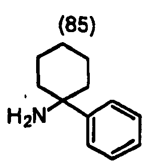 | 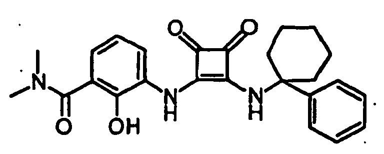 | 1. 75% |
| | | | 2. 434.1 |
| | | | 3. 138-140 |
| 257 | 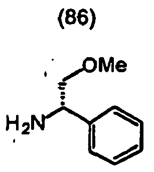 | 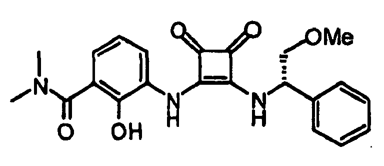 | 1. 67% |
| | | | 2. 410.1 |
| | | | 3. 116-118 |
| 258 | 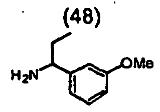 | 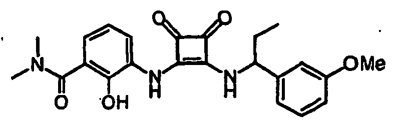 | 1. 76% |
| | | | 2. 424.1 |
| | | | 3. 108-110 |
| 259 | 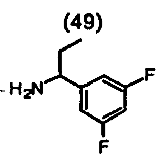 | 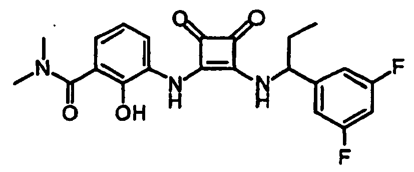 | 1. 72% |
| | | | 2. 430.1 |
| | | | 3. 125 |
| 260 | 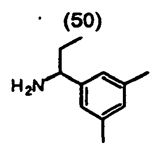 | 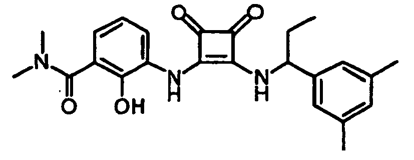 | 1. 78% |
| | | | 2. 422.1 |
| | | | 3. 127 |
| 260.1 | 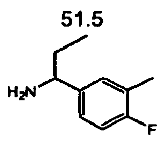 | 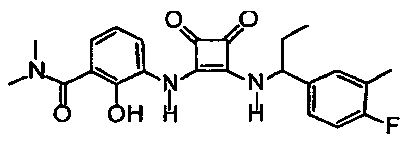 | 1. 74% |
| | | | 2. 426.1 |
| | | | 3. 114 DEC |
| 260.2 | 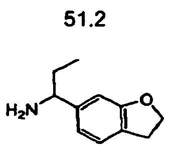 | 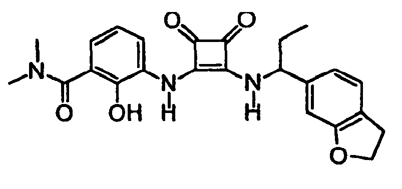 | 1. 85% |
| | | | 2. 436.1 |
| | | | 3. 143 DEC |
| 260.3 | 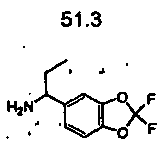 | 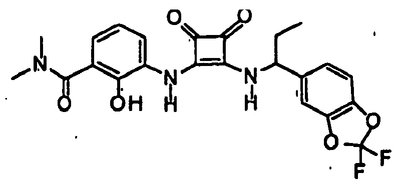 | 1. 56% |
| | | | 2. 474.1 |
| | | | 3. 121-123 |
| 260.4 | 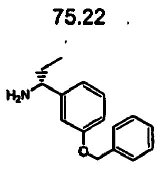 | 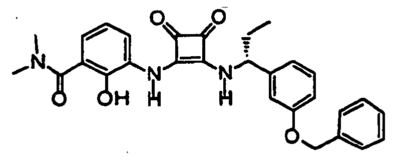 | 1. 71% |
| | | | 2. 500.1 |
| | | | 3. 97(DEC) |
| 260.6 | 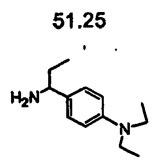 | 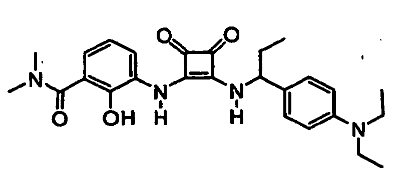 | 1. 61% |
| | | | 2. 465 |
| | | | 3. 102-107 |
| 260.7 | 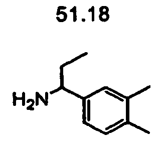 | 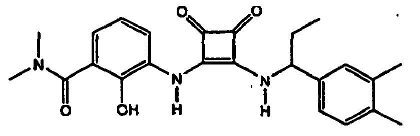 | 1. 78% |
| | | | 2. 422.1 |
| | | | 3.114 DEC |
| 260.8 | 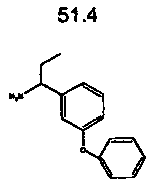 | 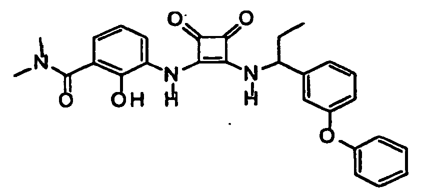 | 1. 35% |
| | | | 2. 486.1 |
| | | | 3. 103-105 |
| 260.9 | 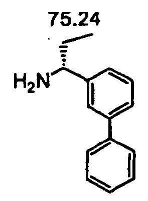 | 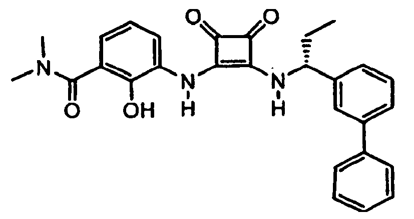 | 1. 79% |
| | | | 2. 470 |
| | | | 3. 110-115 |
| 260.10 | 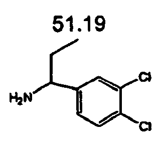 | 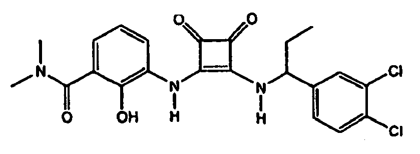 | 1. 62% |
| | | | 2. 462.1 |
| | | | 3. 110 DEC |
| 260,11 | 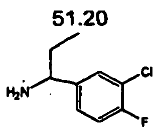 | 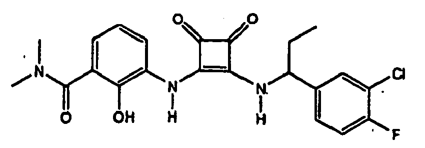 | 1. 61% |
| | | | 2. 446.1 |
| | | | 3. 118 DEC |
| 260.12 | 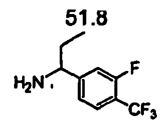 | 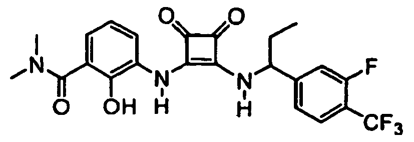 | 1. 58% |
| | | | 2. 480.1 |
| | | | 3. 111 DEC |
| 260.13 | 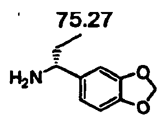 | 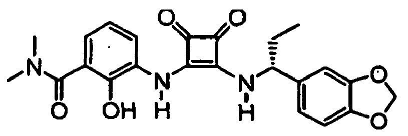 | 1. 87% |
| | | | 2. 438.1 |
| | | | 3. 122 |
| 260.14 | 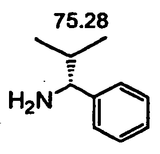 | 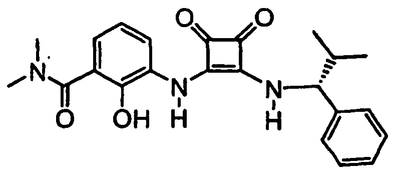 | 1. 74% |
| | | | 2. 408.1 |
| | | | 3. 128-130 |
| 260.15 | 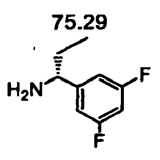 | 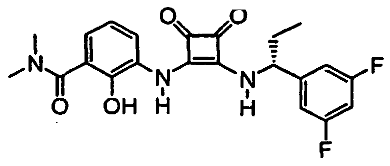 | 1. 78% |
| | | | 2. 430.1 |
| | | | 3. 117 DEC |
| 260.16 | 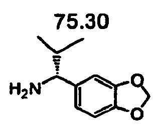 | 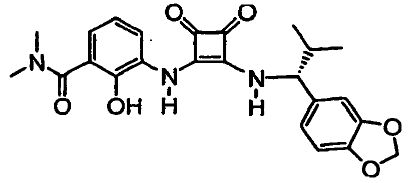 | 1. 81% |
| | | | 2. 452.1 |
| | | | 3. 139 |
| 260.17 | 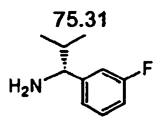 | 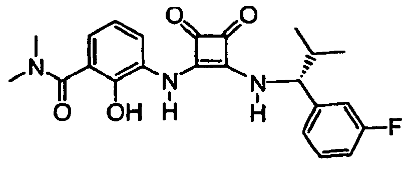 | 1. 85% |
| | | | 2. 426.1 |
| | | | 3. 126 |
| 260.18 | 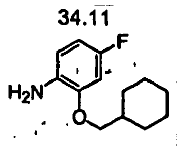 | 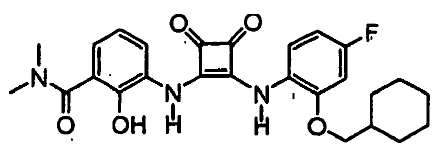 | 1. 50% |
| | | | 2. 482.1 |
| | | | 3. 114-116 |
| 260.19 | 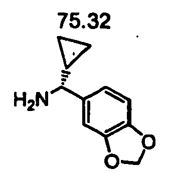 | 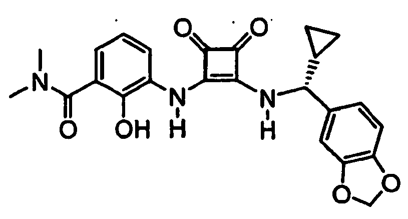 | 1. 64% |
| | | | 2. 450.1 |
| | | | 3. 129 |
| 260.20 | 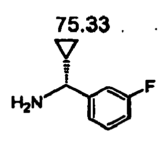 | 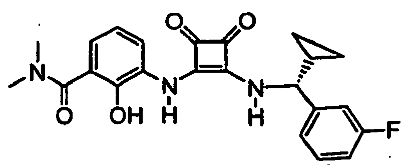 | 1. 72% |
| | | | 2. 424.1 |
| | | | 3. 116 |
| 260.21 | 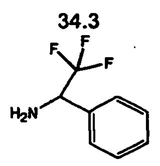 | 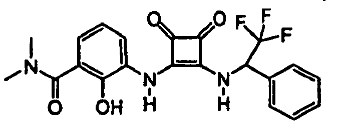 | 1. 35% |
| | | | 2. 434.1 |
| | | | 3. 124 |
| 260.22 | 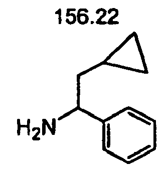 | 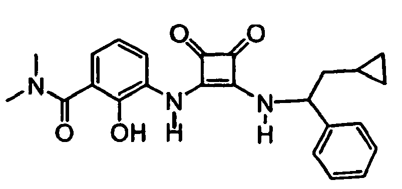 | 1. 58% |
| | | | 2. 420.1 |
| | | | 3. 107-109 |
| 260.23 | 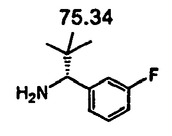 | 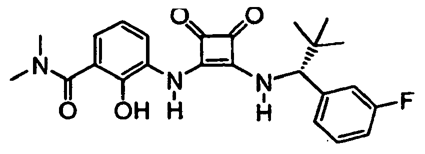 | 1. 69% |
| | | | 2. 440.1 |
| | | | 3. 169 |
| 260.24 | 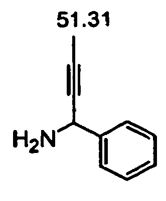 | 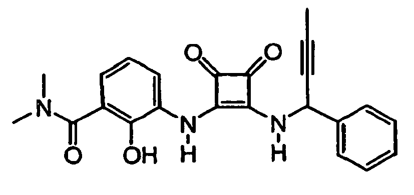 | 1. 15% |
| | | | 2. 404.1 |
| | | | 3. 103-105 |
| 260.25 | 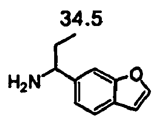 | 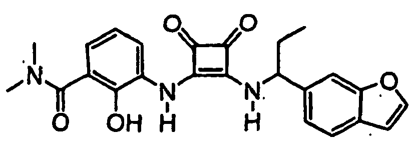 | 1. 92% |
| | | | 2. 434.1 |
| | | | 3. 129 |
| 260.26 | 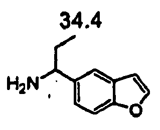 | 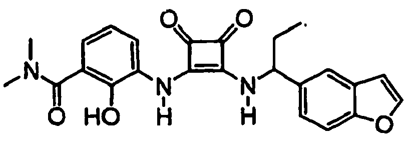 | 1. 77% |
| | | | 2. 434.1 |
| | | | 3. 133 |
| 260.27 | 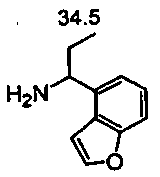 | 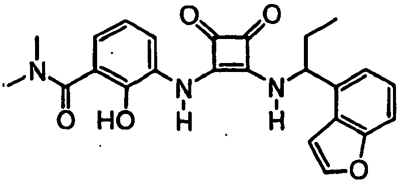 | 1. 73% |
| | | | 2. 434.1 |
| | | | 3. 138 |
| 260.28 | 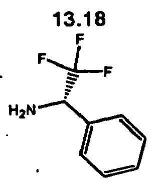 | 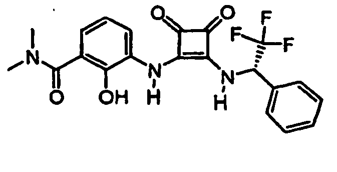 | 1. 37% |
| | | | 2. 434.1 |
| | | | 3. 133 |

### EXAMPLE 261

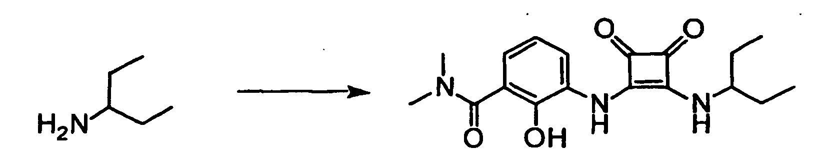

To a solution of the amine (77 µL, 0.66 mmol) in EtOH (3 mL) at room temperature was added the product from Preparative Example 19 (100 mg, 0.33 mmol) in one portion. The resulting mixture was stirred for 5h (until TLC analysis revealed reaction complete) and was then concentrated under reduced pressure. The crude residue was redissolved in CH₂Cl₂ (15 mL) and was washed sequentially with 10% KH₂PO₄ (2 x 15 mL) and brine (1 x 15 mL). The organic layer was dried (Na₂SO₄) and concentrated under reduced pressure to afford the crude adduct. The crude product was purified by prep TLC (4 x 1000 uM plates) eluting with CH₂Cl₂/MeOH (20:1) to afford 82 mg (72% yield) of the desired product as a solid. (mp 126.0-128.0 °C, MH⁺ 346)

### EXAMPLES 262 - 360.108

Following the procedure set forth in Example 261 but using the commercially available amine or the prepared amine from the Preparative Example indicated in the table below, the following cyclobutenedione products were obtained.

| Ex. | Amine | Product | 1.Yield (%) |
|---|---|---|---|
| | | | 2.MH⁺ |
| | | | 3. mp (°C) |
| 262 | 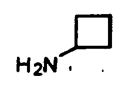 | 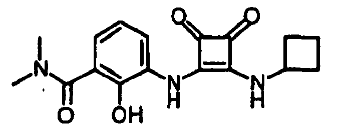 | 1. 74% |
| | | | 2. 330.1 |
| | | | 3. 112-115 |
| 263 | 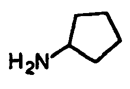 | 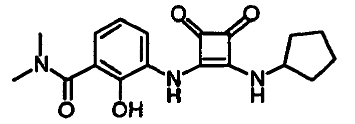 | 1. 64% |
| | | | 2. 344.1 |
| | | | 3. 120-122 |
| 264 | 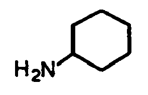 | 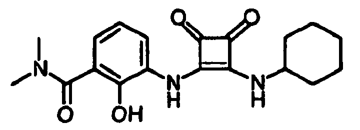 | 1. 72% |
| | | | 2. 358.4 |
| | | | 3. 129-132 |
| 265 | 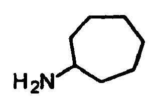 | 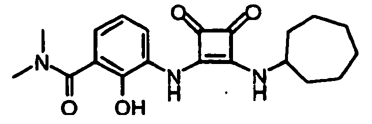 | 1. 76% |
| | | | 2. 372.1 |
| | | | 3. 141-143 |
| 266 | 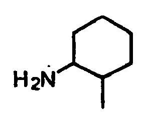 | 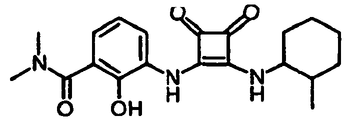 | 1. 57% |
| | | | 2. 372.1 |
| | | | 3. 102 |
| 267 | 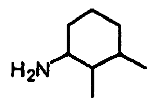 | 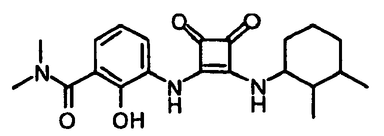 | 1. 65% |
| | | | 2. 386.1 |
| | | | 3. 146 |
| 268 | 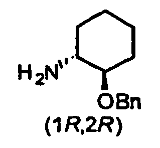 | 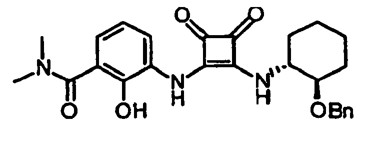 | 1. 65% |
| | | | 2. 464.1 |
| | | | 3. 110-112 |
| 269 | 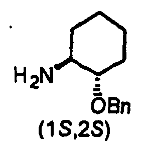 | 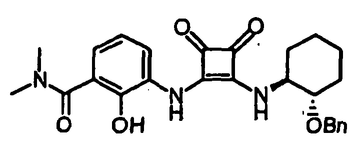 | 1. 85% |
| | | | 2. 464.1 |
| | | | 3. 111-113 |
| 270 | 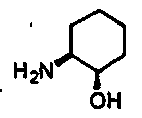 | 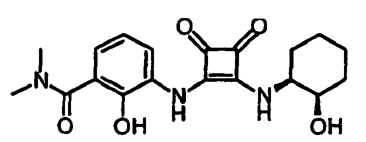 | 1. 49% |
| | | | 2. 374.1 |
| | | | 3. 146 |
| 271 | 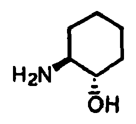 | 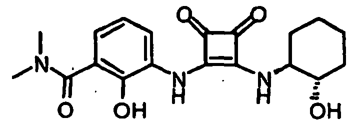 | 1. 69% |
| | | | 2. 374.1 |
| | | | 3. 158-162 |
| 272 | 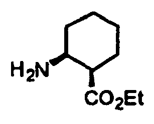 | 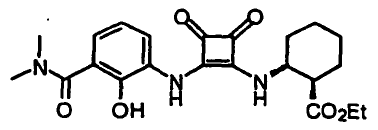 | 1. 54% |
| | | | 2. 430.1 |
| | | | 3. 108 |
| 273 | 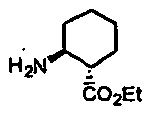 | 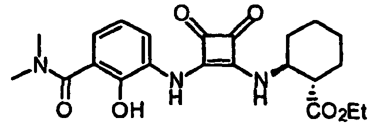 | 1. 65% |
| | | | 2. 430.1 |
| | | | 3. 110 |
| 274 | 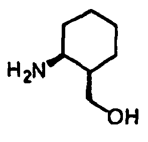 | 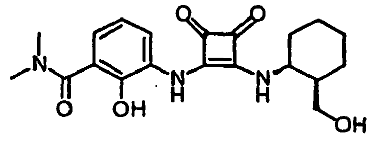 | 1. 53% |
| | | | 2. 388.1 |
| | | | 3. 136 |
| 275 | 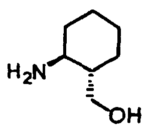 | 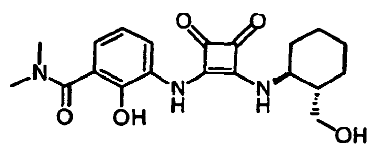 | 1. 30% |
| | | | 2. 388.1 |
| | | | 3. 114 |
| 276 | 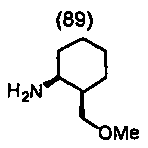 | 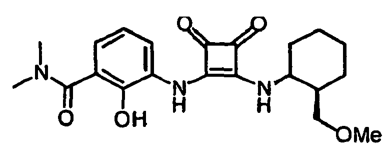 | 1. 53% |
| | | | 2. 402.1 |
| | | | 3. 126 |
| 277 | 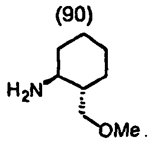 | 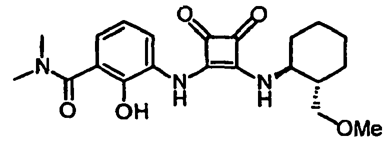 | 1. 68% |
| | | | 2. 402.1 |
| | | | 3. 116 |
| 278 | 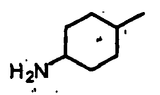 | 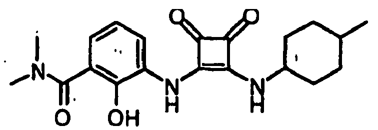 | 1. 64% |
| | | | 2. 372.1 |
| | | | 3. 106 |
| 279 | 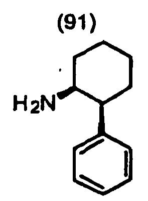 | 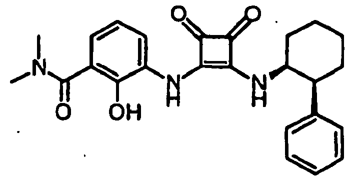 | 1. 69% |
| | | | 2. 434.1 |
| | | | 3. 141-143 |
| 280 | 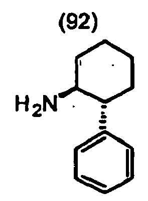 | 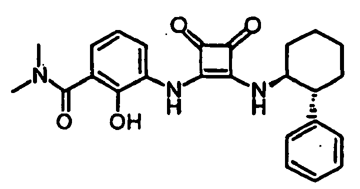 | 1. 51% |
| | | | 2. 434.1 |
| | | | 3. 148-150 |
| 281 | 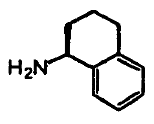 | 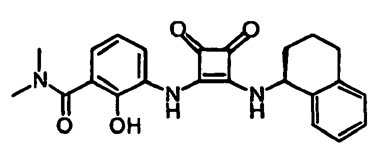 | 1. 71% |
| | | | 2. 406.1 |
| | | | 3. 146-148 |
| 282 | 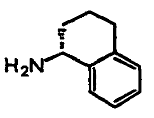 | 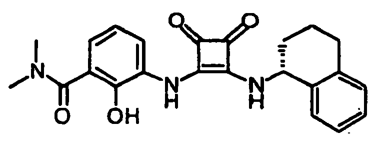 | 1. 66% |
| | | | 2. 406.1 |
| | | | 3. 141-144 |
| 283 | 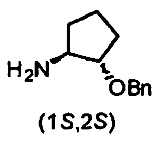 | 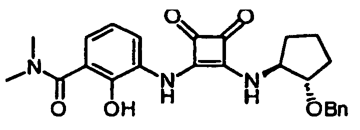 | 1. 70% |
| | | | 2. 450.1 |
| | | | 3. 97-99 |
| 284 | 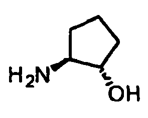 | 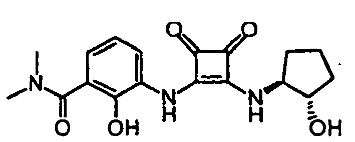 | 1. 25% |
| | | | 2. 360.1 |
| | | | 3. 139 |
| 285 | 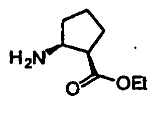 | 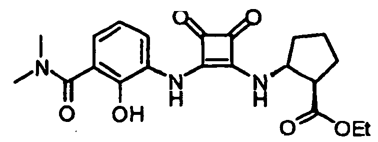 | 1. 78% |
| | | | 2. 416.1 |
| | | | 3. 94 |
| 286 | 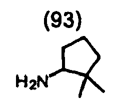 | 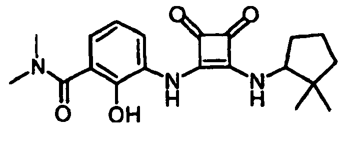 | 1. 49% |
| | | | 2. 372:1 |
| | | | 3. 139 |
| 287 | 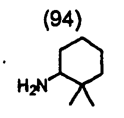 | 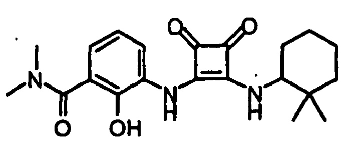 | 1. 95% |
| | | | 2. 386.1 |
| | | | 3. 139 |
| 288 | 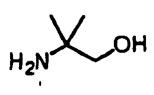 | 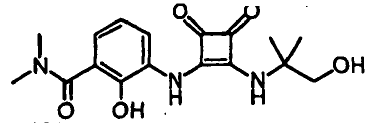 | 1. 32% |
| | | | 2. 348 |
| | | | 3. 130-133 |
| 289 | 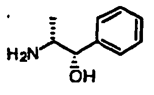 | 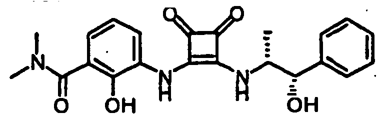 | 1. 72% |
| | | | 2. 410.1 |
| | | | 3. 138 |
| 290 | 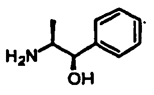 | 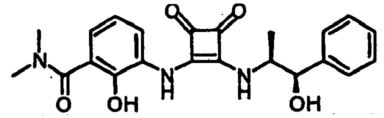 | 1. 72% |
| | | | 2. 410.1 |
| | | | 3. 132-134 |
| 291 | 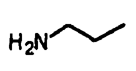 | 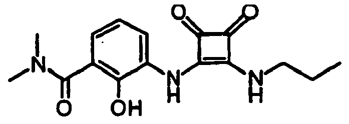 | 1. 75% |
| | | | 2. 318.1 |
| | | | 3. 96-98 |
| 292 | 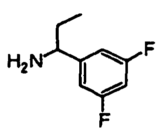 | 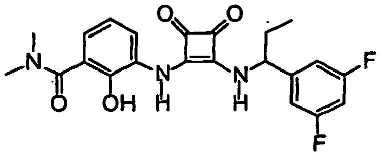 | 1. 72% |
| | | | 2. 430.1 |
| | | | 3. 125 |
| 293 | 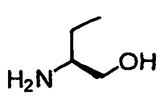 | 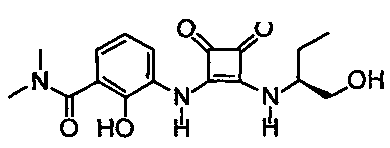 | 1. 51% |
| | | | 2. 348 |
| | | | 3. 109-111 |
| 294 | 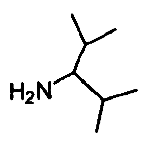 | 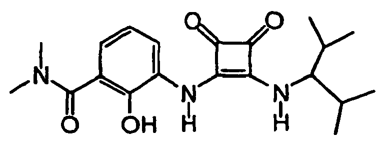 | 1. 84% |
| | | | 2. 374 |
| | | | 3. 150.3 |
| 295 | 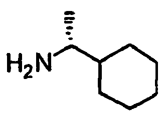 | 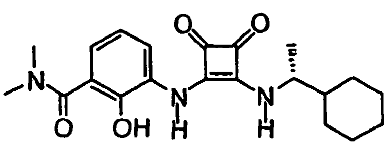 | 1. 56% |
| | | | 2. 386 |
| | | | 3. 142.3 |
| 296 | 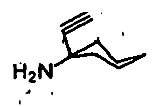 | 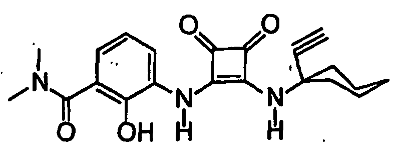 | 1. 38% |
| | | | 2. 382 |
| | | | 3. 173.4 |
| 297 | 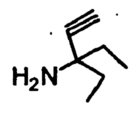 | 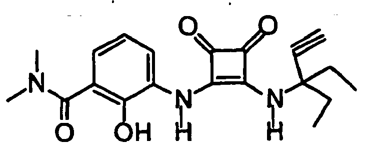 | 1. 13% |
| | | | 2. 370 |
| | | | 3. 135.1 |
| 298 | 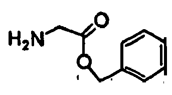 | 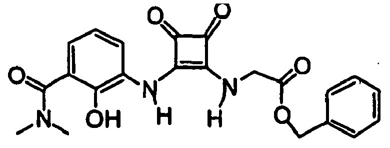 | 1. 47% |
| | | | 2. 424 |
| | | | 3. 231.2-234.5 |
| 299 | 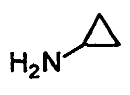 | 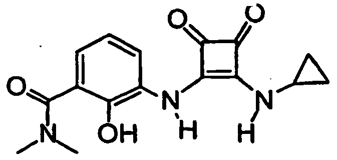 | 1. 34% |
| | | | 2. 316 |
| | | | 3. 209.5 |
| 300 | 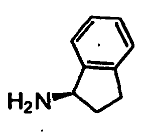 | 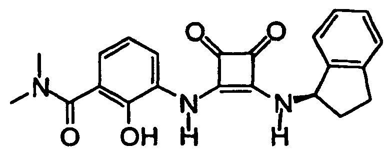 | 1. 92% |
| | | | 2. 392 |
| | | | 3. 152.7 |
| 301 | 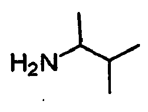 | 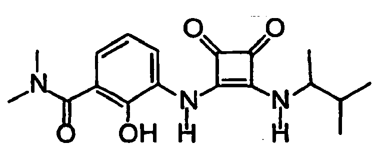 | 1. 52% |
| | | | 2. 346 |
| | | | 3. 124.7 |
| 302 | 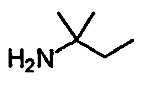 | 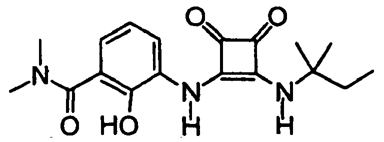 | 1. 51% |
| | | | 2. 346 |
| | | | 3. 139.2 |
| 303 | 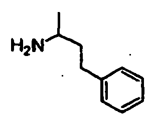 | 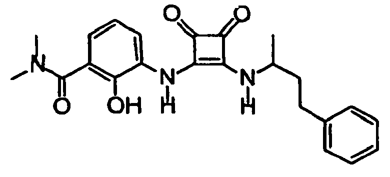 | 1. 29% |
| | | | 2. 408 |
| | | | 3. 105 |
| 304 | 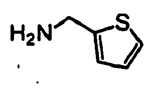 | 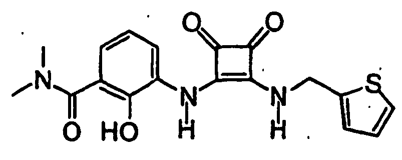 | 1. 24% |
| | | | 2. 372 |
| | | | 3. 223.2 |
| 305 | 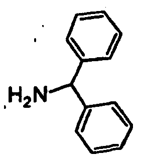 | 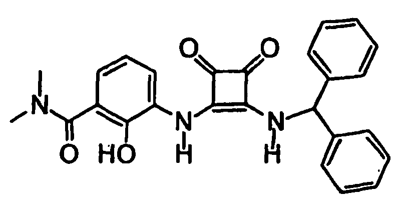 | 1. 25% |
| | | | 2. 442 |
| | | | 3. 219.0 |
| 306 | 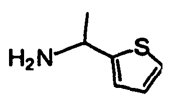 | 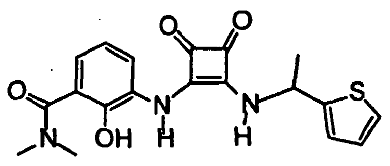 | 1. 83% |
| | | | 2. 386 |
| | | | 3. 145 |
| 307 | 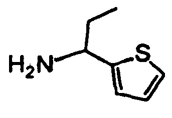 | 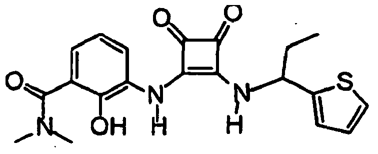 | 1. 58% |
| | | | 2. 400 |
| | | | 3. 99.6 |
| 308 | 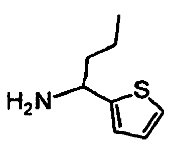 | 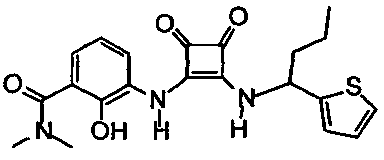 | 1. 60% |
| | | | 2. 414 |
| | | | 3. 123.6 |
| 309 | 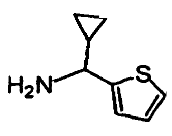 | 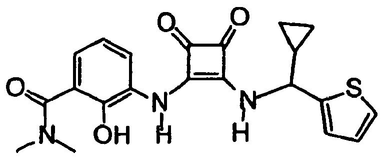 | 1. 44% |
| | | | 2. 412 |
| | | | 3. 146.7 |
| 310 | 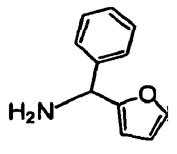 | 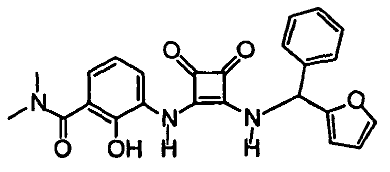 | 1. 39% |
| | | | 2. 432 |
| | | | 3. 156.6 |
| 311 | 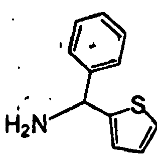 | 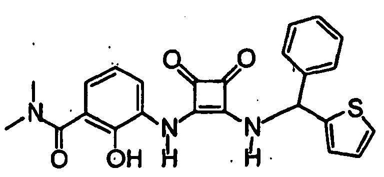 | 1. 65% |
| | | | 2. 448 |
| | | | 3. 162.8 |
| 312 | 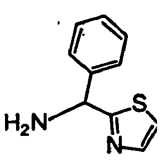 | 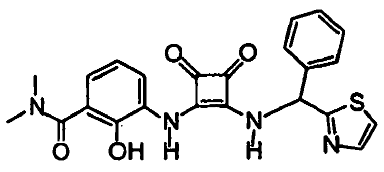 | 1. 53% |
| | | | 2. 449 |
| | | | 3. 139.7 |
| 313 | 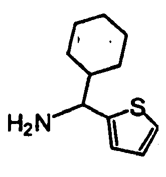 | 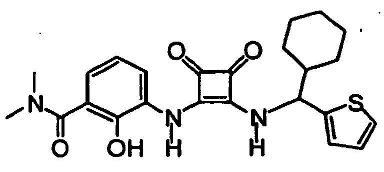 | 1. 64% |
| | | | 2. 454 |
| | | | 3. 143.2 |
| 314 | 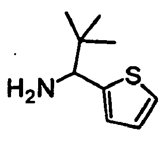 | 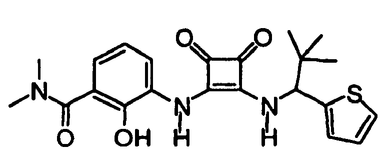 | 1. 35% |
| | | | 2. 428 |
| | | | 3. 146.8 |
| 315 | 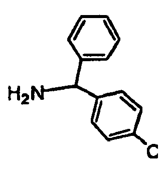 | 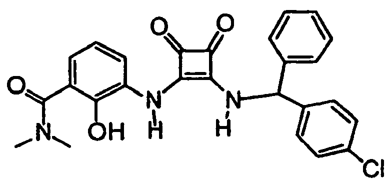 | 1. 72% |
| | | | 2. 476 |
| | | | 3. 139.4 |
| 316 | 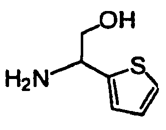 | 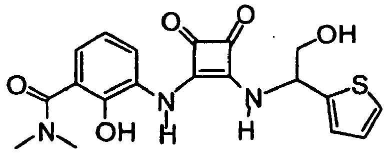 | 1. 36% |
| | | | 2. 402 |
| | | | 3. 89.6 |
| 317 | 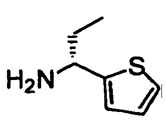 | 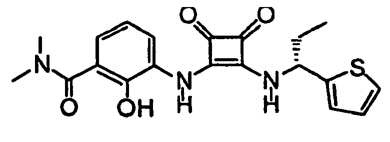 | 1. 62% |
| | | | 2. 400 |
| | | | 3. 130.2 |
| 318 | 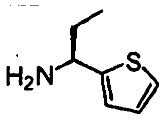 | 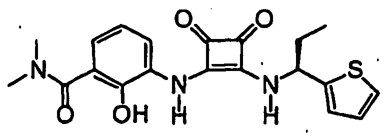 | 1. 46% |
| | | | 2. 400 |
| | | | 3. 123'.6 |
| 319 | 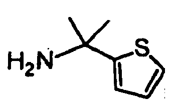 | 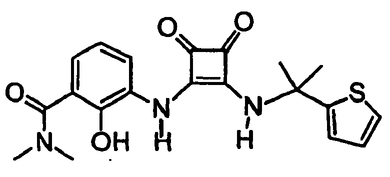 | 1. 64% |
| | | | 2. 400 |
| | | | 3. 132.5 |
| 320 | 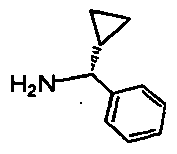 | 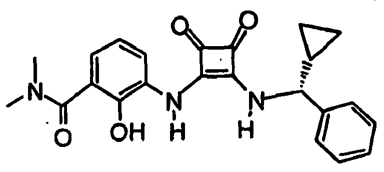 | 1. 79% |
| | | | 2. 406 |
| | | | 3. 123:3 |
| 321 | 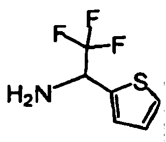 | 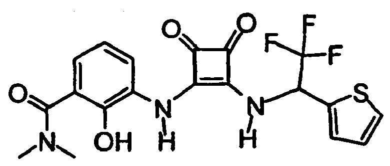 | 1. 17% |
| | | | 2. 440 |
| | | | 3. 157.6 |
| 322 | 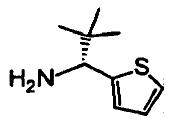 | 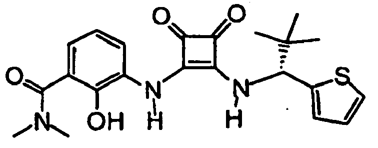 | 1. 58% |
| | | | 2. 428 |
| | | | 3. 167.9 |
| 323 | 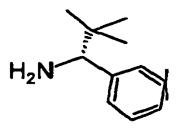 | 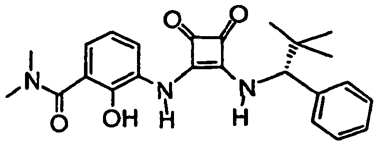 | 1. 50% |
| | | | 2. 422 |
| | | | 3. 150.2 |
| 324 | 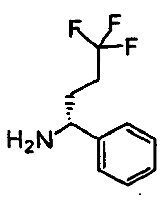 | 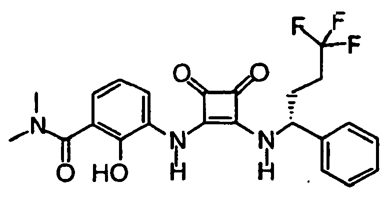 | 1. 20% |
| | | | 2. 462 |
| | | | 3. 113.9 |
| 325 | 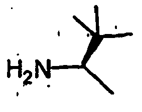 | 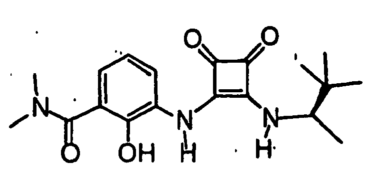 | 1. 95% |
| | | | 2. 360 |
| | | | 3. 129.2 |
| 326 | 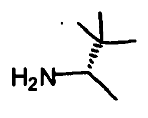 | 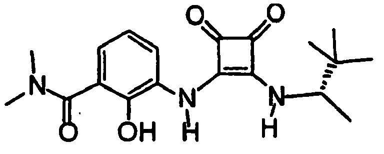 | 1. 97% |
| | | | 2. 360 |
| | | | 3. 131.5 |
| 327 | 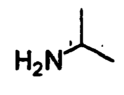 | 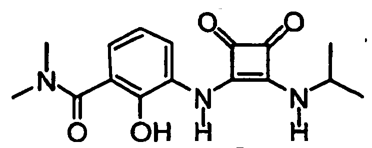 | 1. 39% |
| | | | 2. 318 |
| | | | 3. 138.5 |
| 328 | 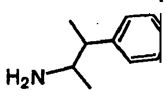 | 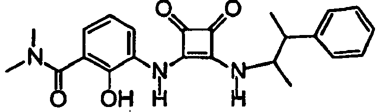 | 1. 54% |
| | | | 2. 408 |
| | | | 3. 152.3 |
| 329 | 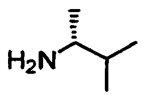 | 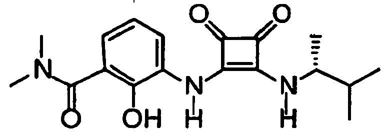 | 1. 62% |
| | | | 2. 346 |
| | | | 3. 134.8 |
| 330 | 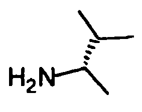 | 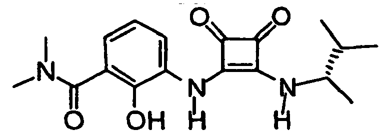 | 1. 55% |
| | | | 2. 346 |
| | | | 3. 145.1 |
| 331 | 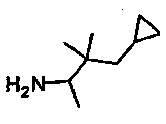 | 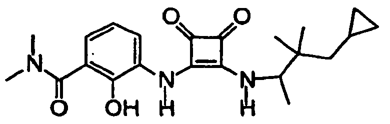 | 1. 61% |
| | | | 2. 400 |
| | | | 3. 137.6 |
| 332 | 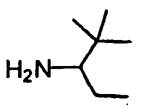 | 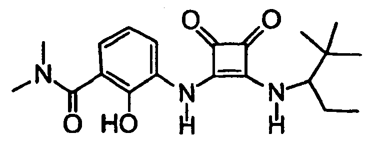 | 1. 42% |
| | | | 2. 374 |
| | | | 3. 155.1 |
| 333 | 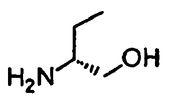 | 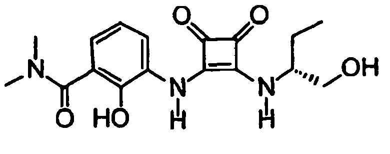 | 1. 45% |
| | | | 2. 348 |
| | | | 3. 108-110 |
| 334 | 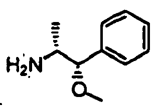 | 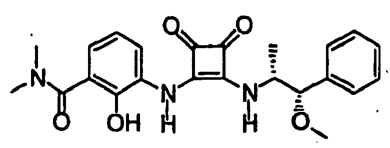 | 1. 29% |
| | | | 2. 424 |
| | | | 3. 116 |
| 335 | 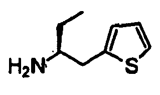 | 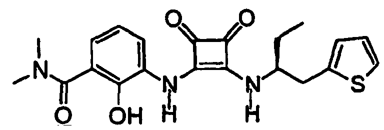 | 1. 15% |
| | | | 2. 414 |
| | | | 3. 108-110 |
| 336 | 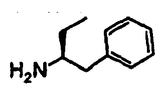 | 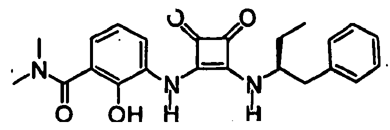 | 1. 75% |
| | | | 2. 408 |
| | | | 3. 116 |
| 337 | 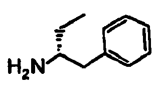 | 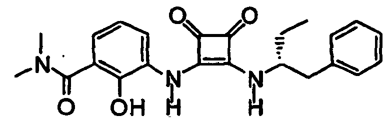 | 1. 75% |
| | | | 2. 408 |
| | | | 3. 116 |
| 338 | 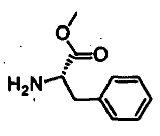 | 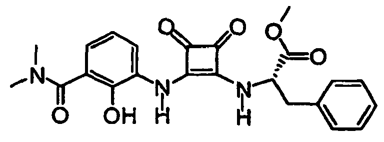 | 1. 59% |
| | | | 2. 424 |
| | | | 3. 115-117 |
| 339 | 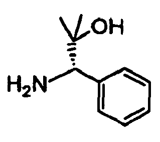 | 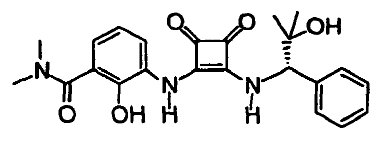 | 1. 72% |
| | | | 2. 424 |
| | | | 3. 157-159 |
| 340 | 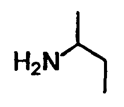 | 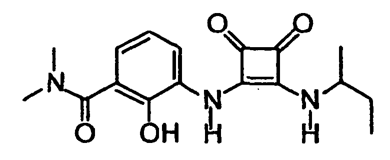 | 1. 19% |
| | | | 2. 332 |
| | | | 3. 131 |
| 341 | 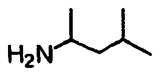 | 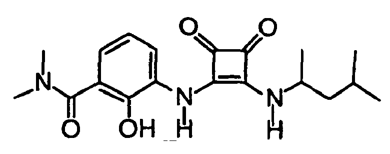 | 1. 86% |
| | | | 2. 360 |
| | | | 3. 127 |
| 342 | 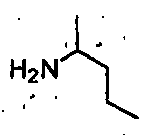 | 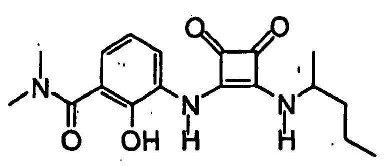 | 1. 98% |
| | | | 2. 346 |
| | | | 3. 128 |
| 343 | 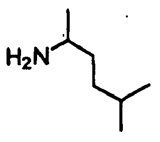 | 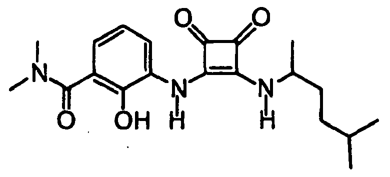 | 1. 80% |
| | | | 2. 374 |
| | | | 3. 131.5 |
| 344 | 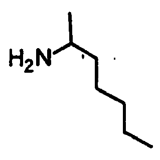 | 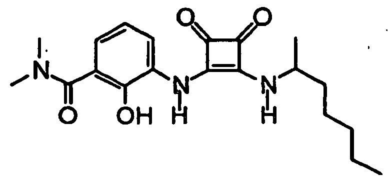 | 1. 46% |
| | | | 2. 374 |
| | | | 3. 102 |
| 345 | 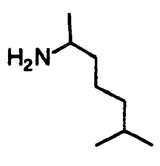 | 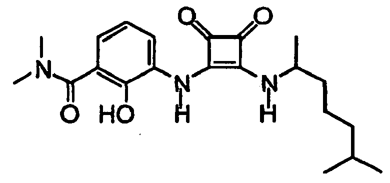 | 1. 75% |
| | | | 2. 388 |
| | | | 3. 104 |
| 346 | 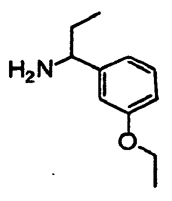 | 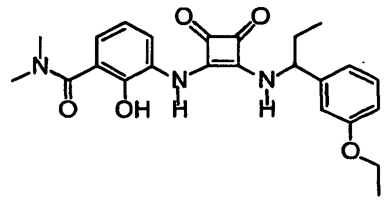 | 1. 76% |
| | | | 2. 438 |
| | | | 3. 95 |
| 347 | 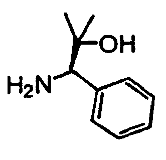 | 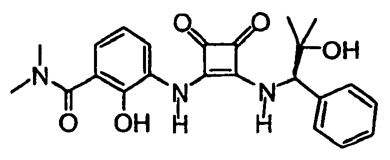 | 1. 72% |
| | | | 2. 424 |
| | | | 3. 163-165 |
| 348 | 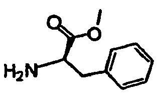 | 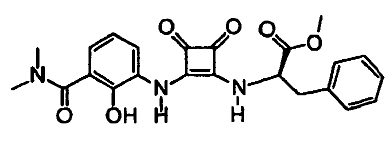 | 1. 73% |
| | | | 2. 438 |
| | | | 3. 96-98 |
| 349 | 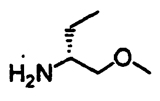 | 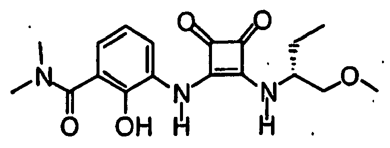 | 1. 53% |
| | | | 2. 362 |
| | | | 3. 89-91 |
| 350 | 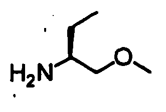 | 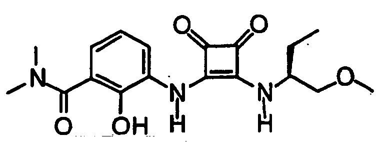 | 1. 59% |
| | | | 2. 362 |
| | | | 3. 90-92 |
| 351 | 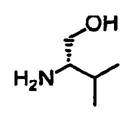 | 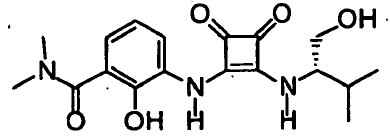 | 1. 61% |
| | | | 2. 362 |
| | | | 3. 120-122 |
| 352 | 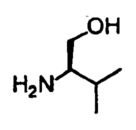 | 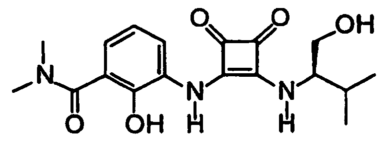 | 1. 70% |
| | | | 2. 362 |
| | | | 3. 121-123 |
| 353 | 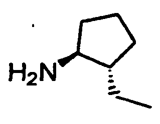 | 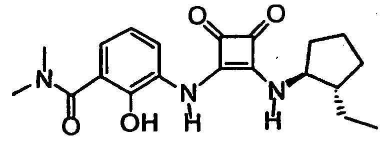 | 1. 23% |
| | | | 2. 371 |
| | | | 3. 126 |
| 354 | 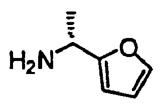 | 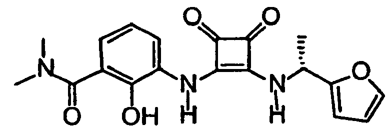 | 1. 79% |
| | | | 2. 370 |
| | | | 3. 108 |
| 355 | 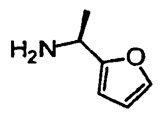 | 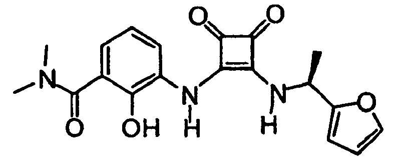 | 1. 80% |
| | | | 2. 370 |
| | | | 3. 106 |
| 356 | 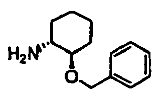 | 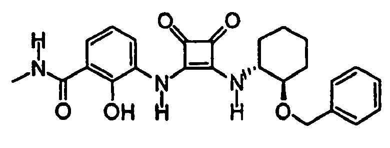 | 1. 56% |
| | | | 2. 450 |
| | | | 3. 138-140 |
| 357 | 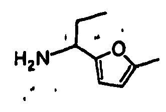 | 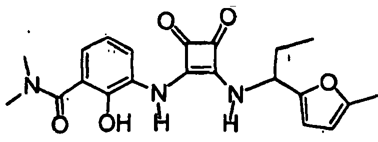 | 1. 76% |
| | | | 2. 398 |
| | | | 3. 116 |
| 358 | 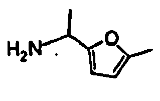 | 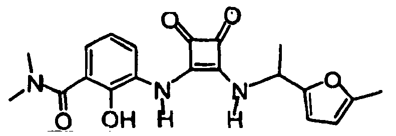 | 1. 85% |
| | | | 2. 384 |
| | | | 3. 100 |
| 359 | 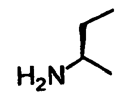 | 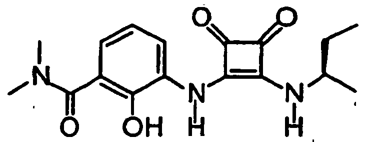 | 1. 59% |
| | | | 2. 332 |
| | | | 3. 138.6 |
| 360 | 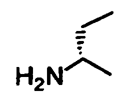 | 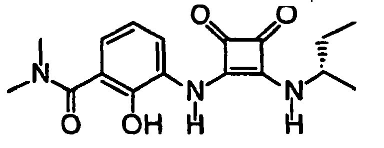 | 1. 47% |
| | | | 2. 332 |
| | | | 3. 141.6 |
| | | | |
| 360.1 | 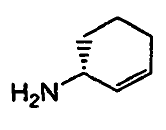 | 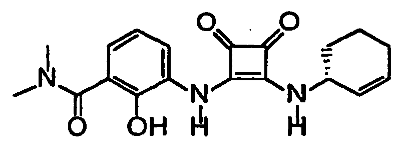 | 1. 89% |
| | | | 2. 356.1 |
| | | | 3. 133-135 |
| 360.2 | 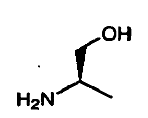 | 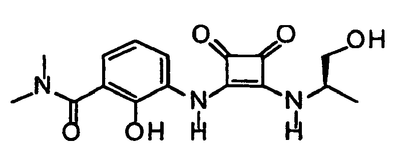 | 1. 65% |
| | | | 2. 334.1 |
| | | | 3. 121-122 |
| 360.3 | 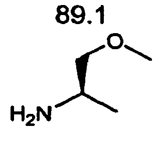 | 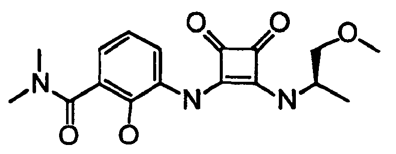 | 1. 60% |
| | | | 2. 348.1 |
| | | | 3. 94-96 |
| 360.4 | 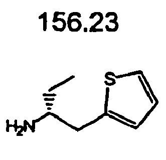 | 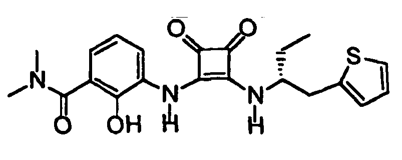 | 1. 29% |
| | | | 2. 414.1 |
| | | | 3. 108-110 |
| 360.5 | 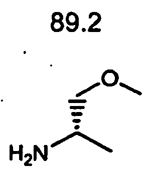 | 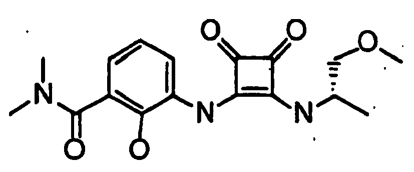 | 1. 67% |
| | | | 2. 348.1 |
| | | | 3. 95-96 |
| 360.6 | 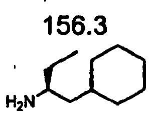 | 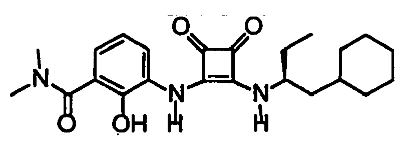 | 1. 62% |
| | | | 2. 414.1 |
| | | | 3. 113-115 |
| 360.7 | 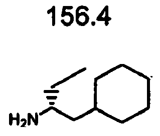 | 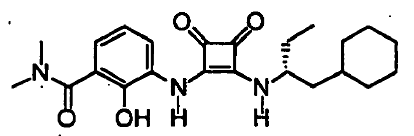 | 1. 68% |
| | | | 2. 414.1 |
| | | | 3. 114-116 |
| 360.8 | 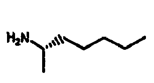 | 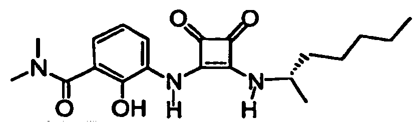 | 1. 74% |
| | | | 2. 374 |
| | | | 3. 129.8 |
| 360.9 | 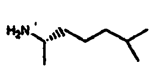 | 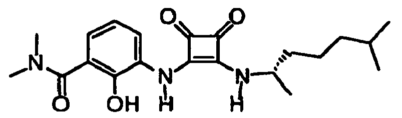 | 1. 61% |
| | | | 2. 388 |
| | | | 3. 123.1 |
| 360.10 | 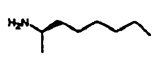 | 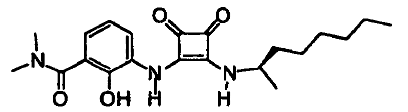 | 1. 53% |
| | | | 2. 388 |
| | | | 3. 117.2 |
| 360.11 | 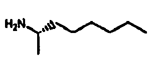 | 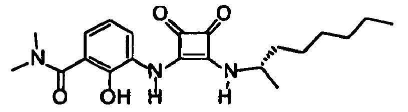 | 1. 37% |
| | | | 2. 388 |
| | | | 3. 129.9 |
| 360.12 | 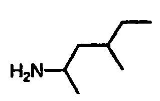 | 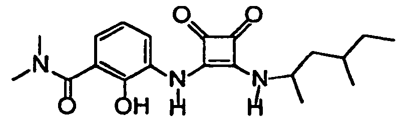 | 1. 62% |
| | | | 2. 374 |
| | | | 3. 126.1 |
| 360.13 | 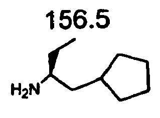 | 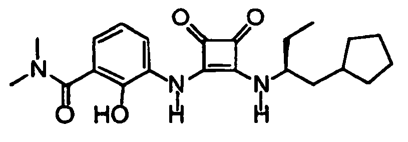 | 1. 71% |
| | | | 2. 400.1 |
| | | | 3. 106-109 |
| 360.14 | 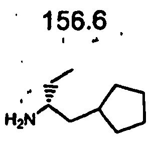 | 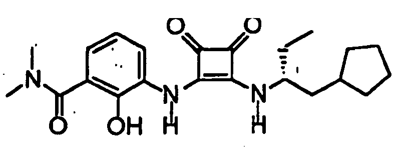 | 1. 66% |
| | | | 2. 400.1 |
| | | | 3. 106-109 |
| 360.15 | 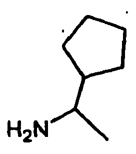 | 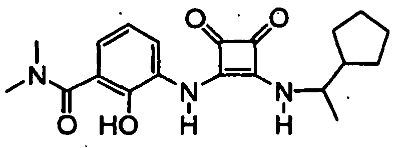 | 1. 69% |
| | | | 2. 372 |
| | | | 3. 138.7 |
| 360.16 | 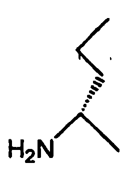 | 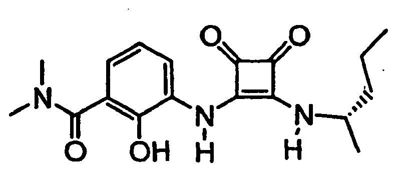 | 1. 54% |
| | | | 2. 346 |
| | | | 3. 123.6 |
| 360.17 | 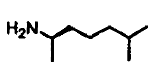 | 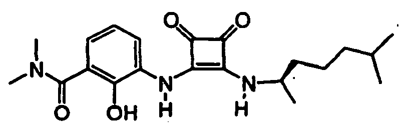 | 1. 53% |
| | | | 2. 388 |
| | | | 3. 116.9 |
| 360.18 | 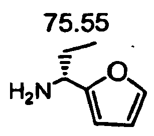 | 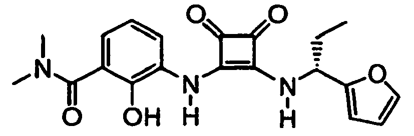 | 1. 87% |
| | | | 2. 384.1 |
| | | | 3. 136 |
| 360.19 | 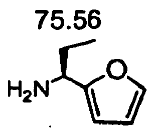 | 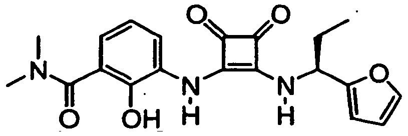 | 1. 92% |
| | | | 2. 384.1 |
| | | | 3. 136 |
| 360.20 | 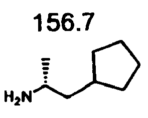 | 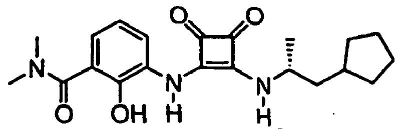 | 1. 27% |
| | | | 2. 386.1 |
| | | | 3. 109-112 |
| 360.21 | 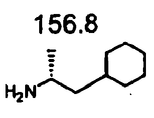 | 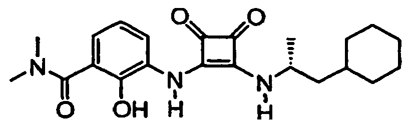 | 1. 31% |
| | | | 2. 400.1 |
| | | | 3. 117-120 |
| 360.22 | 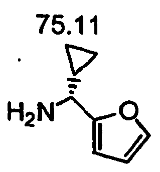 | 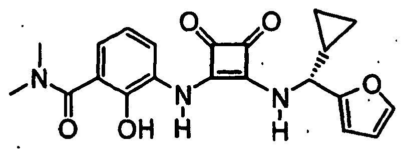 | 1. 61% |
| | | | 2. 396.1 |
| | | | 3. 129 |
| 360.23 | 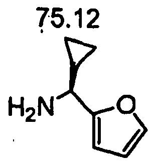 | 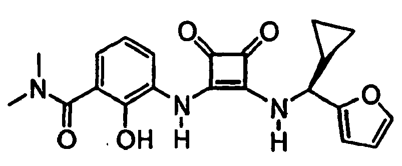 | 1. 69% |
| | | | 2. 396.1 |
| | | | 3. 126 |
| 360.24 | 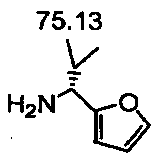 | 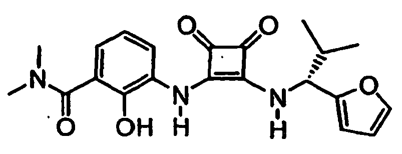 | 1. 74% |
| | | | 2. 398.1 |
| | | | 3. 123 |
| 360.25 | 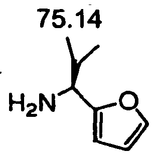 | 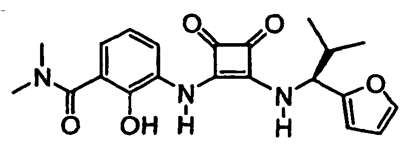 | 1. 76% |
| | | | 2. 398.1 |
| | | | 3. 123 |
| 360.26 | 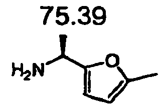 | 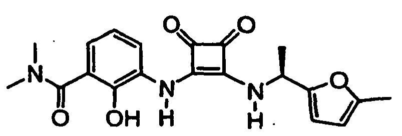 | 1. 60% |
| | | | 2. 384.1 |
| | | | 3. 103-105 |
| 360.27 | 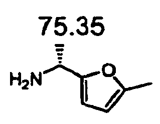 | 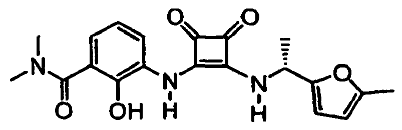 | 1. 67% |
| | | | 2. 384.1 |
| | | | 3. 104-106 |
| 360.28 | 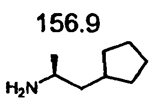 | 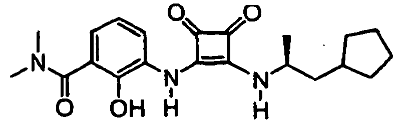 | 1. 70% |
| | | | 2. 386.1 |
| | | | 3. 103-105 |
| 360.29 | 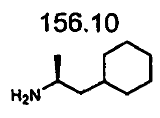 | 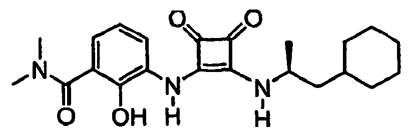 | 1. 64% |
| | | | 2. 400.1 |
| | | | 3. 109-111 |
| 360.30 | 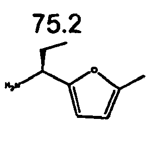 | 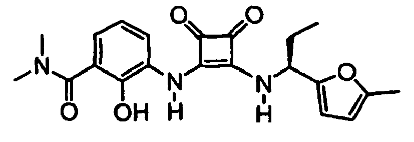 | 1. 63% |
| | | | 2. 398.1 |
| | | | 3. 99-101 |
| 360.31 | 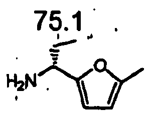 | 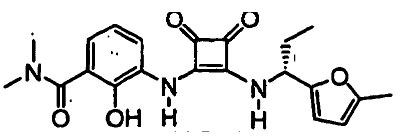 | 1. 57% |
| | | | 2. 398.1 |
| | | | 3. 99-101 |
| 360.32 | 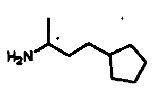 | 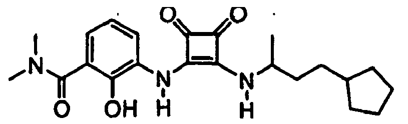 | 1. 45% |
| | | | 2. 400 |
| | | | 3. 104.6 |
| 360.33 | 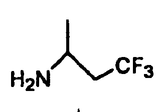 | 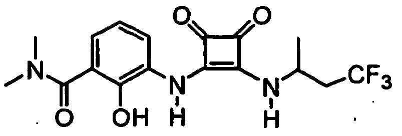 | 1. 44% |
| | | | 2. 386 |
| | | | 3. 143 |
| 360.34 | 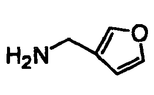 | 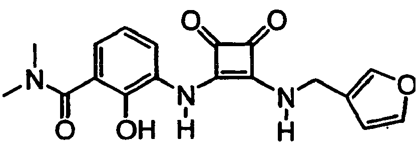 | 1. 73% |
| | | | 2. 356.1 |
| | | | 3. 218-220 |
| 360.35 | 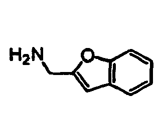 | 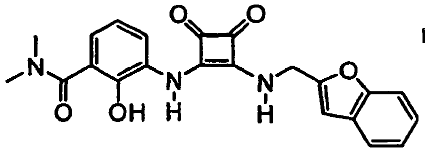 | 1. 97% |
| | | | 2. 406.1 |
| | | | 8. 154 |
| 360.36 | 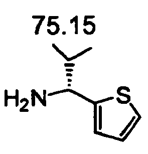 | 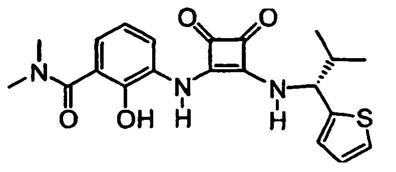 | 1. 77% |
| | | | 2. 414.1 |
| | | | 3. 122-124 |
| 360.37 | 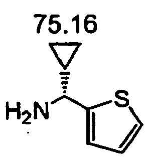 | 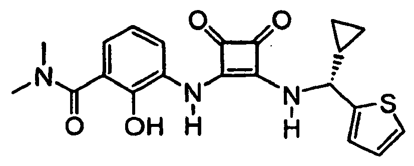 | 1. 70% |
| | | | 2. 412.1 |
| | | | 3. 99-101 |
| 360.38 | 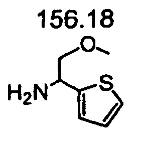 | 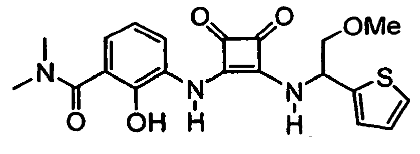 | 1. 69% |
| | | | 2. 416.1 |
| | | | 3. 107-109 |
| 360.39 | 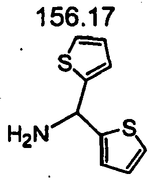 | 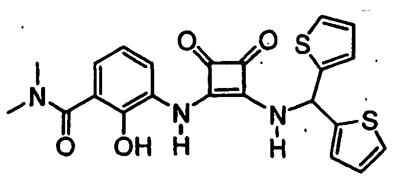 | 1. 43% |
| | | | 2. 454.1 |
| | | | 3. 128-130 |
| 360.40 | 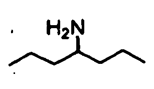 | 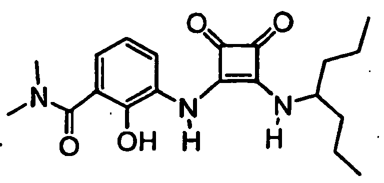 | 1. 40% |
| | | | 2. 374.1 |
| | | | 3. 132-136 |
| 360.41 | 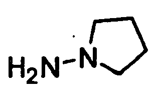 | 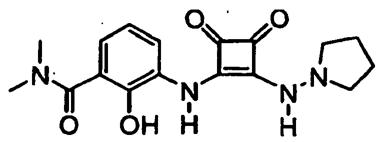 | 1. 60% |
| | | | 2. 345.1 |
| | | | 3. 205-207 |
| 360.42 | 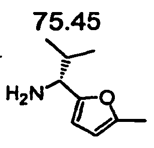 | 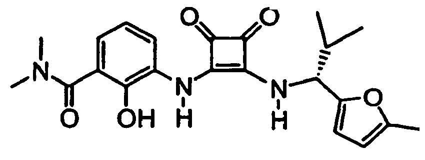 | 1. 96% |
| | | | 2. 412.1 |
| | | | 3. 112 |
| 360.43 | 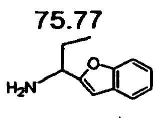 | 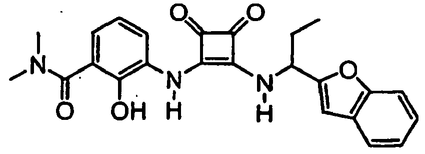 | 1. 30% |
| | | | 2. 434.1 |
| | | | 3. 117-119 |
| 360.44 | 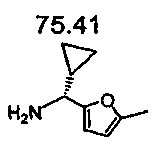 | 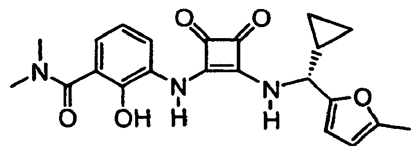 | 1. 96% |
| | | | 2. 410.1 |
| | | | 3. 139 |
| 360.45 | 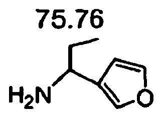 | 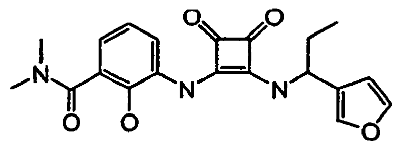 | 1. 65% |
| | | | 2. 384.1 |
| | | | 3. 87-89 |
| 360.46 | 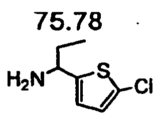 | 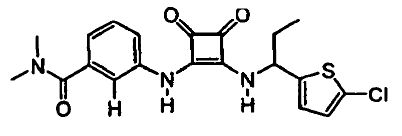 | 1. 50% |
| | | | 2. 434.1 |
| | | | 3. 123-125 |
| 360.47 | 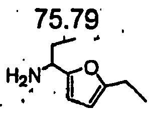 | 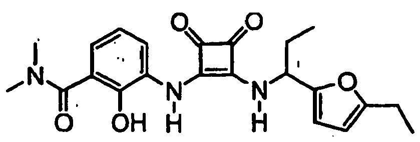 | 1. 74% |
| | | | 2. 412.1 |
| | | | 3. 84-86. |
| 360.48 | 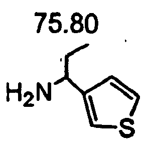 | 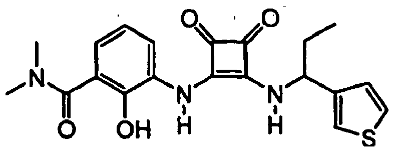 | 1. 73% |
| | | | 2. 400.1 |
| | | | 3. 136-140 |
| 360.49 | 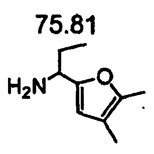 | 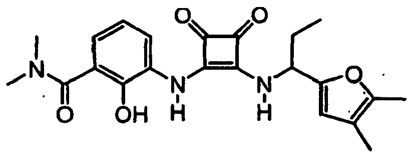 | 1. 74% |
| | | | 2. 412.1 |
| | | | 3. 103-105 |
| 360.50 | 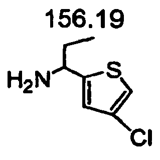 | 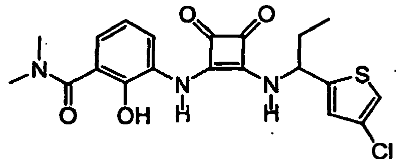 | 1. 63% |
| | | | 2. 434.1 |
| | | | 3. 114-117 |
| 360.51 | 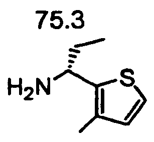 | 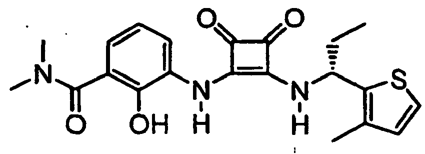 | 1. 74% |
| | | | 2. 414.1 |
| | | | 3. 130-133 |
| 360.52 | 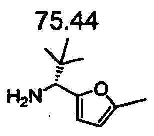 | 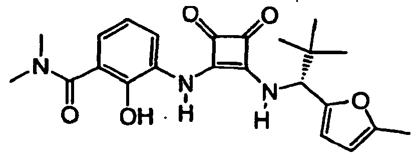 | 1. 71% |
| | | | 2. 426.1 |
| | | | 3. 138 |
| 360.53 | 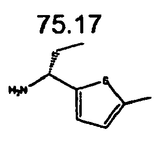 | 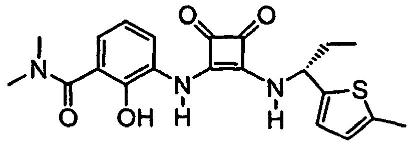 | 1. 41% |
| | | | 2. 414 |
| | | | 3. 139-141 |
| 360.54 | 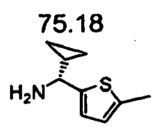 | 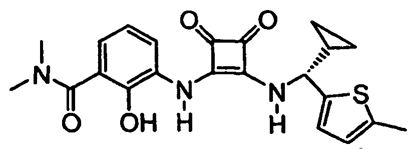 | 1. 32% |
| | | | 2. 426 |
| | | | 3. 148-150 |
| 360.55 | 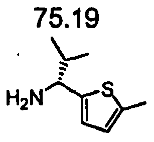 | 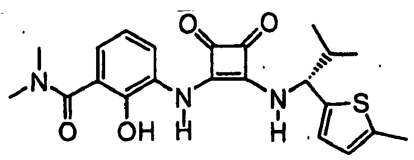 | 1. 57,% |
| | | | 2. 428 |
| | | | 3. 159-163 |
| 360.56 | 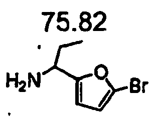 | 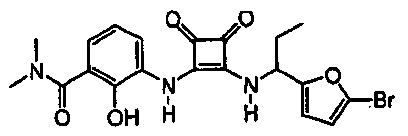 | 1. 44% |
| | | | 2. 464.1 |
| | | | 3. 86-88 |
| 360.57 | 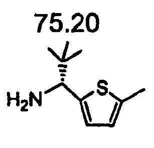 | 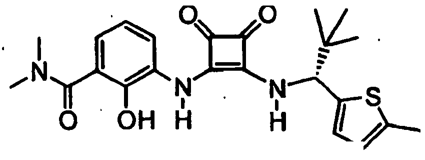 | 1. 37% |
| | | | 2. 442 |
| | | | 3. 158-162 |
| 360.58 | 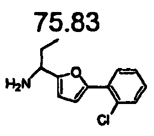 | 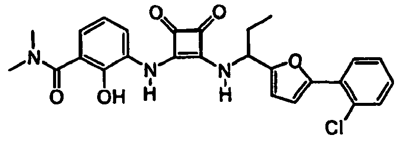 | 1. 53% |
| | | | 2. 494.1 |
| | | | 3. 148-151 |
| 360.59 | 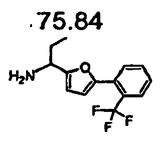 | 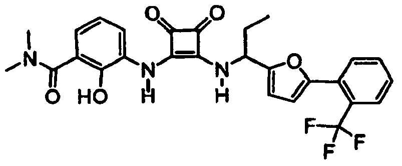 | 1. 63% |
| | | | 2. 528.1 |
| | | | 3. 90-95 |
| 360.60 | 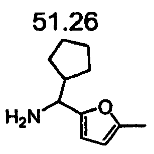 | 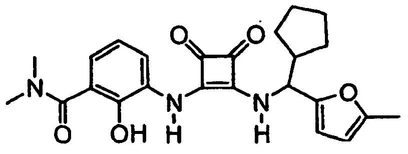 | 1. 73% |
| | | | 2. 438.1 |
| | | | 3. 116 |
| 360.61 | 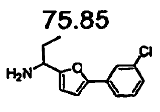 | 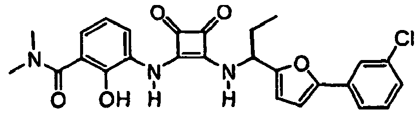 | 1. 55% |
| | | | 2. 494.1 |
| | | | 3. 133-135 |
| 360.62 | 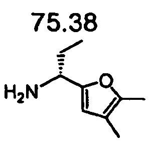 | 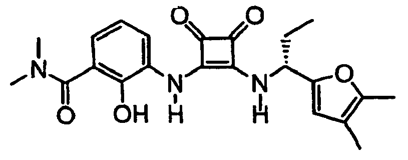 | 1. 83% |
| | | | 2. 412.1 |
| | | | 3. 119 |
| 360.63 | 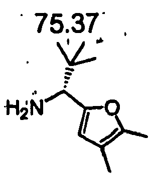 | 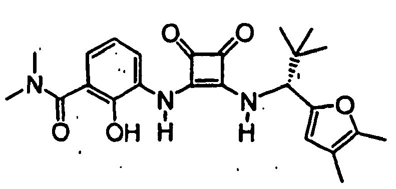 | 1. 66% |
| | | | 2. 440.1 |
| | | | 3. 110 |
| 360.64 | 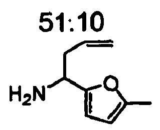 | 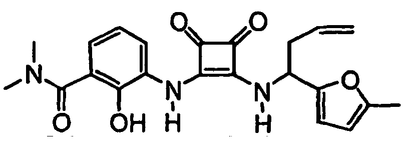 | 1. 49% |
| | | | 2. 410.1 |
| | | | 3. 97 |
| 360.65 | 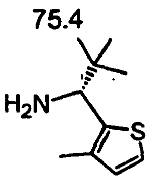 | 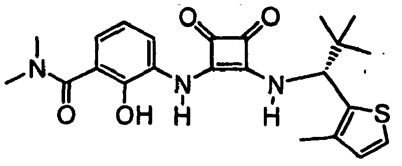 | 1. 40% |
| | | | 2. 442.1 |
| | | | 3. 157-160 |
| 360.66 | 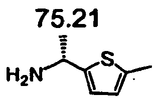 | 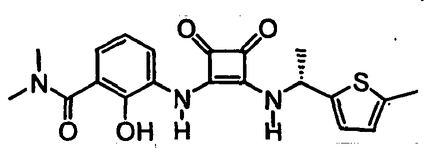 | 1. 75% |
| | | | 2. 400 |
| | | | 3. 136-140 |
| 360.67 | 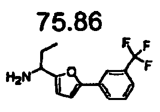 | 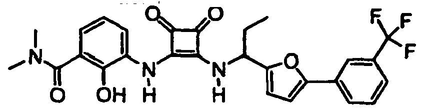 | 1. 63% |
| | | | 2. 528.1 |
| | | | 3. 106-108 |
| 360.68 | 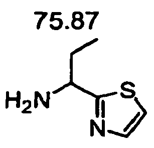 | 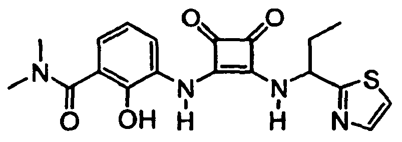 | 1. 10% |
| | | | 2. 401.1 |
| | | | 3. 111-113 |
| 360.69 | 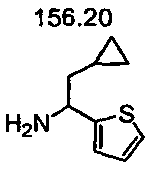 | 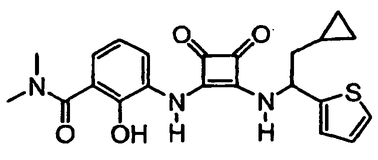 | 1. 5% |
| | | | 2. 426.1 |
| 360.70 | 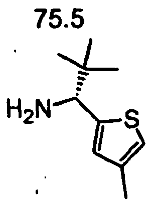 | 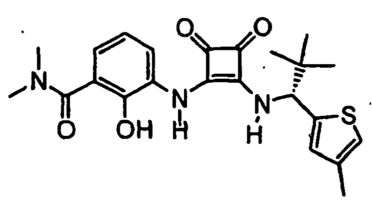 | 1. 56% |
| | | | 2. 442.1 |
| | | | 3. 152-154 |
| 360.71 | 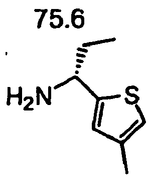 | 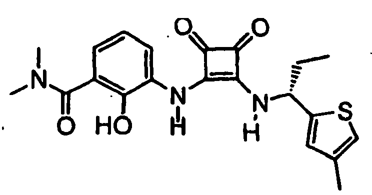 | 1. 46% |
| | | | 2. 414.1 |
| | | | 3. 122-124 |
| 360.72 | 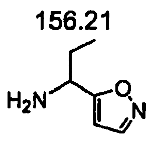 | 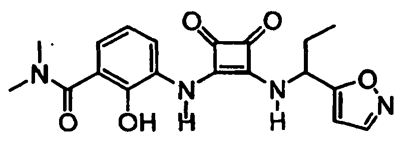 | 1. 62% |
| | | | 2. 385.1 |
| | | | 3. 130-133 |
| 360.73 | 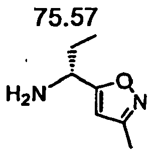 | 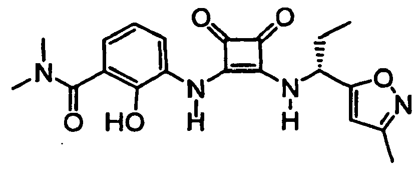 | 1. 41% |
| | | | 2. 399.1 |
| | | | 3. 83-85 |
| 360.74 | 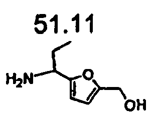 | 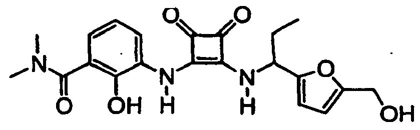 | 1. 70% |
| | | | 2. 414.1 |
| | | | 3. 98-101 |
| 360.75 | 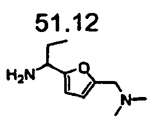 | 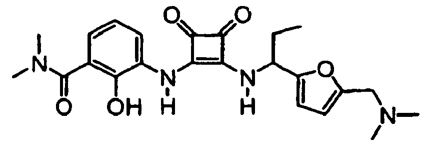 | 1. 62% |
| | | | 2. 441.1 |
| | | | 3. 98-102 |
| 360.76 | 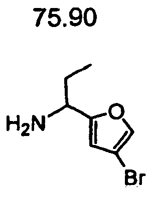 | 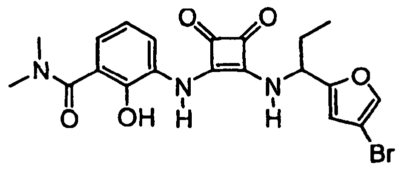 | 1. 79% |
| | | | 2. 464.1 |
| | | | 3. 111 |
| 360.77 | 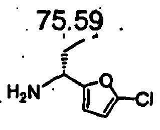 | 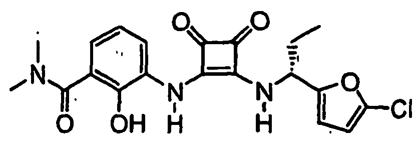 | 1. 79% |
| | | | 2. 418.1 |
| | | | 3. 107 |
| 360.78 | 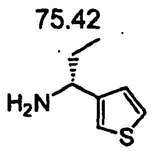 | 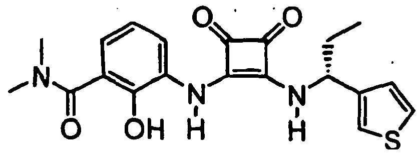 | 1. 65% |
| | | | 2. 400.1 |
| | | | 3. 109-112 |
| 360.79 | 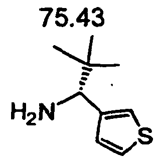 | 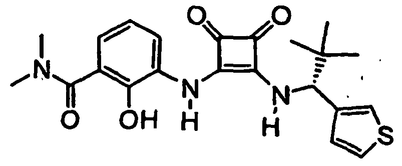 | 1. 21% |
| | | | 2. 428.1 |
| | | | 3. 126 |
| 360.80 | 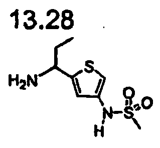 | 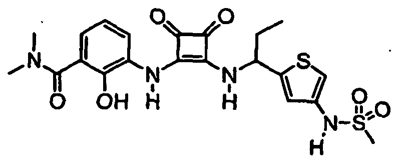 | 1. 55% |
| | | | 2. 493.1 |
| | | | 3. 155-158 |
| 360.81 | 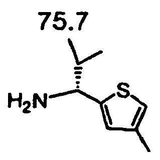 | 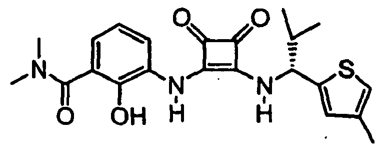 | 1. 67% |
| | | | 2. 428.1 |
| | | | 3. 138-140 |
| 360.82 | 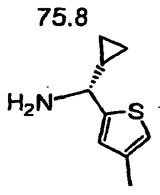 | 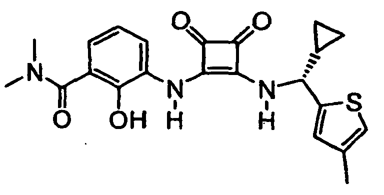 | 1. 68% |
| | | | 2. 426.1 |
| | | | 3. 121-123 |
| 360.83 | 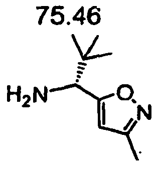 | 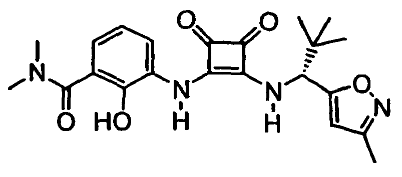 | 1. 25% |
| | | | 2. 427.1 |
| | | | 3. 139 |
| 360.84 | 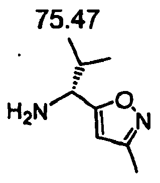 | 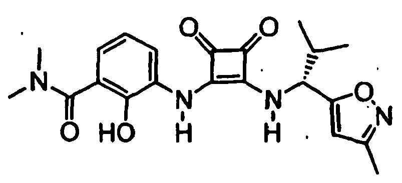 | 1. 62% |
| | | | 2. 413.1 |
| | | | 3. 128 |
| 360.85 | 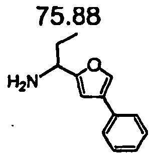 | 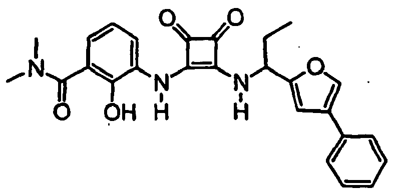 | 1. 49% |
| | | | 2. 460.1 |
| | | | 3. 112-114 |
| 360.86 | 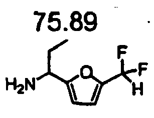 | 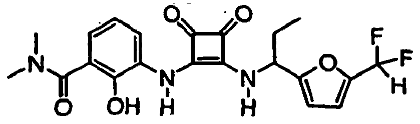 | 1. 71% |
| | | | 2. 434.1 |
| | | | 3. 91-93 |
| 360.87 | 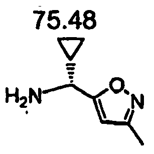 | 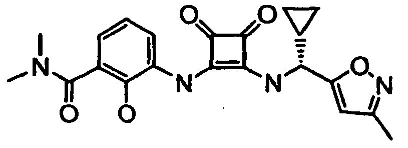 | 1. 57% |
| | | | 2. 411.1 |
| | | | 3. 125 |
| 360.88 | 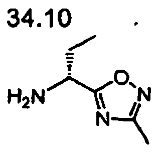 | 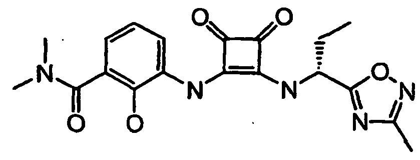 | 1. 12% |
| | | | 2. 400.1 |
| | | | 3. 131-133 |
| 360.89 | 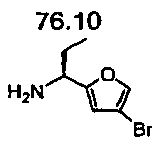 | 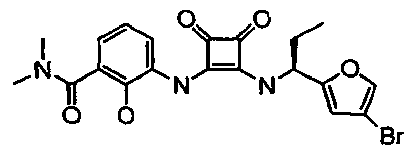 | 1. 60% |
| | | | 2. 464.1 |
| | | | 3. 111-113 |
| 360.90 | 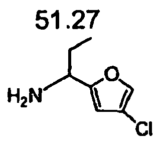 | 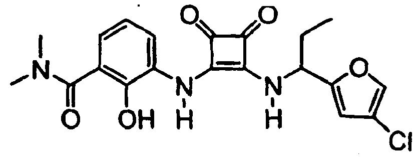 | 1. 60% |
| | | | 2. 418.1 |
| | | | 3. 113 |
| 360.91 | 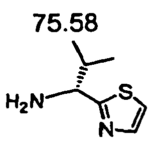 | 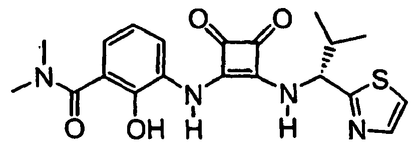 | 1. 55% |
| | | | 2. 415.1 |
| | | | 3. 140-143 |
| 360.92 | 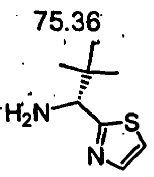 | 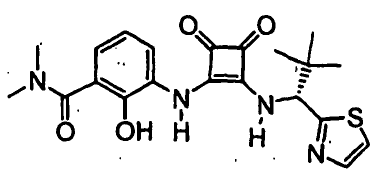 | 1. 55% |
| | | | 2. 429 |
| | | | 3. 185-190 |
| 360.93 | 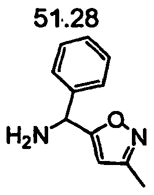 | 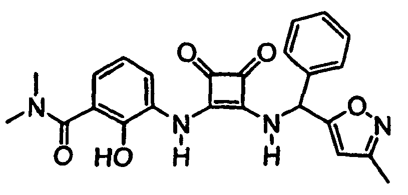 | 1. 3% |
| | | | 2. 447.1 |
| 360.94 | 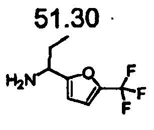 | 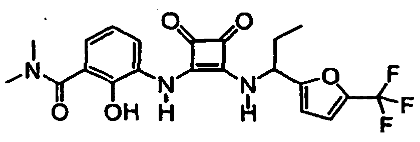 | 1. 71% |
| | | | 2. 452.1 |
| | | | 3. 106 |
| 360.95 | 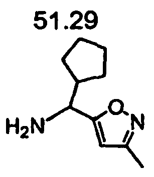 | 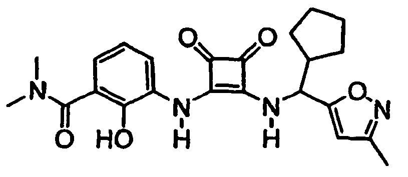 | 1. 44% |
| | | | 2. 439.1 |
| | | | 3. 112 |
| 360.96 | 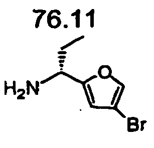 | 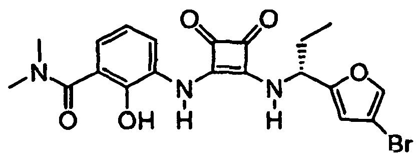 | 1. 71% |
| | | | 2. 464.1 |
| | | | 3. 111-113 |
| 360.97 | 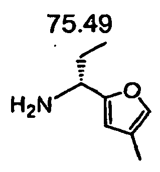 | 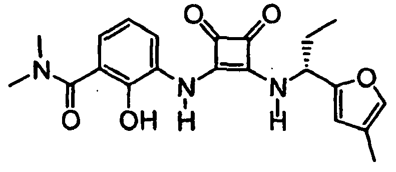 | 1. 70% |
| | | | 2. 398.1 |
| | | | 3: 106-108 |
| 360.98 | 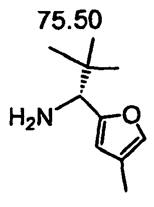 | 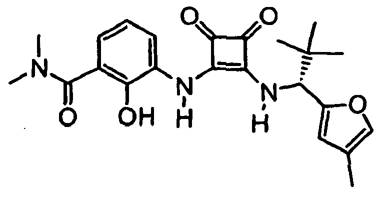 | 1. 46% |
| | | | 2. 426.1 |
| | | | 3. 140-142 |
| 360.99 | 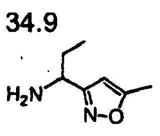 | 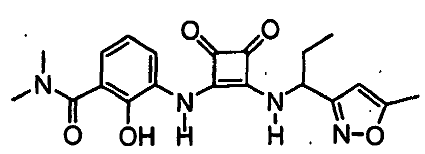 | 1. 62% |
| | | | 2. 399.1 |
| | | | 3. 109-112 |
| 360.100. | 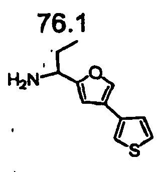 | 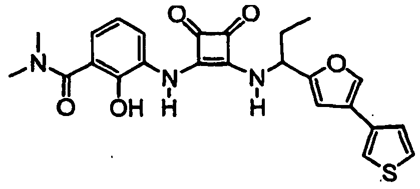 | 1. 60% |
| | | | 2. 466.1 |
| | | | 3. 129-131 |
| 360.101 | 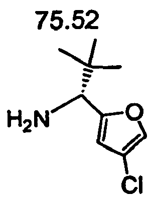 | 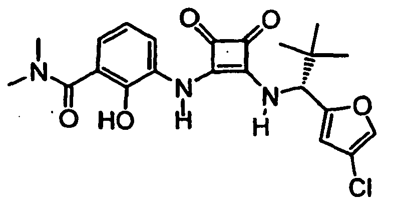 | 1. 49% |
| | | | 2. 446.1 |
| | | | 3. 146 |
| 360.102 | 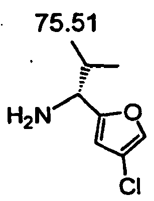 | 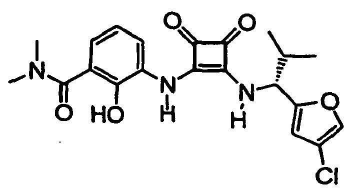 | 1. 48% |
| | | | 2. 432.1 |
| | | | 3. 116 |
| 360.103 | 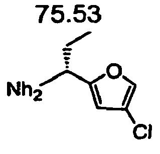 | 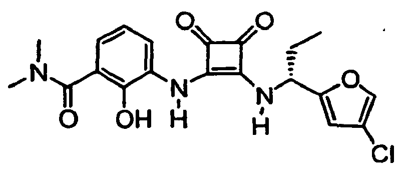 | 1. 62% |
| | | | 2. 418.1 |
| | | | 3. 126 |
| 360.104 | 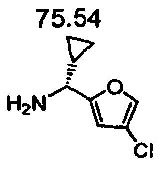 | 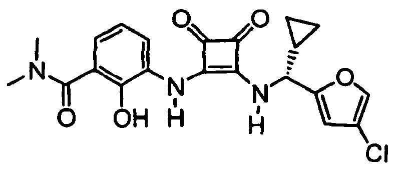 | 1. 47% |
| | | | 2. 430.1 |
| | | | 3. 136 |
| 360.105 | 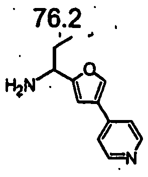 | 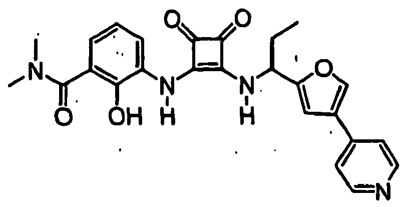 | 1. 42% |
| | | | 2. 461.1 |
| | | | 3. 131-134 |
| 360.106 | 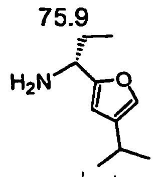 | 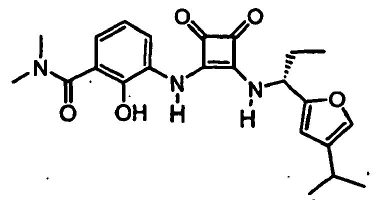 | 1. 93% |
| | | | 2. 426.1 |
| | | | 3. 123-125 |
| 360.107 | 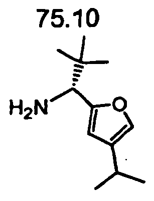 | 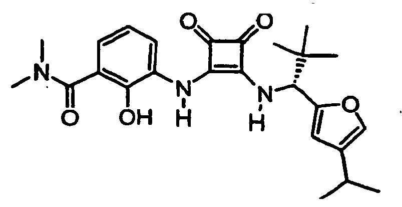 | 1. 26% |
| | | | 2. 454.1 |
| | | | 3. 132-134 |
| 360.108 | 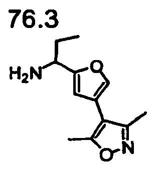 | 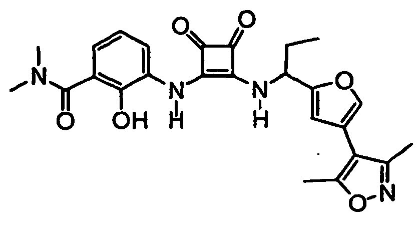 | 1. 12% |
| | | | 2. 479.1 |
| | | | 3. 129-132 |

### EXAMPLES 361-368.31

Following the procedure set forth in Example 261 but using the commercially available amine in the table below and the cyclobutenedione intermediate from the Preparative Example indicated, the following cyclobutenedione products were obtained.

| Ex. | Amine | Prep. | Product | 1.Yield (%) |
|---|---|---|---|---|
| | | Ex. | | 2. MH⁺ |
| | | | | 3. mp (°C) |
| 361 | 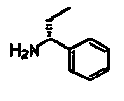 | 20 | 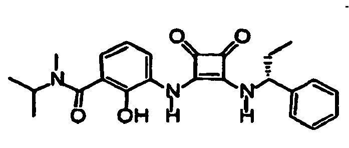 | 1. 57% |
| | | | | 2. 422 |
| | | | | 3. 172.4 |
| 362 | 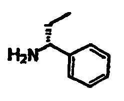 | 21 | 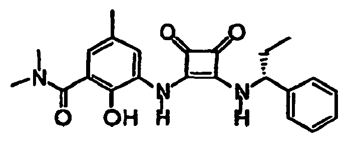 | 1. 53% |
| | | | | 2. 408 |
| | | | | 3. 139.8 |
| 363 | 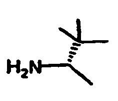 | 21 | 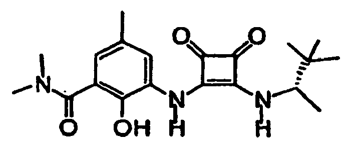 | 1. 70% |
| | | | | 2. 374 |
| | | | | 3. 167.8-170.1 |
| 366 | 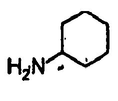 | 21.1 | 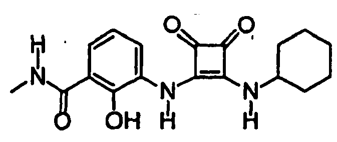 | 1. 75% |
| | | | | 2. 344 |
| | | | | 3. 170-172 |
| 367 | 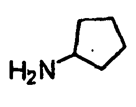 | 21.1 | 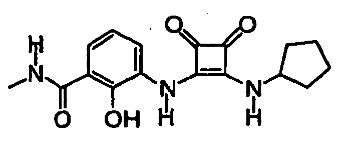 | 1. 66% |
| | | | | 2. 330 |
| | | | | 3. 160-162 |
| 368 | 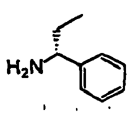 | 22 | 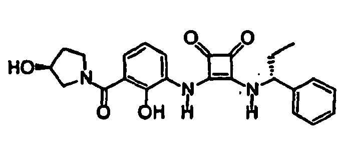 | 1. 31% |
| | | | | 2. 436 |
| | | | | 3. 140-145 |
| 368.1 | 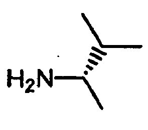 | 20 | 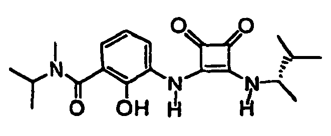 | 1. 8% |
| | | | | 2. 374 |
| | | | | 3. 130-133 |
| 368.2 | 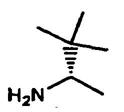 | 23.1 | 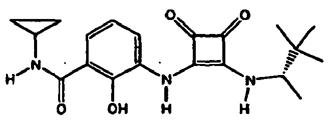 | 1. 56% |
| | | | | 2. 372 |
| | | | | 3. 188-191 |
| 368.3 | 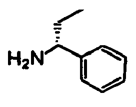 | 23.1 | 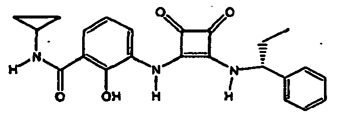 | 1. 67% |
| | | | | 2. 406 |
| | | | | 3. 142-144 |
| 368.4 | 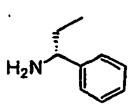 | 23.2 | 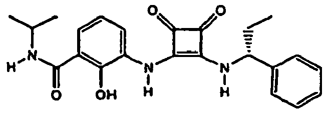 | 1. 69% |
| | | | | 2. 408 |
| | | | | 3. 147-150 |
| 368.5 | 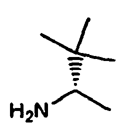 | 23.2 | 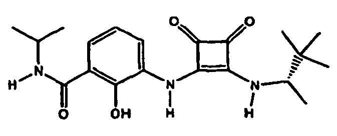 | 1. 67% |
| | | | | 2. 374 |
| | | | | 3. 177-180 |
| 368.6 | 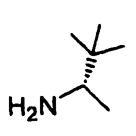 | 23.3 | 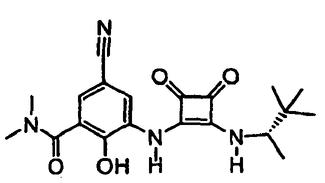 | 1. 45% |
| | | | | 2. 385 |
| | | | | 3. 236-240 |
| 368.7 | 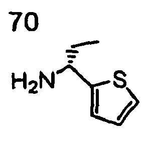 | 23.3 | 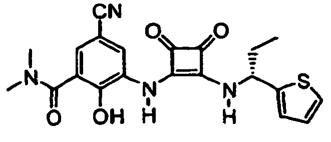 | 1. 35% |
| | | | | 2. 425 |
| | | | | 3. 248-251 |
| 368,8 | 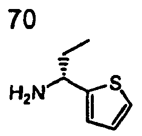 | 23.2 | 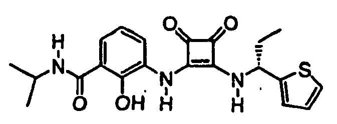 | 1. 66% |
| | | | | 2. 414 |
| | | | | 3. 156-160 |
| 368.9 | 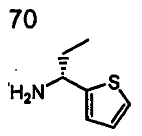 | 23.4 | 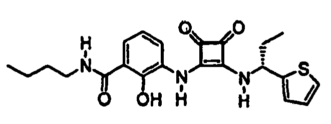 | 1. 78% |
| | | | | 2. 428 |
| | | | | 3. 138-140 |
| 368.10 | 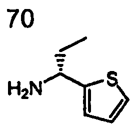 | 23.5 | 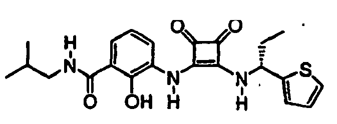 | 1. 46% |
| | | | | 2. 428 |
| | | | | 3. 149-153 |
| 368.11 | 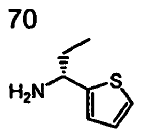 | 23.6 | 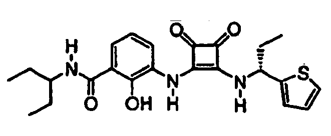 | 1. 54% |
| | | | | 2. 412 |
| | | | | 3. 136-138 |
| 368.12 | 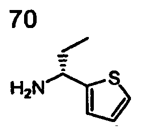 | 21 | 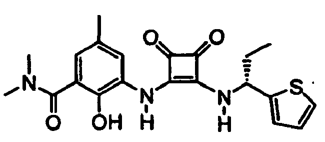 | 1. 30% |
| | | | | 2. 414 |
| | | | | 3. 164-167 |
| 368.13 | 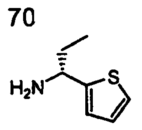 | 23.1 | 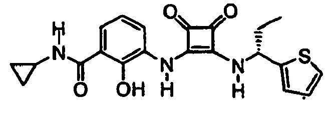 | 1. 25% |
| | | | | 2. 412 |
| | | | | 3. 172-177 |
| 368.14 | 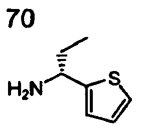 | 23.7 | 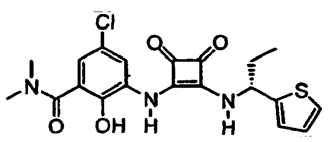 | 1. 21% |
| | | | | 2. 434 |
| | | | | 3. 208-211 |
| 368.15 | 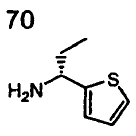 | 23.8 | 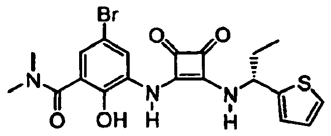 | 1. 27% |
| | | | | 2. 478 |
| | | | | 3. 216-219 |
| 368.16 | 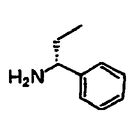 | 23.9 | 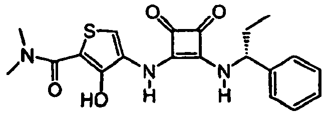 | 1. 63% |
| | | | | 2. 400 |
| 368.17 | 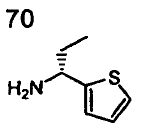 | 23.9 | 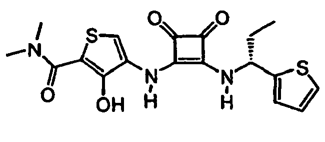 | 1. 61% |
| | | | | 2. 406.1 |
| | | | | 3. 127 DEC |
| 368.18 | 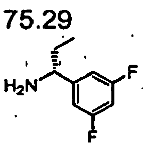 | 23.9 | 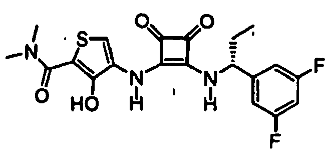 | 1. 68% |
| | | | | 2. 436.1 |
| | | | | 3. 128 DEC |
| 368.19 | 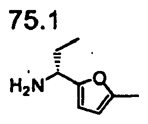 | 23.9 | 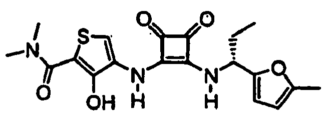 | 1. 72% |
| | | | | 2. 404.1 |
| | | | | 3. 126 DEC |
| 368.20 | 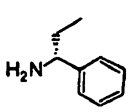 | 23.10 | 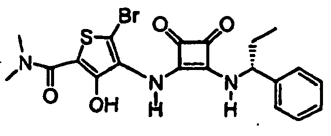 | 1. 8.4% |
| | | | | 2. 478 |
| 368.21 | 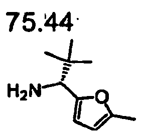 | 23.9 | 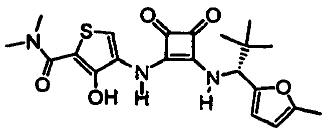 | 1. 39% |
| | | | | 2. 432.1 |
| | | | | 3. 151-153 |
| 368.22 | 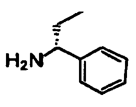 | 23.12 | 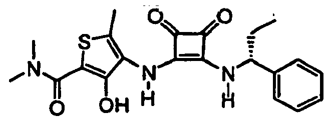 | 1. 78% |
| | | | | 2. 414.1 |
| | | | | 3. 210 DEC |
| 368.23 | 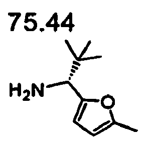 | 23.11 | 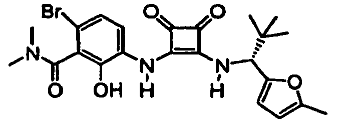 | 1. 4% |
| | | | | 2. 504 |
| 368.24 | 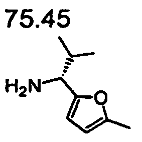 | 23.11 | 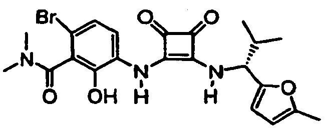 | 1. 31% |
| | | | | 2. 490 |
| | | | | 3. 241-245 |
| 368.25 | 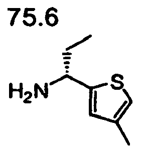 | 23.9. | 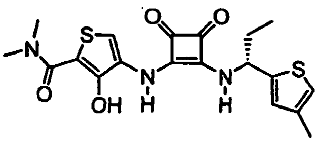 | 1. 81% |
| | | | | 2. 420.1 |
| | | | | 3. 126-128 |
| 368.26 | 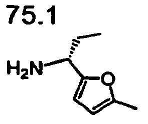 | 23.11 | 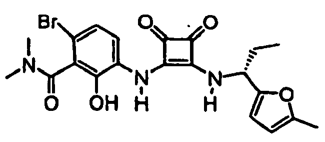 | 1. 8% |
| | | | | 2. 476 |
| | | | | 3. 193-198 |
| 368.27 | 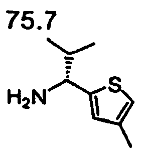 | 23.9 | 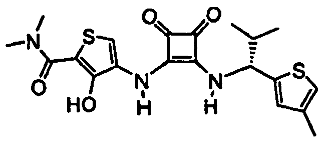 | 1. 70% |
| | | | | 2. 434.1 |
| | | | | 3. 130 DEC |
| 368,28 | 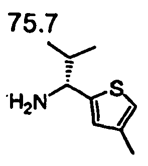 | 23.11 | 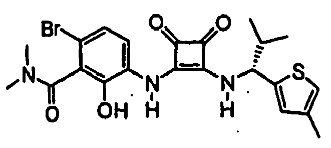 | 1. 83% |
| | | | | 2. 506 |
| | | | | 3. 222-227 |
| 368.29 | 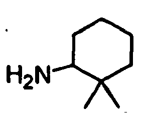 | 23.11 | 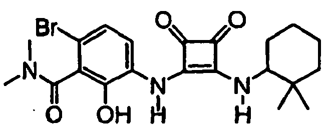 | 1. 17% |
| | | | | 2. 464 |
| | | | | 3. 183-190 |
| 368.30 | 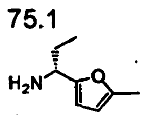 | 23.13 | 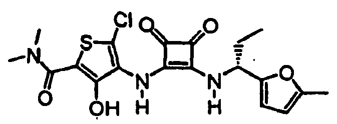 | 1. 6.5% |
| | | | | 2. 438.1 |
| 368.31 | 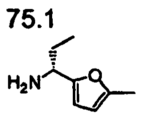 | 23.14 | 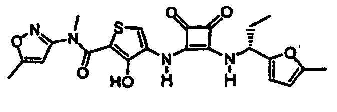 | 1. 71% |
| | | | | 2. 471.1 |
| | | | | 3. 149-151 |

### EXAMPLES 369-378.23

Following the procedure set forth in Example 210 but using the cyclobutenedione intermediate from Preparative Example indicated and the amine from the Preparative Example indicated in the Table below, the following cyclobutenedione products were obtained.

| Ex. | Prep Ex of Amine | Prep Ex of Cyclobutene intermediate | Product | 1.Yield (%) |
|---|---|---|---|---|
| | | | | 2. MH⁺ |
| | | | | 3. mp (°C) |
| 369 | 8 | 87 | 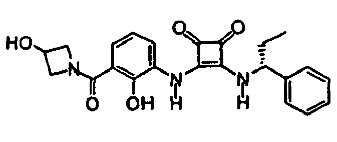 | 1. 41% |
| | | | | 2. 422 |
| | | | | 3. 135-140 |
| 370 | 9 | 87 | 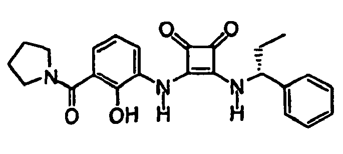 | 1. 60% |
| | | | | 2. 420 |
| | | | | 3. 120-125 |
| 371 | 10 | 87 | | 1. 59% |
| | | | 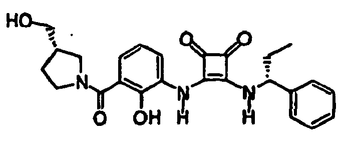 | 2. 450 |
| | | | | 3. 162-167 |
| 372 | 12 | 87 | 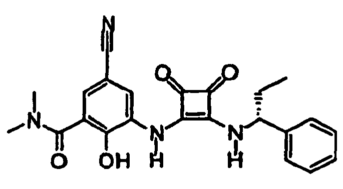 | 1. 34% |
| | | | | 2. 419 |
| | | | | 3. 157.2-168.2 |
| 373 | 12 | 88 | 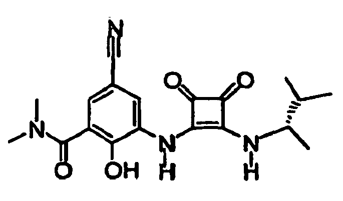 | 1. 18% |
| | | | | 2. 371 |
| | | | | 3. 142.3-144.6 |
| 374 | 13 | 87 | 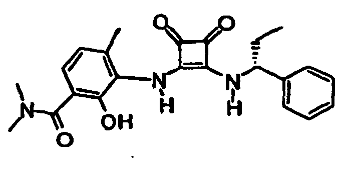 | 1. 41% |
| | | | | 2. 408 |
| | | | | 3. 245.3-247.8 |
| 375 | 5 | 87 | 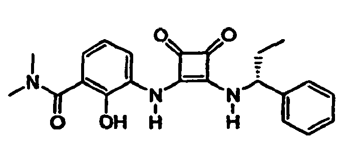 | 1. 32% |
| | | | | 2. 366 |
| | | | | 3. 165.7 |
| 376 | 6 | 87 | 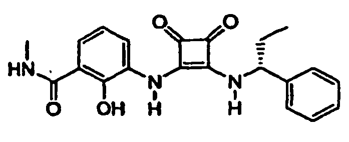 | 1. 17% |
| | | | | 2. 380 |
| | | | | 3. 173.5 |
| 377 | 7 | 87 | 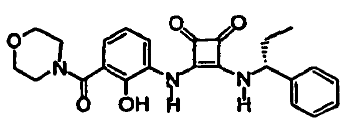 | 1. 48% |
| | | | | 2. 436 |
| | | | | 3. 175.6 |
| 378.1 | 3 | 88.3 | 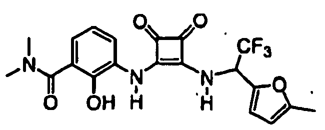 | 1. 73% |
| | | | | 2. 438.1 |
| | | | | 3.116, |
| 378.2 | 3 | 88.2 | 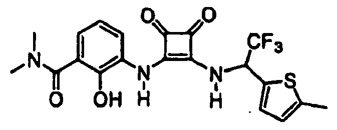 | 1. 58% |
| | | | | 2. 454 |
| | | | | 3. 140-142 |
| 378.3 | 13.3 | 87 | 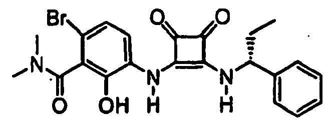 | 1. 43% |
| | | | | 2. 472 |
| | | | | 3. 206-209 |
| 378.4 | 3 | 23.16 | 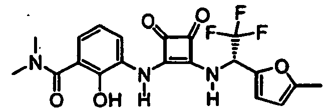 | 1. 69% |
| | | | | 2. 438.1 |
| | | | | 3. 116 |
| 378.5 | 3 | 23.17 | 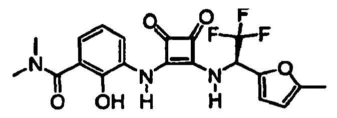 | 1. 73% |
| | | | | 2. 438.1 |
| | | | | 3. 116 |
| 378.6 | 13.4 | 87 | 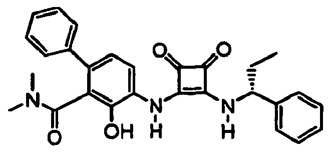 | 1. 10% |
| | | | | 2. 470 |
| | | | | 3. 198-201 DEC |
| 378.7 | 13.5 | 87 | 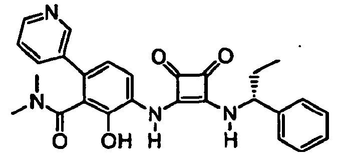 | 1. 16% |
| | | | | 2. 471 |
| | | | | 3. 246-248 |
| 378.8 | 13.3 | 23.16 | 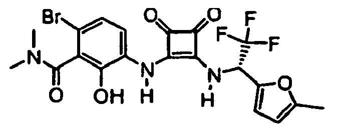 | 1. 30% |
| | | | | 2. 516/518 |
| | | | | 3. 234-240 DEC |
| 378.9 | 13.19 | 23.16 | 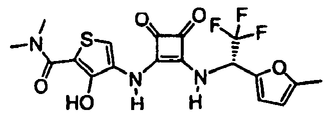 | 1. 65% |
| | | | | 2. 444.1 |
| 378.10 | 3 | 23.20 | 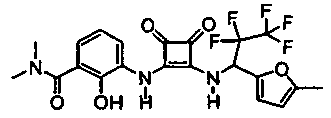 | 1. 78% |
| | | | | 2. 488 |
| | | | | 3. 137-140 |
| 378.11 | 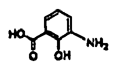 | 88.1 | 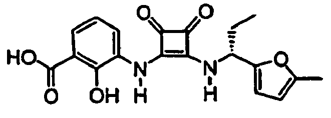 | 1. 24% |
| | | | | 2. 371 |
| | | | | 3. 254-260 DEC |
| 378.12 | 13.6 | 88.1 | 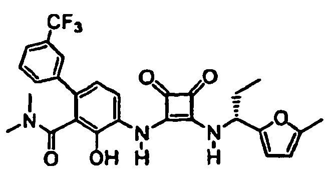 | 1. 3% |
| | | | | 2. 542 |
| 378.13 | 13.7 | 88.1 | 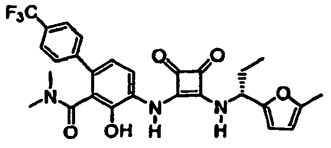 | 1. 9% |
| | | | | 2. 542 |
| 378.15 | 3 | 23.19 | 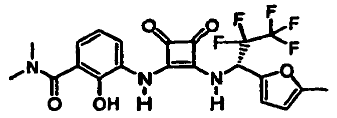 | 1. 71% |
| | | | | 2. 488 |
| | | | | 3. 136-138 |
| 378.16 | 3 | 23.22 | 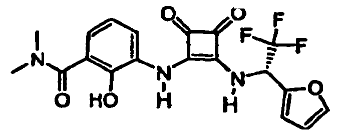 | 1. 35% |
| | | | | 2. 424.1 |
| | | | | 3. 132 |
| 378.17 | 13.9 | 88.1 | 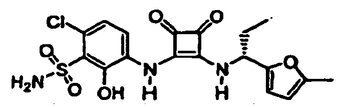 | 1. 13% |
| | | | | 2. 440 |
| | | | | 3. 219-223 |
| 378.18 | 13.10 | 88.1 | 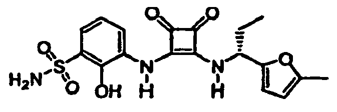 | 1. 26% |
| | | | | 2. 406 |
| | | | | 3. 242-249 DEC |
| 378.19 | 13.8 | 88.1 | 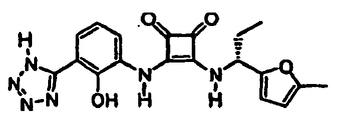 | 1. 18% |
| | | | | 2. 395 |
| 378.20 | 3. | 23.18 | 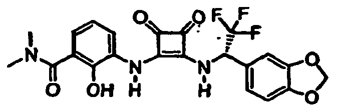 | 1. 53% |
| | | | | 2. 478.1 |
| | | | | 3. 126 |
| 378.21 | 3 | 23.21 | 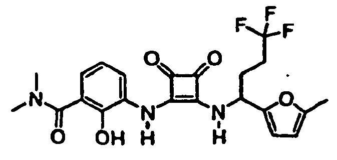 | 1. 66% |
| | | | | 2. 466 |
| | | | | 3. 106 |
| 378.22 | 3 | 23.24 | 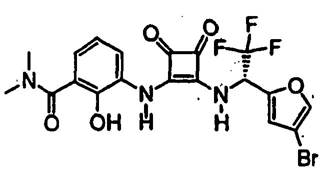 | 1. 73% |
| | | | | 2. 502.1 |
| | | | | 3. 121, |
| 378.23 | 3 | 23.23 | 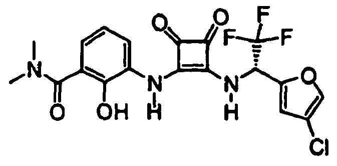 | 1.57% |
| | | | | 2. 458.1 |
| | | | | 3.129 |

### EXAMPLES 378.25-378.89

Following the procedure set forth in Example 210 but using the cyclobutenedione intermediate from Preparative Example indicated and the amine from the Preparative Example indicated in the Table below, the following cyclobutenedione products were obtained.

| Ex. | Prep Ex of Amine | Prep Ex of Cyclobutene intermediate | Product | 1.Yield (%) |
|---|---|---|---|---|
| | | | | 2. MH⁺ |
| 378.25 | 11.10 | 87.1 | 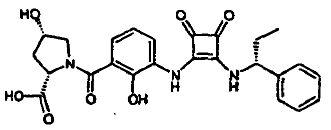 | 1. 71% |
| | | | | 2. 480.0 |
| 378.26 | 10.28 | 87.1 | 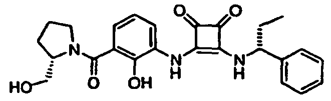 | 1. 60% |
| | | | | 2. 449.9 |
| 378.27 | 11.11 | 88.4 | 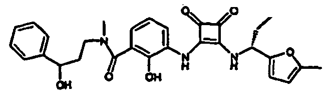 | 1. 25% |
| | | | | 2. 540.1 [M+Na^{+]} |
| 378.28 | 10.36 | 87.1 | 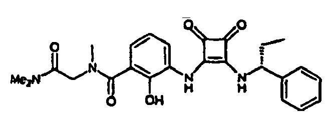 | 1. 16% |
| | | | | 2. 465.0 |
| 378.29 | 10.7 | 88.5 | 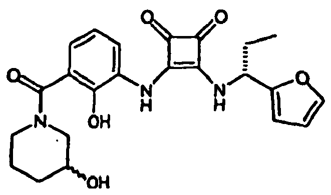 | 1. 46% |
| | | | | 2. 440.4 |
| 378.30 | 10.9 | 88.4 | 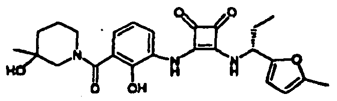 | 1. 43% |
| | | | | 2. 934.9 [dimer+1]⁺ |
| 378.31 | 11.12 | 88.4 | 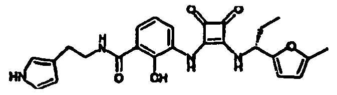 | 1. 48% |
| | | | | 2. 464.0 |
| 378.32 | 10.35 | 87.1 | 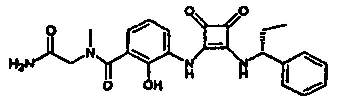 | 1. 17% |
| | | | | 2. 437 |
| 378.33 | 10.8 | 87.1 | 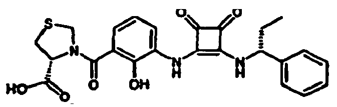 | 1. 10% |
| | | | | 2. 481.9 |
| 378.34 | 11.13 | 87.1 | 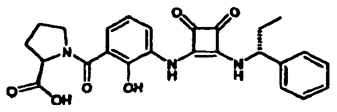 | 1. 55% |
| | | | | 2. 463.9 |
| 378.35 | 10.29 | 87.1 | 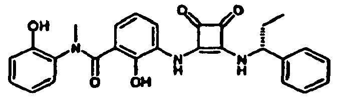 | 1. 34% |
| | | | | 2. 471.9 |
| 378.36 | 10.48. | 87.1 | 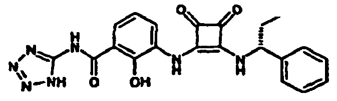 | 1. 4% |
| | | | | 2. 433.9 |
| 378.36 | 10.10 | 87.1 | 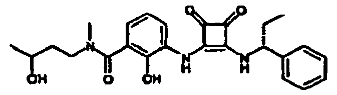 | 1. 85% |
| | | | | 2. 451.9 |
| 378.37 | 10.31 | 87.1 | 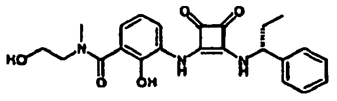 | 1. 36% |
| | | | | 2. 423.8 |
| 378.38 | 10.17 | 87.1 | 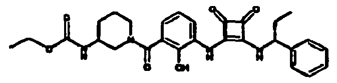 | 1. 85% |
| | | | | 2. 521.1 |
| 378.39 | 10.32 | 87.1 | 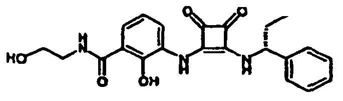 | 1. 63% |
| | | | | 2. 409.9 |
| 378.41 | 10.33 | 87.1 | 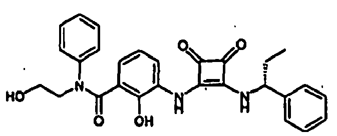 | 1. 20% |
| | | | | 2. 486.0 |
| 378.42 | 10.13 | 87.1 | 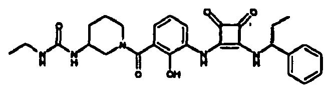 | 1. 47% |
| | | | | 2. 520.1 |
| 378.43 | 10.34 | 87.1 | 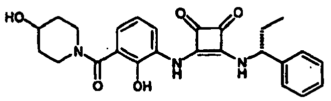 | 1. 18% |
| | | | | 2. 449.9 |
| 378.44 | 11.14 | 87.1 | 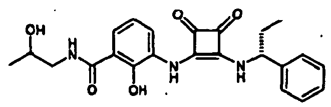 | 1. 13% |
| | | | | 2. 424.0 |
| 378.45 | 2.13 | 87.1 | 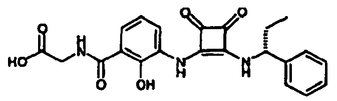 | 1. 13% |
| | | | | 2. 423.8 |
| 378.46 | 12.1 | 87.1 | 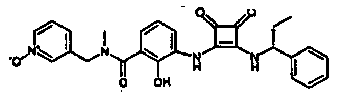 | 1. 51% |
| | | | | 2. 487.1 |
| 378.47 | 10.38 | 88.4 | 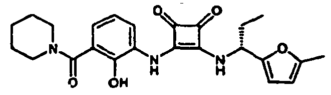 | 1. 72% |
| | | | | 2. 437.7 |
| 378.48 | 11.15 | 87.1 | 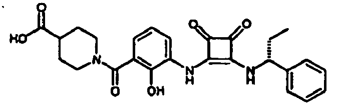 | 1. 29% |
| | | | | 2. 477.9 |
| 378.49 | 10.14 | 87.1 | 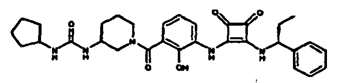 | 1. 61% |
| | | | | 2. 560.2 |
| 378.50 | 11.18 | 87.1 | 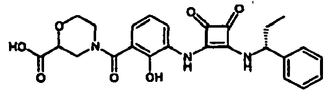 | 1. 25% |
| | | | | 2. 480.0 |
| 378.51 | 10.18 | 87.1 | 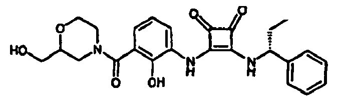 | 1. 51% |
| | | | | 2. 466.0 |
| 378.52 | 12.2 | 87.1 | 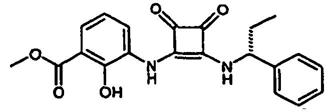 | 1. 32% |
| | | | | 2. 380.9 |
| 378.53 | 10.19 | 87.1 | 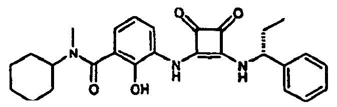 | 1. 14% |
| | | | | 2. 461.4 |
| 378.54 | 11.1 | 87.1 | 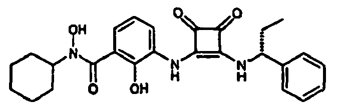 | 1. 41% |
| | | | | 2. 463.9 |
| 378.55 | 11.2 | 87.1 | 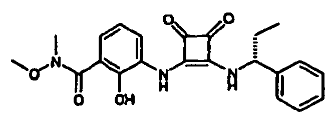 | 1. 5% |
| | | | | 2. 409.9 |
| 378.56 | 10.20 | 87.1 | 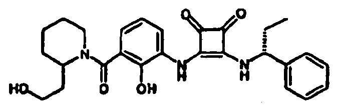 | 1. 70% |
| | | | | 2. 478.1 |
| 378.57 | 10.49 | 87.1 | 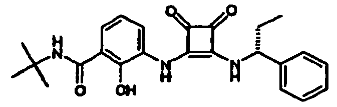 | 1. 17% |
| | | | | 2. 421.9 |
| 378.58 | 10.15 | 87.1 | 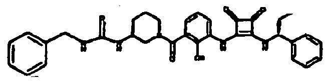 | 1. 51% |
| | | | | 2. 582.1 |
| 378.59 | 10.46 | 87.1 | 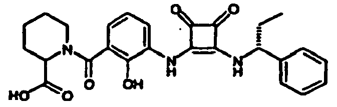 | 1. 18% |
| | | | | 2. 477.9 |
| 378.60 | 11.16 | 88.4 | 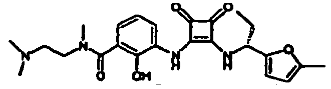 | 1. 54% |
| | | | | 2. 455.1 |
| 378.61 | 10.21 | 87.1 | 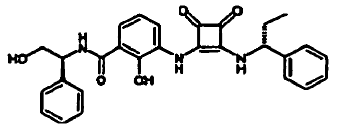 | 1. 84% |
| | | | | 2. 485.9 |
| 378.62 | 10.40 | 87.1 | 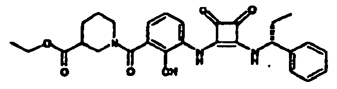 | 1. 4% |
| | | | | 2. 506.1 |
| 378.65 | 2.8 | 87.1 | 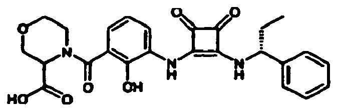 | 1. 34% |
| | | | | 2. 480 |
| 378.66 | 10.22 | 87.1 | 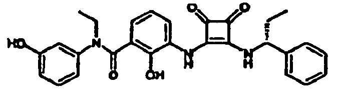 | 1. 16% |
| | | | | 2. 486.0 |
| 378.68 | 10.23 | 87.1 | 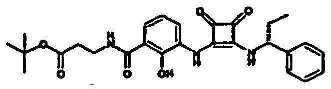 | 1. 26% |
| | | | | 2. 493.9 |
| 378.69 | 2.14 | 87.1 | 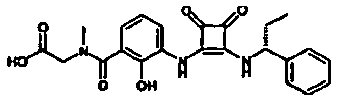 | 1. 60% |
| | | | | 2. 437.9 |
| 378.70 | 10.24 | 87.1 | 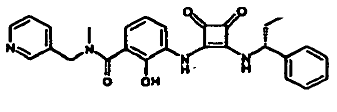 | 1. 64% |
| | | | | 2. 469.9 |
| 378.71 | 10.18 | 88.4 | 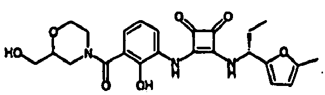 | 1. 64% |
| | | | | 2. 471.1 |
| 378.72 | 10.39 | 88.4 | 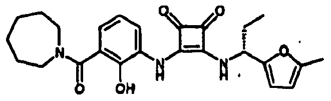 | 1. 41% |
| | | | | 2. 451.7 |
| 378.73 | 10.30 | 87.1 | 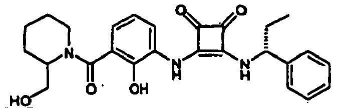 | 1. 60% |
| | | | | 2. 464.0 |
| 378.74 | 10.25 | 87.1 | 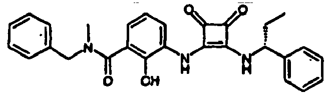 | 1. 63% |
| | | | | 2. 470.1 |
| 378.75 | 10.26 | 87.1 | 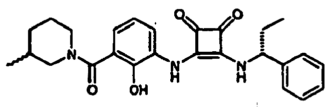 | 1. 10% |
| | | | | 2. 448.0 |
| 378.76 | 10.50 | 87.1 | 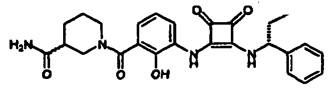 | 1. 5% |
| | | | | 2. 477.0 |
| 378.77 | 10.42 | 88.4 | 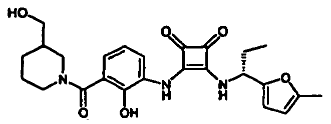 | 1. 57% |
| | | | | 2. 467.7 |
| 378.78 | 11.17 | 87.1 | 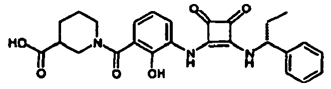 | 1. 75% |
| | | | | 2. 478.0 |
| 378.79 | 2.9 | 87.1 | 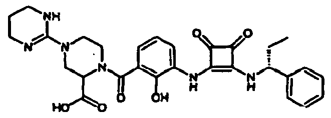 | 1. 21% |
| | | | | 2. 561 |
| 378.80 | 10.43 | 87.1 | 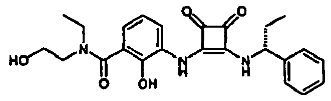 | 1. 69% |
| | | | | 2. 437.9 |
| 378.81 | 10.41 | 87.1 | 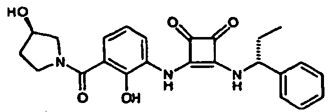 | 1. 3% |
| | | | | 2. 436.0 |
| 378.82 | 10.44 | 87.1 | 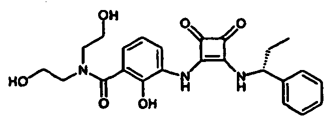 | 1. 90% |
| | | | | 2. 454.0 |
| 378.83 | 10.13 | 88.4 | 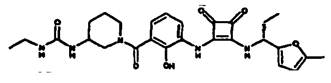 | 1. 29% |
| | | | | 2. 524.1 |
| 378.84 | 10.45 | 88.4 | 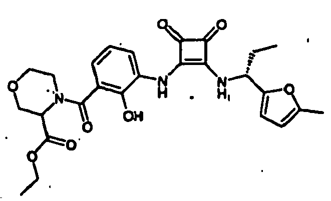 | 1. 46% |
| | | | | 2. 511.7 |
| 378.86 | 10.37 | 87.1 | 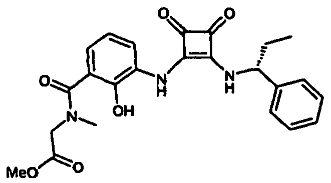 | 1. 53% |
| | | | | 2. 452.0 |
| 378.88 | 10.47 | 87.1 | 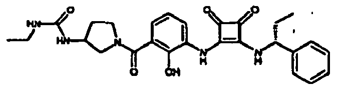 | 1. 61% |
| | | | | 2. 506.1 |
| 378.89 | 10.16 | 87.1 | 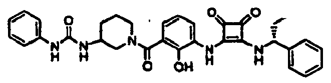 | 1. 30% |
| | | | | 2. 568.1 |

### EXAMPLE 378.90

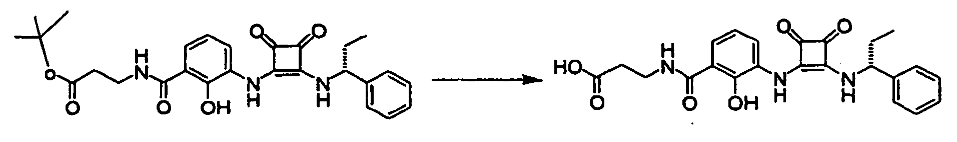

The above compound from Preparative Example 378.68 was stirred with 4N HCl/dioxane to yield the product (23%, MH+=437.9).

### EXAMPLE 378.91

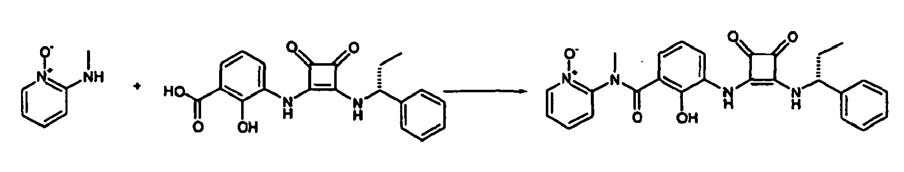

Using the procedure set forth in Preparative Example 2, Step A, but using Preparative Example 2.16 and Preparative Example 2.15, the title compound was prepared (20%, MH+=472.9).

### EXAMPLES 379-393

Following the procedure set forth in Example 210 but using the amine from the Preparative Example indicated and the ethoxy squarate intermediate from Preparative Example 87, the following cyclobutenedione products were obtained.

| Ex. | Aniline | Product | 1.Yield(%) |
|---|---|---|---|
| | | | 2. (MH⁺) |
| 379 | 109 | 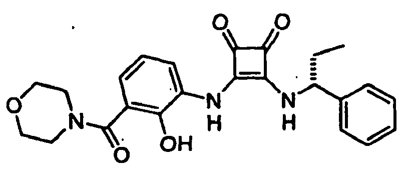 | 1. 29%. |
| | | | 2. 436.0 |
| 380 | 105 | 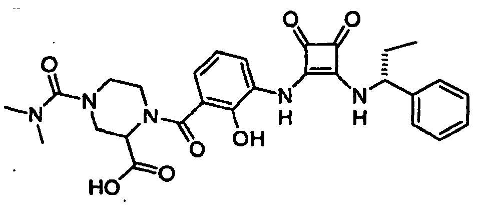 | 1. 6.3% |
| | | | 2. 550.0 |
| 381 | 106 | 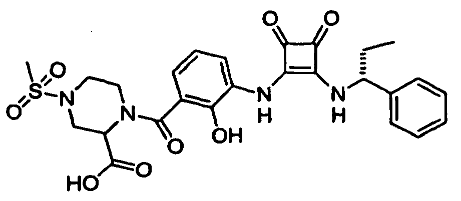 | 1. 12% |
| | | | 2. 557.0 |
| 382 | 107 | 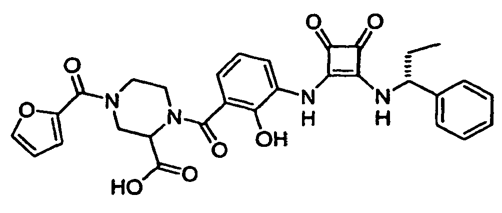 | 1. 8.6% |
| | | | 2. 573.0 |
| 383 | 143 | 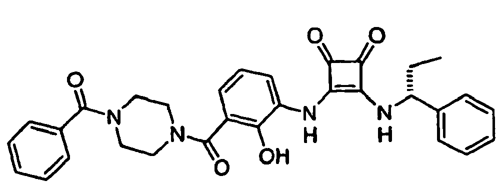 | 1. 3.2% |
| | | | 2. 497.0 |
| 384 | 135 | 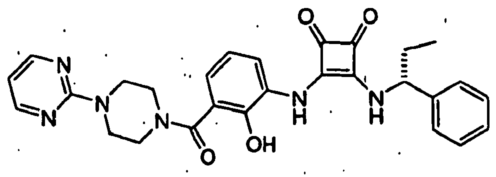 | 1. 36% |
| | | | 2. 529.0 |
| 385 | 130 | 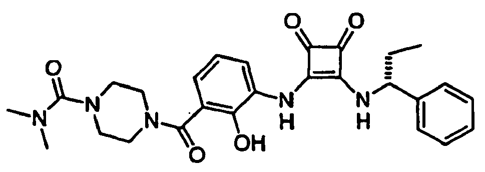 | 1. 33% |
| | | | 2. 506.1 |
| 387 | 145 | 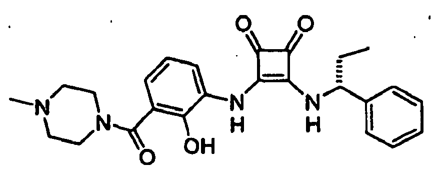 | 1. 27% |
| | | | 2. 449.1 |
| 388 | 140 | 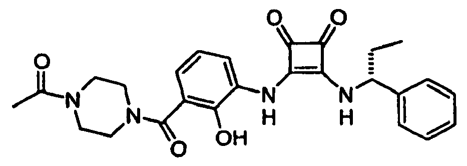 | 1. 25% |
| | | | 2. 477.0 |
| 389 | 98 | 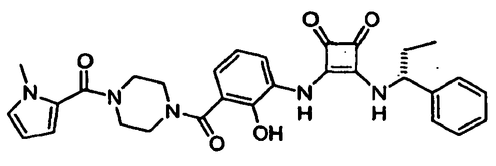 | 1. 66% |
| | | | 2. 542.1 |
| 390 | 96 | 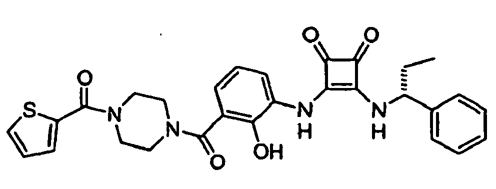 | 1. 60% |
| | | | 2. 545.0 |
| 391 | 97 | 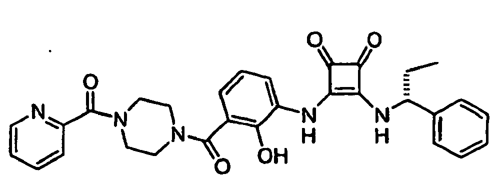 | 1. 66% |
| | | | 2. 540.1 |
| 392 | 100 | 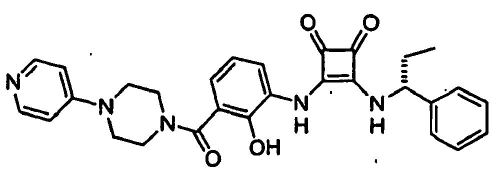 | 1. 47% |
| | | | 2. 512.1 |
| 393 | 99 | 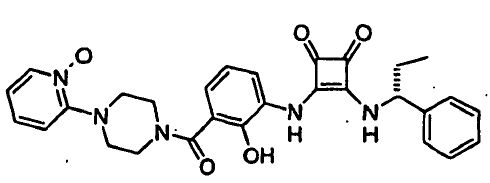 | 1. 60% |
| | | | 2. 528.1 |

### EXAMPLES 394 - 404.4

Following the procedure set forth in Example 261 but using the amines from the Preparative Examples indicated in the table below and the cyclobutenedione derivative from Preparative Example 19, the following cyctobutenedione products were obtained as racemic mixtures.

| Ex. | Prep Ex. of Amine | Product | 1.Yield (%) |
|---|---|---|---|
| | | | 2. MH⁺ |
| | | | 3. mp (°C) |
| 394 | 147 | 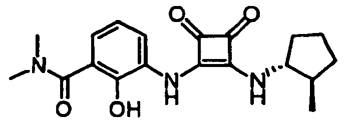 | 1. 64% |
| | | | 2. 358.1 |
| | | | 3. 137 |
| 395 | 148 | 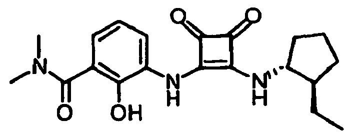 | 1. 23% |
| | | | 2. 372.1 |
| | | | 3. 126 |
| 396 | 149 | 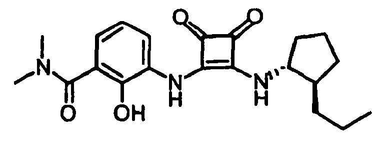 | 1. 94% |
| | | | 2. 386.1 |
| | | | 3. 108 |
| 397 | 150 | 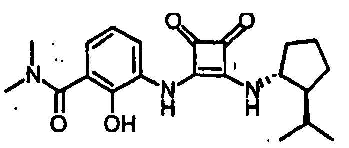 | 1. 86% |
| | | | 2. 386.1 |
| | | | 3. 134 |
| 398 | 146 | 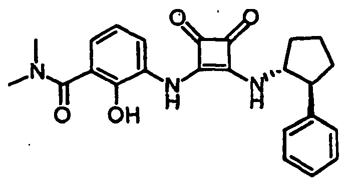 | 1. 87% |
| | | | 2. 420.1 |
| | | | 3. 136 |
| 399 | 151 | 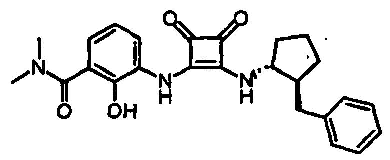 | 1.84% |
| | | | 2. 434.1 |
| | | | 3. 129 |
| 400 | 152 | 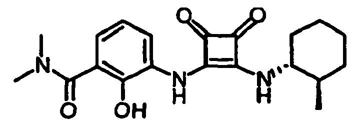 | 1. 90% |
| | | | 2. 372.1 |
| | | | 3. 154 |
| 401 | 153 | 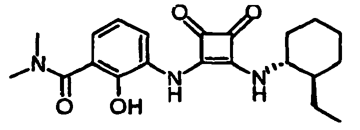 | 1. 86% |
| | | | 2. 386.1 |
| | | | 3. 156 |
| 402 | 154 | 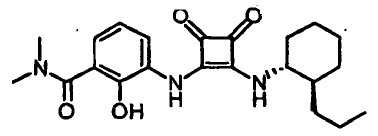 | 1. 90% |
| | | | 2. 400.1 |
| | | | 3. 153 |
| 403 | 155 | 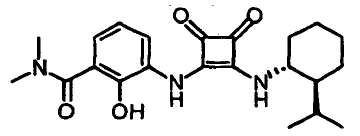 | 1. 91 |
| | | | 2. 400.1 |
| | | | 3. 153 |
| 404 | 156 | 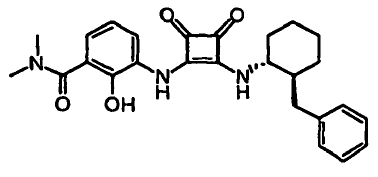 | 1. 83% |
| | | | 2. 448.1 |
| | | | 3. 138 |
| 404.1 | 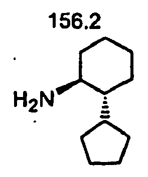 | 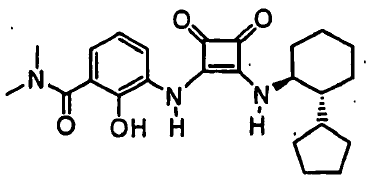 | 1. 30% |
| | | | 2. 426.1 |
| | | | 3. 132 |
| 404.2 | 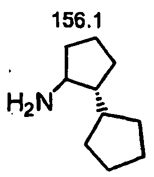 | 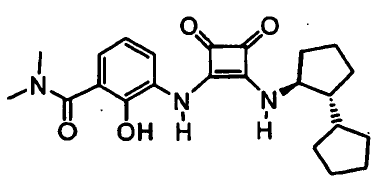 | 1. 74% |
| | | | 2.412.1 |
| | | | 3. 127 |
| 404.3 | 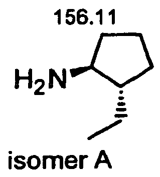 | 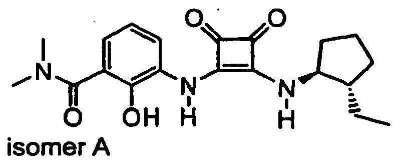 | 1.73.4% |
| | | | 2. 372.1 |
| | | | 3. 128 |
| 404.4 | 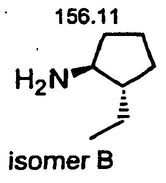 | 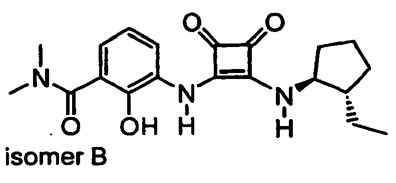 | 1.72% |
| | | | 2. 372.1 |
| | | | 3.128 |

### EXAMPLE 405

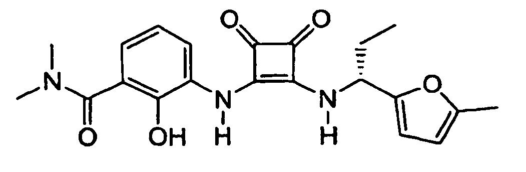

To a solution of the amine from Preparative Example 75.1 (11.3 g) in EtOH (100 mL) at room temperature was added the product from Preparative Example 19 (16.4 g) in one portion. The resulting mixture was stirred at reflux overnight and then concentrated under reduced pressure. The crude residue was redissolved in CH₂Cl₂ (80 mL) and was washed with 10% KH₂PO₄ (120 mL). The solid precipitate that was generated was filtered, washed with water and dried under vacuo. The residue was recrystallized from methanol-methylene chloride to give a cream colored solid (16 g, 75% yield). (mp 105-108 °C, MH⁺ 398.1).

### EXAMPLES 1101-1112.10

If one were to follow the procedure set forth in Example 210 but using the ethoxysquarate from the Preparative Example indicated and the amines from the Preparative Example indicated in the Table below, the following cyclobutenedione products can be obtained.

| Ex. | Prep Ex of Amine | Prep Ex of Squarate | Product |
|---|---|---|---|
| 1101 | 15 | 87 | 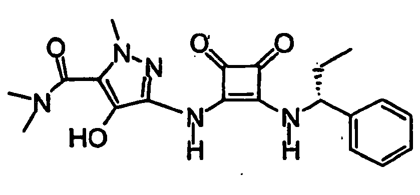 |
| 1102 | 15 | 88 | 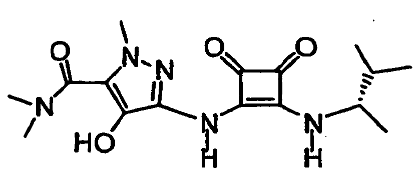 |
| 1103 | 16 | 87 | 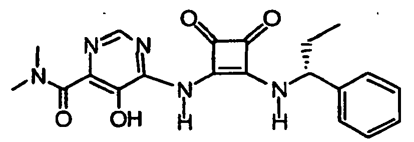 |
| 1104 | 16 | 88 | 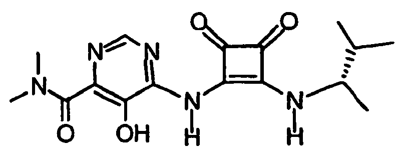 |
| 1105 | 17 | 87 | 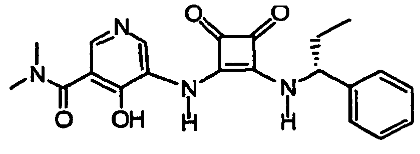 |
| 1106 | 17 | 88 | 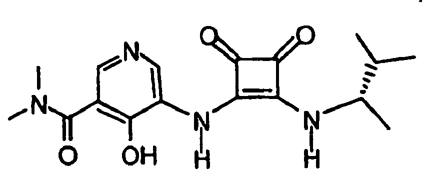 |
| 1112.3 | 500.5 | 19 | 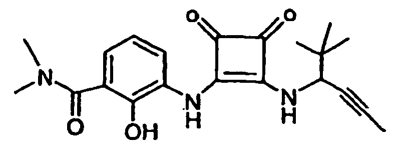 |
| 1112.4 | 75.9 | 23.11 | 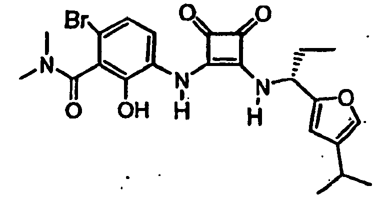 |
| 1112.5 | 10.19 | 88.4 | 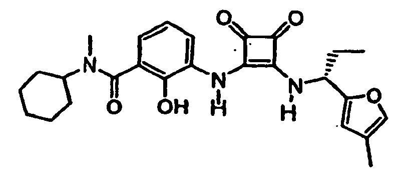 |
| 1112.6 | 75.44 | 23.14 | 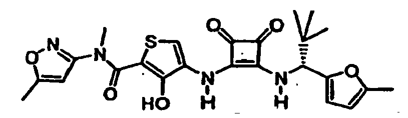 |
| 1112.7 | 75.49 | 23.14 | 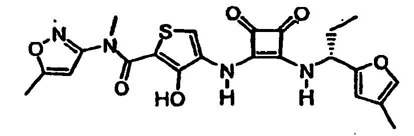 |
| 1112.8 | 75.50 | 23.14 | 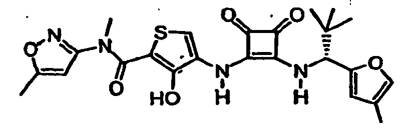 |
| 1112.9 | 75.44 | 500.6 | 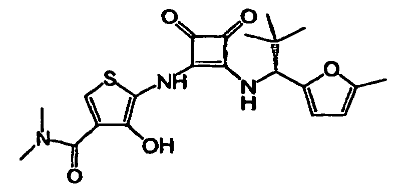 |
| 1112.10 | 75.49 | 500.6 | 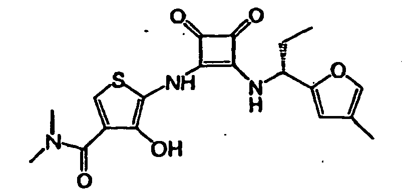 |

The following EXAMPLES 1120.1-1120.12, 1125, 1130 and 1131 illustrate procedures for preparing compounds not in accordance with the invention.

### EXAMPLES 1120.1-1120.12

Following the procedure set forth in Example 210 but using the amine from the Preparative Example indicated and the ethoxy squarate intermediate from the Preparative Example indicated, the following cyclobutenedione products were obtained.

| Ex. | Prep Ex of Amine | Prep Ex of Squara te | Product | 1.Yield (%) |
|---|---|---|---|---|
| | | | | 2. MH⁺ |
| | | | | 3. mp (°C) |
| 1120.1 | 156.16 | 87 | 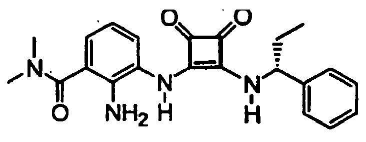 | 1. 9% |
| | | | | 2. 393.1 |
| | | | | 3. 154-158 |
| 1120.2 | 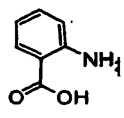 | 88.1 | 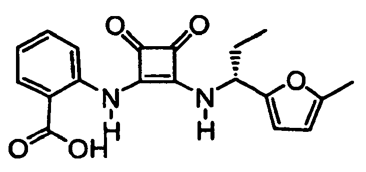 | 1. 55 % |
| | | | | 2. 355 .1 |
| | | | | 3. 199-201 |
| 1120.3 | 156.12 | 88.1 | 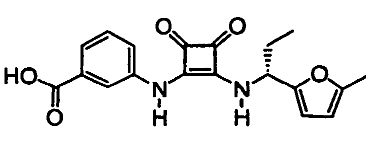 | 1. 37 % |
| | | | | 2. 355.1 |
| | | | | 3. 210-213 |
| 1120.4 | 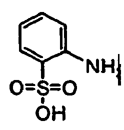 | 88.1 | 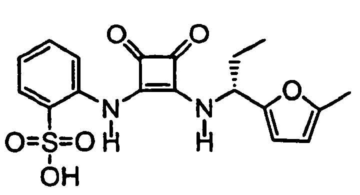 | 1. 30 % |
| | | | | 2. 391.1 |
| | | | | 3. 70-73 |
| 1120.5 | 156.14 | 88.1 | 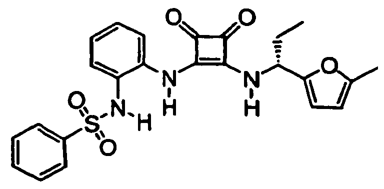 | 1. 73 % |
| | | | | 2. 466 |
| | | | | 3. 105-108 |
| 1120.6 | 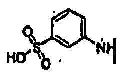 | 88.1 | 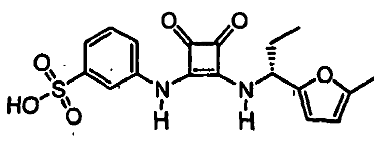 | 1. 21 % |
| | | | | 2. 391 |
| | | | | 3. 79-82 |
| 1120.7 | 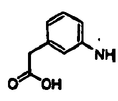 | 88.1 | 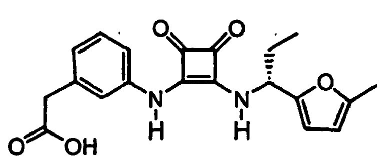 | 1. 15 % |
| | | | | 2. 369 |
| | | | | 3. 167-170 |
| 1120.8 | 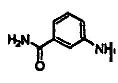 | 88.1 | 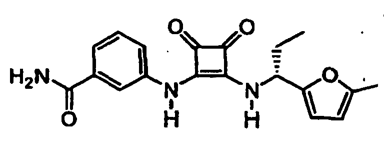 | 1. 47 % |
| | | | | 2. 354 |
| | | | | 3. 121-124 |
| 1120.9 | 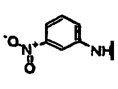 | 88.1 | 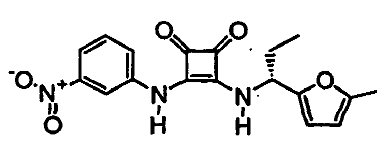 | 1. 15 % |
| | | | | 2. 356 |
| | | | | 3. 200-202 |
| 1120.10 | 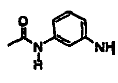 | 88.1 | 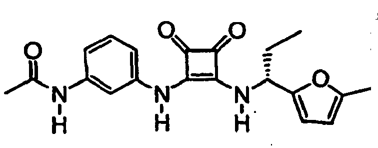 | 1. 25 % |
| | | | | 2. 468 |
| | | | | 3. 154-156 |
| 1120.11 | 156.13 | 88.1 | 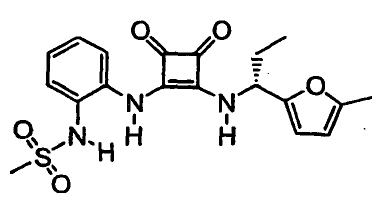 | 1. 57 % |
| | | | | 2. 404 |
| | | | | 3. 92-94 |
| 1120.12 | 156.15 | 88.1 | 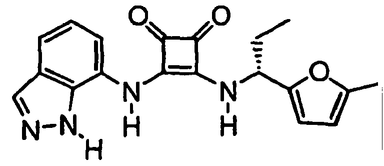 | 1. 61 % |
| | | | | 2. 351 |
| | | | | 3. 155-157 |

### EXAMPLE 1125

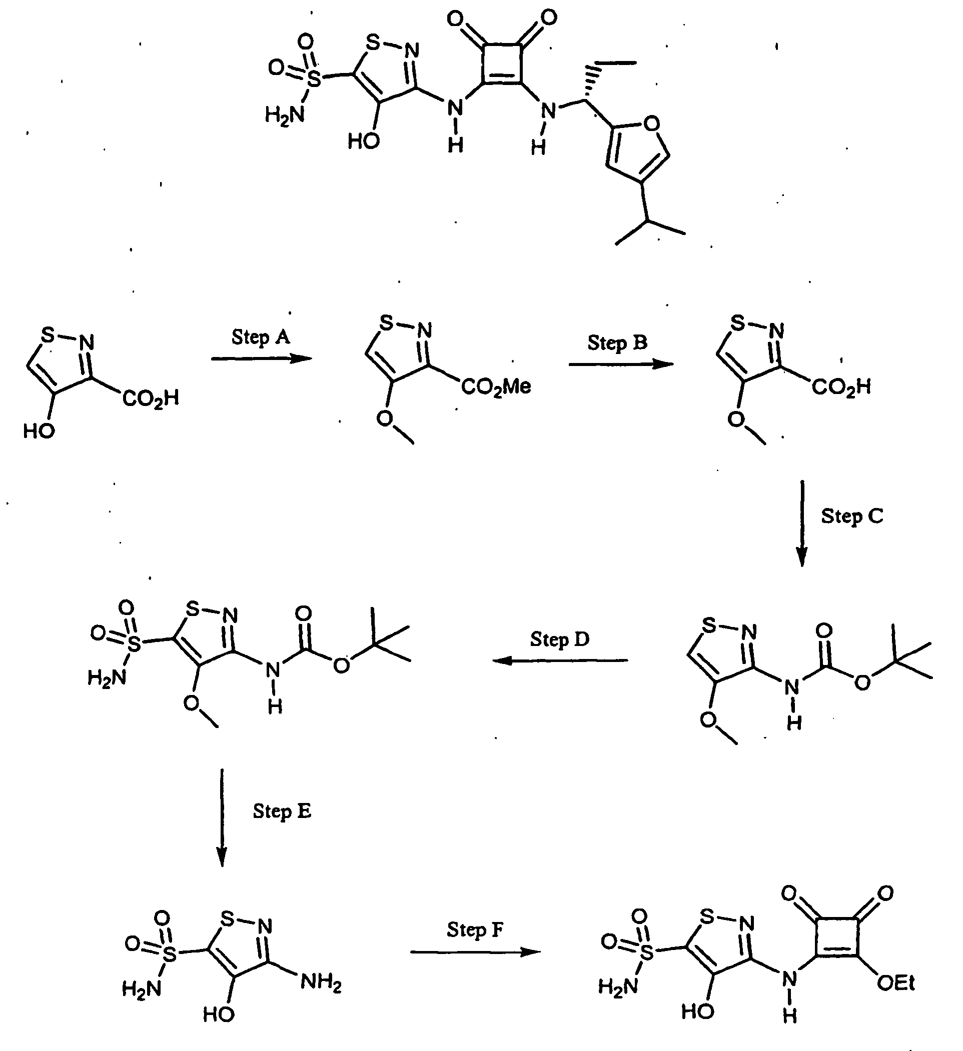

### Step A

If one were to use a similar procedure to that used in Preparative Example 13.3 Step B, except using the hydroxy acid from Bioorg. Med. Chem. Lett. 6(9), 1996, 1043, one would obtain the desired methoxy compound.

### Step B

If one were to use a similar procedure to that used in Preparative Example 13.19 Step B, except using the product from Step A above, one would obtain the desired compound.

### Step C

If one were to use a similar procedure to that used in Synth. Commun. 1980, 10, p. 107, except using the product from Step B above and t-butanol, one would obtain the desired compound.

### Step D

If one were to use a similar procedure to that used in Synthesis, 1986, 1031, except using the product from Step C above, one would obtain the desired sulfonamide compound.

### Step E

If one were to use a similar procedure to that used in Preparative Example 13.19 Step E, except using the product from Step D above, one would obtain the desired compound.

### Step F

If one were to use a similar procedure to that used in Preparative Example 19, except using the product from Step E above and adding potassium carbonate as base, one would obtain the desired compound.

### Step G

If one were to follow the procedure set forth in Example 210, except using the product from Step F above and the amine from Preparative Example 75.9, then one would obtain the title compound.

### EXAMPLE 1130

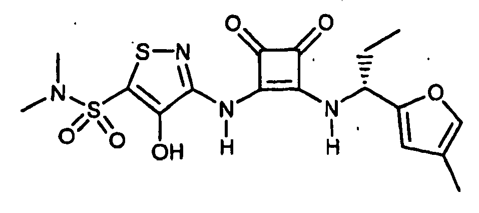

### Step A

If one were to treat the product from Step C of Example 1125 with BuLi (2.2 eq.) in THF followed by quenching of the reaction mixture with N,N,-dimethylsulfamoyl chloride (1.1 eq.) then one would obtain

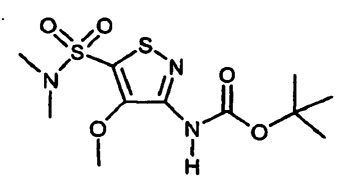

### Step B

If one were to use the product of Step A above and one were to follow Steps E, F and G of Example 1125, except using the amine from Preparative Example 75.49 in Step G, then one would obtain the title compound.

### EXAMPLE 1131

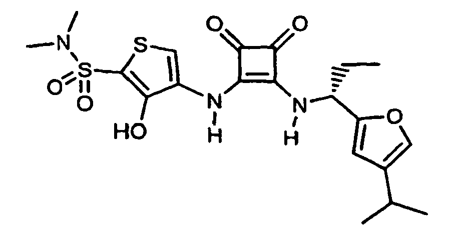

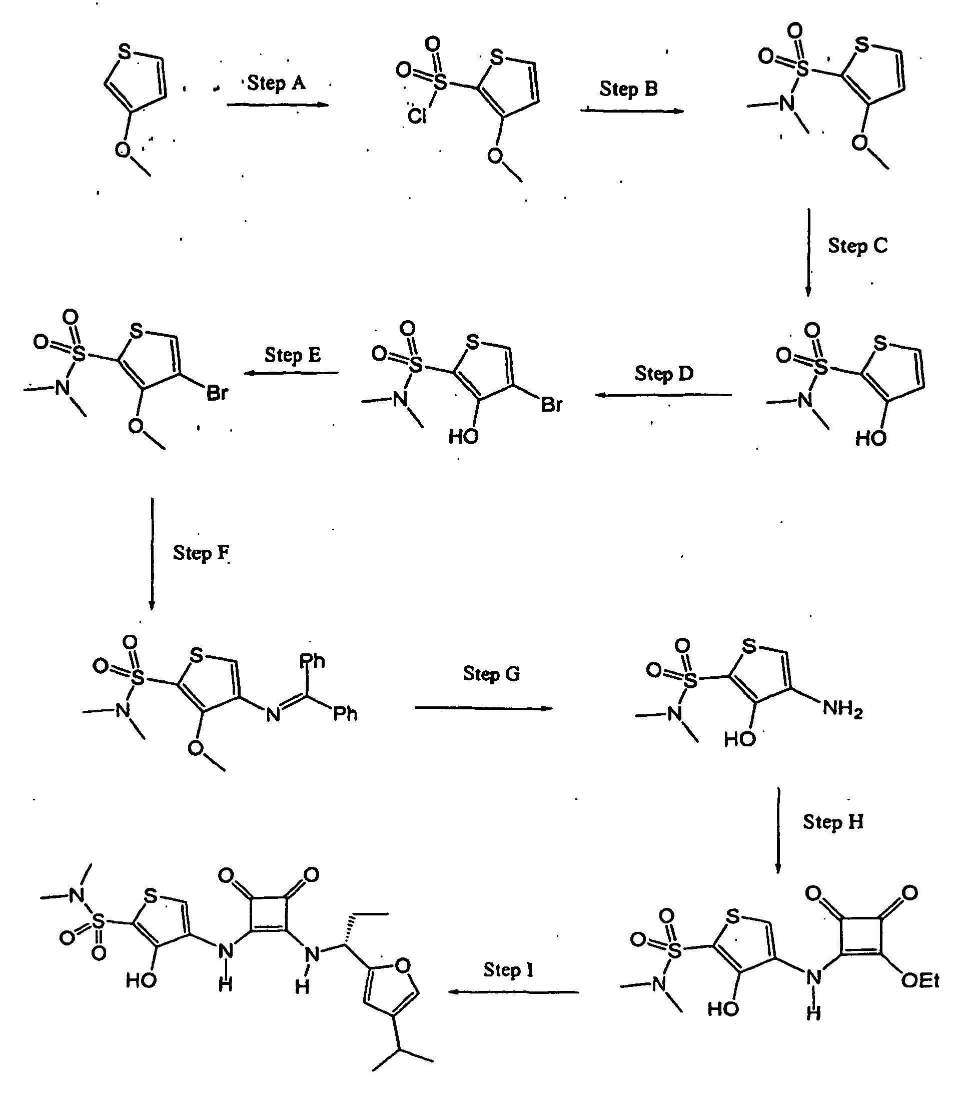

### Step A

To a solution of 3-methoxythiophene (3 g) in dichloromethane (175 mL) at - 78°C was added chlorosulfonic acid (8.5 mL) dropwise. The mixture was stirred for 15 min at -78°C and 1.5 h at room temp. Afterwards, the mixture was poured carefully into crushed ice, and extracted with dichloromethane. The extracts were washed with brine, dried over magnesium sulfate, filtered through a 1-in silica gel pad. The filtrate was concentrated in vacuo to give the desired compound (4.2 g).

### Step B

The product from Step A above (4.5 g) was dissolved in dichloromethane (140 mL) and added with triethylamine (8.8 mL) followed by diethyl amine in THF (2M, 21 mL). The resulting mixture was stirred at room temperature overnight. The mixture was washed with brine and saturated bicarbonate (aq) and brine again, dried over sodium sulfate, filtered through a 1-in silica gel pad. The filtrate was concentrated in vacuo to give the desired compound (4.4 g).

### Step C

The product from Step B above (4.3 g) was dissolved in dichloromethane (125 mL) and cooled in a -78°C bath. A solution of boron tribromide (1.0 M in dichloromethane, 24.3 mL) was added. The mixture was stirred for 4 h while the temperature was increased slowly from -78°C to 10°C. H₂O was added, the two layers were separated, and the aqueous layer was extracted with dichloro- methane. The combined organic layer and extracts were wahed with brine, dried over magnesium sulfate, filtered, and concentrated in vacuo to give 3.96 g of the desired hydroxy-compound.

### Step D

The product from step C above (3.96 g) was dissolved in 125 mL of dichloromethane, and added with potassium carbonate (6.6 g) followed by bromine (2 mL). The mixture was stirred for 5 h at room temperature, quenched with 100 mL of H₂O. The aqueous mixture was addjusted to pH - 5 using a 0.5N hydrogen chloride aqueous solution, and extracted with dichloromethane. The extracts were washed with brine, dried over sodium sulfate, and filtered through a celite pad. The filtrate was concentrated in vacuo to afford 4.2 g of the desired bromo-compound.

### Step E

The product from Step D (4.2 g) was dissolved in 100 mL of acetone and added with potassium carbonate (10 g) followed by iodomethane (9 mL). The mixture was heated to reflux and continued for 3.5 h. After cooled to room temperature, the mixture was tittered through a Celite pad. The filtrate was concentrated in vacuo to a dark brown residue, which was purified by flash column chromatography eluting with dichloromethane-hexanes (1:1, v/v) to give 2.7 g of the,desired product.

### Step F

The product from step E (2.7 g) was converted to the desired imine compound (3 g), following the similar procedure to that of Preparative Example 13.19 step D.

### Step G

The imine product from step F (3 g) was dissolved in 80 mL of dichloromethane and cooled in a -78°C bath. A solution of boron tribromide (1.0 M in dichloromethane, 9.2 mL) was added dropwise. The mixture was stirred for 4.25 h from -78°C to 5°C. H₂O (50 mL) was added, and the layers were separated. The aqueous layer was extracted with dichloromethane. The organic layer and extracts were combined, washed with brine, and concentrated to an oily residue. The residue was dissolved in 80 mL of methanol, stirred with sodium acetate (1.5 g) and hydroxyamine hydrochloride (0.95 g) at room temperature for 2 h. The mixture was poured into an aqueous mixture of sodium hydroxide (1.0 M aq, 50 mL) and ether (100 mL). The two layers were separated. The aqueous layer was washed with ether three times. The combined ether washings were re-extracted with H₂O once. The aqueous layers were combined, washed once with dichloromethane, adjusted to pH ~ 6 using 3.0 M and 0.5 M hydrogen chloride aqueous solutions, and extracted with dichloromethane. The organic extracts were combined, washed with brine, dried over sodium sulfate, and concentrated in vacuo to give 1.2 g of desired amine compound.

### Step H

The product from step F (122 mg) was stirred with diethyoxysquarate (0.25 mL) and potassium carbonate (75 mg) in 5 mL of ethanol at room temperature for 5 h. The mixture was diluted with dichloromethane, filtered through a Celite pad, and concentrated to an oily residue, which was separated by preparative TLC (CH₂Cl₂-MeOH= 15:1, v/v) to give 91 mg of the desired product.

### Step I

Following the procedure set forth in Example 210, and using the amine from Preparative Example 75.9, the product (43 mg) from Step H was converted to the desired compound (20 mg).

This invention provides novel compounds of the formula (I): 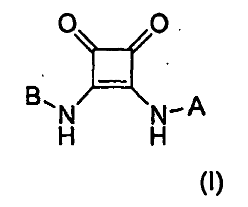 a prodrug thereof, or a pharmaceutically acceptable salt or solvate of the compound or of said prodrug;
wherein
A is selected from: 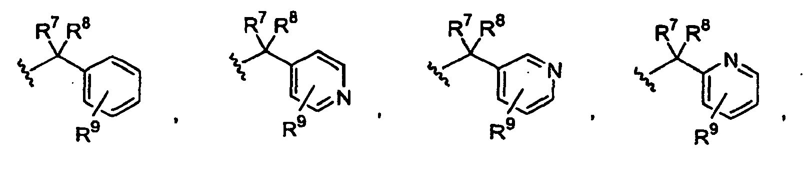 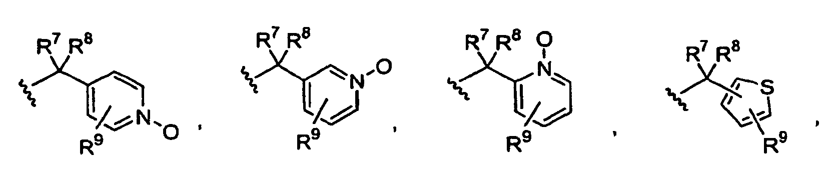 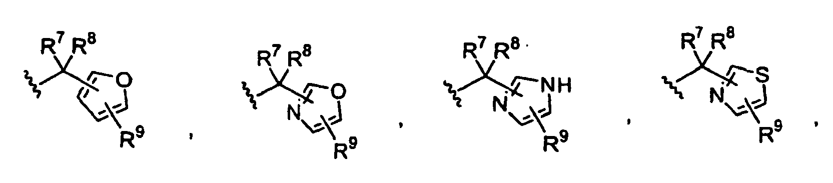 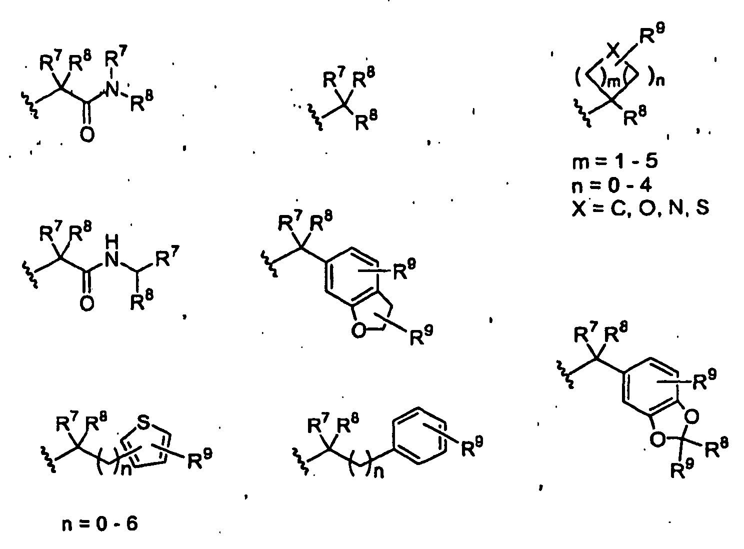 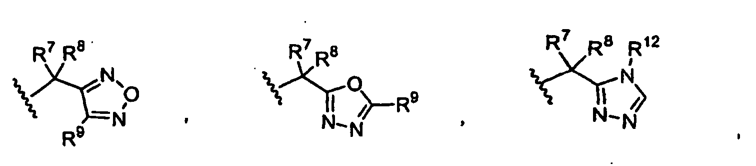 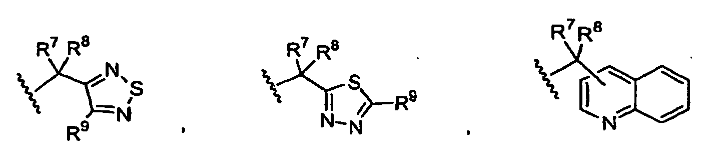 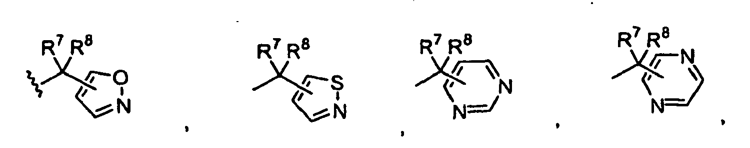 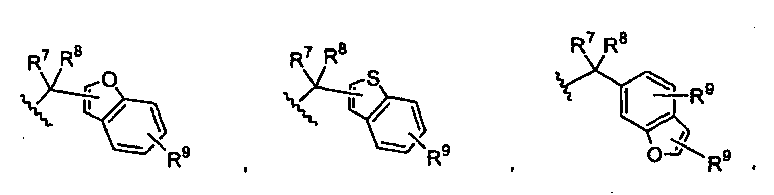 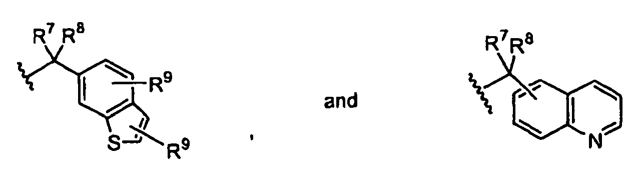 wherein,
R⁷ and R⁸ are the same or different and are independently selected from the group consisting of H; optionally substituted or unsubstituted alkyl, aryl, heteroaryl, arylalkyl, heteroarylalkyl, cycloalkyl, cycloalkylalkyl, CO₂R¹³, CONR¹³R¹⁴, fluoroalkyl, alkynyl, alkenyl, alkynylalkyl, alkenylalkyl, and cycloalkenyl,
wherein said substituents on said substituted groups are selected from the group consisting of:
a) cyano;
b) CO₂R⁷;
c) CONR⁷R⁸;
d) SO₂NR⁷R⁸,
e) SO₂R⁷;
f) NO₂;
g) CF₃;
h) -OR⁷;
i) -NR⁷R⁸;
j) -O(C=O)R⁷;
k) -O(C=O)NR⁷R⁸, and
l) halogen;
R⁹ is selected from one or more of the groups consisting of:
a) R⁷;
b) R⁸;
c) halogen;
d) -CF₃;
e) -COR⁷;
f) -OR⁷;
g) -NR⁷R⁸;
h) -NO₂;
i) -CN;
j) -SO₂R⁷;
k) -SO₂NR⁷R⁸;
l) -NR⁷COR⁸;
m) -CONR⁷R⁸ ;
n) -NR⁷CO₂R⁸;
o) CO₂R⁷, and
p) 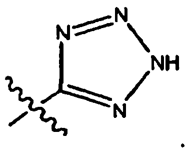
B is an optionally substituted aryl or heteroaryl group selected from: 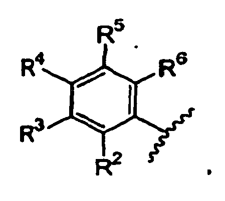 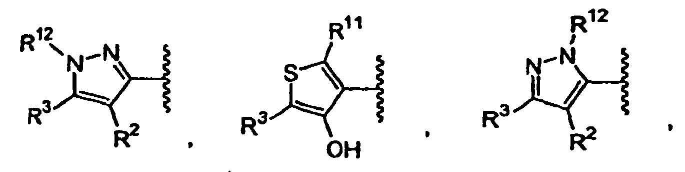 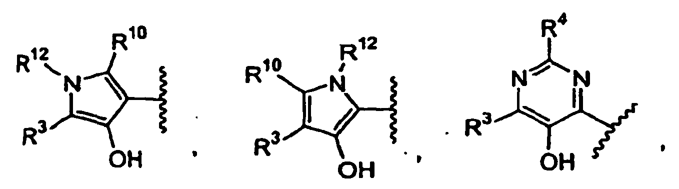 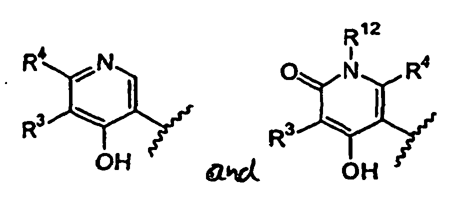 wherein,
R² is OH;
R³ is C(O)NR¹³R¹⁴ or C(O)NR¹³OR¹⁴
R⁴ is hydrogen, halogen, alkyl, alkoxy, OH, CF₃, OCF₃, NO₂, C(O)R¹³, C(O)OR¹³, C(O)NR¹³R¹⁴, SO₍ₜ₎NR¹³R¹⁴,SO₍ₜ₎R¹³, C(O)NR¹³OR¹⁴. 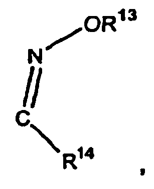 cyano, optionally substituted aryl or heteroaryl,
wherein the substituents on the optionally substituted groups may be selected from one or more R⁹ groups.

R⁵ and R⁶ independently represent hydrogen, halogen, alkyl, alkoxy, CF₃, OCF₃, NO₂, C(O)R¹³, C(O)OR¹³, C(O)NR¹³R¹⁴, SO₍ₜ₎NR¹³R¹⁴, C(O)NR¹³OR¹⁴, cyano, or an optionally substituted aryl or heteroaryl group,
wherein the substituents on the optionally substituted groups may be selected from one or more R⁹ groups.

R¹⁰ and R¹¹ independently represent hydrogen, halogen, CF₃. OCF₃, NR¹³R¹⁴, NR¹³C(O)NR¹³R¹⁴, OH, C(O)OR¹³, SH, SO₍ₜ₎NR¹³R¹⁴,SO₂R¹³, NHC(O)R¹³, NHSO₂NR¹³R¹⁴, NHSO₂R¹³, C(O)NR¹³R¹⁴, C(O)NR¹³OR¹⁴, OC(O)R¹³ or cyano.

R¹² is hydrogen, OC(O)R¹³, or an optionally substituted aryl, heteroaryl, arylalkyl, cycloalkyl, alkyl, cycloalkylalkyl or heteroarylalkyl group;

R¹³ and R¹⁴ are the same or different and are independently selected from the group consisting of H; optionally substituted or unsubstituted alkyl, aryl, heteroaryl, arylalkyl, heteroarylalkyl, cycloalkyl, cycloalkylalkyl, and fluoroalkyl, or

R¹³ and R¹⁴ when taken together form an optionally substituted 3 to 7 membered heterocyclic ring containing one to two heteroatoms selected from O, S and N, and
wherein the substituents on the optionally substituted groups may be selected from one or more R⁹ groups; and
t is 1 or 2.

The definitions below refer to the formula I that directly precedes these definitions.

These definitions apply regardless of whether a term is used by itself or in combination with other terms. Hence the definition of "alkyl" applies to "alkyl" as well as to the "alkyl" portions of "alkoxy", etc.

When any variable (*e.g.*, aryl, R²) occurs more than one time in any constituent, its definition on each occurrence is independent of its definition at every other occurrence. Also, combinations of substituents and/or variables are permissible only if such combinations result in stable compounds.

Alkyl represents a straight or branched saturated hydrocarbon chain having the designated number of carbon atoms. Where the number of carbon atoms is not specified, 1 to 20 carbons are intended.

The term halogen or Halo is intended to include fluorine, chlorine, bromine or iodine.

The term fluoroalkyl represents a straight or branched saturated hydrocarbon chain having the designated number of carbon atoms, substituted with one or more fluorine atoms. Where the number of carbon atoms is not specified, 1 to 20 carbons are intended

Aryl refers to a mono- or bicyclic ring system having one or two aromatic rings including, but not limited to, phenyl, naphthyl, indenyl, tetrahydronaphthyl, indanyl, anthracenyl, fluorenyl and the like. The aryl group can be unsubstituted or substituted with one, two, or three substituents independently selected from lower alkyl, halo, cyano, nitro, haloalkyl, hydroxy, alkoxy, carboxy, carboxyalkyl, carboxamide, mercapto, sulfhydryl, amino, alkylamino, dialkylamino, sulfonyl, sulfonamido, aryl and heteroaryl.

The term heterocycle or heterocyclic ring is defined by all non-aromatic, heterocyclic rings of 3-7 atoms containing 1-3 heteroatoms selected from N, O and S, such as oxirane, oxetane, tetrahydrofuran, tetrahydropyran, pyrrolidine, piperidine, piperazine, tetrahydropyridine, tetrahydropyrimidine, tetrahydrothiophene, tetrahydrothiopyran, morpholine, hydantoin, valerolactam, pyrrolidinone, and the like.

The term heterocyclic acidic functional group is intended to include groups such as, pyrrole, imidazole, triazole, tetrazole, and the like.

Heteroaryl refers to 5- or 10-membered single or benzofused aromatic rings consisting of 1 to 3 heteroatoms independently selected from the group consisting of -O-, -S, and -N=, provided that the rings do not possess adjacent oxygen and/or sulfur atoms. The heteroaryl group can be unsubstituted or substituted with one, two, or three substituents independently selected from lower alkyl, halo, cyano, nitro, haloalkyl, hydroxy, alkoxy, carboxy, carboxyalkyl, carboxamide, sulfhydryl, amino, alkylamino and dialkylamino.

N-oxides can form on a tertiary nitrogen present in an R substituent, or on =N-in a heteroaryl ring substituent and are included in the compounds of formula I.

The preferred groups described below refer to the formula I that directly precedes these preferred groups.

In a preferred group of compounds of formula I, A is selected from the group consisting of: 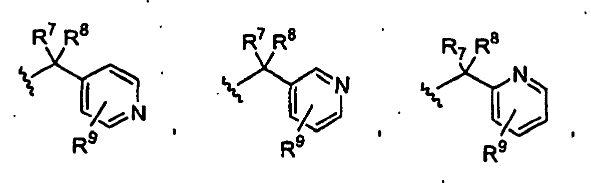 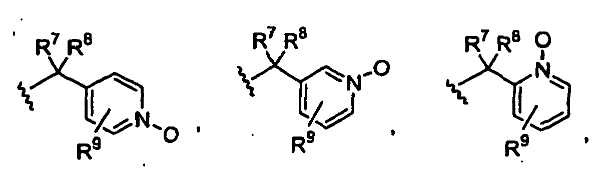 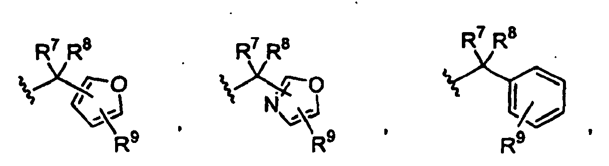 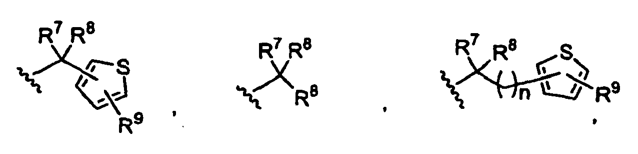 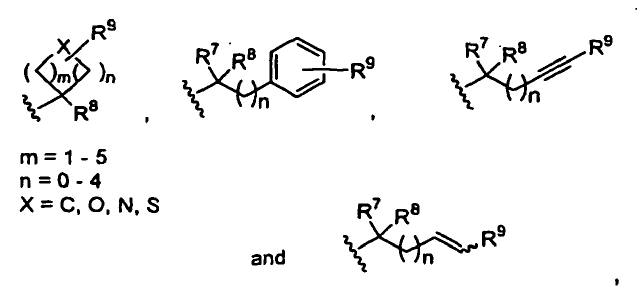 wherein,
R⁷ and R⁸ are the same or different and are independently selected from alkyl and cycloalkyl such as, for example, methyl, ethyl, t-butyl, isopropyl and cyclohexyl with methyl, ethyl, t-butyl and isopropyl being most preferred and,
R⁹ is selected from one or more moieties selected from the group halogen (e.g. Bromine, Fluorine or Chlorine), CH₃, CF₃, cyano, -OCH₃, and NO₂, and
n=0-6.

Preferrably, B is 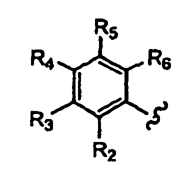 wherein
R² is OH;
R³ is C(O) NR¹³R¹⁴;
R⁴ is selected from the group consisting of H, NO₂, cyano and CF₃;
R⁵ is selected from the group consisting of H, CF₃, NO₂, halogen and cyano; and
R⁶ is selected from the group consisting of H, alkyl and CF₃.
R¹³ and R¹⁴ are independently selected from methyl, ethyl and isopropyl or when taken together R¹³ and R¹⁴ form a 3 to 7 membered heterocyclic ring containing one to two heteroatoms selected from O, S and N, optionally substituted with one or more R⁹ groups.

More preferably, A is selected from: 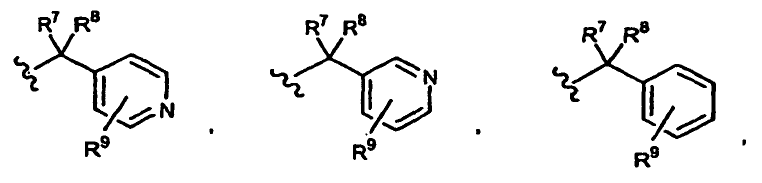 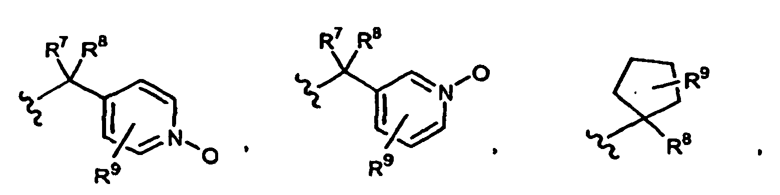 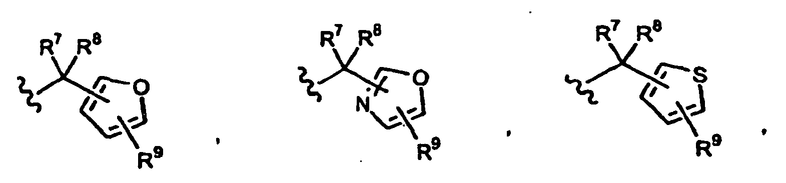 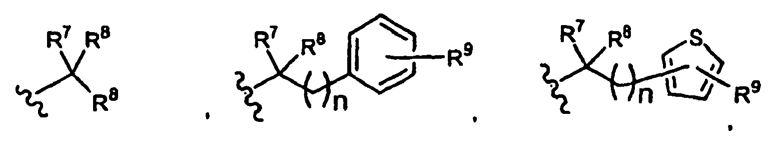 and

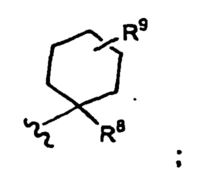

Most preferably, A is selected from:

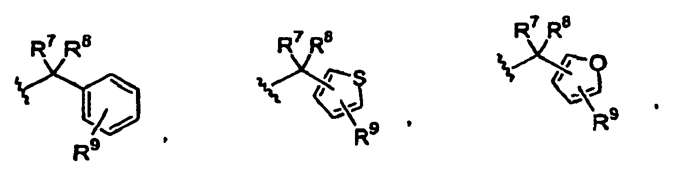

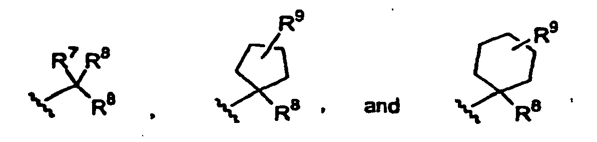

Most Preferably, for compounds of the present invention,
R² is -OH;
R³ is CONR¹³R¹⁴;
R⁴ is H;
R⁵ is H or cyano;
R⁶ is H or alkyl;
R⁷, R⁸ are independently selected from H, methyl, ethyl, isopropyl and t-butyl;
R¹³ and R¹⁴ are independently selected from methyl and ethyl.

Additional representative embodiments of the novel compounds of this invention are described below. The embodiments have been numbered for purposes of reference thereto.

Embodiment No. 47 is directed to compounds of formula IA wherein B is selected from the group consisting of:
(1) 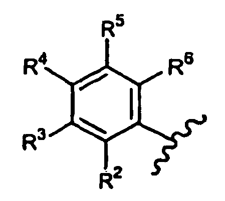 provided that R³ for this group is -C(O)NR¹³R¹⁴;
(2) 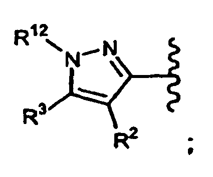
(3) 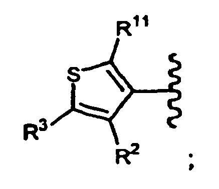
(4) 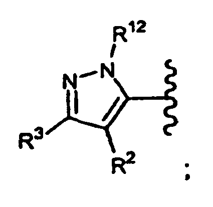
(5) 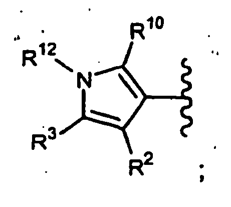
(6) 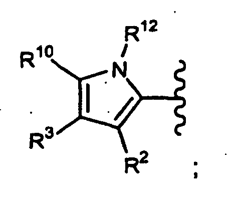
(7) 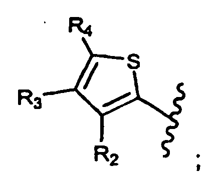
(8) 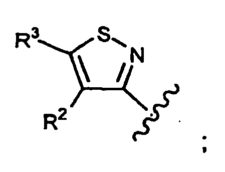
(9) 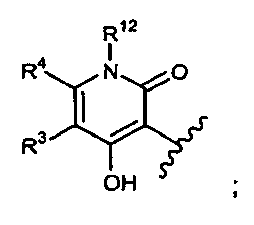
(10) 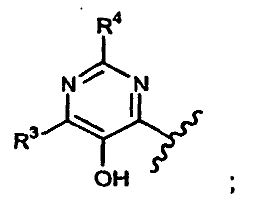
(12) 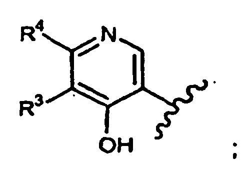 and
(13) 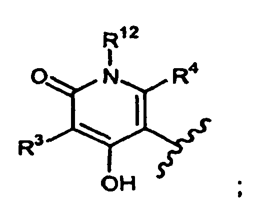
wherein all other substituents are as defined for formula IA.

Embodiment No. 48 is directed to compounds of formula IA wherein B is selected from the group consisting of:
(1) 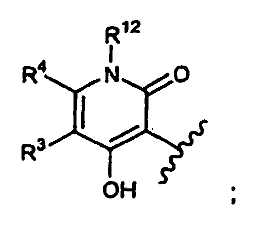
(2) 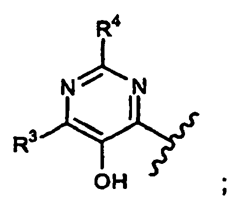
(3) 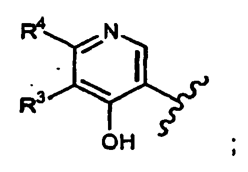 and
(4) 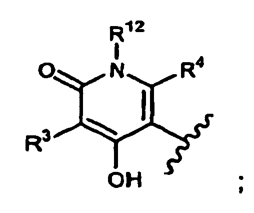
wherein all substituents are as defined for formula IA.

Embodiment No. 49 is directed to compounds of formula IA wherein B is selected from the group consisting of: 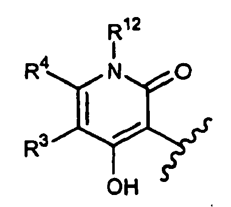 wherein all substituents are as defined for formula IA.

Embodiment No. 50 is directed to compounds of formula IA wherein, B is selected from the group consisting of: 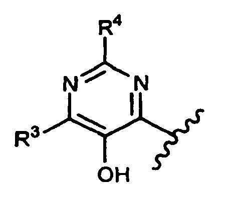 wherein all substituents are as defined for formula IA.

Embodiment No. 51 is directed to compounds of formula IA wherein B is selected from the group consisting of: 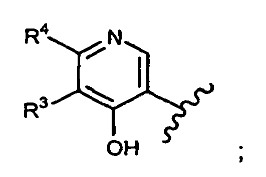 wherein all substituents are as defined for formula IA.

Embodiment No. 52 is directed to compounds of formula IA wherein B is selected from the group consisting of: 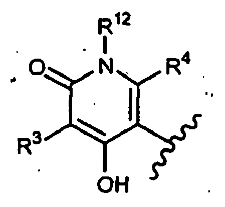 wherein all substituents are as defined for formula IA.

Embodiment No. 53 is directed to compounds of formula IA wherein B is selected from the group consisting of: 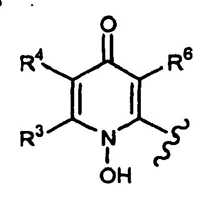 wherein all substituents are as defined for formula IA.

Embodiment No. 54 is directed to any one of the Embodiment Nos. 48 to 53 wherein the compound of formula IA is a pharmaceutically acceptable salt.

Embodiment No. 55 is directed to any one of the Embodiment Nos. 48 to 53 wherein the compound of formula IA is a sodium salt.

Embodiment No. 56 is directed to any one of the Embodiment Nos. 48 to 53 wherein the compound of formula IA is a calcium salt.

Embodiment No. 57 is directed to a pharmaceutical composition comprising at least one (e.g., 1 to 3, usually 1) compound of formula IA as described in any one of the Embodiment Nos. 48 to 53 in combination with a pharmaceutically acceptable carrier.

Embodiment No. 58 is directed to a method of treating any one of the diseases described above comprising administering to a patient in need of such treatment an effective amount (e.g., a therapeutically effective amount) of a compound of formula IA as described in any one of the Embodiment Nos. 48 to 53. The diseases referred to in this embodiment are those described in the methods of treatment using compounds of formula I.

Embodiment No. 59 is directed to the use of a compound of formula IA as described in any one of the Embodiment Nos. 48 to 53 for the manufacture of a medicament for the treatment any one of the diseases described above. The diseases referred in this embodiment are those described in the methods of treatment using compounds of formula I.

## Claims

1. A compound of the formula 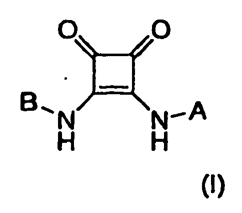 or a pharmaceutically acceptable salt or solvate thereof wherein
A is selected from the group consisting of 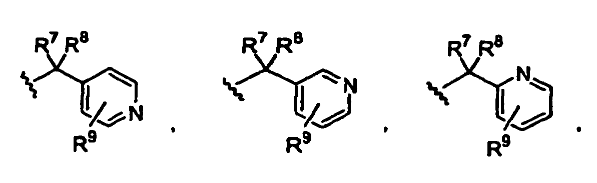 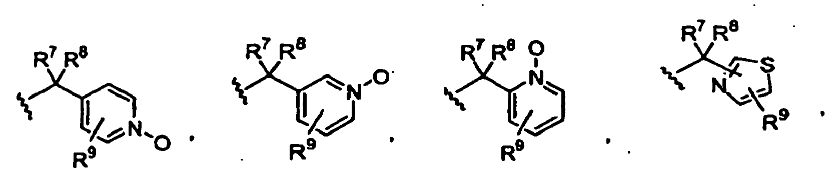 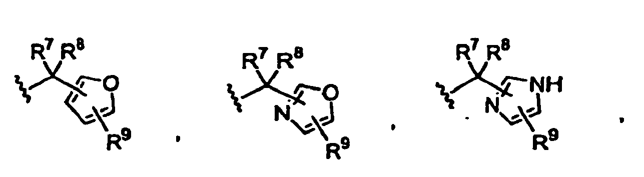 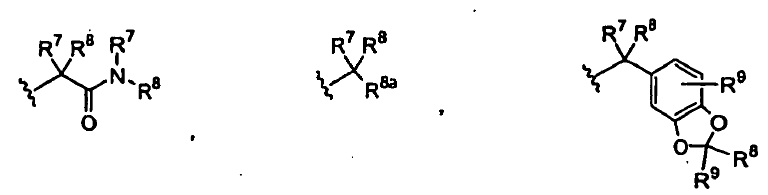 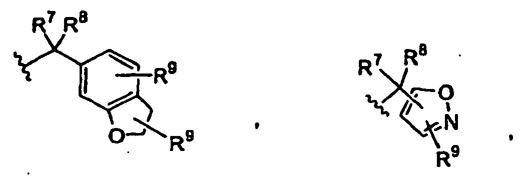 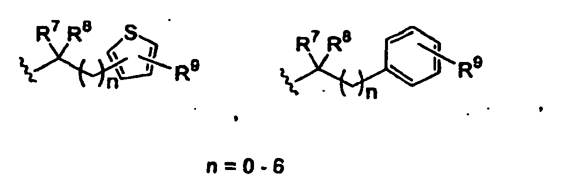 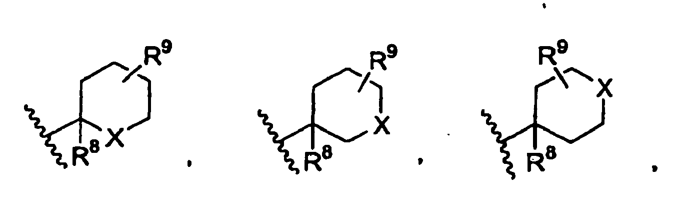 X=-O-, -NH-, -S- 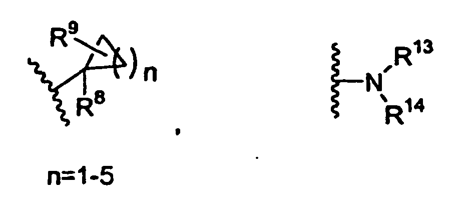 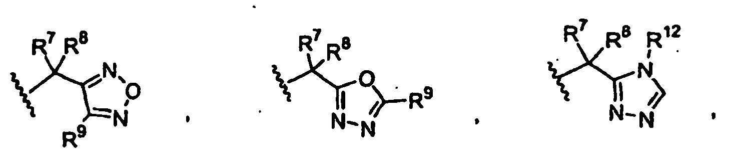 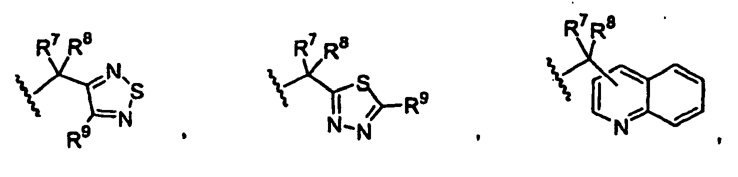 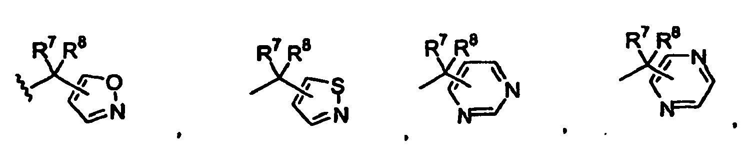 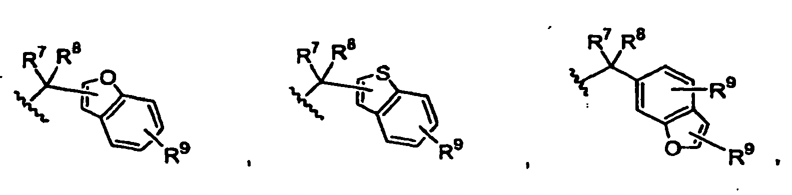 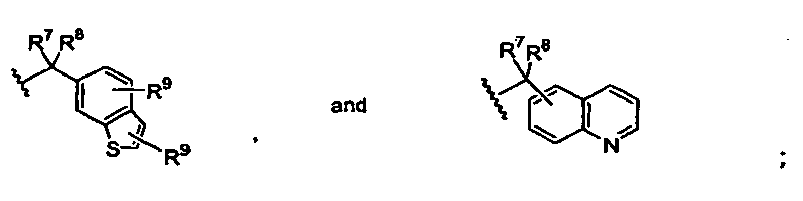 B is selected from the group consisting of 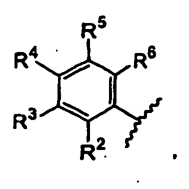 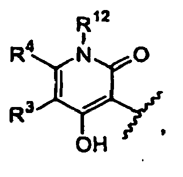 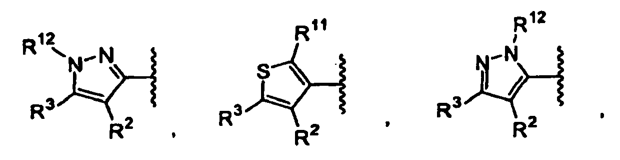 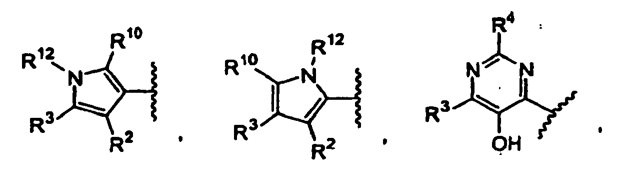 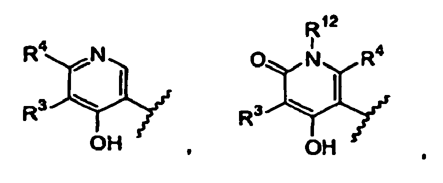 and 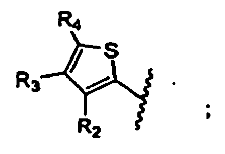 R² is OH;
R³ is selected from the group consisting of -C(O)NHR¹⁷, -C(O)NR¹³R¹⁴ and -C(O)NR¹³OR¹⁴;
R⁴ is selected from the group consisting of hydrogen, cyano, halogen, alkyl, alkoxy, -OH, -CF₃, -OCF₃, -NO₂, -C(O)R¹³, -C(O)OR¹³, -C(O)NHR¹⁷, -C(O)NR¹³R¹⁴, -SO₍₁₎NR¹³R¹⁴, -SO₍ₜ₎R¹³, -C(O)NR¹³OR¹⁴, 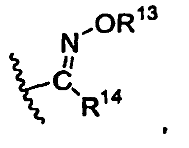 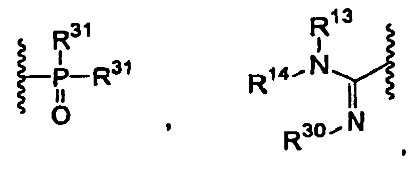 -NHC(O)R¹⁷, unsubstituted or substituted aryl and unsubstituted or substituted heteroaryl,
wherein the substituents on the substituted R⁴ groups are the same or different and independently selected from 1-6 R⁹ groups;
R⁵ and R⁶ are the same or different and are independently selected from the group consisting of hydrogen, halogen, alkyl, alkoxy, -CF₃, -OCF₃, -NO₂, -C(O)R¹³, -C(O)OR¹³, -C(O)NR¹³R¹⁴, -SO₍ₜ₎NR¹³R¹⁴, -C(O)NR¹³OR¹⁴, cyano, an unsubstituted or substituted aryl and unsubstituted or substituted heteroaryl group,
wherein the substituents on the substituted R⁵ and R⁶ groups are the same or different and independently selected from 1-6 R⁹ groups;
R⁷ and R⁸ are the same or different and are independently selected from the group consisting of H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, -CO₂R¹³, -CONR¹³R¹⁴, fluoroalkyl, alkynyl, alkenyl and cycloalkenyl,
wherein the substituents on the substituted R⁷ and R⁸ groups are selected from the group consisting of
a) H,
b) halogen,
c) -CF₃,
d) -COR¹³,
e) -OR¹³,
f) -NR¹³R¹⁴,
g) -NO₂,
h) -CN,
i) -SO₂OR¹³,
j) -Si(alkyl)₃,
k) -Si(aryl)₃,
l) -(R¹³)₂R¹⁴Si,
m) -CO₂R¹³,
n) -C(O)NR¹³R¹⁴,
o) -SO₂NR¹³R¹⁴,
p) -SO₂R¹³,
q) -O(C=O)R¹³,
r) -O(C=O)NR¹³R¹⁴,
s) -NR¹³C(O)R¹⁴, and
t) -NR¹³CO₂R¹⁴;
R^{8a} is selected from the group consisting of hydrogen, alkyl, cycloalkyl and cycloalkylalkyl;
R⁹ is independently selected from 1-6 of the group consisting of:
a) -R¹³,
b) halogen,
c) -CF₃,
d) -COR¹³,
e) -OR¹³,
f) -NR¹³R¹⁴,
g) -NO₂,
h) -CN,
i) -SO₂R¹³,
j) -SO₂NR¹³R¹⁴,
k) -NR¹³COR¹⁴,
l) -CONR¹³R¹⁴,
m) -NR¹³CO₂R¹⁴,
n) -CO₂R¹³, and
o) 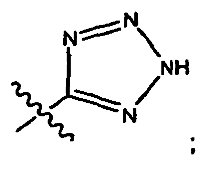
R¹⁰ and R¹¹ are the same or different and are independently selected from the group consisting of hydrogen, halogen, -CF₃, -OCF₃, -NR¹³R¹⁴, -NR¹³C(O)NR¹³R¹⁴, -OH, -C(O)OR¹³, -SH, -SO₍ₜ₎NR³R¹⁴, -SO₂R¹³, -NHC(O)R¹³,-NHSO₂NR¹³R¹⁴, -NHSO₂R¹³, -C(O)NR¹³R¹⁴, -C(O)NR¹³OR¹⁴, -OC(O)R¹³ and cyano;
R¹² is hydrogen, -OC(O)R¹³, or an unsubstituted or substituted aryl, unsubstituted or substituted heteroaryl, unsubstituted or substituted arylalkyl, unsubstituted or substituted cycloalkyl, unsubstituted or substituted alkyl, unsubstituted or substituted cycloalkylalkyl or unsubstituted or substituted heteroarylalkyl group,
wherein the substituents on the substituted R¹² groups are the same or different and independently selected from 1-6 R⁹ groups;
R¹³ and R¹⁴ are the same or different and are independently selected from the group consisting of H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, and unsubstituted or substituted fluoroalkyl, or
R¹³ and R¹⁴ can be taken together when both are attached to a nitrogen atom to form an unsubstituted or substituted 3 to 7 membered heterocylic ring containing one to two heteroatoms selected from oxygen, sulfur and nitrogen,
wherein the substitutents on the substituted R¹³ and R¹⁴ groups are the same or different and independently selected from 1-6 of H, alkyl, aryl, arylalkyl, fluroalkyl, cycloalkyl, cycloalkylalkyl, heteroaryl, heteroarylalkyl, amino, -C(O)OR¹⁵, -C(O)NR¹⁵R¹⁶, -S(O)ₜNR¹⁵R¹⁶, -C(O)R¹⁵, -SO₂R¹⁵, -NHC(O)NR¹³R¹⁴ and halogen;
R¹⁵ and R¹⁶ are the same or different and are independently selected from the group consisting of H, alkyl, aryl, arylalkyl, cycloalkyl and heteroaryl;
R¹⁷ is -SO₂ alkyl, -SO₂ aryl, -SO₂ cycloalkyl or -SO₂heteroaryl;
R³⁰ is alkyl, cycloalkyl, -CN, -NO₂, or -SO₂R¹⁵;
R³¹ are the same or different and are independently selected from the group consisting of unsubstituted or substituted alkyl, unsubstituted or substituted aryl, unsubstituted or substituted heteroaryl and unsubstituted or substituted cycloalkyl;
wherein the substituents on the substituted R³¹ groups are the same or different and independently selected from 1-6 R⁹ groups; and
t is 0,1 or 2.

2. The compound of claim 1, wherein A is selected from the group consisting of: 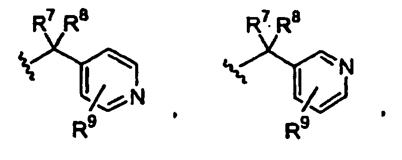 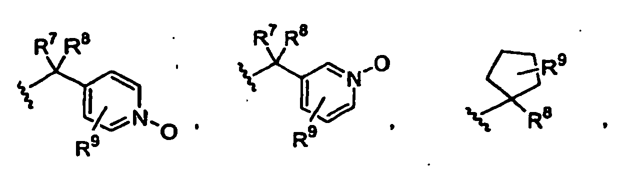 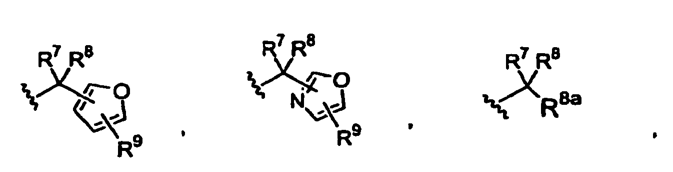 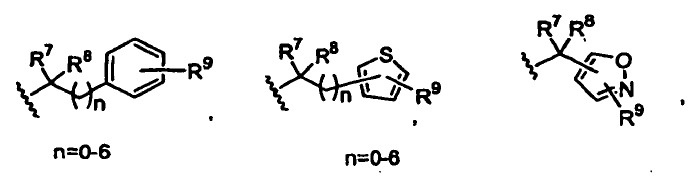 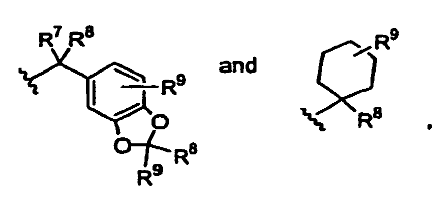 wherein,
R⁷ is H, fluoroalkyl, alkyl or cycloalkyl;
R⁸ is H, alkyl, -CF₂CH₃ or -CF₃;
R⁹ is H, F, Cl, Br, alkyl or -CF₃.

3. The compound of claim 1, wherein A is selected from the group consisting of 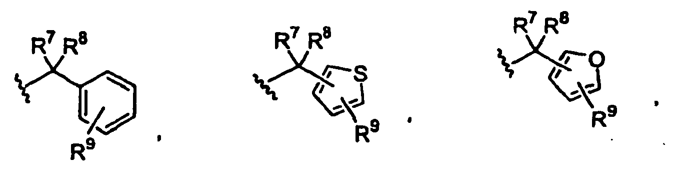 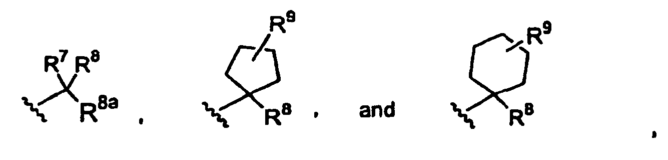 wherein.
R⁷ is H, -CF₃, -CF₂CH₃, methyl, ethyl, isopropyl, cyclopropyl or t-butyl;
R⁸ is H;
R⁹ is H, F, Cl, Br, alkyl or -CF₃, and
B is: 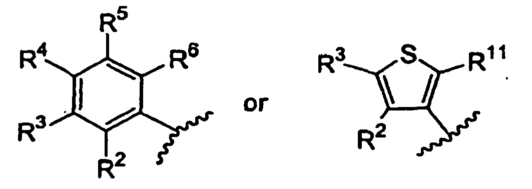 wherein:
R² is OH;
R³ is -C(O) NR¹³R¹⁴;
R⁴ is H, -NO₂, cyano, -CH₃ or -CF₃;
R⁵ is H, -CF₃, -NO₂, halogen or cyano; and
R⁶ is H, alkyl or -CF₃;
R¹¹ is H, halogen or alkyl; and
R¹³ and R¹⁴ are the same or different and are independently methyl, ethyl or isopropyl; or
R¹³ and R¹⁴ when taken together with the nitrogen they are attached to in the groups -NR¹³R¹⁴, -C(O)NR¹³R¹⁴, -SO₂NR¹³R¹⁴, -OC(O)NR¹³R¹⁴, -CONR¹³R¹⁴, -NR¹³C(Q)NR¹³R¹⁴, -SOₜNR¹³R¹⁴, -NHSO₂NR¹³R¹⁴ preferably form an unsubstituted or substituted 3 to 7 membered, saturated heterocyclic ring optionally containing one additional heteroatom selected from O, S or NR¹⁸ wherein R¹⁸ is selected from H, alkyl, aryl, heteroaryl, -C(O)R¹⁹, -SO₂R¹⁹ and -C(O)NR¹⁹R²⁰, wherein R¹⁹ and R²⁰ are the same or different and each is independently selected from alkyl, aryl and heteroaryl, wherein the substituents on the substituted cyclized R¹³ and R¹⁴ groups are the same or different and independently selected from 1 to 3 of alkyl, aryl, hydroxy, hydroxyalkyl, alkoxy, alkoxyalkyl, arylalkyl, fluoroalkyl, cycloalkyl, cycloalkylalkyl, heteroaryl, heteroarylalkyl, amino, -C(O)OR¹⁵, -C(O)NR¹⁵R¹⁶, -SOₜNR¹⁵R¹⁶, -C(O)R¹⁵, -SO₂R¹⁵, -NHC(O)NR¹⁵R¹⁶ and halogen, and wherein R¹⁵ and R¹⁶ are the same or different and are independently selected from the group consisting of H, alkyl, aryl, arylalkyl, cycloalkyl and heteroaryl;

4. The compound of claim 1, wherein
A is selected from the group consisting of 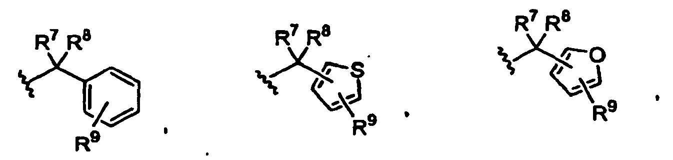 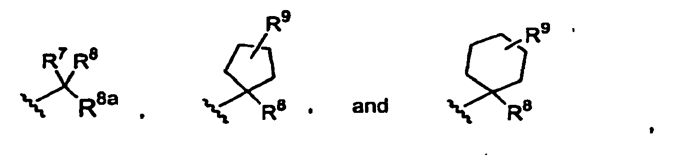 and B is: 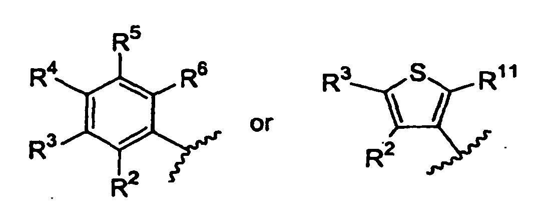 wherein:
R² is OH;
R³ is -C(O)NR¹³R¹⁴:
R⁴ is H, -NO₂, -CF₃, -CH₃ or cyano,
R⁵ is H, halogen, -NO₂, cyano or -CF₃;
R⁶ is H, -CF₃ or alkyl,
R⁷ is H, -CF₃, -CF₂CH₃, methyl, ethyl, isopropyl, cyclopropyl or t-butyl;
R⁸ is H;
R⁹ is H, F, Cl, Br, alkyl, cycloalkyl or -CF₃;
R¹¹ is H, halogen or alkyl; and
R¹³ and R¹⁴ are independently methyl or ethyl.

5. The compound of claim 1, wherein
A is selected from the group consisting of 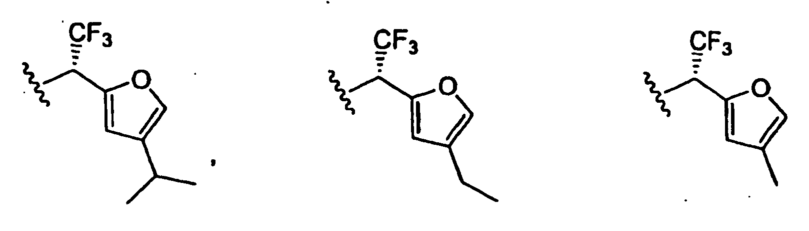 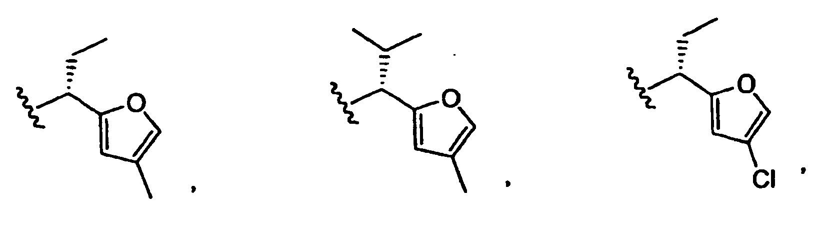 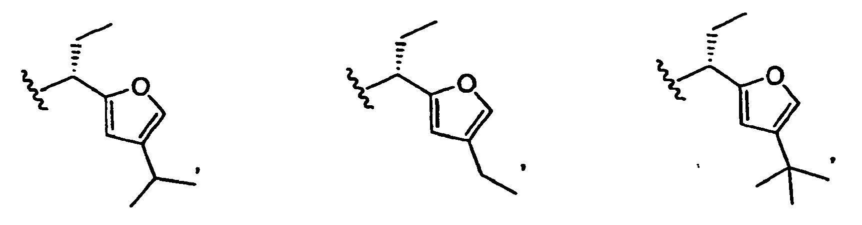 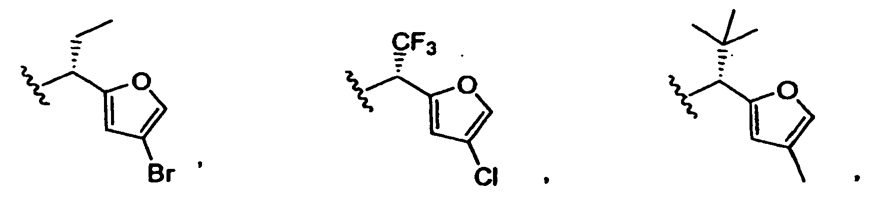 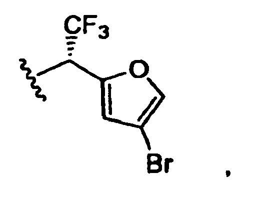 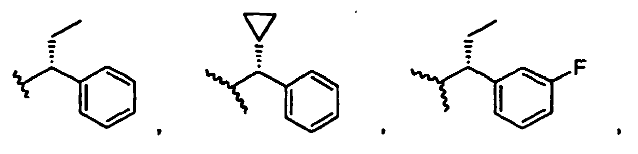 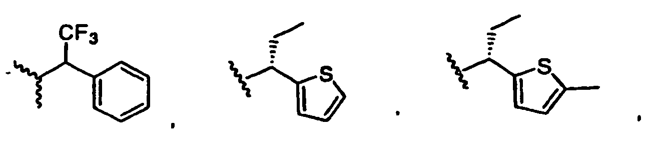 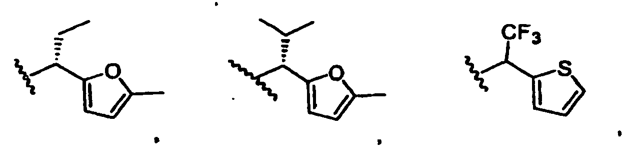 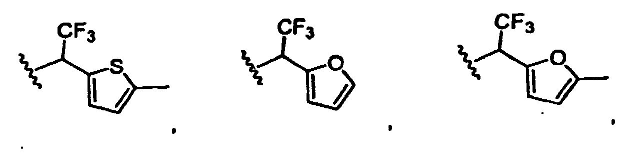 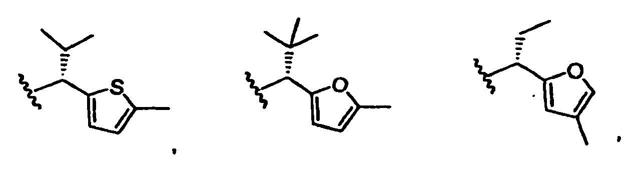 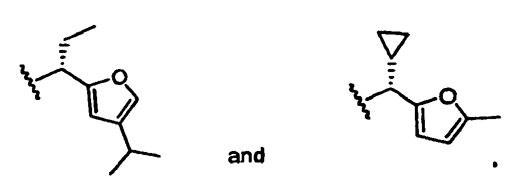 and B is 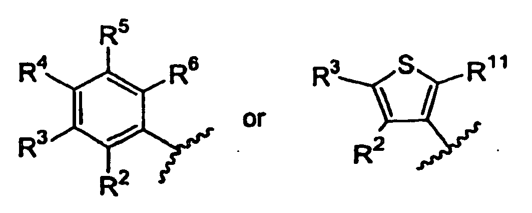 wherein,
R² is -OH;
R³ is -CONR¹³R¹⁴;
R⁴ is H, -CH₃ or -CF₃;
R⁵ is H or cyano;
R⁶ is H, -CH₃ or -CF₃;
R¹¹ is H, and
R¹³ and R¹⁴ are methyl.

6. A compound of the formula 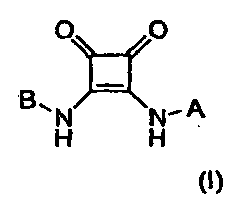 or a pharmaceutically acceptable salt or solvate of the compound wherein
A is selected from: 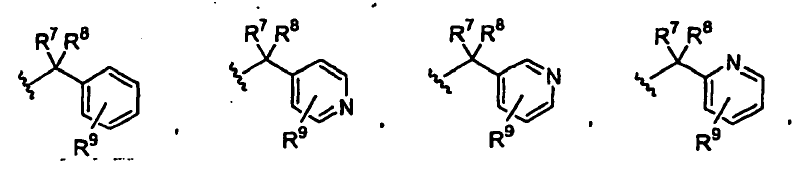 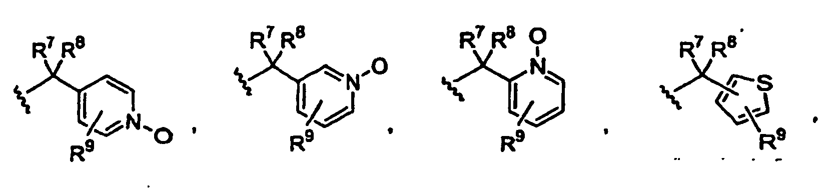 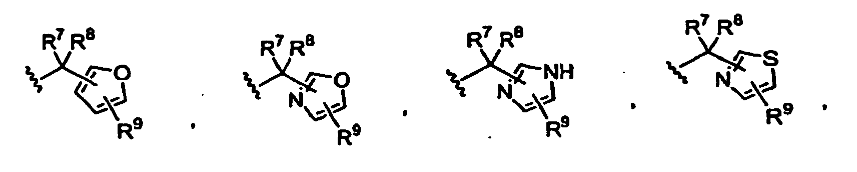 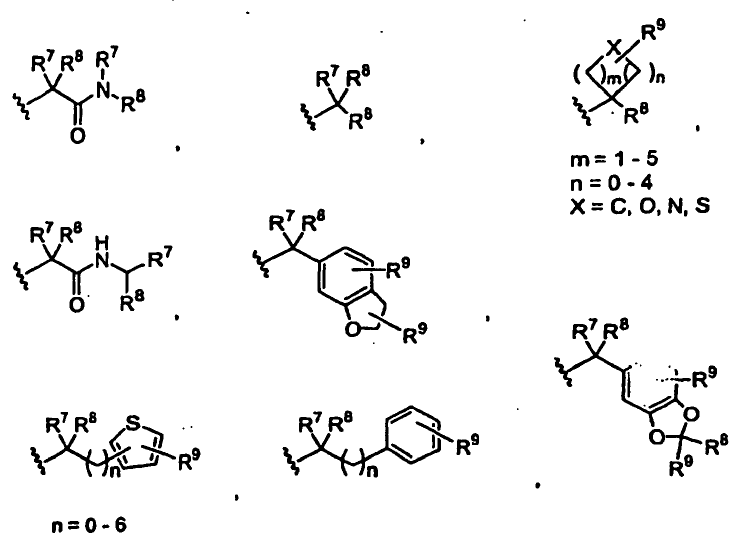 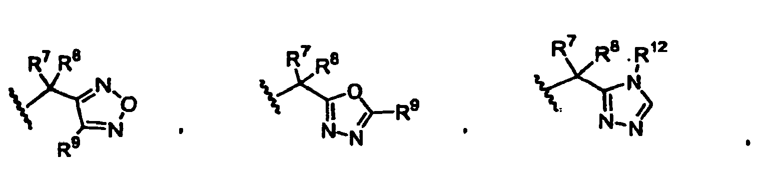 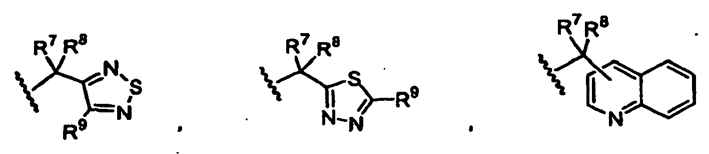 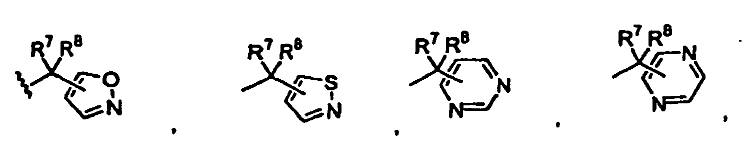 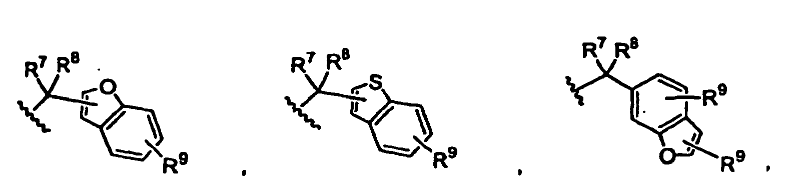 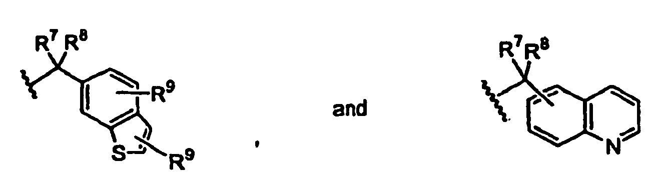 wherein,
R⁷ and R⁸ are the same or different and are independently selected from the group consisting of H; optionally substituted or unsubstituted alkyl, aryl, heteroaryl, arylalkyl, heteroarylalkyl, cycloalkyl, cycloalkylalkyl, CO₂R¹³, CONR¹³R¹⁴, fluoroalkyl, alkynyl, alkenyl, alkynylalkyl, alkenylalkyl, and cycloalkenyl,
wherein said substituents on said substituted groups are selected from the group consisting of:
a) cyano;
b) CO₂R⁷;
c) CONR⁷R⁸;
d) SO₂NR⁷R⁸,
e) SO₂R⁷;
f) NO₂;
g) CF₃;
h) -OR⁷;
i) -NR⁷R⁸;
j) -O(C=O)R ⁷;
k) -O(C=O)NR⁷R⁸, and
l) halogen;
R⁹ is selected from one or more of the groups consisting of:
a) R⁷;
b) R⁸;
c) halogen;
d) -CF₃;
e) -COR⁷;
f) -OR⁷;
g) -NR⁷R⁸;
h) -NO₂;
i) -CN;
j) -SO₂R⁷;
k) -SO₂NR⁷R⁸;
l) -NR⁷COR⁸;
m) -CONR⁷R⁸;
n) -NR⁷CO₂R⁸;
o) CO₂R⁷, and
p) 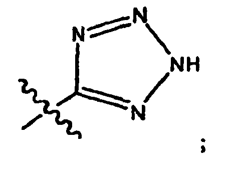
B is an optionally substituted aryl or heteroaryl group selected from: 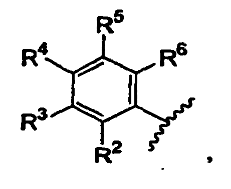 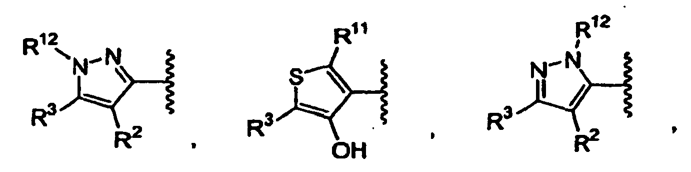 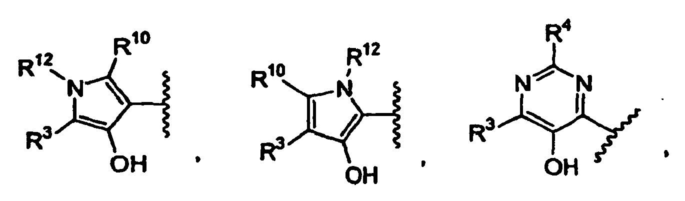 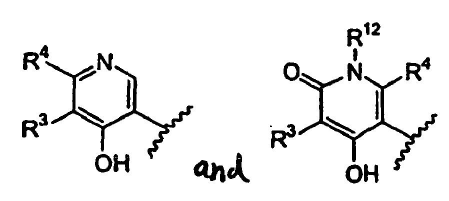 wherein,
R² is OH;
R³ is C(O)NR¹³R¹⁴ or C(O)NR¹³OR¹⁴;
R⁴ is hydrogen, halogen, alkyl, alkoxy, OH, CF₃, OCF₃. NO₂, C(O)R¹³, C(O)OR¹³, C(O)NR¹³R¹⁴, SO₍ₜ₎NR¹³R¹⁴, SO₍ₜ₎R¹³, C(O)NR¹³OR¹⁴, 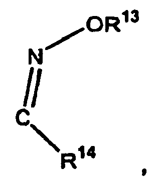 cyano, optionally substituted aryl or heteroaryl,
wherein the substituents on the optionally substituted groups may be selected from one or more R⁹ groups;
R⁵ and R⁶ independently represent hydrogen, halogen, alkyl, alkoxy, CF₃, OCF₃, NO₂, C(O)R¹³, C(O)OR¹³, C(O)NR¹³R¹⁴, SO₍ₜ₎NR¹³R¹⁴, C(O)NR¹³OR¹⁴, cyano, or an optionally substituted aryl or heteroaryl group,
wherein the substituents on the optionally substituted groups may be selected from one or more R⁹ groups;
R¹⁰ and R¹¹ independently represent hydrogen, halogen, CF₃. OCF₃, NR¹³R¹⁴, NR¹³C(O)NR¹³R¹⁴, OH, C(O)OR¹³, SH, SO₍ₜ₎NR¹³R¹⁴,SO₂R¹³, NHC(O)R¹³, NHSO₂NR¹³R¹⁴, NHSO₂R¹³, C(O)NR¹³R¹⁴, C(O)NR¹³OR¹⁴, OC(O)R¹³ or cyano.
R¹² is hydrogen, OC(O)R¹³, or an optionally substituted aryl, heteroaryl, arylalkyl, cycloalkyl, alkyl, cycloalkylalkyl or heteroarylalkyl group;
R¹³ and R¹⁴ are the same or different and are independently selected from the group consisting of H; optionally substituted or unsubstituted alkyl, aryl, heteroaryl, arylalkyl, heteroarylalkyl, cycloalkyl, cycloalkylalkyl, and fluoroalkyl, or
R¹³ and R¹⁴ when taken together form an optionally substituted 3 to 7 membered heterocyclic ring containing one to two heteroatoms selected from O, S and N, and
wherein the substituents on the optionally substituted groups may be selected from one or more R⁹ groups, and
t is 1 or 2.

7. A compound of formula IA 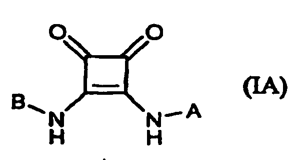 or a pharmaceutically acceptable salt or solvate thereof, wherein:
A is selected from the group consisting of:
(1) 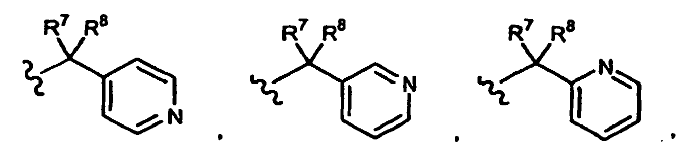 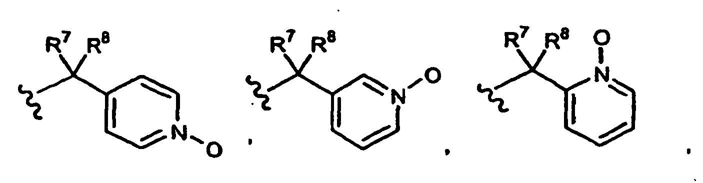 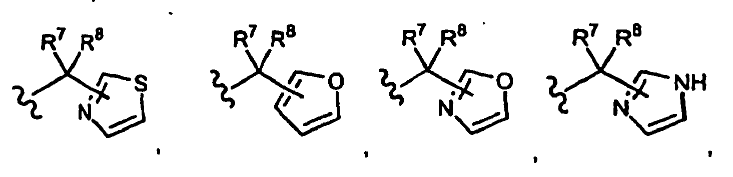 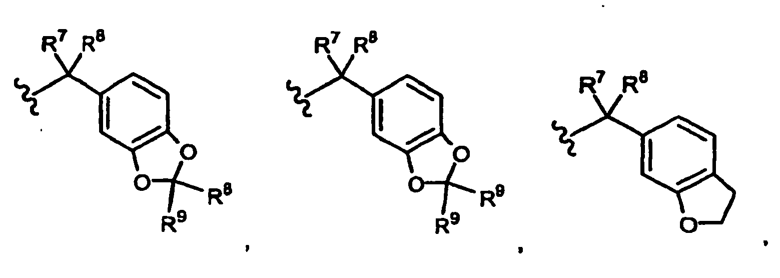 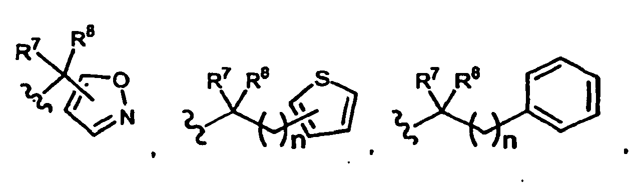 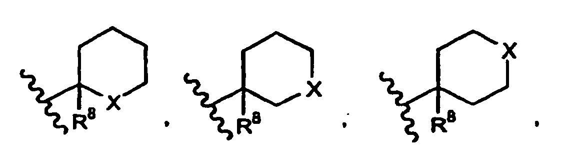 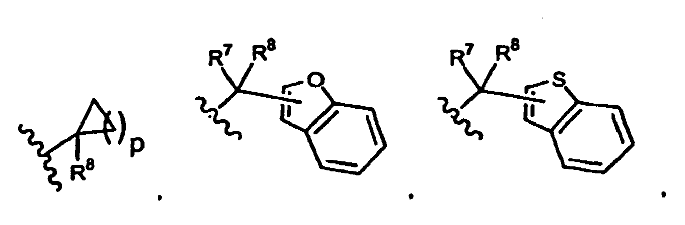 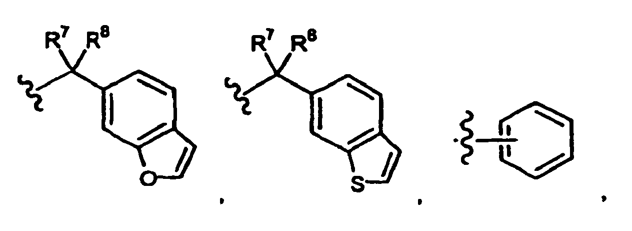 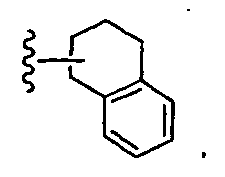 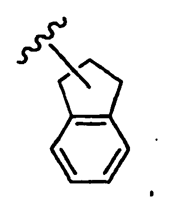 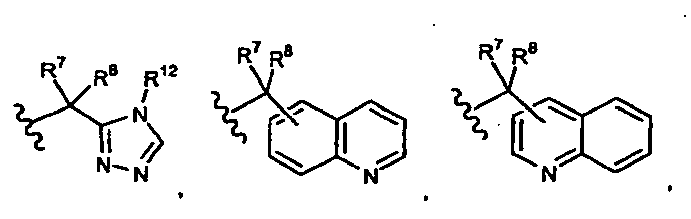 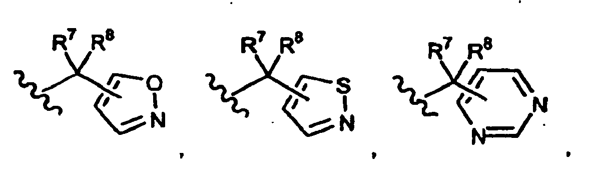 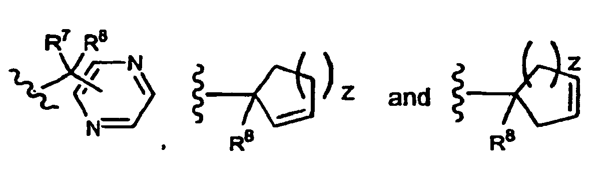
(2) 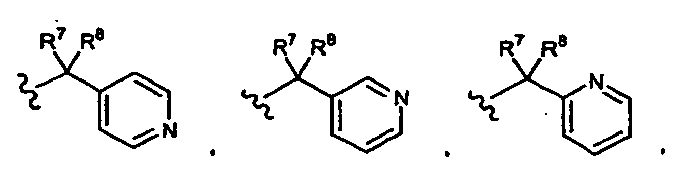 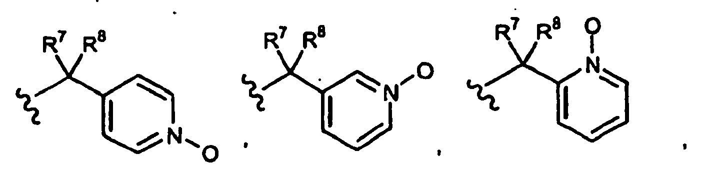 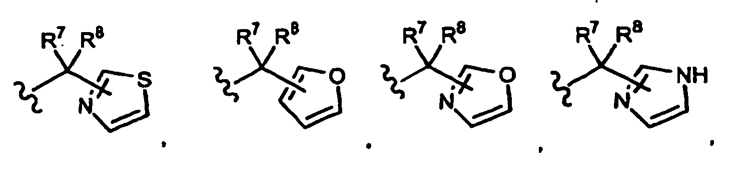 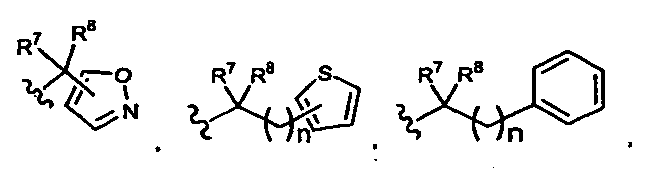 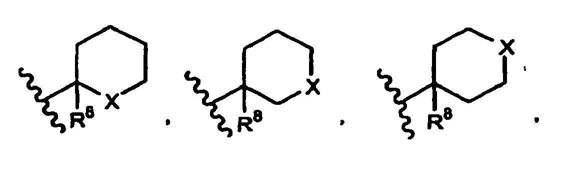 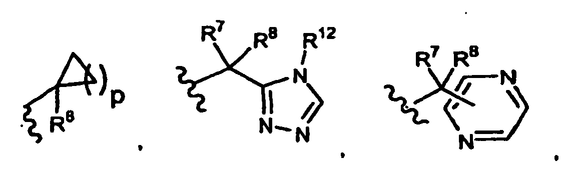 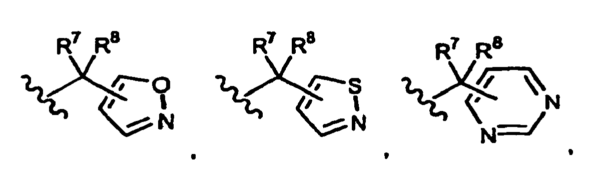 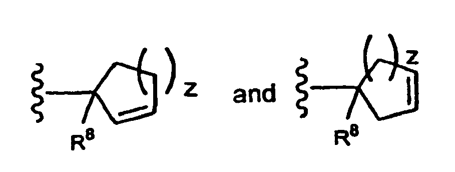 wherein the above rings of said A groups are substituted with 1 to 6 substituents each independently selected from the group consisting of: R⁹ groups;
(3) 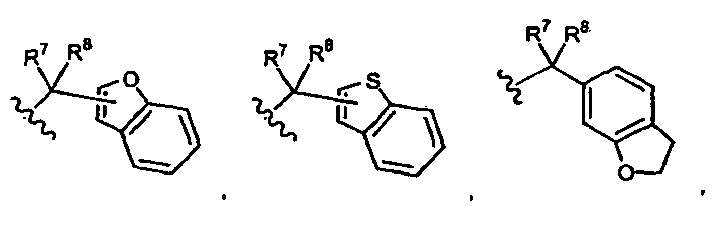 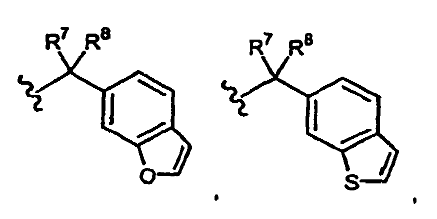 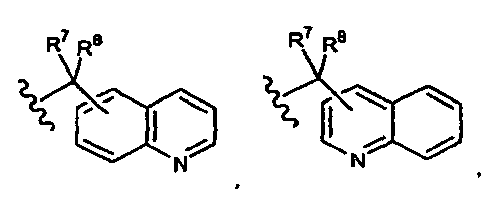 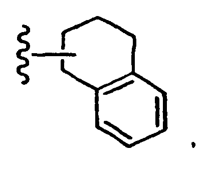 and 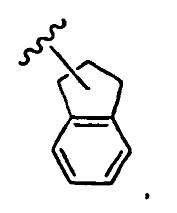 wherein one or both of the above rings of said A groups are substituted with 1 to 6 substituents each independently selected from the group consisting of: R⁹ groups;
(4) 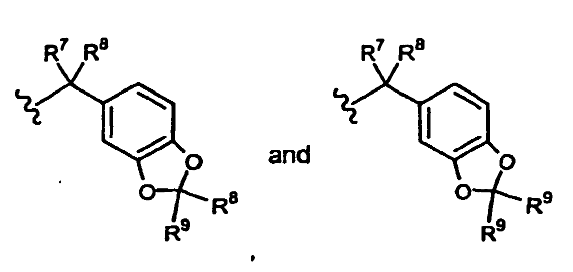 wherein the above phenyl rings of said A groups are substituted with 1 to 3 substituents each independently selected from the group consisting of: R⁹ groups; and
(5) 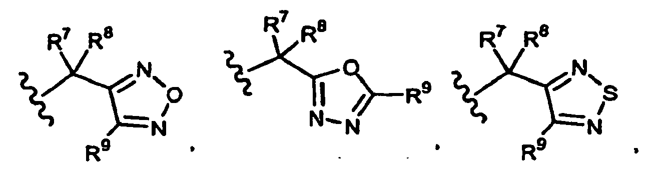 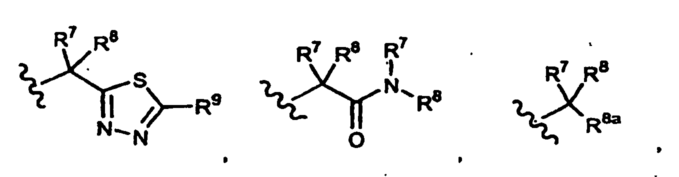 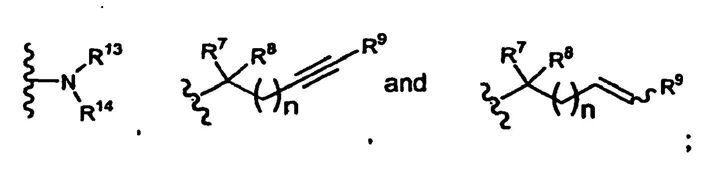
B is selected from the group consisting of 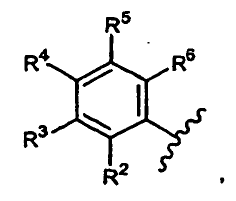 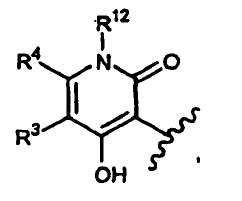 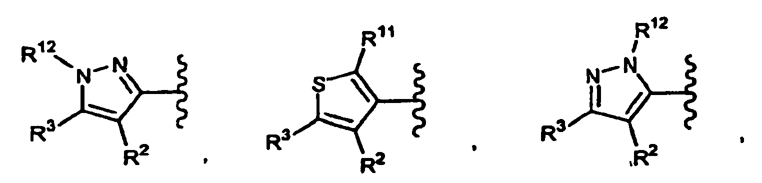 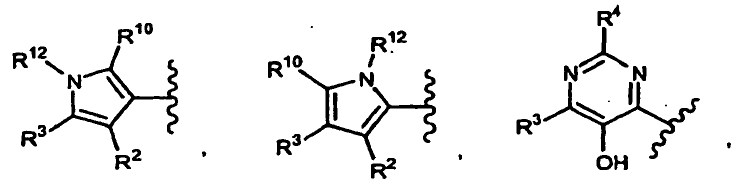 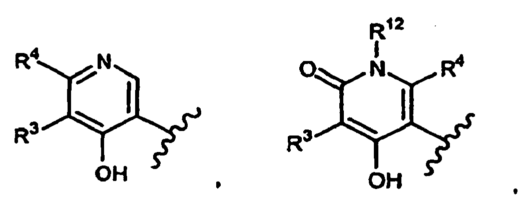 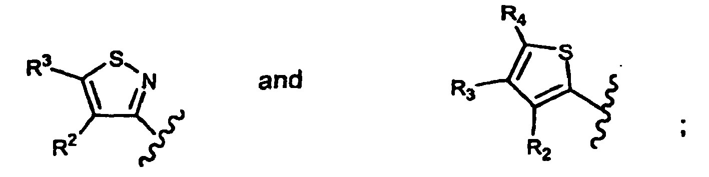 n is 0 to 6; p is 1 to 5; X is O, NH, or S; Z is 1 to 3;
R² is OH;
R³ is selected from the group consisting of: -C(O)NHR¹⁷, -C(O)NR¹³R¹⁴ and C(O)NR¹³OR¹⁴;
R⁴ is selected from the group consisting of: hydrogen, cyano, halogen, alkyl, alkoxy, -OH, -CF₃, -OCF₃, -NO₂, -C(O)R¹³, -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, unsubstituted or substituted aryl, unsubstituted or substituted heteroaryl, 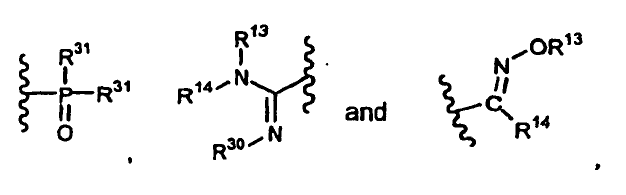 wherein there are 1 to 6 substituents on said substituted aryl group and each substitutent is independently selected from the group consisting of: R⁹ groups; and wherein there are 1 to 6 substituents on said substituted heteroaryl group and each substitutent is independently selected from the group consisting of: R⁹ groups;
each R⁵ and R⁶ are the same or different and are independently selected from the group consisting of hydrogen, halogen, alkyl, alkoxy, -CF₃, -OCF₃, -NO₂, -C(O)R¹³, -C(O)OR¹³, -C(O)NR¹³R¹⁴, -SO₍ₜ₎NR¹³R¹⁴, -C(O)NR¹³OR¹⁴, cyano, unsubstituted or substituted aryl, and unsubstituted or substituted heteroaryl group; wherein there are 1 to 6 substituents on said substituted aryl group and each substitutent is independently selected from the group consisting of: R⁹ groups; and wherein there are 1 to 6 substituents on said substituted heteroaryl group and each substitutent is independently selected from the group consisting of: R⁹ groups;
each R⁷ and R⁸ is independently selected from the group consisting of: H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, -CO₂R¹³, -CONR¹³R¹⁴, alkynyl, alkenyl, and cycloalkenyl; and wherein there are one or more substituents on said substituted R⁷ and R⁸ groups, wherein each substitutent is independently selected from the group consisting of:
a) halogen,
b) -CF₃,
c) -COR¹³,
d) -OR¹³,
e) -NR¹³R¹⁴,
f) -NO₂,
g) -CN,
h) -SO₂OR¹³,
i) -Si(alkyl)₃, wherein each alkyl is independently selected,
j) -Si(aryl)₃, wherein each alkyl is independently selected,
k) -(R¹³)₂R¹⁴Si, wherein each R¹³ is independently selected,
l) -CO₂R¹³,
m) -C(O)NR¹³R¹⁴,
n) -SO₂NR¹³R¹⁴,
o) -SO₂R¹³,
p) -OC(O)R¹³,
q) -OC(O)NR¹³R¹⁴,
r) -NR¹³C(O)R¹⁴, and
s) -NR¹³CO₂R¹⁴;
R^{8a} is selected from the group consisting of: hydrogen, alkyl, cycloalkyl and cycloalkylalkyl;
each R⁹ is independently selected from the group consisting of:
a) -R¹³,
b) halogen,
c) -CF₃,
d) -COR¹³,
e) -OR¹³,
f) -NR¹³R¹⁴,
g) -NO₂,
h) -CN,
i) -SO₂R¹³,
j) -SO₂NR¹³R¹⁴,
k) -NR¹³COR¹⁴,
l) -CONR¹³R¹⁴,
m) -NR¹³CO₂R¹⁴,
n) -CO₂R¹³,
o) 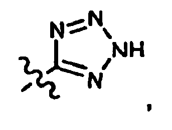
p) alkyl substituted with one or more (e.g., one) -OH groups (e.g., -(CH₂)qOH, wherein q is 1-6, usually 1 to 2, and preferably 1).
q) alkyl substituted with one or more (e.g., one) -NR¹³R¹⁴ group (e.g., -(CH₂)_{q}NR¹³R¹⁴, wherein q is 1-6, usually 1 to 2, and preferably 1), and
r) -N(R¹³)SO₂R¹⁴ (e.g., R¹³ is H and R¹⁴ is alkyl, such as methyl);
each R¹⁰ and R¹¹ is independently selected from the group consisting of hydrogen, alkyl (e.g., C₁ to C₆, such as methyl), halogen, -CF₃, -OCF₃, -NR¹³R¹⁴, -NR¹³C(O)NR¹³R¹⁴, -OH, -C(O)OR¹³, -SH, -SO₍ₜ₎NR¹³R¹⁴, -SO₂R¹³, -NHC(O)R¹³, -NHSO₂NR¹³R¹⁴, -NHSO₂R¹³, -C(O)NR¹³R¹⁴, -C(O)NR¹³OR¹⁴, -OC(O)R¹³ and cyano;
R¹² is selected from the group consisting of: hydrogen, -C(O)OR¹³, unsubstituted or substituted aryl, unsubstituted or substituted heteroaryl, unsubstituted or substituted arylalkyl, unsubstituted or substituted cycloalkyl, unsubstituted or substituted alkyl, unsubstituted or substituted cycloalkylalkyl, and unsubstituted or substituted heteroarylalkyl group; wherein there are 1 to 6 substituents on the substituted R¹² groups and each substituent is independently selected from the group consisting of: R⁹ groups;
each R¹³ and R¹⁴ is independently selected from the group consisting of: H, unsubstituted or substituted alkyl, unsubstituted or substituted aryl, unsubstituted or substituted heteroaryl, unsubstituted or substituted arylalkyl, unsubstituted or substituted heteroarylalkyl, unsubstituted or substituted cycloalkyl, unsubstituted or substituted cycloalkylalkyl, unsubstituted or substituted heterocyclic, unsubstituted or substituted fluoroalkyl, and unsubstituted or substituted heterocycloalkylalkyl; wherein there are 1 to 6 substituents on said substituted R¹³ and R¹⁴ groups and each substituent is independently selected from the group consisting of: alkyl, -CF₃, -OH, alkoxy, aryl, arylalkyl, fluroalkyl, cycloalkyl, cycloalkylalkyl, heteroaryl, heteroarylalkyl, -N(R⁴⁰)₂, -C(O)OR¹⁵, -C(O)NR¹⁵R¹⁶, -S(O)ₜNR¹⁵R¹⁶, -C(O)R¹⁵, -SO₂R¹⁵ provided that R¹⁵ is not H, halogen, and -NHC(O)NR¹⁵R¹⁶; or
R¹³ and R¹⁴ taken together with the nitrogen they are attached to in the groups -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴, -NHSO₂NR¹³R¹⁴ form an unsubstituted or substituted saturated heterocyclic ring, said ring optionally containing one additional heteroatom selected from the group consisting of: O, S and NR¹⁸; wherein there are 1 to 3 substituents on the substituted cyclized R¹³ and R¹⁴ groups and each substituent is independently selected from the group consisting of: alkyl, aryl, hydroxy, hydroxyalkyl, alkoxy, alkoxyalkyl, arylalkyl, fluoroalkyl, cycloalkyl, cycloalkylalkyl, heteroaryl, heteroarylalkyl, amino, -C(O)OR¹⁵, -C(O)NR¹⁵R¹⁶, -SOₜNR¹⁵R¹⁶, -C(O)R¹⁵, -SO₂R¹⁵ provided that R¹⁵ is not H, -NHC(O)NR¹⁵R¹⁶, -NHC(O)OR¹⁵, halogen, and a heterocylcoalkenyl group;
each R¹⁵ and R¹⁶ is independently selected from the group consisting of: H, alkyl, aryl, arylalkyl, cycloalkyl and heteroaryl;
R¹⁷ is selected from the group consisting of: -SO₂alkyl, -SO₂aryl, -SO₂cycloalkyl, and -SO₂heteroaryl;
R¹⁸ is selected from the group consisting of: H, alkyl, aryl, heteroaryl, -C(O)R¹⁹, -SO₂R¹⁹ and -C(O)NR¹⁹R²⁰;
each R¹⁹ and R²⁰ is independently selected from the group consisting of: alkyl, aryl and heteroaryl;
R³⁰ is selected from the group consisting of: alkyl, cycloalkyl, -CN, -NO₂, or -SO₂R¹⁵ provided that R¹⁵ is not H;
each R³¹ is independently selected from the group consisting of: unsubstituted or substituted alkyl, unsubstituted or substituted aryl, unsubstituted or substituted heteroaryl and unsubstituted or substituted cycloalkyl; wherein there are 1 to 6 substituents on said substituted R³¹ groups and each substituent is independently selected from the group consisting of: R⁹ groups;
each R⁴⁰ is independently selected from the group consisting of: H, alkyl and cycloalkyl; and
t is 0, 1 or 2.

8. A compound of Claim 7 wherein B is selected from the group consisting of:
(1) 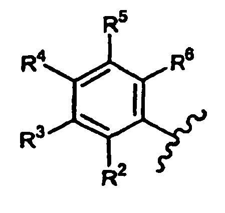 wherein R³ is -C(O)NR¹³R¹⁴,
and all other substituents are as defined for formula IA in Claim 7;
(2) 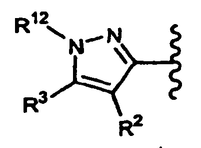 wherein all substituents are as defined for formula IA in Claim 7;
(3) 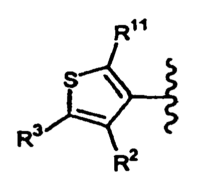 wherein all substituents are as defined for formula IA in Claim 7;
(4) 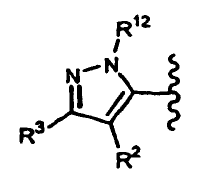 wherein all substituents are as defined for formula IA in Claim 7;
(5) 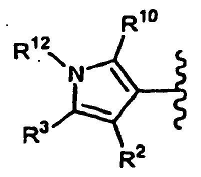 wherein all substituents are as defined for formula IA in claim 7;
(6) 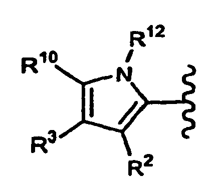 wherein all substituents are as defined for formula 1A in Claim 7;
(7) 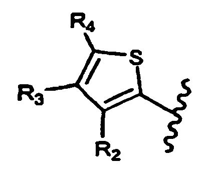 wherein all substituents are as defined for formula IA in Claim 7;
(8) 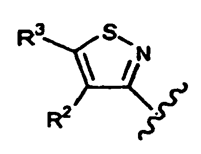 wherein all substituents are as defined for formula IA in Claim 7;
(9) 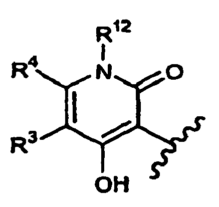 wherein all substituents are as defined for formula IA in Claim 7;
(10) 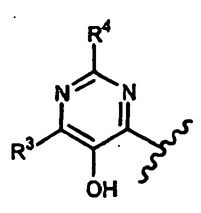 wherein all substituents are as defined for formula IA in Claim 7;
(12) 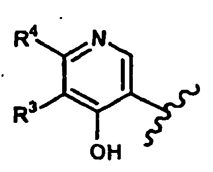 wherein all substituents are as defined for formula IA in Claim 7; and
(13) 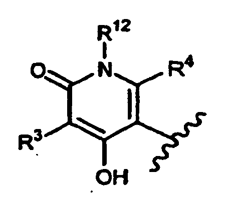
wherein all substituents are as defined for formula IA in Claim 7;

9. The compound of claim 7 wherein B is: 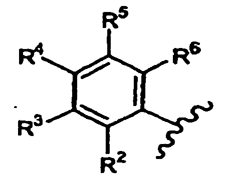 and R³ is -C(O)NR¹³R¹⁴.

10. The compound of claim 9 wherein R¹³ and R¹⁴ are each the same or different alkyl group,

11. The compound of claim 7 wherein B is:

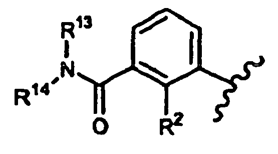

12. The compound of claim 11 wherein R¹³ and R¹⁴ are the same or different alkyl group.

13. The compound of claim 7 wherein B is:

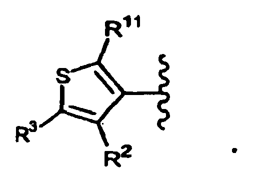

14. The compound of claim 13 wherein R¹¹ is H.

15. The compound of claim 13 or 14 wherein R³ is -C(O)NR¹³R¹⁴.

16. The compound of any one of claims 13 to 15 wherein R¹³ and R¹⁴ are independently selected from the group consisting of: alkyl, unsubstituted heteroaryl and substituted heteroaryl.

17. The compound of claim 16 wherein one of R¹³ or R¹⁴ is alkyl.

18. The compound of claim 17 wherein said alkyl is methyl.

19. The compound of claim 7 wherein 8 is

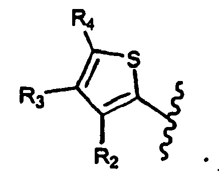

20. The compound of any one of claim 7 to 19 wherein 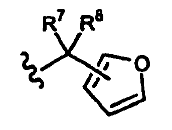 wherein the furan ring is unsubstituted or substitued.

21. The compounds of claim 20 wherein the furan ring is substituted.

22. The compound of claim 21 wherein the furan ring is substituted with at least one alkyl group.

23. The compound of anyone of claims 20 to 22 wherein R⁷ and R⁸ are the same or different and each is selected from the group consisting of: H and alkyl.

24. The compound of claim 23 wherein R⁷ is H, and R⁸ is alkyl.

25. The compound of Claim 1 selected from the group consisting of:

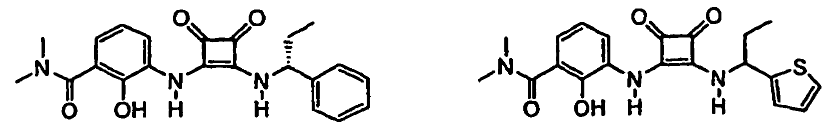

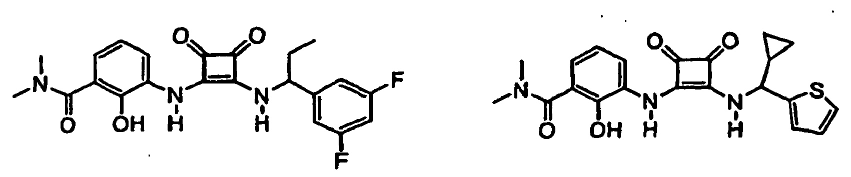

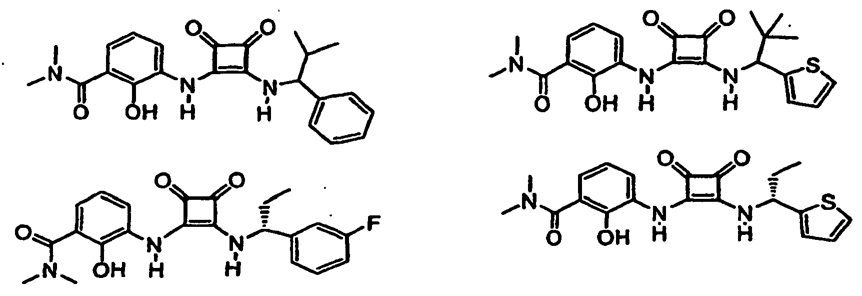

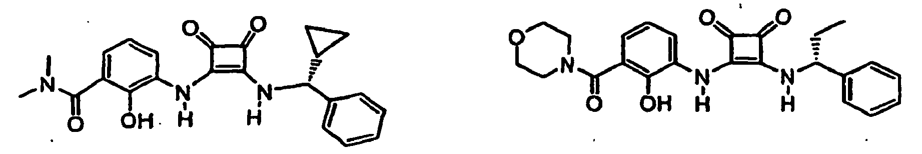

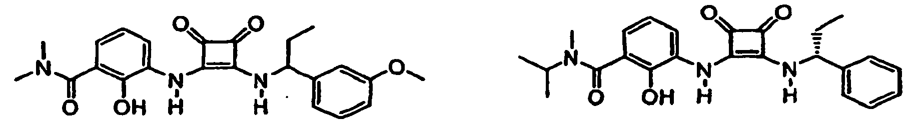

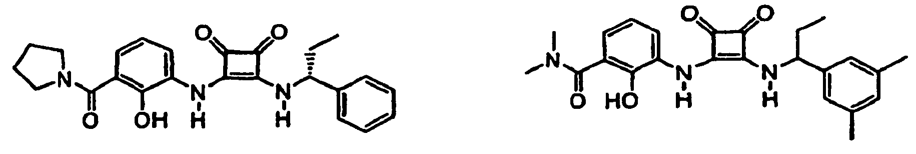

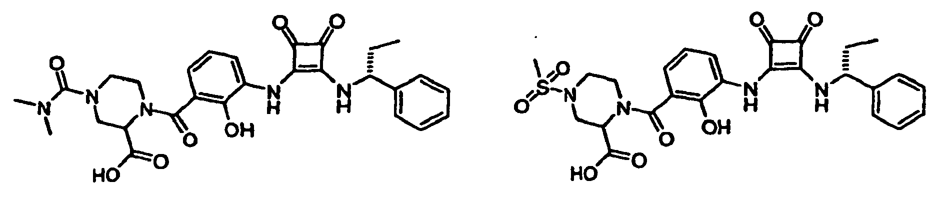

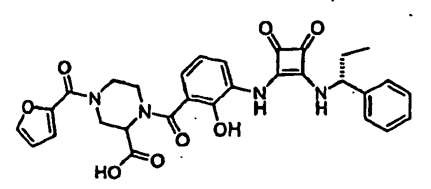

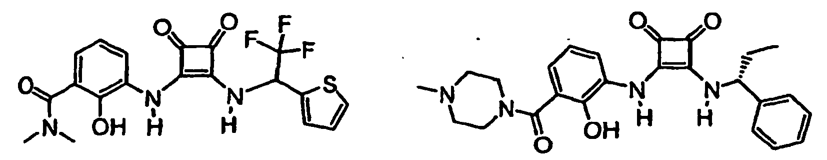

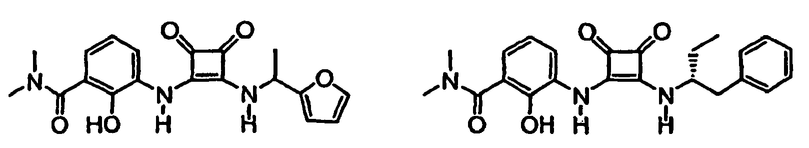

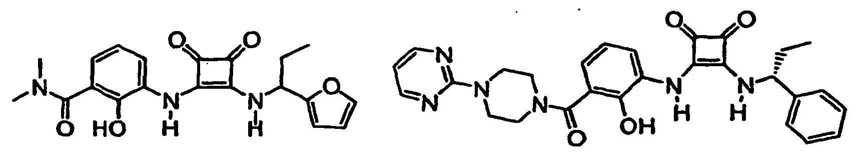

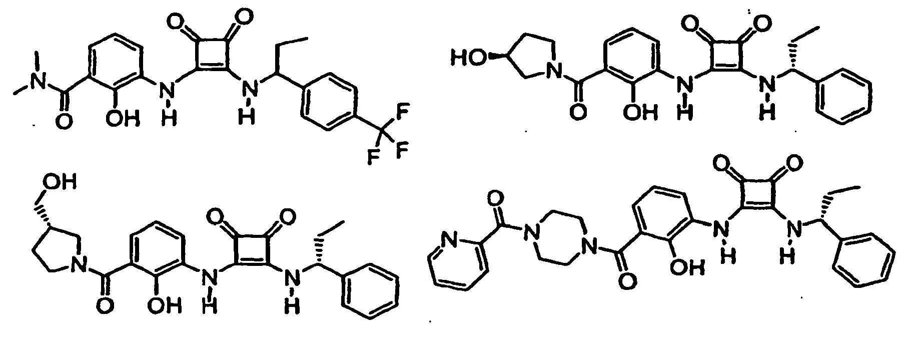

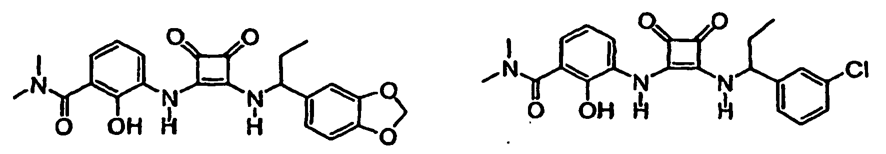

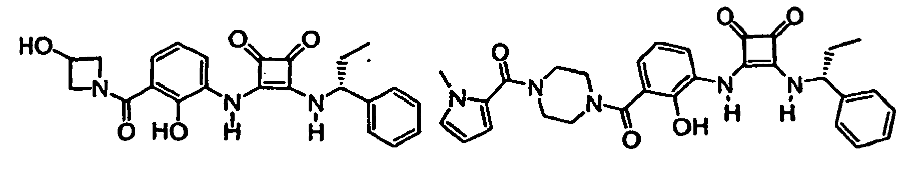

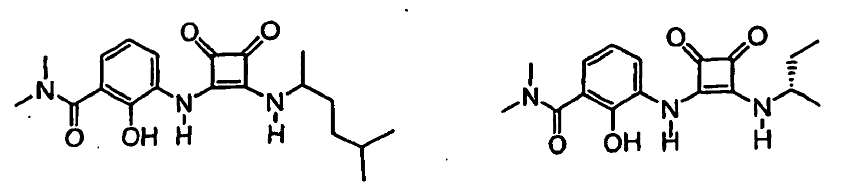

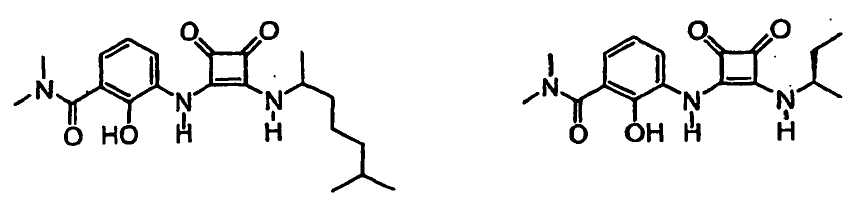

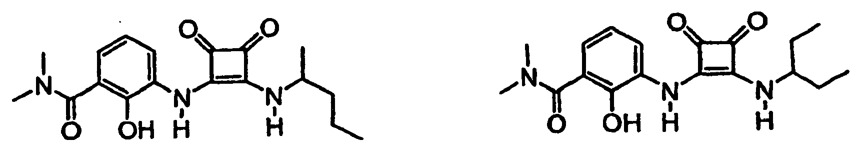

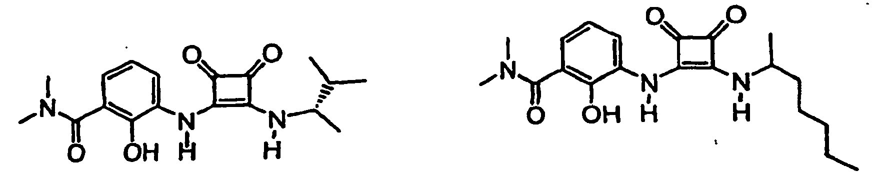

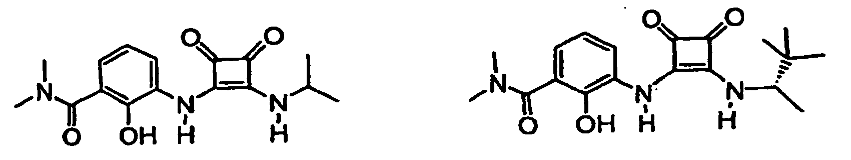

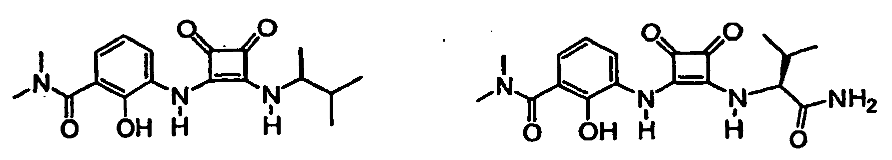

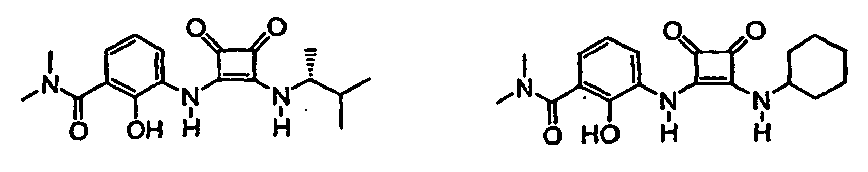

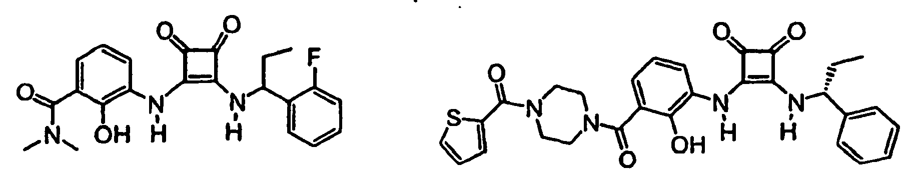

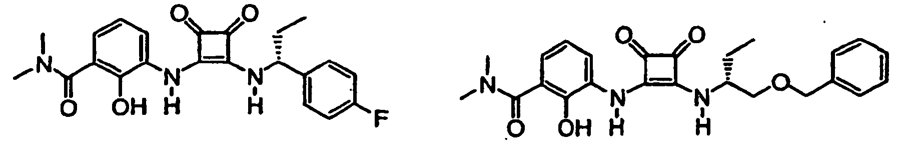

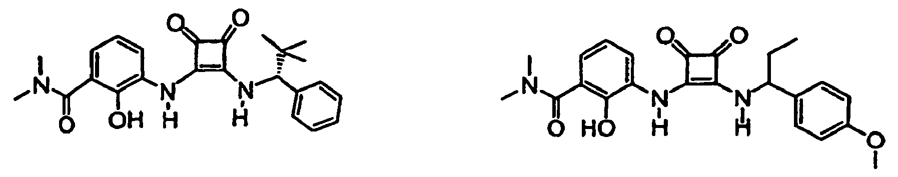

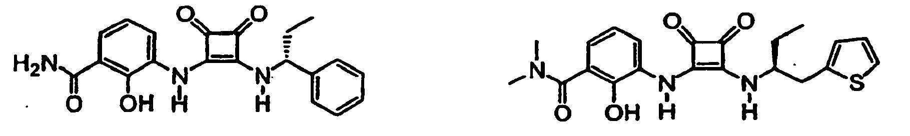

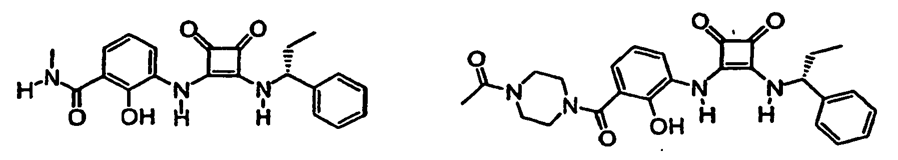

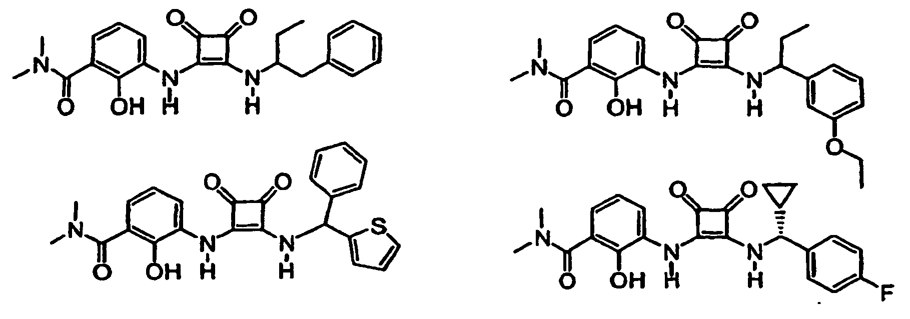

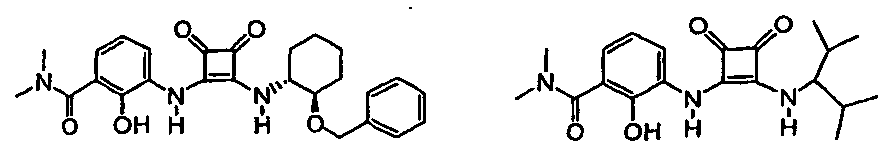

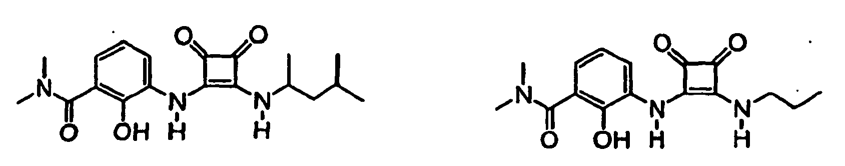

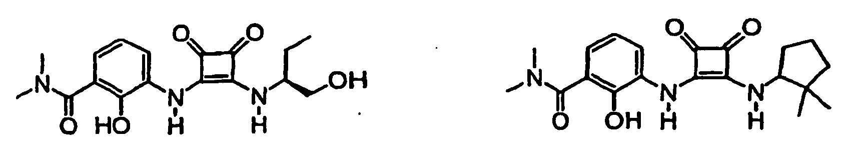

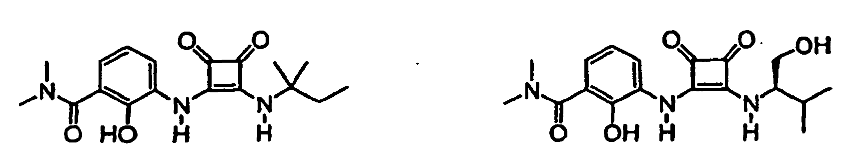

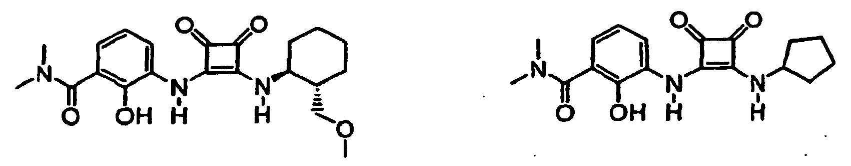

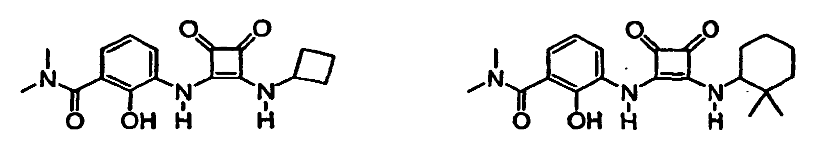

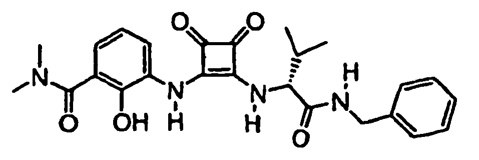

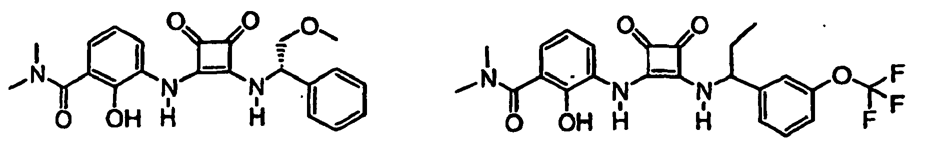

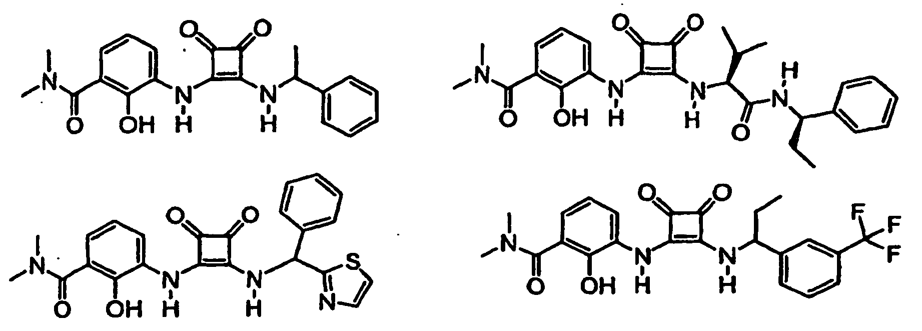

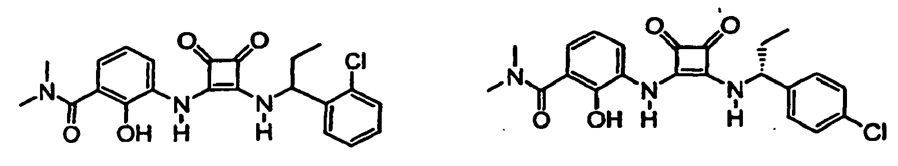

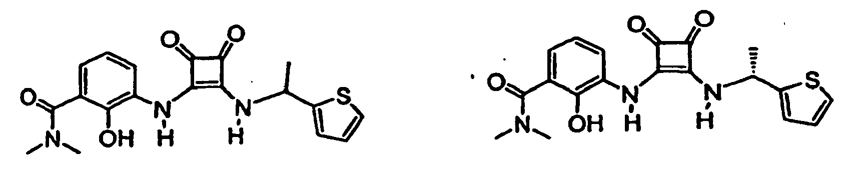

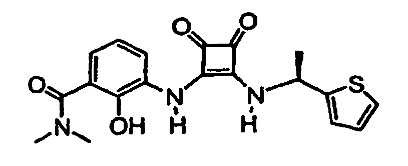

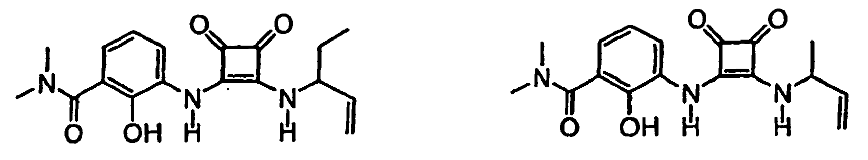

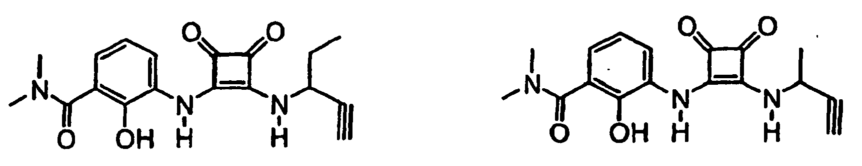

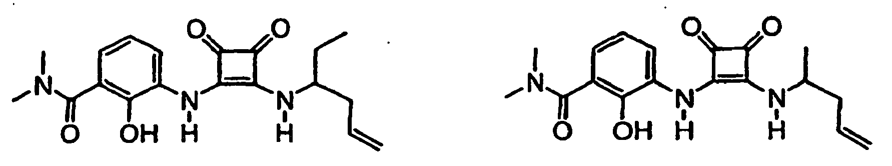

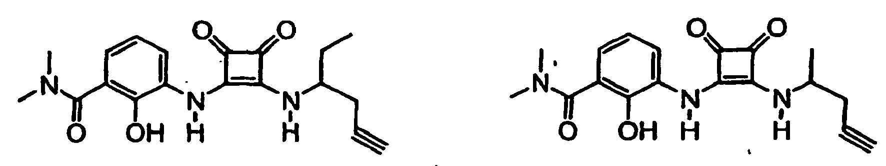

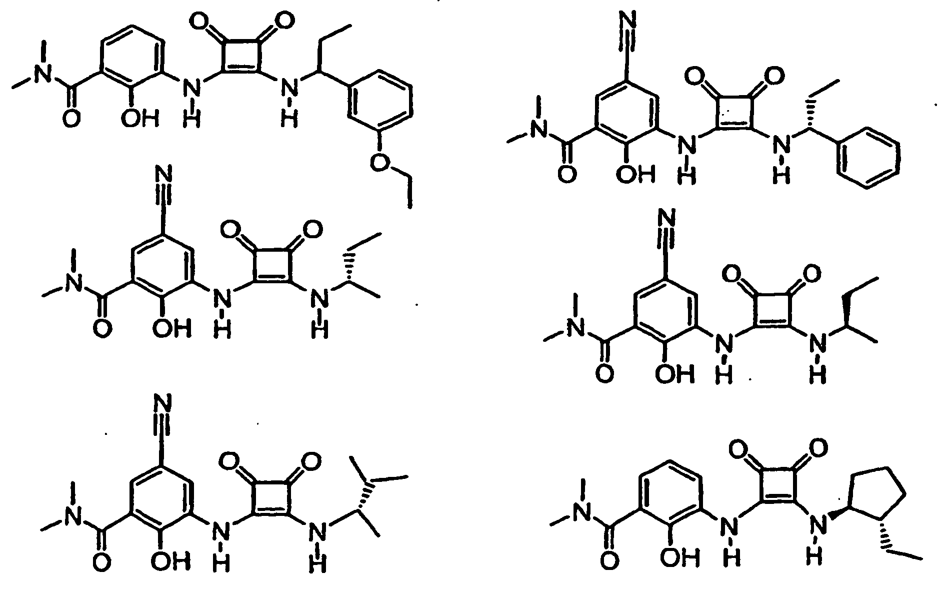

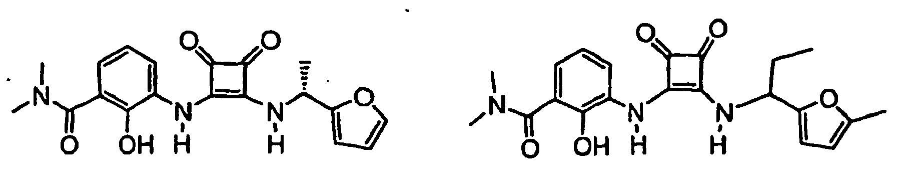

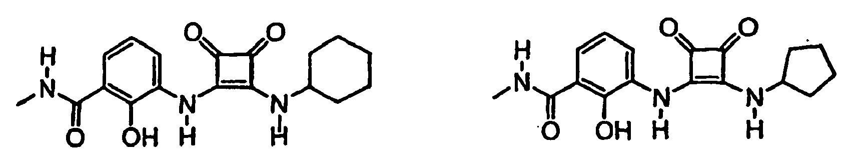

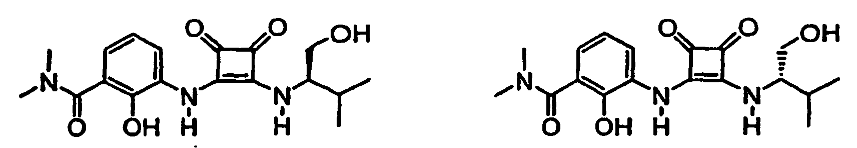

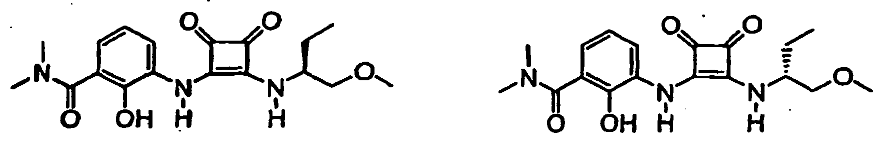

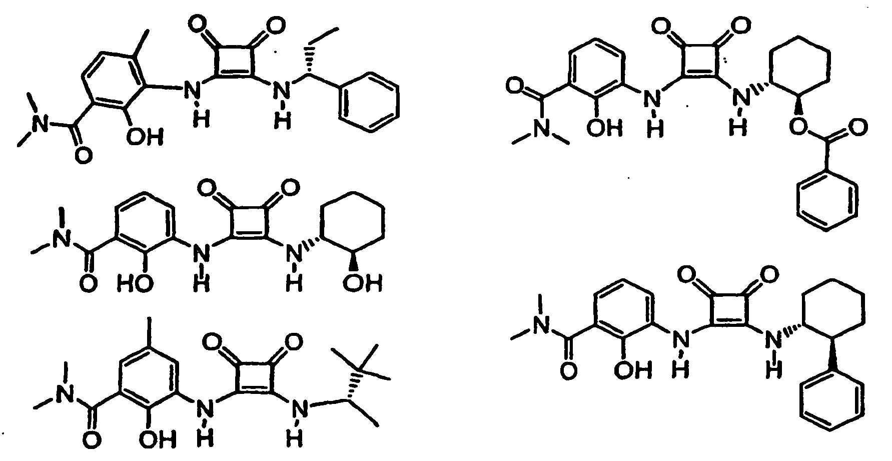

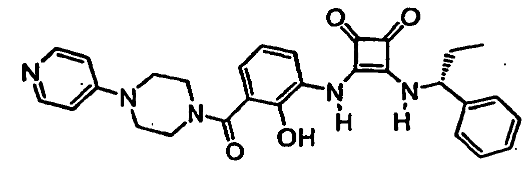

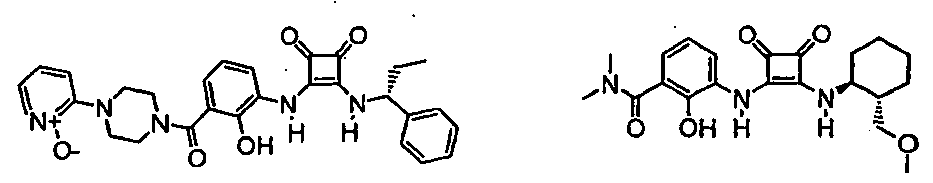

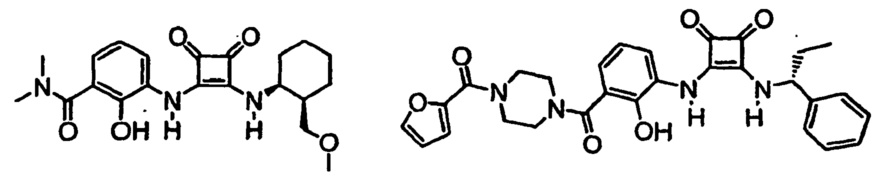

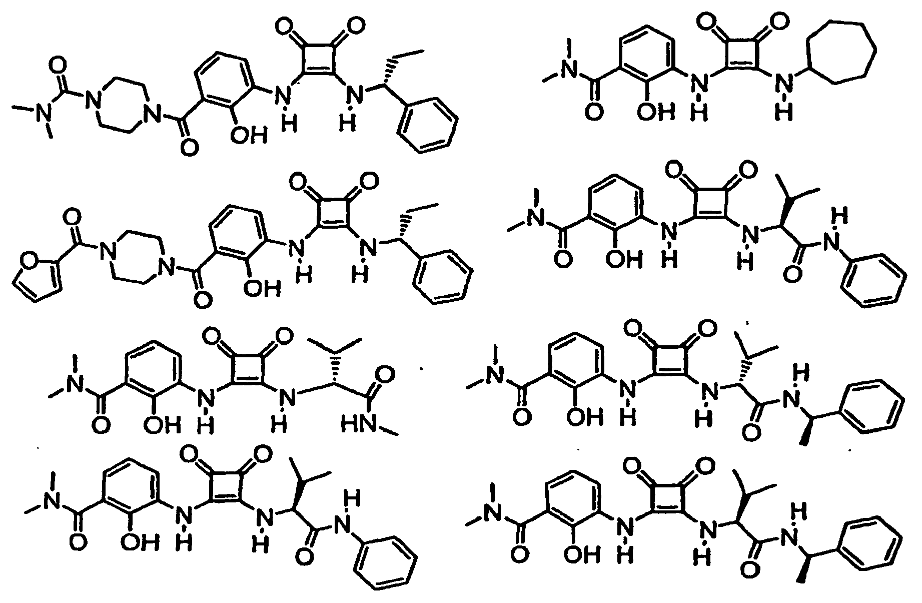

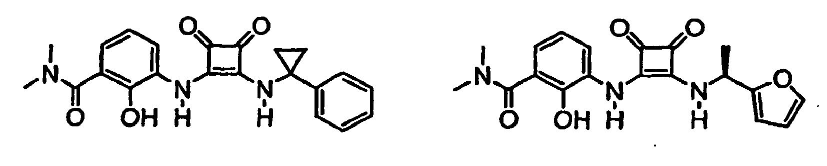

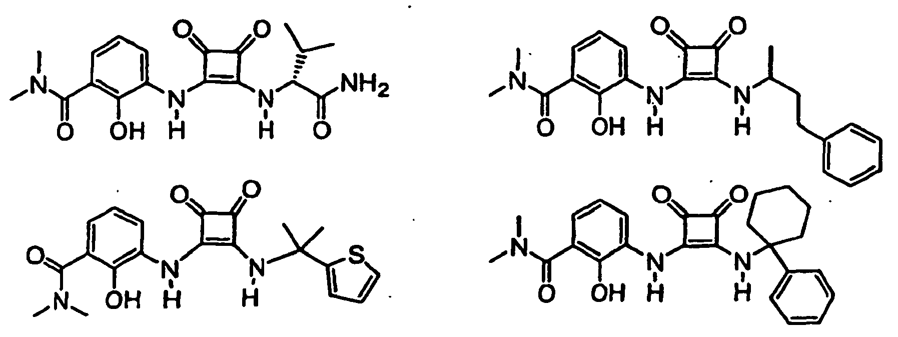

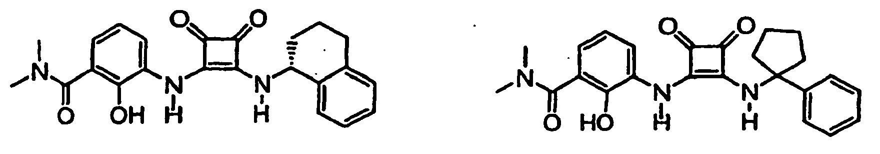

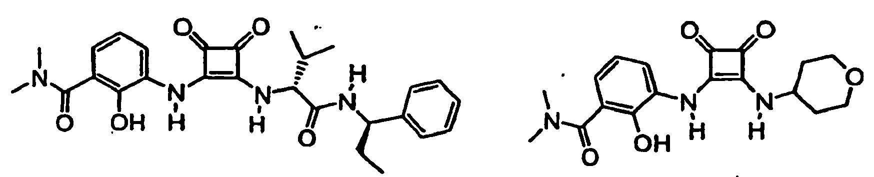

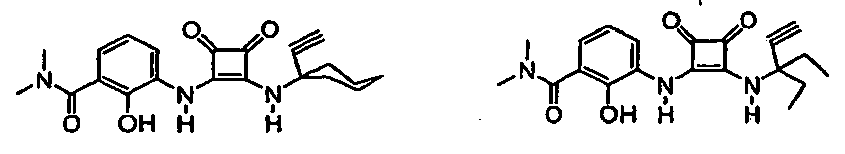

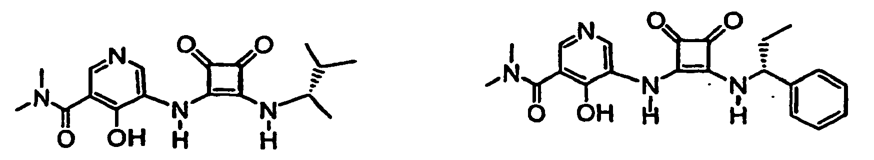

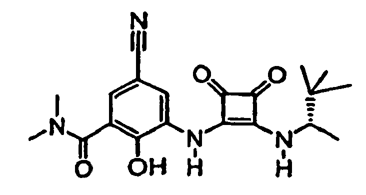

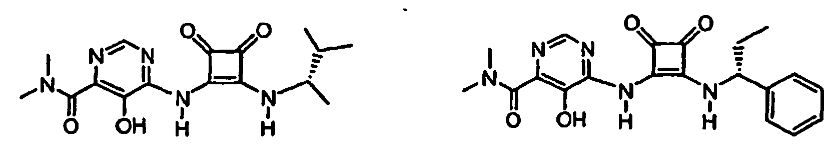

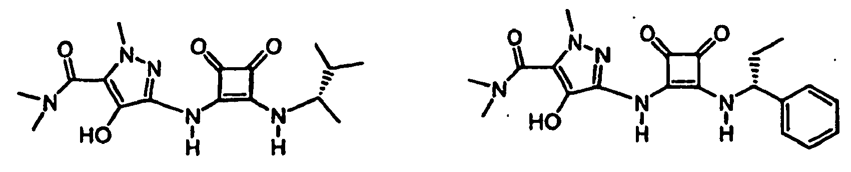

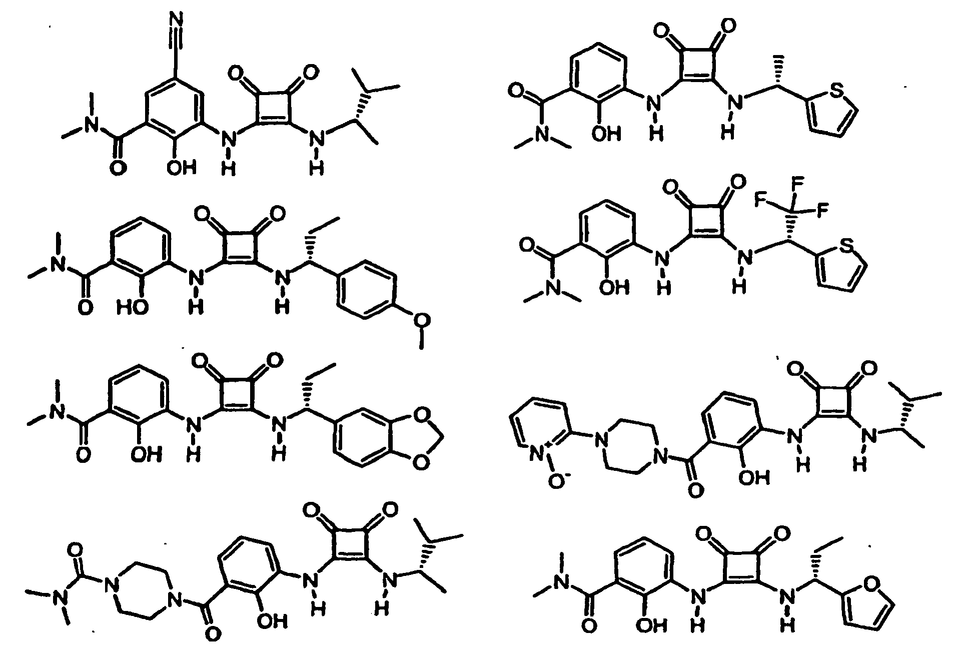

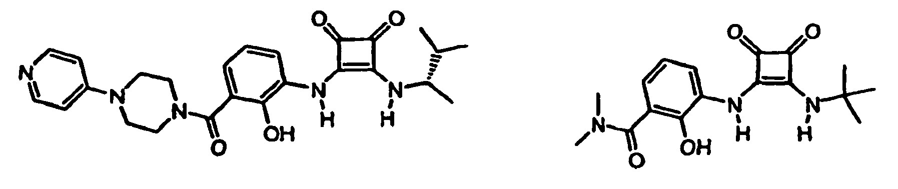

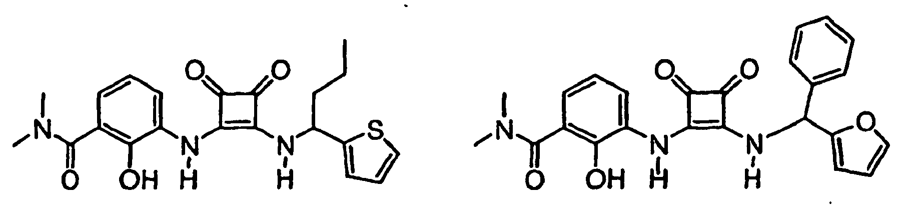

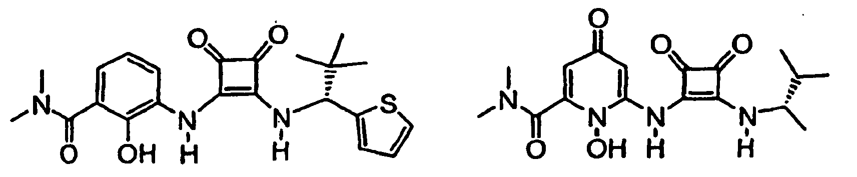

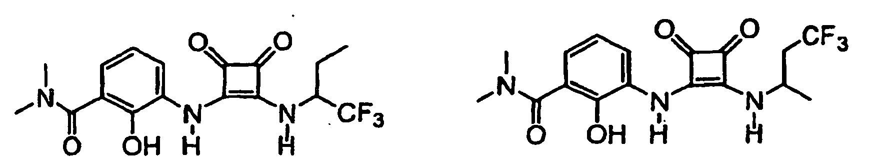

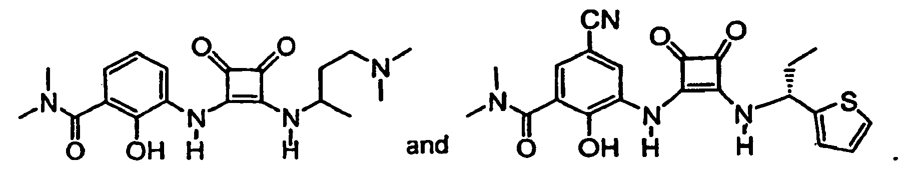

26. The compound of Claim 1 selected from the group consisting of

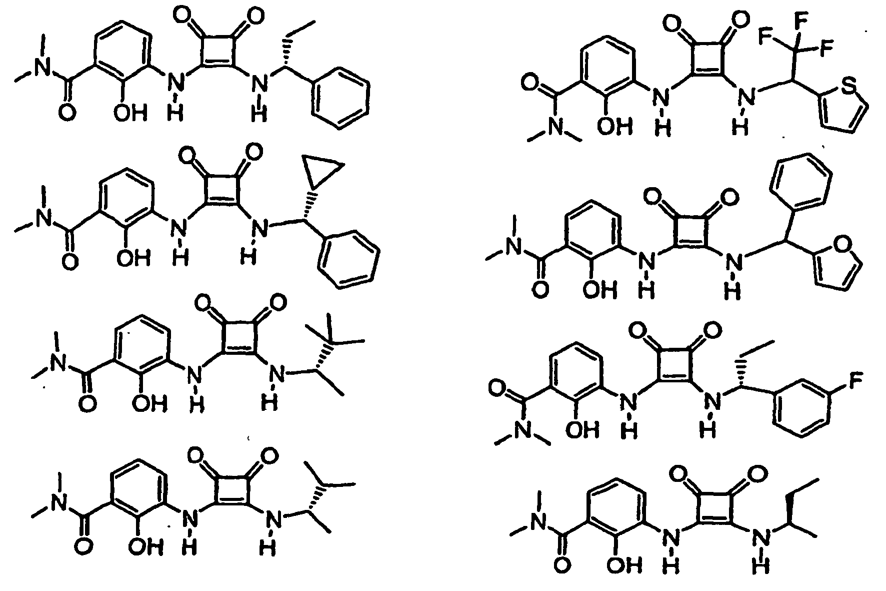

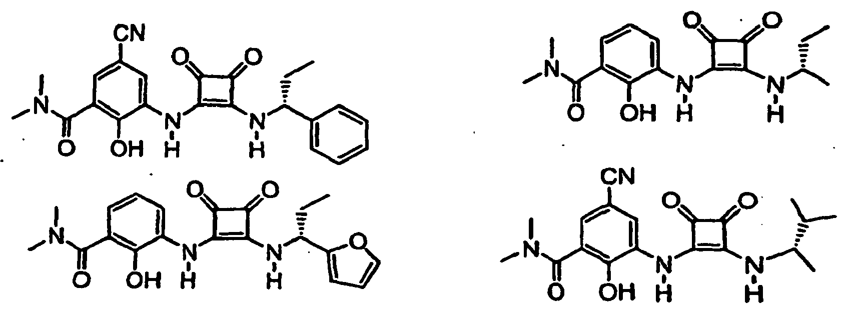

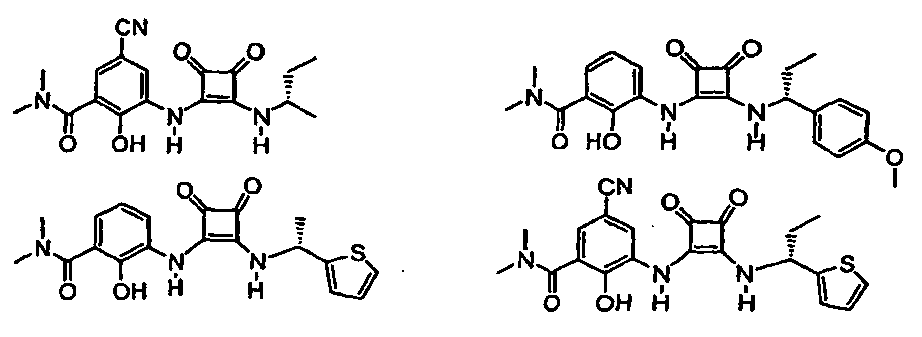

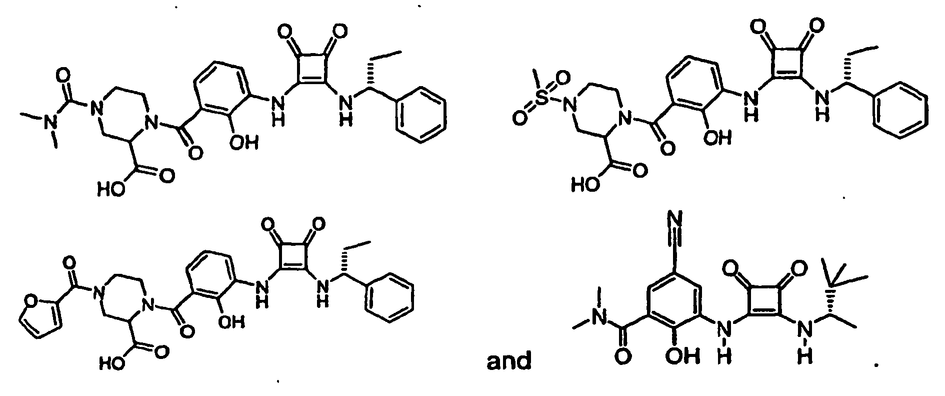

27. A compound of the formula 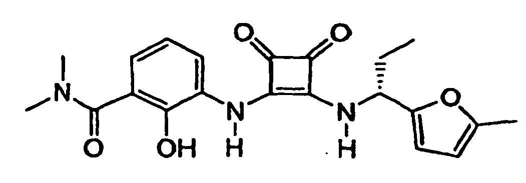 or a pharmaceutically acceptable salt or solvate thereof.

28. A compound of the formula 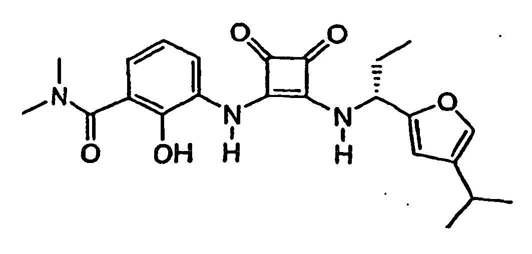 or a pharmaceutically acceptable salt or solvate thereof.

29. A compound of the formula 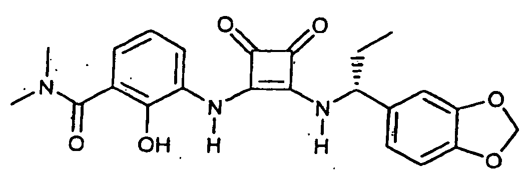 or a pharmaceutically acceptable salt or solvate thereof.

30. A compound of the formula 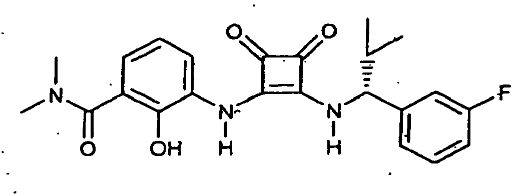 or a pharmaceutically acceptable salt or solvate thereof.

31. A compound of the formula 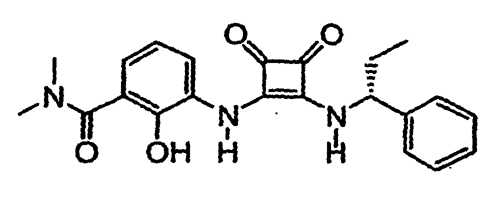 or a pharmaceutically acceptable salt or solvate thereof.

32. A compound of the formula 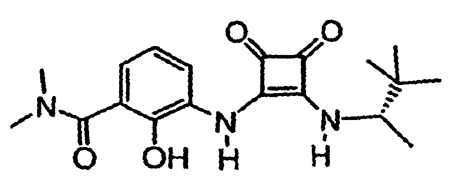 or a pharmaceutically acceptable salt or solvate thereof.

33. A compound of the formula 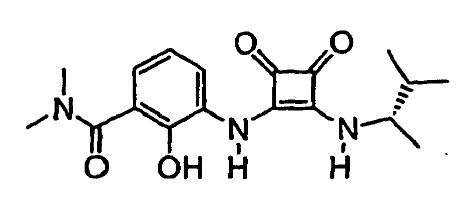 or a pharmaceutically acceptable salt or solvate thereof.

34. A compound of the formula 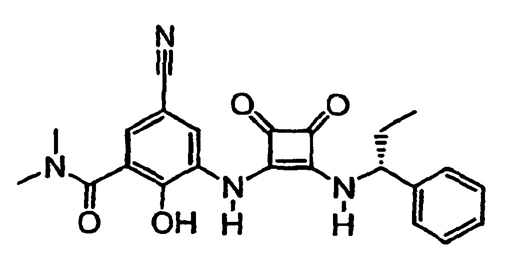 or a pharmaceutically acceptable salt or solvate thereof.

35. A compound of the formula 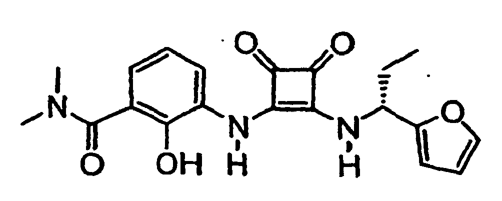 or a pharmaceutically acceptable salt or solvate thereof.

36. A compound of the formula 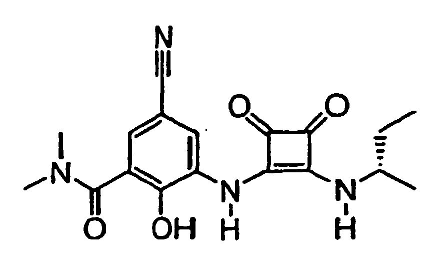 or a pharmaceutically acceptable salt or solvate thereof.

37. A compound of the formula 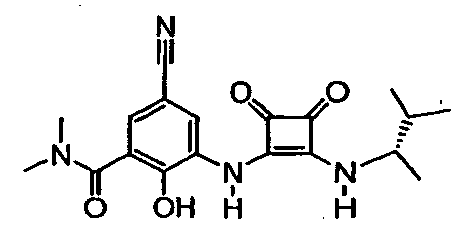 or a pharmaceutically acceptable salt or solvate thereof.

38. A compound of the formula 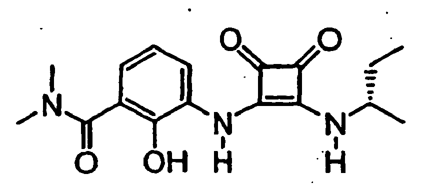 or a pharmaceutically acceptable salt or solvate thereof.

39. A compound of the formula 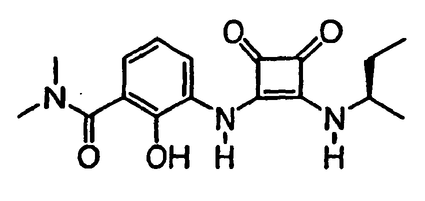 or a pharmaceutically acceptable salt or solvate thereof.

40. A compound of the formula 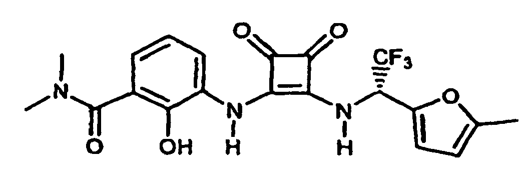 or a pharmaceutically acceptable salt or solvate thereof.

41. A compound of the formula 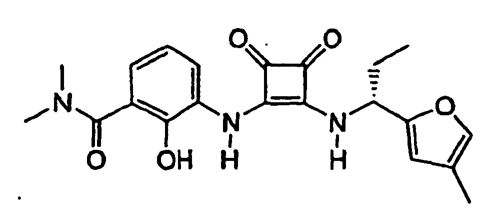 or a pharmaceutically acceptable salt or solvate thereof.

42. A compound of the formula 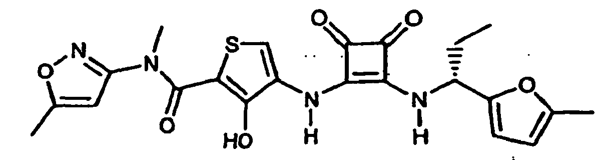 or a pharmaceutically acceptable salt or solvate thereof.

43. The use of a compound of any of Claims 1 to 42 for the manufacture of a medicament for treating a chemokine-mediated disease wherein the chemokine mediated disease is selected from the group consisting of psoriasis, atopic dermatitis, acne, asthma, chronic obstructive pulmonary disease, adult respiratory disease, arthritis, inflammatory bowel disease, Crohn's disease, ulcerative colitis, septic shock, endotoxic shock, gram negative sepsis, toxic shock syndrome, stroke, cardiac and renal reperfusion injury, glomerulonephritis or thrombosis, Alzheimer's disease, graft vs. host reaction, allograft rejections, cystic fibrosis, malaria, acute respiratory distress syndrome, delayted type hypersensitivity reaction, atherosclerosis and cerebral and cardiac ischemia.

44. The use of a compound of any of Claims 1 to 42 for the manufacture of a medicament for treating cancer.

45. The use of a compound of any of Claims 1 to 42 for the manufacture of a medicament for treating cancer in combination with the use of an anticancer agent for the manufacture of a medicament for treating cancer.

46. The use of Claim 45, wherein the anti-cancer agent is selected from the group consisting of alkylating agents, antimetabolites, natural products and their derivatives, hormones, anti-hormones, anti-angiogenic agents and steroids, and synthetics.

47. The use of a compound of any of Claims 1 to 42 for the manufacture of a medicament for inhibiting angiogenesis.

48. The use of a compound of any of Claims 1 to 42 for the manufacture of a medicament for inhibiting angiogenisis in combination with the use of at least one anti-angiogenesis compound for the manufacture of a medicament for inhibiting angiogenesis.

49. The use of a compound of any of Claims 1 to 42 for the manufacture of a medicament for treating a disease selected from the group consisting of gingivitis, respiratory viruses, herpes viruses, hepatitis viruses; HIV, kaposi's sarcoma associated virus and atherosclerosis.

50. The use of a compound of any one of claims 1 to 42 for the manufacture of a medicament for treating an angiogenic ocular disease.

51. The use of Claim 50 wherein the angiogenic ocular disease is selected from the group consisting of ocular inflammation, retinopathy of prematurity, diabetic retinopathy, macular degeneration with the wet type preferred and corneal neovascularization.

52. The use of Claim 44 wherein the cancer treated is melanoma, gastric carcinoma, or non-small cell lung carcinoma.

53. The use of Claim 45 wherein the cancer treated is melanoma, gastric carcinoma, or non-small cell lung carcinoma.

54. The use of Claim 46, wherein the wherein the cancer treated is melanoma, gastric carcinoma, or non-small cell lung carcinoma.

55. A pharmaceutical comosition comprising an effective amount of a compound of any of Claims 1 to 42 in combination with a pharmaceutically acceptable carrier.

56. A sodium salt of any of the compound any of Claims 1 to 42.

57. A calcium salt of any of the compound any of Claims 1 to 42.

58. A pharmaceutical comosition comprising an effective amount of a compound of any of Claims 56 and 57 in combination with a pharmaceutically acceptable carrier.

## Patentansprüche

1. Verbindung mit der Formel: 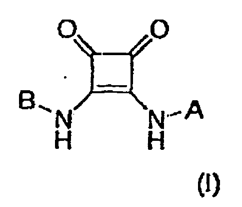 oder ein pharmazeutisch annehmbares Salz oder Solvat davon, worin A ausgewählt ist aus der Gruppe bestehend aus: 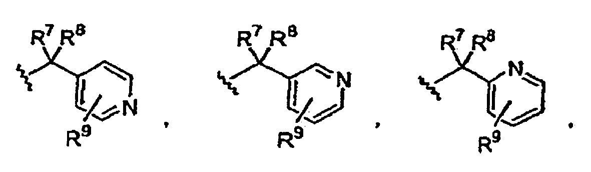 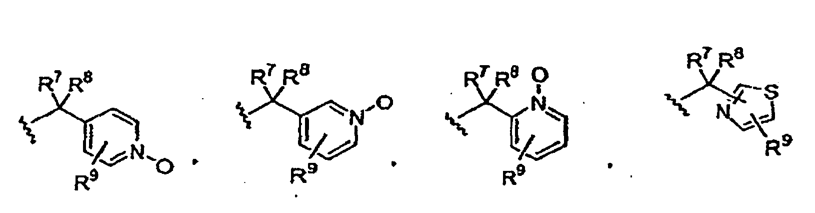 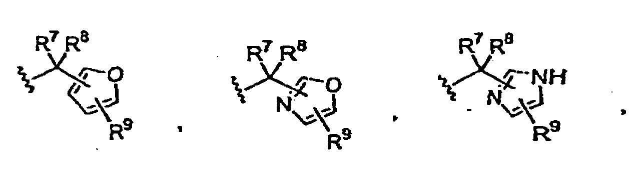 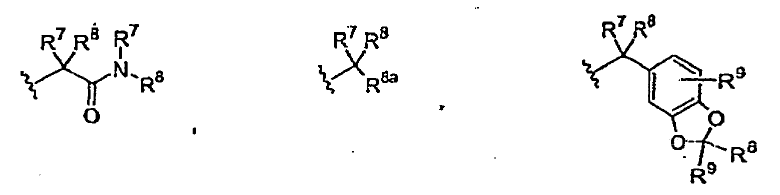 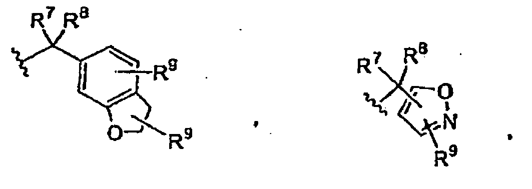 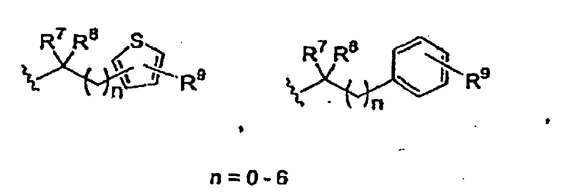 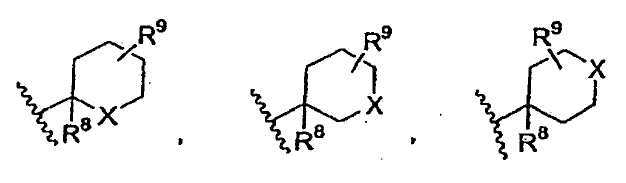 X=-O-, -NH-, -S- 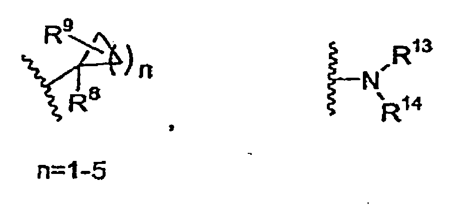 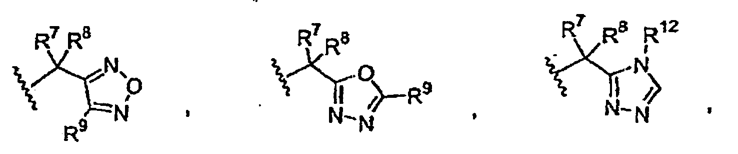 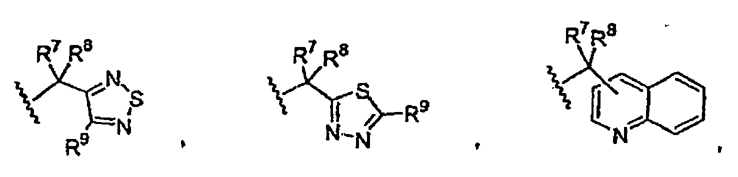 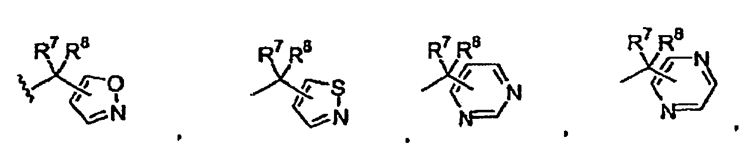 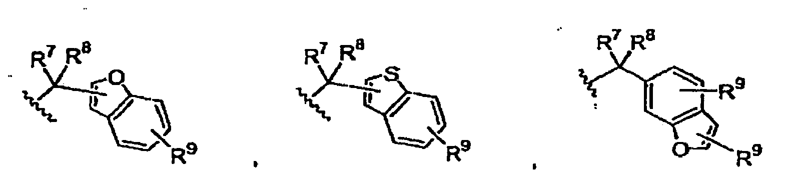 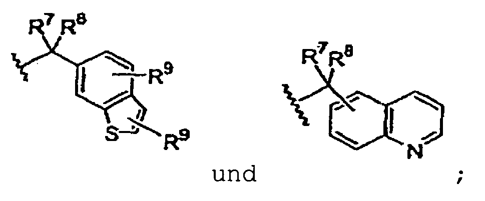 B ausgewählt ist aus der Gruppe bestehend aus: 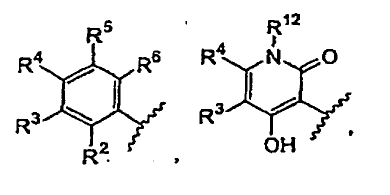 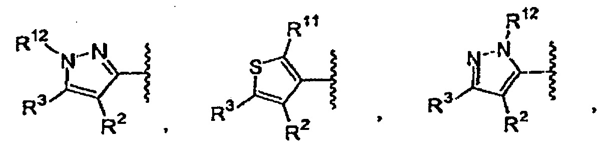 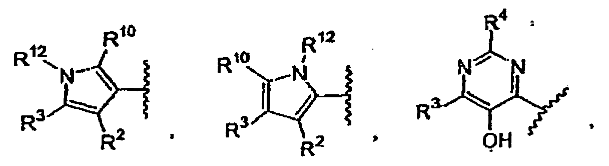 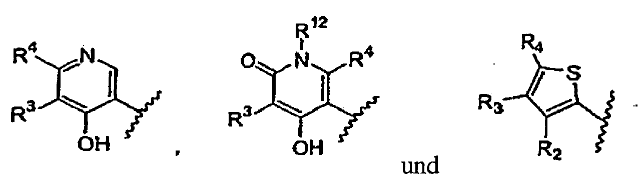 R² OH ist;
R³ ausgewählt ist aus der Gruppe bestehend aus -C(O)NHR¹⁷, -C(O)NR¹³R¹⁴ und -C(O)NR¹³OR¹⁴;
R⁴ ausgewählt ist aus der Gruppe bestehend aus Wasserstoff, Cyano, Halogen, Alkyl, Alkoxy, -OH, -CF₃, -OCF₃, -NO₂, -C(O)R¹³, -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, 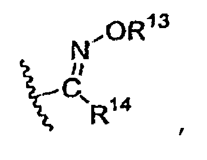 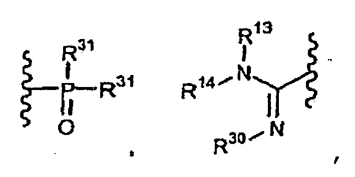 -NHC(O)R¹⁷, unsubstituiertem oder substituiertem Aryl und unsubstituiertem oder substituiertem Heteroaryl,
wobei die Substituenten an den substituierten R⁴-Gruppen gleich oder unterschiedlich sind und unabhängig ausgewählt sind aus 1 bis 6 R⁹-Gruppen;
R⁵ und R⁶ gleich oder unterschiedlich sind und unabhängig ausgewählt sind aus der Gruppe bestehend aus Wasserstoff, Halogen, Alkyl, Alkoxy, -CF₃, -OCF₃, -NO₂, -C(O)R¹³, -C(O)OR¹³, -C(O)NR¹³R¹⁴, -SO₍ₜ₎NR¹³R¹⁴, -C(O)NR¹³OR¹⁴, Cyano, unsubstituierter oder substituierter Aryl- und unsubstituierter oder substituierter Heteroarylgruppe,
wobei die Substituenten an den substituierten R⁵- und R⁶-Gruppen gleich oder unterschiedlich sind und unabhängig ausgewählt sind aus 1 bis 6 R⁹-Gruppen;
R⁷ und R⁸ gleich oder unterschiedlich sind und unabhängig ausgewählt sind aus der Gruppe bestehend aus H, unsubstituiertem oder substituiertem Alkyl, unsubstituiertem oder substituiertem Aryl, unsubstituiertem oder substituiertem Heteroaryl, unsubstituiertem oder substituiertem Arylalkyl, unsubstituiertem oder substituiertem Heteroarylalkyl, unsubstituiertem oder substituiertem Cycloalkyl, unsubstituiertem oder substituiertem Cycloalkylalkyl, -CO₂R¹³, -CONR¹³R¹⁴, Fluoralkyl, Alkinyl, Alkenyl und Cycloalkenyl,
wobei die Substituenten an den substituierten R⁷- und R⁸-Gruppen ausgewählt sind aus der Gruppe bestehend aus:
a) H,
b) Halogen,
c) -CF₃,
d) -COR¹³,
e) -OR¹³,
f) -NR¹³R¹⁴,
g) -NO₂,
h) -CN,
i) -SO₂OR¹³,
j) -Si(alkyl)₃,
k) -Si(aryl)₃,
l) -(R¹³)₂R¹⁴Si,
m) -CO₂R¹³,
n) -C(O)NR¹³R¹⁴,
o) -SO₂NR¹³R¹⁴,
p) -SO₂R¹³,
q) -O(C=O)R¹³,
r) -O(C=O)NR¹³R¹⁴,
s) -NR¹³C(O)R¹⁴ und
t) -NR¹³CO₂R¹⁴;
R^{8a} ausgewählt ist aus der Gruppe bestehend aus Wasserstoff, Alkyl, Cycloalkyl und Cycloalkylalkyl;
R⁹ unabhängig ausgewählt ist aus 1 bis 6 aus der Gruppe bestehend aus:
a) -R¹³,
b) Halogen,
c) -CF₃,
d) -COR¹³,
e) -OR¹³,
f) -NR¹³R¹⁴,
g) -NO₂,
h) -CN,
i) -SO₂R¹³,
j) -SO₂NR¹³R¹⁴,
k) -NR¹³COR¹⁴,
l) -CONR¹³R¹⁴,
m) -NR¹³CO₂R¹⁴,
n) -CO₂R¹³ und
o) 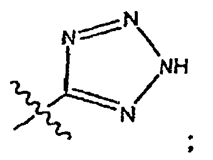
R¹⁰ und R¹¹ gleich oder unterschiedlich sind und unabhängig ausgewählt sind aus der Gruppe bestehend aus Wasserstoff, Halogen, -CF₃, -OCF₃, -NR¹³R¹⁴, -NR¹³C(O)NR¹³R¹⁴, -OH, -C(O)OR¹³, -SH, -SO₍ₜ₎NR¹³R¹⁴, -SO₂R¹³, -NHC(O)R¹³, -NHSO₂NR¹³R¹⁴, -NHSO₂R¹³, -C(O)NR¹³R¹⁴, -C(O)NR¹³OR¹⁴, -OC(O)R¹³ und Cyano;
R¹² Wasserstoff, -OC(O)R¹³ oder eine unsubstituierte oder substituierte Aryl-, unsubstituierte oder substituierte Heteroaryl-, unsubstituierte oder substituierte Arylalkyl-, unsubstituierte oder substituierte Cycloalkyl-, unsubstituierte oder substituierte Alkyl-, unsubstituierte oder substituierte Cycloalkylalkyl- oder unsubstituierte oder substituierte Heteroarylalkylgruppe ist,
wobei die Substituenten an den substituierten R¹²-Gruppen gleich oder unterschiedlich sind und unabhängig ausgewählt sind aus 1 bis 6 R⁹-Gruppen;
R¹³ und R¹⁴ gleich oder unterschiedlich sind und unabhängig ausgewählt sind aus der Gruppe bestehend aus H, unsubstituiertem oder substituiertem Alkyl, unsubstituiertem oder substituiertem Aryl, unsubstituiertem oder substituiertem Heteroaryl, unsubstituiertem oder substituiertem Arylalkyl, unsubstituiertem oder substituiertem Heteroarylalkyl, unsubstituiertem oder substituiertem Cycloalkyl, unsubstituiertem oder substituiertem Cycloalkylalkyl und unsubstituiertem oder substituiertem Fluoralkyl, oder
R¹³ und R¹⁴ zusammengenommen werden können, wenn beide an ein Stickstoffatom gebunden sind, um einen unsubstituierten oder substituierten 3- bis 7-gliedrigen heterocyclischen Ring zu bilden, der ein bis zwei Heteroatome ausgewählt aus Sauerstoff, Schwefel und Stickstoff enthält,
wobei die Substituenten an den substituierten R¹³- und R¹⁴-Gruppen gleich oder unterschiedlich sind und unabhängig ausgewählt sind aus 1 bis 6 von H, Alkyl, Aryl, Arylalkyl, Fluoralkyl, Cycloalkyl, Cycloalkylalkyl, Heteroaryl, Heteroarylalkyl, Amino, -C(O)OR¹⁵ -C(O)NR¹⁵R¹⁶, -S(O)ₜNR¹⁵R¹⁶, -C(O)R¹⁵, -SO₂R¹⁵, -NHC(O)NR¹³R¹⁴ und Halogen,
R¹⁵ und R¹⁶ gleich oder unterschiedlich sind und unabhängig ausgewählt sind aus der Gruppe bestehend aus H, Alkyl, Aryl, Arylalkyl, Cycloalkyl und Heteroaryl,
R¹⁷ SO₂alkyl, SO₂aryl, -SO₂cycloalkyl oder -SO₂heteroaryl ist;
R³⁰ Alkyl, Cycloalkyl, -CN, -NO₂ oder -SO₂R¹⁵ ist;
R³¹ gleich oder verschieden sind und unabhängig ausgewählt sind aus der Gruppe bestehend aus unsubstituiertem oder substituiertem Alkyl, unsubstituiertem oder substituiertem Aryl, unsubstituiertem oder substituiertem Heteroaryl und unsubstituiertem oder substituiertem Cycloalkyl;
wobei die Substituenten an den substituierten R³¹-Gruppen gleich oder unterschiedlich sind und unabhängig ausgewählt sind aus 1 bis 6 R⁹-Gruppen, und
t 0, 1 oder 2 ist.

2. Verbindung nach Anspruch 1, wobei A ausgewählt ist aus der Gruppe bestehend aus: 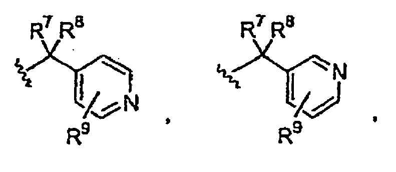 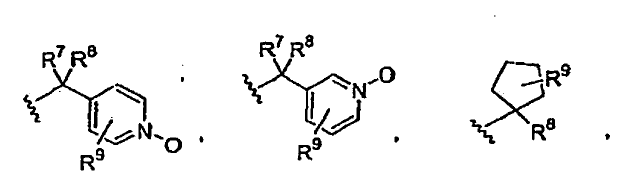 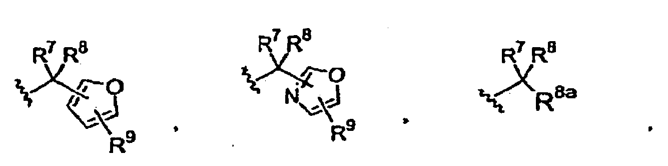 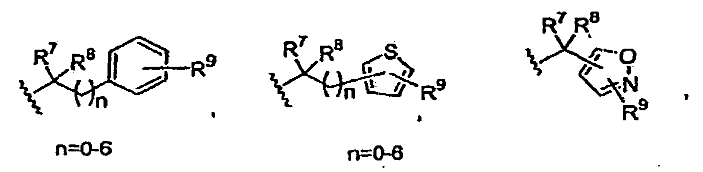 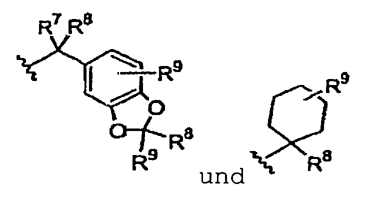 wobei
R⁷ H, Fluoralkyl, Alkyl oder Cycloalkyl ist;
R⁸ H, Alkyl, -CF₂CH₃ oder -CF₃ ist;
R⁹ H, F, Cl, Br, Alkyl oder -CF₃ ist.

3. Verbindung nach Anspruch 1, wobei A ausgewählt ist aus der Gruppe bestehend aus: 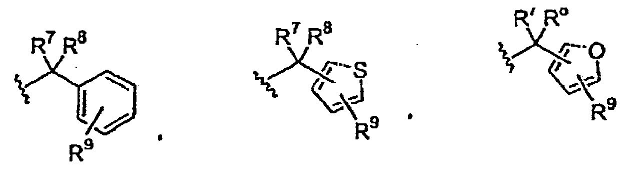 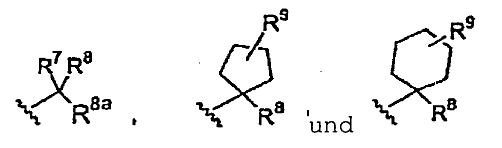 wobei
R⁷ H, -CF₃, -CF₂CH₃, Methyl, Ethyl, Isopropyl, Cyclopropyl oder t-Butyl ist;
R⁸ H ist;
R⁹ H, F, Cl, Br, Alkyl oder -CF₃ ist, und
B 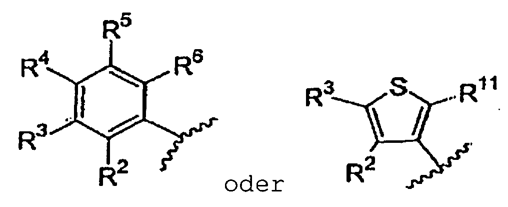 ist,
worin:
R² OH ist;
R³ -C(O)NR¹³R¹⁴ ist;
R⁴ H, -NO₂, Cyano, -CH₃ oder -CF₃ ist;
R⁵ H, CF₃, -NO₂, Halogen oder Cyano ist, und
R⁶ H, Alkyl oder -CF₃ ist;
R¹¹ H, Halogen oder Alkyl ist, und
R¹³ und R¹⁴ gleich oder unterschiedlich sind und unabhängig Methyl, Ethyl oder Isopropyl sind, oder R¹³ und R¹⁴, wenn sie mit dem Stickstoff zusammengenommen werden, an den sie in den Gruppen -NR¹³R¹⁴, -C(O)NR¹³R¹⁴, -SO₂NR¹³R¹⁴, -OC(O)NR¹³R¹⁴, -CONR¹³R¹⁴, -NR¹³C (O)NR¹³R¹⁴, -SOₜNR¹³R¹⁴, -NHSO₂NR¹³R¹⁴ gebunden sind, vorzugsweise einen unsubstituierten oder substituierten 3- bis 7-gliedrigen, gesättigten, heterocyclischen Ring bilden, der gegebenenfalls ein weiteres Heteroatom ausgewählt aus O, S oder NR¹⁸ enthält, wobei R¹⁸ ausgewählt ist aus H, Alkyl, Aryl, Heteroaryl, -C(O)R¹⁹, -SO₂R¹⁹ und -C(O)NR¹⁹R²⁰, wobei R¹⁹ und R²⁰ gleich oder unterschiedlich sind und jeweils unabhängig ausgewählt sind aus Alkyl, Aryl und Heteroaryl, wobei die Substituenten an den substituierten cyclisierten R¹³- und R¹⁴-Gruppen gleich oder unterschiedlich sind und unabhängig ausgewählt sind aus 1 bis 3 von Alkyl, Aryl, Hydroxy, Hydroxyalky], Alkoxy, Alkoxyalkyl, Arylalkyl, Fluoralkyl, Cycloalkyl, Cycloalkylalkyl, Heteroaryl, Heteroarylalkyl, Amino, -C(O)OR¹⁵, -C(O)NR¹⁵R¹⁶, -SOₜNR¹⁵R¹⁶, -C(O)R¹⁵, -SO₂R¹⁵, -NHC(O)NR¹⁵R¹⁶ und Halogen, und wobei R¹⁵ und R¹⁶ gleich oder unterschiedlich sind und unabhängig ausgewählt sind aus der Gruppe bestehend aus H, Alkyl, Aryl, Arylalkyl, Cycloalkyl und Heteroaryl.

4. Verbindung nach Anspruch 1, wobei A ausgewählt ist aus der Gruppe bestehend aus: 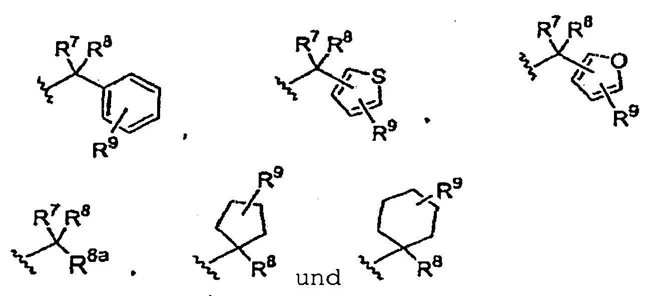 und B 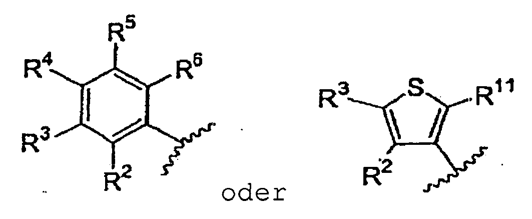 ist,
worin:
R² OH ist;
R³-C(O)NR¹³R¹⁴ ist;
R⁴ H, -NO₂, -CF₃, -CH₃ oder Cyano ist;
R⁵ H, Halogen, -NO₂, Cyano oder -CF₃ ist;
R⁶ H, -CF₃ oder Alkyl ist;
R⁷H, -CF₃, -CF₂CH₃, Methyl, Ethyl, Isopropyl, Cyclopropyl oder t-Butyl ist;
R⁸ H ist;
R⁹ H, F, Cl, Br, Alkyl, Cycloalkyl oder -CF₃ ist;
R¹¹ H, Halogen oder Alkyl ist, und
R¹³ und R¹⁴ unabhängig Methyl oder Ethyl sind.

5. Verbindung nach Anspruch 1, wobei A ausgewählt ist aus der Gruppe bestehend aus: 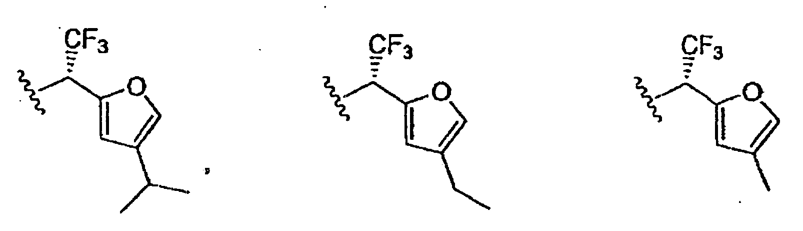 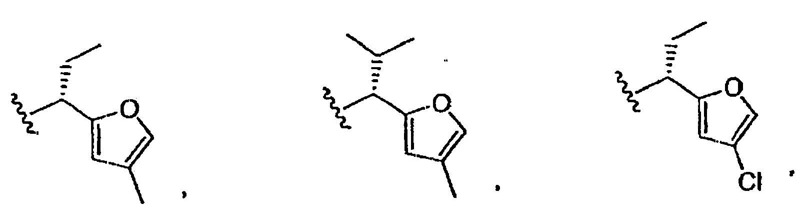 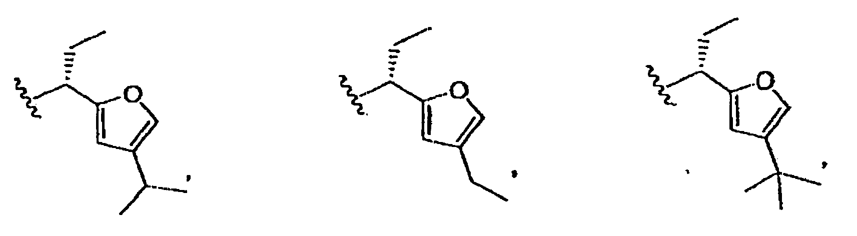 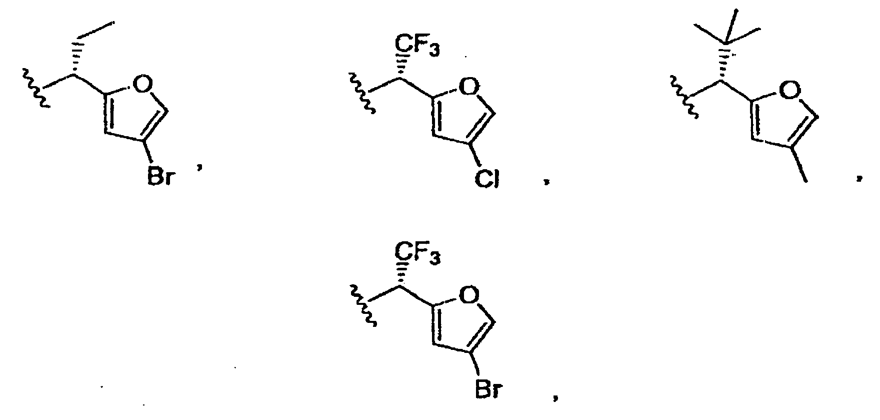 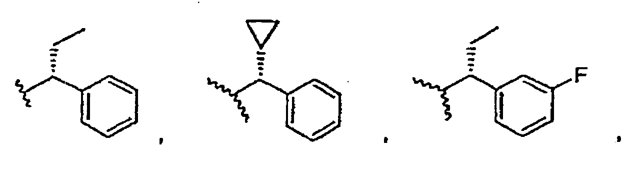 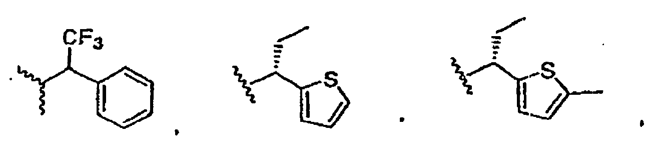 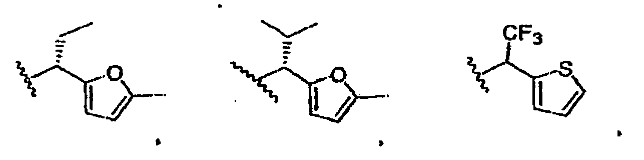 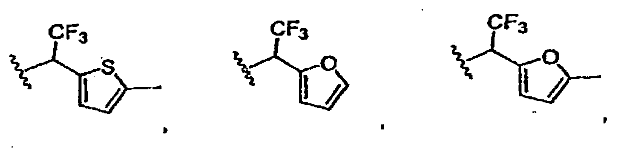 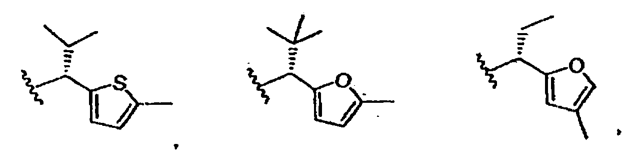 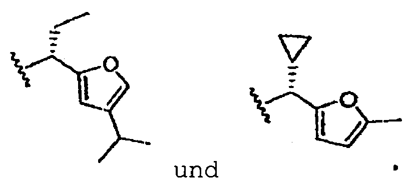 und B 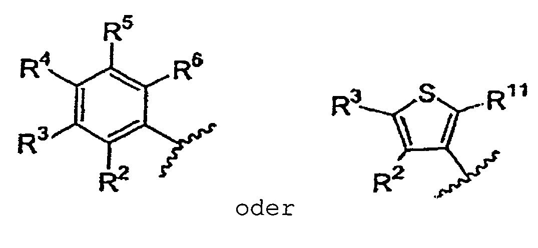 ist,
wobei
R² -OH ist;
R³ -CONR¹³R¹⁴ ist;
R⁴ H, -CH₃ oder -CF₃ ist;
R⁵ H oder Cyano ist;
R⁶ H, -CH₃ oder -CF₃ ist;
R¹¹ H ist, und
R¹³ und R¹⁴ Methyl sind.

6. Verbindung mit der Formel: 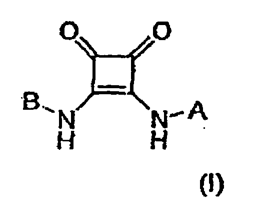 oder ein pharmazeutisch annehmbares Salz oder Solvat der Verbindung, worin:
A ausgewählt ist aus: 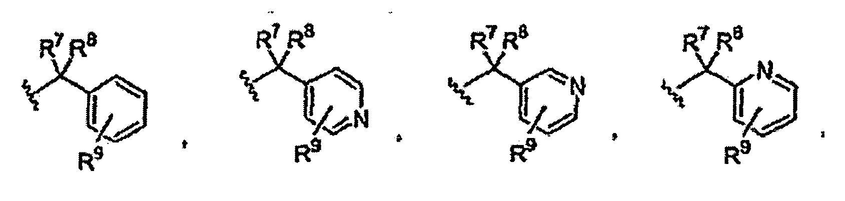 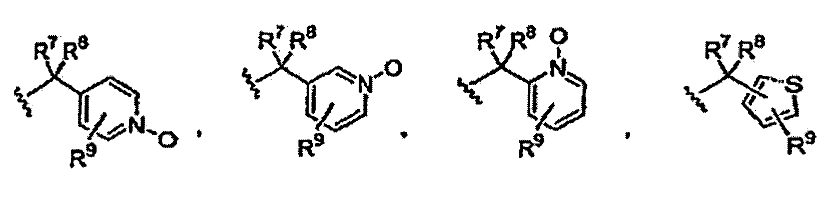 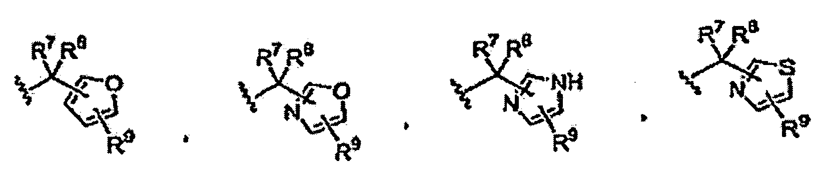 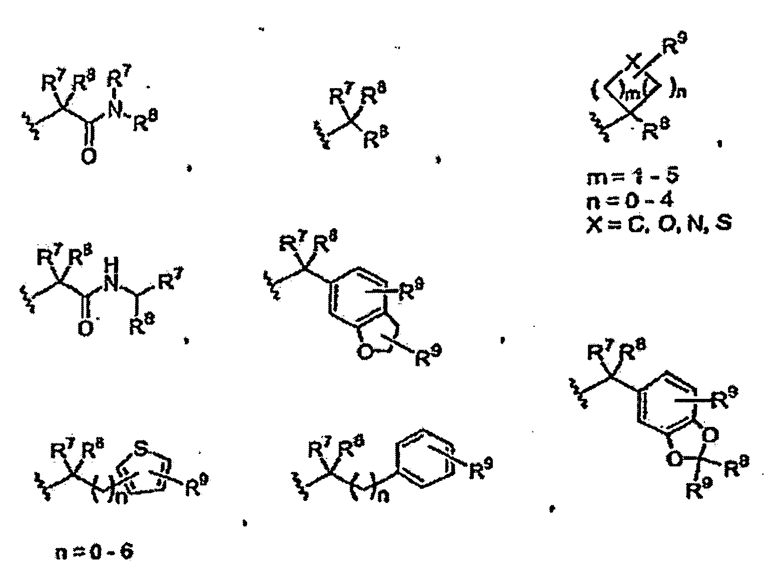 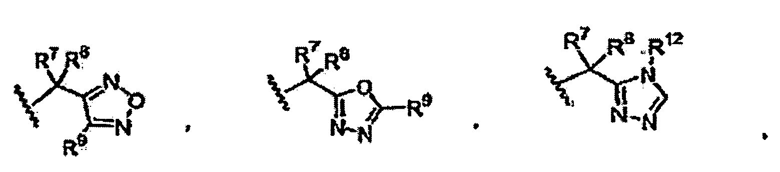 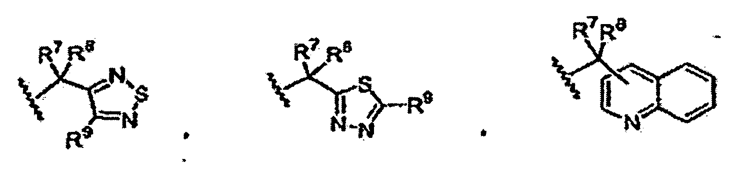 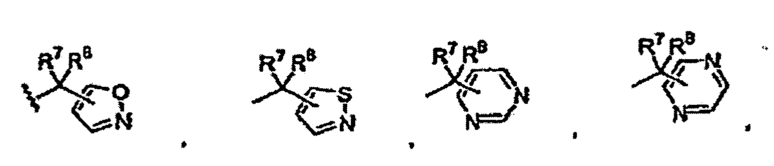 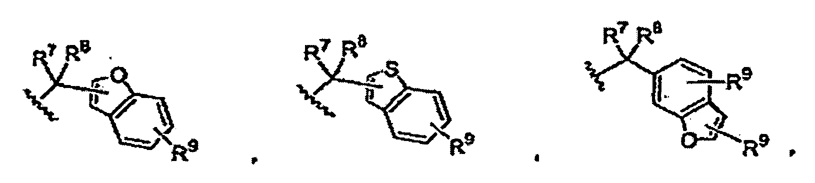 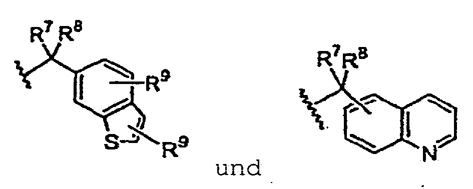 wobei
R⁷ und R⁸ gleich oder verschieden sind und unabhängig ausgewählt sind aus der Gruppe bestehend aus H, gegebenenfalls substituiertem oder unsubstituiertem Alkyl, Aryl, Heteroaryl, Arylalkyl, Heteroarylalkyl, Cycloalkyl, Cycloalkylalkyl, CO₂R¹³, CONR¹³R¹⁴, Fluoralkyl, Alkinyl, Alkenyl, Alkinylalkyl, Alkenylalkyl und Cycloalkenyl,
wobei die Substituenten an den substituierten Gruppen ausgewählt sind aus der Gruppe bestehend aus:
a) Cyano;
b) CO₂R⁷;
c) CONR⁷R⁸;
d) SO₂NR⁷R⁸,
e) SO₂R⁷;
f) NO₂;
g) CF₃;
h) -OR⁷;
i) -NR⁷R⁸;
j) -O(C=O)R⁷;
k) -O(C=O)NR⁷R⁸ und
l) Halogen,
R⁹ ausgewählt ist aus einer oder mehreren der Gruppen bestehend aus:
a) R⁷;
b) R⁸;
c) Halogen;
d) -CF₃;
e) -COR⁷;
f) -OR⁷;
g) -NR⁷R⁸;
h) -NO₂;
i) -CN;
j) -SO₂R⁷;
k) -SO₂NR⁷R⁸;
l) -NR⁷COR⁸;
m) -CONR⁷R⁸;
n) -NR⁷CO₂R⁸;
o) CO₂R⁷ und
p) 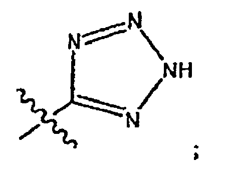
B ist eine gegebenenfalls substituierte Aryl- oder Heteroarylgruppe ausgewählt aus: 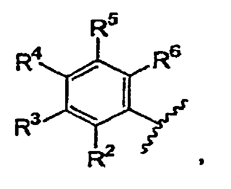 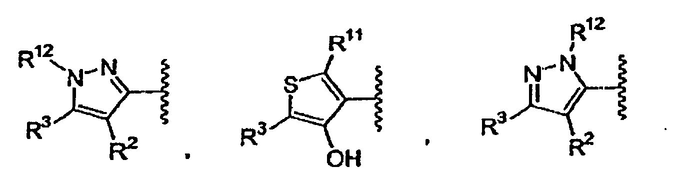 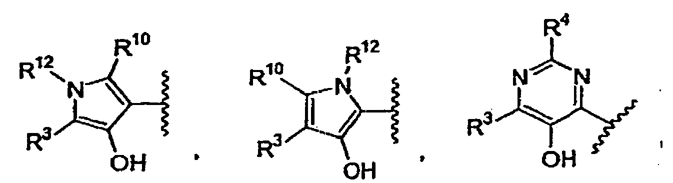 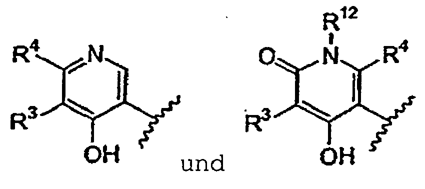 ist,
wobei
R² OH ist;
R³ C(O)NR¹³R¹⁴ oder C(O)NR¹³OR¹⁴ ist,
R⁴ Wasserstoff, Halogen, Alkyl, Alkoxy, OH, CF₃, OCF₃, NO₂, C(O)R¹³, C(O)OR¹³, C(O)NR¹³R¹⁴, SO₍ₜ₎NR¹³R¹⁴, SO₍ₜ₎R¹³, C(O)NR¹³OR¹⁴, 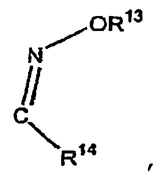 Cyano, gegebenenfalls substituiertes Aryl oder Heteroaryl ist,
wobei die Substituenten an den gegebenenfalls substituierten Gruppen aus einer oder mehreren R⁹-Gruppen ausgewählt sein können;
R⁵ und R⁶ unabhängig für Wasserstoff, Halogen, Alkyl, Alkoxy, CF₃, OCF₃, NO₂, C(O)R¹³, C(O)OR¹³, C(O)NR¹³R¹⁴, SO₍ₜ₎NR¹³R¹⁴, C(O)NR¹³OR¹⁴, Cyano, oder eine gegebenenfalls substituierte Aryl- oder Heteroarylgruppe stehen,
wobei die Substituenten an den gegebenenfalls substituierten Gruppen aus einer oder mehreren R⁹-Gruppen ausgewählt sein können;
R¹⁰ und R¹¹ unabhängig für Wasserstoff, Halogen, -CF₃, -OCF₃, -NR¹³R¹⁴, -NR¹³C(O)NR¹³R¹⁴, -OH, C(O)OR¹³, -SH, -SO₍ₜ₎NR¹³R¹⁴, -SO₂R¹³, -NHC(O)R¹³, -NHSO₂NR¹³R¹⁴, -NHSO₂R¹³, -C(O)NR¹³R¹⁴, -C(O)NR¹³OR¹⁴, -OC(O)R¹³ oder Cyano stehen;
R¹² Wasserstoff, OC(O)R¹³ oder eine gegebenenfalls substituierte Aryl-, Heteroaryl-, Arylalkyl-, Cycloalkyl-, Alkyl-, Cycloalkylalkyl- oder Heteroarylalkylgruppe ist,
R¹³ und R¹⁴ gleich oder verschieden sind und unabhängig ausgewählt sind aus der Gruppe bestehend aus H, gegebenenfalls substituiertem oder unsubstituiertem Alkyl, Aryl, Heteroaryl, Arylalkyl, Heteroarylalkyl, Cycloalkyl, Cycloalkylalkyl und Fluoralkyl, oder
R¹³ und R¹⁴, wenn sie unter Bildung eines gegebenenfalls substituierten, 3- bis 7-gliedrigen heterocyclischen Rings zusammengenommen werden, ein bis zwei Heteroatome ausgewählt aus O, S und N enthalten, und
wobei die Substituenten an den gegebenenfalls substituierten Gruppen aus einer oder mehreren R⁹-Gruppen ausgewählt sein können, und
t 1 oder 2 ist.

7. Verbindung mit der Formel IA: 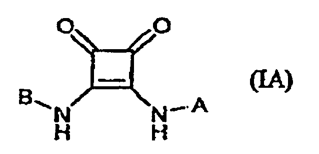 oder ein pharmazeutisch annehmbares Salz oder Solvat davon, worin
A ausgewählt ist aus der Gruppe bestehend aus:
(1) 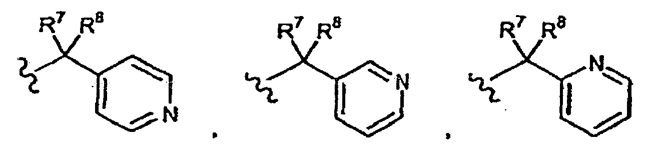 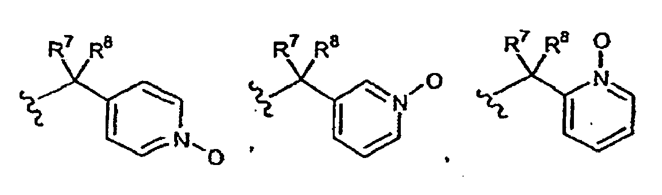 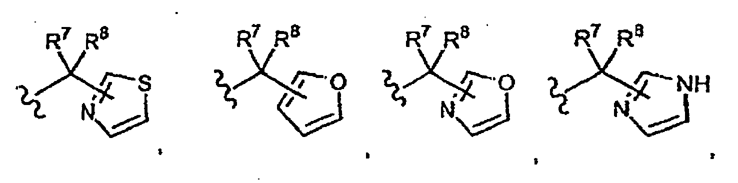 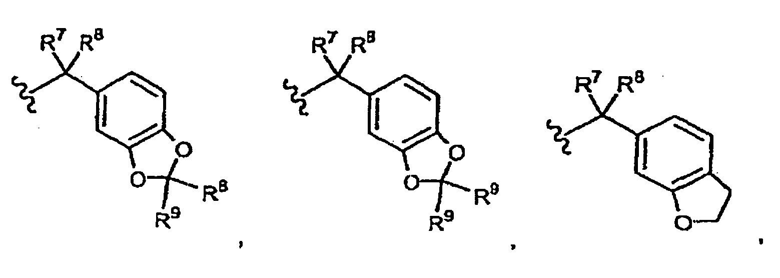 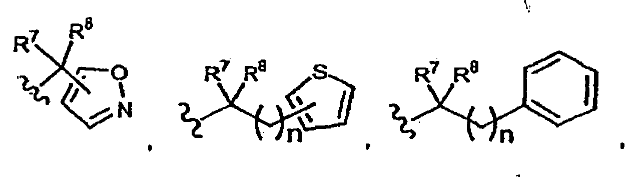 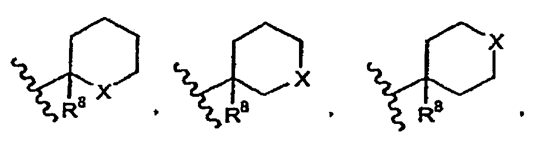 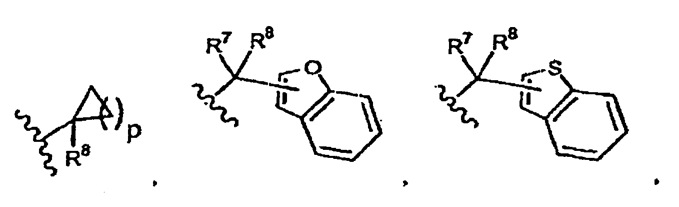 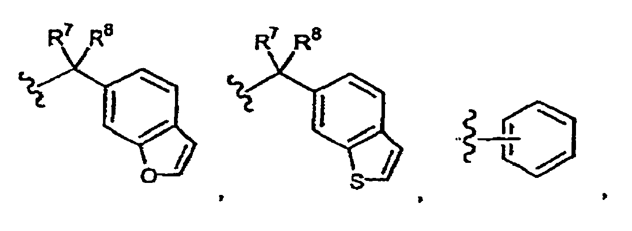 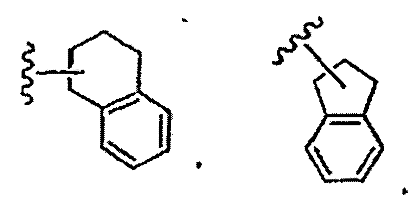 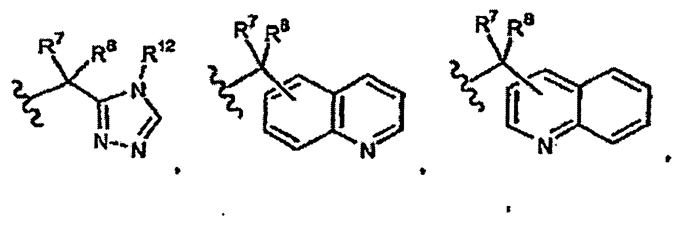 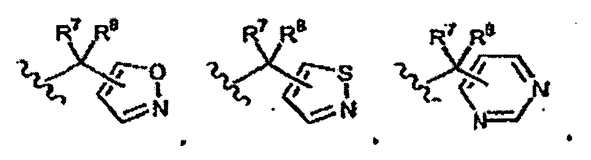 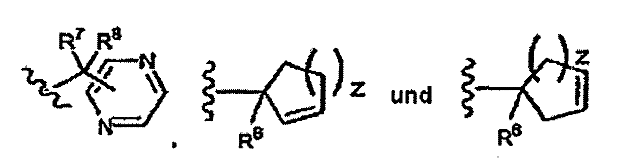
(2) 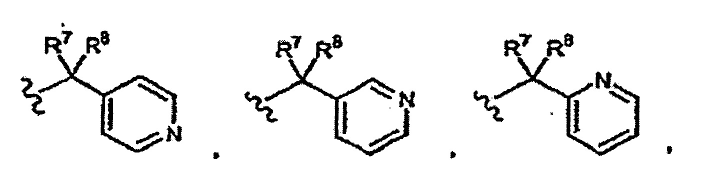 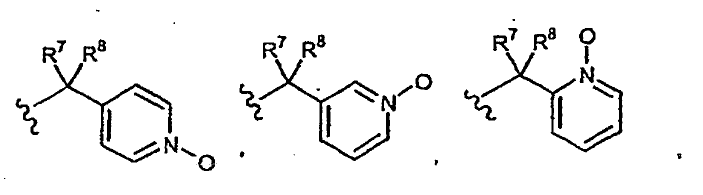 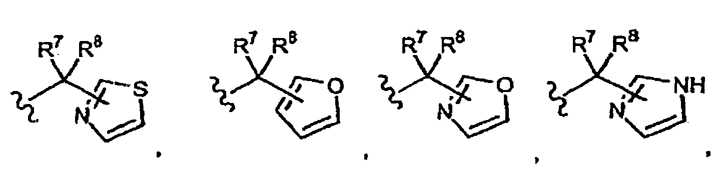 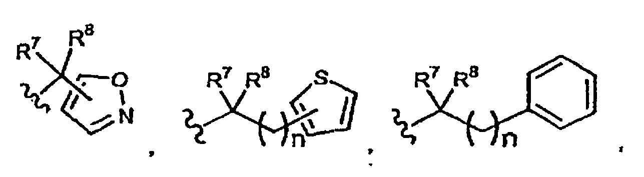 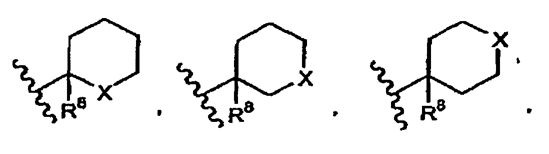 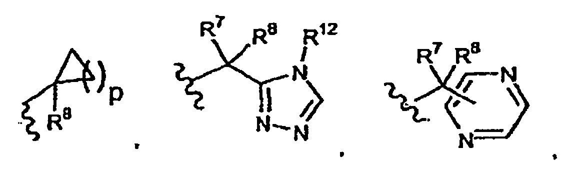 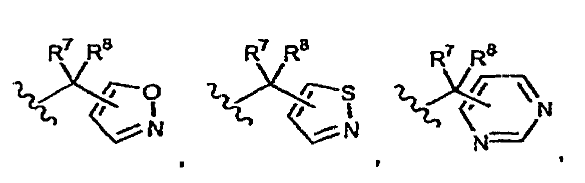 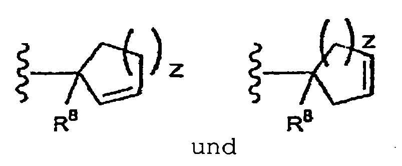 wobei die obigen Ringe der A-Gruppen mit 1 bis 6 Substituenten substituiert sind, die jeweils unabhängig ausgewählt sind aus der Gruppe bestehend aus: R⁹-Gruppen;
(3) 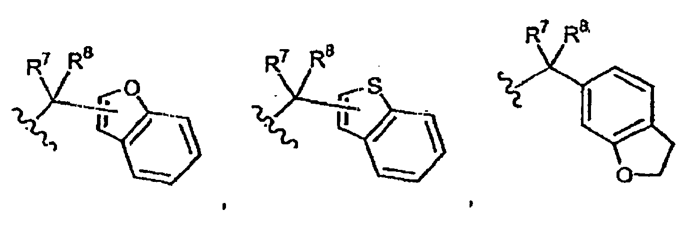 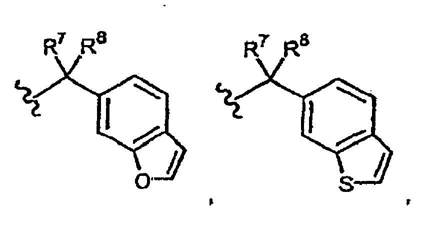 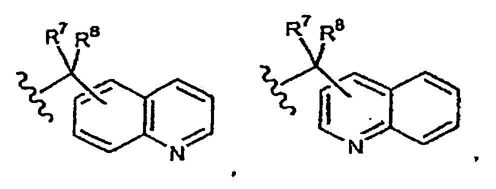 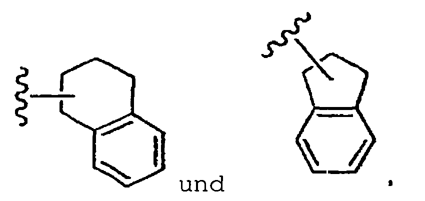 wobei einer oder beide der obigen Ringe der A-Gruppen mit 1 bis 6 Substituenten substituiert sind, die jeweils unabhängig ausgewählt sind aus der Gruppe bestehend aus: R⁹-Gruppen;
(4) 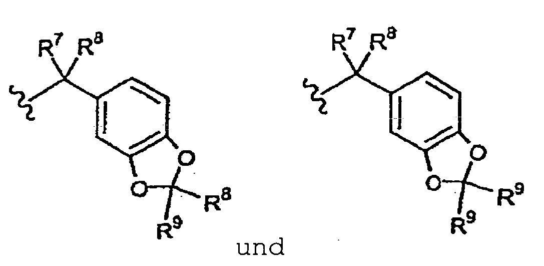 wobei die obigen Phenylringe der A-Gruppen mit 1 bis 3 Substituenten substituiert sind, die jeweils unabhängig ausgewählt sind aus der Gruppe bestehend aus: R⁹-Gruppen, und
(5) 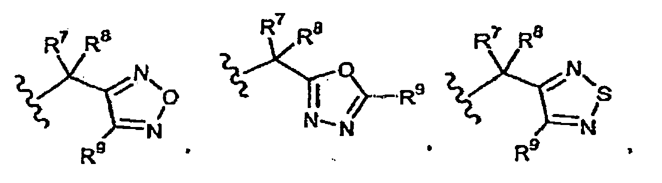 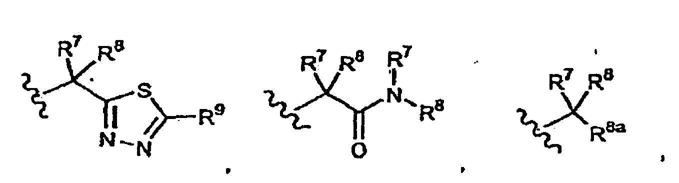 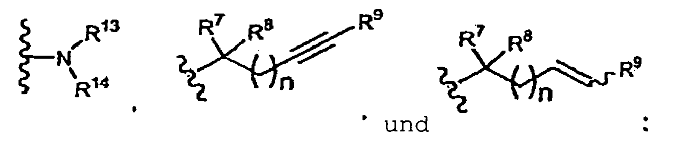
B ausgewählt ist aus der Gruppe bestehend aus: 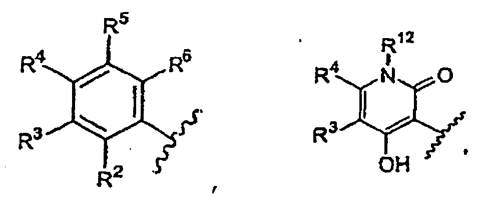 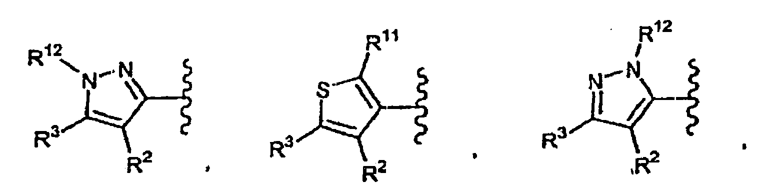 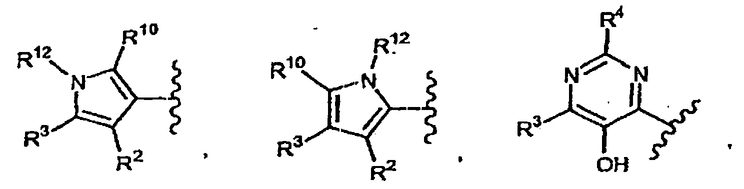 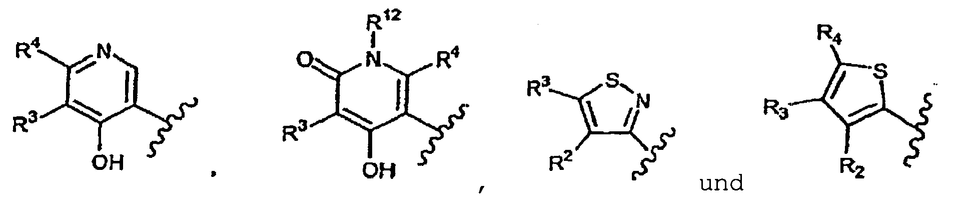 n 0 bis 6 ist,
p 1 bis 5 ist.
X O, NH oder S ist;
Z 1 bis 3 ist,
R² OH ist;
R³ ausgewählt ist aus der Gruppe bestehend aus -C(O)NHR¹⁷, -C(O)NR¹³R¹⁴ und -C(O)NR¹³OR¹⁴;
R⁴ ausgewählt ist aus der Gruppe bestehend aus Wasserstoff, Cyano, Halogen, Alkyl, Alkoxy, -OH, -CF₃, -OCF₃, -NO₂, -C(O)R¹³, -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, unsubstituiertem oder substituiertem Aryl, unsubstituiertem oder substituiertem Heteroaryl, 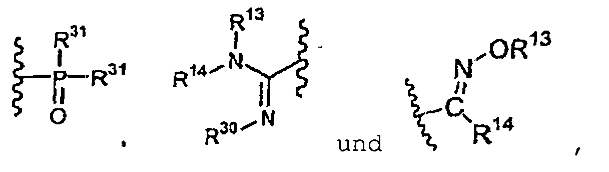 wobei sich an der substituierten Arylgruppe 1 bis 6 Substituenten befinden und jeder Substituent unabhängig ausgewählt ist aus der Gruppe bestehend aus: R⁹-Gruppen, und wobei sich an der substituierten Heteroarylgruppe 1 bis 6 Substituenten befinden und jeder Substituent unabhängig ausgewählt ist aus der Gruppe bestehend aus: R⁹-Gruppen;
jedes R⁵ und R⁶ gleich oder unterschiedlich ist, und sie unabhängig ausgewählt sind aus der Gruppe bestehend aus Wasserstoff, Halogen, Alkyl, Alkoxy, -CF₃, -OCF₃, -NO₂, -C(O)R¹³, -C(O)OR¹³, -C(O)NR¹³R¹⁴, -SO₍ₜ₎NR¹³R¹⁴, -C(O)NR¹³OR¹⁴, Cyano, unsubstituierter oder substituierter Aryl- und unsubstituierter oder substituierter Heteroarylgruppe, wobei sich an der substituierten Arylgruppe 1 bis 6 Substituenten befinden und jeder Substituent unabhängig ausgewählt ist aus der Gruppe bestehend aus: R⁹-Gruppen, und wobei sich an der substituierten Heteroarylgruppe 1 bis 6 Substituenten befinden und jeder Substituent unabhängig ausgewählt ist aus der Gruppe bestehend aus: R⁹-Gruppen;
jedes R⁷ und R⁸ unabhängig ausgewählt ist aus der Gruppe bestehend aus: H, unsubstituiertem oder substituiertem Alkyl, unsubstituiertem oder substituiertem Aryl, unsubstituiertem oder substituiertem Heteroaryl, unsubstituiertem oder substituiertem Arylalkyl, unsubstituiertem oder substituiertem Heteroarylalkyl, unsubstituiertem oder substituiertem Cycloalkyl, unsubstituiertem oder substituiertem Cycloalkylalkyl, -CO₂R¹³, -CONR¹³R¹⁴, Alkinyl, Alkenyl und Cycloalkenyl, und wobei sich an den substituierten R⁷- und R⁸-Gruppen ein oder mehrere Substituenten befinden, wobei jeder Substituent unabhängig ausgewählt ist aus der Gruppe bestehend aus
a) Halogen,
b) -CF₃,
c) -COR¹³,
d) -OR¹³,
e) -NR¹³R¹⁴,
f) -NO₂,
g) -CN,
h) -SO₂OR¹³,
i) -Si(alkyl)₃, wobei jedes Alkyl unabhängig ausgewählt ist,
j) -Si(aryl)₃, wobei jedes Aryl unabhängig ausgewählt ist,
k) - (R¹³) ₂R¹⁴Si, wobei jedes R¹³ unabhängig ausgewählt ist,
l) -CO₂R¹³,
m) -C(O)NR¹³R¹⁴,
n) -SO₂NR¹³R¹⁴,
o) -SO₂R¹³,
p) -OC (O) R¹³,
q) -OC(O)NR¹³R¹⁴,
r) -NR¹³C(O)R¹⁴, und
s) -NR¹³CO₂R¹⁴;
R^{8a} ausgewählt ist aus der Gruppe bestehend aus Wasserstoff, Alkyl, Cycloalkyl und Cycloalkylalkyl;
jedes R⁹ unabhängig ausgewählt sind aus der Gruppe bestehend aus:
a) -R¹³,
b) Halogen,
c) -CF₃,
d) -COR¹³,
e) -OR¹³,
f) -NR¹³R¹⁴,
g) -NO₂,
h) -CN,
i) -SO₂R¹³,
j) -SO₂NR¹³R¹⁴,
k) -NR¹³COR¹⁴,
l) -CONR¹³R¹⁴,
m) -NR¹³CO₂R¹⁴,
n) -CO₂R¹³,
o) 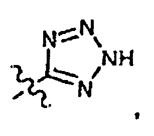
p) Alkyl, das mit einer oder mehreren (z. B. einer) -OH Gruppe(n) substituiert ist (z. B. -(CH₂)_{q}OH, wobei q 1 bis 6, üblicherweise 1 bis 2 und vorzugsweise 1 ist),
q) Alkyl, das mit einer oder mehreren (z. B. einer) -NR¹³R¹⁴ Gruppe (n) substituiert ist (z. B. -(CH₂)_{q}NR¹³R¹⁴, wobei q 1 bis 6, üblicherweise 1 bis 2 und vorzugsweise 1 ist), und
r) -N (R¹³)SO₂R¹⁴ (z. B. ist R¹³ H und R¹⁴ Alkyl, wie Methyl);
jedes R¹⁰ und R¹¹ unabhängig ausgewählt ist aus der Gruppe bestehend aus Wasserstoff, Alkyl (z. B. C₁ bis C₆, wie Methyl), Halogen, -CF₃, -OC_{F3}, -NR¹³R¹⁴, -NR¹³C(O)NR¹³R¹⁴, -OH, -C(O)OR¹³, -SH, -SO₍ₜ₎NR¹³R¹⁴, -SO₂R¹³, -NHC(O)R¹³, -NHSO₂NR¹³R¹⁴, -NHSO₂R¹³, -C(O)NR¹³R¹⁴, -C(O)NR¹³OR¹⁴, -OC(O)R¹³ und Cyano;
R¹² ausgewählt ist aus der Gruppe bestehend aus: Wasserstoff, -C(O)OR¹³, unsubstituierter oder substituierter Aryl-, unsubstituierter oder substituierter Heteroaryl-, unsubstituierter oder substituierter Arylalkyl-, unsubstituierter oder substituierter Cycloalkyl-, unsubstituierter oder substituierter Alkyl-, unsubstituierter oder substituierter Cycloalkylalkyl- und unsubstituierter oder substituierter Heteroarylalkylgruppe, wobei sich an den substituierten R¹²-Gruppen 1 bis 6 Substituenten befinden und jeder Substituent unabhängig ausgewählt ist aus der Gruppe bestehend aus: R⁹-Gruppen;
jedes R¹³ und R¹⁴ unabhängig ausgewählt ist aus der Gruppe bestehend aus: H, unsubstituiertem oder substituiertem Alkyl, unsubstituiertem oder substituiertem Aryl, unsubstituiertem oder substituiertem Heteroaryl, unsubstituiertem oder substituiertem Arylalkyl, unsubstituiertem oder substituiertem Heteroarylalkyl, unsubstituiertem oder substituiertem Cycloalkyl, unsubstituiertem oder substituiertem Cycloalkylalkyl, unsubstituiertem oder substituiertem Heterocyclus, unsubstituiertem oder substituiertem Fluoralkyl und unsubstituiertem oder substituiertem Heterocycloalkylalkyl, wobei sich 1 bis 6 Substituenten an den substituierten R¹³- und R¹⁴-Gruppen befinden und jeder Substituent unabhängig ausgewählt ist aus der Gruppe bestehend aus: Alkyl, -CF₃, -OH, Alkoxy, Aryl, Arylalkyl, Fluoralkyl, Cycloalkyl, Cycloalkylalkyl, Heteroaryl, Heteroarylalkyl, -N(R⁴⁰)₂, -C(O)OR¹⁵, - C(O)NR¹⁵R¹⁶, -S(O)ₜNR¹⁵R¹⁶, -C(O)R¹⁵, -SO₂R¹⁵ mit der Maßgabe, dass R¹⁵ nicht H, Halogen und -NHC(O)NR¹⁵R¹⁶ ist; oder R¹³ und R¹⁴ zusammen mit dem Stickstoff, an den sie in den Gruppe -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴, -NHSO₂NR¹³R¹⁴ gebunden sind, einen unsubstituierten oder substituierten gesättigten heterocyclischen Ring bilden, wobei der Ring gegebenenfalls ein weiteres Heteroatom ausgewählt aus der Gruppe bestehend aus O, S und NR¹⁸ enthält, wobei sich an den substituierten cyclisierten R¹³- und R¹⁴-Gruppen 1 bis zu 3 Substituenten befinden und jeder Substituent unabhängig ausgewählt ist aus der Gruppe bestehend aus: Alkyl, Aryl, Hydroxy, Hydroxyalkyl, Alkoxy, Alkoxyalkyl, Arylalkyl, Fluoralkyl, Cycloalkyl, Cycloalkylalkyl, Heteroaryl, Heteroarylalkyl, Amino, -C(O)OR¹⁵, -C(O)NR¹⁵R¹⁶, -SOₜNR¹⁵R¹⁶, -C(O)R¹⁵, -SO₂R¹⁵ mit der Maßgabe, dass R¹⁵ nicht H, -NHC(O)NR¹⁵R¹⁶, NHC(O)OR¹⁵, Halogen und eine Heterocylcoalkenylgruppe ist;
jedes R¹⁵ und R¹⁶ unabhängig ausgewählt ist aus der Gruppe bestehend aus: H, Alkyl, Aryl, Arylalkyl, Cycloalkyl und Heteroaryl;
R¹⁷ ausgewählt ist aus der Gruppe bestehend aus: -SO₂alkyl, -SO₂aryl, -SO₂cycloalkyl und -SO₂heteroaryl;
R¹⁸ ausgewählt ist aus der Gruppe bestehend aus: H, Alkyl, Aryl, Heteroaryl, -C(O)R¹⁹, -SO₂R¹⁹ und -C(O)NR¹⁹R²⁰;
jedes R¹⁹ und R²⁰ unabhängig ausgewählt ist aus der Gruppe bestehend aus: Alkyl, Aryl und Heteroaryl;
R³⁰ ausgewählt ist aus der Gruppe bestehend aus: Alkyl, Cycloalkyl, -CN, -NO₂ oder -SO₂R¹⁵ mit der Maßgabe, dass R¹⁵ nicht H ist;
jedes R³¹ unabhängig ausgewählt ist aus der Gruppe bestehend aus: unsubstituiertem oder substituiertem Alkyl, unsubstituiertem oder substituiertem Aryl, unsubstituiertem oder substituiertem Heteroaryl und unsubstituiertem oder substituiertem Cycloalkyl, wobei 1 bis 6 Substituenten an den substituierten R³¹-Gruppen vorhanden sind und jeder Substituent unabhängig ausgewählt ist aus: R⁹-Gruppen;
jedes R⁴⁰ unabhängig ausgewählt ist aus der Gruppe bestehend aus: H, Alkyl und Cycloalkyl, und
t 0, 1 oder 2 ist.

8. Verbindung nach Anspruch 7, bei der B ausgewählt ist aus der Gruppe bestehend aus:
(1) 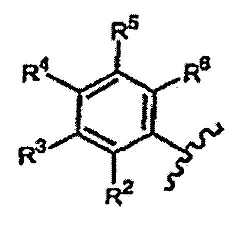 wobei R³ -C(O)NR¹³R¹⁴ ist;
und alle anderen Substituenten wie für Formel IA in Anspruch 7 definiert sind;
(2) 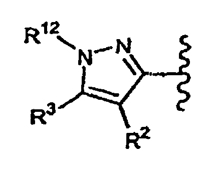 wobei alle Substituenten wie für Formel IA in Anspruch 7 definiert sind;
(3) 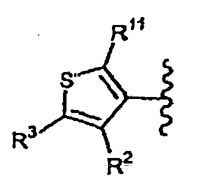 wobei alle Substituenten wie für Formel IA in Anspruch 7 definiert sind;
(4) 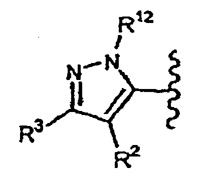 wobei alle Substituenten wie für Formel IA in Anspruch 7 definiert sind;
(5) 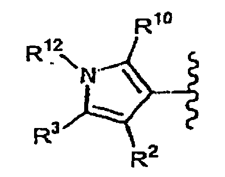 wobei alle Substituenten wie für Formel IA in Anspruch 7 definiert sind;
(6) 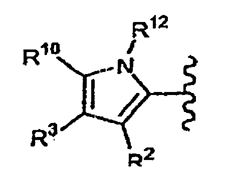 wobei alle Substituenten wie für Formel IA in Anspruch 7 definiert sind;
(7) 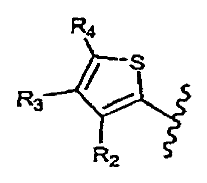 wobei alle Substituenten wie für Formel IA in Anspruch 7 definiert sind;
(8) 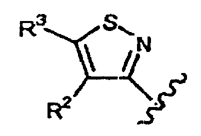 wobei alle Substituenten wie für Formel IA in Anspruch 7 definiert sind;
(9) 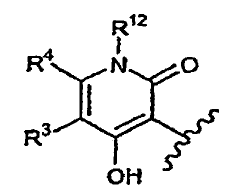 wobei alle Substituenten wie für Formel IA in Anspruch 7 definiert sind;
(10) 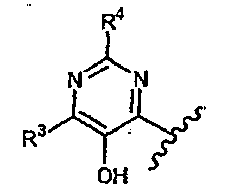 wobei alle Substituenten wie für Formel IA in Anspruch 7 definiert sind;
(12) 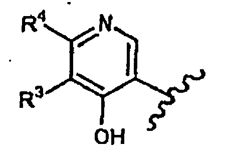 wobei alle Substituenten wie für Formel IA in Anspruch 7 definiert sind; und
(13) 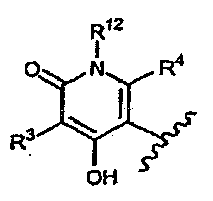 wobei alle Substituenten wie für Formel IA in Anspruch 7 definiert sind.

9. Verbindung nach Anspruch 7, bei der B 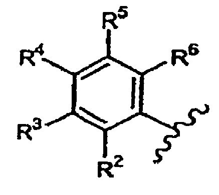 ist und
R³-C(O)NR¹³R¹⁴ ist.

10. Verbindung nach Anspruch 9, bei der R¹³ und R¹⁴ jeweils die gleichen oder verschiedene Alkylgruppen sind.

11. Verbindung nach Anspruch 7, bei der B 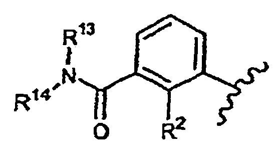 ist.

12. Verbindung nach Anspruch 11, bei der R¹³ und R¹⁴ die gleichen oder verschiedene Alkylgruppen sind.

13. Verbindung nach Anspruch 7, bei der B 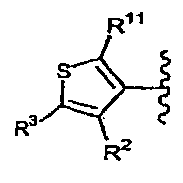 ist.

14. Verbindung nach Anspruch 13, bei der R¹¹ H ist.

15. Verbindung nach Anspruch 13 oder 14, bei der R³ -C(O)NR¹³R¹⁴ ist.

16. Verbindung nach einem der Ansprüche 13 bis 15, bei der R¹³ und R¹⁴ unabhängig ausgewählt sind aus der Gruppe bestehend aus: Alkyl, unsubstituiertem Heteroaryl und substituiertem Heteroaryl.

17. Verbindung nach Anspruch 16, bei der einer von R¹³ und R¹⁴ Alkyl ist.

18. Verbindung nach Anspruch 17, bei der das Alkyl Methyl ist.

19. Verbindung nach Anspruch 7, bei der B 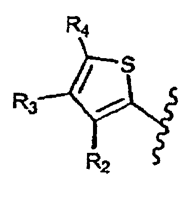 ist.

20. Verbindung nach einem der Ansprüche 7 bis 19, bei der A 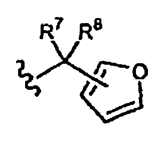 ist,
wobei der Furanring unsubstituiert oder substituiert ist.

21. Verbindung nach Anspruch 20, bei der der Furanring substituiert ist.

22. Verbindung nach Anspruch 21, bei der der Furanring mit mindestens einer Alkylgruppe substituiert ist.

23. Verbindung nach einem der Ansprüche 20 bis 22, bei der R⁷ und R⁸ gleich oder verschieden sind und jeweils unabhängig ausgewählt sind aus der Gruppe bestehend aus: H und Alkyl.

24. Verbindung nach Anspruch 23, bei der R⁷ H ist und R⁸ Alkyl ist.

25. Verbindung nach Anspruch 1 ausgewählt aus der Gruppe bestehend aus

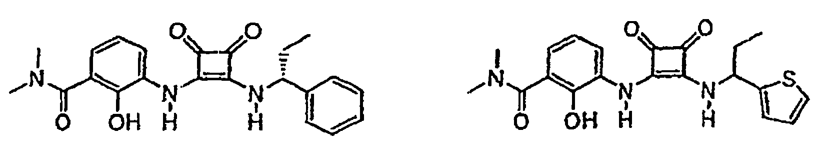

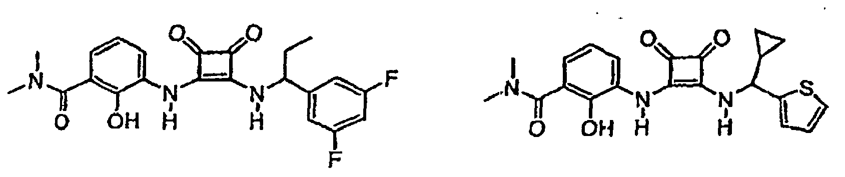

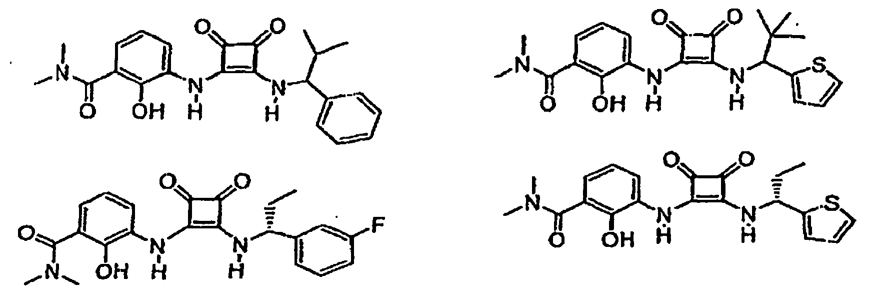

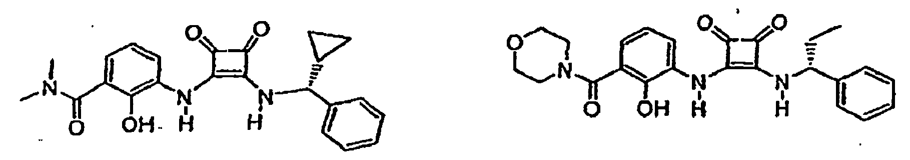

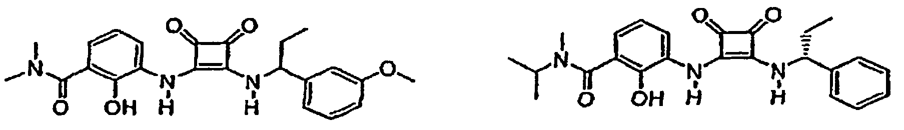

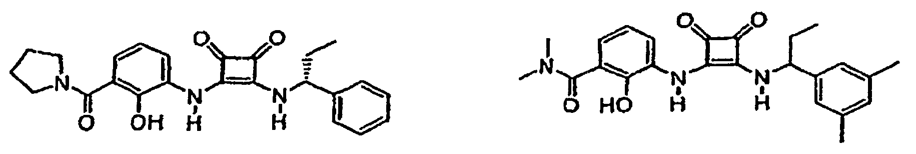

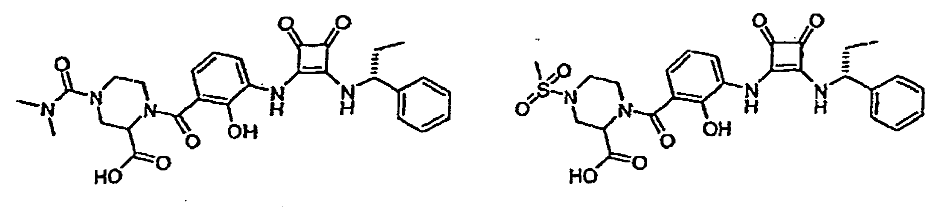

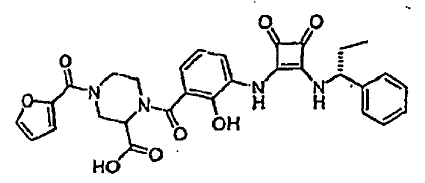

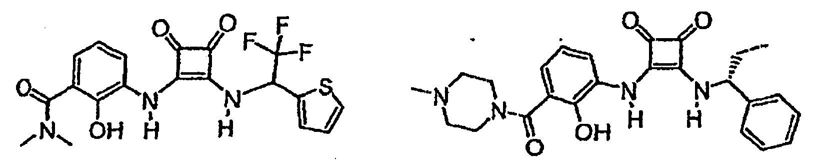

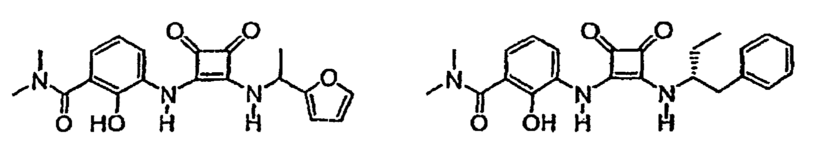

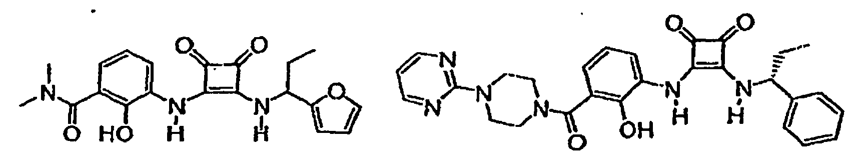

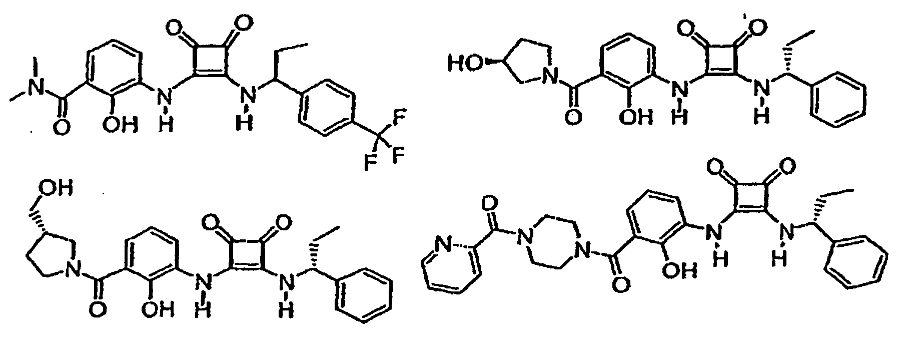

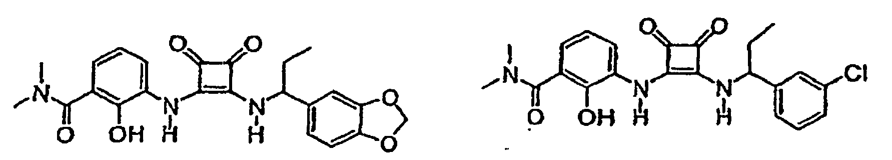

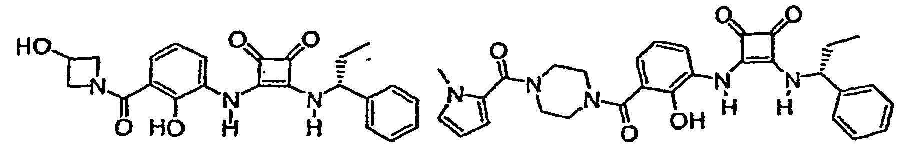

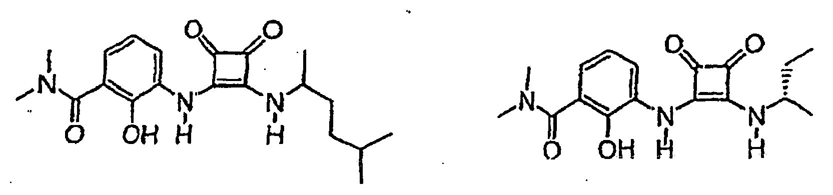

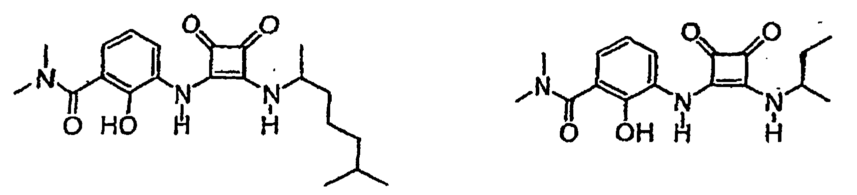

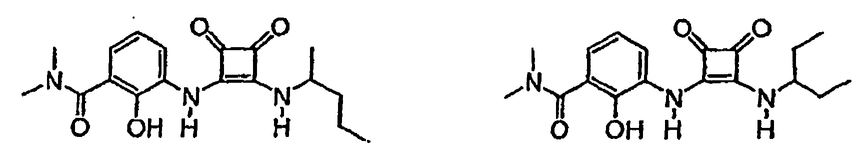

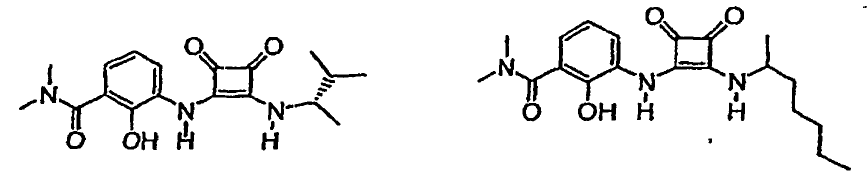

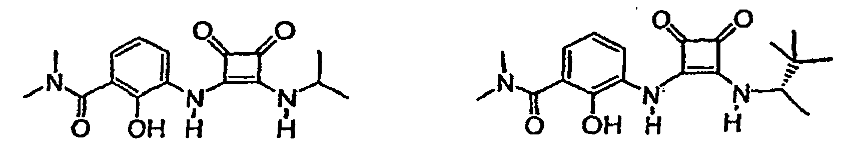

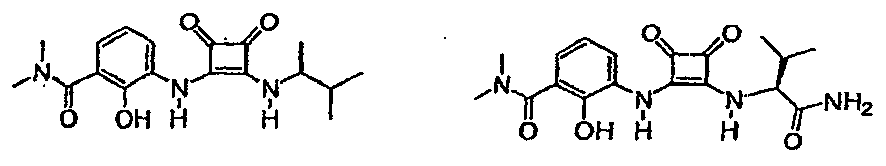

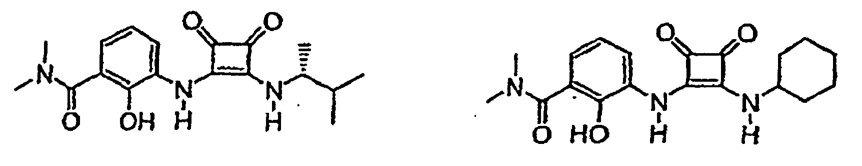

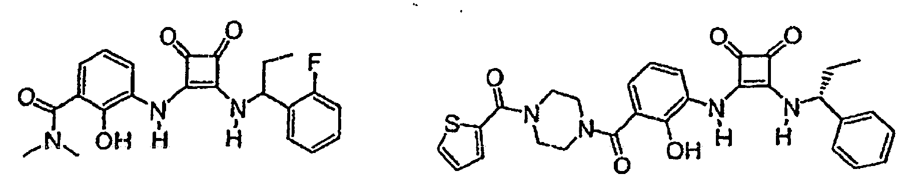

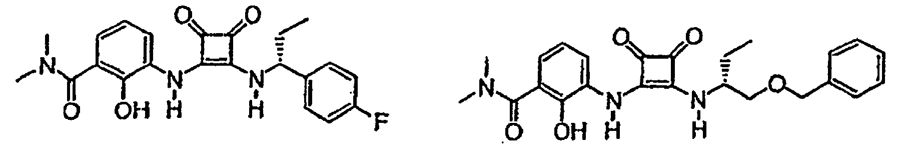

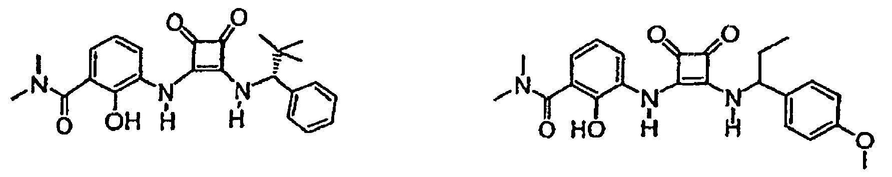

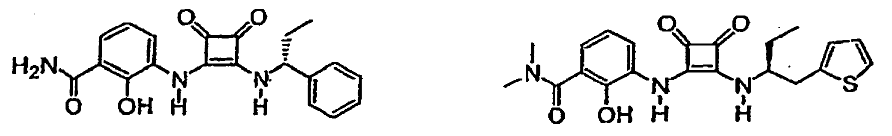

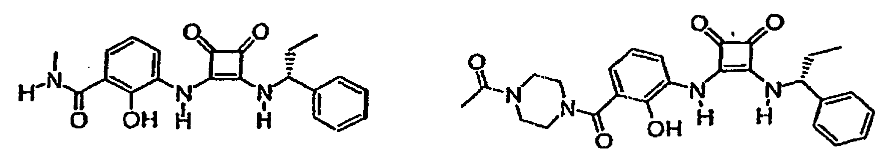

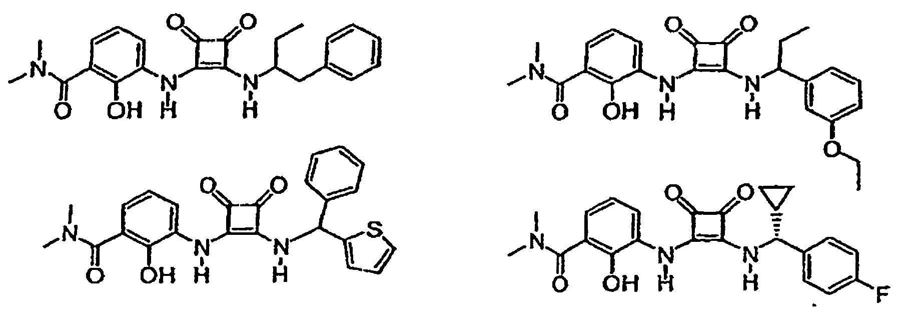

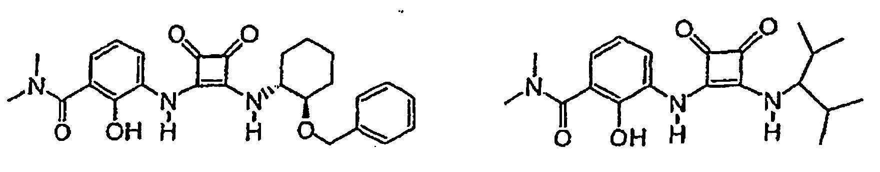

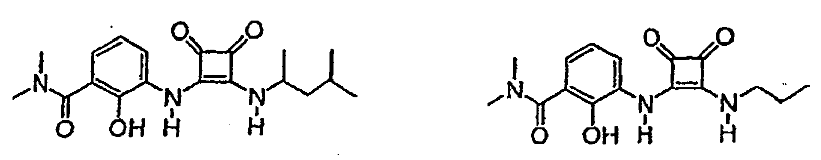

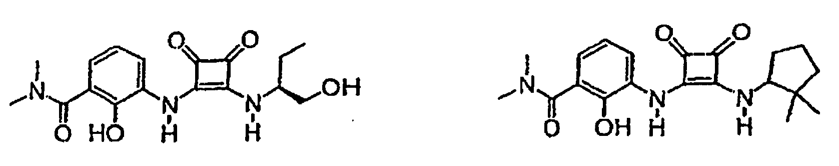

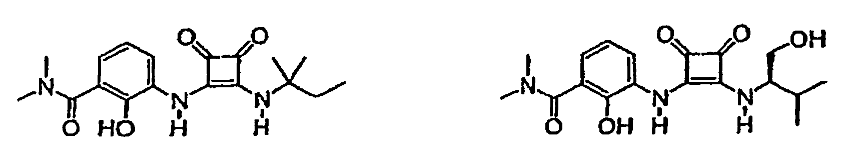

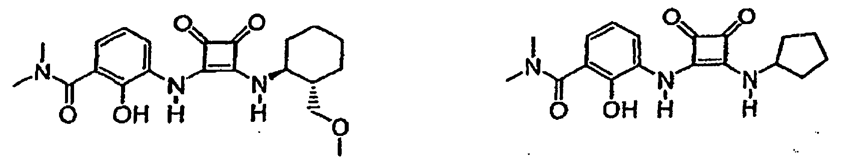

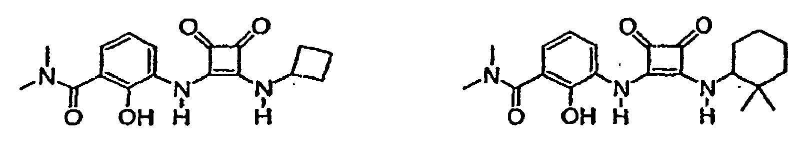

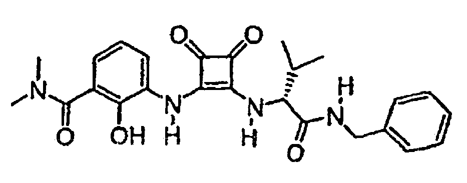

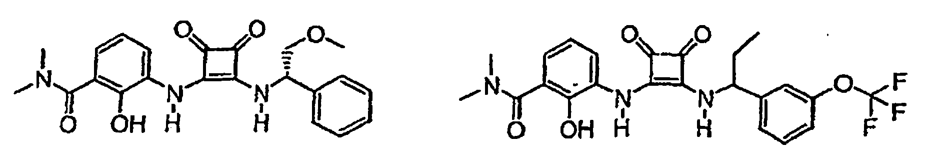

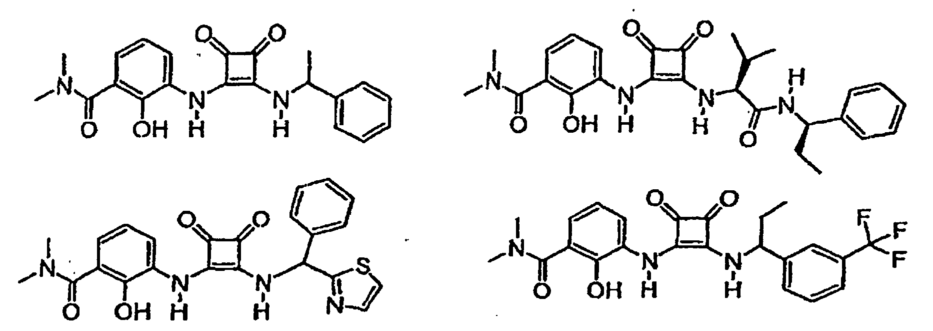

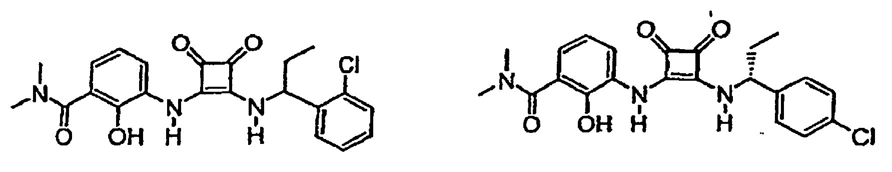

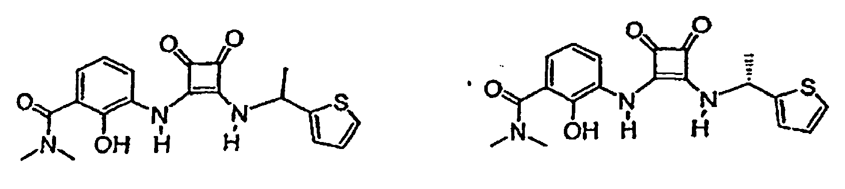

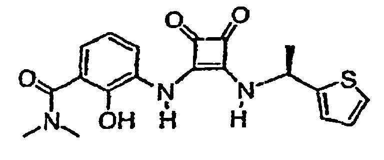

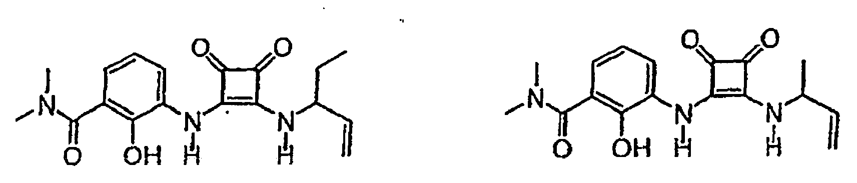

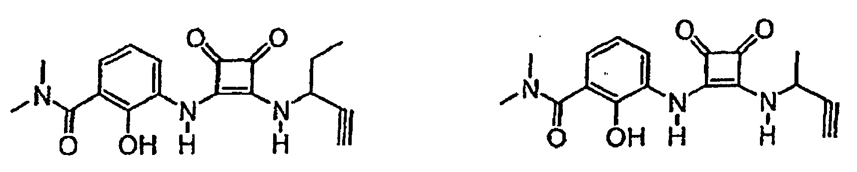

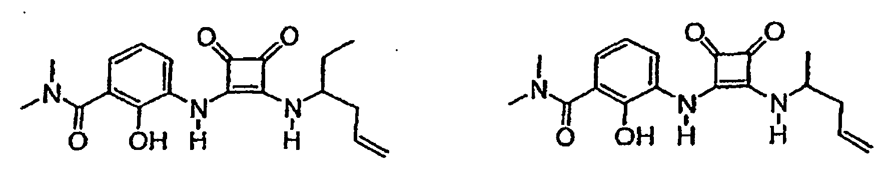

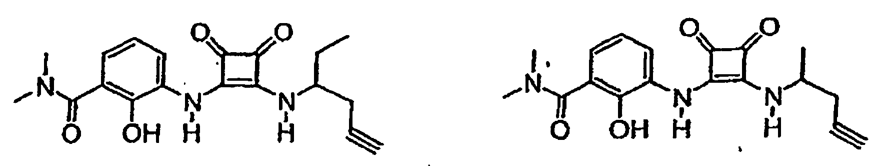

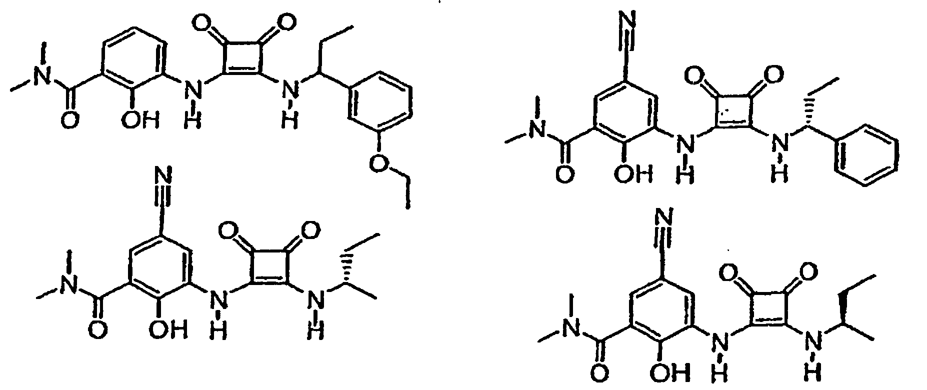

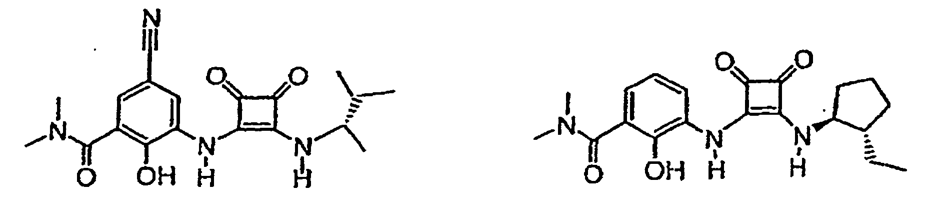

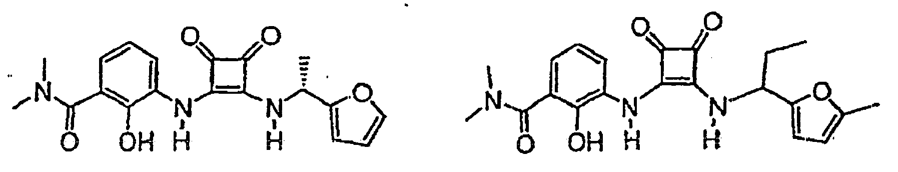

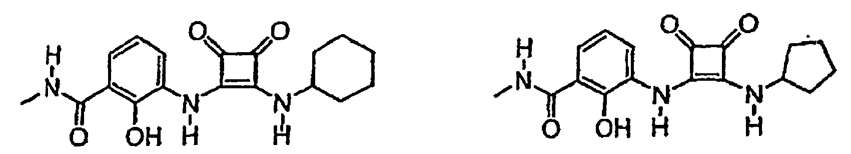

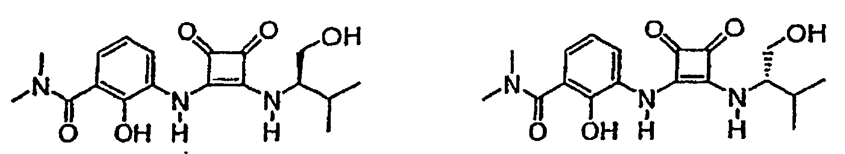

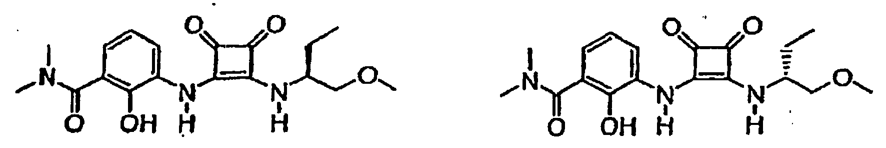

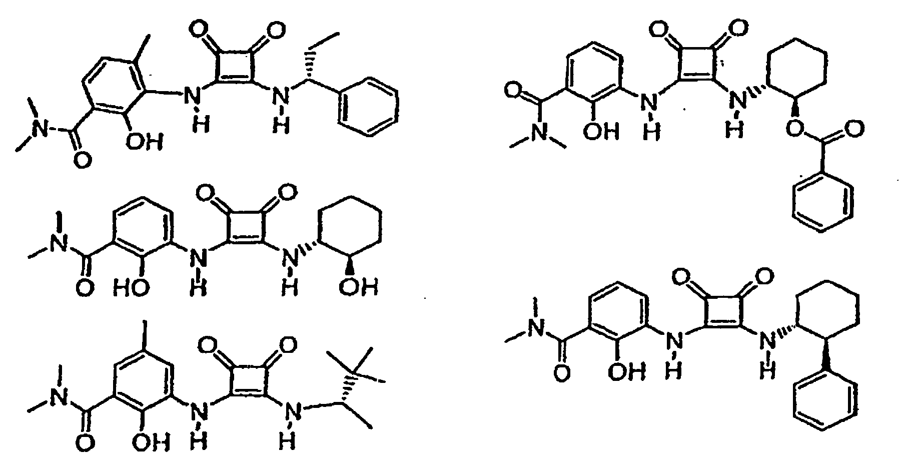

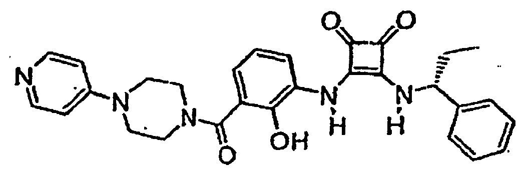

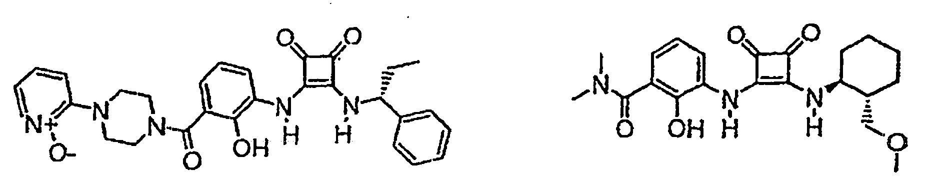

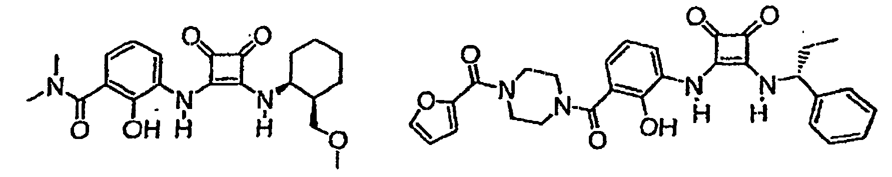

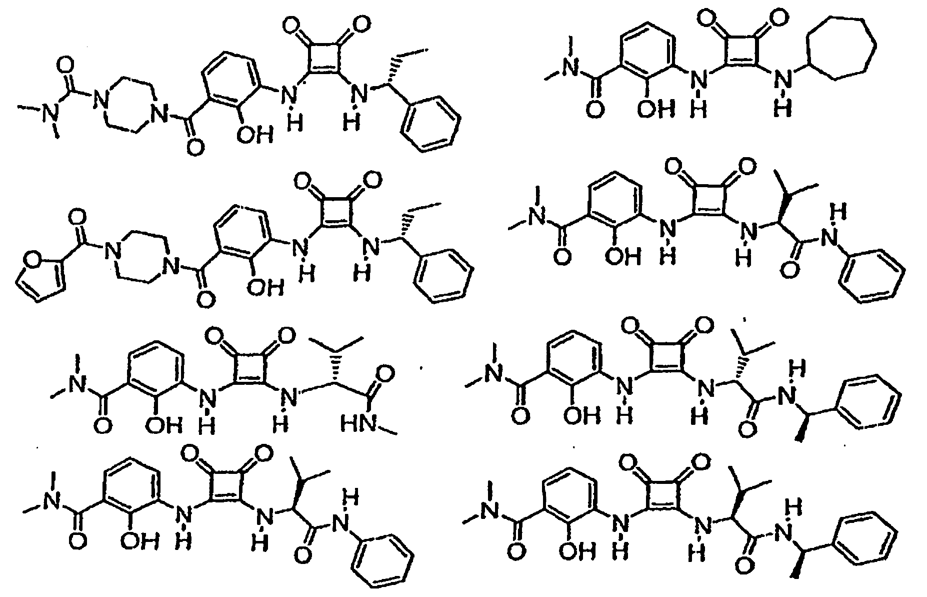

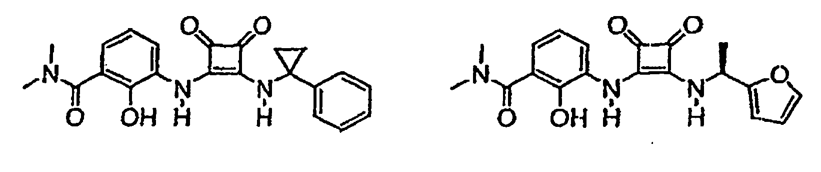

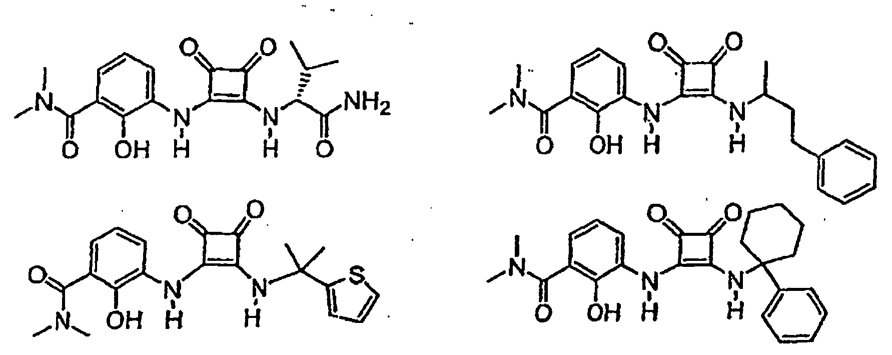

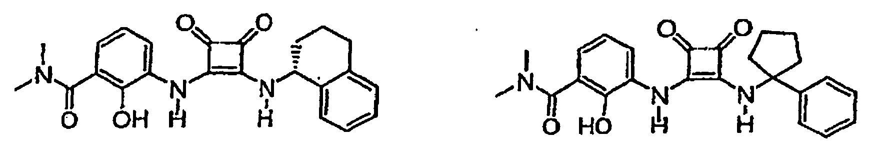

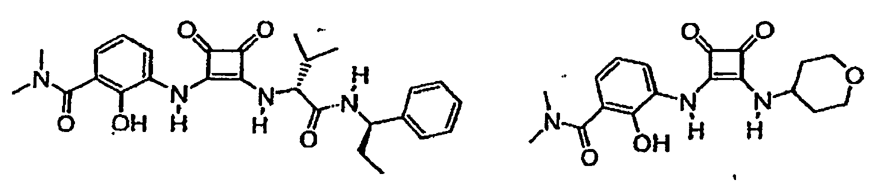

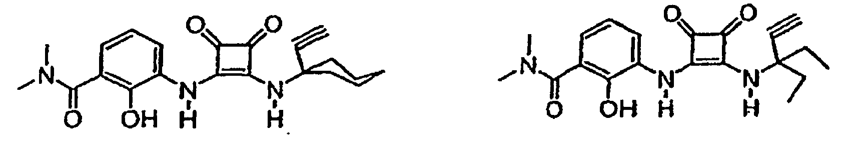

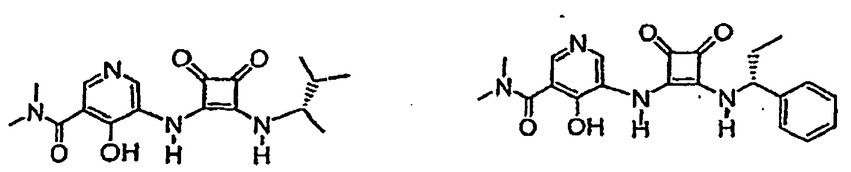

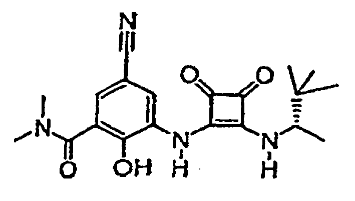

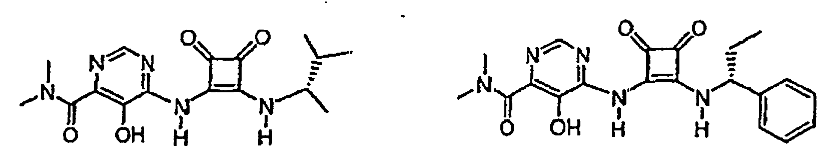

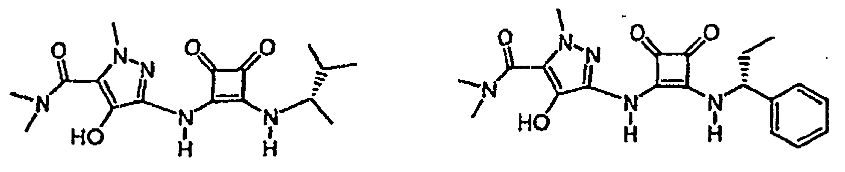

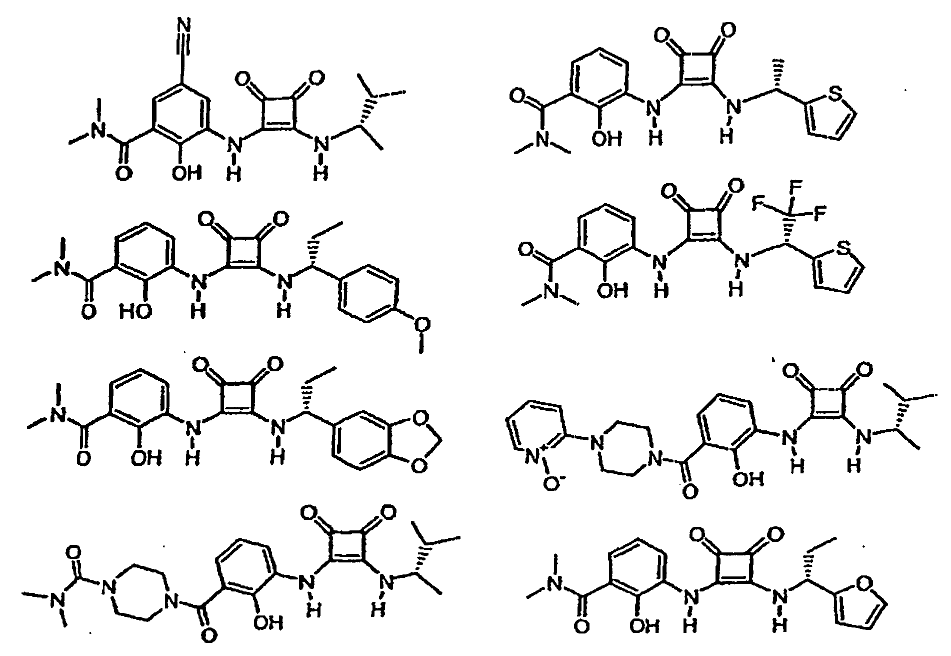

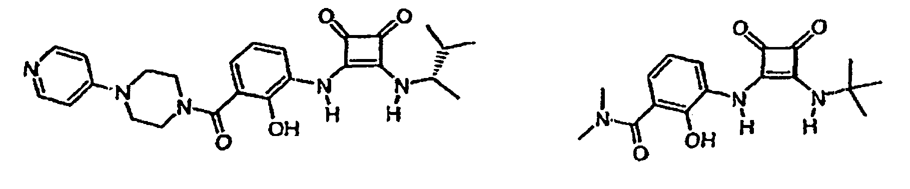

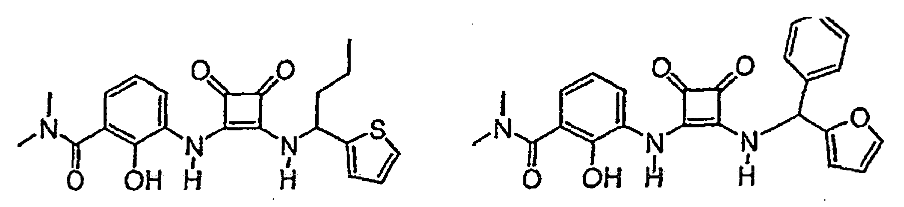

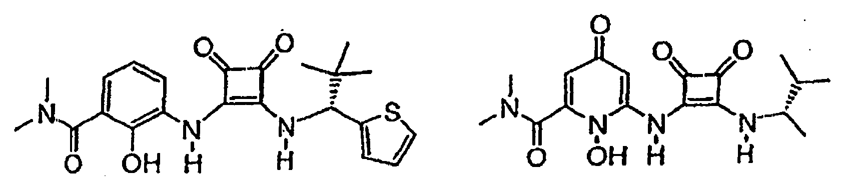

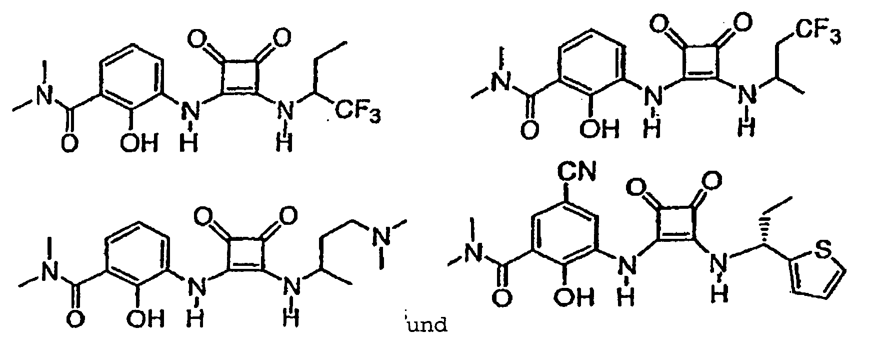

26. Verbindung nach Anspruch 1 ausgewählt aus der Gruppe bestehend aus

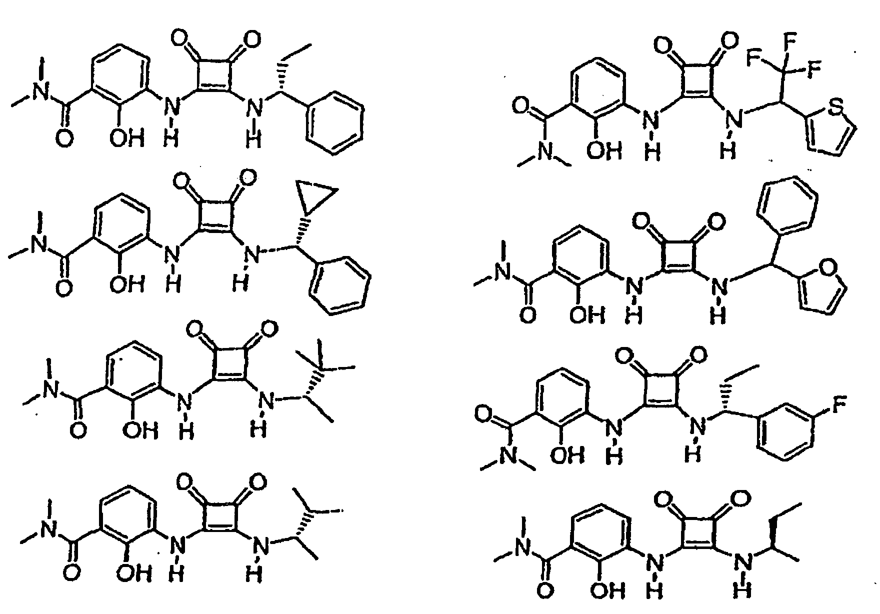

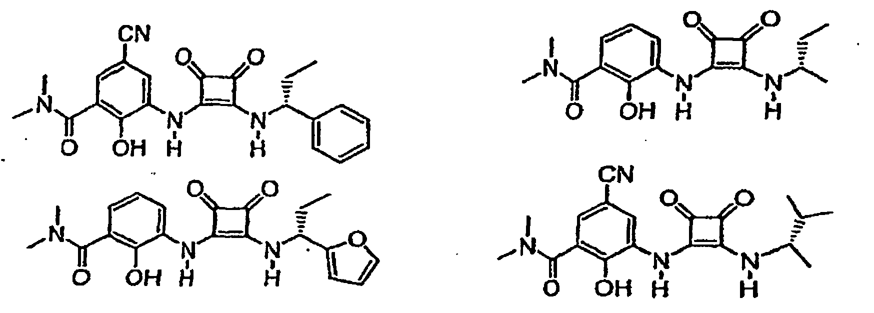

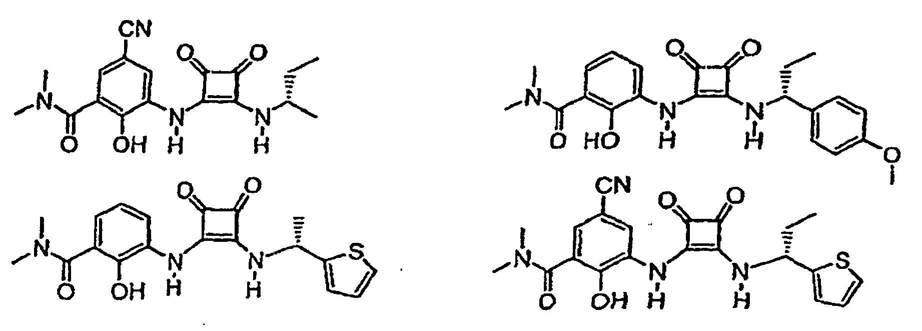

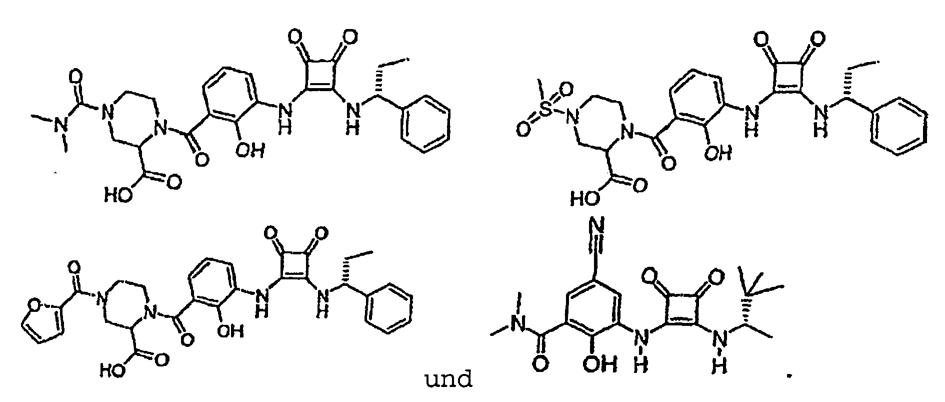

27. Verbindung mit der Formel: 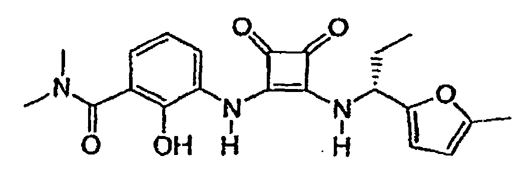 oder ein pharmazeutisch annehmbares Salz oder Solvat davon.

28. Verbindung mit der Formel: 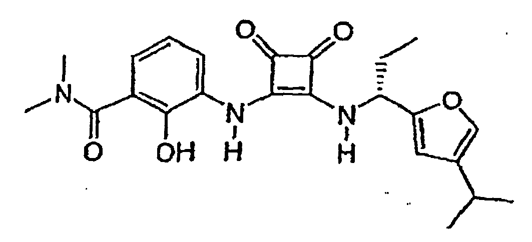 oder ein pharmazeutisch annehmbares Salz oder Solvat davon.

29. Verbindung mit der Formel: 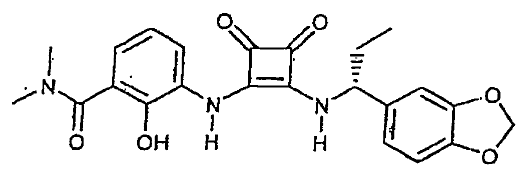 oder ein pharmazeutisch annehmbares Salz oder Solvat davon.

30. Verbindung mit der Formel: 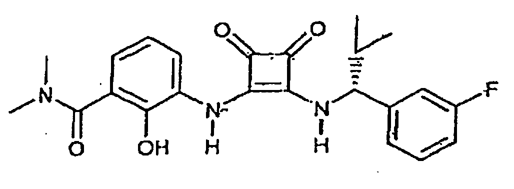 oder ein pharmazeutisch annehmbares Salz oder Solvat davon.

31. Verbindung mit der Formel: 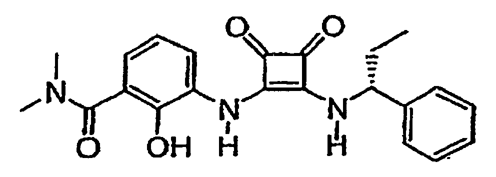 oder ein pharmazeutisch annehmbares Salz oder Solvat davon.

32. Verbindung mit der Formel: 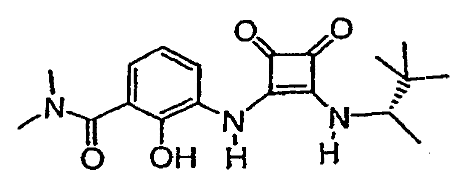 oder ein pharmazeutisch annehmbares Salz oder Solvat davon.

33. Verbindung mit der Formel: 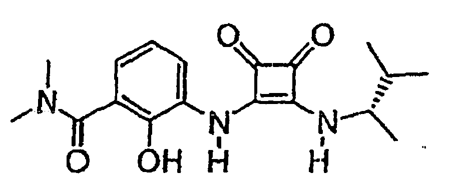 oder ein pharmazeutisch annehmbares Salz oder Solvat davon.

34. Verbindung mit der Formel: 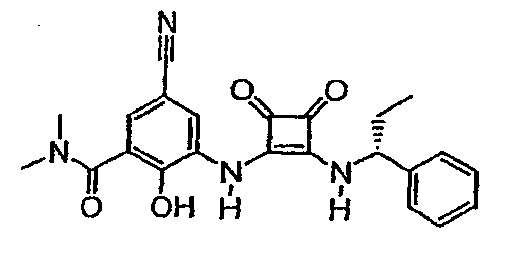 oder ein pharmazeutisch annehmbares Salz oder Solvat davon.

35. Verbindung mit der Formel: 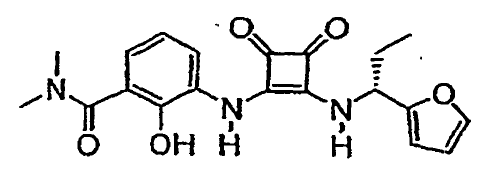 oder ein pharmazeutisch annehmbares Salz oder Solvat davon.

36. Verbindung mit der Formel: 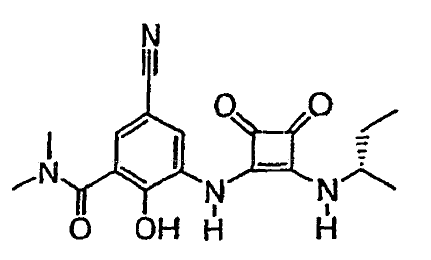 oder ein pharmazeutisch annehmbares Salz oder Solvat davon.

37. Verbindung mit der Formel: 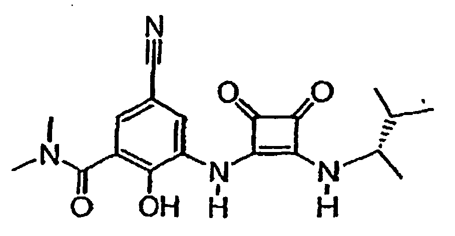 oder ein pharmazeutisch annehmbares Salz oder Solvat davon.

38. Verbindung mit der Formel: 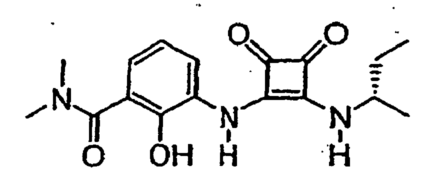 oder ein pharmazeutisch annehmbares Salz oder Solvat davon.

39. Verbindung mit der Formel: 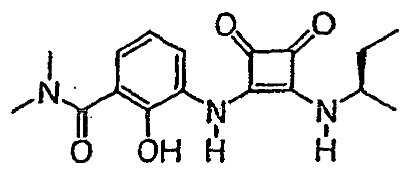 oder ein pharmazeutisch annehmbares Salz oder Solvat davon.

40. Verbindung mit der Formel: 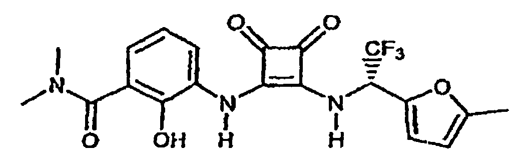 oder ein pharmazeutisch annehmbares Salz oder Solvat davon.

41. Verbindung mit der Formel: 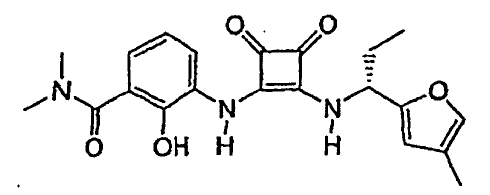 oder ein pharmazeutisch annehmbares Salz oder Solvat davon.

42. Verbindung mit der Formel: 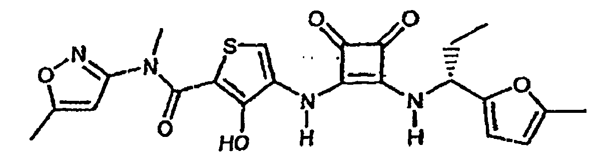 oder ein pharmazeutisch annehmbares Salz oder Solvat davon.

43. Verwendung einer Verbindung nach einem der Ansprüche 1 bis 3 zur Herstellung eines Medikaments zur Behandlung einer Chemokin-vermittelten Erkrankung, wobei die Chemokin-vermittelte Erkrankung ausgewählt ist aus der Gruppe bestehend aus Psoriasis, atopischer Dermatitis, Akne, Asthma, chronisch-obstruktiver Pulmonarerkrankung, adulter respiratorischer Erkrankung, Arthitis, entzündlicher Darmerkrankung, Morbus Crohn, Colitis ulcerosa, septischem Schock, Endotoxinschock, gramnegativer Sepsis, toxischem Schocksyndrom, Schlaganfall, kardialer und renaler Reperfusionsverletzung, Glomerulonephritis oder Thrombose, Morbus Alzheimer, Graft-versus-Host-Reaktion, Transplantatabstoßungen, cystischer Fibrose (Mukoviszidose), Malaria, akutem Atemnotsyndrom, Hypersensitivitätsreaktion vom verzögerten Typ, Atherosklerose und zerebraler und Herzischämie.

44. Verwendung einer Verbindung nach einem der Ansprüche 1 bis 42 zur Herstellung eines Medikaments zur Behandlung von Krebs.

45. Verwendung einer Verbindung nach einem der Ansprüche 1 bis 42 zur Herstellung eines Medikaments zur Behandlung von Krebs in Kombination mit der Verwendung eines Antikrebsmittels zur Herstellung eines Medikaments zur Behandlung von Krebs.

46. Verwendung nach Anspruch 45, bei der das Antikrebsmittel ausgewählt ist aus der Gruppe bestehend aus Alkylierungsmitteln, Antimetaboliten, natürlichen Produkten und ihren Derivaten, Hormonen, Antihormonen, antiangiogenen Mitteln und Steroiden sowie synthetischen Mitteln.

47. Verwendung einer Verbindung nach einem der Ansprüche 1 bis 42 zur Herstellung eines Medikaments zur Hemmung der Angiogenese.

48. Verwendung einer Verbindung nach einem der Ansprüche 1 bis 42 zur Herstellung eines Medikaments zur Inhibierung der Angiogenese in Kombination mit der Verwendung mindestens einer Antiangiogeneseverbindung zur Herstellung eines Medikaments zur Inhibierung der Angiogenese.

49. Verwendung einer Verbindung nach einem der Ansprüche 1 bis 42 zur Herstellung eines Medikaments zur Behandlung einer Erkrankung ausgewählt aus der Gruppe bestehend aus Gingivitis, Viren der Atemwege, Herpesviren, Hepatitisviren, HIV, mit Kaposi-Sarkom assoziertem Virus und Atherosklerose.

50. Verwendung einer Verbindung nach einem der Ansprüche 1 bis 42 zur Herstellung eines Medikaments zur Behandlung von angiogener Augenerkrankung.

51. Verwendung nach Anspruch 50, bei der die angiogene Augenerkrankung ausgewählt ist aus der Gruppe bestehend aus Augenentzündung, Rethinopathie durch Prämaturität, diabetischer Retinopathie, Makuladegeneration, bevorzugt vom feuchten Typ, und Neovaskularisierung der Kornea.

52. Verwendung nach Anspruch 44, bei der der behandelte Krebs Melanom, Magenkrebs oder nicht-kleinzelliges Lungenkarzinom ist.

53. Verwendung nach Anspruch 45, bei der der behandelte Krebs Melanom, Magenkrebs oder nicht-kleinzelliges Lungenkarzinom ist.

54. Verwendung nach Anspruch 46, bei der der behandelte Krebs Melanom, Magenkrebs oder nicht-kleinzelliges Lungenkarzinom ist.

55. Pharmazeutische Zusammensetzung, die eine wirksame Menge einer Verbindung nach einem der Ansprüche 1 bis 42 in Kombination mit einem pharmazeutisch annehmbaren Träger enthält.

56. Natriumsalz von einer der Verbindungen gemäß Anspruch 1. bis 42.

57. Calciumsalz von einer der Verbindungen gemäß Anspruch 1 bis 42.

58. Pharmazeutische Zusammensetzung, die eine wirksame Menge einer Verbindung nach einem der Ansprüche 56 und 57 in Kombination mit einem pharmazeutisch annehmbaren Träger enthält.

## Revendications

1. Composé de formule 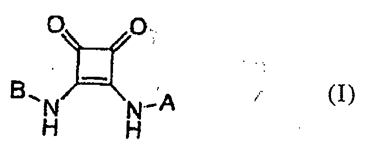 ou sel ou solvat pharmacologiquement admissible d'un tel composé,
dans laquelle formule :
A représente un groupe choisi dans l'ensemble formé par les suivants : 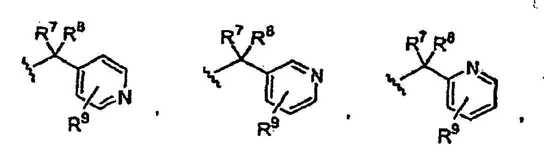 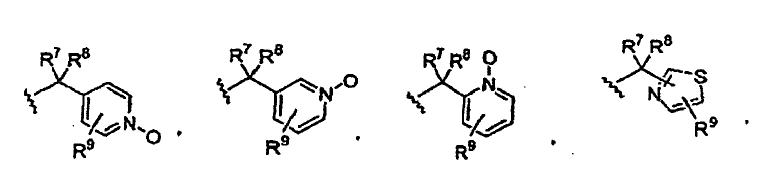 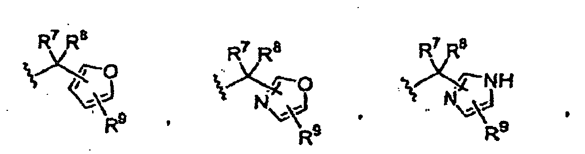 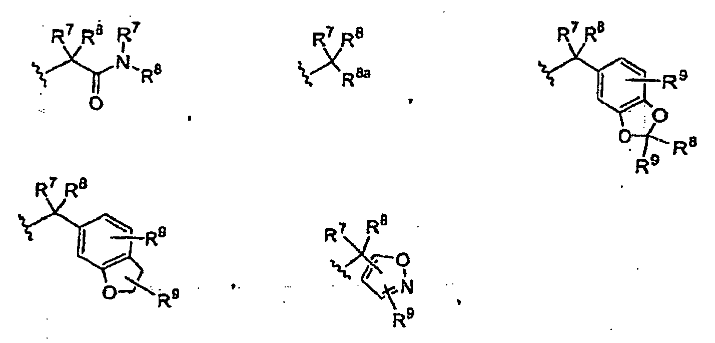 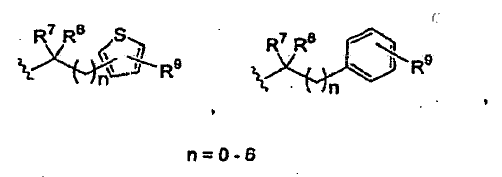 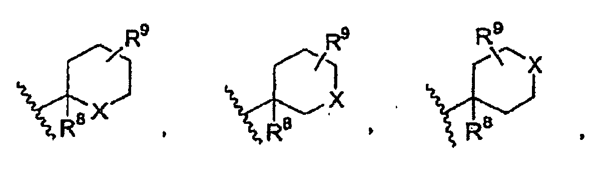 X=-O-, -NH-, -S- 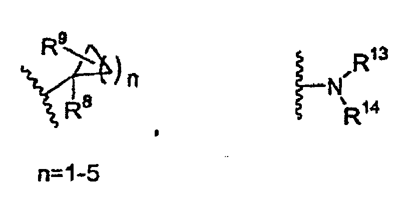 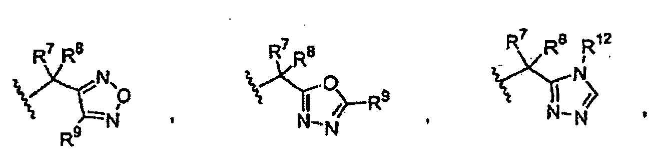 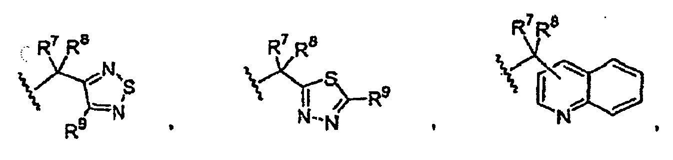 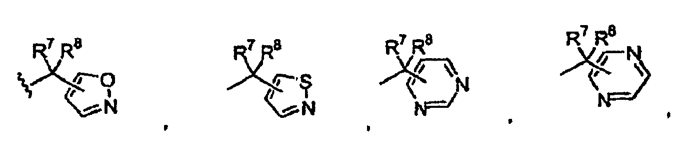 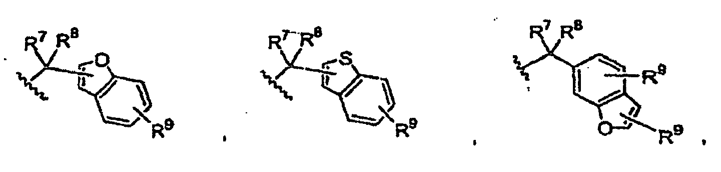 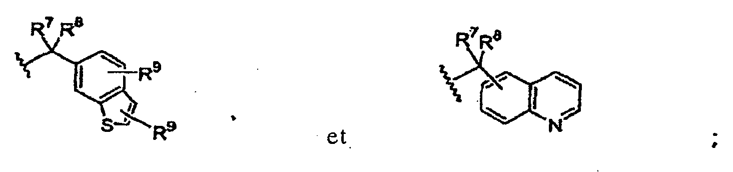
- B représente un groupe choisi dans l'ensemble formé par les suivants : 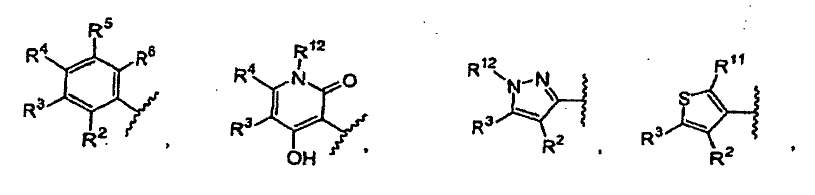 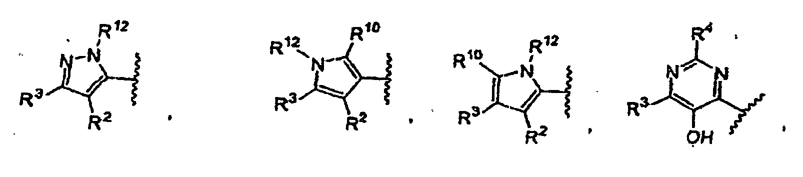 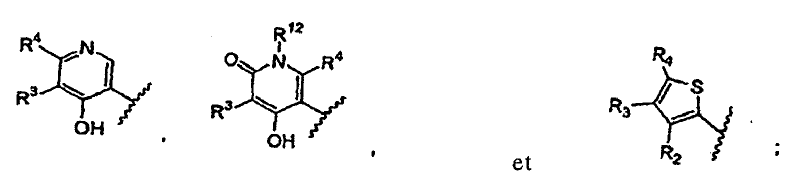
- R² représente un groupe hydroxyle OH ;
- R³ représente un groupe choisi parmi ceux de formule -C(O)NHR¹⁷, -C(O)NR¹³R¹⁴ ou -C(O)NR¹³OR¹⁴;
- R⁴ représente une entité choisie parmi les atomes d'hydrogène et d'halogène, les groupes cyano, alkyle et alcoxy, les groupes de formule -OH, -CF₃, -OCF₃, -NO₂, -C(O)R¹³, -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, 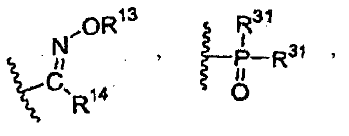 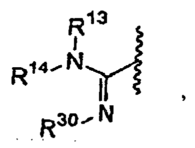 ou -NHC(O)R¹⁷, les groupes aryle avec ou sans substituant(s) et les groupes hétéroaryle avec ou sans substituant(s), les substituants portés par les groupes avec substituant(s) symbolisés par R⁴ étant identiques ou différents et choisis indépendamment, au nombre de 1 à 6, parmi ceux qui sont symbolisés par R⁹ ;
- R⁵ et R⁶ représentent des entités identiques ou différentes qui sont choisies indépendamment parmi les atomes d'hydrogène et d'halogène, les groupes cyano, alkyle et alcoxy, les groupes de formule -CF₃, -OCF₃, -NO₂, -C(O)R¹³, -C(O)OR¹³,-C(O)NR¹³R¹⁴, -SO₍ₜ₎NR¹³R¹⁴ ou -C(O)NR¹³OR¹⁴, les groupes aryle avec ou sans substituant(s) et les groupes hétéroaryle avec ou sans substituant(s),
les substituants portés par les groupes avec substituant(s) symbolisés par R⁵ et R⁶ étant identiques ou différents et choisis indépendamment, au nombre de 1 à 6, parmi ceux qui sont symbolisés par R⁹ ;
- R⁷ et R⁸ représentent des entités identiques ou différentes qui sont choisies indépendamment parmi l'atome d'hydrogène, les groupes alkyle avec ou sans substituant(s), aryle avec ou sans substituant(s), hétéroaryle avec ou sans substituant(s), aryl-alkyle avec ou sans substituant(s), hétéroaryl-alkyle avec ou sans substituant(s), cycloalkyle avec ou sans substituant(s), et cycloalkyl-alkyle avec ou sans substituant(s), les groupes de formule -CO₂R¹³ ou -CONR¹³R¹⁴, et les groupes fluoroalkyle, alcynyle, alcényle et cycloalcényle,
les substituants portés par les groupes avec substituant(s) symbolisés par R⁷ et R⁸ étant choisis dans l'ensemble formé par les suivants :
a) l'atome d'hydrogène H,
b) les atomes d'halogène,
c) le groupe trifluorométhyle -CF₃,
d) les groupes de formule -COR¹³,
e) les groupes de formule -OR¹³,
f) les groupes de formule -NR¹³R¹⁴,
g) le groupe nitro -NO₂,
h) le groupe cyano -CN,
i) les groupes de formule -SO₂OR¹³,
j) les groupes trialkyl-silyle -Si(alkyle)₃,
k) les groupes triaryl-silyle -Si(aryle)₃,
l) les groupes de formule -Si(R¹³)₂R¹⁴,
m) les groupes de formule -CO₂R¹³,
n) les groupes de formule -C(O)NR¹³R¹⁴,
o) les groupes de formule -SO₂NR¹³R¹⁴,
p) les groupes de formule -SO₂R¹³,
q) les groupes de formule -OC(=O)R¹³,
r) les groupes de formule -OC(=O)NR¹³R¹⁴,
s) les groupes de formule -NR`³C(O)R¹⁴,
t) et les groupes de formule -NR¹³CO₂R¹⁴;
- R^{8a} représente une entité choisie parmi l'atome d'hydrogène et les groupes alkyle, cycloalkyle et cycloalkyl-alkyle ;
- R⁹ représente une combinaison de 1 à 6 substituants choisis indépendamment parmi les suivants :
a) les entités symbolisées par R¹³,
b) les atomes d'halogène,
c) le groupe trifluorométhyle -CF₃,
d) les groupes de formule -COR¹³,
e) les groupes de formule -OR¹³,
f) les groupes de formule -NR¹³R¹⁴,
g) le groupe nitro -NO₂,
h) le groupe cyano -CN,
i) les groupes de formule -SO₂R¹³,
j) les groupes de formule -SO₂NR¹³R¹⁴,
k) les groupes de formule -NR¹³C(O)R¹⁴,
l) les groupes de formule -C(O)NR¹³R¹⁴,
m) les groupes de formule -NR¹³CO₂R¹⁴,
n) les groupes de formule -CO₂R¹³,
o) et le groupe de formule 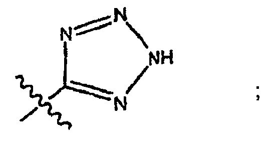
- R¹⁰ et R¹¹ représentent des entités identiques ou différentes qui sont choisies indépendamment parmi les atomes d'hydrogène et d'halogène, les groupes de formule -CF₃, -OCF₃, -NR¹³R¹⁴, -NR¹³C(O)NR¹³R¹⁴, -OH, -C(O)OR¹³, -SH, -SO₍ₜ₎NR¹³R¹⁴, -SO₂R¹³, -NHC(O)R¹³, -NHSO₂NR¹³R¹⁴, -NHSO₂R¹³, -C(O)NR¹³R¹⁴, -C(O)NR¹³OR¹⁴ ou -OC(O)R¹³, et le groupe cyano ;
- R¹² représente un atome d'hydrogène, un groupe de formule -OC(O)R¹³ ou un groupe aryle avec ou sans substituant(s), hétéroaryle avec ou sans substituant(s), aryl-alkyle avec ou sans substituant(s), cycloalkyle avec ou sans substituant(s), alkyle avec ou sans substituant(s), cycloalkyl-alkyle avec ou sans substituant(s), ou hétéroaryl-alkyle avec ou sans substituant(s),
les substituants portés par les groupes avec substituant(s) symbolisés par R¹² étant identiques ou différents et choisis indépendamment, au nombre de 1 à 6, parmi ceux qui sont symbolisés par R⁹ ;
- R¹³ et R¹⁴ représentent des entités identiques ou différentes qui sont choisies indépendamment parmi l'atome d'hydrogène et les groupes alkyle avec ou sans substituant(s), aryle avec ou sans substituant(s), hétéroaryle avec ou sans substituant(s), aryl-alkyle avec ou sans substituant(s), hétéroaryl-alkyle avec ou sans substituant(s), cycloalkyle avec ou sans substituant(s), cycloalkyl-alkyle avec ou sans substituant(s) et fluoroalkyle avec ou sans substituant(s),
ou bien les deux entités représentées par R¹³ et R¹⁴ peuvent, quand elles sont liées à un atome d'azote, former ensemble un hétérocycle avec ou sans substituant(s), comportant 3 à 7 chaînons dont un à deux hétéroatomes choisis parmi les atomes d'oxygène, de soufre et d'azote, les substituants portés par les groupes avec substituant(s) symbolisés par R¹³ et R¹⁴ étant identiques ou différents et choisis indépendamment, au nombre de 1 à 6, parmi les atomes d'hydrogène et d'halogène, les groupes alkyle, aryle, aryl-alkyle, fluoroalkyle, cycloalkyle, cycloalkyl-alkyle, hétéroaryle et hétéroaryl-alkyle, le groupe amino et les groupes de formule -C(O)OR¹⁵, -C(O)NR¹⁵R¹⁶, -S(O)ₜNR¹⁵R¹⁶, -C(O)R¹⁵, -SO₂R¹⁵ ou -NHCONR¹³R¹⁴;
- R¹⁵ et R¹⁶ représentent des entités identiques ou différentes qui sont choisies indépendamment parmi l'atome d'hydrogène et les groupes alkyle, aryle, aryl-alkyle, cycloalkyle et hétéroaryle ;
- R¹⁷ représente un groupe alkyl-sulfonyle (-SO₂-alkyle), aryl-sulfonyle, cycloalkyl-sulfonyle ou hétéroaryl-sulfonyle ;
- R³⁰ représente un groupe alkyle ou cycloalkyle ou un groupe de formule -CN, -NO₂ ou -SO₂R¹⁵;
- les symboles R³¹ représentent des entités identiques ou différentes qui sont choisies indépendamment parmi les groupes alkyle avec ou sans substituant(s), aryle avec ou sans substituant(s), hétéroaryle avec ou sans substituant(s), et cycloalkyle avec ou sans substituant(s),
les substituants portés par les groupes avec substituant(s) symbolisés par R³¹ étant identiques ou différents et choisis indépendamment, au nombre de 1 à 6, parmi ceux qui sont symbolisés par R⁹ ;
- et l'indice t vaut 0, 1 ou 2.

2. Composé conforme à la revendication 1, dans lequel A représente un groupe choisi parmi ceux de formules : 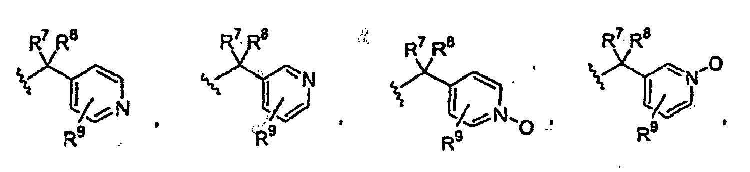 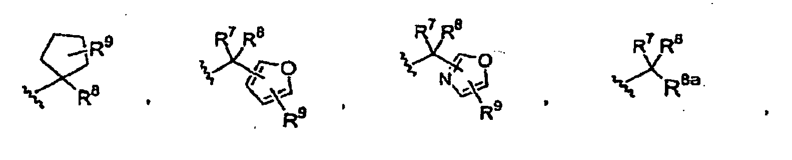 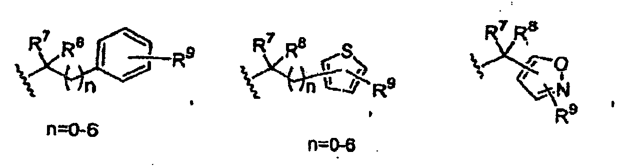 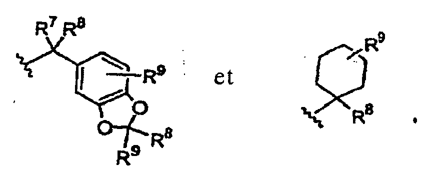 dans lesquelles
- R⁷ représente un atome d'hydrogène ou un groupe fluoroalkyle, alkyle ou cycloalkyle;
- R⁸ représente un atome d'hydrogène, un groupe alkyle ou un groupe de formule -CF₂CH₃ ou -CF₃;
- et R⁹ représente un atome d'hydrogène, de fluor, de chlore ou de brome, un groupe alkyle ou un groupe de formule -CF₃.

3. Composé conforme à la revendication 1, dans lequel A représente un groupe choisi parmi ceux de formules: 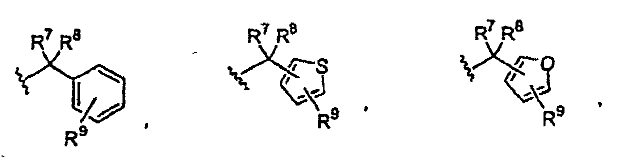 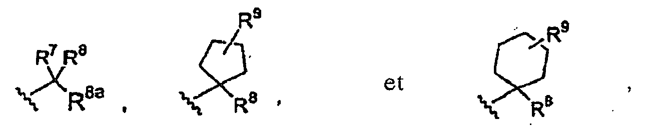 dans lesquelles
- R⁷ représente un atome d'hydrogène, un groupe de formule -CF₂CH₃ ou -CF₃, ou un groupe méthyle, éthyle, isopropyle, cyclopropyle ou tertiobutyle ;
- R⁸ représente un atome d'hydrogène ;
- et R⁹ représente un atome d'hydrogène, de fluor, de chlore ou de brome, un groupe alkyle ou un groupe de formule -CF₃ ;
et B représente un groupe de formule 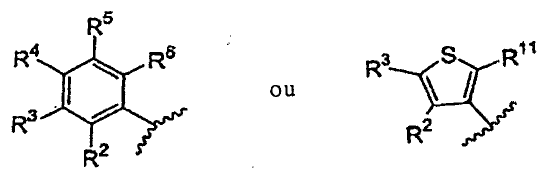 dans lesquelles formules :
- R² représente un groupe hydroxyle -OH ;
- R³ représente un groupe de formule -C(O)NR¹³R¹⁴ ;
- R⁴ représente un atome d'hydrogène ou un groupe nitro -NO₂, cyano, méthyle -CH₃ ou trifluorométhyle -CF₃ ;
- R⁵ représente un atome d'hydrogène ou d'halogène ou un groupe trifluorométhyle, nitro ou cyano ;
- R⁶ représente un atome d'hydrogène, un groupe alkyle ou un groupe trifluorométhyle ;
- R¹¹ représente un atome d'hydrogène ou d'halogène ou un groupe alkyle ;
- et R¹³ et R¹⁴ représentent des entités identiques ou différentes, choisies indépendamment parmi les groupes méthyle, éthyle et isopropyle ;
- ou bien les entités représentées par R¹³ et R¹⁴, considérées conjointement avec l'atome d'azote auquel elles sont liées, dans les groupes de formule -NR¹³R¹⁴, -C(O)N¹³R¹⁴, -SO₂NR¹³R¹⁴, -OC(O)NR¹³R¹⁴, -C(O)NR¹³R¹⁴, -NR¹³C(O)NR¹³R¹⁴, -SO₍ₜ₎NR¹³R¹⁴ ou -NHSO₂NR¹³R¹⁴, forment de préférence un groupe hétérocyclique saturé avec ou sans substituant(s), comportant 3 à 7 chaînons parmi lesquels, en option, un chaînon hétéroatomique supplémentaire choisi parmi un atome d'oxygène, un atome de soufre et un chaînon azoté symbolisé par NR¹⁸ où R¹⁸ représente un atome d'hydrogène, un groupe alkyle, aryle ou hétéroaryle ou un groupe de formule -C(O)R¹⁹, -SO₂R¹⁹ ou -C(O)NR¹⁹R²⁰ où R¹⁹ et R²⁰ représentent des entités identiques ou différentes qui sont choisies, chacune indépendamment, parmi les groupes alkyle, aryle et hétéroaryle,
les substituants portés par les groupes cycliques à substituant(s) formés par les entités représentées par R¹³ et R¹⁴ étant identiques ou différents et choisis indépendamment, au nombre de 1 à 3, parmi les groupes alkyle, aryle, hydroxy, hydroxyalkyle, alcoxy, alcoxyalkyle, aryl-alkyle, fluoroalkyle, cycloalkyle, cycloalkyl-alkyle, hétéroaryle, hétéroaryl-alkyle et amino, les groupes de formule -C(O)OR¹⁵, -C(O)NR¹⁵R¹⁶, -C(O)R¹⁵, -S(O)ₜNR¹⁵R¹⁶,-SO₂R¹⁵ ou -NHCONR¹⁵R¹⁶, et les atomes d'halogène, où R¹⁵ et R¹⁶ représentent des entités identiques ou différentes qui sont choisies indépendamment parmi l'atome d'hydrogène et les groupes alkyle, aryle, aryl-alkyle, cycloalkyle et hétéroaryle.

4. Composé conforme à la revendication 1, dans lequel A représente un groupe choisi parmi ceux de formules : 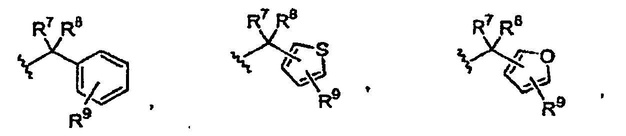 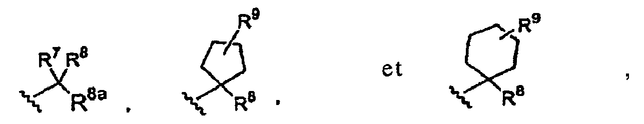 et B représente un groupe de formule 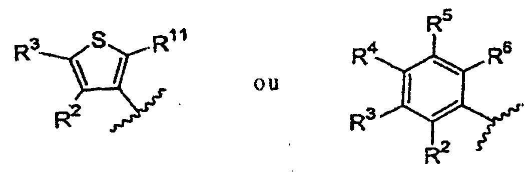 dans lesquelles formules :
- R² représente un groupe hydroxyle -OH ;
- R³ représente un groupe de formule -C(O)NR¹³R¹⁴;
- R⁴ représente un atome d'hydrogène ou un groupe nitro -NO₂, cyano, méthyle -CH₃ ou trifluorométhyle -CF₃ ;
- R⁵ représente un atome d'hydrogène ou d'halogène ou un groupe nitro, cyano ou trifluorométhyle ;
- R⁶ représente un atome d'hydrogène, un groupe alkyle ou un groupe trifluorométhyle ;
- R⁷ représente un atome d'hydrogène, un groupe de formule -CF₂CH₃ ou -CF₃, ou un groupe méthyle, éthyle, isopropyle, cyclopropyle ou tertio-butyle ;
- R⁸ représente un atome d'hydrogène ;
- R⁹ représente un atome d'hydrogène, de fluor, de chlore ou de brome, un groupe alkyle ou cycloalkyle, ou un groupe de formule -CF₃ ;
- R¹¹ représente un atome d'hydrogène ou d'halogène ou un groupe alkyle ;
- et R¹³ et R¹⁴ représentent chacun, indépendamment, un groupe méthyle ou éthyle.

5. Composé conforme à la revendication 1, dans lequel A représente un groupe choisi parmi ceux de formules : 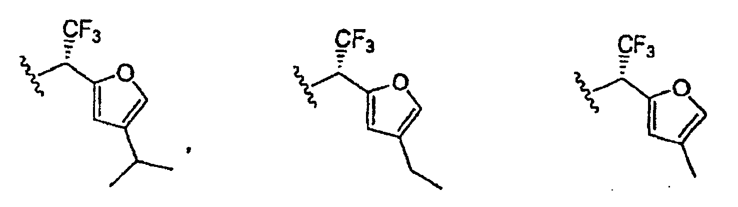 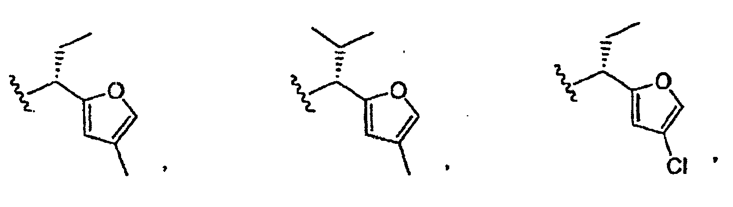 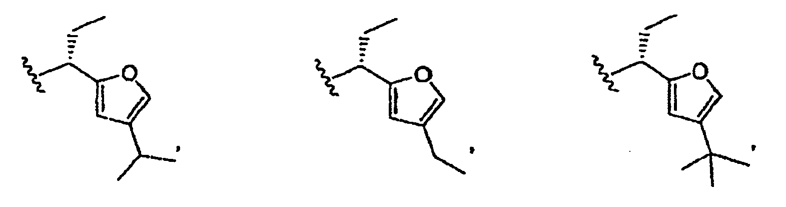 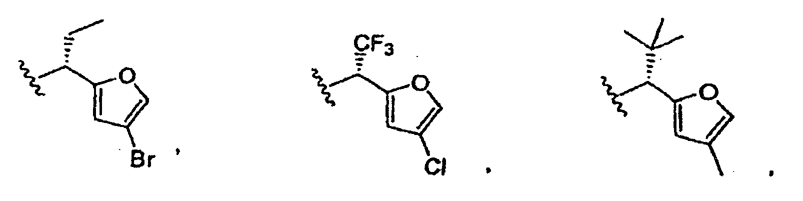 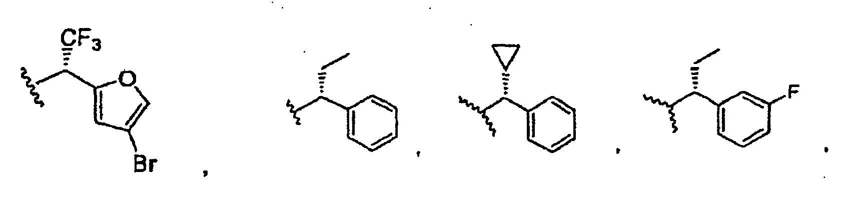 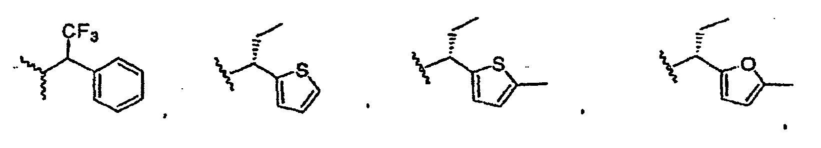 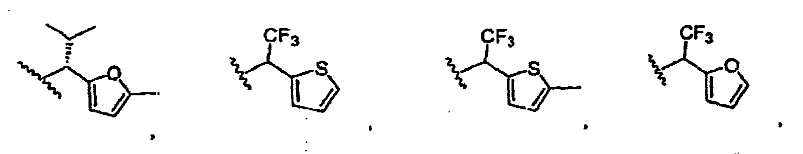 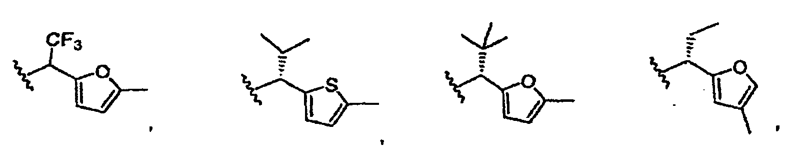 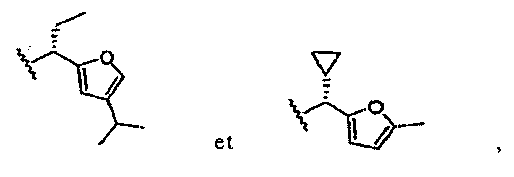 et B représente un groupe de formule 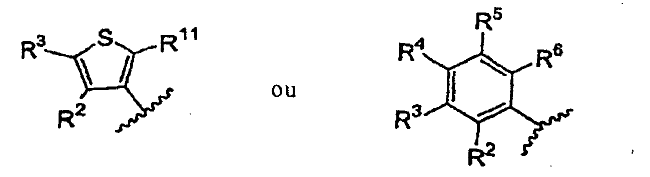 dans lesquelles formules :
- R² représente un groupe hydroxyle -OH ;
- R³ représente un groupe de formule -C(O)NR¹³R¹⁴ ;
- R⁴ représente un atome d'hydrogène ou un groupe méthyle -CH₃ ou trifluorométhyle -CF₃ ;
- R⁵ représente un atome d'hydrogène ou un groupe cyano ;
- R⁶ représente un atome d'hydrogène, un groupe méthyle ou un groupe trifluorométhyle ;
- R¹¹ représente un atome d'hydrogène ;
- et R¹³ et R¹⁴ représentent chacun un groupe méthyle.

6. Composé de formule 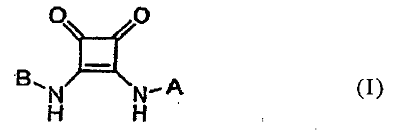 ou sel ou solvat pharmacologiquement admissible d'un tel composé,
dans laquelle formule :
- A représente un groupe choisi dans l'ensemble formé par les suivants : 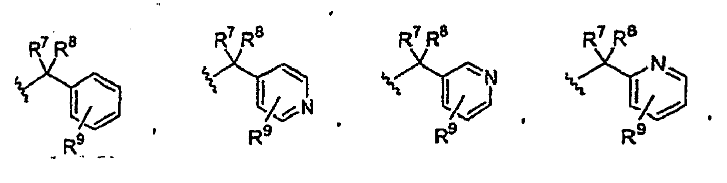 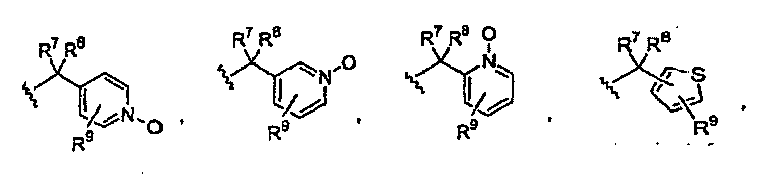 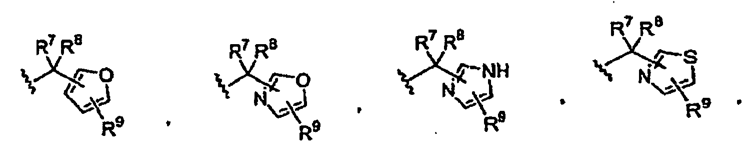 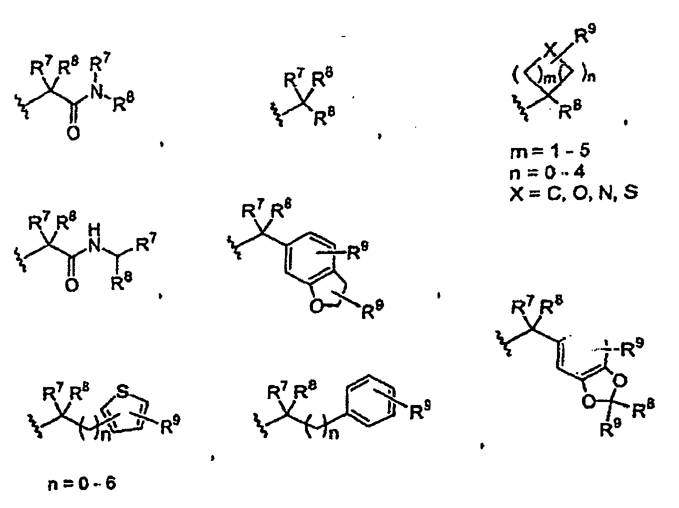 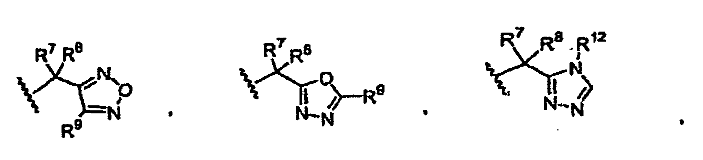 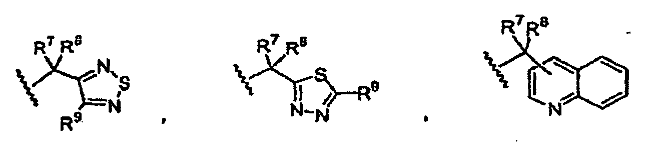 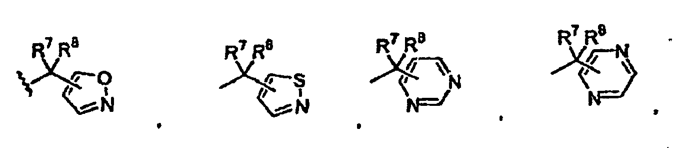 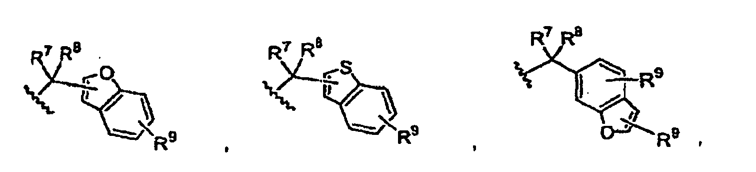 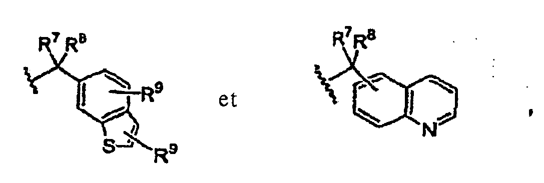 dans lesquelles formules
- R⁷ et R⁸ représentent des entités identiques ou différentes qui sont choisies indépendamment parmi l'atome d'hydrogène et les groupes alkyle, aryle, hétéroaryle, aryl-alkyle, hétéroaryl-alkyle, cycloalkyle et cycloalkyl-alkyle, les groupes de formule -CO₂R¹³ ou -CONR¹³R¹⁴, et les groupes fluoroalkyle, alcynyle, alcényle, alcynyl-alkyle, alcénylalkyle et cycloalcényle, avec ou sans substituant(s),
les substituants portés par ces groupes, lorsqu'ils en portent, étant choisis dans l'ensemble formé par les suivants :
a) le groupe cyano,
b) les groupes de formule -CO₂R⁷,
c) les groupes de formule -CONR⁷R⁸,
d) les groupes de formule -SO₂NR⁷R⁸,
e) les groupes de formule -SO₂R⁷,
f) le groupe nitro -NO₂,
g) le groupe trifluorométhyle CF₃,
h) les groupes de formule -OR⁷,
i) les groupes de formule -NR⁷R⁸,
j) les groupes de formule -OC(=O)R⁷,
k) les groupes de formule -OC(=O)NR⁷R⁸,
l) et les atomes d'halogène ;
- et R⁹ représente une ou plusieurs entités choisies parmi les suivantes:
a) les entités symbolisées par R⁷,
b) les entités symbolisées par R⁸,
c) les atomes d'halogène,
d) le groupe trifluorométhyle -CF₃,
e) les groupes de formule -COR⁷,
f) les groupes de formule -OR⁷,
g) les groupes de formule -NR⁷R⁸,
h) le groupe nitro -NO₂,
i) le groupe cyano -CN,
j) les groupes de formule -SO₂R⁷,
k) les groupes de formule -SO₂NR⁷R⁸,
l) les groupes de formule -NR⁷C(O)R⁸,
m) les groupes de formule -C(O)NR⁷R⁸,
n) les groupes de formule -NR⁷CO₂R⁸,
o) les groupes de formule -CO₂R⁷,
p) et le groupe de formule 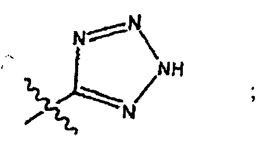
- et B représente un groupe aryle ou hétéroaryle, à substituant(s) optionnel(s), choisi parmi les suivants : 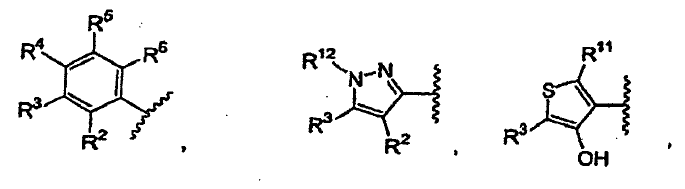 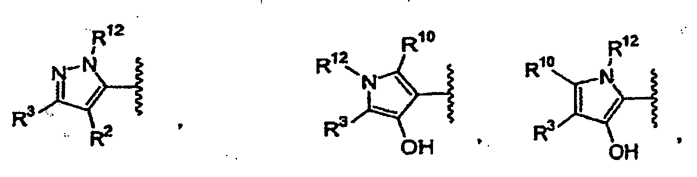 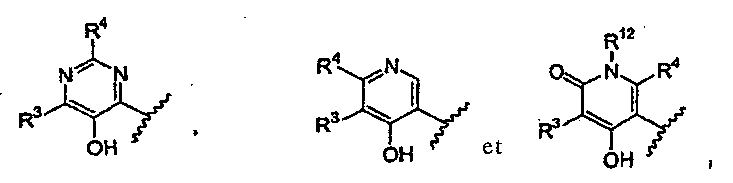 dans lesquelles formules
- R² représente un groupe hydroxyle OH ;
- R³ représente un groupe de formule -C(O)NR¹³R¹⁴ ou -C(O)NR¹³OR¹⁴;
- R⁴ représente une entité choisie parmi les atomes d'hydrogène et d'halogène, les groupes cyano, alkyle et alcoxy, les groupes dé formule -OH, -CF₃, -OCF₃, -NO₂, -C(O)R¹³, -C(O)OR¹³, -C(O)NR¹³R¹⁴, -SO₍ₜ₎NR¹³R¹⁴, -SO₍ₜ₎R¹³, -C(O)NR¹³OR¹⁴ ou -C(=NOR¹³)R¹⁴, les groupes aryle avec ou sans substituant(s) et les groupes hétéroaryle avec ou sans substituant(s),
où les substituants portés par les groupes avec substituant(s) sont choisis parmi un ou plusieurs de ceux qui sont symbolisés par R⁹ ;
- R⁵ et R⁶ représentent chacun, indépendamment, un atome d'hydrogène ou d'halogène, un groupe cyano, alkyle ou alcoxy, un groupe de formule -CF₃, -OCF₃, -NO₂, -C(O)R¹³, -C(O)OR¹³, -C(O)NR¹³R¹⁴, -SO₍ₜ₎NR¹³R¹⁴ ou -C(O)NR¹³OR¹⁴, un groupe aryle avec ou sans substituant(s) ou un groupe hétéroaryle avec ou sans substituant(s),
où les substituants portés par les groupes avec substituant(s) sont choisis parmi un ou plusieurs de ceux qui sont symbolisés par R⁹ ;
- R¹⁰ et R¹¹représentent chacun, indépendamment, un atome d'hydrogène ou d'halogène, un groupe de formule -CF₃, -OCF₃, -NR¹³R¹⁴, -NR¹³C(O)NR¹³R¹⁴ -OH, -C(O)OR¹³, -SH, -SO₍ₜ₎NR¹³R¹⁴, -SO₂R¹³, -NHC(O)R¹³ -NHSO₂NR¹³R¹⁴, -NHSO₂R¹³, -C(O)NR¹³R¹⁴, -C(O)NR¹³OR¹⁴ ou -OC(O)R¹³, ou un groupe cyano ;
- R¹² représente un atome d'hydrogène, un groupe de formule -OC(O)R¹³ ou un groupe aryle, hétéroaryle, aryl-alkyle, cycloalkyle, alkyle, cycloalkyl-alkyle ou hétéroaryl-alkyle, avec ou sans substituant(s) ;
- R¹³ et R¹⁴ représentent des entités identiques ou différentes qui sont choisies indépendamment parmi l'atome d'hydrogène et les groupes alkyle, aryle, hétéroaryle, aryl-alkyle, hétéroaryl-alkyle, cycloalkyle, cycloalkyl-alkyle et fluoroalkyle, avec ou sans substituant(s),
ou bien les deux entités représentées par R¹³ et R¹⁴ peuvent former ensemble un hétérocycle avec ou sans substituant(s), comportant 3 à 7 chaînons dont un à deux hétéroatomes choisis par les atomes d'oxygène, de soufre et d'azote,
où les substituants portés par les groupes avec substituant(s) sont choisis parmi un ou plusieurs de ceux qui sont symbolisés par R⁹ ;
- et l'indice t vaut 1 ou 2.

7. Composé de formule (IA) 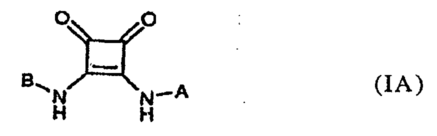 ou sel ou solvat pharmacologiquement admissible d'un tel composé,
dans laquelle formule :
- A représente un groupe choisi dans l'ensemble formé par les suivants :
1) 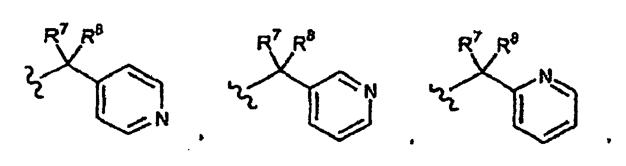 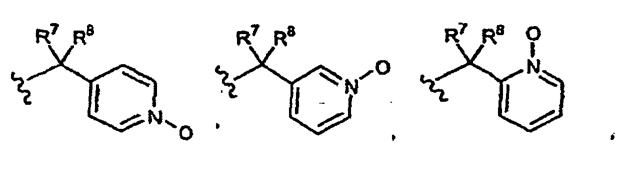 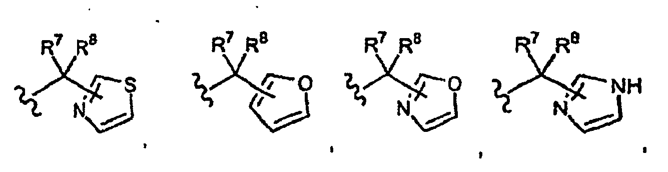 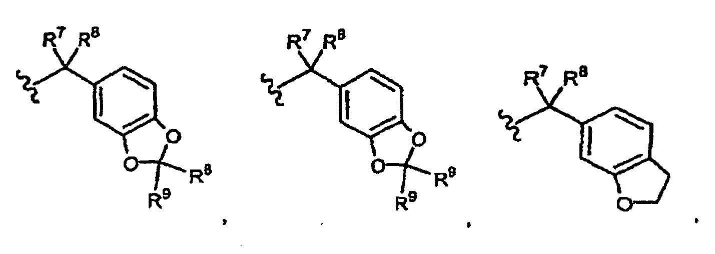 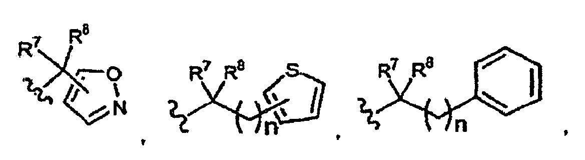 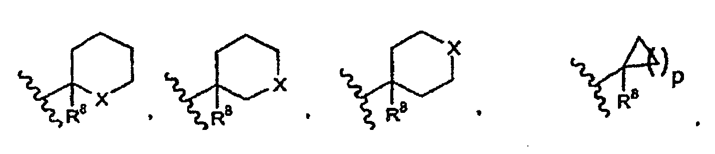 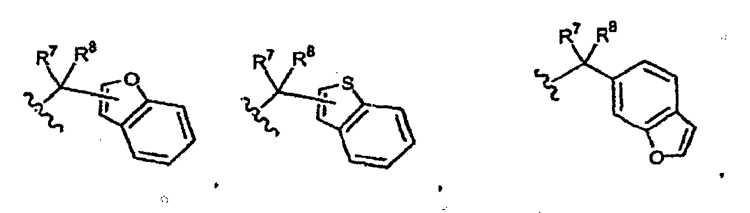 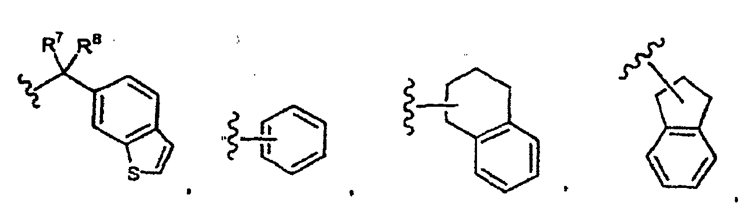 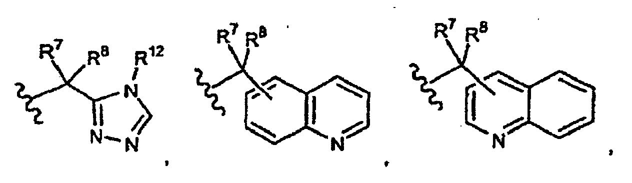 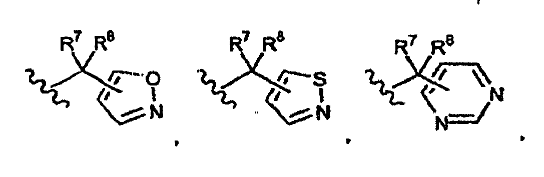 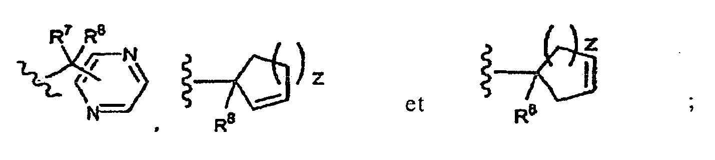
2) 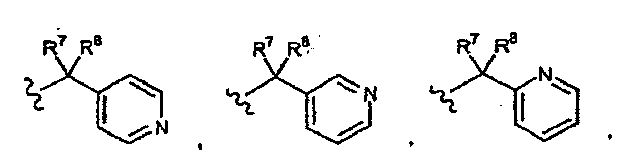 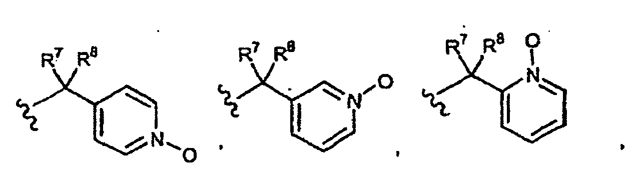 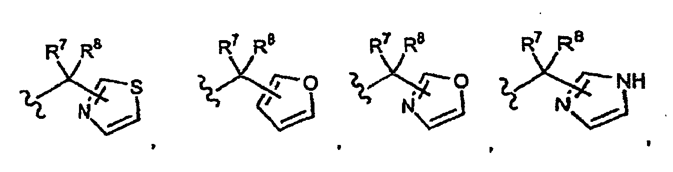 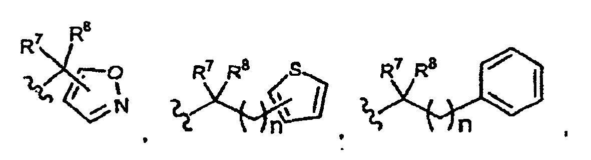 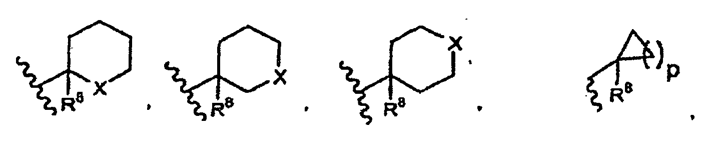 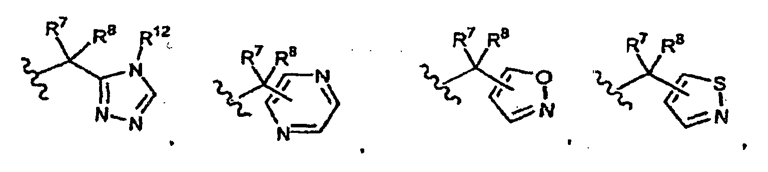 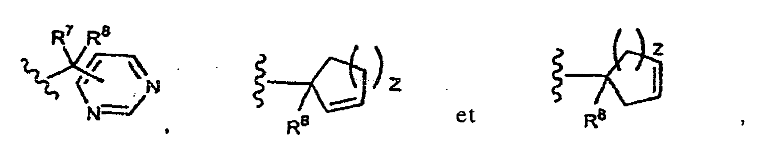 où les cycles de ces groupes symbolisés par A portent 1 à 6 substituants choisis, chacun indépendamment, parmi ceux qui sont symbolisés par R⁹ ;
3) 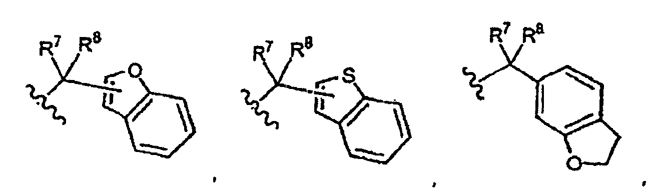 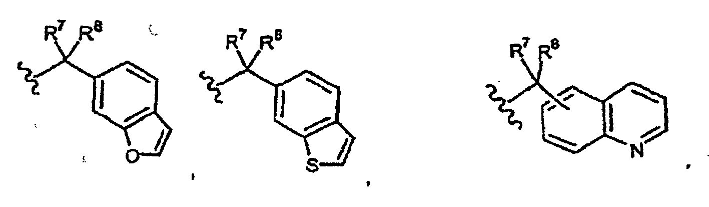 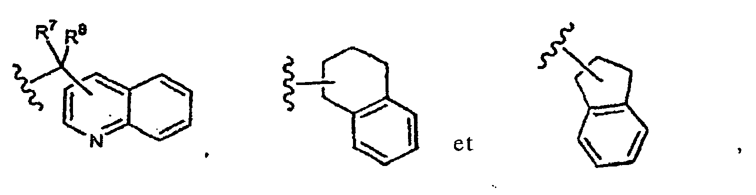 où l'un des cycles, ou les deux, de ces groupes symbolisés par A portent 1 à 6 substituants choisis, chacun indépendamment, parmi ceux qui sont symbolisés par R⁹ ;
4) 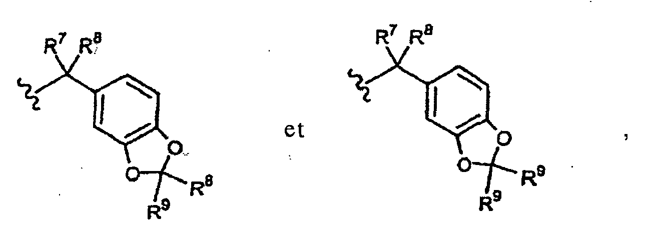 où les cycles phényle de ces groupes symbolisés par A portent 1 à 3 substituants choisis, chacun indépendamment, parmi ceux qui sont symbolisés par R⁹ ;
5) 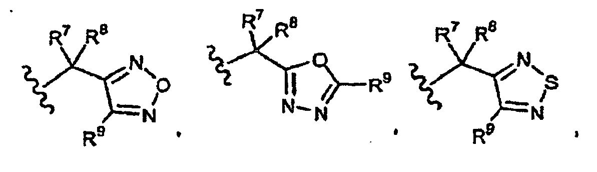 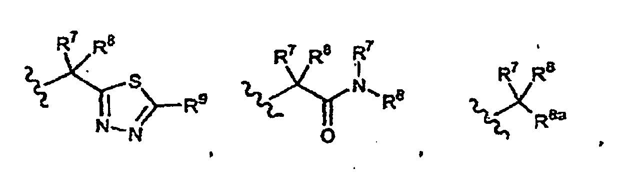 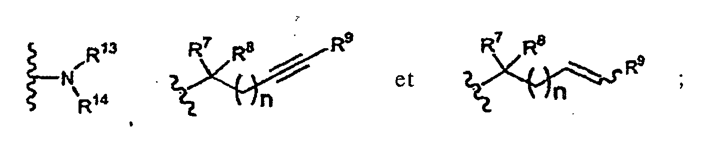
- B représente un groupe choisi dans l'ensemble formé par les suivants : 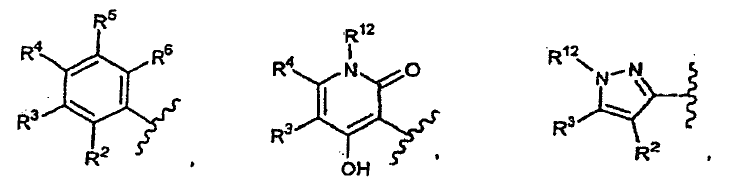 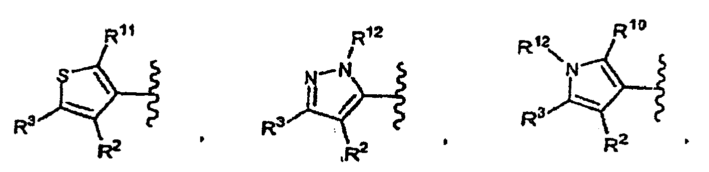 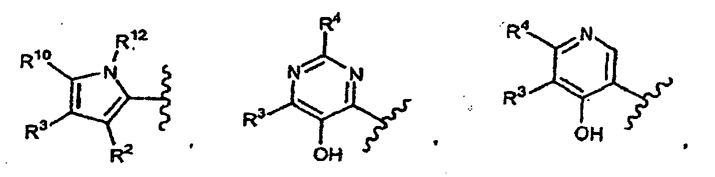 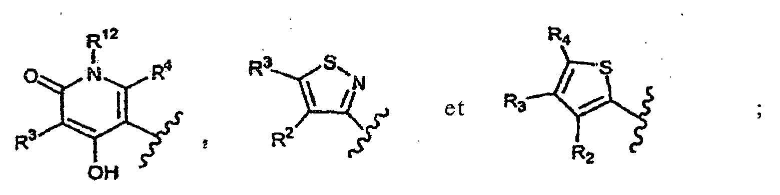
- l'indice n vaut de 0 à 6 ;
- l'indice p vaut de 1 à 5 ;
- le symbole X représente un chaînon O, NH ou S ;
- l'indice z vaut de 1 à 3 ;
- R² représente un groupe hydroxyle OH ;
- R³ représente un groupe choisi parmi ceux de formule -C(O)NHR¹⁷, -C(O)NR¹³R¹⁴ ou -C(O)NR¹³OR¹⁴ ;
- R⁴ représente une entité choisie parmi les atomes d'hydrogène et d'halogène, les groupes cyano, alkyle et alcoxy, les groupes de formule -OH, -CF₃, -OCF₃, -NO₂, -C(O)R¹³, -C(O)OR¹³, -C(O)NHR¹⁷, -C(O)NR¹³R¹⁴, -SO₍ₜ₎NR¹³R¹⁴, -SO₍ₜ₎R¹³, -C(O)NR¹³OR¹⁴, 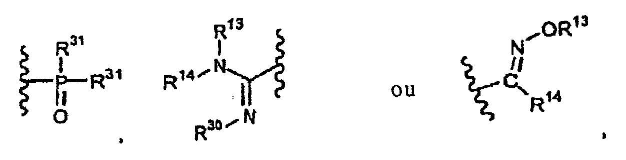 les groupes aryle avec ou sans substituant(s) et les groupes hétéroaryle avec ou sans substituant(s),
où lesdits groupes aryle avec substituant(s) portent 1 à 6 substituants qui sont choisis, chacun indépendamment, parmi ceux qui sont symbolisés par R⁹,
et où lesdits groupes hétéroaryle avec substituant(s) portent 1 à 6 substituants qui sont choisis, chacun indépendamment, parmi ceux qui sont symbolisés par R⁹ ;
- R⁵ et R⁶ représentent des entités identiques ou différentes qui sont choisies indépendamment parmi les atomes d'hydrogène et d'halogène, les groupes cyano, alkyle et alcoxy, les groupes de formule -CF₃, -OCF₃, -NO₂, -C(O)R¹³, -C(O)OR¹³, -C(O)NR¹³R¹⁴, -SO₍ₜ₎NR¹³R¹⁴ ou -C(O)NR¹³OR¹⁴, les groupes aryle avec ou sans substituant(s) et les groupes hétéroaryle avec ou sans substituant(s),
où lesdits groupes aryle avec substituant(s) portent 1 à 6 substituants qui sont choisis, chacun indépendamment, parmi ceux qui sont symbolisés par R⁹,
et où lesdits groupes hétéroaryle avec substituant(s) portent 1 à 6 substituants qui sont choisis, chacun indépendamment, parmi ceux qui sont symbolisés par R⁹ ;
- R⁷ et R⁸ représentent, dans chaque cas, des entités identiques ou différentes qui sont choisies indépendamment parmi l'atome d'hydrogène, les groupes alkyle avec ou sans substituant(s), aryle avec ou sans substituant(s), hétéroaryle avec ou sans substituant(s), aryl-alkyle avec ou sans substituant(s), hétéroaryl-alkyle avec ou sans substituant(s), cycloalkyle avec ou sans substituant(s), et cycloalkyl-alkyle avec ou sans substituant(s), les groupes de formule -CO₂R¹³ ou -CONR¹³R¹⁴, et les groupes alcynyle, alcényle et cycloalcényle,
où lesdits groupes avec substituant(s) symbolisés par R⁷ et R⁸ portent un ou plusieurs substituants qui sont choisis, chacun indépendamment, dans l'ensemble formé par les suivants :
a) les atomes d'halogène,
b) le groupe trifluorométhyle -CF₃,
c) les groupes de formule -COR¹³,
d) les groupes de formule -OR¹³,
e) les groupes de formule -NR¹³R¹⁴,
f) le groupe nitro -NO₂,
g) le groupe cyano -CN,
h) les groupes de formule -SO₂OR¹³,
i) les groupes trialkyl-silyle -Si(alkyle)₃, où chaque groupe alkyle est choisi indépendamment,
j) les groupes triaryl-silyle -Si(aryle)₃, où chaque groupe aryle est choisi indépendamment,
k) les groupes de formule -Si(R¹³)₂R¹⁴, où chaque entité symbolisée par R¹³ est choisie indépendamment,
l) les groupes de formule -CO₂R¹³,
m) les groupes de formule -C(O)NR¹³R¹⁴,
n) les groupes de formule -SO₂NR¹³R¹⁴,
o) les groupes de formule -SO₂R¹³,
p) les groupes de formule -OC(O)R¹³,
q) les groupes de formule -OC(O)NR¹³R¹⁴,
r) les groupes de formule -NR¹³C(O)R¹⁴,
s) et les groupes de formule -NR¹³CO₂R¹⁴ ;
- R^{8a} représente une entité choisie parmi l'atome d'hydrogène et les groupes alkyle, cycloalkyle et cycloalkyl-alkyle ;
- R⁹ représente dans chaque cas un substituant choisi indépendamment parmi les suivants :
a) les entités symbolisées par R¹³,
b) les atomes d'halogène,
c) le groupe trifluorométhyle -CF₃,
d) les groupes de formule -COR¹³,
e) les groupes de formule -OR¹³,
f) les groupes de formule -NR¹³R¹⁴,
g) le groupe nitro -NO₂,
h) le groupe cyano -CN,
i) les groupes de formule -SO₂R¹³,
j) les groupes de formule -SO₂NR¹³R¹⁴,
k) les groupes de formule -NR¹³C(O)R¹⁴,
l) les groupes de formule -CONR¹³R¹⁴,
m) les groupes de formule -NR¹³CO₂R¹⁴,
n) les groupes de formule -CO₂R¹³,
o) le groupe de formule 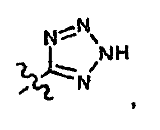
p) les groupes alkyle portant un ou plusieurs (un, par exemple) substituants hydroxy (par exemple un groupe de formule -(CH₂)_{q}OH où l'indice q vaut de 1 à 6, habituellement 1 ou 2 et de préférence 1),
q) les groupes alkyle portant un ou plusieurs (un, par exemple) substituants symbolisés par -NR¹³R¹⁴ (par exemple un groupe de formule -(CH₂)_{q}NR¹³R¹⁴ où l'indice q vaut de 1 à 6, habituellement 1 ou 2 et de préférence 1),
r) et les groupes de formule -N(R¹³)SO₂R¹⁴ (où par exemple R¹³ représente un atome d'hydrogène et R¹⁴ représente un groupe alkyle, tel un groupe méthyle) ;
- R¹⁰ et R¹¹ représentent dans chaque cas des entités identiques ou différentes qui sont choisies indépendamment parmi les atomes d'hydrogène et d'halogène, les groupes alkyle (par exemple en C₁₋₆, tel un groupe méthyle), les groupes de formule -CF₃, -OCF₃, -NR¹³R¹⁴, -NR¹³C(O)NR¹³R¹⁴, -OH, -C(O)OR¹³, -SH, -SOₜNR¹³R¹⁴, -SO₂R¹³, -NHC(O)R¹³, -NHSO₂NR¹³R¹⁴, -NHSO₂R¹³, -C(O)NR¹³R^{I4}, -C(O)NR¹³OR¹⁴ ou -OC(O)R¹³, et le groupe cyano ;
- R¹² représente une entité choisie dans l'ensemble formé par un atome d'hydrogène, un groupe de formule -OC(O)R¹³ et les groupes aryle avec ou sans substituant(s), hétéroaryle avec ou sans substituant(s), aryl-alkyle avec ou sans substituant(s), cycloalkyle avec ou sans substituant(s), alkyle avec ou sans substituant(s), cycloalkyl-alkyle avec ou sans substituant(s), ou hétéroaryl-alkyle avec ou sans substituant(s),
où lesdits groupes avec substituant(s) représentés par R¹² portent 1 à 6 substituants qui sont choisis, chacun indépendamment, parmi ceux qui sont symbolisés par R⁹ ;
- R¹³ et R¹⁴ représentent, dans chaque cas, des entités identiques ou différentes qui sont choisies indépendamment parmi l'atome d'hydrogène et les groupes alkyle avec ou sans substituant(s), aryle avec ou sans substituant(s), hétéroaryle avec ou sans substituant(s), aryl-alkyle avec ou sans substituant(s), hétéroaryl-alkyle avec ou sans substituant(s), cycloalkyle avec ou sans substituant(s), cycloalkyl-alkyle avec ou sans substituant(s), hétérocyclyle avec ou sans substituant(s), fluoroalkyle avec ou sans substituant(s), et hétérocycloalkyl-alkyle avec ou sans substituant(s),
où lesdits groupes avec substituant(s) représentés par R¹³ et R¹⁴ portent 1 à 6 substituants qui sont choisis, chacun indépendamment, dans l'ensemble que constituent les groupes alkyle, trifluorométhyle -CF₃, hydroxyle -OH, alcoxy, aryle, aryl-alkyle, fluoroalkyle, cycloalkyle, cycloalkyl-alkyle, hétéroaryle et hétéroaryl-alkyle, les groupes de formule -N(R⁴⁰)₂, -C(O)OR¹⁵, -C(O)NR¹⁵R¹⁶, -S(O)ₜNR¹⁵R¹⁶, -C(O)R¹⁵, -SO₂R¹⁵ où R¹⁵ ne représente pas un atome d'hydrogène,
ou -NHC(O)NR¹⁵R¹⁶, et les atomes d'halogène,
ou bien les deux entités représentées par R¹³ et R¹⁴ peuvent former, conjointement avec l'atome d'azote auquel elles sont liées dans les groupes symbolisés par -NR¹³R¹⁴, -C(O)NR¹³R¹⁴, -SO₂NR¹³R¹⁴, -OC(O)NR¹³R¹⁴, -CONR¹³R¹⁴, -NR¹³C(O)NR¹³R¹⁴, -SOₜNR¹³R¹⁴ ou -NHSO₂NR¹³R¹⁴, un hétérocycle saturé, avec ou sans substituant(s), lequel cycle peut en option comporter un chaînon hétéroatomique supplémentaire choisi parmi un atome d'oxygène, un atome de soufre et un chaînon azoté symbolisé par -NR¹⁸,
où lesdits groupes cycliques à substituant(s) formés par les entités représentées par R¹³ et R¹⁴ portent 1 à 3 substituant(s) qui sont choisis, chacun indépendamment, parmi les groupes alkyle, aryle, hydroxy, hydroxyalkyle, alcoxy, alcoxyalkyle, aryl-alkyle, fluoroalkyle, cycloalkyle, cycloalkyl-alkyle, hétéroaryle, hétéroaryl-alkyle et amino, les groupes de formule -C(O)OR¹⁵, -C(O)NR¹⁵R¹⁶, -S(O)ₜNR¹⁵R¹⁶, -C(O)R¹⁵, -SO₂R¹⁵ où R¹⁵ ne représente pas un atome d'hydrogène, -NHCONR¹⁵R¹⁶ ou -NHC(O)OR¹⁵, les atomes d'halogène et les groupes hétérocycloalcényle ;
- R¹⁵ et R¹⁶ représentent, dans chaque cas, des entités identiques ou différentes qui sont choisies indépendamment parmi l'atome d'hydrogène et les groupes alkyle, aryle, aryl-alkyle, cycloalkyle et hétéroaryle ;
- R¹⁷ représente un groupe alkyl-sulfonyle (-SO₂-alkyle), aryl-sulfonyle, cycloalkyl-sulfonyle ou hétéroaryl-sulfonyle ;
- R¹⁸ représente un atome d'hydrogène, un groupe alkyle, aryle ou hétéroaryle, ou un groupe de formule -C(O)R¹⁹, -SO₂R¹⁹ ou -C(O)NR¹⁹R²⁰ ;
- R¹⁹ et R²⁰ représentent chacun, indépendamment, un groupe alkyle, aryle ou hétéroaryle ;
- R³⁰ représente un groupe alkyle ou cycloalkyle ou un groupe de formule -CN, -NO₂ ou -SO₂R¹⁵ où R¹⁵ ne représente pas un atome d'hydrogène;
- les symboles R³¹ représentent chacun, indépendamment, un groupe alkyle avec ou sans substituant(s), aryle avec ou sans substituant(s), hétéroaryle avec ou sans substituant(s), ou cycloalkyle avec ou sans substituant(s),
où lesdits groupes avec substituant(s) représentés par R³¹ portent 1 à 6 substituants qui sont choisis, chacun indépendamment, parmi ceux qui sont symbolisés par R⁹ ;
- les symboles R⁴⁰ représentent chacun, indépendamment, un atome d'hydrogène ou un groupe alkyle ou cycloalkyle ;
- et l'indice t vaut 0, 1 ou 2.

8. Composé conforme à la revendication 7, dans lequel B représente un groupe choisi parmi ceux de formules :
1) 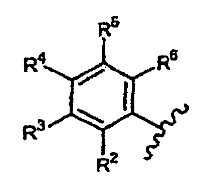 où R³ représente un groupe de formule -C(O)NR¹³R¹⁴ et tous les autres symboles ont les significations indiquées dans la revendication 7 à propos de la formule IA;
2) 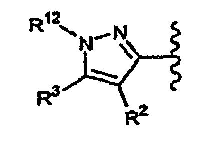 où tous les symboles ont les significations indiquées dans la revendication 7 à propos de la formule IA ;
3) 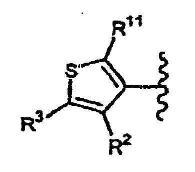 où tous les symboles ont les significations indiquées dans la revendication 7 à propos de la formule IA ;
4) 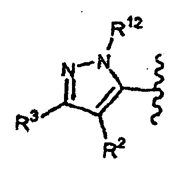 où tous les symboles ont les significations indiquées dans la revendication 7 à propos de la formule IA ;
5) 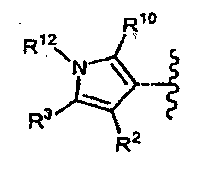 où tous les symboles ont les significations indiquées dans la revendication 7 à propos de la formule IA ;
6) 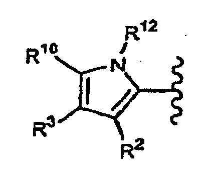 où tous les symboles ont les significations indiquées dans la revendication 7 à propos de la formule IA ;
7) 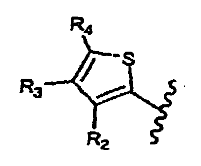 où tous les symboles ont les significations indiquées dans la revendication 7 à propos de la formule IA ;
8) 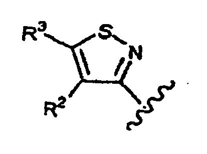 où tous les symboles ont les significations indiquées dans la revendication 7 à propos de la formule IA ;
9) 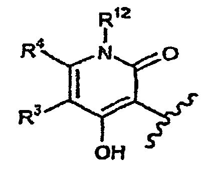 où tous les symboles ont les significations indiquées dans la revendication 7 à propos de la formule IA ;
10) 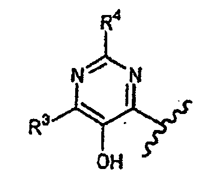 où tous les symboles ont les significations indiquées dans la revendication 7 à propos de la formule IA ;
12) 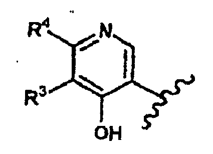 où tous les symboles ont les significations indiquées dans la revendication 7 à propos de la formule IA ;
13) 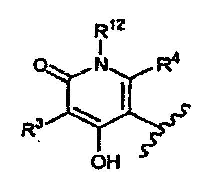
où tous les symboles ont les significations indiquées dans la revendication 7 à propos de la formule IA.

9. Composé conforme à la revendication 7, dans lequel B représente un groupe de formule : 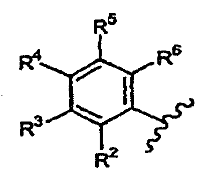 où R³ représente un groupe de formule -C(O)NR¹³R¹⁴.

10. Composé conforme à la revendication 9, dans lequel R¹³ et R¹⁴ représentent des groupes alkyle identiques ou différents.

11. Composé conforme à la revendication 7, dans lequel B représente un groupe de formule :

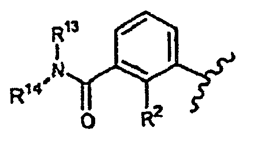

12. Composé conforme à la revendication 11, dans lequel R¹³ et R¹⁴ représentent des groupes alkyle identiques ou différents.

13. Composé conforme à la revendication 7, dans lequel B représente un groupe de formule :

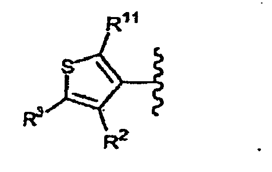

14. Composé conforme à la revendication 13, dans lequel R¹¹ représente un atome d'hydrogène.

15. Composé conforme à la revendication 13 ou 14, dans lequel R³ représente un groupe de formule -C(O)NR¹³R¹⁴.

16. Composé conforme à l'une des revendications 13 à 15, dans lequel R¹³ et R¹⁴ représentent chacun, indépendamment, un groupe alkyle, hétéroaryle sans substituant ou hétéroaryle à substituant(s).

17. Composé conforme à la revendication 16, dans lequel l'un des symboles R¹³ et R¹⁴ représente un groupe alkyle.

18. Composé conforme à la revendication 17, dans lequel ledit groupe alkyle est un groupe méthyle.

19. Composé conforme à la revendication 7, dans lequel B représente un groupe de formule :

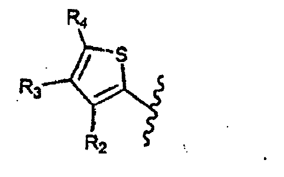

20. Composé conforme à l'une des revendications 7 à 19, dans lequel A représente un groupe de formule : 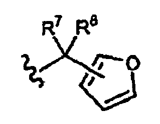 dont le cycle furanique porte un ou des substituant(s) ou n'en porte aucun.

21. Composé conforme à la revendication 20, dans lequel le cycle furanique porte un ou des substituant(s).

22. Composé conforme à la revendication 21, dans lequel le cycle furanique porte, en tant que substituant(s), au moins un groupe alkyle.

23. Composé conforme à l'une des revendications 20 à 22, dans lequel R⁷ et R⁸ représentent des entités identiques ou différentes, qui sont choisies, pour chacun de ces symboles, parmi un atome d'hydrogène et les groupes alkyle.

24. Composé conforme à la revendication 23, dans lequel R⁷ représente un atome d'hydrogène et R⁸ représente un groupe alkyle.

25. Composé conforme à la revendication 1, choisi dans l'ensemble formé par les suivants :

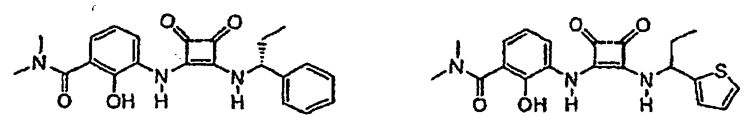

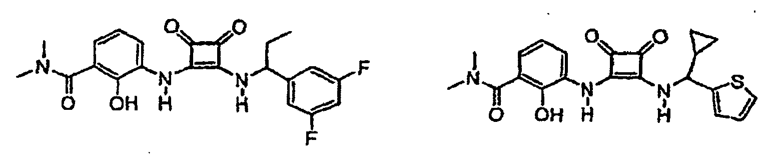

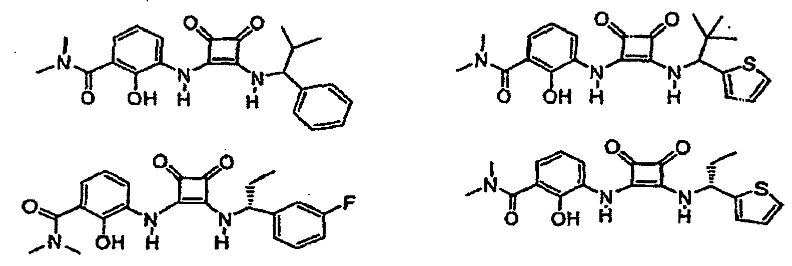

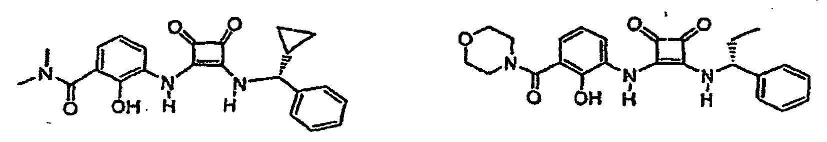

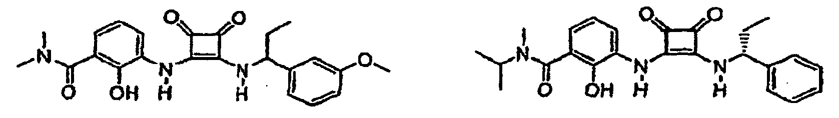

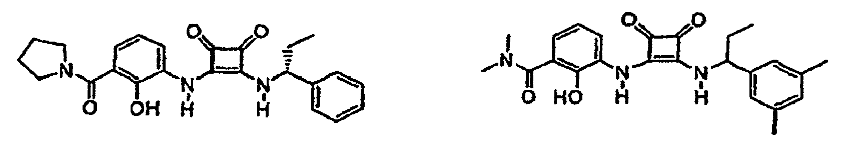

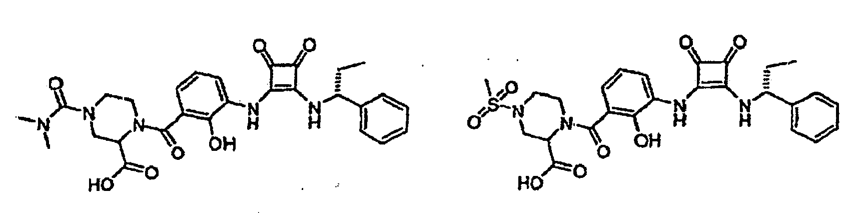

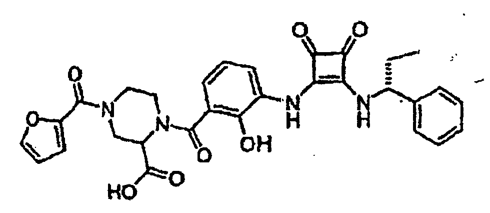

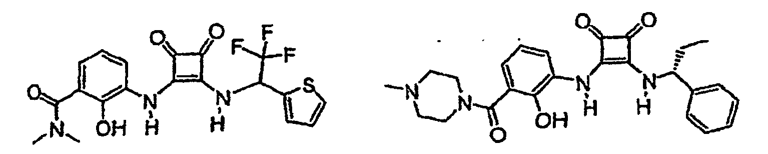

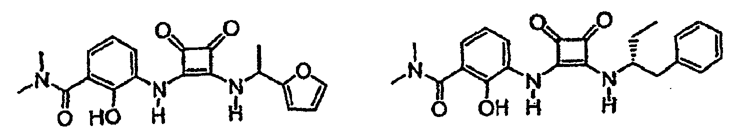

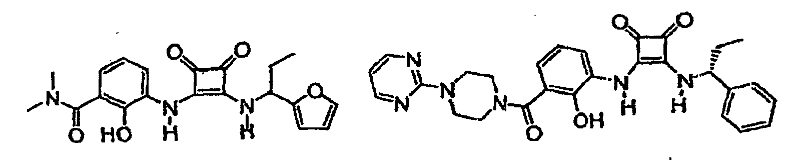

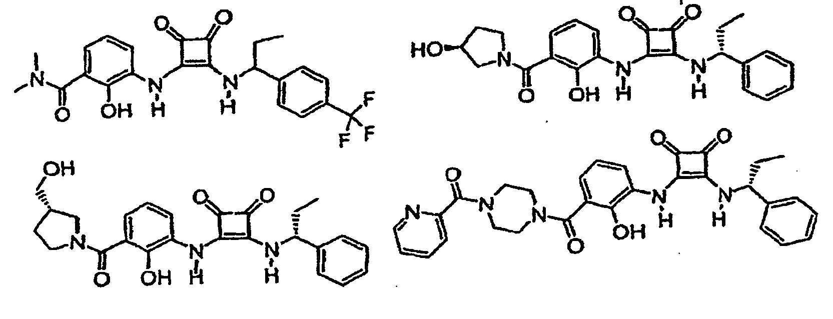

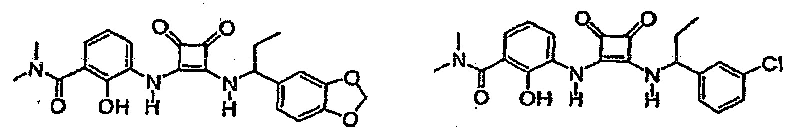

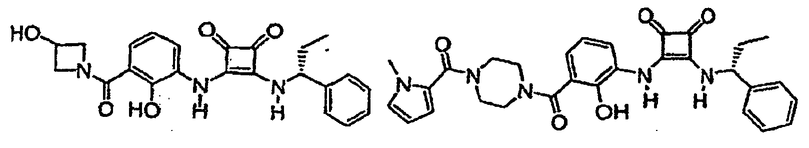

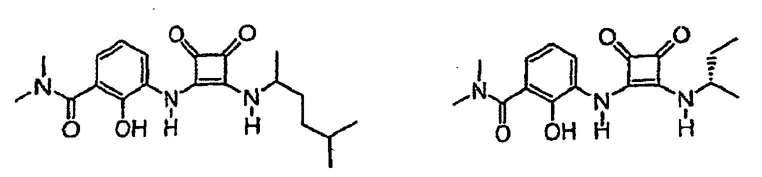

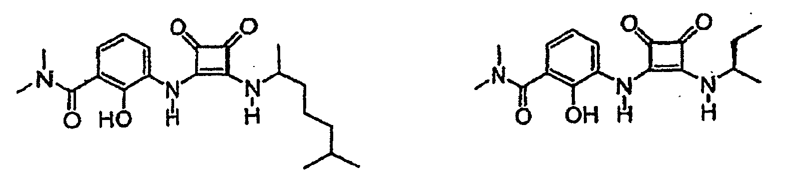

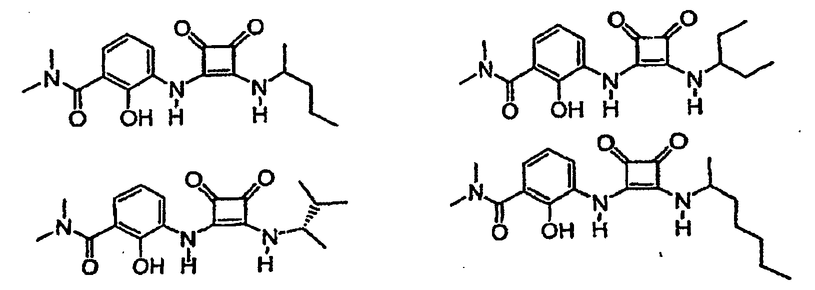

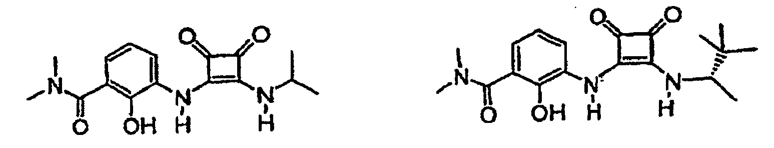

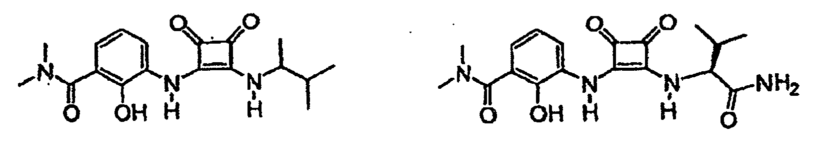

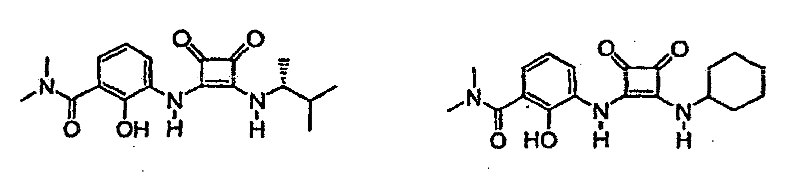

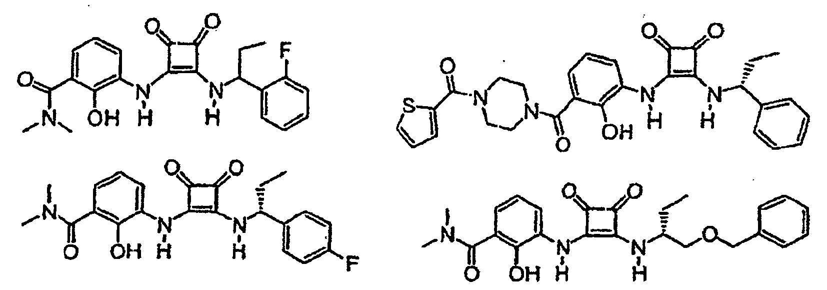

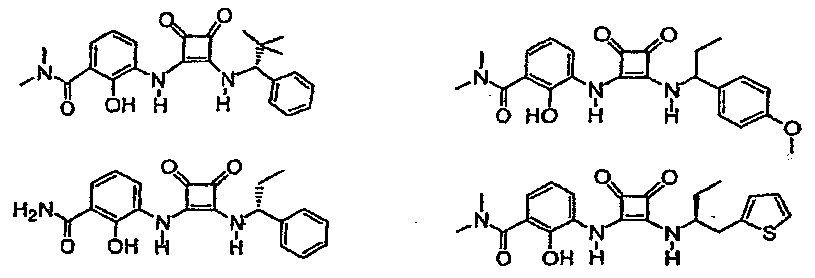

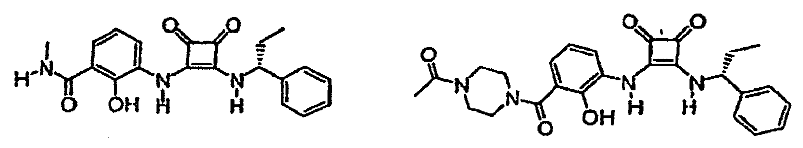

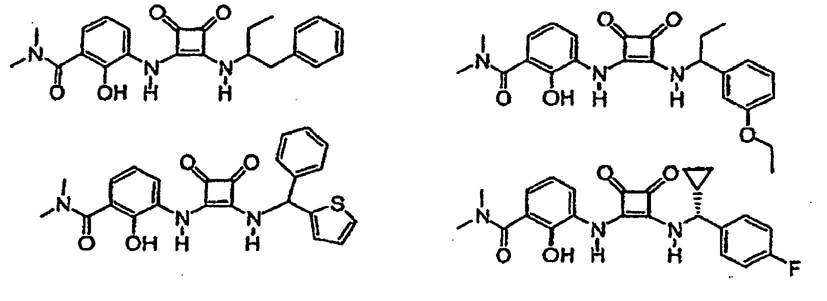

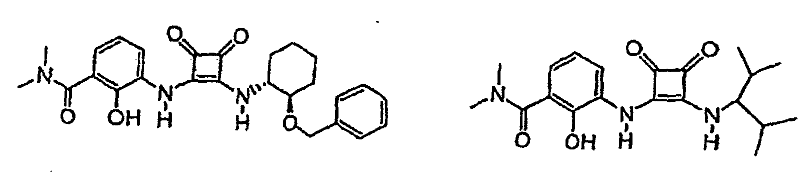

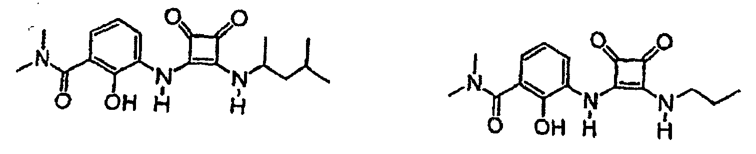

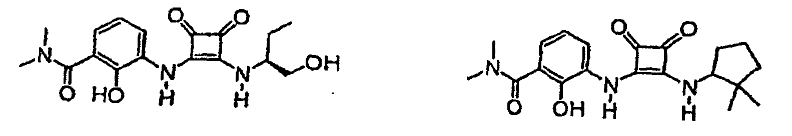

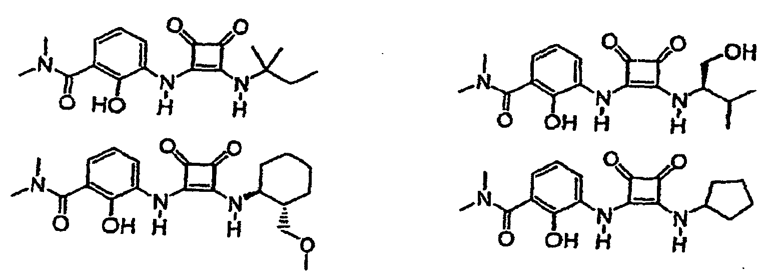

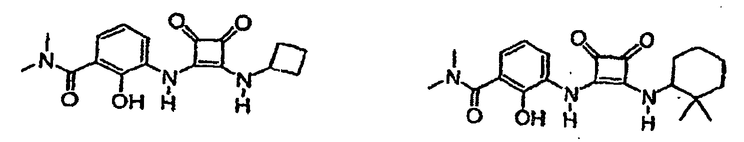

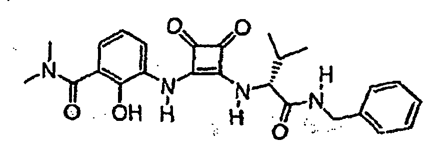

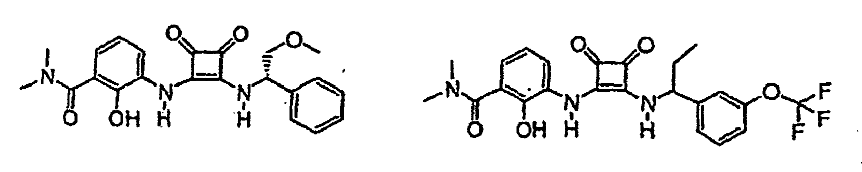

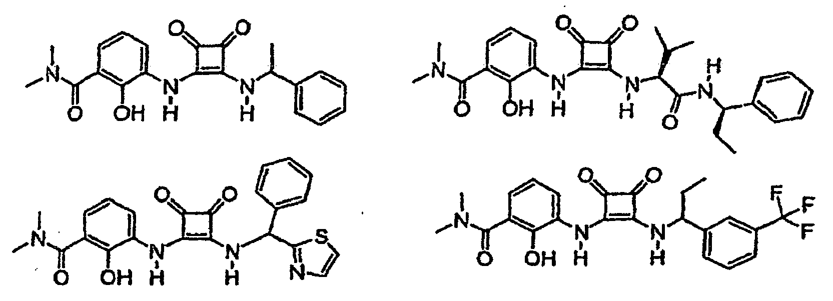

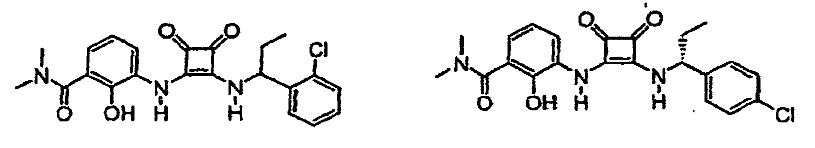

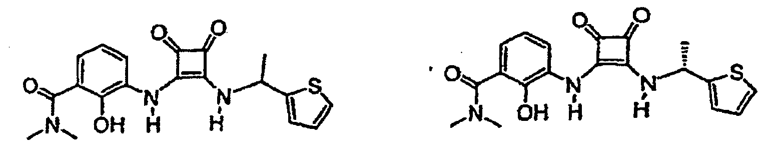

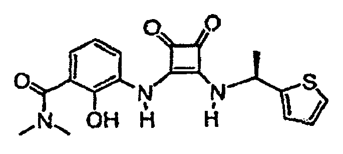

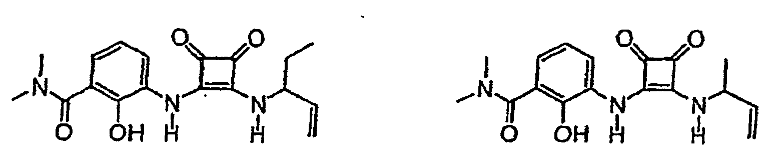

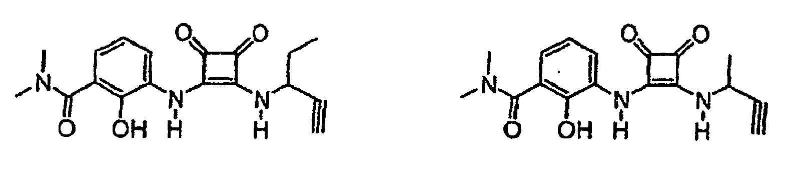

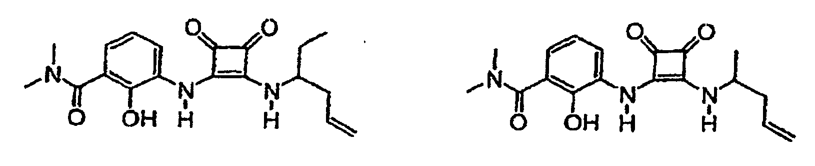

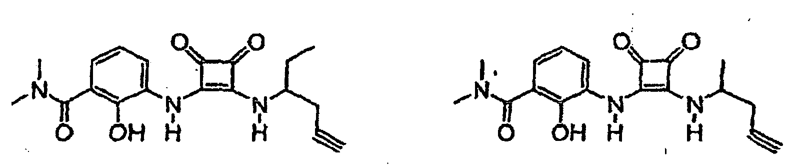

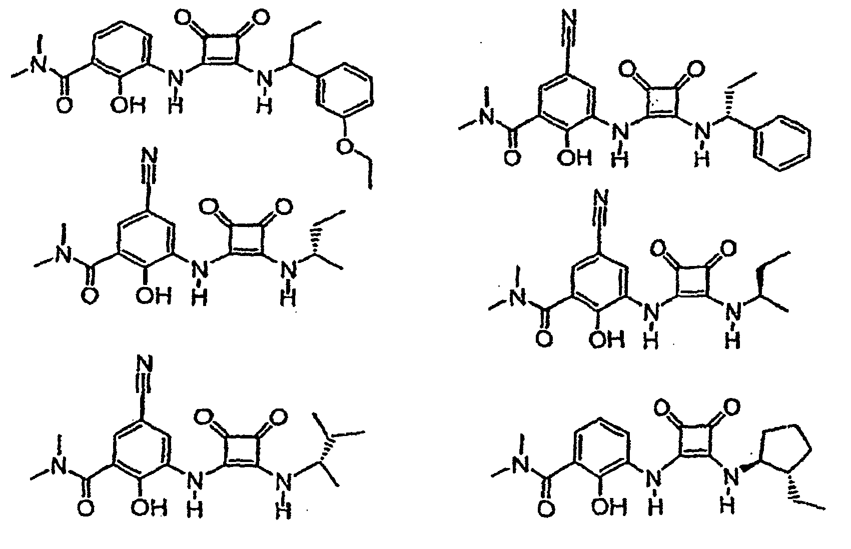

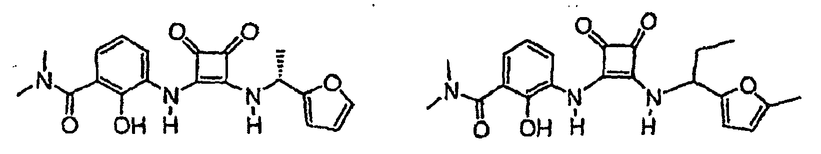

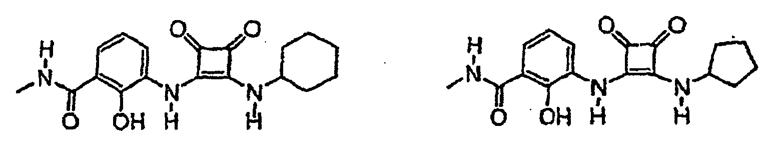

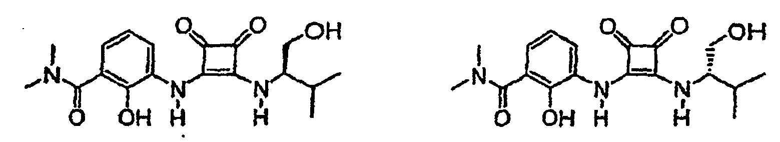

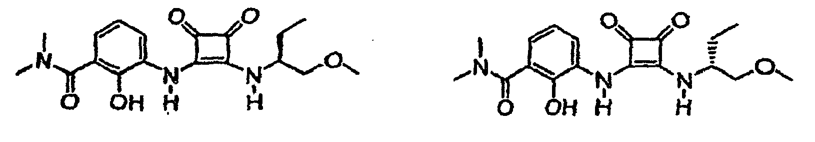

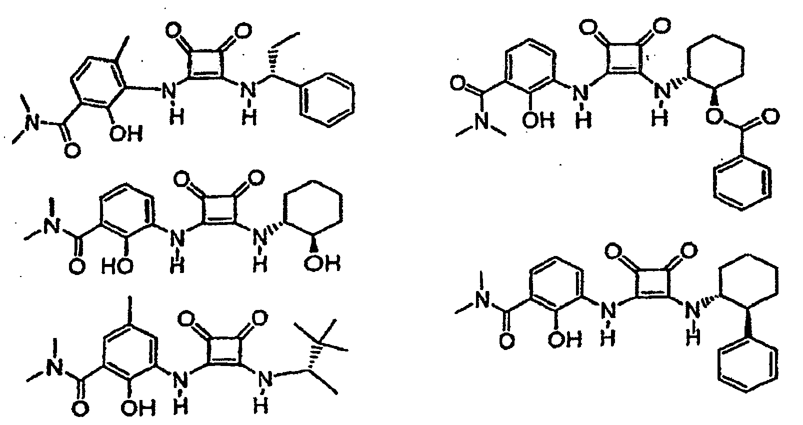

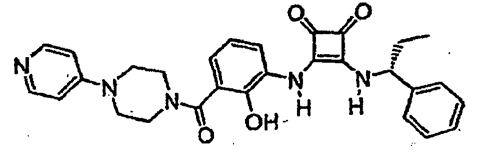

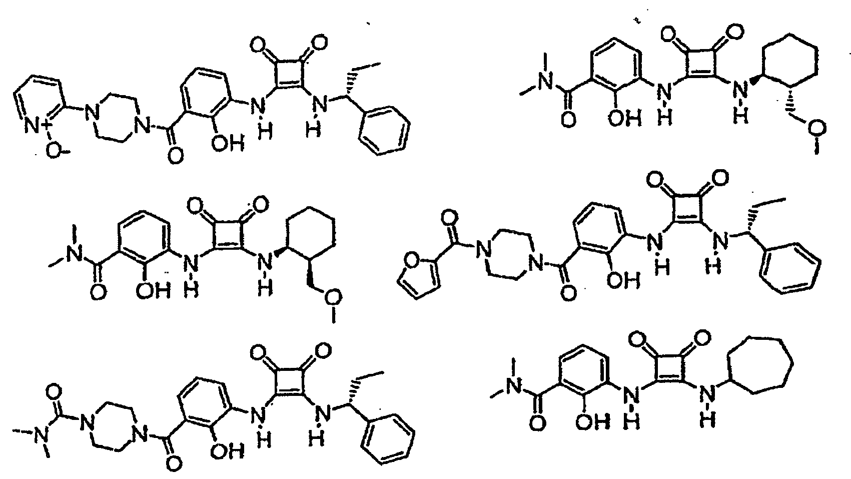

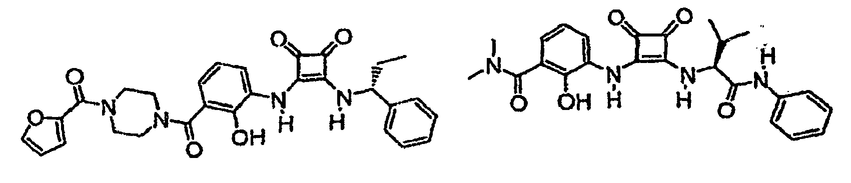

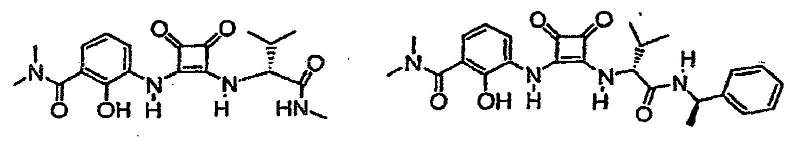

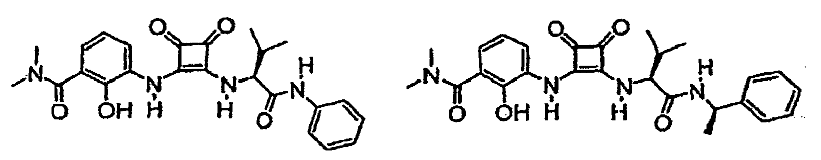

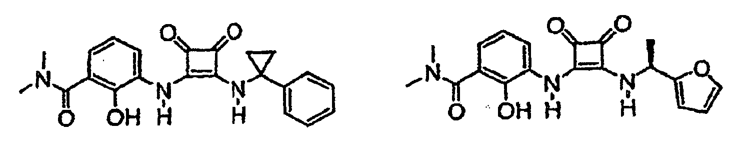

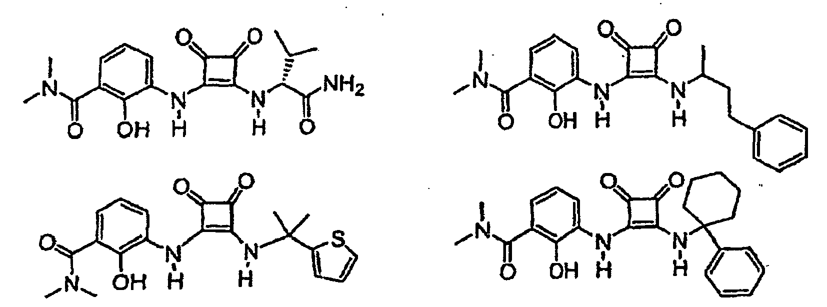

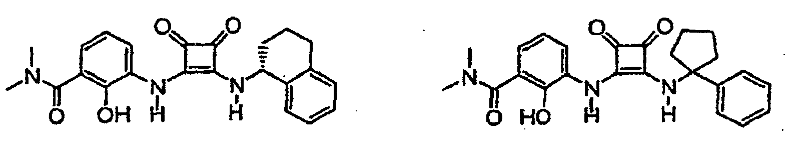

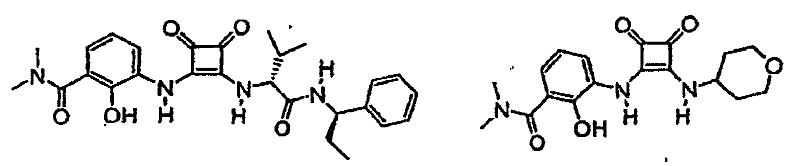

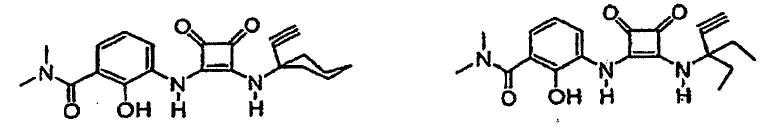

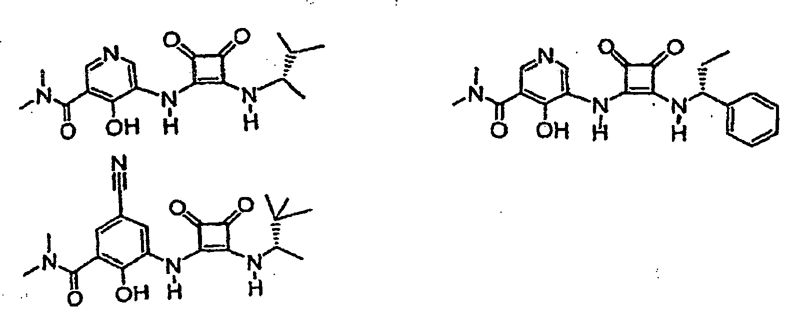

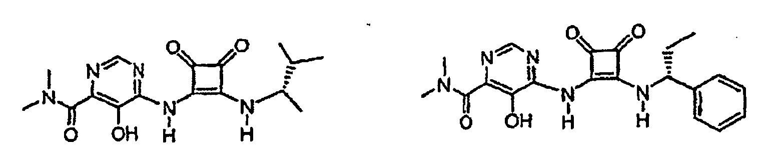

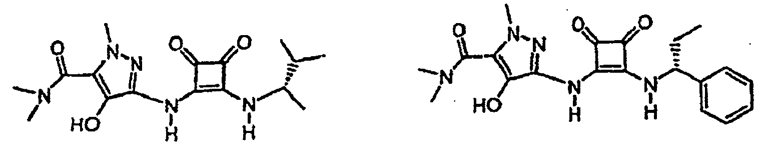

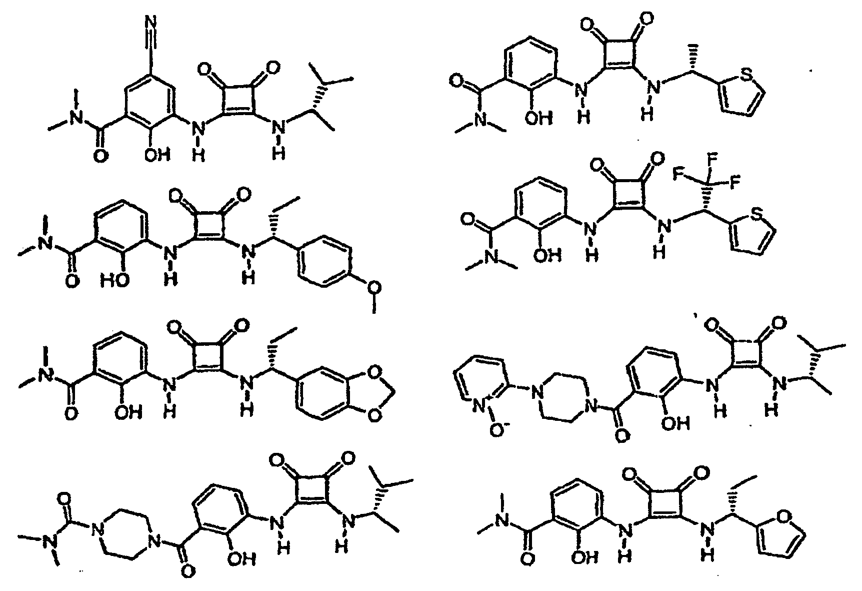

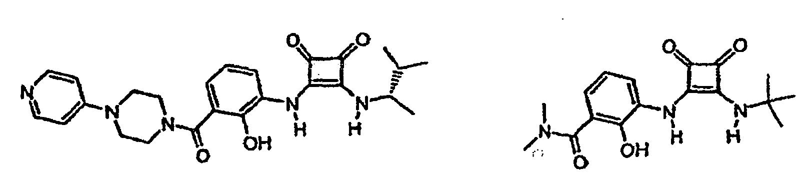

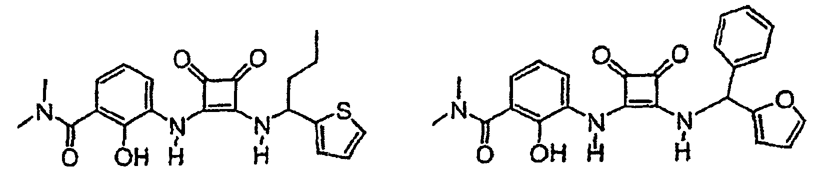

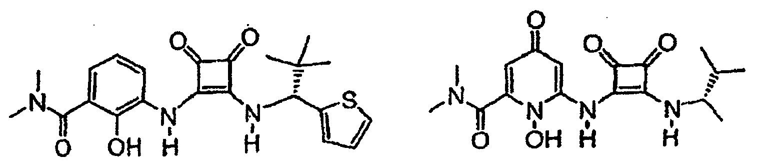

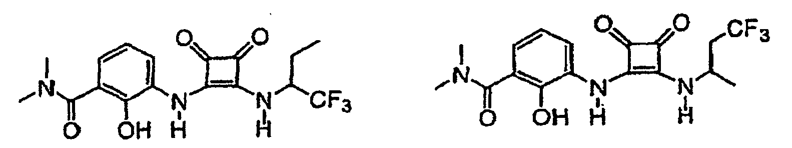

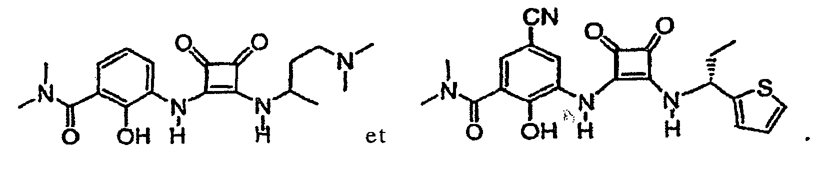

26. Composé conforme à la revendication 1, choisi dans l'ensemble formé par les suivants :

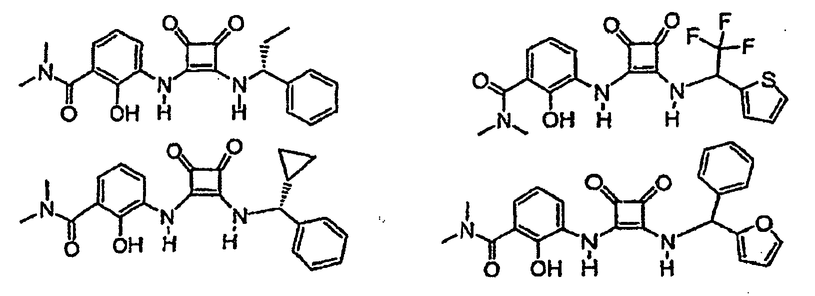

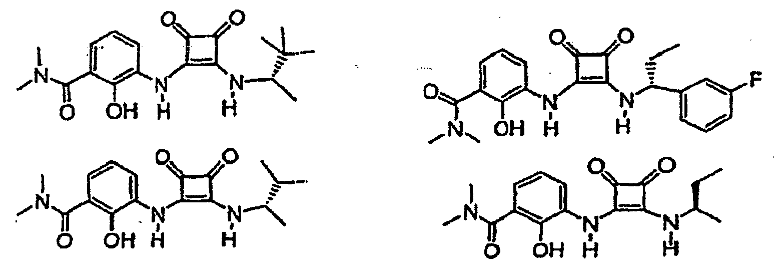

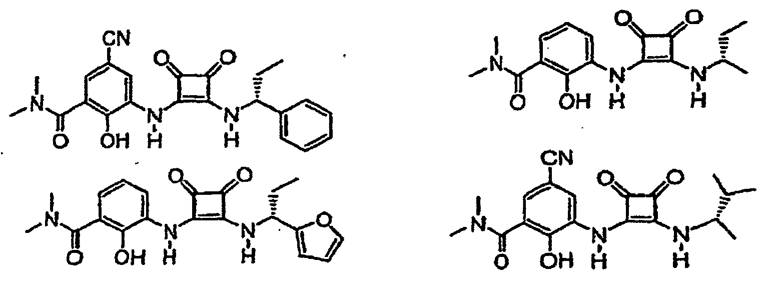

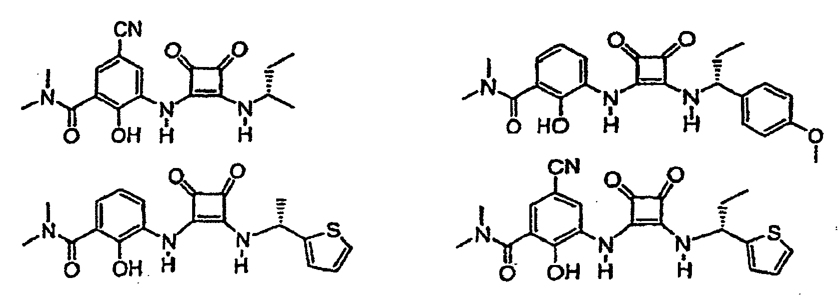

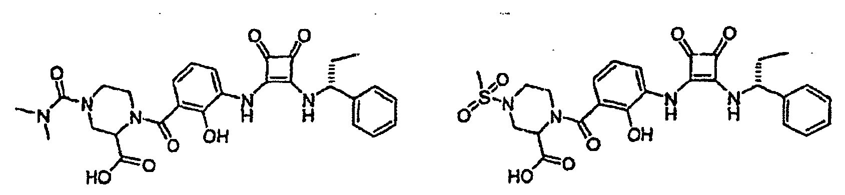

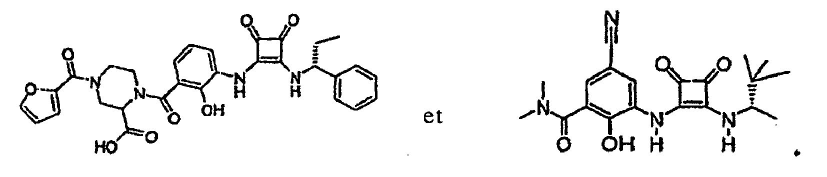

27. Composé de formule 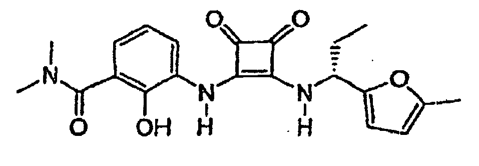 ou sel ou solvat, pharmacologiquement admissible, de ce composé.

28. Composé de formule 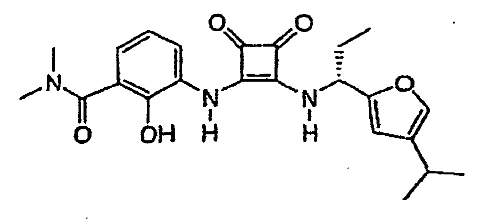 ou sel ou solvat, pharmacologiquement admissible, de ce composé.

29. Composé de formule 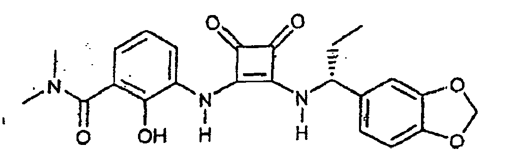 ou sel ou solvat, pharmacologiquement admissible, de ce composé.

30. Composé de formule 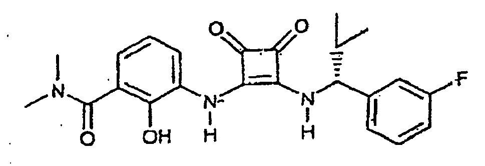 ou sel ou solvat, pharmacologiquement admissible, de ce composé.

31. Composé de formule 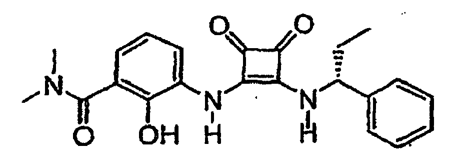 ou sel ou solvat, pharmacologiquement admissible, de ce composé.

32. Composé de formule 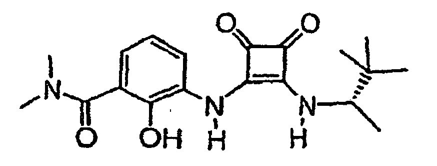 ou sel ou solvat, pharmacologiquement admissible, de ce composé.

33. Composé de formule 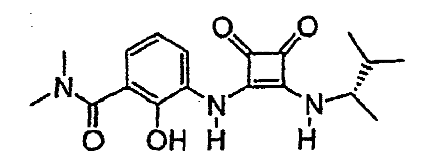 ou sel ou solvat, pharmacologiquement admissible, de ce composé.

34. Composé de formule 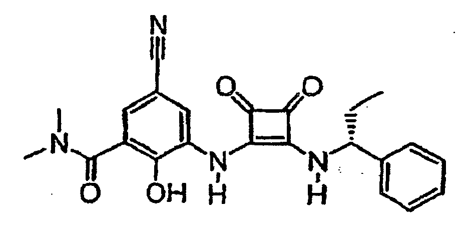 ou sel ou solvat, pharmacologiquement admissible, de ce composé.

35. Composé de formule 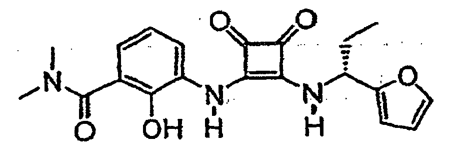 ou sel ou solvat, pharmacologiquement admissible, de ce composé.

36. Composé de formule 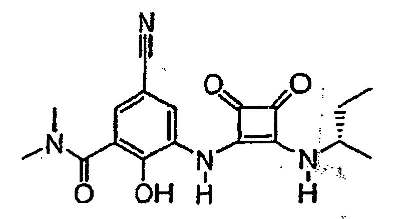 ou sel ou solvat, pharmacologiquement admissible, de ce composé.

37. Composé de formule 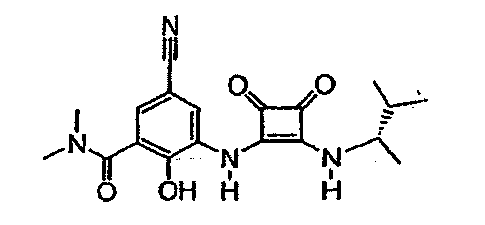 ou sel ou solvat, pharmacologiquement admissible, de ce composé.

38. Composé de formule 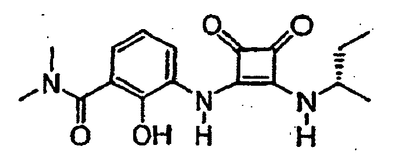 ou sel ou solvat, pharmacologiquement admissible, de ce composé.

39. Composé de formule 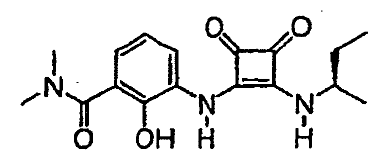 ou sel ou solvat, pharmacologiquement admissible, de ce composé.

40. Composé de formule 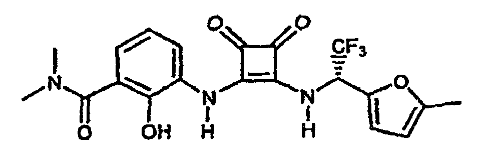 ou sel ou solvat, pharmacologiquement admissible, de ce composé.

41. Composé de formule 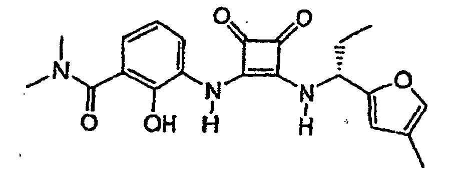 ou sel ou solvat, pharmacologiquement admissible, de ce composé.

42. Composé de formule 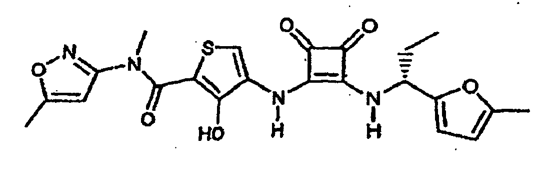 ou sel ou solvat, pharmacologiquement admissible, de ce composé.

43. Emploi d'un composé conforme à l'une des revendications 1 à 42 en vue de la fabrication d'un médicament conçu pour le traitement d'une maladie à médiation par chimiokine, laquelle maladie à médiation par chimiokine est choisie dans l'ensemble formé par les suivantes : psoriasis, dermatite atopique, acné, asthme, bronchopneumopathie chronique obstructive, maladies respiratoires de l'adulte, arthrite, maladies inflammatoires intestinales, maladie de Crohn, colite ulcéreuse, choc septique, choc endotoxique, septicémie à germes Gram-négatifs, syndrome de choc toxique, ictus, lésions de reperfusion cardiaque ou rénale, glomérulonéphrite, thromboses, maladie d'Alzheimer, réactions du greffon contre l'hôte, rejets d'allogreffes, mucoviscidose, malaria, syndrome de détresse respiratoire aiguë, réactions d'hypersensibilité retardée, athérosclérose, et ischémie cérébrale ou cardiaque.

44. Emploi d'un composé conforme à l'une des revendications 1 à 42 en vue de la fabrication d'un médicament conçu pour le traitement d'un cancer.

45. Emploi d'un composé conforme à l'une des revendications 1 à 42 en vue de la fabrication d'un médicament conçu pour le traitement d'un cancer, combiné avec l'emploi d'un agent anti-cancéreux en vue de la fabrication d'un médicament conçu pour le traitement d'un cancer.

46. Emploi conforme à la revendication 45, dans lequel l'agent anti-cancéreux est choisi dans l'ensemble formé par les agents alkylants, anti-métabolites, produits naturels et dérivés de tels produits, hormones, anti-hormones, agents anti-angiogéniques, stéroïdes et produits de synthèse.

47. Emploi d'un composé conforme à l'une des revendications 1 à 42 en vue de la fabrication d'un médicament conçu pour inhiber l'angiogenèse.

48. Emploi d'un composé conforme à l'une des revendications 1 à 42 en vue de la fabrication d'un médicament conçu pour inhiber l'angiogenèse, combiné avec l'emploi d'au moins un composé anti-angiogénique en vue de la fabrication d'un médicament conçu pour inhiber l'angiogenèse.

49. Emploi d'un composé conforme à l'une des revendications 1 à 42 en vue de la fabrication d'un médicament conçu pour le traitement d'une maladie choisie dans l'ensemble formé par les suivantes : gingivite, infections virales respiratoires, infections à virus herpétique, hépatites virales, infections à VIH, infections virales associées au syndrome de Kaposi, et athérosclérose.

50. Emploi d'un composé conforme à l'une des revendications 1 à 42 en vue de la fabrication d'un médicament conçu pour le traitement d'une maladie oculaire angiogénique.

51. Emploi conforme à la revendication 50, la maladie oculaire angiogénique étant choisie dans l'ensemble formé par les inflammations oculaires, la rétinopathie du prématuré, la rétinopathie diabétique, la dégénérescence maculaire, de préférence de type humide, et la néovascularisation de la cornée.

52. Emploi conforme à la revendication 44, le cancer à traiter étant un mélanome, un cancer de l'estomac ou un carcinome pulmonaire non à petites cellules.

53. Emploi conforme à la revendication 45, le cancer à traiter étant un mélanome, un cancer de l'estomac ou un carcinome pulmonaire non à petites cellules.

54. Emploi conforme à la revendication 46, le cancer à traiter étant un mélanome, un cancer de l'estomac ou un carcinome pulmonaire non à petites cellules.

55. Composition pharmaceutique comprenant, en quantité efficace, un composé conforme à l'une des revendications 1 à 42, conjointement avec un véhicule pharmacologiquement admissible.

56. Sel de sodium d'un composé conforme à l'une des revendications 1 à 42.

57. Sel de calcium d'un composé conforme à l'une des revendications 1 à 42.

58. Composition pharmaceutique comprenant, en quantité efficace, un composé conforme à l'une des revendications 56 et 57, conjointement avec un véhicule pharmacologiquement admissible.
